# EUROPEAN PATENT APPLICATION

(11) **EP 3 800 188 A1**
(43) Date of publication of application: **07.04.2021**
(21) Application number: 19201191.4
(22) Date of filing: 02.10.2019
(51) Int. Cl.: C07D 487/04, A61P 25/00, A61P 29/00, A61P 35/00, A61P 37/00, A61K 31/519

(54) **SUBSTITUTED PYRAZOLOPYRIMIDINES AS IRAK4 INHIBITORS**

(71) Applicant: Bayer AG, 51373 Leverkusen (DE); Bayer Pharma Aktiengesellschaft, 13353 Berlin (DE)
(72) Inventor: Bothe, Ulrich, 13353 Berlin (DE); Briem, Hans, 13353 Berlin (DE); Günther, Judith, 13353 Berlin (DE); Nubbemeyer, Reinhard, 13353 Berlin (DE); Peters, Michaele, 13353 Berlin (DE); Bömer, Ulf, 13353 Berlin (DE)
(74) Representative: BIP Patents

(57) **Abstract**

The present application relates to novel pyrazolopyrimidine derivatives for treatment and/or prophylaxis of diseases and to the use thereof for production of medicaments for treatment and/or prophylaxis of diseases in humans and in animals, especially of proliferative disorders, autoimmune disorders, metabolic and inflammatory disorders characterized by an overreacting immune system, in particular rheumatological disorders, inflammatory skin disorders, cardiovascular disorders, lung disorders, eye disorders, neurological disorders, pain disorders and cancer, in human as well as of allergic and/or inflammatory diseases in animals, especially of atopic dermatitis and/or Flea Allergy Dermatitis, and especially in domestic animals, particularly in dogs.

## Description

The present application relates to novel pyrazolopyrimidine derivatives for treatment and/or prophylaxis of diseases and to the use thereof for production of medicaments for treatment and/or prophylaxis of diseases in humans and in animals, especially of proliferative disorders, autoimmune disorders, metabolic and inflammatory disorders characterized by an overreacting immune system, in particular rheumatological disorders, inflammatory skin disorders, cardiovascular disorders, lung disorders, eye disorders, neurological disorders, pain disorders and cancer, in human as well as of allergic and/or inflammatory diseases in animals, especially of atopic dermatitis and/or Flea Allergy Dermatitis, and especially in domestic animals, particularly in dogs.

The present invention relates to novel pyrazolopyrimidine derivatives of the general formula (I) which inhibit interleukin-1 receptor-associated kinase 4 (IRAK4).
Human IRAK4 (interleukin-1 receptor-associated kinase 4) plays a key role in the activation of the immune system. Therefore, this kinase is an important therapeutic target molecule for the development of inflammation-inhibiting substances. IRAK4 is expressed by a multitude of cells and mediates the signal transduction of Toll-like receptors (TLR), except for TLR3, and receptors of the interleukin (IL)-1β family consisting of the IL-1R (receptor), IL-18R, IL-33R and IL-36R (Janeway, Medzhitov, Annu. Rev. Immunol., 2002 20:197-216; Dinarello, Annu. Rev. Immunol., 2009, 27(1): 519-550; Flannery and Bowie, Biochemical Pharmacology, 2010, 80(12): 1981-1991).
Neither IRAK4 knockout mice nor human cells from patients lacking IRAK4 react to stimulation by TLRs (except for TLR3) and the IL-1β family (Suzuki, Suzuki, et al., Nature, 2002, 416(6882): 750-756; Davidson, Currie, et al., The Journal of Immunology, 2006, 177(11): 8202-8211; Ku, von Bernuth, et al., JEM, 2007, 204(10): 2407-2422; Kim, Staschke, et al., JEM, 2007, 204(5): 1025-1036).
The binding of the TLR ligands or the ligands of the IL-1β family to the respective receptor leads to recruitment and binding of MyD88 [Myeloid differentiation primary response gene (88)] to the receptor. As a result, MyD88 interacts with IRAK4,
resulting in the formation of an active complex which interacts with and activates the kinases IRAK1 or IRAK2 (Kollewe, Mackensen, et al., Journal of Biological Chemistry, 2004, 279(7): 5227-5236; Precious et al., J. Biol. Chem., 2009, 284(37): 25404-25411). As a result of this, the NF (nuclear factor)-κB signaling pathway and the MAPK (mitogen-activated protein kinase) signal pathway is activated (Wang, Deng, et al., Nature, 2001, 412(6844): 346-351). The activation of both the NF-κB signal pathway and of the MAPK signal pathway leads to processes associated with different immune processes. For example, there is increased expression of various inflammatory signal molecules and enzymes such as cytokines, chemokines and COX-2 (cyclooxygenase-2), for example, and increased mRNA stability of inflammation-associated genes, for example COX-2, IL-6 (interleukin-6), IL-8 (Holtmann, Enninga, et al., Journal of Biological Chemistry, 2001, 276(5): 3508-3516; Datta, Novotny, et al., The Journal of Immunology, 2004, 173(4): 2755-2761). Furthermore, these processes may be associated with the proliferation and differentiation of particular cell types, for example monocytes, macrophages, dendritic cells, T cells and B cells (Wan, Chi, et al., Nat Immunol, 2006, 7(8): 851-858; McGettrick and J. O'Neill, British Journal of Haematology, 2007, 139(2): 185-193).

The central role of IRAK4 in the pathology of various inflammatory disorders had already been shown by direct comparison of wild-type (WT) mice with genetically modified animals having a kinase-inactivated form of IRAK4 (IRAK4 KDKI). IRAK4 KDKI animals have an improved clinical picture in the animal model of multiple sclerosis, atherosclerosis, myocardial infarction and Alzheimer's disease (Rekhter, Staschke, et al., Biochemical and Biophysical Research Communication, 2008, 367(3): 642-648; Maekawa, Mizue, et al., Circulation, 2009, 120(14): 1401-1414; Staschke, Dong, et al., The Journal of Immunology, 2009, 183(1): 568-577; Kim, Febbraio, et al., The Journal of Immunology, 2011, 186(5): 2871-2880; Cameron, Tse, et al., The Journal of Neuroscience, 2012, 32(43): 15112-15123). Furthermore, it was found that deletion of IRAK4 in the animal model protects against virus-induced myocarditis by virtue of an improved anti-viral reaction with simultaneously reduced systemic inflammation (Valaperti, Nishii, et al., Circulation, 2013, 128(14): 1542-1554). It has also been shown that the expression of IRAK4 correlates with the degree of Vogt-Koyanagi-Harada syndrome (Sun, Yang, et al., PLoS ONE, 2014, 9(4): e93214. doi:10.1371). In addition, the high relevance of IRAK4 for immune complex-mediated IFNα (interferon-alpha) production by plasmacytoid dendritic cells, a key process in the pathogenesis of systemic lupus erythematosus (SLE), has been shown (Chiang et al., The Journal of Immunology, 2010, 186(2):1279-88). Furthermore, the signaling pathway is associated with obesity (Ahmad, R., P. Shihab, et al., Diabetology & Metabolic Syndrome, 2015, 7: 71).
As well as the essential role of IRAK4 in congenital immunity, there are also hints that IRAK4 influences the differentiation of what are called the Th17 T cells, components of adaptive immunity. In the absence of IRAK4 kinase activity, fewer IL-17-producing T cells (Th17 T cells) are generated compared to WT mice. The inhibition of IRAK4 enables the prophylaxis and/or treatment of atherosclerosis, type 1 diabetes mellitus, rheumatoid arthritis, spondyloarthritis (especially psoriatic spondyloarthritis and Bechterev's disease), lupus erythematosus, psoriasis, vitiligo, giant cell arteritis, chronic inflammatory bowel disorder and viral disorders, for example HIV (human immunodeficiency virus), hepatitis virus (Staschke, et al., The Journal of Immunology, 2009, 183(1): 568-577; Marquez, Hernández-Rodríguez et al., Ann Rheum Dis, 2014, 73:1742-1745; Zambrano-Zaragoza, et al., International Journal of Inflammation, 2014, 651503; Wang, et al., Experimental and Therapeutic Medicine, 2015, (1):250-256; Ciccia, et al., Rheumatology, 2015, 54(12): 2264-72).

Owing to the central role of IRAK4 in the MyD88-mediated signal cascade of TLRs (except for TLR3) and the IL-1 receptor family, the inhibition of IRAK4 can be utilized for the prophylaxis and/or treatment of disorders mediated by the receptors mentioned. TLRs as well as components of the IL-1 receptor family are involved in the pathogenesis of rheumatic arthritis, psoriatic arthritis, myasthenia gravis, vasculitis, for example Behçet's disease, granulomatosis with polyangiitis and giant cell arteritis, pancreatitis, systemic lupus erythematosus, dermamyositis and polymyositis, metabolic syndrome including, for example, insulin resistance, hypertension, dyslipoproteinaemia and adipositas, polycystic ovarian syndrome (PCOS), diabetes mellitus (type 1 and type 2), diabetic nephropathy, osteoarthritis, Sjögren syndrome and sepsis (Yang, Tuzun, et al., J Immunol, 2005, 175(3):2018-25; Candia, Marquez et al., The Journal of Rheumatology, 2007, 34(2):374-9; Scanzello, Plaas, et al. Curr Opin Rheumatol, 2008, Scanzello; Deng, Ma-Krupa, et al., Circ Res, 2009, 104(4):488-95; Roger, Froidevaux, et al, PNAS, 2009, 106(7): 2348-2352; Devaraj, Tobias, et al., Arterioscler Thromb Vasc Biol, 2011, 31(8):1796-804; Kim, Cho, et al., Clin Rheumatol, 2010, 29(3):273-9; Carrasco et al., Clinical and Experimental Rheumatology, 2011, 29(6):958-62; Gambuzza, Licata, et al., Journal of Neuroimmunology, 2011, 239(1-2):1-12; Fresno, Archives Of Physiology And Biochemistry, 2011, 117(3):151-64; Volin and Koch, J Interferon Cytokine Res, 2011, (10):745-51; Akash, Shen, et al., Journal of Pharmaceutical Sciences, 2012, 101(5):1647-58; Goh and Midwood, Rheumatology, 2012, 51(1):7-23; Dasu, Ramirez, et al., Clinical Science, 2012, 122(5): 203-214; Ouziel, Gustot, et al., Am J Patho, 2012, 180(6):2330-9; Ramirez and Dasu, Curr Diabetes Rev, 2012, 8(6):480-8, Okiyama et al., Arthritis Rheum, 2012, 64(11):3741-9; Chen et al., Arthritis Research & Therapy, 2013, 4;15(2):R39; Holle, Windmoller, et al., Rheumatology (Oxford), 2013, 52(7):1183-9; Li, Wang, et al., Pharmacology & Therapeutics, 2013, 138(3): 441-451; Sedimbi, Hagglof, et al., Cell Mol Life Sci, 2013, 70(24): 4795-4808; Caso, Costa, et al., Mediators of Inflammation, 2014, 107421; Cordiglieri, Marolda, et al., J Autoimmun, 2014, 52:74-89; Jialal, Major, et al., J Diabetes Complications, 2014, 28(6):755-61; Kaplan, Yazgan, et al., Scand J Gastroenterol, 2014, 49(9):1124-30; Talabot-Aye, et al., Cytokine, 2014, 69(1):68-74; Zong, Dorph, et al., Ann Rheum Di, 2014, 73(5):913-20; Ballak, Stienstra, et al., Cytokine, 2015, 75(2):280-90; Timper, Seelig, et al., J Diabetes Complications, 2015, 29(7):955-60).
Polycystic Ovary Syndrome (PCOS) is characterized by hyperandrogenism, ovulatory dysfunction and polycystic ovaries. Obesity, metabolic issues, insulin resistance, adiopose inflammation are common features of PCOS, and the resultant hyperinsulinemia promotes hyperandrogenism in the disorder. Chronic low-grade inflammation has emerged as a contributor to the pathogenesis of PCOS. The TLR receptor signaling pathway has been shown to mediate the proinflammatory cytokine upregulation in the macrophage from PCOS patients, thereby contributing to maintain and exacerbate inflammation and insulin resistance in PCOS macrophages. (Escobar-Morreale HF, Nature, 2018, 14(5):270-284, Li et al., Clin Lab, 2017, 63(2):301-311, Gonzalez, K, Steroids 2012, 10;77(4):300-5, Gonzalez, K. et al., Metabolism, 1999, 48(4):437-41).

Skin diseases such as psoriasis, atopic dermatitis, Kindler's syndrome, bullous pemphigoid, allergic contact dermatitis, alopecia areata, acne inversa and acne vulgaris are associated with the IRAK4-mediated TLR signaling pathway or the IL-1R family (Schmidt, Mittnacht, et al., J Dermatol Sci, 1996; Hoffmann, J Investig Dermatol Symp Proc, 1999, 4(3):235-8; Gilliet, Conrad, et al., Archives of Dermatology, 2004, 140(12): 1490-1495; Niebuhr, Langnickel, et al., Allergy, 2008, 63(6): 728-734; Miller, Adv Dermatol, 2008, 24: 71-87; Terhorst, Kalali, et al., Am J Clin Dermatol, 2010, 11(1): 1-10; Viguier, Guigue, et al., Annals of Internal Medicine, 2010, 153(1): 66-67; Cevikbas, Steinhoff, J Invest Dermatol, 2012, 132(5): 1326-1329; Minkis, Aksentijevich, et al., Archives of Dermatology, 2012, 148(6): 747-752; Dispenza, Wolpert, et al., J Invest Dermatol, 2012, 132(9): 2198-2205; Minkis, Aksentijevich, et al., Archives of Dermatology, 2012, 148(6): 747-752; Gresnigt and van de Veerdonk, Seminars in Immunology, 2013, 25(6):458-65; Selway, Kurczab, et al., BMC Dermatology, 2013, 13(1): 10; Sedimbi, Hagglof, et al., Cell Mol Life Sci, 2013, 70(24): 4795-4808; Wollina, Koch, et al. Indian Dermatol Online, 2013, 4(1): 2-11; Foster, Baliwag, et al., The Journal of Immunology, 2014, 192(12):6053-61).
Pulmonary disorders such as pulmonary fibrosis, obstructive pulmonary disease (COPD), acute respiratory distress syndrome (ARDS), acute lung injury (ALI), interstitial lung disease (ILD), sarcoidosis and pulmonary hypertension also show an association with various TLR-mediated signal pathways. The pathogenesis of the pulmonary disorders may be either infectiously mediated or non-infectiously mediated processes (Ramirez Cruz, Maldonado Bernal, et al., Rev Alerg Mex, 2004, 51(6): 210-217; Jeyaseelan, Chu, et al., Infection and Immunity, 2005, 73(3): 1754-1763; Seki, Tasaka, et al., Inflammation Research, 2010, 59(10): 837-845; Xiang, Fan, et al., Mediators of Inflammation, 2010; Margaritopoulos, Antoniou, et al., Fibrogenesis & Tissue Repair, 2010, 3(1): 20; Hilberath, Carlo, et al., The FASEB Journal, 2011, 25(6): 1827-1835; Nadigel, Prefontaine, et al., Respiratory Research, 2011, 12(1): 149; Kovach and Standiford, International Immunopharmacology, 2011, 11(10): 1399-1406; Bauer, Shapiro, et al., Mol Med, 2012, 18: 1509-1518; Deng, Yang, et al., PLoS One, 2013, 8(5): e64375; Freeman, Martinez, et al., Respiratory Research, 2013, 14(1): 13; Dubaniewicz, A., Human Immunology, 2013, 74(12):1550-8). TLRs and also IL-1R family members are also involved in the pathogenesis of other inflammatory disorders such as allergy, Behçet's disease, gout, lupus erythematosus, adult-onset Still's disease, pericarditis and chronic inflammatory bowel disorders such as ulcerative colitis and Crohn's disease, transplant rejection and graft-versus-host reaction, and so inhibition of IRAK4 here is a suitable prophylactic and/or therapeutic approach (Liu-Bryan, Scott, et al., Arthritis & Rheumatism, 2005, 52(9): 2936-2946; Piggott, Eisenbarth, et al., J Clin Inves, 2005, 115(2):459-67; Christensen, Shupe, et al., Immunity, 2006, 25(3): 417-428; Cario, Inflammatory Bowel Diseases, 2010, 16(9): 1583-1597; Nickerson, Christensen, et al., The Journal of Immunology, 2010, 184(4): 1840-1848; Rakoff-Nahoum, Hao, et al., Immunity, 2006, 25(2): 319-329; Heimesaat, Fischer, et al., PLoS ONE, 2007, 2(7): e662; Heimesaat, Nogai, et al., Gut, 2010, 59(8):1079-87; Kobori, Yagi, et al., J Gastroenterol, 2010, 45(10): 999-1007; Schmidt, Raghavan, et al., Nat Immunol, 2010, 11(9):814-9; Shi, Mucsi, et al., Immunological Reviews, 2010, 233(1): 203-217; Leventhal and Schroppel, Kidney Int, 2012, 81(9):826-32; Chen, Lin, et al., Arthritis Res Ther, 2013, 15(2):R39; Hao, Liu, et al., Curr Opin Gastroenterol, 2013, 29(4):363-9; Kreisel and Goldstein, Transplant International, 2013, 26(1):2-10; Li, Wang, et al., Pharmacology & Therapeutics, 2013; Walsh, Carthy, et al., Cytokine & Growth Factor Reviews, 2013, 24(2): 91-104; Zhu, Jiang, et al., Autoimmunity, 2013, 46(7): 419-428; Yap and Lai, Nephrology, 2013, 18(4): 243-255; Vennegaard, Dyring-Andersen, et al., Contact Dermatitis, 2014, 71(4):224-32; D'Elia, Brucato, et al., Clin Exp Rheumatol, 2015, 33(2):294-5; Jain, Thongprayoon, et al., Am J Cardiol., 2015, 116(8):1277-9; Li, Zhang, et al., Oncol Rep., 2015).
Gynaecological disorders mediated by TLR and the IL-1R family, such as adenomyosis, dysmenorrhoea, dyspareunia and endometriosis, especially endometriosis-associated pain and other endometriosis-associated symptoms such as dysmenorrhoea, dyspareunia, dysuria and dyschezia, can be positively influenced by the prophylactic and/or therapeutic use of IRAK4 inhibitors (Akoum, Lawson, et al., Human Reproduction, 2007, 22(5): 1464-1473; Allhorn, Boing, et al., Reproductive Biology and Endocrinology, 2008, 6(1): 40; Lawson, Bourcier, et al., Journal of Reproductive Immunology, 2008, 77(1): 75-84; Sikora, Mielczarek-Palacz, et al., American Journal of Reproductive Immunology, 2012, 68(2): 138-145; Khan, Kitajima, et al., Journal of Obstetrics and Gynaecology Research, 2013, 39(8): 1281-1292; Santulli, Borghese, et al., Human Reproduction, 2013, 27(7): 2001-2009). The prophylactic and/or therapeutic use of IRAK4 inhibitors can also have a positive influence on atherosclerosis (Seneviratne, Sivagurunathan, et al., Clinica Chimica Acta, 2012, 413(1-2): 3-14; Falck-Hansen, Kassiteridi, et al., International Journal of Molecular Sciences, 2013, 14(7): 14008-14023; Sedimbi, Hagglof, et al., Cell Mol Life Sci, 2013, 70(24): 4795-4808).
In addition to the disorders already mentioned, IRAK4-mediated TLR processes have been described in the pathogenesis of eye disorders such as retinal ischemia, keratitis, allergic conjunctivitis, keratoconjunctivitis sicca, macular degeneration and uveitis (Kaarniranta and Salminen, J Mol Med (Berl), 2009, 87(2): 117-123; Sun and Pearlman, Investigative Ophthalmology & Visual Science, 2009, 50(3): 1247-1254; Redfern and McDermott, Experimental Eye Research, 2010, 90(6): 679-687; Kezic, Taylor, et al., J Leukoc Biol, 2011, 90(2): 305-311; Chang, McCluskey, et al., Clinical & Experimental Ophthalmology, 2012, 40(8): 821-828; Guo, Gao, et al., Immunol Cell Biol, 2012, 90(3): 352-357; Lee, Hattori, et al., Investigative Ophthalmology & Visual Science, 2012, 53(9): 5632-5640; Qi, Zhao, et al., Investigative Ophthalmology & Visual Science, 2014, 55(9):5466-75).
The inhibition of IRAK4 is also a suitable therapeutic approach for fibrotic disorders, for example hepatic fibrosis, myocarditis, primary biliary cirrhosis, cystic fibrosis (Zhao, Zhao, et al., Scand J Gastroenterol, 2011, 46(5):627-33; Benias, Gopal, et al., Clin Res Hepatol Gastroenterol, 2012, 36(5):448-54; Yang, L. and E. Seki, Front Physiol, 2012, 22;3:138; Liu, Hu, et al., Biochim Biophys Acta., 2015, 1852(11):2456-66).
By virtue of the key position that IRAK4 has in disorders mediated by TLR- and the IL-1R family, it is possible to treat chronic liver disorders, for example fatty liver hepatitis and especially non-alcoholic fatty liver disease (NAFLD) and/or non-alcoholic steatohepatitis (NASH), alcoholic steatohepatitis (ASH) in a preventative and/or therapeutic manner with IRAK4 inhibitors (Nozaki, Saibara, et al., Alcohol Clin Exp Res, 2004, 28(8 Suppl Proceedings): 106S-110S; Csak, T., A. Velayudham, et al., Am J Physiol Gastrointest Liver Physiol, 2011, 300(3):G433-41; Miura, Kodama, et al., Gastroenterology, 2010, 139(1):323-34; Kamari, Shaish, et al., J Hepatol, 2011, 55(5):1086-94; Ye, Li, et al., Gut, 2012, 61(7):1058-67; Roh, Seki, J Gastroenterol Hepatol, 2013, 28 Suppl 1:38-42; Ceccarelli, S., V. Nobili, et al., World J Gastroenterol, 2014, 20(44):16443-51; Miura, Ohnishi, World J Gastroenterol, 2014, 20(23):7381-91; Stojsavljevic, Palcic, et al., World J Gastroenterol, 2014, 20(48):18070-91).
In addition, IRAK4 inhibition is also a possible therapeutic approach for treatment of kidney dysfunction and kidney disorders as for examples chronic kidney disease (CKD), chronic kidney failure, glomerular diseases, diabetic nephropathy, lupus nephritis, IgA-Nephritis (Morbus Berger), nephrosklersois. These mentioned diseases are associated with the TLR signal transduction pathway as well as with components of the IL-1 receptor familiy (Suzuki, et al., Journal of the American Society of Nephrology, 2008, 19(12):2384-95; Hahn, Cho, et al., Pediatric Nephrology, 2009, 24(7):1329-36; Conti, Spinelli, et al., Clinical Reviews in Allergy & Immunology, 2011;40(3):192-198; Conti, et al., Mediators Inflamm. 2016, 7697592 ; Bao, Na, et al., Journal of Clinical Immunology, 2011, 32(3):587-94; Devaraj, Tobias, et al., Arterioscler Thromb Vasc Biol, 2011, 31(8):1796-804; Rosa Ramirez, and Ravi Krishna Dasu, Curr Diabetes Rev, 2012, 8(6):480-8; Urbonaviciute, Starke, et al. Arthritis & Rheumatism, 2013, 65(6):1612-23; Batal, et al., Transplantation, 2014, 27;97(10):1034-42; Jialal, Major, et al., J Diabetes Complications, 2014, 28(6):755-61; Lin, and Tang, Nephrology Dialysis Transplantation, 2014, 29(4):746-54; Zawada, Rogacev, et al., Epigenetic, 2014, 9(1):161-72; Elsherbiny and Al-Gayyar, Cytokine, 2016, 81:15-22; Yang, et al., Mol Med Rep, 2016, 13(1):3-8).

Because of the central role of IRAK4 in TLR-mediated processes, the inhibition of IRAK4 also enables the treatment and/or prevention of cardiovascular and neurological disorders, for example myocardial reperfusion damage, myocardial infarction, hypertension (Oyama, Blais, et al., Circulation, 2004, 109(6): 784-789; Timmers, Sluijter, et al., Circulation Research, 2008, 102(2): 257-264; Fang and Hu, Med Sci Monit, 2011, 17(4): RA100-109; Bijani, International Reviews of Immunology, 2012, 31(5): 379-395; Bomfim, Dos Santos, et al., Clin Sci (Lond), 2012, 122(11): 535-543; Christia and Frangogiannis, European Journal of Clinical Investigation, 2013, 43(9): 986-995; Thompson and Webb, Clin Sci (Lond), 2013, 125(1): 19-25; Hernanz, Martinez-Revelles, et al., British Journal of Pharmacology, 2015, 172(12):3159-76; Frangogiannis, Curr Opin Cardiol, 2015; Bomfim, Echem, et al., Life Sciences, 2015, 43(9): 986-995, and also Alzheimer's disease, stroke, craniocerebral trauma, amyotrophic lateral sclerosis (ALS) and Parkinson's (Brough, Tyrrell, et al., Trends in Pharmacological Sciences, 2011, 32(10): 617-622; Carty and Bowie, Biochemical Pharmacology, 2011, 81(7): 825-837; Denes, A., E. Pinteaux, et al. (2011). Cerebrovascular Diseases 32(6): 517-527; Kitazawa, Cheng, et al., The Journal of Immunology, 2011, 187(12): 6539-6549; Lim, Kou, et al., The American Journal of Pathology, 2011, 179(3): 1095-1103; Béraud and Maguire-Zeiss, Parkinsonism & Related Disorders, 2012, 18, Supplement 1(0): S17-S20; Denes, Wilkinson, et al., Disease Models & Mechanisms, 2013, 6(4): 1043-1048; Noelker, Morel, et al., Sci. Rep., 2013, 3: 1393; Wang, Wang, et al., Stroke, 2013, 44(9): 2545-2552; Xiang Chao, et al., Rev Neurosci, 2015, 26(4):407-14; Lee, Lee, et al., J Neuroinflammation, 2015, 13;12:90).
Because of the involvement of TLR-mediated signals and IL-1 receptor family-mediated signals via IRAK4 in the case of pruritus and pain, including acute, chronic, inflammatory and neuropathic pain, there may be assumed to be a therapeutic effect in the indications mentioned through the inhibition of IRAK4. Examples of pain include hyperalgesia, allodynia, premenstrual pain, endometriosis-associated pain, post-operative pain, interstitial cystitis, CRPS (complex regional pain syndrome), trigeminal neuralgia, prostatitis, pain caused by spinal cord injury, inflammation-induced pain, lower back pain, cancer pain, chemotherapy-associated pain, HIV treatment-induced neuropathy, burn-induced pain and chronic pain (Wolf, Livshits, et al., Brain, Behavior, and Immunity, 2008, 22(7): 1072-1077; Kim, Lee, et al., Toll-like Receptors: Roles in Infection and Neuropathology, 2009, 336: 169-186; del Rey, Apkarian, et al., Annals of the New York Academy of Sciences, 2012, 1262(1): 101-107; Guerrero, Cunha, et al., European Journal of Pharmacology, 2012, 674(1): 51-57; Kwok, Hutchinson, et al., PLoS ONE, 2012, 7(8): e44232; Nicotra, Loram, et al., Experimental Neurology, 2012, 234(2): 316-329; Chopra and Cooper, J Neuroimmune Pharmacol, 2013, 8(3): 470-476; David, Ratnayake, et al., Neurobiology of Disease, 2013, 54(0): 194-205; Han, Zhao, et al., Neuroscience, 2013, 241(0): 59-66; Liu and Ji, Pflugers Arch., 2013, 465(12): 1671-1685; Stokes, Cheung, et al., Journal of Neuroinflammation, 2013, 9;10:148; Zhao, Zhang, et al., Neuroscience, 2013, 3;253:172-82; Liu, Zhang, et al., Cell Research, 2014; Park, Stokes, et al., Cancer Chemother Pharmacol, 2014, 73(1):25-34; Van der Watt, Wilkinson, et al., BMC Infect Dis, 2014, 10;14:71; Won, K. A., M. J. Kim, et al., J Pain, 2014, 15(3):250-61; Min, Ahmad, et al., Photochem Photobiol., 2015, 91(6):1435-43; Schrepf, Bradley, et al., Brain Behav Immun, 2015, 49:66-74; Wong, L., J. D. Done, et al., Prostate, 2015, 75(1):50-9).
This also applies to some oncological disorders. Particular lymphomas, for example ABC-DLBCL (activated B-cell diffuse large-cell B-cell lymphoma), mantle cell lymphoma and Waldenström's disease, and also chronic lymphatic leukaemia, melanoma, pancreatic tumour and liver cell carcinoma, are characterized by mutations in MyD88 or changes in MyD88 activity which can be treated by an IRAK4 inhibitor (Ngo, Young, et al., Nature, 2011, 470(7332):115-9; Puente, Pinyol, et al., Nature, 2011, 475(7354): 101-105; Ochi, Nguyen, et al., J Exp Med, 2012, 209(9):1671-87; Srivastava, Geng, et al., Cancer Research, 2012, 72(23): 6209-6216; Treon, Xu, et al., New England Journal of Medicine, 2012, 367(9): 826-833; Choi, Kim, et al., Human Pathology, 2013, 44(7): 1375-1381; Liang, Chen, et al., Clinical Cancer Research, 2013, 19(11): 2905-2916). In addition, MyD88 plays an important role in ras-dependent tumours, and so IRAK4 inhibitors are also suitable for treatment thereof (Kfoury, A., K. L. Corf, et al., Journal of the National Cancer Institute, 2013, 105(13): 937-946). There can also be assumed to be a therapeutic effect in breast cancer, ovarian carcinoma, colorectal carcinoma, head and neck carcinoma, lung cancer, prostate cancer through the inhibition of IRAK4, since the indications mentioned are associated with the signaling pathway (Szczepanski, Czystowska, et al., Cancer Res, 2009, 69(7):3105-13; Zhang, He, et al., Mol Biol Rep, 2009, 36(6):1475-81; Wang, Qian, et al., Br J Cancer, 2010, 102(5):908-15; Kim, Jo, et al., World J Surg Oncol, 2012, Sep 17;10:193; Zhao, Zhang, et al.; Front Immunol, 2014, Jul 23;5:352; Chen, Zhao, et al., Int J Clin Exp Pathol, 2015, 8(6):7034-9).
WO2016/174183 describes combinations of IRAK4 inhibiting substituted indazoles with specific BTK inhibitors such as ibrutinib showing synergistic anti-proliferative effects in tumour cell lines like THP-1, TMD-8, HBL-1. A distinct rise in the antitumour effect of the combinations *in vitro* and in animal models has been observed compared to respective monotherapies.

Inflammatory disorders such as CAPS (cryopyrin-associated periodic syndromes) including FCAS (familial cold autoinflammatory syndrome), MWS (Muckle-Wells syndrome), NOMID (neonatal-onset multisystem inflammatory disease) and CONCA (chronic infantile, neurological, cutaneous, and articular) syndrome; FMF (familial mediterranean fever), HIDS (hyper-IgD syndrome), TRAPS (tumour necrosis factor receptor 1-associated periodic syndrome), juvenile idiopathic arthritis, adult-onset Still's disease, Adamantiades-Behçet's disease, rheumatoid arthritis, osteoarthritis, keratoconjunctivitis sicca, PAPA syndrome (pyogenic arthritis, Pyoderma gangraenosum and acne), Schnitzler's syndrome and Sjögren syndrome are treated by blocking the IL-1 signal pathway; therefore here, too, an IRAK4 inhibitor is suitable for treatment of the diseases mentioned (Narayanan, Corrales, et al., Cornea, 2008, 27(7): 811-817; Brenner, Ruzicka, et al., British Journal of Dermatology, 2009, Nov;161(5):1199-201; Henderson and Goldbach-Mansky, Clinical Immunology, 2010, 135(2): 210-222; Dinarello, European Journal of Immunology, 2011, 41(5): 1203-1217; Gul, Tugal-Tutkun, et al., Ann Rheum Dis, 2012, 71(4): 563-566; Pettersson, Annals of MedicinePetterson, 2012, 44(2): 109-118; Ruperto, Brunner, et al., New England Journal of Medicine, 2012, 367(25): 2396-2406; Nordström, Knight, et al., The Journal of Rheumatology, 2012, 39(10): 2008-2011; Vijmasi, Chen, et al., Mol Vis, 2013, 19: 1957-1965; Yamada, Arakaki, et al., Opinion on Therapeutic Targets, 2013, 17(4): 393-401; de Koning, Clin Transl Allergy, 2014, Dec 5;4:41). The ligand of IL-33R, IL-33, is involved particularly in the pathogenesis of acute kidney failure, and so the inhibition of IRAK4 for prophylaxis and/or treatment is a suitable therapeutic approach (Akcay, Nguyen, et al., Journal of the American Society of Nephrology, 2011, 22(11):2057-67). Components of the IL-1 receptor family are associated with myocardial infarction, different pulmonary disorders such as asthma, COPD, idiopathic interstitial pneumonia, allergic rhinitis, pulmonary fibrosis and acute respiratory distress syndrome (ARDS), and so prophylactic and/or therapeutic action is to be expected in the indications mentioned through the inhibition of IRAK4 (Kang, Homer, et al., The Journal of Immunology, 2007, 178(3): 1948-1959; Imaoka, Hoshino, et al., European Respiratory Journal, 2008, 31(2): 287-297; Couillin, Vasseur, et al., The Journal of Immunology, 2009, 183(12): 8195-8202; Abbate, Kontos, et al., The American Journal of Cardiology, 2010, 105(10): 1371-1377.e1371; Lloyd, Current Opinion in Immunology, 2010, 22(6): 800-806; Pauwels, Bracke, et al., European Respiratory Journal, 2011, 38(5): 1019-1028; Haenuki, Matsushita, et al., Journal of Allergy and Clinical Immunology, 2012, 130(1):184-94.e11; Yin, Li, et al., Clinical & Experimental Immunology, 2012, 170(1): 1-9; Abbate, Van Tassell, et al., The American Journal of Cardiology, 2013, 111(10): 1394-1400; Byers, Alexander-Brett, et al., The Journal of Clinical Investigation, 2013, 123(9): 3967-3982; Bunting, Shadie, et al., BioMed Research International, 2013, 2013:250938; Kawayama, Okamoto, et al., J Interferon Cytokine Res, 2013, 32(10): 443-449; Martinez-Gonzalez, Roca, et al., American Journal of Respiratory Cell and Molecular Biology, 2013, 49(4): 552-562; Nakanishi, Yamaguchi, et al., PLoS ONE, 2013, 8(10):e78099; Qiu, Li, et al., Immunology, 2013, 138(1): 76-8; Li, Guabiraba, et al., Journal of Allergy and Clinical Immunology, 2014, 134(6):1422-1432.e11; Saluja, Ketelaar, et al., Molecular Immunology, 2014, 63(1):80-5; Lugrin, Parapanov, et al., The Journal of Immunology, 2015, 194(2):499-503).

WO2017207481 discloses IRAK4 inhibiting substituted indazoles which are especially suitable for treatment and for prevention of inflammatory disorders in animals characterized by an overreacting immune system, in particular for Canine Atopic Dermatitis, Flea Allergy Dermatitis in dogs and cats, inflammatory bowel disease in dogs and cats, osteoarthritis and inflammatory pain in dogs, cats, horses and cattle, non-infectious recurrent airway disease in horses (also known as chronic obstructive pulmonary disease, heaves), insect hypersensitivity in horses (also known as sweet itch, summer eczema), feline asthma, bovine respiratory disease, mastitis and endometritis in cattle, and swine respiratory disease.

The prior art discloses a multitude of IRAK4 inhibitors (see, for example, W. M. Seganish, Expert Opinion on Therapeutic Patents, 2016, Vol. 26, No. 8, 917-932).

The state of the art discloses a multitude of substituted pyrazolopyrimidines as well.

Compound L-1 (racemic mixture) has been described to interact with adenosine A receptors (Bioorg. Med. Chem. Lett. 11 (2001) 191 - 193). The compound displays an unsubstituted phenyl ring but does not show a meta-substituted phenyl ring and shows a 2-aminopropane amide moiety at the 6-position but not a cyclic substituent. In addition, the compound displays an amino (-NH2) group at the position 4 but no cyclic substituent.

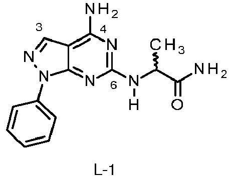

Selective p38alpha inhibitors such as compound L-2, L-3 and L-4 have been described (Bioorg. Med. Chem. Lett. 18 (2008) 2652-2657). All compounds reported display an aniline based substituent linked via the nitrogen atom to the position 4 of the pyrazolopyrimidine core. There is no report about compounds showing a heterocycloalkyl moiety directly linked to the position 4. Moreover, a compound with a meta-substituted phenyl moiety linked to the 1-position has not been described in the paper.

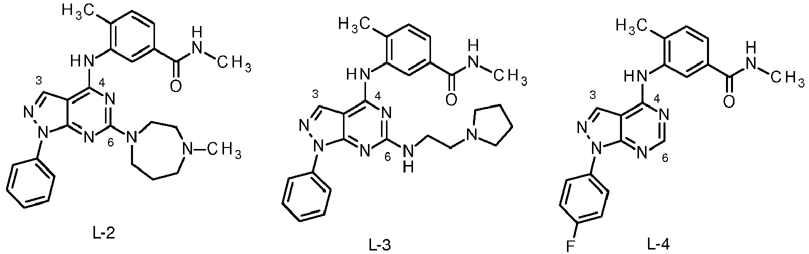

Compound L-5 and others of the general formula L-6 have been described in DE2430454A1 as compounds having antithrombotic properties. Substituent R¹ of formula L-6 has the meaning hydrogen, alkyl, or phenyl optionally substituted by halogen atom or methoxy, but compounds displaying a meta-substituted phenyl moiety have not been reported. 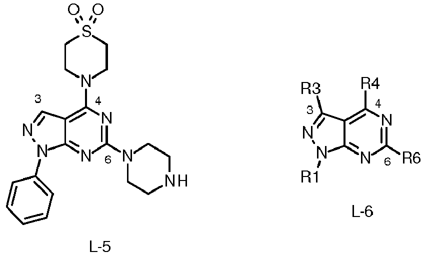 Moreover, compounds of formula L-6 in which R1 has the meaning phenyl substituted by optionally substituted alkyl, C₁-C₄-alkoxy substituted with 1 to 6 fluorine atoms, or C₁-C₆-alkylsulfonyl
have not been described in DE2430454A1.
Heterocycloalkyl is listed as part of the definition of R⁶ in formula L-6 but said heterocycloalkyl ring is directly linked to the rest of the molecule (compare piperazine moiety of compound L-5).

WO2018098561 describes bicyclic inhibitors inhibiting the activity of BRD4 and/or JAK2 tyrosine kinase. The general structure L-7 of the compounds is characterized by a phenyl substituent linked to position Z² of the bicyclic core and said phenyl moiety displays a functional group based on a nitrogen atom in meta-position.

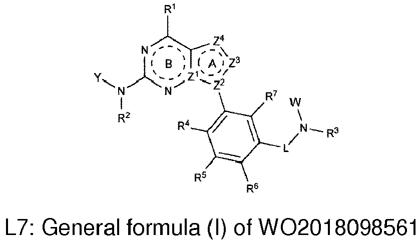

In WO2012/009258, table 6, compounds such as compound L-8 (333-2316) have been reported to be modulators of galanin receptors. Neither compounds with a meta-substituted phenyl moiety attached to the nitrogen N-1 nor compounds with a heterocycloalkyl moiety linked to the 6-position of the pyrazolopyrimidine core are disclosed.

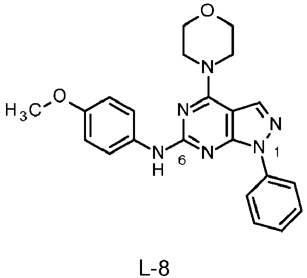

Pyrazolopyrimidine based compounds with antiviral properties can be found in WO2010118367 (see library G, page 115). However, the compounds displaying an aniline moiety at position 4 are directly connected at position 6 to the heterocycloalkyl moiety (compare e.g. the pyrrolidine moiety of compound series L-9 (library G3 in WO2010118367, page 122).

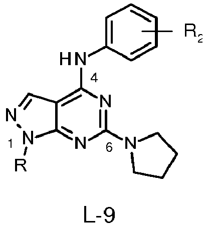

Pyrazolopyrimidines of the general formula L-10 acting as mTOR kinase and PI3 kinase inhibitors have been described in WO2009052145. The substituent "het" in the formula L-10 has the meaning 5-10 membered bridged bicyclic group containing at least on O atom, at least one N atom, and optionally additional heteroatoms. The compounds disclosed in WO2009052145 are not described to be active as IRAK4 inhibitors.
Only one compound based on a 4,6-diamino pyrazolopyrimidine structure is described in WO 2009052145. However, this compound L-11 displays a piperidine moiety and not a phenyl moiety at the N-1 of the pyrazolopyrimidine core.

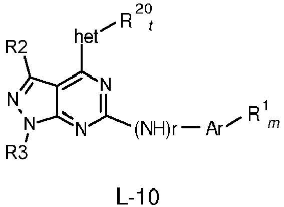

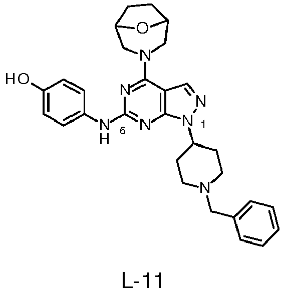

A variety of bicyclic alkylene diamine-substituted heterocycles including pyrazolopyrimidines has been described to act as NPY₁ receptor modifiers in WO200123389. 6-methyl substituted pyrazolopyrimidines are disclosed. There is no report about compounds based on a 4,6-diaminopyrazolopyrimidine core. The compounds disclosed in WO200123389 are not described to be active as IRAK4 inhibitors.

The commercially available compound CAS No. 1313851-93-2 displays a para-methylphenyl substituent but not a *meta*-substituted phenyl substituent. There is no information available about biological activity and synthesis of the compound.

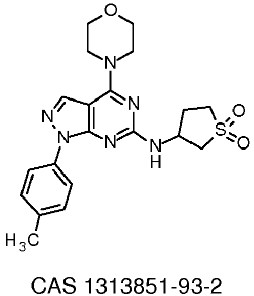

Bicyclic IRAK4 inhibitors of general formula L-12 have been reported in WO2017205762. Cy¹ has the meaning heteroaryl but not heterocycloalkyl, Cy² has the meaning of C₃-C₁₂-cycloalkyl or 5- to 7-membered heteroaryl, and R² is selected from H, or substituted or unsubstituted C₁-C₃-alkyl. Heterocycloalkyl substituents directly attached to the 4-position via a nitrogen atom have not been described.

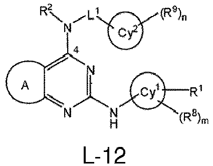

Pyrimidine based bicyclic cores such as pyrrolo pyrimidines, but no pyrazolo pyridines have been described in J. Med. Chem. 2017, 60, 10071-10091.

To summarize, pyrazolo pyrimidines of formula (I) are not described in the prior art.

It has now been found, and this constitutes the basis of the present invention, that the compounds of formula (I) of the present invention have surprising and advantageous properties.

In particular, the compounds of the present invention have surprisingly been found to effectively inhibit IRAK4 for which data are given in biological experimental section and may therefore be used for the treatment or prophylaxis of proliferative disorders, autoimmune disorders, metabolic and inflammatory disorders, in particular of inflammatory rheumatological disorders, inflammatory skin disorders, cardiovascular disorders, lung disorders, eye disorders, neurological disorders, pain disorders and cancer in human and in animals.

### Description of the invention

In accordance with a first aspect, the present invention covers compounds of general formula (I): 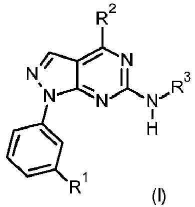 wherein
- R¹: represents C₁-C₄-alkyl, C₁-C₄-alkoxy, C₁-C₆-alkylsulfonyl, or cyano,
• wherein said C₁-C₄-alkyl is optionally substituted with one or more substituents which are the same or different and independently selected from the group consisting of fluorine atom and hydroxy, and
• wherein said C₁-C₄-alkoxy is substituted with 1 to 6 fluorine atoms;
- R²: represents 4- to 7-membered monocyclic saturated heterocycloalkyl containing one ring nitrogen atom and optionally one additional heteroatom or heteroatom-containing group independently selected from the group consisting of N, O, S, S(=O), S(=O)₂ and S(=O)(=NH),
- wherein said 4- to 7-membered monocyclic saturated heterocycloalkyl is bound to the rest of the molecule via any nitrogen atom, and
- wherein said 4- to 7-membered monocyclic saturated heterocycloalkyl is optionally substituted with one or more substituents which are the same or different and independently selected from the group consisting of an oxo substituent, halogen, hydroxy, cyano, C₁-C₆-alkoxy, CO₂H, NH₂, NR^{a}R^{b}, -S(=O)₂R^{b}, -S(=O)₂R⁴ -(C=O)R^{b}, -(C=O)NH₂, -(C=O)NR^{a}R^{b}, -(C=O)R⁴, R⁵, R⁶, R⁷, R⁸, and R⁹;
- R³: represents 4- to 7-membered monocyclic saturated heterocycloalkyl containing one to two heteroatom or heteroatom-containing groups selected from N, O, S, S(=O), S(=O)₂ and S(=O)(=NH), C₃-C₆-cycloalkyl, 6- to 11-membered heterospirocycloalkyl containing one heteroatom or heteroatom-containing group independently selected from N, O and S(=O)₂, or 6- to 12-membered bridged heterocycloalkyl group containing one heteroatom or heteroatom-containing group independently selected from N, O, and S(=O)₂,
- wherein said 4- to 7-membered monocyclic saturated heterocycloalkyl, said 6- to 11-membered heterospirocycloalkyl and said 6- to 12-membered bridged heterocycloalkyl group are bound to the rest of the molecule via any of the carbon atoms, and
- wherein said 4- to 7-membered monocyclic saturated heterocycloalkyl is optionally substituted with one or more substituents which are the same or different and independently selected from the group consisting of oxo substituent, halogen, hydroxy, cyano, CO₂H, C₁-C₆-alkoxy, NH₂, NR^{a}R^{b}, -S(=O)₂R^{b}, -S(=O)₂R^{c}, -S(=O)₂NR^{a}R^{b}, cyclopropyl, cyclobutyl, -CH₂-CH₂-forming a cyclopropyl group together with any carbon atom of the 4- to 7-membered saturated heterocycloalkyl, and C₁-C₆-alkyl optionally substituted with one to five halogens atoms or with one hydroxy,
- wherein said C₃-C₆-cycloalkyl is optionally substituted with one or more substituents which are the same or different and independently selected from the group consisting of oxo substituent, halogen, hydroxy, cyano, CO₂H, C₁-C₆-alkoxy, NH₂, NR^{a}R^{b}, -S(=O)₂R^{b}, -S(=O)₂NH₂, -S(=O)₂NR^{a}R^{b}, C(=O)R^{b}, -C(=O)NH₂, -C(=O)NR^{a}R^{b}, and C₁-C₆-alkyl, wherein said C₁-C₆-alkyl is optionally substituted with one to five halogen atoms or with one hydroxy,
- wherein said 6- to 11-membered heterospirocycloalkyl is optionally substituted with one or more substituents which are the same or different and independently selected from the group consisting of C₁-C₄-alkyl, fluorine atom, hydroxy, cyano, CO₂H, NH₂, and -S(=O)₂R^{b}, and
- wherein said 6- to 12-membered bridged heterocycloalkyl group is optionally substituted with one or more substituent which are the same or different and independently selected from the group consisting of C₁-C₆-alkyl, fluorine atom, and hydroxy;
- R⁴: represents 4- to 7-membered monocyclic saturated heterocycloalkyl group containing one nitrogen atom and optionally one additional heteroatom or heteroatom-containing group independently selected from N, O, and S(=O)₂, or 6- to 12-membered bridged heterocycloalkyl containing one nitrogen atom and optionally one additional heteroatom or heteroatom-containing group independently selected from the group consisting of N, O, and S(=O)₂,
- wherein said 4- to 7-membered monocyclic saturated heterocycloalkyl and said 6- to 12-membered bridged heterocycloalkyl are bound to the rest of the molecule via any nitrogen atom,
- wherein said 4- to 7-membered monocyclic saturated heterocycloalkyl and said 6- to 12-membered bridged heterocycloalkyl are optionally substituted with one or more substituents which are the same or different and independently selected from the group consisting of C₁-C₆-alkyl, fluorine atom, and hydroxy;
- R⁵: represents C₁-C₆-alkyl group optionally substituted with one or more substituents which are the same or different and independently selected from the group consisting of halogen, hydroxy, CO₂H, cyclopropyl, cyano, C₁-C₆-alkoxy, NH₂, NR^{a}R^{b}, -S(=O)₂R^{b}, -S(=O)₂R⁴ -(C=O)R^{b}, -(C=O)NH₂, -(C=O)R^{a}R^{b}, -(C=O)R⁴, 4- to 7-membered monocyclic saturated heterocycloalkyl containing one or two identical or different heteroatoms or heteroatom-containing groups independently selected from the group consisting of N, O, S, S(=O), and S(=O)₂, 6- to 12-membered bridged heterocycloalkyl group containing one nitrogen atom and optionally one additional heteroatom or heteroatom-containing group independently selected from the group consisting of N, O, and S(=O)₂, and 6- to 11-membered heterospirocycloalkyl containing one nitrogen atom and optionally one additional heteroatom or heteroatom-containing group independently selected from the group consisting of N, O, and S(=O)₂,
- wherein said 6- to 12-membered bridged heterocycloalkyl group and said 6- to 11-membered heterospirocycloalkyl are connected to the rest of the molecule via any nitrogen atom, and
- wherein said 4- to 7-membered monocyclic saturated heterocycloalkyl and said 6- to 12-membered bridged heterocycloalkyl group are optionally substituted with one or more substituents which are the same or different and independently selected from the group consisting of C₁-C₆-alkyl, fluorine atom, oxo substituent, and hydroxy, and
- wherein said 6- to 11-membered heterospirocycloalkyl is optionally substituted with one or more substituents which are the same or different and independently selected from the group consisting of C₁-C₄-alkyl, fluorine atom, hydroxy, cyano, CO₂H, NH₂, and -S(=O)₂R^{b};
- R⁶: represents -C₃-C₆-cycloalkyl optionally substituted with one or more substituents which are the same or different and independently selected from the group consisting of halogen, C₁-C₆-alkyl, hydroxy, NH₂, and NR^{a}R^{b};
- R⁷: represents 4- to 7-membered monocyclic saturated heterocycloalkyl containing one or two identical or different heteroatoms or heteroatom-containing groups independently selected from the group consisting of N, O, S, S(=O), S(=O)₂ and S(=O)(=NH),
- wherein said 4- to 7-membered monocyclic saturated heterocycloalkyl is optionally substituted with one or more substituents which are the same or different and independently selected from the group consisting of an oxo substituent, halogen, hydroxy, NH₂, NR^{a}R^{b}, cyano, C₁-C₆-alkoxy, CO₂H,-S(=O)₂R^{b}, and C₁-C₆-alkyl,
   - wherein said C₁-C₆-alkyl is optionally substituted with one or more substituents which are the same or different and independently selected from the group consisting of fluorine atom, hydroxy, NH₂, and NR^{a}R^{b};
- R⁸: represents 6- to 12-membered bridged heterocycloalkyl containing one nitrogen atom and optionally one additional heteroatom or heteroatom-containing group independently selected from the group consisting of N, O, and S(=O)₂,
- wherein said 6- to 12-membered bridged heterocycloalkyl is connected to the rest of the molecule via any nitrogen atom, and
- wherein said 6- to 12-membered bridged heterocycloalkyl is optionally substituted with one or more substituents which are the same or different and independently selected from the group consisting of C₁-C₆-alkyl, fluorine atom, and hydroxy;
- R⁹: represents 6- to 11-membered heterospirocycloalkyl containing one nitrogen atom and optionally one additional heteroatom or heteroatom-containing group independently selected from the group consisting of N, O and S(=O)₂,
- wherein said 6- to 11-membered heterospirocycloalkyl is connected to the rest of the molecule via any nitrogen atom, and
- wherein said 6- to 11-membered heterospirocycloalkyl is optionally substituted with one or more substituents which are the same or different and independently selected from the group consisting of C₁-C₄-alkyl, fluorine atom, hydroxy, cyano, CO₂H, NH₂, and -S(=O)₂C₁-C₆-alkyl;
- R^{a}: represents hydrogen or C₁-C₆-alkyl,
- R^{b}: represents C₁-C₆-alkyl, and
- R^{c}: represents C₃-C₆-cycloalkyl;
and stereoisomers, tautomers, N-oxides, hydrates, solvates, and salts thereof, and mixtures of same.

### DEFINITIONS

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.
The term "optionally substituted" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon or nitrogen atom. Commonly, it is possible for the number of optional substituents, when present, to be 1, 2, 3, 4, 5 or 6, in particular 1, 2 or 3.
As used herein, the term "one or more", e.g. in the definition of the substituents of the compounds of general formula (I) of the present invention, means 1, 2, 3, 4, 5 or 6, particularly 1, 2, 3 or 4, more particularly 1, 2 or 3, even more particularly 1 or 2.
As used herein, an oxo substituent represents an oxygen atom, which is bound to a carbon atom via a double bond.
The term "ring substituent" means a substituent attached to an aromatic or nonaromatic ring, which replaces an available hydrogen atom on the ring.
If within the present text any item is referred to as "as mentioned herein", it means that it may be mentioned anywhere in the present text.
The term "comprising" when used in the specification includes "consisting of".
The terms as mentioned in the present text have the following meanings:
The term "halogen atom" or "halogen" means a fluorine, chlorine, bromine or iodine atom, particularly a fluorine, or chlorine atom.
The term "C₁-C₆-alkyl" means a linear or branched, saturated, monovalent hydrocarbon group having 1, 2, 3, 4, 5 or 6 or 1, 2, 3 or 4 carbon atoms, *e.g*. a methyl, ethyl, propyl, isopropyl, butyl, *sec*-butyl, isobutyl, *tert*-butyl, pentyl, isopentyl, 2-methylbutyl, 1-methylbutyl, 1-ethylpropyl, 1,2-dimethylpropyl, *neo*-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer thereof.
The same applies to the term "C₁-C₄-alkyl".
The term "C₁-C₆-alkoxy" means a linear or branched, saturated, monovalent group of formula (C₁-C₆-alkyl)-O-, in which the term "C₁-C₆-alkyl" is as defined *supra, e.g.* a methoxy, ethoxy, *n*-propoxy, isopropoxy, *n*-butoxy, sec-butoxy, isobutoxy, *tert*-butoxy, pentyloxy, isopentyloxy or n-hexyloxy group, or an isomer thereof.
The same applies to the term "C₁-C₄-alkoxy".
The term "C₃-C₆-cycloalkyl" means a saturated, monovalent, monocyclic hydrocarbon ring which contains 3, 4, 5 or 6 carbon atoms. Said C₃-C₆-cycloalkyl group is is for example, a monocyclic hydrocarbon ring, *e.g*. a a cyclopropyl, cyclobutyl, cyclopentyl or cyclohexyl group.
The terms "4- to 7-membered monocyclic saturated heterocycloalkyl" and "4- to 6-membered heterocycloalkyl" mean a monocyclic, saturated heterocycle with 4, 5, 6 or 7 or, respectively, 4, 5 or 6 ring atoms in total, which contains one or two identical or different ring heteroatoms from the series N, O, S, S(=O), S(=O)₂ and S(=O)(=NH), it being possible for said heterocycloalkyl group to be attached to the rest of the molecule via any one of the carbon atoms or, if present, a nitrogen atom.
Said heterocycloalkyl group, without being limited thereto, can be a 4-membered ring, such as azetidinyl, oxetanyl or thietanyl, for example; or a 5-membered ring, such as tetrahydrofuranyl, 1,3-dioxolanyl, thiolanyl, pyrrolidinyl, imidazolidinyl, pyrazolidinyl, 1,1-dioxidothiolanyl, 1,2-oxazolidinyl, 1,3-oxazolidinyl or 1,3-thiazolidinyl, for example; or a 6-membered ring, such as tetrahydropyranyl, tetrahydrothiopyranyl, piperidinyl, morpholinyl, dithianyl, thiomorpholinyl, piperazinyl, 1,3-dioxanyl, 1,4-dioxanyl or 1,2-oxazinanyl, for example, or a 7-membered ring, such as azepanyl, 1,4-diazepanyl or 1,4-oxazepanyl, for example.
The term "heterospirocycloalkyl" means a bicyclic, saturated heterocycle with 6, 7, 8, 9, 10 or 11 ring atoms in total, in which the two rings share one common ring carbon atom, which "heterospirocycloalkyl" contains one or two identical or different ring heteroatoms or heteroatom-containing group selectedfrom the series N, O, S, S(=O)₂; it being possible for said heterospirocycloalkyl group to be attached to the rest of the molecule via any one of the carbon atoms, except the spiro carbon atom, or, if present, a nitrogen atom.
Said heterospirocycloalkyl group is, for example, azaspiro[2.3]hexyl, azaspiro[3.3]heptyl, azaspiro[4.3]octyl, oxaazaspiro[3.3]heptyl, thiaazaspiro[3.3]heptyl, oxaspiro[3.3]heptyl, oxazaspiro[5.3]nonyl, oxazaspiro[4.3]octyl, azaspiro[4,5]decyl, oxazaspiro [5.5]undecyl, diazaspiro[3.3]heptyl, thiazaspiro[3.3]heptyl, thiazaspiro[4.3]octyl, azaspiro[5.5]undecyl, or one of the further homologous scaffolds such as spiro[3.4]-, spiro[4.4]-, spiro[2.4]-, spiro[2.5]-, spiro[2.6]-, spiro[3.5]-, spiro[3.6]-, spiro[4.5]- and spiro[4.6]-. Said heterospirocycloalkyl group containing a SO₂ group is, for example 7-thia-2-azaspiro[3.5]nonane 7,7-dioxide.
The term "bridged heterocycloalkyl" means a bicyclic, saturated heterocycle with 6, 7, 8, 9, 10, 11 or 12 ring atoms in total, in which the two rings share two common ring atoms which are not adjacent, and in which the "bridged heterocycloalkyl" contains one or two identical or different ring heteroatoms or heteroatom-containing group selected from the series N, O, S, SO₂; it being possible for said bridged heterocycloalkyl group to be attached to the rest of the molecule via any one of the carbon atoms or, if present, a nitrogen atom.
Said bridged heterocycloalkyl group is, for example, azabicyclo[2.2.1]heptyl, oxazabicyclo[2.2.1]heptyl, thiazabicyclo[2.2.1]heptyl, diazabicyclo[2.2.1]heptyl, azabicyclo-[2.2.2]octyl, diazabicyclo[2.2.2]octyl, oxazabicyclo[2.2.2]octyl, thiazabicyclo[2.2.2]octyl, azabicyclo[3.2.1]octyl, diazabicyclo[3.2.1]octyl, oxazabicyclo[3.2.1]octyl, thiazabicyclo[3.2.1]octyl, azabicyclo[3.3.1]nonyl, diazabicyclo[3.3.1]nonyl, oxazabicyclo[3.3.1]nonyl, thiazabicyclo-[3.3.1]nonyl, azabicyclo[4.2.1]nonyl, diazabicyclo[4.2.1]nonyl, oxazabicyclo[4.2.1]nonyl, thiazabicyclo[4.2.1]nonyl, azabicyclo[3.3.2]decyl, diazabicyclo[3.3.2]decyl, oxazabicyclo-[3.3.2]decyl, thiazabicyclo[3.3.2]decyl or azabicyclo[4.2.2]decyl. Said bridged heterocycloalkyl group containing a SO₂ group is, for example 8-thia-3-azabicyclo[3.2.1]octane 8,8-dioxide, 2-thia-5-azabicyclo[2.2.2]octane 2,2-dioxide, 3-thia-8-azabicyclo[3.2.1]octane 3,3-dioxide.
The term "C₁-C₆", as used in the present text, *e.g*. in the context of the definition of "C₁-C₆-alkyl" or "C₁-C₆-alkoxy" means an alkyl group having a finite number of carbon atoms of 1 to 6, *i.e.* 1, 2, 3, 4, 5 or 6 carbon atoms.
Further, as used herein, the term "C₃-C₆", as used in the present text, *e.g*. in the context of the definition of "C₃-C₆-cycloalkyl", means a cycloalkyl group having a finite number of carbon atoms of 3 to 6, *i.e.* 3, 4, 5 or 6 carbon atoms.
When a range of values is given, said range encompasses each value and sub-range within said range.
For example:
"C₁-C₆" encompasses C₁, C₂, C₃, C₄, C₅, C₆, C₁-C₆, C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅, and C₅-C₆;
"C₁-C₃" encompasses C₁, C₂, C₃, C₁-C₃, C₁-C₂, and C₂-C₃,;
"C₃-C₆" encompasses C₃, C₄, C₅, C₆, C₃-C₆, C₃-C₅, C₃-C₄, C₄-C₆, C₄-C₅, and C₅-C₆.
As used herein, the term "leaving group" means an atom or a group of atoms that is displaced in a chemical reaction as stable species taking with it the bonding electrons. In particular, such a leaving group is selected from the group comprising: halide, in particular fluoride, chloride, bromide or iodide, (methylsulfonyl)oxy, [(trifluoromethyl)sulfonyl]oxy, [(nonafluorobutyl)sulfonyl]oxy, (phenylsulfonyl)oxy, [(4-methylphenyl)sulfonyl]oxy, [(4-bromophenyl)-sulfonyl]oxy, [(4-nitrophenyl)sulfonyl]oxy, [(2-nitrophenyl)sulfonyl]oxy, [(4-isopropylphenyl)-sulfonyl]oxy, [(2,4,6-triisopropylphenyl)sulfonyl]oxy, [(2,4,6-trimethylphenyl)sulfonyl]oxy, [(4-*tert*-butylphenyl)sulfonyl]oxy and [(4-methoxyphenyl)sulfonyl]oxy.

It is possible for the compounds of general formula (I) to exist as isotopic variants. The invention therefore includes one or more isotopic variant(s) of the compounds of general formula (I), particularly deuterium-containing compounds of general formula (I).
The term "Isotopic variant" of a compound or a reagent is defined as a compound exhibiting an unnatural proportion of one or more of the isotopes that constitute such a compound.
The term "Isotopic variant of the compound of general formula (I)" is defined as a compound of general formula (I) exhibiting an unnatural proportion of one or more of the isotopes that constitute such a compound.
The expression "unnatural proportion" means a proportion of such isotope which is higher than its natural abundance. The natural abundances of isotopes to be applied in this context are described in "Isotopic Compositions of the Elements 1997", Pure Appl. Chem., 70(1), 217-235, 1998.
Examples of such isotopes include stable and radioactive isotopes of hydrogen, carbon, nitrogen, oxygen, phosphorus, sulfur, fluorine, chlorine, bromine and iodine, such as 2H (deuterium), 3H (tritium), 11C, 13C, 14C, 15N, 17O, 18O, 32P, 33P, 33S, 34S, 35S, 36S, 18F, 36Cl, 82Br, 123I, 124I, 125I, 129I and 131I, respectively.
With respect to the treatment and/or prophylaxis of the disorders specified herein the isotopic variant(s) of the compounds of general formula (I) preferably contain deuterium ("deuterium-containing compounds of general formula (I)"). Isotopic variants of the compounds of general formula (I) in which one or more radioactive isotopes, such as 3H or 14C, are incorporated are useful e.g. in drug and/or substrate tissue distribution studies. These isotopes are particularly preferred for the ease of their incorporation and detectability. Positron emitting isotopes such as 18F or 11C may be incorporated into a compound of general formula (I). These isotopic variants of the compounds of general formula (I) are useful for in vivo imaging applications. Deuterium-containing and 13C-containing compounds of general formula (I) can be used in mass spectrometry analyses in the context of preclinical or clinical studies.
Isotopic variants of the compounds of general formula (I) can generally be prepared by methods known to a person skilled in the art, such as those described in the schemes and/or examples herein, by substituting a reagent for an isotopic variant of said reagent, preferably for a deuterium-containing reagent. Depending on the desired sites of deuteration, in some cases deuterium from D2O can be incorporated either directly into the compounds or into reagents that are useful for synthesizing such compounds. Deuterium gas is also a useful reagent for incorporating deuterium into molecules. Catalytic deuteration of olefinic bonds and acetylenic bonds is a rapid route for incorporation of deuterium. Metal catalysts (i.e. Pd, Pt, and Rh) in the presence of deuterium gas can be used to directly exchange deuterium for hydrogen in functional groups containing hydrocarbons. A variety of deuterated reagents and synthetic building blocks are commercially available from companies such as for example C/D/N Isotopes, Quebec, Canada; Cambridge Isotope Laboratories Inc., Andover, MA, USA; and CombiPhos Catalysts, Inc., Princeton, NJ, USA.
The term "deuterium-containing compound of general formula (I)" is defined as a compound of general formula (I), in which one or more hydrogen atom(s) is/are replaced by one or more deuterium atom(s) and in which the abundance of deuterium at each deuterated position of the compound of general formula (I) is higher than the natural abundance of deuterium, which is about 0.015%. Particularly, in a deuterium-containing compound of general formula (I) the abundance of deuterium at each deuterated position of the compound of general formula (I) is higher than 10%, 20%, 30%, 40%, 50%, 60%, 70% or 80%, preferably higher than 90%, 95%, 96% or 97%, even more preferably higher than 98% or 99% at said position(s). It is understood that the abundance of deuterium at each deuterated position is independent of the abundance of deuterium at other deuterated position(s).
The selective incorporation of one or more deuterium atom(s) into a compound of general formula (I) may alter the physicochemical properties (such as for example acidity [C. L. Perrin, et al., J. Am. Chem. Soc., 2007, 129, 4490], basicity [C. L. Perrin et al., J. Am. Chem. Soc., 2005, 127, 9641], lipophilicity [B. Testa et al., Int. J. Pharm., 1984, 19(3), 271]) and/or the metabolic profile of the molecule and may result in changes in the ratio of parent compound to metabolites or in the amounts of metabolites formed. Such changes may result in certain therapeutic advantages and hence may be preferred in some circumstances. Reduced rates of metabolism and metabolic switching, where the ratio of metabolites is changed, have been reported (A. E. Mutlib et al., Toxicol. Appl. Pharmacol., 2000, 169, 102). These changes in the exposure to parent drug and metabolites can have important consequences with respect to the pharmacodynamics, tolerability and efficacy of a deuterium-containing compound of general formula (I). In some cases, deuterium substitution reduces or eliminates the formation of an undesired or toxic metabolite and enhances the formation of a desired metabolite (e.g. Nevirapine: A. M. Sharma et al., Chem. Res. Toxicol., 2013, 26, 410; Efavirenz: A. E. Mutlib et al., Toxicol. Appl. Pharmacol., 2000, 169, 102). In other cases, the major effect of deuteration is to reduce the rate of systemic clearance. As a result, the biological half-life of the compound is increased. The potential clinical benefits would include the ability to maintain similar systemic exposure with decreased peak levels and increased trough levels. This could result in lower side effects and enhanced efficacy, depending on the particular compound's pharmacokinetic/ pharmacodynamic relationship. ML-337 (C. J. Wenthur et al., J. Med. Chem., 2013, 56, 5208) and Odanacatib (K. Kassahun et al., WO2012/112363) are examples for this deuterium effect. Still other cases have been reported in which reduced rates of metabolism result in an increase in exposure of the drug without changing the rate of systemic clearance (e.g. Rofecoxib: F. Schneider et al., Arzneim. Forsch./ Drug. Res., 2006, 56, 295; Telaprevir: F. Maltais et al., J. Med. Chem., 2009, 52, 7993). Deuterated drugs showing this effect may have reduced dosing requirements (e.g. lower number of doses or lower dosage to achieve the desired effect) and/or may produce lower metabolite loads.
A compound of general formula (I) may have multiple potential sites of attack for metabolism. To optimize the above-described effects on physicochemical properties and metabolic profile, deuterium-containing compounds of general formula (I) having a certain pattern of one or more deuterium-hydrogen exchange(s) can be selected. Particularly, the deuterium atom(s) of deuterium-containing compound(s) of general formula (I) is/are attached to a carbon atom and/or is/are located at those positions of the compound of general formula (I), which are sites of attack for metabolizing enzymes such as e.g. cytochrome P450.

Where the plural form of the word compounds, salts, polymorphs, hydrates, solvates and the like, is used herein, this is taken to mean also a single compound, salt, polymorph, isomer, hydrate, solvate or the like.
By "stable compound' or "stable structure" is meant a compound that is sufficiently robust to survive isolation to a useful degree of purity from a reaction mixture, and formulation into an efficacious therapeutic agent.
The compounds of the present invention optionally contain one or more asymmetric centres, depending upon the location and nature of the various substituents desired. It is possible that one or more asymmetric carbon atoms are present in the (R) or (S) configuration, which can result in racemic mixtures in the case of a single asymmetric centre, and in diastereomeric mixtures in the case of multiple asymmetric centres. In certain instances, it is possible that asymmetry also be present due to restricted rotation about a given bond, for example, the central bond adjoining two substituted aromatic rings of the specified compounds.
Preferred compounds are those, which produce the more desirable biological activity. Separated, pure or partially purified isomers and stereoisomers or racemic or diastereomeric mixtures of the compounds of the present invention are also included within the scope of the present invention. The purification and the separation of such materials can be accomplished by standard techniques known in the art.
The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e.g*., HPLC columns using a chiral phase), with or without conventional derivatisation, optimally chosen to maximise the separation of the enantiomers. Suitable HPLC columns using a chiral phase are commercially available, such as those manufactured by Daicel, e.g., Chiracel OD and Chiracel OJ, for example, among many others, which are all routinely selectable. Enzymatic separations, with or without derivatisation, are also useful. The optically active compounds of the present invention can likewise be obtained by chiral syntheses utilizing optically active starting materials.
In order to distinguish different types of isomers from each other reference is made to IUPAC Rules Section E (Pure Appl Chem 45, 11-30, 1976).
The present invention includes all possible stereoisomers of the compounds of the present invention as single stereoisomers, or as any mixture of said stereoisomers, e.g. (R)- or (S)-isomers, in any ratio. Isolation of a single stereoisomer, *e.g*. a single enantiomer or a single diastereomer, of a compound of the present invention is achieved by any suitable state of the art method, such as chromatography, especially chiral chromatography, for example.

Further, the compounds of the present invention can exist as N-oxides, which are defined in that at least one nitrogen of the compounds of the present invention is oxidised. The present invention includes all such possible N-oxides.

The present invention also covers useful forms of the compounds of the present invention, such as metabolites, hydrates, solvates, prodrugs, salts, in particular pharmaceutically acceptable salts, and/or co-precipitates.
The compounds of the present invention can exist as a hydrate, or as a solvate, wherein the compounds of the present invention contain polar solvents, in particular water, methanol or ethanol for example, as structural element of the crystal lattice of the compounds. It is possible for the amount of polar solvents, in particular water, to exist in a stoichiometric or non-stoichiometric ratio. In the case of stoichiometric solvates, *e.g*. a hydrate, hemi-, (semi-), mono-, sesqui-, di-, tri-, tetra-, penta- *etc.* solvates or hydrates, respectively, are possible. The present invention includes all such hydrates or solvates.
Further, it is possible for the compounds of the present invention to exist in free form, *e.g*. as a free base, or as a free acid, or as a zwitterion, or to exist in the form of a salt. Said salt may be any salt, either an organic or inorganic addition salt, particularly any pharmaceutically acceptable organic or inorganic addition salt, which is customarily used in pharmacy, or which is used, for example, for isolating or purifying the compounds of the present invention.
The term "pharmaceutically acceptable salt" refers to an inorganic or organic acid addition salt of a compound of the present invention. For example, see S. M. Berge, et al. "Pharmaceutical Salts," J. Pharm. Sci. 1977, 66, 1-19.
A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, or "mineral acid", such as hydrochloric, hydrobromic, hydroiodic, sulfuric, sulfamic, bisulfuric, phosphoric, or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, 3-phenylpropionic, pivalic, 2-hydroxyethanesulfonic, itaconic, trifluoromethanesulfonic, dodecylsulfuric, ethanesulfonic, benzenesulfonic, paratoluenesulfonic, methanesulfonic, 2-naphthalenesulfonic, naphthaline-disulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, or thiocyanic acid, for example.
Further, another suitably pharmaceutically acceptable salt of a compound of the present invention which is sufficiently acidic, is an alkali metal salt, for example a sodium or potassium salt, an alkaline earth metal salt, for example a calcium, magnesium or strontium salt, or an aluminium or a zinc salt, or an ammonium salt derived from ammonia or from an organic primary, secondary or tertiary amine having 1 to 20 carbon atoms, such as ethylamine, diethylamine, triethylamine, ethyldiisopropylamine, monoethanolamine, diethanolamine, triethanolamine, dicyclohexylamine, dimethylaminoethanol, diethylaminoethanol, tris(hydroxymethyl)aminomethane, procaine, dibenzylamine, *N-*methylmorpholine, arginine, lysine, 1,2-ethylenediamine, *N*-methylpiperidine, *N*-methylglucamine, *N,N*-dimethyl-glucamine, *N*-ethyl-glucamine, 1,6-hexanediamine, glucosamine, sarcosine, serinol, 2-amino-1,3-propanediol, 3-amino-1,2-propanediol, 4-amino-1,2,3-butanetriol, or a salt with a quarternary ammonium ion having 1 to 20 carbon atoms, such as tetramethylammonium, tetraethylammonium, tetra(*n*-propyl)ammonium, tetra(n-butyl)ammonium, *N*-benzyl-*N,N,N*-trimethylammonium, choline or benzalkonium.
Those skilled in the art will further recognise that it is possible for acid addition salts of the claimed compounds to be prepared by reaction of the compounds with the appropriate inorganic or organic acid via any of a number of known methods. Alternatively, alkali and alkaline earth metal salts of acidic compounds of the present invention are prepared by reacting the compounds of the present invention with the appropriate base via a variety of known methods.
The present invention includes all possible salts of the compounds of the present invention as single salts, or as any mixture of said salts, in any ratio.
In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.
Unless specified otherwise, suffixes to chemical names or structural formulae relating to salts, such as "hydrochloride", "trifluoroacetate", "sodium salt", or "x HCl", "x CF3COOH", "x Na+", for example, mean a salt form, the stoichiometry of which salt form not being specified.
This applies analogously to cases in which synthesis intermediates or example compounds or salts thereof have been obtained, by the preparation and/or purification processes described, as solvates, such as hydrates, with (if defined) unknown stoichiometric composition.
Furthermore, the present invention includes all possible crystalline forms, or polymorphs, of the compounds of the present invention, either as single polymorph, or as a mixture of more than one polymorph, in any ratio.
Moreover, the present invention also includes prodrugs of the compounds according to the invention. The term "prodrugs" here designates compounds which themselves can be biologically active or inactive but are converted (for example metabolically or hydrolytically) into compounds according to the invention during their residence time in the body.

In accordance with a second embodiment of the first aspect, the present invention covers compounds of general formula (I): 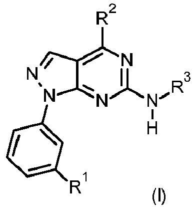 wherein
- R¹: represents C₁-C₄-alkyl, C₁-C₄-alkoxy, C₁-C₆-alkylsulfonyl, or cyano,
- wherein said C₁-C₄-alkyl is optionally substituted with one or more substituents which are the same or different and independently selected from the group consisting of fluorine atom and hydroxy, and
- wherein said C₁-C₄-alkoxy is substituted with 1 to 6 fluorine atoms.

In accordance with a third embodiment of the first aspect, the present invention covers compounds of general formula (I), wherein
- R²: represents 4- to 7-membered monocyclic saturated heterocycloalkyl containing one ring nitrogen atom and optionally one additional heteroatom or heteroatom-containing group independently selected from the group consisting of N, O, and S(=O)₂,
- wherein said 4- to 7-membered monocyclic saturated heterocycloalkyl is bound to the rest of the molecule via any nitrogen atom, and
- wherein said 4- to 7-membered monocyclic saturated heterocycloalkyl is optionally substituted with one or more substituents which are the same or different and independently selected from the group consisting of an oxo substituent, fluorine atom, hydroxy, cyano, C₁-C₆-alkoxy, CO₂H, NH₂, NR^{a}R^{b}, -S(=O)₂R^{b}, -S(=O)₂R⁴ -(C=O)R^{b}, -(C=O)NH₂, -(C=O)NR^{a}R^{b}, -(C=O)R⁴, R⁵, R⁶, R⁷, R⁸, and R⁹, and
with R⁴, R⁵, R⁶, R⁷, R⁸, and R⁹ as well as R^{a} and R^{b} having the same meaning as defined in general formula (I).

In accordance with a fourth embodiment of the first aspect, the present invention covers compounds of general formula (I), wherein
- R³: represents 4- to 6-membered monocyclic saturated heterocycloalkyl containing one to two heteroatom or heteroatom-containing groups selected from N, O, and S(=O)₂, C₃-C₆-cycloalkyl, 6- to 11-membered heterospirocycloalkyl containing one heteroatom or heteroatom-containing group independently selected from N, O and S(=O)₂, or 6- to 12-membered bridged heterocycloalkyl group containing one heteroatom or heteroatom-containing group independently selected from N, O, and S(=O)₂,
- wherein said 4- to 6-membered monocyclic saturated heterocycloalkyl, said 6-to 11-membered heterospirocycloalkyl and said 6- to 12-membered bridged heterocycloalkyl group are bound to the rest of the molecule via any of the carbon atoms, and
- wherein said 4- to 6-membered monocyclic saturated heterocycloalkyl is optionally substituted with one or more substituents which are the same or different and independently selected from the group consisting of oxo substituent, fluorine atom, hydroxy, cyano, CO₂H, C₁-C₆-alkoxy, NH₂, NR^{a}R^{b}, - S(=O)₂R^{b}, -S(=O)₂R^{c}, cyclopropyl, cyclobutyl, and C₁-C₆-alkyl optionally substituted with one to five fluorine atoms or with one hydroxy,
- wherein said C₃-C₆-cycloalkyl is optionally substituted with one or more substituents which are the same or different and independently selected from the group consisting of oxo substituent, fluorine atom, hydroxy, cyano, CO₂H, C₁-C₆-alkoxy, NH₂, NR^{a}R^{b}, -S(=O)₂R^{b}, -S(=O)₂NH₂, -S(=O)₂NR^{a}R^{b}, -C(=O)NH₂, -C(=O)NR^{a}R^{b}, and C₁-C₆-alkyl, wherein said C₁-C₆-alkyl is optionally substituted with one to five halogen atoms or with one hydroxy,
- wherein said 6- to 11-membered heterospirocycloalkyl is optionally substituted with one or more substituents which are the same or different and independently selected from the group consisting of C₁-C₄-alkyl, fluorine atom, hydroxy, cyano, CO₂H, NH₂, and -S(=O)₂R^{b}, and
- wherein said 6- to 12-membered bridged heterocycloalkyl group is optionally substituted with one or more substituent which are the same or different and independently selected from the group consisting of C₁-C₆-alkyl, fluorine atom, and hydroxy, and
with R^{a}, R^{b} and R^{c} having the same meaning as defined in general formula (I).

In accordance with a fifth embodiment of the first aspect, the present invention covers compounds of general formula (I), wherein
- R⁴: represents 4- to 6-membered monocyclic saturated heterocycloalkyl group containing one nitrogen atom and optionally one additional heteroatom or heteroatom-containing group independently selected from N, O, and S(=O)₂, or 6- to 12-membered bridged heterocycloalkyl containing one nitrogen atom and optionally one additional heteroatom or heteroatom-containing group independently selected from the group consisting of N, O, and S(=O)₂,
- wherein said 4- to 6-membered monocyclic saturated heterocycloalkyl and said 6- to 12-membered bridged heterocycloalkyl are bound to the rest of the molecule via any nitrogen atom,
- wherein said 4- to 6-membered monocyclic saturated heterocycloalkyl and said 6- to 12-membered bridged heterocycloalkyl are optionally substituted with one or more substituents which are the same or different and independently selected from the group consisting of C₁-C₆-alkyl, fluorine atom, and hydroxy.

In accordance with a sixth embodiment of the first aspect, the present invention covers compounds of general formula (I), wherein
- R⁵: represents C₁-C₆-alkyl group optionally substituted with one or more substituents which are the same or different and independently selected from the group consisting of fluorine atom, hydroxy, CO₂H, cyclopropyl, cyano, C₁-C₆-alkoxy, NH₂, NR^{a}R^{b}, -S(=O)₂R^{b}, -S(=O)₂R⁴ -(C=O)R^{b}, -(C=O)NH₂, -(C=O)R^{a}R^{b}, -(C=O)R⁴, 4- to 6-membered monocyclic saturated heterocycloalkyl containing one or two identical or different heteroatoms or heteroatom-containing groups independently selected from the group consisting of N, O, and S(=O)₂, 6- to 12-membered bridged heterocycloalkyl group containing one nitrogen atom and optionally one additional heteroatom or heteroatom-containing group independently selected from the group consisting of N, O, and S(=O)₂, and 6- to 11-membered heterospirocycloalkyl containing one nitrogen atom and optionally one additional heteroatom or heteroatom-containing group independently selected from the group consisting of N, O, and S(=O)₂,
- wherein said 6- to 12-membered bridged heterocycloalkyl group and said 6- to 11-membered heterospirocycloalkyl are connected to the rest of the molecule via any nitrogen atom, and
- wherein said 4- to 6-membered monocyclic saturated heterocycloalkyl and said 6- to 12-membered bridged heterocycloalkyl group are optionally substituted with one or more substituents which are the same or different and independently selected from the group consisting of C₁-C₆-alkyl, fluorine atom, oxo substituent, and hydroxy, and
   - wherein said 6- to 11-membered heterospirocycloalkyl is optionally substituted with one or more substituents which are the same or different and independently selected from the group consisting of C₁-C₄-alkyl, fluorine atom, and hydroxy, and
with R^{a} and R^{b} having the same meaning as defined in general formula (I).

In accordance with a seventh embodiment of the first aspect, the present invention covers compounds of general formula (I), wherein
- R⁶: represents -C₃-C₆-cycloalkyl optionally substituted with one or more substituents which are the same or different and independently selected from the group consisting of fluorine atom, C₁-C₆-alkyl, and hydroxy.

In accordance with an eighth embodiment of the first aspect, the present invention covers compounds of general formula (I), wherein
- R⁷: represents 4- to 6-membered monocyclic saturated heterocycloalkyl containing one or two identical or different heteroatoms or heteroatom-containing groups independently selected from the group consisting of N, O, and S(=O)₂,
- wherein said 4- to 6-membered monocyclic saturated heterocycloalkyl is optionally substituted with one or more substituents which are the same or different and independently selected from the group consisting of an oxo substituent, fluorine atom, hydroxy, NH₂, NR^{a}R^{b}, cyano, C₁-C₆-alkoxy, CO₂H, - S(=O)₂R^{b}, and C₁-C₆-alkyl,
   - wherein said C₁-C₆-alkyl is optionally substituted with one or more substituents which are the same or different and independently selected from the group consisting of fluorine atom, hydroxy, NH₂, and NR^{a}R^{b}, and
with R^{a} and R^{b} having the same meaning as defined in general formula (I).

In accordance with a ninth embodiment of the first aspect, the present invention covers compounds of general formula (I), wherein
- R⁸: represents 6- to 12-membered bridged heterocycloalkyl containing one nitrogen atom and optionally one additional heteroatom or heteroatom-containing group independently selected from the group consisting of N, O, and S(=O)₂,
- wherein said 6- to 12-membered bridged heterocycloalkyl is connected to the rest of the molecule via any nitrogen atom, and
- wherein said 6- to 12-membered bridged heterocycloalkyl is optionally substituted with one or more substituents which are the same or different and independently selected from the group consisting of C₁-C₆-alkyl, fluorine atom, and hydroxy.

In accordance with a tenth embodiment of the first aspect, the present invention covers compounds of general formula (I), wherein
- R⁹: represents 6- to 11-membered heterospirocycloalkyl containing one nitrogen atom and optionally one additional heteroatom or heteroatom-containing group independently selected from the group consisting of N, O and S(=O)₂,
- wherein said 6- to 11-membered heterospirocycloalkyl is connected to the rest of the molecule via any nitrogen atom, and
- wherein said 6- to 11-membered heterospirocycloalkyl is optionally substituted with one or more substituents which are the same or different and independently selected from the group consisting of C₁-C₄-alkyl, fluorine atom, hydroxy, cyano, CO₂H, NH₂, and -S(=O)₂C₁-C₆-alkyl.

In accordance with a further embodiment of the first aspect, the present invention covers compounds of general formula (I), supra, in which
- R¹: represents trifluoromethyl, difluoromethyl, trifluoromethoxy, difluoromethoxy, methylsulfonyl, cyano or 2-hydroxypropan-2-yl.

In accordance with a further embodiment of the first aspect, the present invention covers compounds of general formula (I), supra, in which
- R¹: represents trifluoromethyl, difluromethyl or difluoromethoxy.

In accordance with a further embodiment of the first aspect, the present invention covers compounds of general formula (I), supra, in which
- R¹: represents trifluoromethyl.

In accordance with a further embodiment of the first aspect, the present invention covers compounds of general formula (I), supra, in which
R² represents R⁷, wherein R⁷ has the same meaning as defined in general formula (I).

In accordance with a further embodiment of the first aspect, the present invention covers compounds of general formula (I), supra, in which
- R²: represents 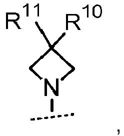 wherein
R¹⁰ represents hydrogen, NH₂, NR^{a}R^{b}, fluorine atom, hydroxy or C₁-C₆-alkyl,
R¹¹ represents C₁-C₆-alkyl optionally substituted with one or more substituents which are the same or different and independently selected from the group consisting of fluorine atom, hydroxy, NH₂, N(CH₃)₂, CO₂H, -S(=O)₂CH₃, -C(=O)(morpholin-4-yl),-C(=O)(pyrrolidin-1-yl), -C(=O)(piperazin-1-yl), -C(=O)(4-methylpiperazin-1-yl),-C(=O)(4-hydroxy-4-methylpiperidin-1-yl), -C(=O)(2-(6-oxa-3-azabicyclo[3.1.1]heptan-3-yl) and 4- to 6-membered monocyclic saturated heterocycloalkyl group containing one or two identical or different heteroatoms or heteroatom-containing groups independently selected from the group consisting of N, O, and S(=O)₂,
- wherein said 4- to 6-membered monocyclic saturated heterocycloalkyl group is bound to the rest of the molecule via any nitrogen atom, and
- wherein said 4- to 6-membered monocyclic saturated heterocycloalkyl group is optionally substituted with one or more substituents independently selected from the group consisting of fluorine atom, hydroxy, oxo substituent and C₁-C₆-alkyl group;
R^{a} represents hydrogen or C₁-C₆-alkyl; and
R^{b} represents C₁-C₆-alkyl.

In accordance with a further embodiment of the first aspect, the present invention covers compounds of general formula (I), supra, in which
- R²: represents 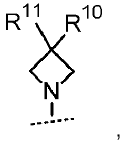 wherein
R¹⁰ represents hydrogen, NR^{a}R^{b}, or hydroxy,
R¹¹ represents C₁-C₄-alkyl optionally substituted with one or more substituents which are the same or different and independently selected from the group consisting of fluorine atom, hydroxy, NH₂, N(CH₃)₂, CO₂H, -S(=O)₂CH₃, -C(=O)(morpholin-4-yl), -C(=O)(4-hydroxy-4-methylpiperidin-1-yl), -C(=O)(2-(6-oxa-3-azabicyclo-[3.1.1]-heptan-3-yl) and 6-membered monocyclic saturated heterocycloalkyl group containing one or two identical or different heteroatoms or heteroatom-containing groups independently selected from the group consisting of N, O, and S(=O)₂,
- wherein said 6-membered monocyclic saturated heterocycloalkyl group is bound to the rest of the molecule via any nitrogen atom, and
- wherein said 6-membered monocyclic saturated heterocycloalkyl is optionally substituted with one or more substituents independently selected from the group consisting of fluorine atom, hydroxy, and C₁-C₆-alkyl group;
R^{a} represents C₁-C₆-alkyl; and
R^{b} represents C₁-C₆-alkyl.

In accordance with a further embodiment of the first aspect, the present invention covers compounds of general formula (I), supra, in which
- R²: represents 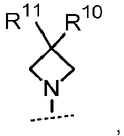 wherein
R¹⁰ represents hydrogen, NR^{a}R^{b}, hydroxy, and
R¹¹ represents C₁-C₄-alkyl optionally substituted with one or more substituents which are the same or different and independently selected from the group consisting of hydroxy, NH₂, N(CH₃)₂, CO₂H, -S(=O)₂CH₃, -C(=O)(morpholin-4-yl), -C(=O)(2-(6-oxa-3-azabicyclo-[3.1.1]-heptan-3-yl) and 6-membered monocyclic saturated heterocycloalkyl group containing one or two identical or different heteroatoms or heteroatom-containing groups independently selected from the group consisting of N, O, and S(=O)₂,
- wherein said 6-membered monocyclic saturated heterocycloalkyl group is bound to the rest of the molecule via any nitrogen atom, and
- wherein said 6-membered monocyclic saturated heterocycloalkyl is optionally substituted with one or more substituents independently selected from the group consisting of fluorine atom, hydroxy, methyl and ethyl group;
R^{a} represents C₁-C₃-alkyl;
R^{b} represents C₁-C₃-alkyl.

In accordance with a further embodiment of the first aspect, the present invention covers compounds of general formula (I), supra, in which
- R²: represents 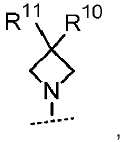 wherein
R¹⁰ represents hydrogen, NR^{a}R^{b}, hydroxy, and
R¹¹ represents C₁-C₄-alkyl optionally substituted with one or more substituents which are the same or different and independently selected from the group consisting of hydroxy, NH₂, N(CH₃)₂, CO₂H, -S(=O)₂CH₃, -C(=O)(morpholin-4-yl), and -C(=O)(2-(6-oxa-3-azabicyclo-[3.1.1]-heptan-3-yl), azetidin-1-yl, 3-hydroxyazetidin-1-yl, 3-fluoro-azetidin-1-yl, 3-hydroxy-3-methylazetidin-1-yl, pyrrolidine, 3-hydroxypyrrolidin-1-yl, 3-hydroxy-3-methylpyrrolidin-1-yl, 3-fluoropyrrolidin-1-yl, 3,3-difluoropyrrolidin-1-yl, morpholin-4-yl, 3-methylmorpholin-4-yl, 1,1-dioxidothiomorpholin-4-yl, piperidin-1-yl, 4-fluoropiperidin-1-yl, 4,4-difluoropiperidin-1-yl, 4-hydroxypiperidin-1-yl, and 4-hydroxy-4-methylpiperidin-1-yl;
R^{a} represents methyl;
R^{b} represents methyl.

In accordance with a further embodiment of the first aspect, the present invention covers compounds of general formula (I), supra, in which
- R²: represents 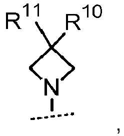 wherein
R¹⁰ represents hydrogen, and
R¹¹ represents C₁-C₄-alkyl optionally substituted with one or more substituents which are the same or different and independently selected from the group consisting of hydroxy, NH₂, N(CH₃)₂, CO₂H, -S(=O)₂CH₃, -C(=O)(morpholin-4-yl), and -C(=O)(2-(6-oxa-3-azabicyclo-[3.1.1]-heptan-3-yl), azetidin-1-yl, 3-hydroxyazetidin-1-yl, 3-fluoroazetidin-1-yl, 3-hydroxy-3-methylazetidin-1-yl, pyrrolidine, 3-hydroxypyrrolidin-1-yl, 3-hydroxy-3-methylpyrrolidin-1-yl, 3-fluoropyrrolidin-1-yl, 3,3-difluoropyrrolidin-1-yl, morpholin-4-yl, 3-methylmorpholin-4-yl, 1,1-dioxidothiomorpholin-4-yl, piperidin-1-yl, 4-fluoropiperidin-1-yl, 4,4-difluoropiperidin-1-yl, 4-hydroxypiperidin-1-yl, and 4-hydroxy-4-methylpiperidin-1-yl.

In accordance with a further embodiment of the first aspect, the present invention covers compounds of general formula (I), supra, in which
- R²: represents 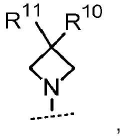 wherein
R¹⁰ represents hydrogen, and
R¹¹ represents C₁-C₄-alkyl substituted with one hydroxy.

In accordance with a further embodiment of the first aspect, the present invention covers compounds of general formula (I), supra, in which
- R²: represents 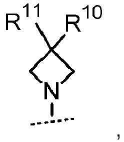 wherein
R¹⁰ represents hydrogen, and
R¹¹ represents C₁-C₄-alkyl substituted with one substituent selected from the group consisting of azetidin-1-yl, 3-hydroxyazetidin-1-yl, 3-fluoroazetidin-1-yl, 3-hydroxy-3-methylazetidin-1-yl, pyrrolidine, 3-hydroxypyrrolidin-1-yl, 3-hydroxy-3-methylpyrrolidin-1-yl, 3-fluoropyrrolidin-1-yl, 3,3-difluoropyrrolidin-1-yl, morpholin-4-yl, 3-methylmorpholin-4-yl, 1,1-dioxidothiomorpholin-4-yl, piperidin-1-yl, 4-fluoropiperidin-1-yl, 4,4-difluoropiperidin-1-yl, 4-hydroxypiperidin-1-yl, and 4-hydroxy-4-methylpiperidin-1-yl.

In accordance with a further embodiment of the first aspect, the present invention covers compounds of general formula (I), supra, in which
- R²: represents 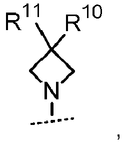 wherein
R¹⁰ represents hydrogen, and
R¹¹ represents morpholin-4-ylmethyl, (3-methylmorpholin-4-yl)methyl or (4-hydroxy-4-methylpiperidin-1-yl)methyl.

In accordance with a further embodiment of the first aspect, the present invention covers compounds of general formula (I), supra, in which
- R²: represents 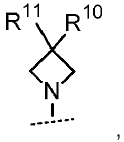 wherein
R¹⁰ represents hydrogen or C₁-C₆-alkyl, and
R¹¹ represents 4- to 6-membered monocyclic saturated heterocycloalkyl group containing one or two identical or different heteroatoms or heteroatom-containing groups independently selected from the group consisting of N, O, and S(=O)₂, wherein said heterocycloalkyl group is bound to the rest of the molecule via any nitrogen atom, and wherein said 4- to 6-membered monocyclic saturated heterocycloalkyl is optionally substituted with one or more substituents which are the same or different and independently selected from the group consisting of oxo substituent, fluorine atom, hydroxy, and C₁-C₆-alkyl,
- wherein said C₁-C₆-alkyl is optionally substituted with one to five fluorine atoms.

In accordance with a further embodiment of the first aspect, the present invention covers compounds of general formula (I), supra, in which
- R²: represents 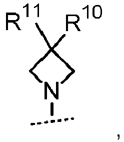 wherein
R¹⁰ represents hydrogen, methyl or ethyl, and
R¹¹ represents azetidin-1-yl, 3-hydroxyazetidin-1-yl, 3-fluoroazetidin-1-yl, 3-hydroxy-3-methylazetidin-1-yl, pyrrolidine, 3-hydroxypyrrolidin-1-yl, 3-hydroxy-3-methylpyrrolidin-1-yl, 3-fluoropyrrolidin-1-yl, 3,3-difluoropyrrolidin-1-yl, morpholin-4-yl, 3-methylmorpholin-4-yl, 1,1-dioxidothiomorpholin-4-yl, piperidin-1-yl, 4-fluoropiperidin-1-yl, 4,4-difluoropiperidin-1-yl, 4-hydroxypiperidin-1-yl, or 4-hydroxy-4-methylpiperidin-1-yl

In accordance with a further embodiment of the first aspect, the present invention covers compounds of general formula (I), supra, in which R² represents 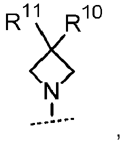 wherein
- R¹⁰: represents hydrogen or methyl, and
- R¹¹: represents morpholin-4-yl, 4-hydroxy-4-methylpiperidin-1-yl or 3,3-difluoropyrrolidin-1-yl

In accordance with a further embodiment of the first aspect, the present invention covers compounds of general formula (I), supra, in which R² represents 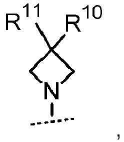 wherein
- R¹⁰: represents hydrogen, and
- R¹¹: represents morpholin-4-yl or 4-hydroxy-4-methylpiperidin-1-yl.

In accordance with a further embodiment of the first aspect, the present invention covers compounds of general formula (I), supra, in which R² represents 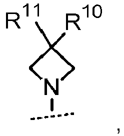 wherein
- R¹⁰: represents hydrogen, methyl or ethyl, and
- R¹¹: represents 2-oxopyrrolidin-1-yl, 2-oxopiperidin-1-yl, 3-oxomorpholin-4-yl, 2-oxomorpholin-4-yl, 2-oxopiperazin-1-yl, 3-oxopiperazin-1-yl, 4-methyl-3-oxopiperazin-1-yl or 4-methyl-2-oxopiperazin-1-yl.

In accordance with a further embodiment of the first aspect, the present invention covers compounds of general formula (I), supra, in which R² represents 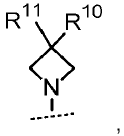 wherein
- R¹⁰: represents hydrogen, and
- R¹¹: represents 2-oxopyrrolidin-1-yl, 2-oxopiperidin-1-yl, 3-oxomorpholin-4-yl, 2-oxomorpholin-4-yl, 2-oxopiperazin-1-yl, 3-oxopiperazin-1-yl, 4-methyl-3-oxopiperazin-1-yl or 4-methyl-2-oxopiperazin-1-yl.

In accordance with a further embodiment of the first aspect, the present invention covers compounds of general formula (I), supra, in which R² represents 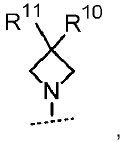 wherein
- R¹⁰: represents hydrogen, and
- R¹¹: represents 2-oxopyrrolidin-1-yl, 2-oxopiperidin-1-yl or 3-oxomorpholin-4-yl.

In accordance with a further embodiment of the first aspect, the present invention covers compounds of general formula (I), supra, in which
- R²: represents 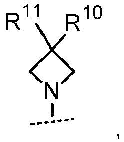 wherein
R¹⁰ represents hydrogen;
R¹¹ represents fluorine atom, hydroxy, cyano, C₁-C₆-alkoxy, CO₂H, NH₂, -N(CH₃)₂, N(CH₂CH₃)(CH₃), -S(=O)₂CH₃, -(C=O)NH₂, -(C=O)NR^{a}R^{b} or -(C=O)R⁴;
R⁴ represents 4- to 6-membered monocyclic saturated heterocycloalkyl group containing one nitrogen atom and optionally one additional heteroatom or heteroatom-containing group independently selected from N, O, and S(=O)₂, wherein said 4- to 6-membered monocyclic saturated heterocycloalkyl and said 6- to 12-membered bridged heterocycloalkyl are bound to the rest of the molecule via any nitrogen atom,
- wherein said 4- to 6-membered monocyclic saturated heterocycloalkyl is optionally substituted with one or more substituents which are the same or different and independently selected from the group consisting of C₁-C₆-alkyl, fluorine atom, and hydroxy;
R^{a} represents hydrogen or C₁-C₆-alkyl; and
R^{b} represents C₁-C₆-alkyl.

In accordance with a further embodiment of the first aspect, the present invention covers compounds of general formula (I), supra, in which
- R²: represents 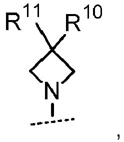 wherein
R¹⁰ represents hydrogen;
R¹¹ represents fluorine atom, hydroxy, -N(CH₃)₂, N(CH₂CH₃)(CH₃) or -(C=O)R⁴.

In accordance with a further embodiment of the first aspect, the present invention covers compounds of general formula (I), supra, in which
- R²: represents 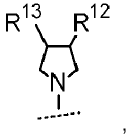 wherein
R¹² represents 4- to 6-membered monocyclic saturated heterocycloalkyl group containing one or two identical or different heteroatoms or heteroatom-containing groups independently selected from the group consisting of N, O, and S(=O)₂, wherein said heterocycloalkyl group is bound to the rest of the molecule via any nitrogen atom, and wherein said 4- to 6-membered monocyclic saturated heterocycloalkyl is optionally substituted with one or more substituents which are the same or different and independently selected from the group consisting of oxo substituent, fluorine atom, hydroxy, and C₁-C₆-alkyl,
- wherein said C₁-C₆-alkyl is optionally substituted with one to five fluorine atoms; and
R¹³ represents hydrogen.

In accordance with a further embodiment of the first aspect, the present invention covers compounds of general formula (I), supra, in which
- R²: represents 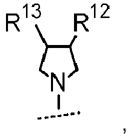 wherein
R¹² represents azetidin-1-yl, 3-hydroxyazetidin-1-yl, 3-fluoroazetidin-1-yl, 3-hydroxy-3-methylazetidin-1-yl, pyrrolidine, 3-hydroxypyrrolidin-1-yl, 3-hydroxy-3-methylpyrrolidin-1-yl, 3-fluoropyrrolidin-1-yl, 3,3-difluoropyrrolidin-1-yl, morpholin-4-yl, 3-methylmorpholin-4-yl, 1,1-dioxidothiomorpholin-4-yl, piperidin-1-yl, 4-fluoropiperidin-1-yl, 4,4-difluoropiperidin-1-yl, 4-hydroxypiperidin-1-yl, or 4-hydroxy-4-methylpiperidin-1-yl, and
R¹³ represents hydrogen.

In accordance with a further embodiment of the first aspect, the present invention covers compounds of general formula (I), supra, in which
- R²: represents 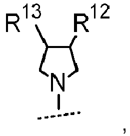 wherein
R¹² represents morpholin-4-yl, 4-hydroxy-4-methylpiperidin-1-yl or 3,3-difluoropyrrolidin-1-yl; and
R¹³ represents hydrogen.

In accordance with a further embodiment of the first aspect, the present invention covers compounds of general formula (I), supra, in which
- R²: represents 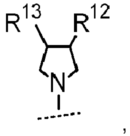 wherein
R¹² represents morpholin-4-yl; and
R¹³ represents hydrogen.

In accordance with a further embodiment of the first aspect, the present invention covers compounds of general formula (I), supra, in which
- R²: represents 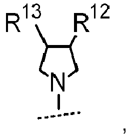 wherein
R¹² represents 2-oxopyrrolidin-1-yl, 2-oxopiperidin-1-yl, 3-oxomorpholin-4-yl, 2-oxomorpholin-4-yl, 2-oxopiperazin-1-yl, 3-oxopiperazin-1-yl, 4-methyl-3-oxopiperazin-1-yl or 4-methyl-2-oxopiperazin-1-yl; and
R¹³ represents hydrogen.

In accordance with a further embodiment of the first aspect, the present invention covers compounds of general formula (I), supra, in which
- R²: represents 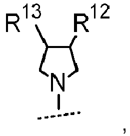 wherein
R¹² represents 2-oxopyrrolidin-1-yl, 2-oxopiperidin-1-yl or 3-oxomorpholin-4-yl; and
R¹³ represents hydrogen.

In accordance with a further embodiment of the first aspect, the present invention covers compounds of general formula (I), supra, in which
- R²: represents 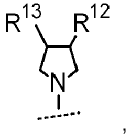 wherein
R¹² and R¹³ both are hydroxy.

In accordance with a further embodiment of the first aspect, the present invention covers compounds of general formula (I), supra, in which
- R²: represents 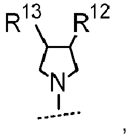 wherein
R¹² represents methyl or fluorine atom, and
R¹³ represents hydroxy, or
R¹² represents hydroxy, and
R¹³ represents hydrogen.

In accordance with a further embodiment of the first aspect, the present invention covers compounds of general formula (I), supra, in which
- R²: represents 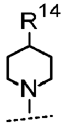 wherein
R¹⁴ represents fluorine atom, hydroxy, cyano, 1-aminocyclopropyl, -N(CH₃)₂, N(CH₂CH₃)(CH₃), -C(=O)(morpholin-4-yl), -C(=O)(pyrrolidin-1-yl), -C(=O)(piperazin-1-yl), -C(=O)(4-methylpiperazin-1-yl), -C(=O)(4-hydroxy-4-methylpiperidin-1-yl) or -C(=O)(2-(6-oxa-3-azabicyclo[3.1.1]heptan-3-yl), or
R¹⁴ represents C₁-C₄-alkyl optionally substituted with one or more substituents which are the same or different and independently selected from the group consisting of fluorine atom, hydroxy, NH₂, CO₂H, and -S(=O)₂CH₃, or
R¹⁴ represents 4- to 6-membered monocyclic saturated heterocycloalkyl group containing one or two identical or different heteroatoms or heteroatom-containing groups independently selected from the group consisting of N, O, and S(=O)₂,
- wherein said 4- to 6-membered monocyclic saturated heterocycloalkyl I group is bound to the rest of the molecule via any nitrogen atom, and
- wherein said 4- to 6-membered monocyclic saturated heterocycloalkyl group is optionally substituted with one or more substituents which are the same or different and independently selected from the group consisting of fluorine atom, oxo substituent, hydroxy, methyl and ethyl.

In accordance with a further embodiment of the first aspect, the present invention covers compounds of general formula (I), supra, in which
- R²: represents: 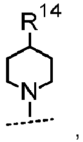 wherein
R¹⁴ represents fluorine atom, hydroxy, cyano, 1-aminocyclopropyl, -N(CH₃)₂, N(CH₂CH₃)(CH₃), -C(=O)(morpholin-4-yl), -C(=O)(pyrrolidin-1-yl), -C(=O)(piperazin-1-yl), -C(=O)(4-methylpiperazin-1-yl), -C(=O)(4-hydroxy-4-methylpiperidin-1-yl) or -C(=O)(2-(6-oxa-3-azabicyclo[3.1.1]heptan-3-yl).

In accordance with a further embodiment of the first aspect, the present invention covers compounds of general formula (I), supra, in which
- R²: represents: 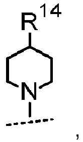 wherein
R¹⁴ represents C₁-C₄-alkyl optionally substituted with one or more substituents which are the same or different and independently selected from the group consisting of fluorine atom, hydroxy, NH₂, CO₂H, and -S(=O)₂CH₃.

In accordance with a further embodiment of the first aspect, the present invention covers compounds of general formula (I), supra, in which
- R²: represents 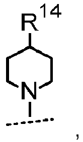 wherein
R¹⁴ represents C₁-C₄-alkyl optionally substituted with one hydroxy.

In accordance with a further embodiment of the first aspect, the present invention covers compounds of general formula (I), supra, in which
- R²: represents:

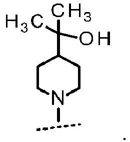

In accordance with a further embodiment of the first aspect, the present invention covers compounds of general formula (I), supra, in which
- R²: represents 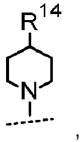 wherein
R¹⁴ represents 4- to 6-membered monocyclic saturated heterocycloalkyl group containing one or two identical or different heteroatoms or heteroatom-containing groups independently selected from the group consisting of N, O, and S(=O)₂,
- wherein said 4- to 6-membered monocyclic saturated heterocycloalkyl I group is bound to the rest of the molecule via any nitrogen atom, and
- wherein said 4- to 6-membered monocyclic saturated heterocycloalkyl group is optionally substituted with one or more substituents which are the same or different and independently selected from the group consisting of fluorine atom, oxo substituent, hydroxy, methyl and ethyl.

In accordance with a further embodiment of the first aspect, the present invention covers compounds of general formula (I), supra, in which
- R²: represents 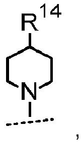 wherein
R¹⁴ represents azetidin-1-yl, 3-hydroxyazetidin-1-yl, 3-fluoroazetidin-1-yl, 3-hydroxy-3-methylazetidin-1-yl, pyrrolidine, 3-hydroxypyrrolidin-1-yl, 3-hydroxy-3-methyl-pyrrolidin-1-yl, 3-fluoropyrrolidin-1-yl, 3,3-difluoropyrrolidin-1-yl, morpholin-4-yl, 3-methylmorpholin-4-yl, 1,1-dioxidothiomorpholin-4-yl, piperidin-1-yl, 4-fluoropiperidin-1-yl, 4,4-difluoropiperidin-1-yl, 4-hydroxypiperidin-1-yl, or 4-hydroxy-4-methylpiperidin-1-yl.

In accordance with a further embodiment of the first aspect, the present invention covers compounds of general formula (I), supra, in which
- R²: represents 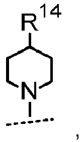 wherein
R¹⁴ represents 2-oxopyrrolidin-1-yl, 2-oxopiperidin-1-yl, 3-oxomorpholin-4-yl, 2-oxomorpholin-4-yl, 2-oxopiperazin-1-yl, 3-oxopiperazin-1-yl, 4-methyl-3-oxo-piperazin-1-yl or 4-methyl-2-oxopiperazin-1-yl.

In accordance with a further embodiment of the first aspect, the present invention covers compounds of general formula (I), supra, in which
- R²: represents 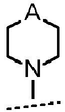 wherein
A represents oxygen atom, -S(=O)₂- or -NR¹⁵, and
R¹⁵ has the meaning of hydrogen, -C(=O)C₁-C₆-alkyl, -C(=O)C₃-C₅-cycloalkyl, -C(=O)(morpholin-4-yl), -C(=O)(pyrrolidin-1-yl), -C(=O)(piperazin-1-yl), -C(=O)(4-methylpiperazin-1-yl), -C(=O)(4-hydroxy-4-methylpiperidin-1-yl), or -C(=O)(2-(6-oxa-3-azabicyclo[3.1.1]heptan-3-yl), or
R¹⁵ has the meaning of C₁-C₆-alkyl optionally substituted with one or more substituents which are the same or different and independently selected from the group consisting of fluorine atom, hydroxy, and C₁-C₆-alkoxy, or
R¹⁵ has the meaning of 4- to 6-membered monocyclic saturated heterocycloalkyl group containing one or two identical or different heteroatoms or heteroatom-containing groups independently selected from the group consisting of N, O, and S(=O)₂,
- wherein said 4- to 6-membered monocyclic saturated heterocycloalkyl group is bound to the rest of the molecule via any one of the carbon atoms, and
- wherein said 4- to 6-membered monocyclic saturated heterocycloalkyl group is optionally substituted with one or more substituents which are the same or different and independently selected from the group consisting of fluorine atom, hydroxy, methyl and ethyl group.

In accordance with a further embodiment of the first aspect, the present invention covers compounds of general formula (I), supra, in which
- R³: has the meaning of a 4- to 6-membered monocyclic saturated heterocycloalkyl group containing one to two heteroatoms or heteroatom-containing groups selected from N, O or S(=O)₂,
- which is bound to the rest of the molecule via any of the carbon atoms, and
- which is optionally substituted with one or more substituents which are the same or different and independently selected from the group consisting of oxo substituent, fluorine atom, hydroxy, NH₂, NR^{a}R^{b}, -S(=O)₂C₁-C₆-alkyl,-S(=O)₂cyclopropyl, and C₁-C₄ alkyl group,
   - wherein said C₁-C₄ alkyl group is optionally substituted with one to three fluorine atoms.

In accordance with a further embodiment of the first aspect, the present invention covers compounds of general formula (I), supra, in which
R³ represents 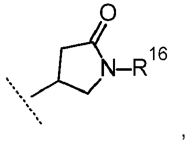 wherein R¹⁶ has the meaning of hydrogen or methyl.

In accordance with a further embodiment of the first aspect, the present invention covers compounds of general formula (I), supra, in which 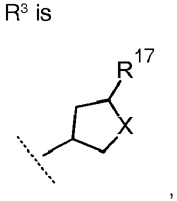 wherein
- R¹⁷: represents methyl, and
- X: represents -NH-,
or
- R¹⁷: represents hydrogen, and
- X: represents -NH- or -NCH₃-,
or
- R¹⁷: represents hydrogen, and
- X: represents -O-.

In accordance with a further embodiment of the first aspect, the present invention covers compounds of general formula (I), supra, in which
R³ has the meaning of

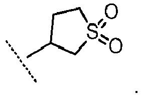

In accordance with a further embodiment of the first aspect, the present invention covers compounds of general formula (I), supra, in which
R³ has the meaning of

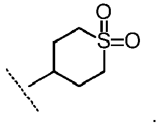

In accordance with a further embodiment of the first aspect, the present invention covers compounds of general formula (I), supra, in which
R³ has the meaning of 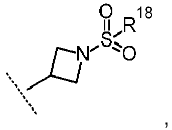 wherein
- R¹⁸: represents C₁-C₆-alkyl or cyclopropyl.

In accordance with a further embodiment of the first aspect, the present invention covers compounds of general formula (I), supra, in which
- R³: represents a C₃-C₆-cycloalkyl optionally substituted with one or more substituents which are the same or different and independently selected from the group consisting of fluorine atom, hydroxy, C₁-C₄-alkoxy, NH₂, NR^{a}R^{b}, -(C=O)NH₂, -(C=O)NHC₁-C₆-alkyl, -S(=O)₂C₁-C₆-alkyl, and C₁-C₆ alkyl,
- wherein said C₁-C₆ alkyl group is optionally substituted with one to three fluorine atoms, and
wherein R^{a} and R^{b} have the same meaning as defined in general formula (I).

In accordance with a further embodiment of the first aspect, the present invention covers compounds of general formula (I), supra, in which
R³ has the meaning of 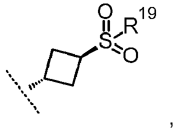 wherein
- R¹⁹: represents C₁-C₆-alkyl.

In accordance with a further embodiment of the first aspect, the present invention covers compounds of general formula (I), supra, in which
- R³: has the meaning of 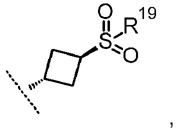 wherein
R¹⁹ represents methyl or ethyl.

In accordance with a further embodiment of the first aspect, the present invention covers compounds of general formula (I), supra, in which
- R³: represents 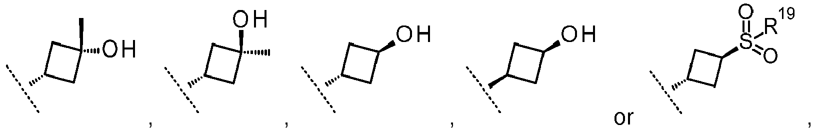 wherein
R¹⁹ represents methyl or ethyl.

In accordance with a further embodiment of the first aspect, the present invention covers compounds of general formula (I), supra, in which
- R³: represents

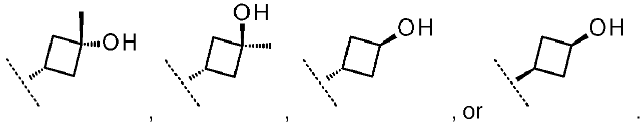

In accordance with a further embodiment of the first aspect, the present invention covers compounds of general formula (I), supra, in which
R³ is

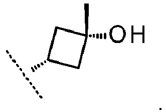

In accordance with a further embodiment of the first aspect, the present invention covers compounds of general formula (I), supra, in which
R³ is

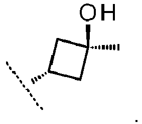

In accordance with a further embodiment of the first aspect, the present invention covers compounds of general formula (I), supra, in which
R³ is

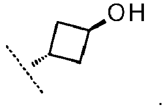

In accordance with a further embodiment of the first aspect, the present invention covers compounds of general formula (I), supra, in which
R³ is

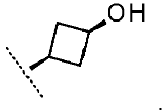

In accordance with a further embodiment of the first aspect, the present invention covers compounds of general formula (I), supra, in which
- R³: represents 6- to 11-membered heterospirocycloalkyl containing one heteroatom or heteroatom-containing group independently selected from N, O and S(=O)₂,
- which is bound to the rest of the molecule via any of the carbon atoms, and
- which is optionally substituted with one or more substituents which are the same or different and independently selected from the group consisting of C₁-C₄-alkyl, and fluorine atom.

In accordance with a further embodiment of the first aspect, the present invention covers compounds of general formula (I), supra, in which
- R³: represents 2-oxaspiro[3.3]heptan-6-yl, 2-azaspiro[3.3]heptan-6-yl, 1-azaspiro[3.3]-heptan-6-yl, 5-azaspiro[3.4]octan-2-yl or 6-azaspiro[3.4]octan-2-yl, each of which is optionally substituted with one or more substituents which are the same or different and independently selected from the group consisting of C₁-C₄ alkyl and fluorine atom.

In accordance with a further embodiment of the first aspect, the present invention covers compounds of general formula (I), supra, in which
- R³: represents unsubstituted 2-oxaspiro[3.3]heptan-6-yl, 2-azaspiro[3.3]heptan-6-yl, 1-azaspiro[3.3]heptan-6-yl, 5-azaspiro[3.4]octan-2-yl or 6-azaspiro[3.4]octan-2-yl.

In accordance with a further embodiment of the first aspect, the present invention covers compounds of general formula (I), supra, in which
- R³: is 1-azaspiro[3.3]heptan-6-yl.

In accordance with a further embodiment of the first aspect, the present invention covers compounds of general formula (I), supra, in which
- R³: represents 6- to 12-membered bridged heterocycloalkyl group containing one heteroatom or heteroatom-containing group independently selected from N, O, and S(=O)₂,
- which is bound to the rest of the molecule via any of the carbon atoms, and
- which is optionally substituted with one or more substituents which are the same or different and independently selected from the group consisting of C₁-C₄-alkyl, and fluorine atom.

In accordance with a further embodiment of the first aspect, the present invention covers compounds of general formula (I), supra, in which
- R³: represents unsubstituted 6- to 8-membered bridged heterocycloalkyl group containing one heteroatom or heteroatom-containing group independently selected from N, O, and S(=O)₂,

In accordance with a further embodiment of the first aspect, the present invention covers compounds of general formula (I), supra, in which
R³ is

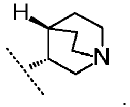

In accordance with a further embodiment of the first aspect, the present invention covers compounds of general formula (I), supra, in which
- R¹: represents trifluoromethyl, difluoromethyl or difluoromethoxy,
- R²: represents 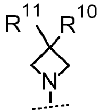 wherein
R¹⁰ represents hydrogen, methyl or ethyl,
R¹¹ represents hydroxy, -N(CH₃)₂, azetidin-1-yl, 3-hydroxyazetidin-1-yl, 3-fluoroazetidin-1-yl, 3-hydroxy-3-methylazetidin-1-yl, pyrrolidine, 3-hydroxypyrrolidin-1-yl, 3-hydroxy-3-methylpyrrolidin-1-yl, 3-fluoropyrrolidin-1-yl, 3,3-difluoropyrrolidin-1-yl, morpholin-4-yl, 3-methylmorpholin-4-yl, 1,1-dioxidothiomorpholin-4-yl, piperidin-1-yl, 4-fluoropiperidin-1-yl, 4,4-difluoropiperidin-1-yl, 4-hydroxypiperidin-1-yl, 4-hydroxy-4-methylpiperidin-1-yl, or C₁-C₄-alkyl,
- wherein said C₁-C₄-alkyl is optionally substituted with one or more substituents which are the same or different and independently selected from the group consisting of fluorine atom, hydroxy, NH₂, CO₂H, morpholin-4-yl,-S(=O)₂CH₃, -C(=O)(morpholin-4-yl), -C(=O)(4-hydroxy-4-methylpiperidin-1-yl), -C(=O)(2-(6-oxa-3-azabicyclo[3.1.1]heptan-3-yl), and
R³ represents 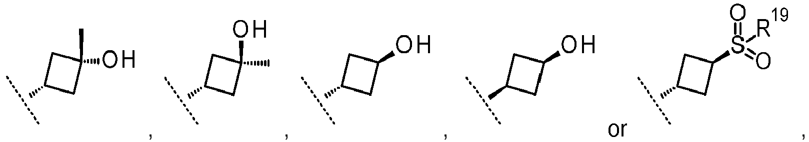 wherein
R¹⁹ represents methyl or ethyl.

In accordance with a further embodiment of the first aspect, the present invention covers compounds of general formula (I), supra, in which
- R¹: represents trifluoromethyl, difluoromethyl or difluoromethoxy,
- R²: represents 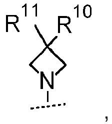 wherein
R¹⁰ represents hydrogen,
R¹¹ represents azetidin-1-yl, 3-hydroxyazetidin-1-yl, 3-fluoroazetidin-1-yl, 3-hydroxy-3-methyl azetidin-1-yl, pyrrolidine, 3-hydroxypyrrolidin-1-yl, 3-hydroxy-3-methyl-pyrrolidin-1-yl, 3-fluoropyrrolidin-1-yl, 3,3-difluoropyrrolidin-1-yl, morpholin-4-yl, 3-methylmorpholin-4-yl, 1,1-dioxidothiomorpholin-4-yl, piperidin-1-yl, 4-fluoropiperidin-1-yl, 4,4-difluoropiperidin-1-yl, 4-hydroxypiperidin-1-yl or 4-hydroxy-4-methylpiperidin-1-yl, and
R³ represents

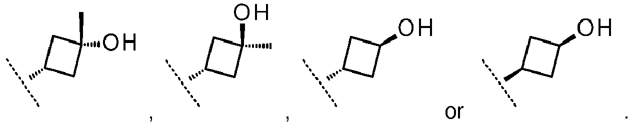

In accordance with a further embodiment of the first aspect, the present invention covers compounds of general formula (I), supra, in which
- R¹: represents trifluoromethyl, difluoromethyl or difluoromethoxy,
- R²: represents 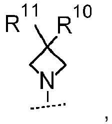 wherein
R¹⁰ represents hydrogen,
R¹¹ represents azetidin-1-yl, 3-hydroxyazetidin-1-yl, 3-fluoroazetidin-1-yl, 3-hydroxy-3-methylazetidin-1-yl, pyrrolidine, 3-hydroxypyrrolidin-1-yl, 3-hydroxy-3-methylpyrrolidin-1-yl, 3-fluoropyrrolidin-1-yl, 3,3-difluoropyrrolidin-1-yl, morpholin-4-yl, 3-methylmorpholin-4-yl, 1,1-dioxidothiomorpholin-4-yl, piperidin-1-yl, 4-fluoropiperidin-1-yl, 4,4-difluoro-piperidin-1-yl, 4-hydroxypiperidin-1-yl or 4-hydroxy-4-methylpiperidin-1-yl, and
R³ represents

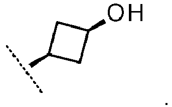

In accordance with a further embodiment of the first aspect, the present invention covers compounds of general formula (I), supra, in which
R¹ represents trifluoromethyl, difluoromethyl or difluoromethoxy,
R² represents 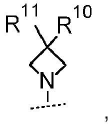 wherein
- R¹⁰: represents hydrogen,
- R¹¹: represents hydroxy, -N(CH₃)₂, azetidin-1-yl, 3-hydroxyazetidin-1-yl, 3-fluoroazetidin-1-yl, 3-hydroxy-3-methylazetidin-1-yl, pyrrolidine, 3-hydroxypyrrolidin-1-yl, 3-hydroxy-3-methylpyrrolidin-1-yl, 3-fluoropyrrolidin-1-yl, 3,3-difluoropyrrolidin-1-yl, morpholin-4-yl, 3-methylmorpholin-4-yl, 1,1-dioxidothiomorpholin-4-yl, piperidin-1-yl, 4-fluoropiperidin-1-yl, 4,4-difluoropiperidin-1-yl, 4-hydroxypiperidin-1-yl, 4-hydroxy-4-methylpiperidin-1-yl, or C₁-C₄-alkyl,
- wherein said C₁-C₄-alkyl is optionally substituted with one or more substituents which are the same or different and independently selected from the group consisting of fluorine atom, hydroxy, NH₂, N(CH₃)₂, CO₂H, morpholin-4-yl,-S(=O)₂CH₃ , -C(=O)(morpholin-4-yl), -C(=O)(4-hydroxy-4-methylpiperidin-1-yl) and -C(=O)(2-(6-oxa-3-azabicyclo[3.1.1]heptan-3-yl), and
- R³: represents

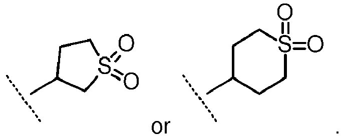

In accordance with a further embodiment of the first aspect, the present invention covers compounds of general formula (I), supra, in which
- R¹: represents trifluoromethyl, difluoromethyl or difluoromethoxy,
- R²: represents 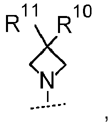 wherein
R¹⁰ represents hydrogen,
R¹¹ represents 2-oxopyrrolidin-1-yl, 2-oxopiperidin-1-yl, or 3-oxomorpholin-4-yl, and
R³ represents C₃-C₆-cycloalkyl optionally substituted with one or more substituents which are the same or different and independently selected from the group consisting of fluorine atoms, hydroxy, C₁-C₄-alkoxy, NH2, NR^{a}R^{b}, -S(=O)₂C₁-C₆-alkyl and C₁-C₆ alkyl
- wherein said C₁-C₆ alkyl is optionally substituted with one to three fluorine atoms, and
wherein R^{a} and R^{b} have the same meaning as defined in general formula (I).

In accordance with a further embodiment of the first aspect, the present invention covers compounds of general formula (I), supra, in which
- R¹: represents trifluoromethyl, difluoromethyl or difluoromethoxy,
- R²: represents 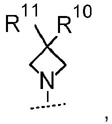 wherein
R¹⁰ represents hydrogen,
R¹¹ represents 2-oxopyrrolidin-1-yl, 2-oxopiperidin-1-yl, 3-oxomorpholin-4-yl, and
R³ represents

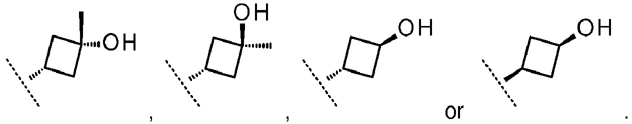

In accordance with a further embodiment of the first aspect, the present invention covers compounds of general formula (I), supra, in which
- R¹: represents trifluoromethyl, difluoromethyl or difluoromethoxy
- R²: represents 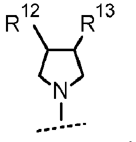 wherein
R¹² and R¹³ both have the meaning of hydroxy, and
R³ represents 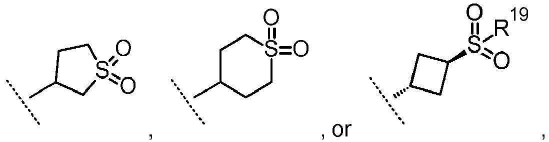 wherein
R¹⁹ represents methyl or ethyl.

In accordance with a further embodiment of the first aspect, the present invention covers compounds of general formula (I), supra, in which
- R¹: represents trifluoromethyl, difluoromethyl or difluoromethoxy
- R²: represents: 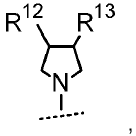 wherein
R¹² represents hydrogen, methyl or fluorine atom, and
R¹³ represents hydroxy, and
R³ represents 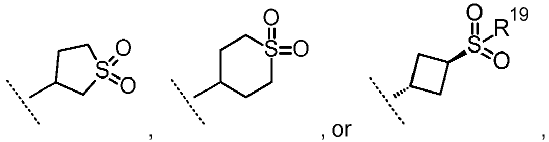 wherein
R¹⁹ represents methyl or ethyl.

The following compounds are disclosed, namely:

| Example | Name |
|---|---|
| I-1-1 | (4S)-4-({4-[3-(morpholin-4-yl)azetidin-1-yl]-1-[3-(trifluoromethyl)phenyl]-1H-pyrazolo[3,4-d]pyrimidin-6-yl}amino)pyrrolidin-2-one |
| I-1-2 | (4R)-4-({4-[3-(morpholin-4-yl)azetidin-1-yl]-1-[3-(trifluoromethyl)phenyl]-1H-pyrazolo[3,4-d]pyrimidin-6-yl}amino)pyrrolidin-2-one |
| I-2-1 | (4S)-4-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one |
| I-2-2 | ((4R)-4-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one |
| I-2-3 | 4-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one |
| I-3-1 | (4S)-4-({4-(3-ethyl-3-hydroxyazetidin-1-yl)-1-[3-(trifluoromethyl)phenyl]-1H-pyrazolo[3,4-d]pyrimidin-6-yl}amino)pyrrolidin-2-one |
| I-3-2 | (4R)-4-({4-(3-ethyl-3-hydroxyazetidin-1-yl)-1-[3-(trifluoromethyl)phenyl]-1H-pyrazolo[3,4-d]pyrimidin-6-yl}amino)pyrrolidin-2-one |
| I-4-1 | 2-(1-{6-[(1,1-dioxidotetrahydrothiophen-3-yl)amino]-1-[3-(trifluoromethyl)phenyl]-1H-pyrazolo[3,4-d]pyrimidin-4-yl}piperidin-4-yl)propan-2-ol |
| I-4-2 | (+)-2-(1-[6-[(1,1-dioxidotetrahydrothiophen-3-yl)amino]-1-[3-(trifluoromethyl)phenyl]-1H-pyrazolo[3,4-d]pyrimidin-4-yl}piperidin-4-yl)propan-2-ol |
| I-4-3 | (-)-2-(1-{6-[(1,1-dioxidotetrahydrothiophen-3-yl)amino]-1-[3-(trifluoromethyl)phenyl]-1H-pyrazolo[3,4-d]pyrimidin-4-yl}piperidin-4-yl)propan-2-ol |
| I-5 | 2-(1-{6-[(1,1-dioxidotetrahydro-2H-thiopyran-4-yl)amino]-1-[3-(trifluoromethyl)phenyl]-1H-pyrazolo[3,4-d]pyrimidin-4-yl}piperidin-4-yl)propan-2-ol |
| I-6 | 4-({4-[4-(2-hydroxypropan-2-yl)piperidin-1-yl]-1-[3-(trifluoromethyl)phenyl]-1H-pyrazolo[3,4-d]pyrimidin-6-yl}amino)-5,5-dimethylpyrrolidin-2-one |
| I-7-1 | (5R)-5-({4-[4-(2-hydroxypropan-2-yl)piperidin-1-yl]-1-[3-(trifluoromethyl)phenyl]-1H-pyrazolo[3,4-d]pyrimidin-6-yl}amino)piperidin-2-one |
| I-7-2 | (5S)-5-({4-[4-(2-hydroxypropan-2-yl)piperidin-1-yl]-1-[3-(trifluoromethyl)phenyl]-1H-pyrazolo[3,4-d]pyrimidin-6-yl}amino)piperidin-2-one |
| I-8 | 4-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylpyrrolidin-2-one |
| I-9 | (3R)-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylpyrrolidin-2-one |
| I-10 | 2-(1-{6-[(1,1-dioxidotetrahydro-2H-thiopyran-3-yl)amino]-1-[3-(trifluoromethyl)phenyl]-1H-pyrazolo[3,4-d]pyrimidin-4-yl}piperidin-4-yl)propan-2-ol |
| I-11-1 | (3S)-3-({4-[4-(2-hydroxypropan-2-yl)piperidin-1-yl]-1-[3-(trifluoromethyl)phenyl]-1H-pyrazolo[3,4-d]pyrimidin-6-yl}amino)piperidin-2-one |
| I-11-2 | (3R)-3-({4-[4-(2-hydroxypropan-2-yl)piperidin-1-yl]-1-[3-(trifluoromethyl)phenyl]-1H-pyrazolo[3,4-d]pyrimidin-6-yl}amino)piperidin-2-one |
| I-12 | (3S)-3-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one |
| I-13-1 | 2-[1-(6-{[(3S)-1-methylpyrrolidin-3-yl]amino}-1-[3-(trifluoromethyl)phenyl]-1H-pyrazolo[3,4-d]pyrimidin-4-yl)piperidin-4-yl]propan-2-ol |
| I-13-2 | 2-[1-(6-{[(3R)-1-methylpyrrolidin-3-yl]amino}-1-[3-(trifluoromethyl)phenyl]-1H-pyrazolo[3,4-d]pyrimidin-4-yl)piperidin-4-yl]propan-2-ol |
| I-14 | 2-[1-(6-{[1-(methylsulfonyl)azetidin-3-yl]amino}-1-[3-(trifluoromethyl)phenyl]-1H-pyrazolo[3,4-d]pyrimidin-4-yl)piperidin-4-yl]propan-2-ol |
| I-15 | (3R)-3-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one |
| I-16-1 | 2-(1-{6-[(3S)-tetrahydrofuran-3-ylamino]-1-[3-(trifluoromethyl)phenyl]-1H-pyrazolo[3,4-d]pyrimidin-4-yl}piperidin-4-yl)propan-2-ol |
| I-16-2 | 2-(1-{6-[(3R)-tetrahydrofuran-3-ylamino]-1-[3-(trifluoromethyl)phenyl]-1H-pyrazolo[3,4-d]pyrimidin-4-yl}piperidin-4-yl)propan-2-ol |
| I-17 | (4S)-4-({4-[4-(2-hydroxypropan-2-yl)piperidin-1-yl]-1-[3-(trifluoromethyl)phenyl]-1H-pyrazolo[3,4-d]pyrimidin-6-yl}amino)-3,3-dimethylpyrrolidin-2-one |
| I-18 | 3-ethyl-1-(6-{[(3S)-1-methylpyrrolidin-3-yl]amino}-1-[3-(trifluoromethyl)phenyl]-1H-pyrazolo[3,4-d]pyrimidin-4-yl)azetidin-3-ol |
| I-19 | 1-{6-[(1,1-dioxidotetrahydro-2H-thiopyran-4-yl)amino]-1-[3-(trifluoromethyl)phenyl]-1H-pyrazolo[3,4-d]pyrimidin-4-yl}-3-ethylazetidin-3-ol |
| I-20-1 | (4R)-4-({4-[(3S,4S)-3,4-dihydroxypyrrolidin-1-yl]-1-[3-(trifluoromethyl)phenyl]-1H-pyrazolo[3,4-d]pyrimidin-6-yl}amino)pyrrolidin-2-one |
| I-20-2 | (4S)-4-({4-[(3S,4S)-3,4-dihydroxypyrrolidin-1-yl]-1-[3-(trifluoromethyl)phenyl]-1H-pyrazolo[3,4-d]pyrimidin-6-yl}amino)pyrrolidin-2-one |
| I-21 | (4S)-4-({4-[(3R)-3-(morpholin-4-yl)pyrrolidin-1-yl]-1-[3-(trifluoromethyl)phenyl]-1H-pyrazolo[3,4-d]pyrimidin-6-yl}amino)pyrrolidin-2-one |
| I-22 | N-[(3R)-1,1-dioxidotetrahydrothiophen-3-yl]-4-[3-(morpholin-4-yl)azetidin-1-yl]-1-[3-(trifluoromethyl)phenyl]-1H-pyrazolo[3,4-d]pyrimidin-6-amine |
| I-23 | N-(1,1-dioxidotetrahydro-2H-thiopyran-4-yl)-4-[3-(morpholin-4-yl)azetidin-1-yl]-1-[3-(trifluoromethyl)phenyl]-1H-pyrazolo[3,4-d]pyrimidin-6-amine |
| I-24 | (4S)-4-({4-[4-(2-hydroxy-2-methylpropyl)piperidin-1-yl]-1-[3-(trifluoromethyl)phenyl]-1H-pyrazolo[3,4-d]pyrimidin-6-yl}amino)pyrrolidin-2-one |
| I-25-1 | 2-methyl-1-[1-(6-{[(3R)-1-methylpyrrolidin-3-yl]amino}-1-[3-(trifluoromethyl)phenyl]-1H-pyrazolo[3,4-d]pyrimidin-4-yl)piperidin-4-yl]propan-2-ol |
| I-25-2 | 2-methyl-1-[1-(6-{[(3S)-1-methylpyrrolidin-3-yl]amino}-1-[3-(trifluoromethyl)phenyl]-1H-pyrazolo[3,4-d]pyrimidin-4-yl)piperidin-4-yl]propan-2-ol |
| I-26-1 | 4-[3-(1,1-dioxidothiomorpholin-4-yl)azetidin-1-yl]-N-[(3R)-tetrahydrofuran-3-yl]-1-[3-(trifluoromethyl)phenyl]-1 H-pyrazolo[3,4-d]pyrimidin-6-amine |
| I-26-2 | 4-[3-(1,1-dioxidothiomorpholin-4-yl)azetidin-1-yl]-N-[(3S)-tetrahydrofuran-3-yl]-1-[3-(trifluoromethyl)phenyl]-1H-pyrazolo[3,4-d]pyrimidin-6-amine |
| I-27 | (4S)-4-({4-[4-(morpholin-4-yl)piperidin-1-yl]-1-[3-(trifluoromethyl)phenyl]-1H-pyrazolo[3,4-d]pyrimidin-6-yl}amino)pyrrolidin-2-one |
| I-28 | (4S)-4-({4-(morpholin-4-yl)-1-[3-(trifluoromethyl)phenyl]-1H-pyrazolo[3,4-d]pyrimidin-6-yl}amino)pyrrolidin-2-one |
| I-29 | (1 R,3S)-3-({4-[4-(2-hydroxypropan-2-yl)piperidin-1-yl]-1-[3-(trifluoromethyl)phenyl]-1H-pyrazolo[3,4-d]pyrimidin-6-yl}amino)cyclopentanol |
| I-30 | (4S)-4-({4-[3-(4-hydroxy-4-methylpiperidin-1-yl)azetidin-1-yl]-1-[3-(trifluoromethyl)phenyl]-1H-pyrazolo[3,4-d]pyrimidin-6-yl}amino)pyrrolidin-2-one |
| I-31 | (3R,4R)-1-(6-{[(3S)-1-methylpyrrolidin-3-yl]amino}-1-[3-(trifluoromethyl)phenyl]-1H-pyrazolo[3,4-d]pyrimidin-4-yl)pyrrolidine-3,4-diol |
| I-32 | (4S)-4-({4-(3-hydroxyazetidin-1-yl)-1-[3-(trifluoromethyl)phenyl]-1H-pyrazolo[3,4-d]pyrimidin-6-yl}amino)pyrrolidin-2-one |
| I-33 | N-[(3S)-1,1-dioxidotetrahydrothiophen-3-yl]-4-[4-(morpholin-4-yl)piperidin-1-yl]-1-[3-(trifluoromethyl)phenyl]-1H-pyrazolo[3,4-d]pyrimidin-6-amine |
| I-34 | N-[(3S)-1,1-dioxidotetrahydrothiophen-3-yl]-4-[3-(morpholin-4-yl)azetidin-1-yl]-1-[3-(trifluoromethyl)phenyl]-1H-pyrazolo[3,4-d]pyrimidin-6-amine |
| I-35 | (4S)-4-[(4-{3-[(methylsulfonyl)methyl]azetidin-1-yl}-1-[3-(trifluoromethyl)phenyl]-1H-pyrazolo[3,4-d]pyrimidin-6-yl)amino]pyrrolidin-2-one |
| I-36 | N-[(3S)-1-methylpyrrolidin-3-yl]-4-{3-[(methylsulfonyl)methyl]azetidin-1-yl}-1-[3-(trifluoromethyl)phenyl]-1H-pyrazolo[3,4-d]pyrimidin-6-amine |
| I-37 | rel-(3R,4R)-4-({4-[3-(morpholin-4-yl)azetidin-1-yl]-1-[3-(trifluoromethyl)phenyl]-1H-pyrazolo[3,4-d]pyrimidin-6-yl}amino)tetrahydrothiophene-3-ol 1,1-dioxide |
| I-38 | (4S)-4-({4-[3-(dimethylamino)-3-methylazetidin-1-yl]-1-[3-(trifluoromethyl)phenyl]-1H-pyrazolo[3,4-d]pyrimidin-6-yl}amino)pyrrolidin-2-one |
| I-39 | 4-[3-(dimethylamino)-3-methylazetidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine |
| I-40 | 2-[1-(6-{[(3S,5S)-5-methylpyrrolidin-3-yl]amino}-1-[3-(trifluoromethyl)phenyl]-1H-pyrazolo[3,4-d]pyrimidin-4-yl)piperidin-4-yl]propan-2-ol |
| I-41-1 | 3-methyl-1-(6-{[(3R)-1-(methylsulfonyl)pyrrolidin-3-yl]amino}-1-[3-(trifluoromethyl)phenyl]-1H-pyrazolo[3,4-d]pyrimidin-4-yl)azetidin-3-ol |
| I-41-2 | 3-methyl-1-(6-{[(3S)-1-(methylsulfonyl)pyrrolidin-3-yl]amino}-1-[3-(trifluoromethyl)phenyl]-1H-pyrazolo[3,4-d]pyrimidin-4-yl)azetidin-3-ol |
| I-42 | 4-[3-(dimethylamino)azetidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine |
| I-43 | N-(3-methyl-1,1-dioxidotetrahydrothiophen-3-yl)-4-[3-(morpholin-4-yl)azetidin-1-yl]-1-[3-(trifluoromethyl)phenyl]-1H-pyrazolo[3,4-d]pyrimidin-6-amine |
| I-44 | (4S)-4-({4-[3-(dimethylamino)azetidin-1-yl]-1-[3-(trifluoromethyl)phenyl]-1H-pyrazolo[3,4-d]pyrimidin-6-yl}amino)pyrrolidin-2-one |
| I-45 | 4-[3-(2-aminopropan-2-yl)azetidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine |
| I-46 | 4-[4-(2-aminopropan-2-yl)piperidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine |
| I-47 | (4S)-4-({4-[4-(2-aminopropan-2-yl)piperidin-1-yl]-1-[3-(trifluoromethyl)phenyl]-1H-pyrazolo[3,4-d]pyrimidin-6-yl}amino)pyrrolidin-2-one |
| I-48 | (4S)-4-({4-[4-(1-aminocyclopropyl)piperidin-1-yl]-1-[3-(trifluoromethyl)phenyl]-1H-pyrazolo[3,4-d]pyrimidin-6-yl}amino)pyrrolidin-2-one |
| I-49 | (4S)-4-({4-[3-(2-hydroxy-2-methylpropyl)azetidin-1-yl]-1-[3-(trifluoromethyl)phenyl]-1H-pyrazolo[3,4-d]pyrimidin-6-yl}amino)pyrrolidin-2-one |
| I-50 | [1-(6-{[(3S)-5-oxopyrrolidin-3-yl]amino}-1-[3-(trifluoromethyl)phenyl]-1H-pyrazolo[3,4-d]pyrimidin-4-yl)azetidin-3-yl]acetic acid |
| I-51 | (4S)-4-[(4-{3-[2-(morpholin-4-yl)-2-oxoethyl]azetidin-1-yl}-1-[3-(trifluoromethyl)phenyl]-1H-pyrazolo[3,4-d]pyrimidin-6-yl)amino]pyrrolidin-2-one |
| I-52 | (4S)-4-[(4-{3-[2-(6-oxa-3-azabicyclo[3.1.1]heptan-3-yl)-2-oxoethyl]azetidin-1-yl}-1-[3-(trifluoromethyl)phenyl]-1H-pyrazolo[3,4-d]pyrimidin-6-yl)amino]pyrrolidin-2-one |
| I-53 | N-cyclopropyl-4-[3-(1,1-dioxidothiomorpholin-4-yl)azetidin-1-yl]-1-[3-(trifluoromethyl)phenyl]-1H-pyrazolo[3,4-d]pyrimidin-6-amine |
| I-54 | N-(3,3-difluorocyclobutyl)-4-[3-(dimethylamino)azetidin-1-yl]-1-[3-(trifluoromethyl)phenyl]-1H-pyrazolo[3,4-d]pyrimidin-6-amine |
| I-55 | N-[cis-3-(methylsulfonyl)cyclobutyl]-4-[3-(morpholin-4-yl)azetidin-1-yl]-1-[3-(trifluoromethyl)phenyl]-1H-pyrazolo[3,4-d]pyrimidin-6-amine |
| I-56 | N-[trans-3-(methylsulfonyl)cyclobutyl]-4-[3-(morpholin-4-yl)azetidin-1-yl]-1-[3-(trifluoromethyl)phenyl]-1H-pyrazolo[3,4-d]pyrimidin-6-amine |
| I-57 | 2-(1-{6-[(trans-3-amino-3-methylcyclobutyl)amino]-1-[3-(trifluoromethyl)phenyl]-1H-pyrazolo[3,4-d]pyrimidin-4-yl}piperidin-4-yl)propan-2-ol |
| I-58 | cis-1-methyl-N³-{4-(morpholin-4-yl)-1-[3-(trifluoromethyl)phenyl]-1H-pyrazolo[3,4-d]pyrimidin-6-yl}cyclobutane-1,3-diamine |
| I-59 | 4-[3-methyl-3-(morpholin-4-yl)azetidin-1-yl]-N-[trans-3-(methylsulfonyl)cyclobutyl]-1-[3-(trifluoromethyl)phenyl]-1H-pyrazolo[3,4-d]pyrimidin-6-amine |
| I-60 | N-(1,1-dioxidotetrahydro-2H-thiopyran-4-yl)-4-[3-(morpholin-4-ylmethyl)azetidin-1-yl]-1-[3-(trifluoromethyl)phenyl]-1H-pyrazolo[3,4-d]pyrimidin-6-amine |
| I-61 | 4-methyl-1-[1-(6-{[trans-3-(methylsulfonyl)cyclobutyl]amino}-1-[3-(trifluoromethyl)phenyl]-1H-pyrazolo[3,4-d]pyrimidin-4-yl)azetidin-3-yl]piperidin-4-ol |
| I-62 | 4-[4-(1-aminocyclopropyl)piperidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine |
| I-63 | trans-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-(trifluoromethyl)cyclobutanol |
| I-64-1 | 2-(1-{6-[(3R)-1-azabicyclo[2.2.2]octan-3-ylamino]-1-[3-(trifluoromethyl)phenyl]-1H-pyrazolo[3,4-d]pyrimidin-4-yl}piperidin-4-yl)propan-2-ol |
| I-64-2 | 2-(1-{6-[(3S)-1-azabicyclo[2.2.2]octan-3-ylamino]-1-[3-(trifluoromethyl)phenyl]-1H-pyrazolo[3,4-d]pyrimidin-4-yl}piperidin-4-yl)propan-2-ol |
| I-65 | 2-[1-(6-{[rel-(3R,4R)-4-fluoropyrrolidin-3-yl]amino}-1-[3-(trifluoromethyl)phenyl]-1H-pyrazolo[3,4-d]pyrimidin-4-yl)piperidin-4-yl]propan-2-ol |
| I-66 | cis-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylcyclobutanecarboxylic acid |
| I-67 | 2-methyl-1-[1-(6-{[trans-3-(methylsulfonyl)cyclobutyl]amino}-1-[3-(trifluoromethyl)phenyl]-1H-pyrazolo[3,4-d]pyrimidin-4-yl)azetidin-3-yl]propan-2-ol |
| I-68 | 2-methyl-1-[1-(6-{[1-(methylsulfonyl)azetidin-3-yl]amino}-1-[3-(trifluoromethyl)phenyl]-1H-pyrazolo[3,4-d]pyrimidin-4-yl)azetidin-3-yl]propan-2-ol |
| I-69 | (3R,4S)-4-({4-[3-(morpholin-4-yl)azetidin-1-yl]-1-[3-(trifluoromethyl)phenyl]-1H-pyrazolo[3,4-d]pyrimidin-6-yl}amino)tetrahydrofuran-3-ol |
| I-70 | rel-(3R,4R)-4-({4-[3-(morpholin-4-yl)azetidin-1-yl]-1-[3-(trifluoromethyl)phenyl]-1H-pyrazolo[3,4-d]pyrimidin-6-yl}amino)tetrahydrofuran-3-ol |
| I-71 | trans-1 -methyl-N³-{4-[3-(methylsulfonyl)azetidin-1-yl]-1-[3-(trifluoromethyl)phenyl]-1H-pyrazolo[3,4-d]pyrimidin-6-yl}cyclobutane-1,3-diamine |
| I-72 | cis-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-73 | rel-(1 R,2S)-2-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-74 | trans-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-75 | rel-(1R,2R)-2-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-76 | cis-3-({4-[3-methyl-3-(morpholin-4-yl)azetidin-1-yl]-1-[3-(trifluoromethyl)phenyl]-1H-pyrazolo[3,4-d]pyrimidin-6-yl}amino)cyclobutanol |
| I-77 | trans-1-methyl-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-78 | cis-1-methyl-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-79 | 1-(1-{6-[(trans-3-hydroxycyclobutyl)amino]-1-[3-(trifluoromethyl)phenyl]-1H-pyrazolo[3,4-d]pyrimidin-4-yl}azetidin-3-yl)-4-methylpiperidin-4-ol |
| I-80 | 1-(1-{6-[(1,1-dioxidotetrahydro-2H-thiopyran-4-yl)amino]-1-[3-(trifluoromethyl)phenyl]-1H-pyrazolo[3,4-d]pyrimidin-4-yl}azetidin-3-yl)-4-methylpiperidin-4-ol |
| I-81 | N-[1-(ethylsulfonyl)azetidin-3-yl]-4-[3-(morpholin-4-yl)azetidin-1-yl]-1-[3-(trifluoromethyl)phenyl]-1H-pyrazolo[3,4-d]pyrimidin-6-amine |
| I-82 | N-[trans-3-(ethylsulfonyl)cyclobutyl]-4-[3-(morpholin-4-yl)azetidin-1-yl]-1-[3-(trifluoromethyl)phenyl]-1H-pyrazolo[3,4-d]pyrimidin-6-amine |
| I-83 | 2-[1-(6-{[trans-3-(methylsulfonyl)cyclobutyl]amino}-1-[3-(trifluoromethyl)phenyl]-1H-pyrazolo[3,4-d]pyrimidin-4-yl)azetidin-3-yl]propan-2-ol |
| I-84 | (3R,4R)-1-(6-{[1-(ethylsulfonyl)azetidin-3-yl]amino}-1-[3-(trifluoromethyl)phenyl]-1H-pyrazolo[3,4-d]pyrim idin-4-yl)pyrrolidine-3,4-diol |
| I-85 | (3R,4R)-1-(6-1[(1s,3S)-3-hydroxycyclobutyl]amino}-1-[3-(trifluoromethyl)phenyl]-1H-pyrazolo[3,4-d]pyrimidin-4-yl)pyrrolidine-3,4-diol |
| I-86 | (3R,4R)-4-fluoro-1-(6-{[(1r,3R)-3-(methylsulfonyl)cyclobutyl]amino}-1-[3-(trifluoromethyl)phenyl]-1H-pyrazolo[3,4-d]pyrimidin-4-yl)pyrrolidin-3-ol |
| I-87 | (3R,4R)-1-(6-{[(1r,3R)-3-(ethylsulfonyl)cyclobutyl]amino}-1-[3-(trifluoromethyl)phenyl]-1H-pyrazolo[3,4-d]pyrimidin-4-yl)-4-fluoropyrrolidin-3-ol |
| I-88 | (3R,4R)-4-fluoro-1-(6-{[(1r,3R)-3-hydroxycyclobutyl]amino}-1-[3-(trifluoromethyl)phenyl]-1H-pyrazolo[3,4-d]pyrimidin-4-yl)pyrrolidin-3-ol |
| I-89 | (3R,4R)-4-fluoro-1-(6-{[(1s,3S)-3-hydroxycyclobutyl]amino}-1-[3-(trifluoromethyl)phenyl]-1H-pyrazolo[3,4-d]pyrimidin-4-yl)pyrrolidin-3-ol |
| I-90 | cis-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylcyclobutanol |
| I-91 | trans-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylcyclobutanol |
| I-92 | 3-ethyl-1-(6-{[trans-3-(methylsulfonyl)cyclobutyl]amino}-1-[3-(trifluoromethyl)phenyl]-1H-pyrazolo[3,4-d]pyrimidin-4-yl)azetidin-3-ol |
| I-93 | 1-(6-{[trans-3-(methylsulfonyl)cyclobutyl]amino}-1-[3-(trifluoromethyl)phenyl]-1 H-pyrazolo[3,4-d]pyrimidin-4-yl)piperidin-4-ol |
| I-94 | (3S,4S)-4-fluoro-1-(6-{[(1r,3S)-3-(methylsulfonyl)cyclobutyl]amino}-1-[3-(trifluoromethyl)phenyl]-1H-pyrazolo[3,4-d]pyrimidin-4-yl)pyrrolidin-3-ol |
| I-95 | (3S,4S)-1-(6-{[(1r,3S)-3-(ethylsulfonyl)cyclobutyl]amino}-1-[3-(trifluoromethyl)phenyl]-1H-pyrazolo[3,4-d]pyrimidin-4-yl)-4-fluoropyrrolidin-3-ol |
| I-96 | (3S,4S)-4-fluoro-1-(6-{[(1r,3S)-3-hydroxycyclobutyl]amino}-1-[3-(trifluoromethyl)phenyl]-1H-pyrazolo[3,4-d]pyrimidin-4-yl)pyrrolidin-3-ol |
| I-97 | (3S,4S)-4-fluoro-1-(6-{[(1s,3R)-3-hydroxycyclobutyl]amino}-1-[3-(trifluoromethyl)phenyl]-1H-pyrazolo[3,4-d]pyrimidin-4-yl)pyrrolidin-3-ol |
| I-98 | (3S,4S)-1-{6-[(4S,6s)-1-azaspiro[3.3]heptan-6-ylamino]-1-[3-(trifluoromethyl)phenyl]-1H-pyrazolo[3,4-d]pyrimidin-4-yl}-4-fluoropyrrolidin-3-ol |
| I-99 | 4-(3-fluoroazetidin-1-yl)-N-[trans-3-(methylsulfonyl)cyclobutyl]-1-[3-(trifluoromethyl)phenyl]-1H-pyrazolo[3,4-d]pyrimidin-6-amine |
| I-100 | 1-{6-[(trans-3-hydroxycyclobutyl)amino]-1-[3-(trifluoromethyl)phenyl]-1H-pyrazolo[3,4-d]pyrimidin-4-yl}piperidin-4-ol |
| I-101 | (3R,4R)-1-(6-{[(1r,3R)-3-hydroxycyclobutyl]amino}-1-[3-(trifluoromethyl)phenyl]-1H-pyrazolo[3,4-d]pyrim idin-4-yl)pyrrolidine-3,4-diol |
| I-102 | (3R)-1-(6-{[(1r,3R)-3-hydroxycyclobutyl]amino}-1-[3-(trifluoromethyl)phenyl]-1H-pyrazolo[3,4-d]pyrimidin-4-yl)-3-methylpyrrolidin-3-ol |
| I-103 | (3R)-3-methyl-1-(6-{[(1r,3R)-3-(methylsulfonyl)cyclobutyl]amino}-1-[3-(trifluoromethyl)phenyl]-1H-pyrazolo[3,4-d]pyrimidin-4-yl)pyrrolidin-3-ol |
| I-104 | trans-3-({4-[(4RS)-4-amino-3,3-difluoropiperidin-1-yl]-1-[3-(trifluoromethyl)phenyl]-1H-pyrazolo[3,4-d]pyrimidin-6-yl}amino)cyclobutanol |
| I-105 | 4-[(4RS)-4-amino-3,3-difluoropiperidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine |
| I-106 | N-(1,1-dioxidotetrahydro-2H-thiopyran-4-yl)-4-[3-methyl-3-(morpholin-4-yl)azetidin-1-yl]-1-[3-(trifluoromethyl)phenyl]-1H-pyrazolo[3,4-d]pyrimidin-6-amine |
| I-107 | (3R,4R)-1-(6-{[(1s,3S)-3-hydroxy-3-methylcyclobutyl]amino}-1-[3-(trifluoromethyl)phenyl]-1H-pyrazolo[3,4-d]pyrimidin-4-yl)pyrrolidine-3,4-diol |
| I-108 | (3R,4R)-1-(6-{[(1r,3R)-3-hydroxy-3-methylcyclobutyl]amino}-1-[3-(trifluoromethyl)phenyl]-1H-pyrazolo[3,4-d]pyrimidin-4-yl)pyrrolidine-3,4-diol |
| I-109 | 1-(1-{6-[(cis-3-hydroxycyclobutyl)amino]-1-[3-(trifluoromethyl)phenyl]-1H-pyrazolo[3,4-d]pyrimidin-4-yl}azetidin-3-yl)-4-methylpiperidin-4-ol |
| I-110 | 1-(1-{6-[(trans-3-hydroxy-3-methylcyclobutyl)amino]-1-[3-(trifluoromethyl)phenyl]-1H-pyrazolo[3,4-d]pyrimidin-4-yl}azetidin-3-yl)-4-methylpiperidin-4-ol |
| I-111 | 1-(1-{6-[(cis-3-hydroxy-3-methylcyclobutyl)amino]-1-[3-(trifluoromethyl)phenyl]-1H-pyrazolo[3,4-d]pyrimidin-4-yl}azetidin-3-yl)-4-methylpiperidin-4-ol |
| I-112 | 1-(1-{6-[(cis-3-hydroxycyclobutyl)amino]-1-[3-(trifluoromethyl)phenyl]-1H-pyrazolo[3,4-d]pyrimidin-4-yl}azetidin-3-yl)pyrrolidin-2-one |
| I-113 | 1-(1-{6-[(trans-3-hydroxycyclobutyl)amino]-1-[3-(trifluoromethyl)phenyl]-1H-pyrazolo[3,4-d]pyrimidin-4-yl}azetidin-3-yl)pyrrolidin-2-one |
| I-114 | 1-{6-[(cis-3-hydroxycyclobutyl)amino]-1-[3-(trifluoromethyl)phenyl]-1H-pyrazolo[3,4-d]pyrimidin-4-yl}-4-(pyrrolidin-1-ylmethyl)piperidin-4-ol |
| I-115 | 4-(1-{6-[(cis-3-hydroxycyclobutyl)amino]-1-[3-(trifluoromethyl)phenyl]-1H-pyrazolo[3,4-d]pyrimidin-4-yl}azetidin-3-yl)piperazin-2-one |
| I-116 | cis-3-({4-[3-(2-hydroxypropan-2-yl)azetidin-1-yl]-1-[3-(trifluoromethyl)phenyl]-1H-pyrazolo[3,4-d]pyrimidin-6-yl}amino)cyclobutanol |
| I-117 | (3R)-1-(6-{[(1s,3S)-3-hydroxycyclobutyl]amino}-1-[3-(trifluoromethyl)phenyl]-1H-pyrazolo[3,4-d]pyrimidin-4-yl)pyrrolidin-3-ol |
| I-118 | cis-3-({4-[3-(3,3-difluoropyrrolidin-1-yl)azetidin-1-yl]-1-[3-(trifluoromethyl)phenyl]-1H-pyrazolo[3,4-d]pyrimidin-6-yl}amino)cyclobutanol |
| I-119 | trans-3-({4-(3-fluoro[1,3'-biazetidin]-1'-yl)-1-[3-(trifluoromethyl)phenyl]-1H-pyrazolo[3,4-d]pyrimidin-6-yl}amino)cyclobutanol |
| I-120 | trans-3-({4-(3-fluoro[1,3'-biazetidin]-1'-yl)-1-[3-(trifluoromethyl)phenyl]-1H-pyrazolo[3,4-d]pyrimidin-6-yl}amino)-1-methylcyclobutanol |
| I-121 | 4-(1-{6-[(cis-3-hydroxycyclobutyl)amino]-1-[3-(trifluoromethyl)phenyl]-1H-pyrazolo[3,4-d]pyrimidin-4-yl}azetidin-3-yl)morpholin-3-one |
| I-122 | 4-(1-{6-[(trans-3-hydroxy-3-methylcyclobutyl)amino]-1-[3-(trifluoromethyl)phenyl]-1H-pyrazolo[3,4-d]pyrimidin-4-yl}azetidin-3-yl)morpholin-3-one |
| I-123 | trans-1-methyl-3-({4-[3-methyl-3-(morpholin-4-yl)azetidin-1-yl]-1-[3-(trifluoromethyl)phenyl]-1H-pyrazolo[3,4-d]pyrimidin-6-yl}amino)cyclobutanol |
| I-124 | (3S)-1-(6-{[(1s,3R)-3-hydroxycyclobutyl]amino}-1-[3-(trifluoromethyl)phenyl]-1H-pyrazolo[3,4-d]pyrimidin-4-yl)-3-methylpyrrolidin-3-ol |
| II-1 | (4S)-4-({1-[3-(difluoromethyl)phenyl]-4-[3-(morpholin-4-yl)azetidin-1-yl]-1H-pyrazolo[3,4-d]pyrimidin-6-yl}amino)pyrrolidin-2-one |
| II-2 | 1-[3-(difluoromethyl)phenyl]-N-(1,1-dioxidotetrahydro-2H-thiopyran-4-yl)-4-[3-(morpholin-4-yl)azetidin-1-yl]-1H-pyrazolo[3,4-d]pyrimidin-6-amine |
| II-3 | 1-[3-(difluoromethyl)phenyl]-N-[(3S)-1,1-dioxidotetrahydrothiophen-3-yl]-4-[3-(morpholin-4-yl)azetidin-1-yl]-1H-pyrazolo[3,4-d]pyrimidin-6-amine |
| II-4 | 2-[1-(1-[3-(difluoromethyl)phenyl]-6-{[(3S)-1-methylpyrrolidin-3-yl]amino}-1H-pyrazolo[3,4-d]pyrimidin-4-yl)piperidin-4-yl]propan-2-ol |
| II-5 | 2-(1-{1-[3-(difluoromethyl)phenyl]-6-[(1,1-dioxidotetrahydro-2H-thiopyran-4-yl)amino]-1H-pyrazolo[3,4-d]pyrimidin-4-yl}piperidin-4-yl)propan-2-ol |
| II-6 | 2-[1-(1-[3-(difluoromethyl)phenyl]-6-{[(3S)-1,1-dioxidotetrahydrothiophen-3-yl]amino}-1H-pyrazolo[3,4-d]pyrimidin-4-yl)piperidin-4-yl]propan-2-ol |
| II-7 | 1-(1-{1-[3-(difluoromethyl)phenyl]-6-[(1,1-dioxidotetrahydro-2H-thiopyran-4-yl)amino]-1H-pyrazolo[3,4-d]pyrimidin-4-yl}azetidin-3-yl)-2-methylpropan-2-ol |
| II-8 | 1-[3-(difluoromethyl)phenyl]-N-[trans-3-(methylsulfonyl)cyclobutyl]-4-[3-(morpholin-4-yl)azetidin-1-yl]-1H-pyrazolo[3,4-d]pyrimidin-6-amine |
| II-9 | (1-{1-[3-(difluoromethyl)phenyl]-6-[(1,1-dioxidotetrahydro-2H-thiopyran-4-yl)amino]-1H-pyrazolo[3,4-d]pyrimidin-4-yl}azetidin-3-yl)acetic acid |
| II-10 | 2-(1-{1-[3-(difluoromethyl)phenyl]-6-[(1,1-dioxidotetrahydro-2H-thiopyran-4-yl)amino]-1H-pyrazolo[3,4-d]pyrimidin-4-yl}azetidin-3-yl)-1-(morpholin-4-yl)ethanone |
| II-11 | 2-[1-(1-[3-(difluoromethyl)phenyl]-6-{[trans-3-(methylsulfonyl)cyclobutyl]amino}-1H-pyrazolo[3,4-d]pyrimidin-4-yl)piperidin-4-yl]propan-2-ol |
| II-12 | 2-[1-(1-[3-(difluoromethyl)phenyl]-6-{[1-(methylsulfonyl)azetidin-3-yl]amino}-1H-pyrazolo[3,4-d]pyrimidin-4-yl)piperidin-4-yl]propan-2-ol |
| II-13 | 1-[3-(difluoromethyl)phenyl]-N-[1-(methylsulfonyl)azetidin-3-yl]-4-[3-(morpholin-4-yl)azetidin-1-yl]-1H-pyrazolo[3,4-d]pyrimidin-6-amine |
| II-14 | 1-[1-(1-[3-(difluoromethyl)phenyl]-6-{[1-(methylsulfonyl)azetidin-3-yl]amino}-1H-pyrazolo[3,4-d]pyrimidin-4-yl)azetidin-3-yl]-2-methylpropan-2-ol |
| II-15 | 1-{1-[3-(difluoromethyl)phenyl]-6-[(1,1-dioxidotetrahydro-2H-thiopyran-4-yl)amino]-1H-pyrazolo[3,4-d]pyrimidin-4-yl}-3-(trifluoromethyl)pyrrolidin-3-ol |
| II-16-1 | rel-(3R,4R)-1-(1-[3-(difluoromethyl)phenyl]-6-{[trans-3-(methylsulfonyl)cyclobutyl]amino}-1H-pyrazolo[3,4-d]pyrimidin-4-yl)-4-fluoropyrrolidin-3-ol |
| II-16-2 | (3S,4S)-1-(1-[3-(difluoromethyl)phenyl]-6-{[trans-3-(methanesulfonyl)cyclobutyl]amino}-1H-pyrazolo[3,4-d]pyrimidin-4-yl)-4-fluoropyrrolidin-3-ol |
| II-16-3 | (3R,4R)-1-(1-[3-(difluoromethyl)phenyl]-6-{[trans-3-(methanesulfonyl)cyclobutyl]amino}-1H-pyrazolo[3,4-d]pyrimidin-4-yl)-4-fluoropyrrolidin-3-ol |
| II-17-1 | rel-(3R,4S)-1-(1-[3-(difluoromethyl)phenyl]-6-{[trans-3-(methylsulfonyl)cyclobutyl]amino}-1H-pyrazolo[3,4-d]pyrimidin-4-yl)-4-methylpyrrolidin-3-ol |
| II-17-2 | (3R*,4S*)-1-(1-[3-(difluoromethyl)phenyl]-6-{[trans-3-(methylsulfonyl)cyclobutyl]amino}-1H-pyrazolo[3,4-d]pyrimidin-4-yl)-4-methylpyrrolidin-3-ol (single enantiomer 1) |
| II-17-3 | (3R*,4S*)-1-(1-[3-(difluoromethyl)phenyl]-6-{[trans-3-(methylsulfonyl)cyclobutyl]amino}-1H-pyrazolo[3,4-d]pyrimidin-4-yl)-4-methylpyrrolidin-3-ol (single enantiomer 2) |
| II-18 | 2-(1-16-(*trans*-1-azaspiro[3.3]heptan-6-ylamino)-1-[3-(difluoromethyl)phenyl]-1H-pyrazolo[3,4-d]pyrimidin-4-yl}piperidin-4-yl)propan-2-ol |
| II-19 | 1-[1-(1-[3-(difluoromethyl)phenyl]-6-{[trans-3-(methylsulfonyl)cyclobutyl]amino}-1 H-pyrazolo[3,4-d]pyrimidin-4-yl)azetidin-3-yl]-2-methylpropan-2-ol |
| II-20 | 4-[4-(1-aminocyclopropyl)piperidin-1-yl]-1-[3-(difluoromethyl)phenyl]-N-[1-(methylsulfonyl)azetidin-3-yl]-1H-pyrazolo[3,4-d]pyrimidin-6-amine |
| II-21 | 1-[3-(difluoromethyl)phenyl]-4-[3-methyl-3-(morpholin-4-yl)azetidin-1-yl]-N-[trans-3-(methylsulfonyl)cyclobutyl]-1 H-pyrazolo[3,4-d]pyrimidin-6-amine |
| II-22 | (3S,4S)-1-(1-[3-(difluoromethyl)phenyl]-6-{[1-(methylsulfonyl)azetidin-3-yl]amino}-1H-pyrazolo[3,4-d]pyrimidin-4-yl)-4-fluoropyrrolidin-3-ol |
| II-23 | (3R,4R)-1-(1-[3-(difluoromethyl)phenyl]-6-{[1-(methylsulfonyl)azetidin-3-yl]amino}-1H-pyrazolo[3,4-d]pyrimidin-4-yl)-4-fluoropyrrolidin-3-ol |
| II-24 | cis-3-({1-[3-(difluoromethyl)phenyl]-4-[3-(morpholin-4-yl)azetidin-1-yl]-1H-pyrazolo[3,4-d]pyrimidin-6-yl}amino)cyclobutanol |
| II-25 | trans-3-({1-[3-(difluoromethyl)phenyl]-4-[3-(morpholin-4-yl)azetidin-1-yl]-1H-pyrazolo[3,4-d]pyrimidin-6-yl}amino)cyclobutanol |
| II-26 | 2-(1-{1-[3-(difluoromethyl)phenyl]-6-[(1,1-dioxidotetrahydro-2H-thiopyran-4-yl)amino]-1H-pyrazolo[3,4-d]pyrimidin-4-yl}azetidin-3-yl)-2-methylpropanoic acid |
| II-27 | 4-(1-{1-[3-(difluoromethyl)phenyl]-6-[(trans-3-hydroxycyclobutyl)amino]-1H-pyrazolo[3,4-d]pyrimidin-4-yl}azetidin-3-yl)piperazin-2-one |
| II-28 | 1-[1-(1-[3-(difluoromethyl)phenyl]-6-{[1-(ethylsulfonyl)azetidin-3-yl]amino}-1H-pyrazolo[3,4-d]pyrimidin-4-yl)azetidin-3-yl]-2-methylpropan-2-ol |
| II-29 | (3R)-1-(1-[3-(difluoromethyl)phenyl]-6-{[1-(methylsulfonyl)azetidin-3-yl]amino}-1H-pyrazolo[3,4-d]pyrimidin-4-yl)-3-methylpyrrolidin-3-ol |
| II-30 | 2-[1-(6-{[1-(cyclopropylsulfonyl)azetidin-3-yl]amino}-1-[3-(difluoromethyl)phenyl]-1H-pyrazolo[3,4-d]pyrimidin-4-yl)piperidin-4-yl]propan-2-ol |
| II-31 | 2-[1-(1-[3-(difluoromethyl)phenyl]-6-{[1-(ethylsulfonyl)azetidin-3-yl]amino}-1H-pyrazolo[3,4-d]pyrimidin-4-yl)piperidin-4-yl]propan-2-ol |
| II-32 | 1-[3-(difluoromethyl)phenyl]-N-[1-(ethylsulfonyl)azetidin-3-yl]-4-[3-(morpholin-4-yl)azetidin-1-yl]-1H-pyrazolo[3,4-d]pyrimidin-6-amine |
| II-33 | (3R)-1-(1-[3-(difluoromethyl)phenyl]-6-{[(1r,3R)-3-hydroxycyclobutyl]amino}-1H-pyrazolo[3,4-d]pyrimidin-4-yl)pyrrolidin-3-ol |
| II-34 | (3R)-1-(1-[3-(difluoromethyl)phenyl]-6-{[(1r,3R)-3-(methylsulfonyl)cyclobutyl]amino}-1H-pyrazolo[3,4-d]pyrimidin-4-yl)pyrrolidin-3-ol |
| II-35 | (3R)-1-(1-[3-(difluoromethyl)phenyl]-6-{[(1r,3R)-3-(methylsulfonyl)cyclobutyl]amino}-1H-pyrazolo[3,4-d]pyrimidin-4-yl)-3-methylpyrrolidin-3-ol |
| II-36 | 1-(1-{6-[(4r,6s)-1-azaspiro[3.3]heptan-6-ylamino]-1-[3-(difluoromethyl)phenyl]-1H-pyrazolo[3,4-d]pyrimidin-4-yl}azetidin-3-yl)-2-methylpropan-2-ol |
| III-1 | (4S)-4-({4-[4-(2-hydroxypropan-2-yl)piperidin-1-yl]-1-[3-(trifluoromethoxy)phenyl]-1H-pyrazolo[3,4-d]pyrimidin-6-yl}amino)pyrrolidin-2-one |
| IV-1 | (4S)-4-({1-[3-(difluoromethoxy)phenyl]-4-[4-(2-hydroxypropan-2-yl)piperidin-1-yl]-1H-pyrazolo[3,4-d]pyrimidin-6-yl}amino)pyrrolidin-2-one |
| IV-2 | 2-[1-(1-[3-(difluoromethoxy)phenyl]-6-{[trans-3-(methylsulfonyl)cyclobutyl]amino}-1H-pyrazolo[3,4-d]pyrimidin-4-yl)piperidin-4-yl]propan-2-ol |
| IV-3 | 4-[4-(1-aminocyclopropyl)piperidin-1-yl]-1-[3-(difluoromethoxy)phenyl]-N-[1-(methylsulfonyl)azetidin-3-yl]-1H-pyrazolo[3,4-d]pyrimidin-6-amine |
| IV-4 | 1-[1-(1-[3-(difluoromethoxy)phenyl]-6-{[trans-3-(methylsulfonyl)cyclobutyl]amino}-1H-pyrazolo[3,4-d]pyrimidin-4-yl)azetidin-3-yl]-2-methylpropan-2-ol |
| IV-5 | (3S,4S)-1-(1-[3-(difluoromethoxy)phenyl]-6-{[trans-3-(methylsulfonyl)cyclobutyl]amino}-1H-pyrazolo[3,4-d]pyrimidin-4-yl)-4-fluoropyrrolidin-3-ol |
| IV-6 | (3S,4S)-1-(1-[3-(difluoromethoxy)phenyl]-6-{[1-(methylsulfonyl)azetidin-3-yl]amino}-1H-pyrazolo[3,4-d]pyrimidin-4-yl)-4-fluoropyrrolidin-3-ol |
| IV-7 | (3S,4S)-1-{1-[3-(difluoromethoxy)phenyl]-6-[(1,1-dioxidotetrahydro-2H-thiopyran-4-yl)amino]-1H-pyrazolo[3,4-d]pyrimidin-4-yl}-4-fluoropyrrolidin-3-ol |
| V-1 | 3-ethyl-1-(6-{[(3S)-1-methylpyrrolidin-3-yl]amino}-1-[3-(methylsulfonyl)phenyl]-1H-pyrazolo[3,4-d]pyrimidin-4-yl)azetidin-3-ol |
| V-2 | 3-ethyl-1-{1-[3-(methylsulfonyl)phenyl]-6-[(3R)-tetrahydrofuran-3-ylamino]-1H-pyrazolo[3,4-d]pyrimidin-4-yl}azetidin-3-ol |
| VI-1 | 2-[1-(1-[3-(2-hydroxypropan-2-yl)phenyl]-6-{[(3S)-1-methylpyrrolidin-3-yl]amino}-1H-pyrazolo[3,4-d]pyrimidin-4-yl)piperidin-4-yl]propan-2-ol |

The following compounds are preferred, namely:

| | |
|---|---|
| I-72 | cis-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-74 | trans-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-76 | cis-3-({4-[3-methyl-3-(morpholin-4-yl)azetidin-1-yl]-1-[3-(trifluoromethyl)phenyl]-1H-pyrazolo[3,4-d]pyrimidin-6-yl}amino)cyclobutanol |
| I-77 | trans-1-methyl-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-78 | cis-1-methyl-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-79 | 1-(1-{6-[(trans-3-hydroxycyclobutyl)amino]-1-[3-(trifluoromethyl)phenyl]-1H-pyrazolo[3,4-d]pyrimidin-4-yl}azetidin-3-yl)-4-methylpiperidin-4-ol |
| I-109 | 1-(1-{6-[(cis-3-hydroxycyclobutyl)amino]-1-[3-(trifluoromethyl)phenyl]-1H-pyrazolo[3,4-d]pyrimidin-4-yl}azetidin-3-yl)-4-methylpiperidin-4-ol |
| I-110 | 1-(1-{6-[(trans-3-hydroxy-3-methylcyclobutyl)amino]-1-[3-(trifluoromethyl)phenyl]-1H-pyrazolo[3,4-d]pyrimidin-4-yl}azetidin-3-yl)-4-methylpiperidin-4-ol |
| I-111 | 1-(1-{6-[(cis-3-hydroxy-3-methylcyclobutyl)amino]-1-[3-(trifluoromethyl)phenyl]-1H-pyrazolo[3,4-d]pyrimidin-4-yl}azetidin-3-yl)-4-methylpiperidin-4-ol |
| I-112 | 1-(1-{6-[(cis-3-hydroxycyclobutyl)amino]-1-[3-(trifluoromethyl)phenyl]-1H-pyrazolo[3,4-d]pyrimidin-4-yl}azetidin-3-yl)pyrrolidin-2-one |
| I-113 | 1-(1-{6-[(trans-3-hydroxycyclobutyl)amino]-1-[3-(trifluoromethyl)phenyl]-1H-pyrazolo[3,4-d]pyrimidin-4-yl}azetidin-3-yl)pyrrolidin-2-one |
| I-118 | cis-3-({4-[3-(3,3-difluoropyrrolidin-1-yl)azetidin-1-yl]-1-[3-(trifluoromethyl)phenyl]-1H-pyrazolo[3,4-d]pyrimidin-6-yl}amino)cyclobutanol |
| I-121 | 4-(1-{6-[(cis-3-hydroxycyclobutyl)amino]-1-[3-(trifluoromethyl)phenyl]-1H-pyrazolo[3,4-d]pyrimidin-4-yl}azetidin-3-yl)morpholin-3-one |
| I-122 | 4-(1-{6-[(trans-3-hydroxy-3-methylcyclobutyl)amino]-1-[3-(trifluoromethyl)phenyl]-1H-pyrazolo[3,4-d]pyrimidin-4-yl}azetidin-3-yl)morpholin-3-one |
| I-123 | trans-1-methyl-3-({4-[3-methyl-3-(morpholin-4-yl)azetidin-1-yl]-1-[3-(trifluoromethyl)phenyl]-1H-pyrazolo[3,4-d]pyrimidin-6-yl}amino)cyclobutanol |

In a further embodiment of the first aspect, the present invention covers combinations of two or more of the above-mentioned embodiments under the heading "further embodiments of the first aspect of the present invention".

The present invention covers any sub-combination within any embodiment or aspect of the present invention of compounds of general formula (I), supra.

The present invention covers the compounds of general formula (I) which are disclosed in the Example Section of this text, infra.

The compounds according to the invention of general formula (I) can be prepared according to the following scheme 1. The scheme and procedures described below illustrate synthetic routes to the compounds of general formula (I) of the invention and are not intended to be limiting. It is clear to the person skilled in the art that the order of transformations as exemplified in scheme 1 can be modified in various ways. The order of transformations exemplified in these schemes is therefore not intended to be limiting. In addition, interconversion of any of the substituents, R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸ or R¹⁹ as well as R^{a}, R^{b} or R^{c} can be achieved before and/or after the exemplified transformations. These modifications can be such as the introduction of protecting groups, cleavage of protecting groups, reduction or oxidation of functional groups, halogenation, metalation, substitution or other reactions known to the person skilled in the art. These transformations include those which introduce a functionality which allows for further interconversion of substituents. Appropriate protecting groups and their introduction and cleavage are well-known to the person skilled in the art (see for example T.W. Greene and P.G.M. Wuts in Protective Groups in Organic Synthesis, 3rd edition, Wiley 1999). Specific examples are described in the subsequent paragraphs.

The synthesis of compounds of formula (I) can be accomplished as depicted in scheme 1:

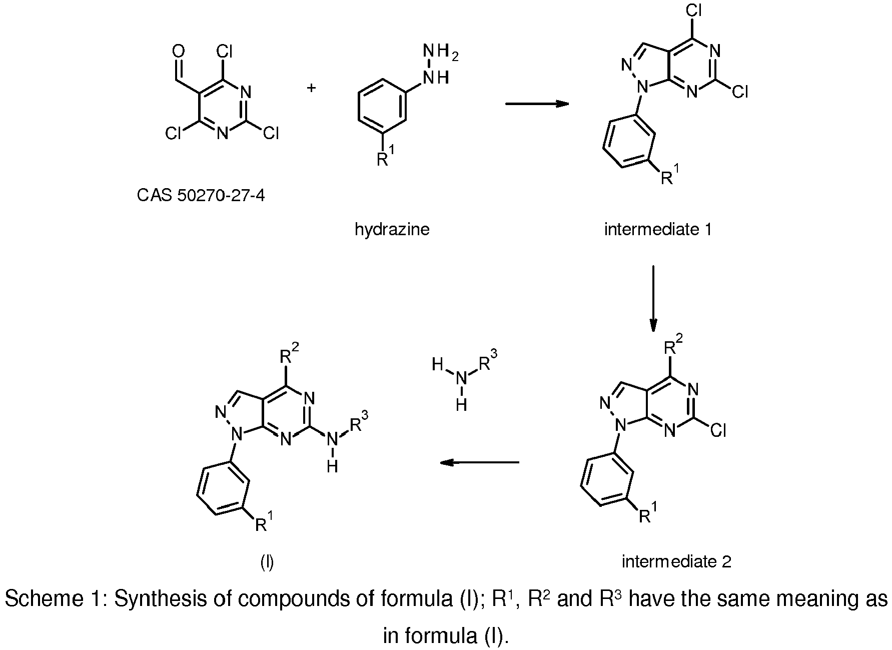

Reaction of 2,4,6-trichloropyrimidine-5-carbaldehyde with hydrazines (or salt forms (e.g. hydrochloride salts) of hydrazines) in a suitable solvent, preferably ethanol in the presence of a suitable base, preferably trimethylamine affords intermediate 1 (compare J. Med. Chem. 2009, 52, 24, 8010 - 8024). The hydrazines or their respective salts are commercially available or can be synthesized according to known procedures (see e.g. Org. Lett, 2009, Vol. 11, Issue 22, 5142-5145, Scheme 3; Angewandte Chemie - International Edition, 2013, vol. 52, # 12, p. 3434 - 3437). For example hydrazines can be obtained from starting materials displaying a nitro group by reducing the nitro group with iron in the presence of ammonium chloride and reaction of the resulting aniline with sodium nitrite and tin(II)chloride dihydrate. Intermediate 1 is then transformed to intermediate 2 by the reaction with a secondary amine in a suitable solvent in the presence of a base. Solvents such as NMP, DMSO, acetonitrile, ethanol, 2-methyltetrahydrofuran or DMF can be applied (see J. Med. Chem. 2009, 52, 24, 8010 - 8024). The solvents NMP and 2-methyltetrahydrofuran are preferred. Suitable bases are DIPEA, triethylamine, cesium carbonate or potassium carbonate. DIPEA and potassium carbonate are preferred. In some cases, the secondary amine may contain a functional group which is protected, and the corresponding protection group has to be cleaved by an additional synthetic step known to a person skilled in the art. For example, the functional group can be based on an amino group protected by a *tert*-butyloxycarbonyl group (BOC-group). The BOC-group can be cleaved with acids such as TFA to form the corresponding amino group. In some cases, the secondary amine may contain a carboxylic acid ester group which can be cleaved by hydrolysis (using e.g. aq. sodium hydroxide or lithium hydroxide solution) by procedures known to a person skilled in the art.
The secondary amines or their respective salts used for the synthesis of intermediate 2 are commercially available or can be synthesized by a person skilled to the art using known procedures or in analogy to know procedures.
Selected secondary amines used for the synthesis of intermediates 2 display an azetidine structure. In some cases, tert-butyl 3-oxoazetidine-1-carboxylate can be used as starting material for the synthesis. For example a reductive amination reaction can be applied with an amine such as morpholine (compare e.g. WO201353051 for the synthesis of tert-butyl 3-(morpholin-4-yl)azetidine-1-carboxylate). Afterwards, deprotection using e.g. hydrogenchloride in 1,4-dioxane or TFA then leads to the desired amines (see e.g. WO201353051, WO2017132474 for the synthesis of salt forms of 4-(azetidin-3-yl)morpholine).
Other azetidines displaying a 3-methylgroup can for example be synthesized in analogy to J. Frigola et. al, J. Med. Chem. 1993, 36, 801-810.

Intermediate 2 can then be transformed with amine H₂NR³ to the compounds of general formula (I) in a suitable solvent in the presence of a base (see also Bioorganic and Medicinal Chemistry Letters, 2008, vol. 18, # 8, p. 2652 - 2657 and Bioorganic and Medicinal Chemistry Letters, 2001, vol. 11, # 2, p. 191 - 193). Solvents such as NMP, isopropyl alcohol, DMSO or DMF can be applied. The solvent NMP is preferred. Bases such as DIPEA, triethylamine, cesium carbonate, sodium hydride or potassium carbonate can be applied. DIPEA is preferred. In some cases, the amine H₂NR³ may contain a functional group which is protected and the corresponding protection group has to be cleaved by an additional synthetic step. For example, R³ can contain an amino group protected by a BOC group and the BOC group can be cleaved with acids such as trifluoroacetic acid (TFA). The amine H₂NR³ or its respective salt can be commercially available or can be synthesized according to known procedures.
Selected amines H₂NR³ based on a cyclobutyl motive are commercially available or can be synthesized starting from intermediates known in literature. For example the compounds cis-3-[(tert-butoxycarbonyl)amino]cyclobutyl methanesulfonate or trans-3-[(tert-butoxycarbonyl)-amino]cyclobutyl methanesulfonate (see D. S. Radchenko et al., J. Org. Chem. 2010, 75, 5941 - 5952) can be used as starting materials using reactions known to a person skilled in the art (transformations comprise e.g. nucleophilic substitution reactions using thiolate salts such as sodium methanethiolate and subsequent oxidation of the sulfide to the sulfone e.g. with 3-chlorobenzenecarboperoxoic acid or peracetic acid and cleavage of the BOC-group by using e.g. hydrogenchloride in 1,4-dioxane or TFA) (in analogy to the synthesis of deuterated 4-(methylsulfonyl)piperidine described in J. Label Compd. Radiopharm 2016, 59, 300 - 304). In some cases, additional synthetic steps known to persons skilled in the art can be applied to generate subsets of compounds of the general formula (I). For example, compounds displaying a carboxylic acid moiety can be synthesized by saponification of the corresponding esters; carboxamides can be prepared by amide formation using amines starting from the corresponding carboxylic acids and esters of carboxylic acids can be transformed to alcohols by addition of an excess of Grignard reagents such as methylmagnesium bromide.

The compounds of general formula (I) of the present invention can be converted to any salt, preferably pharmaceutically acceptable salts, as described herein, by any method which is known to the person skilled in the art. Similarly, any salt of a compound of general formula (I) of the present invention can be converted into the free compound, by any method which is known to the person skilled in the art.

Compounds of general formula (I) of the present invention demonstrate a valuable pharmacological spectrum of action and pharmacokinetic profile, both of which could not have been predicted. Compounds of the present invention have surprisingly been found to effectively inhibit IRAK4 and it is possible therefore that said compounds be used for the treatment or prophylaxis of diseases, preferably for proliferative disorders, for autoimmune (allergic) disorders, for metabolic and inflammatory disorders in humans and animals.

Compounds of the present invention can be utilized to inhibit, block, reduce, decrease, etc., IRAK4. This method comprises administering to a mammal in need thereof, including a human as well as domestic animals, an amount of a compound of this invention, or a pharmaceutically acceptable salt, isomer, polymorph, metabolite, hydrate, solvate or ester thereof; which is effective to treat the disorder.

The present invention also provides methods of treating proliferative, metabolic and inflammatory disorders characterized by an overreacting immune system. Particular mention should be made here of inflammatory skin disorders, cardiovascular disorders, lung disorders, eye disorders, neurological disorders, pain disorders and cancer.
In particular, the novel IRAK4 inhibitors are suitable for treatment and prevention in human
- of autoimmune and inflammatory disorders, especially rheumatoid arthritis, spondylarthritis especially psoriatic arthritis and ankylosing spondyilits/ Bechterev's disease, multiple sclerosis, systemic lupus erythematosus, lupus nephritis, gout, gouty arthritis, reactive arthritis, Behcet's disease, systemic/polyarthritic idiopathic juvenile arthritis (sJIA/pJIA), Still's disease in pediatric patients, adult onset Still's disease, macrophage activiation syndrome (MAS), familial Mediterranean fever (FMF), all forms of myositis including polymyositis and dermatomyositis, systemic sarcoidosis including Sjögren's syndrome, localized or systemic amyloidosis, vasculitis including ANCA associated vasculitis and Kawasaki-Syndrome, autoinflammatory disorders with an activation of the inflammasome such as Schnitzler's syndrome, Cryopyrin-associated-periodic syndrome (CAPS) including Familial Cold-induced autoinflammatory Syndrome (FACS), Muckle-Wells-Syndrome (MWS), neonatal onset inflammatory disease (NOMID), chronic infantile neurological cutaneous and articular disorder (CINCA), TNF-receptor 1-associated periodic syndrome (TRAPS), HIDS (Hyper-IgD syndrome), Blau-syndrome,
- of inflammatory skin diseases and auto-immune or allergic diseases such as atopic dermatitis, exogenous asthma, pollinosis, nasal polyposis, cutaneous lupus (subacute and chronic), plaque psoriasis, pustular psoriasis, hidradenitis suppurativa, alopecia areata (localized, total and universalis), vitiligo, pyoderma gangrenosum, acute or chronic contact dermatitis (toxic or allergic),
- of all forms of inflammatory bowel diseases such as ulcerative colitis (UC) and Crohn's disease (CD),
- of ophthalmological disorders such as inflammatory/autoimmune uveitis,
- of neuroinflammatory disorders such as multiple sclerosis and other demyelinating disorders,
- of metabolic disorders like primary or secondary insulin resistance or polycystic ovary syndrome (PCOS),
- of gynaecological disorders, especially of endometriosis and of endometriosis-associated pain and other endometriosis-associated symptoms such as dysmenorrhoea, dyspareunia, dysuria and dyschezia,
- of inflammation-induced or chronic pain such as lower back pain, neuropathic pain, post-zoster neuralgia, cytokine release syndrome (CRS), sepsis, antibody formation in therapy with biologics, and
- of B-and T-cell lymphoma, especially forms with an IRAK4/MyD88 activating mutation.

These disorders have been well characterized in humans, but also exist with a similar etiology in other mammals and can be treated by administering pharmaceutical compositions of the present invention.

Furthermore, the novel IRAK4 inhibitors are suitable for treatment and prevention in animals
- of allergic and/or inflammatory diseases, especially of atopic dermatitis and/or Flea Allergy Dermatitis, and especially in domestic animals, particularly in dogs.

The term "therapy" as used in the present text is used conventionally, e.g. includes the prevention as well as the treatment or cure of disease.
The term "treating", or "treatment" as used in the present text is used conventionally, e.g. the management or care of a subject for the purpose of combating, alleviating, reducing, relieving, improving the condition of a disease or disorder, such as a proliferative, metabolic or inflammatory disorder characterized by an overreacting immune system.
The term "preventing", or "prevention" as used in the present text is used conventionally, e.g. interventions for primary and secondary (early detection) prevention, aiming to minimize the burden of diseases and associated risk factors.
"Primary prevention" refers to actions aimed at avoiding the manifestation of a disease.
"Secondary prevention" deals with early detection when this improves the chances for positive health outcomes. This comprises activities such as evidence-based screening programs for early detection of diseases and preventive drug therapies of proven effectiveness when administered at an early stage of the disease.

In the context of the present invention, the treatment of autoimmune and inflammatory disorders, especially rheumatoid arthritis, spondylarthritis especially psoriatic arthritis and ankylosing spondyilits/Bechterev's disease means a disease-modifiying treatment with inducation and maintenance of remission.
In the context of the present invention, the treatment of multiple sclerosis means a disease-modifying treatment with prevention of relapses and slowing of disease progression based on Expanded Disability Status Scale (EDSS).
In the context of the present invention, the treatment of systemic lupus erythematosus and lupus nephritis means inducation and maintenance of remission.
In the context of the present invention, the treatment of gout means prevention of flares and gouty arthritis. For gouty arthritis symptomatic treatment of acute flare is meant.
In the context of the present invention, the treatment of reactive arthritis means prevention of flares and symptomatic treatment of acute flare.
In the context of the present invention, the treatment of Behcet's disease means a beneficial effect for example on skin nodules, uveitis and joints.
In the context of the present invention, the treatment of systemic/polyarthritic idiopathic juvenile arthritis (sJIA/pJIA), Still's disease in pediatric patients and adult onset Still's disease menas a beneficial effect for example on systemic inflammatory signs and symptoms such as fever, reduction of flares and beneficial effect on joint inflammation.

In the context of the present invention, the treatment of macrophage activiation syndrome (MAS) means for example the amelioration of MAS, reduction of systemic inflammatory signs and prevention of lethal outcome.
In the context of the present invention, the treatment of familial Mediterranean fever (FMF) means a beneficial effect on systemic inflammatory signs and symptoms such as fever, reduction of fever flares.
In the context of the present invention, the treatment of all forms of myositis including polymyositis and dermatomyositis means a disease modifying treatment and beneficial effect for example on myositis and skin changes.
In the context of the present invention, the treatment of systemic sarcoidosis including Sjögren's syndrome means a therapeutic effect on lung function.
In the context of the present invention, the treatment of localized or systemic amyloidosis means a therapeutic effect on organ function.
In the context of the present invention, the treatment of vasculitis including ANCA associated vasculitis and Kawasaki-Syndrome means a benefical effect for example on renal function, skin vascultitis and vision.
In the context of the present invention, the treatment of autoinflammatory disorders with an activation of the inflammasome such as Schnitzler's syndrome, Cryopyrin-associated-periodic syndrome (CAPS) including Familial Cold-induced autoinflammatory Syndrome (FACS), Muckle-Wells-Syndrome (MWS) means inducation and maintenance of remission.
In the context of the present invention, the treatment of neonatal onset inflammatory disease (NOMID), chronic infantile neurological cutaneous and articular disorder (CINCA), TNF-receptor 1-associated periodic syndrome (TRAPS), HIDS (Hyper-IgD syndrome), Blau-syndrome means inducation and maintenance of remission and systemic inflammation signs. In the context of the present invention, the treatment of inflammatory skin diseases and auto-immune or allergic diseases such as atopic dermatitis means for example a reduction of IGA score, EASI50/75/90, and reduction of pruritus.
In the context of the present invention, the treatment of exogenous asthma means for example reduction of exacerbations, steroid use and lung function parameters such FEV1.
In the context of the present invention, the treatment of pollinosis means for example reduction of symptoms including rhinitis and conjunctivitis.
In the context of the present invention, the treatment of cutaneous lupus, e.g. subacute and chronic means for example reduction of skin lesions, area and intensity.
In the context of the present invention, the treatment of plaque psoriasis means reduction of Psoriasis Area Severity Index (PASI).
In the context of the present invention, the treatment of pustular psoriasis means reduction of extent and severity of pustules.

In the context of the present invention, the treatment of hidradenitis suppurativa means reduction of Sartorius Score.
In the context of the present invention, the treatment of alopecia areata (localized, total and universalis) means growing back of hair.
In the context of the present invention, the treatment of vitiligo means repigmentation.
In the context of the present invention, the treatment of pyoderma gangrenosum means for example reduction of severity and extent of lesions, remission and prevention of new flares.
In the context of the present invention, the treatment of acute or chronic contact dermatitis (toxic or allergic) means symptomatic treatment of skin eczema and pruritus.
In the context of the present invention, the treatment of all forms of inflammatory bowel diseases such as ulcerative colitis (UC) and Crohn's disease (CD) means induction of remission and maintenance of remission and treatment of fistulas.
In the context of the present invention, the treatment of ophthalmological disorders such as inflammatory/autoimmune uveitis means a beneficial effect for example on uveitis signs and vision as well as prevention of vision loss.
In the context of the present invention, the treatment of neuroinflammatory disorders such as multiple sclerosis (relapsing, relapsing remitting or progressive forms) and other demyelinating disorders means for example reduction of flares, prevention of disability according to EDSS, either with standalone therapy or in combination with other immunomodulatory agents such as Beta-interferone, Glatiramer acetate or Dimethylfumarate. In the context of the present invention, the treatment of metabolic disorders with primary or secondaryinsulin resistance including polycystic ovary syndrome (PCOS) means for example weight reduction, reduction of required anti-diabetic treatment or enhanced conception rate.
In the context of the present invention, the treatment of of inflammation-induced or chronic pain such as lower back pain, neuropathic pain means for example reduction of Visual Analog Scale for Pain (pain VAS) or Numeric Rating Scale (NRS).
In the context of the present invention, the treatment of post-zoster neuralgia means for example reduction of frequency of flares and pain according to VAS or NRS.
In the context of the present invention, the treatment of cytokine release syndrome (CRS) and sepsis means reduction of systemic inflammation signs, clinical as well as lab, inflammatory parameters with better outcome with regard to organ function and letality.
In the context of the present invention, the treatment of antibody formation in therapy with biologics (reduction of anti-drug-antibodies [neutralizing or not] and maintenance of therapeutic effect, e.g. with anti-TNF-antibodies).
In the context of the present invention, the treatment of B-and T-cell lymphoma, especially forms with an IRAK4/MyD88 activating mutation means for example reduction of lymphoma cell count, progression free survival and overall survival.

The compounds of the present invention can be used in particular in therapy and prevention, *i.e.* prophylaxis of proliferative, autoimmune disorders, metabolic, and inflammatory disorders characterized by an overreacting immune system. Particular mention should be made here of rheumatological disorders, inflammatory skin disorders, cardiovascular disorders, lung disorders, eye disorders, neurological disorders, pain disorders and cancer.
In particular, the novel IRAK4 inhibitors are suitable in therapy and prevention, *i.e.* prophylaxis in human
- of autoimmune and inflammatory disorders, especially rheumatoid arthritis, spondyloarthritis (especially psoriatic arthritis and Bechterev's disease), multiple sclerosis, systemic lupus erythematosus, psoriasis, and gout,
- of metabolic disorders, especially metabolic syndrome, primary or secondary insulin resistance, polycystic ovary syndrome (PCOS),
- of gynaecological disorders, especially of endometriosis and of endometriosis-associated pain and other endometriosis-associated symptoms such as dysmenorrhoea, dyspareunia, dysuria and dyschezia,
- of inflammation-induced or chronic pain such as lower back pain, neuropathic pain,
- of inflammatory skin diseases and auto-immune or allergic diseases such as atopic dermatitis, exogenous asthma, pollinosis, nasal polyposis, cutaneous lupus (subacute and chronic), plaque psoriasis, pustular psoriasis, hidradenitis suppurativa, alopecia areata (localized, total and universalis), vitiligo, pyoderma gangrenosum, acute or chronic contact dermatitis (toxic or allergic),
- of neuroinflammatory disorders such as multiple sclerosis and other demyelinating disorders,
- of inflammation-induced or chronic pain such as lower back pain, neuropathic pain, post-zoster neuralgia, cytokine release syndrome (CRS), sepsis, antibody formation in therapy with biologics, and
- of B-and T-cell lymphoma, especially forms with an IRAK4/MyD88 activating mutation.

Furthermore, the novel IRAK4 inhibitors are suitable in therapy and prevention, *i.e.* prophylaxis in animals
- of allergic and/or inflammatory diseases, especially of atopic dermatitis and/or Flea Allergy Dermatitis, and especially in domestic animals, particularly in dogs.

In accordance with a further aspect, the present invention covers a compound of general formula (I), as described *supra,* or stereoisomers, tautomers, N-oxides, hydrates, solvates, and salts thereof, particularly pharmaceutically acceptable salts thereof, or mixtures of same, for use in the treatment or prophylaxis of diseases, in particular proliferative, metabolic and inflammatory disorders characterized by an overreacting immune system.

In accordance with a further aspect, the present invention covers the use of a compound of general formula (I), as described *supra,* or stereoisomers, tautomers, N-oxides, hydrates, solvates, and salts thereof, particularly pharmaceutically acceptable salts thereof, or mixtures of same, for the treatment or prophylaxis of diseases in human, in particular proliferative, metabolic and inflammatory disorders characterized by an overreacting immune system, particularly
- of autoimmune and inflammatory disorders, especially rheumatoid arthritis, psoriatic arthritis, Bechterev's disease, multiple sclerosis, systemic lupus erythematosus, psoriasis, and gout,
- of metabolic disorders, especially metabolic syndrome, insulin resistance, polycystic ovary syndrome (PCOS),
- of gynaecological disorders, especially of endometriosis and of endometriosis-associated pain and other endometriosis-associated symptoms such as dysmenorrhoea, dyspareunia, dysuria and dyschezia,
- inflammation-induced or chronic pain, and of B-and T-cell lymphoma, especially forms with an IRAK4/MyD88 activating mutation.

Furthermore, the present invention covers the use of a compound of general formula (I), as described *supra,* or stereoisomers, tautomers, N-oxides, hydrates, solvates, and salts thereof, particularly pharmaceutically acceptable salts thereof, or mixtures of same, for the treatment or prophylaxis of diseases in animals, particularly
- of allergic and/or inflammatory diseases, especially of atopic dermatitis and/or Flea Allergy Dermatitis, and especially in domestic animals, particularly in dogs.
-

In accordance with a further aspect, the present invention covers the use of a compound of formula (I), described *supra,* or a stereoisomer, a tautomer, an N-oxide, a hydrate, a solvate, or a salt thereof, particularly a pharmaceutically acceptable salt thereof, or a mixture of same, for the prophylaxis or treatment of diseases in human, in particular proliferative, metabolic and inflammatory disorders characterized by an overreacting immune system, particularly
- of autoimmune and inflammatory disorders, especially rheumatoid arthritis, psoriatic arthritis, Bechterev's disease, multiple sclerosis, systemic lupus erythematosus, psoriasis, and gout,
- of metabolic disorders, especially metabolic syndrome, insulin resistance, polycystic ovary syndrome (PCOS),
- of gynaecological disorders, especially of endometriosis and of endometriosis-associated pain and other endometriosis-associated symptoms such as dysmenorrhoea, dyspareunia, dysuria and dyschezia,
- inflammation-induced or chronic pain,
- and of B-and T-cell lymphoma, especially forms with an IRAK4/MyD88 activating mutation.

Furthermore, the present invention covers the use of a compound of formula (I), described *supra,* or a stereoisomer, a tautomer, an N-oxide, a hydrate, a solvate, or a salt thereof, particularly a pharmaceutically acceptable salt thereof, or a mixture of same, for the prophylaxis or treatment of diseases in animals, particularly
- of allergic and/or inflammatory diseases, especially of atopic dermatitis and/or Flea Allergy Dermatitis, and especially in domestic animals, particularly in dogs.

In accordance with a further aspect, the present invention covers the use of a compound of general formula (I), as described *supra,* or stereoisomers, tautomers, N-oxides, hydrates, solvates, and salts thereof, particularly pharmaceutically acceptable salts thereof, or mixtures of same, in a method of treatment or prophylaxis of diseases in human, in particular proliferative, metabolic and inflammatory disorders characterized by an overreacting immune system, particularly
- of autoimmune and inflammatory disorders, especially rheumatoid arthritis, psoriatic arthritis, Bechterev's disease, multiple sclerosis, systemic lupus erythematosus, psoriasis, and gout,
- of metabolic disorders, especially metabolic syndrome, insulin resistance, polycystic ovary syndrome (PCOS),
- of gynaecological disorders, especially of endometriosis and of endometriosis-associated pain and other endometriosis-associated symptoms such as dysmenorrhoea, dyspareunia, dysuria and dyschezia,
- inflammation-induced or chronic pain,
- and of B-and T-cell lymphoma, especially forms with an IRAK4/MyD88 activating mutation.

Furthermore, the present invention covers the use of a compound of formula (I), as described *supra,* or stereoisomers, tautomers, N-oxides, hydrates, solvates, and salts thereof, particularly pharmaceutically acceptable salts thereof, or mixtures of same, in a method of treatment or prophylaxis of diseases in animals, particularly
- of allergic and/or inflammatory diseases, especially of atopic dermatitis and/or Flea Allergy Dermatitis, and especially in domestic animals, particularly in dogs.

In accordance with a further aspect, the present invention covers use of a compound of general formula (I), as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases in human, in particular proliferative, metabolic and inflammatory disorders characterized by an overreacting immune system, particularly
- of autoimmune and inflammatory disorders, especially rheumatoid arthritis, psoriatic arthritis, Bechterev's disease, multiple sclerosis, systemic lupus erythematosus, psoriasis, and gout,
- of metabolic disorders, especially metabolic syndrome, insulin resistance, polycystic ovary syndrome (PCOS),
- of gynaecological disorders, especially of endometriosis and of endometriosis-associated pain and other endometriosis-associated symptoms such as dysmenorrhoea, dyspareunia, dysuria and dyschezia,
- inflammation-induced or chronic pain,
- and of B-and T-cell lymphoma, especially forms with an IRAK4/MyD88 activating mutation.

Furthermore, the present invention covers the use of a compound of formula (I), as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases in animals, particularly
- of allergic and/or inflammatory diseases, especially of atopic dermatitis and/or Flea Allergy Dermatitis, and especially in domestic animals, particularly in dogs.

In accordance with a further aspect, the present invention covers a method of treatment or prophylaxis of diseases in human, in particular proliferative, metabolic and inflammatory disorders characterized by an overreacting immune system, particularly
- of autoimmune and inflammatory disorders, especially rheumatoid arthritis, psoriatic arthritis, Bechterev's disease, multiple sclerosis, systemic lupus erythematosus, psoriasis, and gout,
- of metabolic disorders, especially metabolic syndrome, insulin resistance, polycystic ovary syndrome (PCOS),
- of gynaecological disorders, especially of endometriosis and of endometriosis-associated pain and other endometriosis-associated symptoms such as dysmenorrhoea, dyspareunia, dysuria and dyschezia,
- inflammation-induced or chronic pain,
- and of B-and T-cell lymphoma, especially forms with an IRAK4/MyD88 activating mutation,
using an effective amount of a compound of general formula (I), as described *supra,* or stereoisomers, tautomers, N-oxides, hydrates, solvates, and salts thereof, particularly pharmaceutically acceptable salts thereof, or mixtures of same.

Furthermore, the present invention covers a method of treatment or prophylaxis of diseases in animals, particularly
- of allergic and/or inflammatory diseases, especially of atopic dermatitis and/or Flea Allergy Dermatitis, and especially in domestic animals, particularly in dogs,
using an effective amount of a compound of general formula (I), as described *supra,* or stereoisomers, tautomers, N-oxides, hydrates, solvates, and salts thereof, particularly pharmaceutically acceptable salts thereof, or mixtures of same.
In accordance with a further aspect, the present invention covers pharmaceutical compositions, in particular a medicament, comprising a compound of general formula (I), as described *supra,* or a stereoisomer, a tautomer, an N-oxide, a hydrate, a solvate, a salt thereof, particularly a pharmaceutically acceptable salt, or a mixture of same, and one or more excipients), in particular one or more pharmaceutically acceptable excipient(s). Conventional procedures for preparing such pharmaceutical compositions in appropriate dosage forms can be utilized.
The present invention furthermore covers pharmaceutical compositions, in particular medicaments, which comprise at least one compound according to the invention, conventionally together with one or more pharmaceutically suitable excipients, and to their use for the above-mentioned purposes.
It is possible for the compounds according to the invention to have systemic and/or local activity. For this purpose, they can be administered in a suitable manner, such as, for example, via the oral, parenteral, pulmonary, nasal, sublingual, lingual, buccal, rectal, vaginal, dermal, transdermal, conjunctival, otic route or as an implant or stent.
For these administration routes, it is possible for the compounds according to the invention to be administered in suitable administration forms.
For oral administration, it is possible to formulate the compounds according to the invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.
Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal). Administration forms which are suitable for parenteral administration are, inter alia, preparations for injection and infusion in the form of solutions, suspensions, emulsions, lyophylisates or sterile powders.
Examples which are suitable for other administration routes are pharmaceutical forms for inhalation [inter alia powder inhalers, nebulizers], nasal drops, nasal solutions, nasal sprays; tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, aqueous suspensions (lotions, mixturae agitandae), lipophilic suspensions, emulsions, ointments, creams, transdermal therapeutic systems (such as, for example, patches), milk, pastes, foams, dusting powders, implants or stents.
The compounds according to the invention can be incorporated into the stated administration forms. This can be effected in a manner known per se by mixing with pharmaceutically suitable excipients. Pharmaceutically suitable excipients include, inter alia,
- fillers and carriers (for example cellulose, microcrystalline cellulose (such as, for example, Avicel®), lactose, mannitol, starch, calcium phosphate (such as, for example, Di-Cafos®)),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette®), sorbitan fatty acid esters (such as, for example, Span®), polyoxyethylene sorbitan fatty acid esters (such as, for example, Tween®), polyoxyethylene fatty acid glycerides (such as, for example, Cremophor®), polyoxethylene fatty acid esters, polyoxyethylene fatty alcohol ethers, glycerol fatty acid esters, poloxamers (such as, for example, Pluronic®),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol®); alginates, gelatine),
- disintegrants (for example modified starch, carboxymethylcellulose-sodium, sodium starch glycolate (such as, for example, Explotab®), cross- linked polyvinylpyrrolidone, croscarmellose-sodium (such as, for example, AcDiSol®)),
- flow regulators, lubricants, glidants and mould release agents (for example magnesium stearate, stearic acid, talc, highly-disperse silicas (such as, for example, Aerosil®)),
- coating materials (for example sugar, shellac) and film formers for films or diffusion membranes which dissolve rapidly or in a modified manner (for example polyvinylpyrrolidones (such as, for example, Kollidon®), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit®)),
- capsule materials (for example gelatine, hydroxypropylmethylcellulose),
- synthetic polymers (for example polylactides, polyglycolides, polyacrylates, polymethacrylates (such as, for example, Eudragit®), polyvinylpyrrolidones (such as, for example, Kollidon®), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate),
- colourants (for example inorganic pigments such as, for example, iron oxides, titanium dioxide),
- flavourings, sweeteners, flavour- and/or odour-masking agents.
The present invention furthermore relates to a pharmaceutical composition which comprise at least one compound according to the invention, conventionally together with one or more pharmaceutically suitable excipient(s), and to their use according to the present invention.

In accordance with another aspect, the present invention covers pharmaceutical combinations, in particular medicaments, comprising at least one compound of general formula (I) of the present invention and at least one or more further active ingredients, in particular for the treatment and/or prophylaxis of diseases in human, in particular proliferative, metabolic and inflammatory disorders characterized by an overreacting immune system, particularly
- of autoimmune and inflammatory disorders, especially rheumatoid arthritis, psoriatic arthritis, Bechterev's disease, multiple sclerosis, systemic lupus erythematosus, psoriasis, and gout,
- of metabolic disorders, especially metabolic syndrome, insulin resistance, polycystic ovary syndrome (PCOS),
- of gynaecological disorders, especially of endometriosis and of endometriosis-associated pain and other endometriosis-associated symptoms such as dysmenorrhoea, dyspareunia, dysuria and dyschezia,
- inflammation-induced or chronic pain,
- and of B-and T-cell lymphoma, especially forms with an IRAK4/MyD88 activating mutation.

Furthermore, the present invention covers pharmaceutical combinations, in particular medicaments, comprising at least one compound of general formula (I) of the present invention and at least one or more further active ingredients, in particular for the treatment and/or prophylaxis of diseases in animals, particularly
- of allergic and/or inflammatory diseases, especially of atopic dermatitis and/or Flea Allergy Dermatitis, and especially in domestic animals, particularly in dogs.

Particularly, the present invention covers a pharmaceutical combination, which comprises:
- one or more first active ingredients, in particular compounds of general formula (I) as defined *supra,* and
- one or more further active ingredients for the treatment in human, in particular of proliferative, metabolic and inflammatory disorders characterized by an overreacting immune system, particularly
   - of autoimmune and inflammatory disorders, especially rheumatoid arthritis, psoriatic arthritis, Bechterev's disease, multiple sclerosis, systemic lupus erythematosus, psoriasis, and gout,
   - of metabolic disorders, especially metabolic syndrome, insulin resistance, polycystic ovary syndrome (PCOS),
   - of gynaecological disorders, especially of endometriosis and of endometriosis-associated pain and other endometriosis-associated symptoms such as dysmenorrhoea, dyspareunia, dysuria and dyschezia,
   - inflammation-induced or chronic pain,
   - and of B-and T-cell lymphoma, especially forms with an IRAK4/MyD88 activating mutation.

Furthermore, the present invention covers a pharmaceutical combination, which comprises:
- one or more first active ingredients, in particular compounds of general formula (I) as defined *supra,* and
- one or more further active ingredients for the treatment in animals, particularly
   - of allergic and/or inflammatory diseases, especially of atopic dermatitis and/or Flea Allergy Dermatitis, and especially in domestic animals, particularly in dogs.

The term "combination" in the present invention is used as known to persons skilled in the art, it being possible for said combination to be a fixed combination, a non-fixed combination or a kit-of-parts.
A "fixed combination" in the present invention is used as known to persons skilled in the art and is defined as a combination wherein, for example, a first active ingredient, such as one or more compounds of general formula (I) of the present invention, and a further active ingredient are present together in one unit dosage or in one single entity. One example of a "fixed combination" is a pharmaceutical composition wherein a first active ingredient and a further active ingredient are present in admixture for simultaneous administration, such as in a formulation. Another example of a "fixed combination" is a pharmaceutical combination wherein a first active ingredient and a further active ingredient are present in one unit without being in admixture.
A non-fixed combination or "kit-of-parts" in the present invention is used as known to persons skilled in the art and is defined as a combination wherein a first active ingredient and a further active ingredient are present in more than one unit. One example of a non-fixed combination or kit-of-parts is a combination wherein the first active ingredient and the further active ingredient are present separately. It is possible for the components of the non-fixed combination or kit-of-parts to be administered separately, sequentially, simultaneously, concurrently or chronologically staggered.
The compounds of the present invention can be administered as the sole pharmaceutical agent or in combination with one or more other pharmaceutically active ingredients where the combination causes no unacceptable adverse effects. The present invention also covers such pharmaceutical combinations. For example, the compounds of the present invention can be combined with known active ingredients.
Examples of active ingredients include, but are not limited to:
General mention may be made of active ingredients such as antibacterial (e.g. penicillins, vancomycin, ciprofloxacin), antiviral (e.g. aciclovir, oseltamivir) and antimycotic (e.g. naftifin, nystatin) substances and gamma globulins, immunomodulatory and immunosuppressive compounds such as cyclosporin, Methotrexat®, TNF antagonists (e.g. Humira®,, Etanercept, Infliximab), IL-1 inhibitors (e.g. Anakinra, Canakinumab, Rilonacept), phosphodiesterase inhibitors (e.g. Apremilast), Jak/STAT inhibitors (e.g. Tofacitinib, Baricitinib, GLPG0634), leflunomid, cyclophosphamide, rituximab, belimumab, tacrolimus, rapamycin, mycophenolate mofetil, interferons, corticosteroids (e.g. prednisone, prednisolone, methylprednisolone, hydrocortisone, betamethasone), cyclophosphamide, azathioprine and sulfasalazine; paracetamol, non-steroidal anti-inflammatory substances (NSAIDS) (aspirin, ibuprofen, naproxen, etodolac, celecoxib, colchicine).

The following should be mentioned for tumour therapy: immunotherapy (e.g. aldesleukin, alemtuzumab, basiliximab, catumaxomab, celmoleukin, denileukin diftitox, eculizumab, edrecolomab, gemtuzumab, ibritumomab tiuxetan, imiquimod, interferon-alpha, interferon beta, interferon-gamma, ipilimumab, lenalidomide, lenograstim, mifamurtide, ofatumumab, oprelvekin, picibanil, plerixafor, polysaccharide-K, sargramostim, sipuleucel-T, tasonermin, teceleukin, tocilizumab), antiproliferative substances, for example but not exclusively amsacrine, arglabin, arsenic trioxide, asparaginase, bleomycin, busulfan, dactinomycin, docetaxel, epirubicin, peplomycin, trastuzumab, rituximab, obinutuzumab, ofatumumab, tositumomab, aromatase inhibitors (e.g. exemestane, fadrozole, formestane, letrozole, anastrozole, vorozole), antioestrogens (e.g. chlormadinone, fulvestrant, mepitiostane, tamoxifen, toremifen), oestrogens (e.g. oestradiol, polyoestradiol phosphate, raloxifen), gestagens (e.g. medroxyprogesterone, megestrol), topoisomerase I inhibitors (e.g. irinotecan, topotecan), topoisomerase II inhibitors (e.g. amrubicin, daunorubicin, elliptiniumacetate, etoposide, idarubicin, mitoxantrone, teniposide), microtubuli-active substances (e.g. cabazitaxel, eribulin, paclitaxel, vinblastine, vincristine, vindesine, vinorelbine), telomerase inhibitors (e.g. imetelstat), alkylating substances and histone deacetylase inhibitors (e.g. bendamustine, carmustine, chlormethine, dacarbazine, estramustine, ifosfamide, lomustine, mitobronitol, mitolactol, nimustine prednimustine, procarbazine, ranimustine, streptozotocin, temozolomide, thiotepa, treosulfan, trofosfamide, vorinostat, romidepsin, panobinostat); substances which affect cell differentation processes, such as abarelix, aminoglutethimide, bexarotene, MMP inhibitors (peptide mimetics, non-peptide mimetics and tetracyclines, for example marimastat, BAY 12-9566, BMS-275291, clodronate, prinomastat, doxycycline), mTOR inhibitors (e.g. sirolimus, everolimus, temsirolimus, zotarolimus), antimetabolites (e.g. clofarabine, doxifluridine, methotrexate, 5-fluorouracil, cladribine, cytarabine, fludarabine, mercaptopurine, methotrexate, pemetrexed, raltitrexed, tegafur, tioguanine), platinum compounds (e.g. carboplatin, cisplatin, cisplatinum, eptaplatin, lobaplatin, miriplatin, nedaplatin, oxaliplatin); antiangiogenic compounds (e.g. bevacizumab), antiandrogenic compounds (e.g. bevacizumab, enzalutamide, flutamide, nilutamide, bicalutamide, cyproterone, cyproterone acetate), proteasome inhibitors (e.g. bortezomib, carfilzomib, oprozomib, ONYX0914), gonadoliberin agonists and antagonists (e.g. abarelix, buserelin, deslorelin, ganirelix, goserelin, histrelin, triptorelin, degarelix, leuprorelin), methionine aminopeptidase inhibitors (e.g. bengamide derivatives, TNP-470, PPI-2458), heparanase inhibitors (e.g. SST0001, PI-88); inhibitors against genetically modified Ras protein (e.g. farnesyl transferase inhibitors such as lonafarnib, tipifarnib), HSP90 inhibitors (e.g. geldamycin derivatives such as 17-allylaminogeldanamycin, 17-demethoxygeldanamycin (17AAG), 17-DMAG, retaspimycin hydrochloride, IPI-493, AUY922, BIIB028, STA-9090, KW-2478), kinesin spindle protein inhibitors (e.g. SB715992, SB743921, pentamidine/chlorpromazine), MEK (mitogen-activated protein kinase kinase) inhibitors (e.g. trametinib, BAY 86-9766 (refametinib), AZD6244), kinase inhibitors (e.g.: sorafenib, regorafenib, lapatinib, Sutent®, dasatinib, cetuximab, BMS-908662, GSK2118436, AMG 706, erlotinib, gefitinib, imatinib, nilotinib, pazopanib, roniciclib, sunitinib, vandetanib, vemurafenib), hedgehog signalling inhibitors (e.g. cyclopamine, vismodegib), BTK (Bruton's tyrosine kinase) inhibitor (e.g. ibrutinib, acalabrutinib, evobrutinib), JAK/pan-JAK (janus kinase) inhibitor (e.g. SB-1578, baricitinib, tofacitinib, pacritinib, momelotinib, ruxolitinib, VX-509, AZD-1480, TG-101348), PI3K inhibitor (e.g. BAY 1082439, BAY 80-6946 (copanlisib), ATU-027, SF-1126, DS-7423, GSK-2126458, buparlisib, PF-4691502, BYL-719, XL-147, XL-765, idelalisib), SYK (spleen tyrosine kinase) inhibitors (e.g. fostamatinib, Excellair, PRT-062607), p53 gene therapy, bisphosphonates (e.g. etidronate, clodronate, tiludronate, pamidronate, alendronic acid, ibandronate, risedronate, zoledronate). For combination, the following active ingredients should also be mentioned by way of example but not exclusively: rituximab, cyclophosphamide, doxorubicin, doxorubicin in combination with oestrone, vincristine, chlorambucil, fludarabin, dexamethasone, cladribin, prednisone, 131I-chTNT, abiraterone, aclarubicin, alitretinoin, bisantrene, calcium folinate, calcium levofolinate, capecitabin, carmofur, clodronic acid, romiplostim, crisantaspase, darbepoetin alfa, decitabine, denosumab, dibrospidium chloride, eltrombopag, endostatin, epitiostanol, epoetin alfa, filgrastim, fotemustin, gallium nitrate, gemcitabine, glutoxim, histamine dihydrochloride, hydroxycarbamide, improsulfan, ixabepilone, lanreotide, lentinan, levamisole, lisuride, lonidamine, masoprocol, methyltestosterone, methoxsalen, methyl aminolevulinate, miltefosine, mitoguazone, mitomycin, mitotane, nelarabine, nimotuzumab, nitracrin, omeprazole, palifermin, panitumumab, pegaspargase, PEG epoetin beta (methoxy-PEG epoetin beta), pegfilgrastim, peg interferon alfa-2b, pentazocine, pentostatin, perfosfamide, pirarubicin, plicamycin, poliglusam, porfimer sodium, pralatrexate, quinagolide, razoxane, sizofirane, sobuzoxan, sodium glycididazole, tamibarotene, the combination of tegafur and gimeracil and oteracil, testosterone, tetrofosmin, thalidomide, thymalfasin, trabectedin, tretinoin, trilostane, tryptophan, ubenimex, vapreotide, yttrium-90 glass microspheres, zinostatin, zinostatin stimalamer.

Also suitable for tumour therapy is a combination of a non-drug therapy such as chemotherapy (e.g. azacitidine, belotecan, enocitabine, melphalan, valrubicin, vinflunin, zorubicin), radiotherapy (e.g. I-125 seeds, palladium-103 seed, radium-223 chloride) or phototherapy (e.g. temoporfin, talaporfin) which is accompanied by a drug treatment with the inventive IRAK4 inhibitors or which, after the non-drug tumour therapy such as chemotherapy, radiotherapy or phototherapy has ended, are supplemented by a drug treatment with the inventive IRAK4 inhibitors.
In addition to those mentioned above, the inventive IRAK4 inhibitors can also be combined with the following active ingredients:
active ingredients for Alzheimer's therapy, for example acetylcholinesterase inhibitors (e.g. donepezil, rivastigmine, galantamine, tacrine), NMDA (N-methyl-D-aspartate) receptor antagonists (e.g. memantine); L-DOPA/carbidopa (L-3,4-dihydroxyphenylalanine), COMT (catechol-O-methyltransferase) inhibitors (e.g. entacapone), dopamine agonists (e.g. ropinirole, pramipexole, bromocriptine), MAO-B (monoaminooxidase-B) inhibitors (e.g. selegiline), anticholinergics (e.g. trihexyphenidyl) and NMDA antagonists (e.g. amantadine) for treatment of Parkinson's; beta-interferon (IFN-beta) (e.g. IFN beta-1b, IFN beta-1a Avonex® and Betaferon®), glatiramer acetate, immunoglobulins, natalizumab, fingolimod and immunosuppressants such as mitoxantrone, azathioprine and cyclophosphamide for treatment of multiple sclerosis; substances for treatment of pulmonary disorders, for example beta-2-sympathomimetics (e.g. salbutamol), anticholinergics (e.g. glycopyrronium), methylxanthines (e.g. theophylline), leukotriene receptor antagonists (e.g. montelukast), PDE-4 (phosphodiesterase type 4) inhibitors (e.g. roflumilast), methotrexate, IgE antibodies, azathioprine and cyclophosphamide, cortisol-containing preparations; substances for treatment of osteoarthritis such as non-steroidal anti-inflammatory substances (NSAIDs). In addition to the two therapies mentioned, methotrexate and biologics for B-cell and T-cell therapy (e.g. rituximab, abatacept) should be mentioned for rheumatoid disorders, for example rheumatoid arthritis, spondyloarthritis and juvenile idiopathic arthritis. Neurotrophic substances such as acetylcholinesterase inhibitors (e.g. donepezil), MAO (monoaminooxidase) inhibitors (e.g. selegiline), interferons und anticonvulsives (e.g. gabapentin); active ingredients for treatment of cardiovascular disorders such as beta-blockers (e.g. metoprolol), ACE inhibitors (e.g. benazepril), angiotensin receptor blockers (e.g. losartan, valsartan), diuretics (e.g. hydrochlorothiazide), calcium channel blockers (e.g. nifedipine), statins (e.g. simvastatin, fluvastatin); anti-diabetic drugs, for example metformin, glinides (e.g. nateglinide), DPP-4 (dipeptidyl peptidase-4) inhibitors (e.g. linagliptin, saxagliptin, sitagliptin, vildagliptin), SGLT2 (sodium/glucose cotransporter 2) inhibitors/ gliflozin (e.g. dapagliflozin, empagliflozin), incretin mimetics (hormone glucose-dependent insulinotropic peptide (GIP) and glucagon-like peptid 1 (GLP-1) analogues/agonists) (e.g. exenatide, liraglutide, lixisenatide), α-glucosidase inhibitors (e.g. acarbose, miglitol, voglibiose) and sulphonylureas (e.g. glibenclamide, tolbutamide), insulin sensitizers (e.g. pioglitazone) and insulin therapy (e.g. NPH insulin, insulin lispro), substances for treatment of hypoglycaemia, for treatment of diabetes and metabolic syndrome. Lipid-lowering drugs, for example fibrates (e.g. bezafibrate, etofibrate, fenofibrate, gemfibrozil), nicotinic acid derivatives (e.g. nicotinic acid/laropiprant), ezetimib, statins (e.g. simvastatin, fluvastatin), anion exchangers (e.g. colestyramine, colestipol, colesevelam). Active ingredients such as mesalazine, sulfasalazine, azathioprine, 6-mercaptopurine or methotrexate, probiotic bacteria (Mutaflor, VSL#3®, Lactobacillus GG, Lactobacillus plantarum, L. acidophilus, L. casei, Bifidobacterium infantis 35624, Enterococcus fecium SF68, Bifidobacterium longum, Escherichia coli Nissle 1917), antibiotics, for example ciprofloxacin and metronidazole, anti-diarrhoea drugs, for example loperamide, or laxatives (bisacodyl) for treatment of chronic inflammatory bowel diseases. Immunosuppressants such as glucocorticoids and non-steroidale anti-inflammatory substances (NSAIDs), cortisone, chloroquine, cyclosporine, azathioprine, belimumab, rituximab, cyclophosphamide for treatment of lupus erythematosus. By way of example but not exclusively, calcineurin inhibitors (e.g. tacrolimus and ciclosporin), cell division inhibitors (e.g. azathioprine, mycophenolate mofetil, mycophenolic acid, everolimus or sirolimus), rapamycin, basiliximab, daclizumab, anti-CD3 antibodies, anti-T-lymphocyte globulin/anti-lymphocyte globulin for organ transplants. Vitamin D3 analogues, for example calcipotriol, tacalcitol or calcitriol, salicylic acid, urea, ciclosporine, methotrexate, efalizumab for dermatological disorders.

Mention should also be made of medicaments comprising at least one of the compounds of formula (I) and one or more further active ingredients, especially EP4 inhibitors (prostaglandin E2 receptor 4 inhibitors), P2X3 inhibitors (P2X purinoceptor 3), P2X4 inhibitors (P2X purinoceptor 4), PTGES inhibitors (prostaglandin E synthase inhibitors), AKR1C3 inhibitors (aldo-keto reductase family 1 member C3 inhibitors), or Bradykinin receptor B1 (B1; BDKRB1) antagonists for treatment and/or prevention of the aforementioned disorders.

Based upon standard laboratory techniques known to evaluate compounds useful for the treatment of diseases in humans and in animals, especially of proliferative disorders, of autoimmune (allergic) disorders, of metabolic and inflammatory disorders, by standard toxicity tests and by standard pharmacological assays for the determination of treatment of the conditions identified above in mammals, and by comparison of these results with the results of known active ingredients or medicaments that are used to treat these conditions, the effective dosage of the compounds of the present invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, it is possible for "drug holidays", in which a patient is not dosed with a drug for a certain period of time, to be beneficial to the overall balance between pharmacological effect and tolerability. It is possible for a unit dosage to contain from about 0.5 mg to about 1500 mg of active ingredient and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.
Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

### EXPERIMENTAL SECTION

NMR peak forms are stated as they appear in the spectra, possible higher order effects have not been considered.

The ¹H-NMR data of selected compounds are listed in the form of ¹H-NMR peaklists. Therein, for each signal peak the δ value in ppm is given, followed by the signal intensity, reported in round brackets. The δ value-signal intensity pairs from different peaks are separated by commas. Therefore, a peaklist is described by the general form: δ₁ (intensity₁), δ₂ (intensity₂), ... , δᵢ (intensityᵢ), ... , δₙ (intensityₙ).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A ¹H-NMR peaklist is similar to a classical ¹H-NMR readout, and thus usually contains all the peaks listed in a classical NMR interpretation. Moreover, similar to classical ¹H-NMR printouts, peaklists can show solvent signals, signals derived from stereoisomers of the particular target compound, peaks of impurities, ¹³C satellite peaks, and/or spinning sidebands. The peaks of stereoisomers, and/or peaks of impurities are typically displayed with a lower intensity compared to the peaks of the target compound (e.g., with a purity of >90%). Such stereoisomers and/or impurities may be typical for the particular manufacturing process, and therefore their peaks may help to identify a reproduction of the manufacturing process on the basis of "by-product fingerprints". An expert who calculates the peaks of the target compound by known methods (MestReC, ACD simulation, or by use of empirically evaluated expectation values), can isolate the peaks of the target compound as required, optionally using additional intensity filters. Such an operation would be similar to peak-picking in classical ¹H-NMR interpretation. A detailed description of the reporting of NMR data in the form of peaklists can be found in the publication "Citation of NMR Peaklist Data within Patent Applications" (cf. http://www.researchdisclosure.com/searching-disclosures, Research Disclosure Database Number 605005, 2014, 01 Aug 2014). In the peak picking routine, as described in the Research Disclosure Database Number 605005, the parameter "MinimumHeight" can be adjusted between 1% and 4%. However, depending on the chemical structure and/or depending on the concentration of the measured compound it may be reasonable to set the parameter "MinimumHeight" <1%.

Chemical names were generated using the ACD/Name software from ACD/Labs. In some cases generally accepted names of commercially available reagents were used in place of ACD/Name generated names.

The following table 1 lists the abbreviations used in this paragraph and in the Intermediates and Examples sections as far as they are not explained within the text body. Other abbreviations have their meanings customary per se to the skilled person.

**Table 1**

| **Abbreviation** | **Meaning** |
|---|---|
| aq. | aqueous |
| BOC | t-butoxycarbonyl |
| br | broad |
| CI | chemical ionisation |
| d | doublet |
| DAD | diode array detector |
| DCM | dichloromethane |
| dd | double-doublet |
| DIPEA | diisopropylethylamine |
| DMA | Dimethylacetamide |
| DMF | *N,N*-dimethylformamide |
| DMSO | dimethyl sulfoxide |
| dt | double-triplet |
| ELSD | Evaporative Light Scattering Detector |
| eq. | equivalent |
| ESI | electrospray (ES) ionisation |
| h | hour |
| HATU | 1-[bis(dimethylamino)methylene]-1 H-1,2,3-triazolo[4,5-b]pyridinium 3-oxid hexafluorophosphate |
| HCl | hydrochloric acid |
| HPLC | high performance liquid chromatography |
| LC-MS | liquid chromatography mass spectrometry |
| m | multiplet |
| min | minute |
| MeCN | acetonitrile |
| MeOH | methanol |
| MS | mass spectrometry |
| MTBE | methyl-tert-butyl ether |
| NaCl | sodium chloride |
| NaHCO₃ | sodium hydrogen carbonate or sodium bicarbonate |
| NMR | nuclear magnetic resonance spectroscopy: chemical shifts (δ) are given in ppm. The chemical shifts were corrected by setting the DMSO signal to 2.50 ppm unless otherwise stated. |
| PDA | Photo Diode Array |
| Pd/C | palladium on activated charcoal |
| q | quartet |
| r.t. or rt or RT | room temperature |
| Rt | retention time (as measured either with HPLC or UPLC) in minutes |
| s | singlet |
| sat. | saturated |
| SQD | Single-Quadrupole-Detector |
| t | triplet |
| td | triple-doublet |
| TEA | triethylamine |
| TFA | trifluoroacetic acid |
| THF | tetrahydrofuran |
| UPLC | ultra performance liquid chromatography |

The various aspects of the invention described in this application are illustrated by the following examples, which are not meant to limit the invention in any way.

The example testing experiments described herein serve to illustrate the present invention and the invention is not limited to the examples given.

### EXPERIMENTAL SECTION - GENERAL PART

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartridges KP-Sil® or KP-NH® in combination with a Biotage autopurifier system (SP4® or Isolera Four®) and eluents such as gradients of hexane/ethyl acetate or DCM/methanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

In the following section, a selection of methods for the analysis and purification of compounds and compound mixtures can be found:
Examples for analytical LC-MS and UPLC-MS methods can be found below. The masses (m/z) are reported from the positive mode electrospray ionisation unless the negative mode is indicated (ESI-).

### Method A:0-60AB, Shimadzu

Instrument: SHIMADZU LCMS-2020 SingleQuad; Column: Chromolith@Flash RP-18E 25-2 MM; eluent A: water + 0.0375 vol % trifluoroacetic acid, eluent B: acetonitrile + 0.01875 vol % trifluoroacetic acid; gradient: 0-0.8 min 0-60% B, 0.8-1.2 min 60% B; flow 1.5 ml/min; temperature: 50 °C; PDA: 220 nm & 254 nm.

### Method B:0-60AB, Agilent

Instrument: Agilent 1100\G1956A SingleQuad; Column: Kinetex@ 5um EVO C18 30*2.1 mm; eluent A: water + 0.0375 vol % trifluoroacetic acid, eluent B: acetonitrile + 0.01875 vol % trifluoroacetic acid; gradient: 0-0.8 min 0-60% B, 0.8-1.2 min 60% B; flow 1.5 ml/min; temperature: 50 °C; PDA: 220 nm & 254 nm.

### Method C:5-95AB, Shimadzu

Instrument: SHIMADZU LCMS-2020 SingleQuad; Column: Chromolith@Flash RP-18E 25-2 MM; eluent A: water + 0.0375 vol % trifluoroacetic acid, eluent B: acetonitrile + 0.01875 vol % trifluoroacetic acid; gradient: 0-0.8 min, 5-95% B, 0.8-1.2 min 95% B; flow 1.5 ml/min; temperature: 50 °C; PDA: 220 nm & 254 nm.

### Method D:5-95AB, Agilent

Instrument: Agilent 1100\G1956A SingleQuad; Column: Kinetex@ 5 µm EVO C18 30*2.1 mm; eluent A: water + 0.0375 vol % trifluoroacetic acid, eluent B: acetonitrile + 0.01875 vol % trifluoroacetic acid; gradient: 0-0.8 min 5-95% B, 0.8-1.2 min 95% B; flow 1.5 ml/min; temperature: 50 °C; PDA: 220 nm & 254 nm.

### Method E:10-80CD, Agilent

Instrument: Agilent 1200\G6110A SingleQuad; Column: XBridge C18 2.1*50 mm, 5 µm; eluent A: water + 0.025 vol % ammonium hydroxide, eluent B: acetonitrile; gradient: 0-1.2 min 10-80% B, 1.2-1.6 min 80% B; flow 1.2 ml/min; temperature: 40 °C; DAD: 220 nm & 254 nm.

### Method F:0-60CD, Agilent

Instrument: Agilent 1200\G6110A SingleQuad; Column: XBridge C18 2.1*50 mm, 5 µm; eluent A: water + 0.025 vol % ammonium hydroxide, eluent B: acetonitrile; gradient: 0-1.2 min 0-60% B, 1.2-1.6 min 60% B; flow 1.0 ml/min; temperature: 40 °C; DAD: 220 nm & 254 nm.

### Method G:5-95CD, Shimadzu

Instrument: SHIMADZU LCMS-2020 SingleQuad; Column: Kinetex EVO C18 2.1*30 mm, 5 µm; eluent A: water + 0.025 vol % ammonium hydroxide, eluent B: acetonitrile; gradient: 0-0.8 min, 5-95% B, 0.8-1.2 min 95% B; flow 1.5 ml/min; temperature: 40 °C; PDA: 220 nm & 254 nm.

### Method H:0-60CD, Shimadzu

Instrument: SHIMADZU LCMS-2020 SingleQuad; Column: Kinetex EVO C18 2.1*30 mm, 5 µm; eluent A: water + 0.025 vol % ammonium hydroxide, eluent B: acetonitrile; gradient: 0-1.2 min, 0-60% B, 1.2-1.6 min, 60% B; flow 1.0 ml/min; temperature: 40 °C; PDA: 220 nm & 254 nm.

### Method I:5-95CD, Shimadzu

Instrument: SHIMADZU LCMS-2020 SingleQuad; Column: Kinetex EVO C18 2.1*30 mm, 5um; eluent A: water + 0.025 vol % ammonium hydroxide, eluent B: acetonitrile; gradient: 0-0.8 min, 5-95% B, 0.8-1.2 min, 95% B; flow 1.5 ml/min; temperature: 40 °C; PDA: 220 nm & 254 nm.

### Method J:0-60CD, Shimadzu

Instrument: SHIMADZU LCMS-2020 SingleQuad; Column: Kinetex EVO C18 2.1*30 mm, 5 µm; eluent A: water + 0.025 vol % ammonium hydroxide, eluent B: acetonitrile; gradient: 0-0.8 min, 0-60% B, 0.8-1.2 min, 60% B; flow 1.5 ml/min; temperature: 40 °C; PDA: 220 nm & 254 nm.

### Method a01:

Instrument: Waters Acquity UPLCMS SingleQuad; Column: Acquity UPLC BEH C18 1.7 µm, 50x2.1 mm; eluent A: water + 0.1 vol % formic acid (99%), eluent B: acetonitrile; gradient: 0-1.6 min 1-99% B, 1.6-2.0 min 99% B; flow 0.8 ml/min; temperature: 60 °C; DAD scan: 210-400 nm.

### Method b02:

Instrument: Waters Acquity UPLCMS SingleQuad; Column: Acquity UPLC BEH C18 1.7 µm, 50x2.1 mm; eluent A: water + 0.2 vol % aqueous ammonia (32%), eluent B: acetonitrile; gradient: 0-1.6 min 1-99% B, 1.6-2.0 min 99% B; flow 0.8 ml/min; temperature: 60 °C; DAD scan: 210-400 nm.

### Method HTpost_acid:

Instrument: Waters Acquity UPLCMS SingleQuad; Column: Acquity UPLC BEH C18 1.7 50x2.1 mm; eluent A: water + 0.1 vol % formic acid (99%), eluent B: acetonitrile; gradient: 0-1.6 min 1-99% B, 1.6-2.0 min 99% B; flow 0.8 ml/min; temperature: 60 °C; DAD scan: 210-400 nm

### Method HTpost_bas:

Instrument: Waters Acquity UPLCMS SingleQuad; Colum: Acquity UPLC BEH C18 1.7 50x2.1 mm; eluent A: water + 0.2 vol % aqueous ammonia (32%), eluent B: acetonitrile; gradient: 0-1.6 min 1-99% B, 1.6-2.0 min 99% B; flow 0.8 ml/min; temperature: 60 °C; DAD scan: 210-400 nm

The purification of selected compounds and compound mixtures was performed by the following methods:

### Method HT_PREP_acid:

Instrument: Waters Autopurificationsystem; Column: Waters XBrigde C18 5µ 100x30mm; Eluent A: water + 0.1 Vol-% formic acid (99%), Eluent B: acetonitrile, DAD scan: 210-400 nm

### Method HT_PREP_bas:

Instrument: Waters Autopurificationsystem; Column: Waters XBrigde C18 5µ 100x30mm; Eluent A: water + 0.2 Vol-% aqueous ammonia (32%), Eluent B: acetonitrile, DAD scan: 210-400 nm

### Standard workup procedure:

For some of the reactions a standard workup procedure was applied which can be adapted by a person skilled in the art.

Standard workup procedure as used and described in relation to the present invention and the synthesis of compounds of formula (I) means the addition of water to the reaction; subsequent followed by extraction of the mixture with ethyl acetate, mainly for three times, and concentration of the combined organic layers, for example with a rotary evaporator. In some cases, the residue was then dried in vacuum.

That procedure will be named in the following description of working protocols and experiments as standard workup procedure.

### Synthesis of intermediates:

Synthesis of selected amines R³-NH₂ (see general synthesis scheme 1):

### Intermediate 0-1

### trans-3-(methylsulfonyl)cyclobutanamine hydrochloride

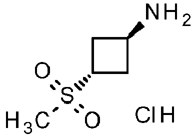

### Step a)

### cis-3-[(tert-butoxycarbonyl)amino]cyclobutyl methanesulfonate

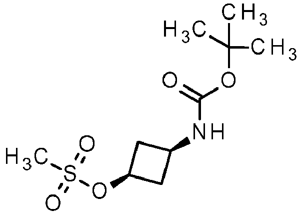

To a solution of tert-butyl (cis-3-hydroxycyclobutyl)carbamate (2.00 g, 10.7 mmol) in DCM (20 ml) were added triethylamine (2.2 ml, 16 mmol) and methanesulfonyl chloride (1.35 g, 11.7 mmol) at 0 °C. The mixture was stirred at rt for 2 h. The reaction mixture was diluted with DCM, washed with water and brine. The organic phase was dried over anhydrous sodium sulfate, filtered and the filtrate was concentrated to give cis-3-[(tert-butoxycarbonyl)amino]cyclobutyl methanesulfonate (2.60 g, 92% yield) as a yellow solid.
¹H NMR (400 MHz, CDCl₃) δ [ppm] = 4.76-4.60 (m, 2H), 3.92-3.77 (m, 1H), 2.99 (s, 3H), 2.96-2.83 (m, 2H), 2.26-2.13 (m, 2H), 1.44 (s, 9H).

### Step b)

### tert-butyl [trans-3-(methylsulfanyl)cyclobutyl]carbamate

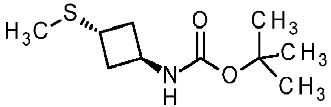

A mixture of cis-3-[(tert-butoxycarbonyl)amino]cyclobutyl methanesulfonate (2.80 g, 10.6 mmol) and sodium thiomethoxide (888 mg, 12.7 mmol) in N,N-dimethylformamide (30 ml) was stirred at room temperature for 12 hours. The mixture was diluted with ethyl acetate and washed with brine. The organic phase was concentrated and purified by silica gel chromatography (1000 mesh, petroleum ether: ethyl acetate = 1: 0, then 50: 1, then 20: 1) to give tert-butyl [trans-3-(methylsulfanyl)cyclobutyl]carbamate (1.15 g, 50% yield) as a yellow solid.
¹H NMR (400 MHz, CDCl₃) δ [ppm] = 4.87-4.61 (m, 1H), 4.43-4.25 (m, 1H), 3.39-3.27 (m, 1H), 2.44-2.18 (m, 4H), 2.05 (s, 3H), 1.44 (s, 9H).

### Step c)

### tert-butyl [trans-3-(methylsulfonyl)cyclobutyl]carbamate

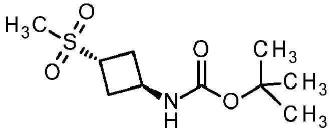

To a solution of tert-butyl [trans-3-(methylsulfanyl)cyclobutyl]carbamate
(850 mg, 3.91 mmol) in DCM (15 ml) was added meta-chloroperoxybenzoic acid (2.53 g, 80% purity, 11.7 mmol) at rt. The mixture was stirred at rt for 3 hours. Saturated sodium bicarbonate solution was added to the mixture and the mixture was stirred for 15 minutes. The mixture was diluted with DCM and washed with brine. The organic phase was concentrated and purified by silica gel chromatography (1000 mesh, petroleum ether: ethyl acetate = 5: 1, then 0: 1) to give tert-butyl [trans-3-(methylsulfonyl)cyclobutyl]carbamate (800 mg, 82% yield) as a yellow solid.
¹H NMR (400 MHz, DMSO-*d₆*) δ [ppm] = 7.29 (d, *J* = 7.6 Hz, 1H), 4.25-4.03 (m, 1H), 3.80-3.64 (m, 1H), 2.90 (s, 3H), 2.60-2.51 (m, 2H), 2.37-2.22 (m, 2H), 1.37 (s, 9H).

### Step d)

### trans-3-(methylsulfonyl)cyclobutanamine hydrochloride

A solution of tert-butyl [trans-3-(methylsulfonyl)cyclobutyl]carbamate (1.02 g, 4.09 mmol) in hydrochloric acid (15 ml, 4 M in ethyl acetate) was stirred at rt for 1 hour. The mixture was filtered and the filter cake was concentrated to give trans-3-(methylsulfonyl)cyclobutanamine hydrochloride (554 mg, 99% purity) as a white solid.

LC-MS (SIL_85_85AB_7MIN, Instrument: Agilent 1100 LC & Agilent G1956A; Software: Agilent Chemstation Rev. B. 04.03[54]; Column: Agilent ZORBAX RX-SIL, 4.6*150mm,5µm; eluent A: water + 0.0375 vol % trifluoroacetic acid, eluent B: acetonitrile + 0.01875 vol % trifluoroacetic acid; gradient: 0-7 min 85-85% B, flow 1 ml/min; temperature: 40 °C; DAD&ELSD): Rₜ = 2.661 min; MS (ESIpos): m/z = 150.1
¹H NMR (400 MHz, DMSO-*d₆*) δ [ppm] = 8.59 (s, 3H), 4.04-3.93 (m, 1H), 3.80-3.67 (m, 1H), 2.96 (s, 3H), 2.61 (t, *J* = 7.6 Hz, 4H).

### Intermediate 0-2

### trans-3-(ethylsulfonyl)cyclobutanamine hydrochloride

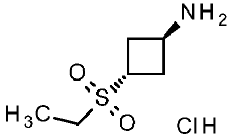

### Step a)

### tert-butyl [trans-3-(ethylsulfanyl)cyclobutyl]carbamate

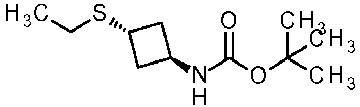

A mixture of cis-3-[(tert-butoxycarbonyl)amino]cyclobutyl methanesulfonate (2.74 g, 10.3 mmol) and sodium ethanethiolate (1.04 g, 12.4 mmol) in N,N-dimethylformamide (40 ml) was stirred at rt for 12 hours. The mixture was diluted with ethyl acetate and washed with brine. The organic phase was concentrated and purified by silica gel chromatography (1000 mesh, petroleum ether: ethyl acetate = 1: 0, then 100: 1, then 20: 1) to give tert-butyl [trans-3-(ethylsulfanyl)cyclobutyl]carbamate (1.70 g, 71% yield) as a yellow solid.
¹H NMR (400 MHz, DMSO-*d₆*) δ [ppm] = 7.23 (d, *J* = 7.6 Hz, 1H), 4.26-4.03 (m, 1H), 3.40-3.32 (m, 1H), 2.45 (q, *J* = 7.2 Hz, 2H), 2.33-2.21 (m, 2H), 2.12-2.00 (m, 2H), 1.37 (s, 9H), 1.15 (t, *J* = 7.6 Hz, 3H).

### Step b)

### tert-butyl [trans-3-(ethylsulfonyl)cyclobutyl]carbamate

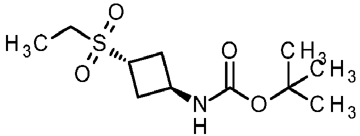

To a solution of tert-butyl [trans-3-(ethylsulfanyl)cyclobutyl]carbamate (1.70 g, 7.35 mmol) in DCM (30 ml) was added meta-chloroperoxybenzoic acid (4.75 g, 80% purity, 22.0 mmol) at rt. The mixture was stirred at rt for 3 hours. Saturated sodium bicarbonate solution was added to the mixture and the mixture was stirred for 15 minutes. The mixture was diluted with DCM and washed with brine. The organic phase was concentrated and purified by silica gel chromatography (1000 mesh, petroleum ether: ethyl acetate = 5: 1, then 0: 1) to give tert-butyl [trans-3-(ethylsulfonyl)cyclobutyl]carbamate (1.34 g, 69% yield) as a yellow solid.
¹H NMR (400 MHz, DMSO-*d₆*) δ [ppm] = 7.31 (d, *J* = 8 Hz, 1H), 4.20-4.05 (m, 1H), 3.81-3.69 (m, 1H), 3.03 (q, *J* = 7.6 Hz, 2H), 2.61-2.51 (m, 2H), 2.38-2.24 (m, 2H), 1.37 (s, 9H), 1.18 (t, *J* = 7.6 Hz, 3H).

### Step c)

A solution of tert-butyl [trans-3-(ethylsulfonyl)cyclobutyl]carbamate (1.34 g, 5.09 mmol) in hydrochloric acid (20 ml, 4 M in ethyl acetate) was stirred at rt for 1 hour. The mixture was filtered and the filter cake was concentrated to give trans-3-(ethylsulfonyl)cyclobutanamine hydrochloride (719 mg, 99% purity, 70% yield) as a white solid.

LC-MS (SIL_85_85AB_7MIN, Instrument: Agilent 1100 LC & Agilent G1956A; Software: Agilent Chemstation Rev. B. 04.03[54]; Column: Agilent ZORBAX RX-SIL, 4.6*150mm,5µm; eluent A: water + 0.0375 vol % trifluoroacetic acid, eluent B: acetonitrile + 0.01875 vol % trifluoroacetic acid; gradient: 0-7 min 85-85% B, flow 1 ml/min; temperature: 40 °C; DAD&ELSD): Rₜ = 2.553 min; MS (ESIpos): m/z = 164.1
¹H NMR (400 MHz, DMSO-*d₆*) δ [ppm] = 8.61 (s, 3H), 4.12-3.98 (m, 1H), 3.83-3.65 (m, 1H), 3.08 (q, *J* = 7.2 Hz, 2H), 2.65-2.55 (m, 4H), 1.18 (t, *J* = 7.2 Hz, 3H).

### Intermediate 0-3

### 1-(ethylsulfonyl)azetidin-3-amine trifluoroacetate

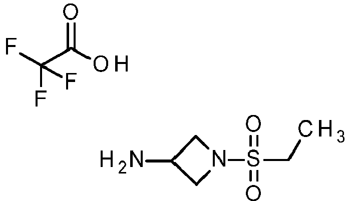

Ethanesulfonyl chloride (250 µl) was added to tert-butyl azetidin-3-ylcarbamate (300 mg) in DCM (4.0 ml). Then DIPEA (0.91 ml) was added and the mixture was stirred at RT for 65 h. The mixture was diluted with water and extracted with ethyl acetate three times. The combined organic layers were washed with aqueous hydrochloric acid (1M), saturated sodium hydrogen carbonate solution, filtered and evaporated. DCM (4.0 ml) and TFA (1.0 ml) were added and the mixture was stirred at RT for 21 h. Evaporation afforded the title compound as a crude product, which was used without further purification.

### Intermediate 0-4

### 1-(cyclopropylsulfonyl)azetidin-3-amine trifluoroacetate

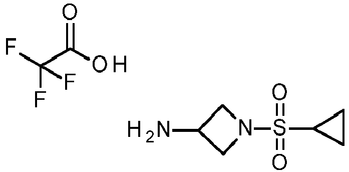

The synthesis was preformed in a similar fashion using cyclopropanesulfonyl chloride

### Synthesis of intermediates (see synthesis scheme 1):

### Intermediate 1-01

### 4,6-dichloro-1-[3-(trifluoromethyl)phenyl]-1H-pyrazolo[3,4-d]pyrimidine

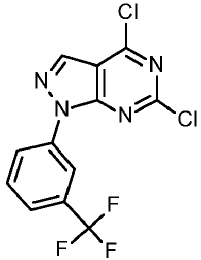

To a solution of 2,4,6-trichloropyrimidine-5-carbaldehyde (29.8 g, 141 mmol) in ethanol (500 ml) was added (3-(trifluoromethyl)phenyl)hydrazine hydrochloride (30.0 g, 141 mmol, CAS number 3107-33-3) at -78 °C, followed by the addition of triethylamine (42.8 g, 423 mmol) at -78 °C. The resulting mixture was stirred at this temperature for 30 min and warmed up to 0 °C. The mixture was stirred at 0 °C for 2 h. Upon completion of the reaction, the solvent was removed under vacuum. The residue was purified by silica gel column chromatography (ethyl acetate / petroleum ether) and re-purified by recrystallization from n-hexane/ethyl acetate to give 8.13 g of the product as a yellow solid.
¹H-NMR (400 MHz, DMSO-*d₆*): *δ* [*ppm*] = 7.85-7.88 (m, 2H), 8.31-8.40 (m, 2H), 8.87 (s, 1H).

### Intermediate 1-02

### 4,6-dichloro-1-[3-(difluoromethyl)phenyl]-1H-pyrazolo[3,4-d]pyrimidine

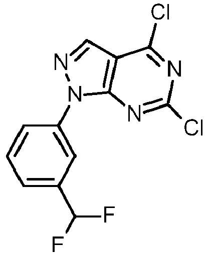

### Step a)

### 1-(difluoromethyl)-3-nitrobenzene

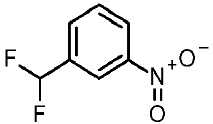

To a solution of 3-nitrobenzaldehyde (50.0 g, 331 mmol) in DCM (800 ml) was added diethylaminosulfur trifluoride (130 ml, 990 mmol) at -78 °C. After stirring at rt for 2 h, the reaction mixture was poured into ice-water and extracted with DCM. The organic phase was concentrated and purified by silica gel chromatography (1000 mesh, petroleum ether: ethyl acetate = 1: 0, then 100: 1) to give 1-(difluoromethyl)-3-nitrobenzene (55.0 g) as yellow oil.
¹H NMR (400 MHz, DMSO-*d₆*): δ [ppm] = 8.42-8.38 (m, 2H), 8.06 (d, *J* = 8.0 Hz, 1H), 7.86-7.82 (m, 1H), 7.22 (t, *J* = 55.2 Hz, 1H).

### Step b)

### 3-(difluoromethyl)aniline

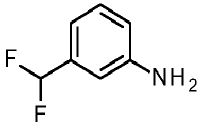

To a solution of 1-(difluoromethyl)-3-nitrobenzene (55.0 g, 318 mmol) in a mixed solvent of methanol (500 ml) and water (200 ml) were added iron (53.2 g, 953 mmol) and ammonium chloride (85.0 g, 1.59 mol). The reaction mixture was stirred at 70 °C for 12 h. The reaction mixture was cooled to rt and filtered. The filtrate was diluted with water and extracted with ethyl acetate. The organic phase was washed with brine, dried over anhydrous sodium sulfate and filtered. The filtrate was concentrated under reduced pressure to give 3-(difluoromethyl)aniline (42.0 g) as yellow oil.
¹H NMR (400 MHz, DMSO-*d₆*): δ [ppm] = 7.11 (t, *J* = 8.0 Hz, 1H), 6.82 (t, *J* = 56.4 Hz, 1H), 6.72-6.62 (m, 3H), 5.35 (s, 2H).

### Step c)

### [3-(difluoromethyl)phenyl]hydrazine

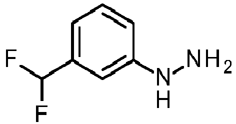

To a solution of 3-(difluoromethyl)aniline (8.50 g, 59.4 mmol) in hydrochloric acid (40 ml, 12 M, 0.5 mol) was added a solution of sodium nitrite (4.10 g, 59.4 mmol) in water (20 ml) dropwise at -5 - 0 °C. The reaction mixture was stirred at -5 - 0 °C for 1 h. To the reaction mixture was added a solution of tin(II) chloride dihydrate (40.2 g, 178 mmol) in hydrochloric acid (100 ml, 12 M, 1.2 mol) dropwise at -5 - 0 °C. After stirring at the same temperature for 4 h, the reaction mixture was combined with the mixture of a parallel reaction using the same amounts of starting materials and conditions as described before. After filtration and washing the filter cake with hydrochloric acid (1M), the filter cake was dissolved in water. Sodium hydroxide solution (4M) was added to adjust pH∼10. The mixture was extracted with ethyl acetate. The organic phase was washed with brine, dried over anhydrous sodium sulfate, filtered and concentrated to give a residue. The residue was purified by silica gel chromatography (1000 mesh, petroleum ether: ethyl acetate = 10: 1, 5: 1, then 2: 1) to give [3-(difluoromethyl)phenyl]hydrazine (6.0 g) as a yellow solid.
¹H NMR (400 MHz, DMSO-*d₆*): δ [ppm] = 7.20 (t, *J* = 8.0 Hz, 1H), 6.98 (d, *J* = 8.0 Hz, 2H), 6.92-6.87 (m, 1H), 6.87 (t, *J* = 56.0 Hz, 1H), 6.72 (s, 1H), 4.11 (br.s, 2H).

### Step d)

To a solution of 2,4,6-trichloropyrimidine-5-carbaldehyde (6.02 g, 28.5 mmol) in ethanol (50 ml) was added a solution of [3-(difluoromethyl)phenyl]hydrazine (4.50 g, 28.5 mmol) in ethanol (40 ml) dropwise, followed by triethylamine (12 ml, 85 mmol) at -78 °C. After stirring at -78 °C for 0.5 h and 0 °C for 2 h, the mixture w as concentrated at rt to give a residue. The residue was diluted with ethyl acetate, washed with water and brine. The organic phase was concentrated and purified by silica gel chromatography (1000 mesh, petroleum ether: ethyl acetate = 10:1, then 5:1) to give 4,6-dichloro-1-[3-(difluoromethyl)phenyl]-1H-pyrazolo[3,4-d]pyrimidine (2.95 g) as yellow oil.
LC-MS (Method **C**): Rₜ = 1.009 min; MS (ESIpos): m/z = 315.0 [M+H]+.
¹H NMR (400 MHz, DMSO-*d₆*): δ [ppm] = 0.84 (s, 1H), 8.30-8.24 (m, 2H), 7.82-7.77 (m, 1H), 7.66 (d, *J* = 7.6 Hz, 1H), 7.20 (t, *J* = 55.6 Hz, 1H).

### Intermediate 1-03

### 4,6-dichloro-1-[3-(trifluoromethoxy)phenyl]-1H-pyrazolo[3,4-d]pyrimidine

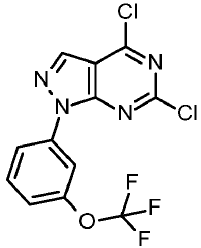

To a solution of 2,4,6-trichloropyrimidine-5-carbaldehyde (7.15 g, 33.8 mmol) in ethanol (70 ml) was added a solution of [3-(trifluoromethoxy)phenyl]hydrazine (6.50 g, 33.8 mmol) in ethanol (70 ml) dropwise, followed by triethylamine (14 ml, 100 mmol) at -78 °C. After stirring at -78 °C for 0.5 h and 0 °C for 0.5 h, the mixture was concentrated at rt to give a residue. The residue was diluted with ethyl acetate, washed with water and brine. The organic phase was concentrated and purified by silica gel chromatography (1000 mesh, petroleum ether: ethyl acetate = 1: 0, then 100: 1) to give 4,6-dichloro-1-[3-(trifluoromethoxy)phenyl]-1H-pyrazolo[3,4-d]pyrimidine (630 mg) as a yellow solid.
LC-MS (Method C): Rₜ = 1.059 min; MS (ESIpos): m/z = 349.0
¹H NMR (400 MHz, DMSO-*d₆*) δ [ppm] = 8.87 (s, 1H), 8.18-8.15 (m, 1H), 8.10-8.06 (m, 1H), 7.79 (t, *J* = 8.0 Hz, 1H), 7.51-7.46 (m, 1H).

### Intermediate 1-04

### 4,6-dichloro-1-[3-(difluoromethoxy)phenyl]-1H-pyrazolo[3,4-d]pyrimidine

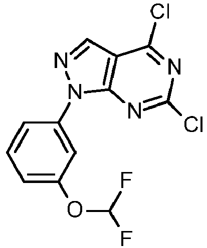

### Step a)

### [3-(difluoromethoxy)phenyl]hydrazine

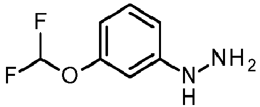

To a solution of 3-(difluoromethoxy)aniline (5.00 g, 31.4 mmol) in hydrochloric acid (20 ml, 12 M, 240 mmol) was added a solution of sodium nitrite (2.17 g, 31.4 mmol) in water (10 ml) dropwise at 0 °C. After stirring at 0 °C for 1 h, a solution of tin(II) chloride in hydrochloric acid (50 ml, 12 M, 600 mmol) was added dropwise. The reaction mixture was stirred at 0 °C for 4 h. The reaction mixture was filtered and the filter cake was washed with hydrochloric acid (1M). The filter cake was dissolved in water. Sodium hydroxide (4 M) was added to adjust pH∼10. The mixture was extracted with ethyl acetate. The organic phase was washed with brine, dried over anhydrous sodium sulfate, filtered and concentrated to give a residue. The residue was purified by silica gel chromatography (1000 mesh, petroleum ether: ethyl acetate = 10: 1, then 5: 1) to give [3-(difluoromethoxy)phenyl]hydrazine (3.40 g) as a light yellow solid.
¹H NMR (400 MHz, CDCl₃): δ [ppm] = 7.19 (t, *J* = 8.0 Hz, 1H), 6.65-6.62 (m, 2H), 6.55-6.53 (m, 1H), 6.51 (t, *J* = 76.0 Hz, 1H), 5.31 (s, 1H), 3.59 (s, 2H).

### Step b)

To a solution of 2,4,6-trichloropyrimidine-5-carbaldehyde (2.91 g, 13.8 mmol) in ethanol (20 ml) was added a solution of [3-(difluoromethoxy)phenyl]hydrazine (2.40 g, 13.8 mmol) in ethanol (30 ml) dropwise, followed by triethylamine (5.8 ml, 41 mmol) at -78 °C. The mixture was stirred at -78 °C for 0.5 h and 0 °C for 2 h. The mixture was concentrated below 30 °C to give a residue. The residue was diluted with ethyl acetate, washed with water and brine. The organic phase was concentrated and purified by silica gel chromatography (1000 mesh, petroleum ether: ethyl acetate = 10: 1, then 5: 1) to give 4,6-dichloro-1-[3-(difluoromethoxy)phenyl]-1H-pyrazolo[3,4-d]pyrimidine (1.6 g) as a yellow solid.
LC-MS (Method **C**): Rₜ = 1.111 min; MS (ESIpos): m/z = 331.3 [M+H]+.
¹H NMR (400 MHz, DMSO-*d₆*): δ [ppm] = 8.87 (s, 1H), 8.02 (dd, *J* = 8.4, 1.2 Hz, 1H), 7.90 (t, *J* = 2.0 Hz, 1H), 7.71 (t, *J* = 8.0 Hz, 1H), 7.37 (t, *J* = 73.6 Hz, 1H), 7.30 (dd, *J* = 8.0, 2.0 Hz, 1H).

### Intermediate 1-05

### 4,6-dichloro-1-[3-(methylsulfonyl)phenyl]-1H-pyrazolo[3,4-d]pyrimidine

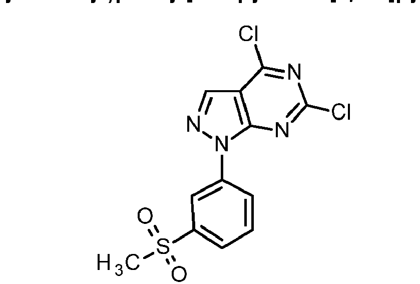

### Step a)

### (3-(methylsulfonyl)phenyl)hydrazine

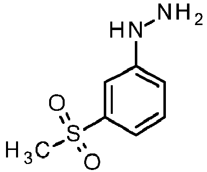

To a solution of 3-(methylsulfonyl)aniline (12.0 g, 70.1 mmol) in hydrochloric acid (300 mL) was added a solution of sodium nitrite (5.8 g, 84.1 mmol) in water (100 mL) dropwise with stirring at -20 °C ∼ -10 °C for 1 h. To the reaction mixture was added a solution of tin(II) chloride dihydrate (47.4 g, 210 mmol) in hydrochloric acid (200 mL) slowly. The mixture was stirred at -20 °C ∼ -10 °C for 2 h. Upon completion of the reaction, the mixture was basified to pH = 7 with sodium hydrogen carbonate and extracted with ethyl acetate. The organic layers were dried over anhydrous sodium sulfate and concentrated in vacuo to give 6.0 g of the crude product. The residue was used for the next step without further purification.

### Step b)

To a solution of 2,4,6-trichloropyrimidine-5-carbaldehyde (3.4 g, 16.1 mmol) in tetrahydrofuran (60 mL) was added a solution of (3-(methylsulfonyl)phenyl)hydrazine (3.0 g, 16.1 mmol) in tetrahydrofuran/water (40 mL, v: v = 9: 1) at -78 °C . Triethylamine (6.7 mL, 48.3 mmol) was added dropwise and the mixture was stirred at -78 °C for 30 min. Then the mixture was warmed to -10 °C and stirred for further 1 h. Upon completion of the reaction, the solvent was removed in vacuo. The residue was purified with silica gel column chromatography (petroleum ether: ethyl acetate = 3: 1) to give 518 mg (82% purity) 4,6-dichloro-1-(3-(methylsulfonyl)phenyl)-1*H*-pyrazolo[3,4-*d*]pyrimidine as a light yellow solid.
¹H-NMR (300 MHz, DMSO-*d₆*): *δ* [*ppm*] = 3.34 (s, 3H), 7.93-8.05 (m, 3H), 8.50-8.51 (m, 1H), 8.93 (s, 1H).

### Intermediate 1-06

### ethyl 3-(4,6-dichloro-1H-pyrazolo[3,4-d]pyrimidin-1-yl)benzoate

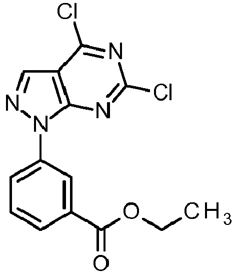

To a solution of 2,4,6-trichloropyrimidine-5-carbaldehyde (3.9 g, 18.5 mmol) in tetrahydrofuran (100 mL) was added dropwise a solution of ethyl 3-hydrazinobenzoate hydrochloride (4.0 g, 18.5 mmol) in tetrahydrofuran/water (50 mL, v: v = 9: 1) at -78 °C under nitrogen atmosphere. Then triethylamine (7.7 mL, 55.4 mmol) was added dropwise and the mixture was stirred at -78 °C for 30 minutes under nitrogen atmosphere. Then the mixture was warmed to -10 °C and stirred for further 2 h. Upon completion of the reaction, the solvent was removed in vacuo. The residue was purified with silica gel column chromatography (ethyl acetate: petroleum ether = 1: 10) to give 1.01 g of the product (batch contained impurities) as a light yellow solid.
MS (ESIpos): m/z = 337 (M+H)+

### Intermediate 2-I-01

### 6-chloro-4-[3-(morpholin-4-yl)azetidin-1-yl]-1-[3-(trifluoromethyl)phenyl]-1H-pyrazolo[3,4-d]pyrimidine

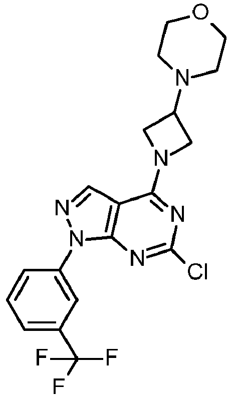

A solution of 4,6-dichloro-1-[3-(trifluoromethyl)phenyl]-1H-pyrazolo[3,4-d]pyrimidine (1.20 g, 3.60 mmol) and 4-(azetidin-3-yl)morpholine dihydrochloride (852 mg, 3.96 mmol) in NMP (30 ml) was treated with DIPEA (1.9 ml, 11 mmol) and the mixture was stirred at rt for 16 h. Water was added to the reaction mixture and the precipitated solid was filtered off with vacuum and dried to afford 1.51 g (95%) of the title compound as a white solid.
¹H-NMR (400 MHz, DMSO-d6) δ [ppm]: 2.420 (3.94), 2.429 (4.08), 2.518 (3.15), 2.523 (2.12), 3.391 (0.42), 3.403 (0.95), 3.408 (1.04), 3.420 (1.81), 3.432 (1.07), 3.437 (1.09), 3.449 (0.50), 3.618 (4.95), 3.629 (8.35), 3.641 (4.92), 4.113 (1.03), 4.126 (1.04), 4.140 (1.37), 4.151 (1.31), 4.283 (1.36), 4.301 (1.46), 4.308 (1.22), 4.327 (0.98), 4.404 (0.97), 4.416 (1.03), 4.428 (1.40), 4.440 (1.31), 4.534 (1.31), 4.553 (1.54), 4.575 (0.90), 7.737 (1.90), 7.756 (3.01), 7.811 (1.92), 7.813 (1.92), 7.833 (2.79), 7.853 (1.26), 7.855 (1.25), 8.400 (16.00), 8.443 (2.68), 8.454 (4.63), 8.458 (5.27).

### Intermediate 2-I-02

### 2-(1-16-chloro-1-[3-(trifluoromethyl)phenyl]-1H-pyrazolo[3,4-d]pyrimidin-4-yl)piperidin-4-yl)propan-2-ol

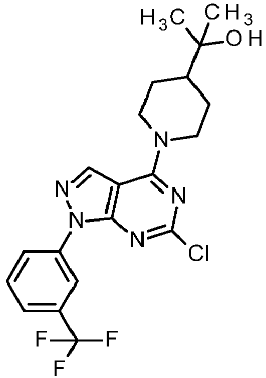

A solution of 4,6-dichloro-1-[3-(trifluoromethyl)phenyl]-1H-pyrazolo[3,4-d]pyrimidine (2.00 g, 6.00 mmol) and 2-(piperidin-4-yl)propan-2-ol (946 mg, 6.60 mmol) in NMP (30 ml) was treated with DIPEA (3.1 ml, 18 mmol) and the mixture was stirred at rt for 16h. Water was added to the reaction mixture and the mixture was extracted with ethyl acetate three times, the organic layers were filtered using a water-repellent filter and concentrated. Purification by column chromatography (Biotage ULTRA 50g column, eluent: hexane / ethyl acetate) afforded 2.65 g of the title compound.
LC-MS (method a01): Rₜ = 1.49 min; MS (ESIpos): m/z = 440 [M+H]⁺

### Intermediate 2-I-03

### 1-{6-chloro-1-[3-(trifluoromethyl)phenyl]-1H-pyrazolo[3,4-d]pyrimidin-4-yl}-3-ethylazetidin-3-ol

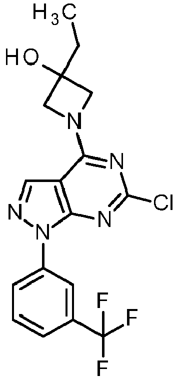

A solution of 4,6-dichloro-1-[3-(trifluoromethyl)phenyl]-1H-pyrazolo[3,4-d]pyrimidine (500 mg, 1.50 mmol) and 3-ethylazetidin-3-ol hydrochloride (227 mg, 1.65 mmol) in NMP (10 ml) was treated with potassium carbonate (622 mg, 4.50 mmol) and the mixture was stirred at rt for 16h. Water was added to the reaction mixture and the mixture was extracted with ethyl acetate three times, the organic layers were filtered using a water-repellent filter and concentrated. Purification by column chromatography (Biotage ULTRA 10g column, eluent: hexane / ethyl acetate) and subsequent purification by HPLC afforded 330 mg of the title compound.
LC-MS (method a01): Rₜ = 1.38 min; MS (ESIpos): m/z = 398 [M+H]⁺

### Intermediate 2-I-04

### (3S,4S)-1-{6-chloro-1-[3-(trifluoromethyl)phenyl]-1H-pyrazolo[3,4-d]pyrimidin-4-yl}pyrrolidine-3,4-diol

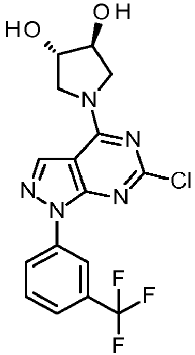

A solution of 4,6-dichloro-1-[3-(trifluoromethyl)phenyl]-1H-pyrazolo[3,4-d]pyrimidine (370 mg, 1.11 mmol) and (3S,4S)-pyrrolidine-3,4-diol (126 mg, 1.22 mmol) in NMP (7.4 ml) was treated with DIPEA (0.58 ml, 3.3 mmol) and the mixture was stirred at rt. Water was added to the reaction mixture and the mixture was extracted with ethyl acetate three times, the organic layers were filtered using a water-repellent filter and concentrated. Purification by column chromatography (Biotage ULTRA 10g column, eluent: hexane / ethyl acetate) afforded 350 mg of the title compound.
LC-MS (method a01): Rₜ = 1.16 min; MS (ESIpos): m/z = 400 [M+H]⁺

### Intermediate 2-I-05

### 6-chloro-4-[(3R)-3-(morpholin-4-yl)pyrrolidin-1-yl]-1-[3-(trifluoromethyl)phenyl]-1H-pyrazolo[3,4-d]pyrimidine

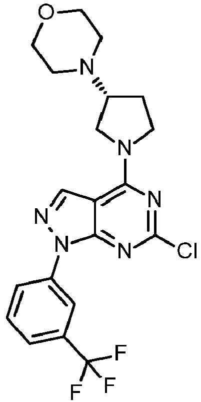

A solution of 4,6-dichloro-1-[3-(trifluoromethyl)phenyl]-1H-pyrazolo[3,4-d]pyrimidine (300 mg, 901 µmol) and 4-[(3R)-pyrrolidin-3-yl]morpholine hydrochloride (191 mg, 991 µmol) in NMP (4.5 ml) was treated with DIPEA (470 µl, 2.7 mmol) and the mixture was stirred at rt for 16h. Water was added to the reaction mixture and the mixture was extracted with ethyl acetate three times, the organic layers were filtered using a water-repellent filter and concentrated. The crude product was purified by column chromatography (Biotage ULTRA 25g column, eluent: hexane / ethyl acetate) to afford 450 mg of the title compound (crude batch).
LC-MS (method b02): Rₜ = 1.41 min; MS (ESIpos): m/z = 453 [M+H]⁺

### Intermediate 2-I-06

### methyl (1-{6-chloro-1-[3-(trifluoromethyl)phenyl]-1H-pyrazolo[3,4-d]pyrimidin-4-yl}azetidin-3-yl)acetate

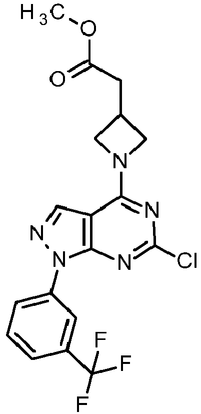

To a mixture of 4,6-dichloro-1-[3-(trifluoromethyl)phenyl]-1H-pyrazolo[3,4-d]pyrimidine (500 mg, 1.50 mmol) and methyl azetidin-3-ylacetate trifluoroacetate (365 mg, 1.50 mmol) in NMP (4.0 ml) was added DIPEA (0.78 ml, 4.5 mmol) and the mixture was stirred for 19 h at rt. Water was added and the mixture was extracted with ethyl acetate three times and the combined organic layers were concentrated. The residue was treated with DMSO, the resulting solid was filtered, washed with water and 2-methoxy-2-methylpropane and dried. A solid precipitated from the mother liquor, which was filtered, washed with water and 2-methoxy-2-methylpropane and dried. Both solids were combined to afford 148 mg of title compound. Another batch of the title compound (248 mg) was obtained by purification of the remaining mother liquor by HPLC.
LC-MS (method a01): Rₜ = 1.43 min; MS (ESIpos): m/z = 426 [M+H]⁺
¹H-NMR (400 MHz, DMSO-d6) δ [ppm]: 2.074 (0.42), 2.522 (1.31), 2.838 (3.10), 2.857 (3.50), 3.164 (0.49), 3.184 (0.67), 3.204 (0.48), 3.628 (16.00), 3.958 (0.64), 3.973 (0.66), 3.983 (0.75), 3.997 (0.69), 4.217 (0.63), 4.231 (0.70), 4.240 (0.76), 4.255 (0.66), 4.354 (0.72), 4.377 (1.10), 4.400 (0.61), 4.650 (0.67), 4.672 (1.23), 4.694 (0.59), 7.735 (0.98), 7.754 (1.54), 7.811 (0.94), 7.832 (1.42), 7.853 (0.64), 8.393 (6.42), 8.454 (2.44), 8.458 (2.60).

### Intermediate 2-I-07

### 1-(1-{6-chloro-1-[3-(trifluoromethyl)phenyl]-1H-pyrazolo[3,4-d]pyrimidin-4-yl}piperidin-4-yl)-2-methylpropan-2-ol

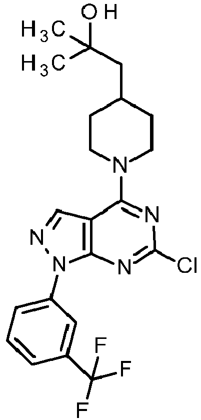

DIPEA (1.3 ml, 7.5 mmol) was added to 4,6-dichloro-1-[3-(trifluoromethyl)phenyl]-1H-pyrazolo[3,4-d]pyrimidine (500 mg, 1.50 mmol) and 2-methyl-1-(piperidin-4-yl)propan-2-ol hydrochloride (291 mg, 1.50 mmol)] in NMP (4.0 ml, 42 mmol) and the mixture was stirred for 15 h at rt. Water was added and the mixture was extracted three times with ethyl acetate. The combined organic layers were concentrated and the residue was purified by HPLC affording 540 mg of the title compound.
LC-MS (method a01): Rₜ = 1.55 min; MS (ESIpos): m/z = 454 [M+H]⁺

### Intermediate 2-I-08

### 6-chloro-4-[3-(1,1-dioxidothiomorpholin-4-yl)azetidin-1-yl]-1-[3-(trifluoromethyl)phenyl]-1H-pyrazolo[3,4-d]pyrimidine

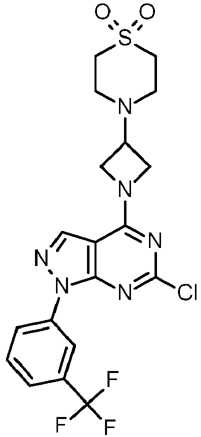

A solution of 4,6-dichloro-1-[3-(trifluoromethyl)phenyl]-1H-pyrazolo[3,4-d]pyrimidine (299 mg, 898 µmol) and 4-(azetidin-3-yl)thiomorpholine 1,1-dioxide dihydrochloride (260 mg, 988 µmol) in NMP (5.0 ml) was treated with DIPEA (470 µl, 2.7 mmol) and the mixture was stirred at rt for 16 h. Water was added to the reaction mixture and the mixture was extracted with ethyl acetate three times, the organic layers were filtered using a water-repellent filter and concentrated. 2-methoxy-2-methylpropane was added, the precipitated solid was separated by filtration, washed with 2-methoxy-2-methylpropane and dried affording 210 mg of the title compound.
LC-MS (method a01): Rₜ = 1.29 min; MS (ESIpos): m/z = 487 [M+H]⁺

### Intermediate 2-I-09

### 6-chloro-4-[4-(morpholin-4-yl)piperidin-1-yl]-1-[3-(trifluoromethyl)phenyl]-1H-pyrazolo[3,4-d]pyrimidine

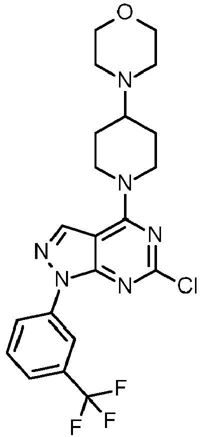

A solution of 4,6-dichloro-1-[3-(trifluoromethyl)phenyl]-1H-pyrazolo[3,4-d]pyrimidine (300 mg, 901 µmol) in 2-methyltetrahydrofuran (5.0 ml) was treated with DIPEA (470 µl, 2.7 mmol). The solution was cooled down with a cold water bath, 4-(piperidin-4-yl)morpholine (169 mg, 991 µmol) was added in portions and the mixture was stirred at rt for 16h. Water was added and the mixture was extracted with ethyl acetate three times, the organic layers were filtered using a water-repellent filter and concentrated. 400 mg of the crude title compound was obtained which was used without further purification.

### Intermediate 2-I-10

### 6-chloro-4-(morpholin-4-yl)-1-[3-(trifluoromethyl)phenyl]-1H-pyrazolo[3,4-d]pyrimidine

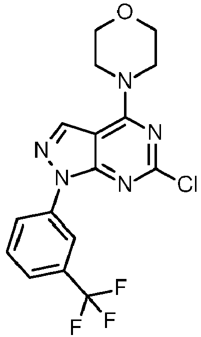

A solution of 4,6-dichloro-1-[3-(trifluoromethyl)phenyl]-1H-pyrazolo[3,4-d]pyrimidine (300 mg, 901 µmol) in 2-methyltetrahydrofuran (5.0 ml) was treated with DIPEA (470 µl, 2.7 mmol). The solution was cooled down with a cold water bath, morpholine (87 µl, 990 µmol) was added in portions and the mixture was stirred at rt for 16h. Water was added to the reaction mixture and the mixture was extracted with ethyl acetate three times, the organic layers were filtered using a water-repellent filter and concentrated.

2-methoxy-2-methylpropane was added, the precipitated solid was separated by filtration, washed with 2-methoxy-2-methylpropane and dried affording 260 mg of the title compound which was used without purification.

### Intermediate 2-1-11

### 1-(1-{6-chloro-1-[3-(trifluoromethyl)phenyl]-1H-pyrazolo[3,4-d]pyrimidin-4-yl}azetidin-3-yl)-4-methylpiperidin-4-ol

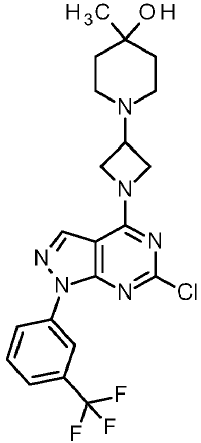

A solution of [4,6-dichloro-1-[3-(trifluoromethyl)phenyl]-1H-pyrazolo[3,4-d]pyrimidine (200 mg, 600 µmol) and 1-(azetidin-3-yl)-4-methylpiperidin-4-ol dihydrochloride (161 mg, 660 µmol) in NMP was treated with DIPEA and the mixture was stirred at rt for 72 h. Water was added to the reaction mixture and the mixture was extracted with ethyl acetate three times, the organic layers were filtered using a water-repellent filter and concentrated. Purification by column chromatography (Biotage ULTRA 10 g column, eluent: hexane / ethyl acetate) afforded the title compound (225 mg).
LC-MS (method b02): Rₜ = 1.31 min; MS (ESIpos): m/z = 467 [M+H]⁺

### Intermediate 2-I-12

### (3R,4R)-1-{6-chloro-1-[3-(trifluoromethyl)phenyl]-1H-pyrazolo[3,4-d]pyrimidin-4-yl}pyrrolidine-3,4-diol

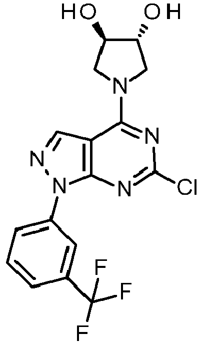

A solution of 4,6-dichloro-1-[3-(trifluoromethyl)phenyl]-1H-pyrazolo[3,4-d]pyrimidine (800 mg, 2.40 mmol) and (3R,4R)-pyrrolidine-3,4-diol (272 mg, 2.64 mmol) in 2-methyltetrahydrofuran (8.0 ml) was treated with DIPEA (1.3 ml, 7.2 mmol) and the mixture was stirred at rt for 16 h. Standard workup and purification by column chromatography (Biotage ULTRA 25g column; eluent: ethyl acetate / ethanol) afforded 455 mg of the title compound.

### Intermediate 2-I-13

### 1-{6-chloro-1-[3-(trifluoromethyl)phenyl]-1H-pyrazolo[3,4-d]pyrimidin-4-yl}azetidin-3-ol

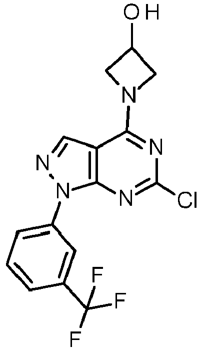

DIPEA (470 µl, 2.7 mmol) was added to 4,6-dichloro-1-[3-(trifluoromethyl)phenyl]-1H-pyrazolo[3,4-d]pyrimidine (300 mg, 901 µmol) and azetidin-3-ol hydrochloride (98.7 mg, 901 µmol) in 2-methyltetrahydrofuran (3.0 ml) and the mixture was stirred at rt for 15.5 h. Standard workup and purification of the crude product by HPLC (water + 0.1% formic acid/ acetonitrile) afforded 283 mg of the title compound

### Intermediate 2-I-14

### 1-{6-chloro-1-[3-(trifluoromethyl)phenyl]-1H-pyrazolo[3,4-d]pyrimidin-4-yl}-3-methylazetidin-3-ol

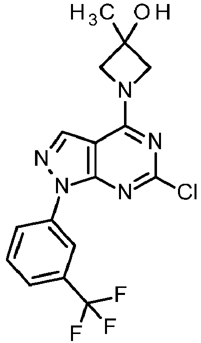

To solution of 4,6-dichloro-1-[3-(trifluoromethyl)phenyl]-1H-pyrazolo[3,4-d]pyrimidine (800 mg) in 2-methyltetrahydrofuran (15 ml) were added DIPEA (1.0 ml) and 3-methylazetidin-3-ol (174 mg) at 0° C. The mixture was stirred at rt for 12 h. Water was added to the reaction mixture and the mixture was extracted with ethyl acetate, the organic layers were dried over anhydrous sodium sulfate and concentrated to give a residue. The residue was purified by flash column chromatography (petroleum ether / ethyl acetate) to give 312 mg of the title compound as a yellow solid.

### Intermediate 2-I-15

### tert-butyl [2-(1-{6-chloro-1-[3-(trifluoromethyl)phenyl]-1H-pyrazolo[3,4-d]pyrimidin-4-yl}azetidin-3-yl)propan-2-yl]carbamate

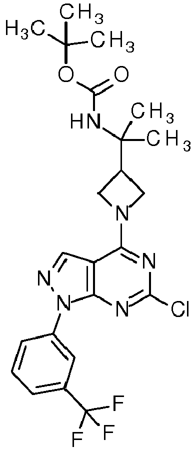

A solution of 4,6-dichloro-1-[3-(trifluoromethyl)phenyl]-1H-pyrazolo[3,4-d]pyrimidine (500 mg, 1.50 mmol) and tert-butyl [2-(azetidin-3-yl)propan-2-yl]carbamate hydrochloride (414 mg, 1.65 mmol) in 2-methyltetrahydrofuran (7.0 ml) was treated with DIPEA (0.78 ml) and the mixture was stirred at rt for 16 h. Standard workup and purification (Biotage ULTRA 25 g column, hexane / ethyl acetate) afforded the title compound (750 mg).

### Intermediate 2-I-16

### 1-{6-chloro-1-[3-(trifluoromethyl)phenyl]-1H-pyrazolo[3,4-d]pyrimidin-4-yl}-N,N-dimethylazetidin-3-amine

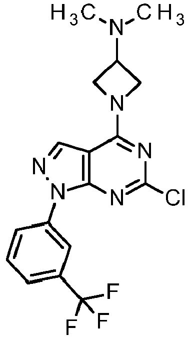

To solution of 4,6-dichloro-1-[3-(trifluoromethyl)phenyl]-1H-pyrazolo[3,4-d]pyrimidine (800 mg) in 2-methyltetrahydrofuran (15 ml) were added DIPEA (1.54 ml, 8.84 mmol) and N,N-dimethylazetidin-3-amine hydrochloride (302 mg, 2.21 mmol) at 0 °C. The mixture was stirred at rt for 12 h. Water was added to the reaction mixture and the mixture was combined with a mixture of an experiment performed in a similar fashion (726 mg 4,6-dichloro-1-[3-(trifluoromethyl)phenyl]-1H-pyrazolo[3,4-d]pyrimidine was used for the 2^{nd} experiment). The combined reaction mixtures were extracted with ethyl acetate. The organic layers were dried over anhydrous sodium sulfate and concentrated to give a residue. The residue was purified by flash chromatography (petroleum ether: ethyl acetate = 8: 1 to 2: 1, then 2: 1, 2: 1 to 0: 1, then 0: 1) to give 1-{6-chloro-1-[3-(trifluoromethyl)phenyl]-1 H-pyrazolo[3,4-d]pyrimidin-4-yl}-N,N-dimethylazetidin-3-amine (760 mg) as a yellow solid.
LC-MS (method **C**): Rₜ = 0.822 min; MS (ESIpos): m/z = 397.3 [M+H]⁺.
¹H NMR (400 MHz, DMSO-*d*₆): δ [ppm] = 8.45-8.39 (m, 2H), 8.32 (s, 1H), 7.79 (t, *J* = 8.4 Hz, 1H), 7.70 (d, *J* = 7.6 Hz, 1H), 4.47 (t, *J* = 7.6 Hz, 1H), 4.36-4.28 (m, 1H), 4.27-4.20 (m, 1H), 4.06-3.99 (m, 1H), 3.37-3.28 (m, 1H), 2.16 (s, 6H).

### Intermediate 2-I-17

### tert-butyl [1-(1-{6-chloro-1-[3-(trifluoromethyl)phenyl]-1H-pyrazolo[3,4-d]pyrimidin-4-yl}piperidin-4-yl)cyclopropyl]carbamate

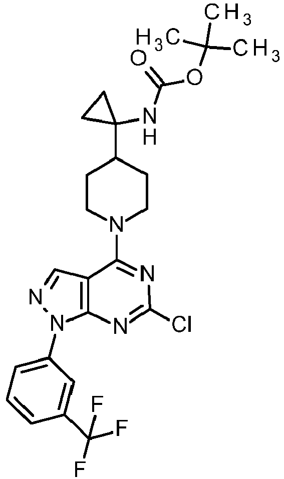

DIPEA (1.3 ml) was added to 4,6-dichloro-1-[3-(trifluoromethyl)phenyl]-1H-pyrazolo[3,4-d]pyrimidine (500 mg, 1.50 mmol) and tert-butyl [1-(piperidin-4-yl)cyclopropyl]carbamate (379 mg, 1.58 mmol, CAS 1330766-47-6) in 2-methyltetrahydrofuran (5.0 ml) and the mixture was stirred at rt for 67 h. Standard workup and purification (Biotage Isolera, hexane / ethyl acetate) afforded 843 mg of the title compound.
LC-MS (method a01): Rₜ = 1.63 min; MS (ESIpos): m/z = 537 [M+H]⁺

### Intermediate 2-I-18

### 1-{6-chloro-1-[3-(trifluoromethyl)phenyl]-1H-pyrazolo[3,4-d]pyrimidin-4-yl}-N,N,3-tri-methylazetidin-3-amine

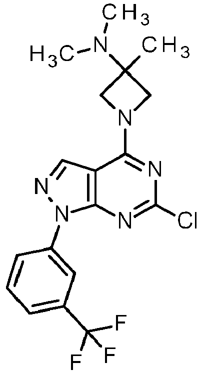

A solution of 4,6-dichloro-1-[3-(trifluoromethyl)phenyl]-1H-pyrazolo[3,4-d]pyrimidine (1.00 g, 3.00 mmol) and N,N,3-trimethylazetidin-3-amine hydrochloride (498 mg, 3.30 mmol) in 2-methyltetrahydrofuran (10 ml) was treated with DIPEA (1.6 ml, 9.0 mmol) and the mixture was stirred at rt for 16 h. Standard workup and purification (Biotage ULTRA 50 g column, hexane / ethyl acetate) afforded 900 mg of the title compound.

### Intermediate 2-I-19

### 6-chloro-4-{3-[(methanesulfonyl)methyl]azetidin-1-yl}-1-[3-(trifluoromethyl)phenyl]-1H-pyrazolo[3,4-d]pyrimidine

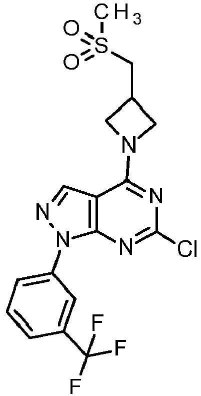

DIPEA (0.44 ml, 2.5 mmol) was added to 4,6-dichloro-1-[3-(trifluoromethyl)phenyl]-1H-pyrazolo[3,4-d]pyrimidine (281 mg, 0.84 mmol) and 3-[(methylsulfonyl)methyl]azetidine 4-methylbenzenesulfonate (271 mg, 0.84 mmol) in 2-methyltetrahydrofuran (5.0 ml) and the mixture was stirred 19 h at rt. Standard workup and purification by HPLC (acetonitrile / water + 0.1% formic acid) afforded 299 mg of the title compound.
LC-MS (method a01): Rₜ = 1.26 min; MS (ESIpos): m/z = 446 [M+H]⁺

### Intermediate 2-I-20

### 6-chloro-4-[3-methyl-3-(morpholin-4-yl)azetidin-1-yl]-1-[3-(trifluoromethyl)phenyl]-1H-pyrazolo[3,4-d]pyrimidine

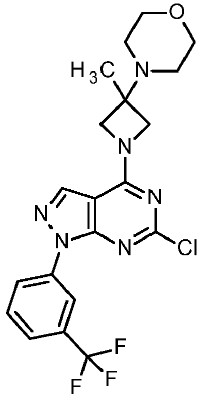

### Step a)

### 1-(diphenylmethyl)-3-methylazetidin-3-ol

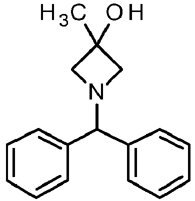

To a solution of 1-(diphenylmethyl)azetidin-3-one (10.0 g, 42.1 mmol)] in tetrahydrofuran (100 ml) was added methylmagnesium bromide solution (3M in tetrahydrofuran, 17 ml, 51 mmol) at 0 ∼ 10°C. The mixture was stirred at 0°C for 20 min utes. Then the mixture was stirred at rt for 2 h. The mixture was added to saturated ammonium chloride solution and extracted with ethyl acetate. The organic layers were dried over anhydrous sodium sulfate and concentrated to give a residue. The residue was purified by column chromatography (1000 mesh, petroleum ether: ethyl acetate = 10: 1, then 5: 1) to give 1-(diphenylmethyl)-3-methylazetidin-3-ol (9.00 g, 84% yield) as light-yellow oil.
¹H NMR (400 MHz, DMSO-*d₆*): δ [ppm] = 7.41 (d, *J* = 7.2 Hz, 4H), 7.26 (t, *J* = 7.6 Hz, 4H), 7.19-7.12 (m, 2H), 5.12 (s, 1H), 4.38 (s, 1H), 3.04 (d, *J* = 7.6 Hz, 2H), 2.81 (d, *J* = 7.6 Hz, 2H), 1.40 (s, 3H).

### Step b)

### 1-(diphenylmethyl)-3-methylazetidin-3-yl methanesulfonate

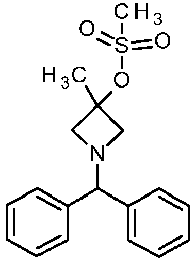

To a solution of 1-(diphenylmethyl)-3-methylazetidin-3-ol (6.00 g, 23.7 mmol) in DCM (60 ml) were added triethylamine (5.0 ml, 36 mmol) and methanesulfonyl chloride (2.2 ml, 28 mmol) at 0 °C . The reaction was stirred at 0 °C for 30 mi nutes, warmed to rt and stirred for 2 h. The reaction mixture was quenched with saturated aq. sodium bicarbonate solution and extracted with ethyl acetate. The organic phase was washed with brine, dried over anhydrous sodium sulfate, filtered and concentrated to give crude product 1-(diphenylmethyl)-3-methylazetidin-3-yl methanesulfonate (6.50 g). The crude product was directly used for next step.

### 4-[1-(diphenylmethyl)-3-methylazetidin-3-yl]morpholine

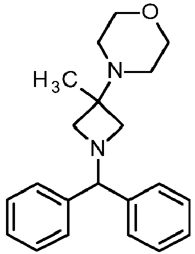

To a solution of 1-(diphenylmethyl)-3-methylazetidin-3-yl methanesulfonate (5.20 g, 15.7 mmol) in 2-propanol (50 ml) was added morpholine (2.7 ml, 31 mmol) at rt. The reaction mixture was stirred at 70°C for 12 h. The mixture was concentrated, and ethyl acetate was added. The solution was washed with water, brine and concentrated to give a residue. The residue was purified by silica gel chromatography (1000 mesh, petroleum ether: ethyl acetate = 10: 1, then 3: 1) to give 4-[1-(diphenylmethyl)-3-methylazetidin-3-yl]morpholine (3.50 g, 69% yield) as light yellow oil.
¹H NMR (400 MHz, DMSO-*d₆*): δ [ppm] = 7.44 (d, *J* = 7.2 Hz, 4H), 7.27 (t, *J* = 7.2 Hz, 4H), 7.20-7.13 (m, 2H), 4.45 (s, 1H), 3.59-3.48 (m, 4H), 2.93 (d, *J* = 7.2 Hz, 2H), 2.75 (d, *J* = 6.8 Hz, 2H), 2.28-2.21 (m, 4H), 1.28 (s, 3H).

### Step d)

### 4-(3-methylazetidin-3-yl)morpholine hydrochloride (1:1)

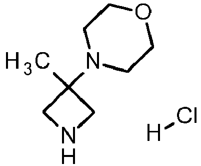

To a solution of 4-[1-(diphenylmethyl)-3-methylazetidin-3-yl]morpholine (5.20 g, 16.1 mmol) in ethanol (30 ml) were added palladium (10% on carbon, 1 g) and palladium hydroxide (15% on carbon,1 g). The reaction mixture was stirred at rt for 6 h under hydrogen atmosphere (15 psi). The reaction mixture was filtered, and the filter cake was washed with DCM. The filtrate was concentrated under reduced pressure to give a residue. Hydrochloric acid (4M in ethyl acetate, 10 ml) was added to the residue dropwise and the mixture was stirred for 0.5 h. The reaction mixture was filtered and the filter cake was washed with ethyl acetate. The filter cake was dried under reduced pressure to give 4-(3-methylazetidin-3-yl)morpholine hydrochloride (1.19 g, 38% yield) as white solid.

LC-MS (Instrument: Agilent 1100\G1956A SingleQuad; Column: Agilent ZORBAX RX-SIL, 4.6*150mm, 5µm; eluent A: water + 0.0375 vol % trifluoroacetic acid, eluent B: acetonitrile + 0.01875 vol % trifluoroacetic acid; gradient: 0-7 min 5-90% B; flow 1 ml/min; temperature: 40 °C; PDA: 220 nm & 254 nm): Rₜ = 3.051 min; MS (ESIpos): m/z = 157.1 [M+H]⁺.
¹H NMR (400 MHz, DMSO-*d₆*): δ [ppm] = 10.64 (s, 1H), 9.24 (s, 1H), 4.74-4.58 (m, 2H), 4.07-3.94 (m, 4H), 3.79-3.68 (m, 2H), 3.32-3.02 (m, 4H), 1.72 (s, 3H).

### Step e)

To solution of 4-(3-methylazetidin-3-yl)morpholine hydrochloride (752 mg, 3.90 mmol) in 2-methyltetrahydrofuran (30 ml) were added DIPEA (2.1 ml, 12 mmol) and 4,6-dichloro-1-[3-(trifluoromethyl)phenyl]-1H-pyrazolo[3,4-d]pyrimidine (1000 mg, 3.00 mmol) at 0 °C . After stirring at rt for 12 h, the reaction mixture was concentrated under reduced pressure and the residue was purified by silica gel chromatography (1000 mesh, petroleum ether: ethyl acetate = 10: 1, then 5: 1, 2: 1) to give 6-chloro-4-[3-methyl-3-(morpholin-4-yl)azetidin-1-yl]-1-[3-(trifluoromethyl) phenyl]-1H-pyrazolo[3,4-d] pyrimidine (720 mg, 95% purity, 39% yield) as a white solid.
LC-MS (method **C**): Rₜ = 0.856 min; MS (ESIpos): m/z = 453.3 [M+H]⁺.
¹H NMR (400 MHz, DMSO-*d₆*): δ [ppm] = 8.46-8.44 (m, 2H), 8.38 (s, 1H), 7.83 (t, *J* = 8.4 Hz, 1H), 7.75 (d, *J* = 8.0 Hz, 1H), 4.39 (d, *J* = 9.2 Hz, 1H), 4.19 (d, *J* = 9.2 Hz, 1H), 4.11 (d, *J* = 10.0 Hz, 1H), 3.93 (d, *J* = 10.0 Hz, 1H), 3.62 (t, *J* = 4.0 Hz, 4H), 2.48-2.41 (m, 4H), 1.34 (s, 3H).

### Intermediate 2-I-21

### 6-chloro-4-[3-(morpholin-4-ylmethyl)azetidin-1-yl]-1-[3-(trifluoromethyl)phenyl]-1H-pyrazolo[3,4-d]pyrimidine

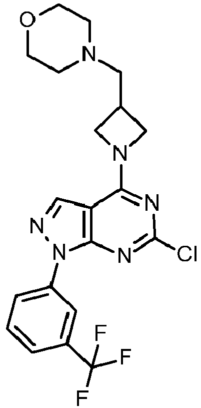

DIPEA (0.52 ml) was added to 4,6-dichloro-1-[3-(trifluoromethyl)phenyl]-1H-pyrazolo[3,4-d]pyrimidine (200 mg, 0.60 mmol) and 4-(azetidin-3-ylmethyl)morpholine trifluoroacetate (178 mg, 0.66 mmol) in 2-methyltetrahydrofuran (2.0 ml) and the mixture was stirred at rt for 23 h. Standard workup afforded a crude product which was treated with DMSO. The remaining solid was filtered, washed three times with 2-methoxy-2-methylpropane and dried affording 102 mg of the title compound. An additional batch (46 mg) was obtained by purification of the filtrate by preparative HPLC.
LC-MS (method b02): Rₜ = 1.39 min; MS (ESIpos): m/z = 453 [M+H]⁺

### Intermediate 2-I-22

### 1-(1-{6-chloro-1-[3-(trifluoromethyl)phenyl]-1H-pyrazolo[3,4-d]pyrimidin-4-yl}azetidin-3-yl)-2-methylpropan-2-ol

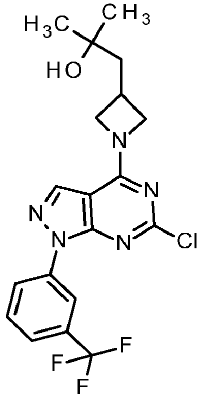

### Step a)

### benzyl 3-(2-methoxy-2-oxoethyl)azetidine-1-carboxylate

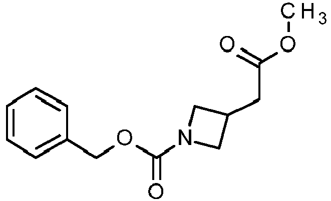

To a solution of methyl azetidin-3-ylacetate trifluoroacetate (10.5 g, 43.2 mmol) in THF were added saturated sodium hydrogen carbonate and benzyl carbonochloridate (14.7 g, 86.4 mmol) at 0 °C. The mixture was stirred at rt for 12 h. The mixture diluted with ethyl acetate and washed with water. The organic layer was dired over anhydrous sodium sulfate and concentrated to give a residue. The residue was purified by flash chromatography (petroleum ether: ethyl acetate) to afford 10.2 g of the title compound as a yellow oil.

### Step b)

### benzyl 3-(2-hydroxy-2-methylpropyl)azetidine-1-carboxylate

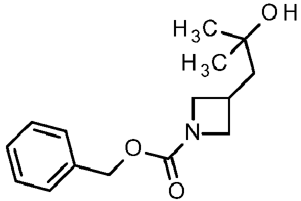

To a solution of benzyl 3-(2-methoxy-2-oxoethyl)azetidine-1-carboxylate (12.5 g, 47.5 mmol) in THF (150 ml) was added methylmagnesium bromide (47 ml, 3 M, in diethyl ether) at 0 °C. Then the mixture was stirred at rt for 12 h. The mixture was added to saturated ammonium chloride solution and extracted with ethyl acetate. The organic layers were dried over anhydrous sodium sulfate and concentrated to give a residue. The residue was purified by silica gel chromatography (petroleum ether: ethyl acetate) to give 4.6 g benzyl 3-(2-hydroxy-2-methylpropyl)azetidine-1-carboxylate as a yellow oil.

### Step c)

### 1-(azetidin-3-yl)-2-methylpropan-2-ol

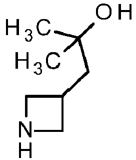

To a solution of benzyl 3-(2-hydroxy-2-methylpropyl)azetidine-1-carboxylate (4.60 g, 17.5 mmol) in ethanol (60 ml) were added palladium on carbon (10%) (400 mg) and palladium(II) hydroxide palladium on carbon (15%) (400 mg). The mixture was stirred at rt under hydrogen atmosphere (15 Psi) for 12 h. The mixture was filtered, and the filtrate was concentrated to give 1.75 g 1-(azetidin-3-yl)-2-methylpropan-2-ol as yellow oil.

### Step d)

DIPEA (0.78 ml) was added to 4,6-dichloro-1-[3-(trifluoromethyl)phenyl]-1H-pyrazolo[3,4-d]pyrimidine (500 mg, 1.50 mmol) and 1-(azetidin-3-yl)-2-methylpropan-2-ol (233 mg) in 2-methyltetrahydrofuran and the mixture was stirred at rt for 20 h. Standard workup and purification by HPLC afforded 295 mg 1-(1-{6-chloro-1-[3-(trifluoromethyl)phenyl]-1H-pyrazolo[3,4-d]pyrimidin-4-yl}azetidin-3-yl)-2-methylpropan-2-ol.
LC-MS (method a01): Rₜ = 1.40 min; MS (ESIpos): m/z = 426 [M+H]⁺

### Intermediate 2-I-23

### 6-chloro-4-[3-(methylsulfonyl)azetidin-1-yl]-1-[3-(trifluoromethyl)phenyl]-1H-pyrazolo[3,4-d]pyrimidine

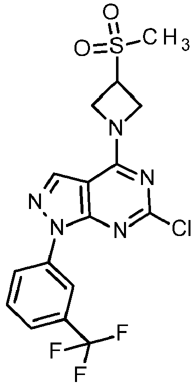

DIPEA (0.31 ml) was added to 4,6-dichloro-1-[3-(trifluoromethyl)phenyl]-1H-pyrazolo[3,4-d]-pyrimidine (200 mg, 0.60 mmol) and 3-(methylsulfonyl)azetidine hydrochloride (124 mg) in 2-methyltetrahydrofuran (3.0 ml) and the mixture was stirred at rt for 16 h. Standard workup afforded 258 mg of the title compound.
LC-MS (method a01): Rₜ = 1.26 min; MS (ESIpos): m/z = 432 [M+H]⁺

### Intermediate 2-I-24

### 2-(1-{6-chloro-1-[3-(trifluoromethyl)phenyl]-1H-pyrazolo[3,4-d]pyrimidin-4-yl}azetidin-3-yl)propan-2-ol

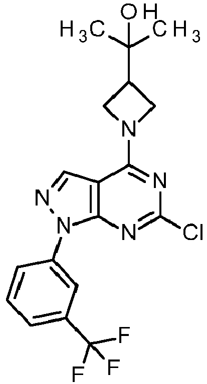

DIPEA (1.26 ml) was added to 4,6-dichloro-1-[3-(trifluoromethyl)phenyl]-1H-pyrazolo[3,4-d]pyrimidine (400 mg, 1.20 mmol) and 2-(azetidin-3-yl)propan-2-ol hydrochloride (200 mg, 1.32 mmol) in 2-methyltetrahydrofuran (10 ml) and the mixture was stirred at rt for 16 h. Standard workup and purification by column chromatography (Biotage ULTRA 25 g column, ethyl acetate / hexane) afforded 400 mg of the title compound.
LC-MS (method a01): Rₜ = 1.37 min; MS (ESIpos): m/z = 412 [M+H]⁺

### Intermediate 2-I-25

### (3R,4R)-1-{6-chloro-1-[3-(trifluoromethyl)phenyl]-1H-pyrazolo[3,4-d]pyrimidin-4-yl}-4-fluoropyrrolidin-3-ol

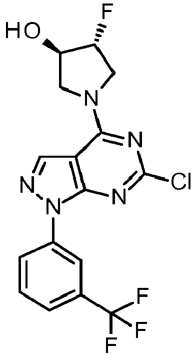

DIPEA (1.10 ml) was added to 4,6-dichloro-1-[3-(trifluoromethyl)phenyl]-1H-pyrazolo[3,4-d]pyrimidine (350 mg, 1.05 mmol) and (3R,4R)-4-fluoropyrrolidin-3-ol hydrochloride (164 mg, 1.16 mmol) in 2-methyltetrahydrofuran (6.0 ml) and the mixture was stirred at rt for 16 h. Standard workup and purification by column chromatography (Biotage ULTRA 25 g column, ethyl acetate / hexane) afforded 310 mg of the title compound.
LC-MS (method a01): Rₜ = 1.32 min; MS (ESIpos): m/z = 402 [M+H]⁺

### Intermediate 2-I-26

### 1-{6-chloro-1-[3-(trifluoromethyl)phenyl]-1H-pyrazolo[3,4-d]pyrimidin-4-yl}piperidin-4-ol

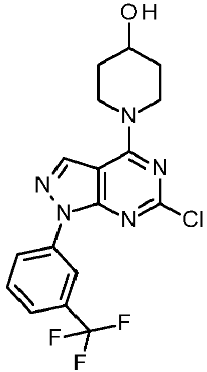

DIPEA (0.63 ml) was added to 4,6-dichloro-1-[3-(trifluoromethyl)phenyl]-1H-pyrazolo[3,4-d]pyrimidine (200 mg, 0.60 mmol) and piperidin-4-ol (121 mg, 1.20 mmol) in 2-methyltetrahydrofuran (2.0 ml) and the mixture was stirred at rt for 19 h. Standard workup and purification by HPLC afforded 203 mg of the title compound.
LC-MS (method a01): Rₜ = 1.34 min; MS (ESIpos): m/z = 398 [M+H]⁺

### Intermediate 2-I-27

### (3S,4S)-1-{6-chloro-1-[3-(trifluoromethyl)phenyl]-1H-pyrazolo[3,4-d]pyrimidin-4-yl}-4-fluoropyrrolidin-3-ol

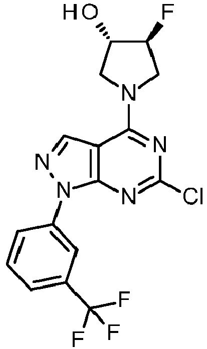

DIPEA (1.10 ml) was added to 4,6-dichloro-1-[3-(trifluoromethyl)phenyl]-1H-pyrazolo[3,4-d]pyrimidine (350 mg, 1.05 mmol) and (3S,4S)-4-fluoropyrrolidin-3-ol hydrochloride (164 mg, 1.16 mmol) in 2-methyltetrahydrofuran (3.5 ml) and the mixture was stirred at rt for 22 h. Standard workup and purification by column chromatography (hexane/ethyl acetate) afforded 384 mg of the title compound.
LC-MS (method a01): Rₜ = 1.33 min; MS (ESIpos): m/z = 402 [M+H]⁺

### Intermediate 2-I-28

### 6-chloro-4-(3-fluoroazetidin-1-yl)-1-[3-(trifluoromethyl)phenyl]-1H-pyrazolo[3,4-d]pyrimidine

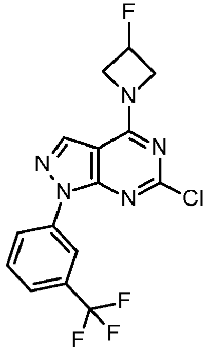

DIPEA (0.31 ml) was added to 4,6-dichloro-1-[3-(trifluoromethyl)phenyl]-1H-pyrazolo[3,4-d]pyrimidine (100 mg, 0.30 mmol) and 3-fluoroazetidine hydrochloride (50 mg, 0.45 mmol) in 2-methyltetrahydrofuran (2.0 ml) and the mixture was stirred at rt for 21 h. Standard workup and purification by HPLC afforded 80 mg of the title compound.
LC-MS (method a01): Rₜ = 1.40 min; MS (ESIpos): m/z = 372 [M+H]⁺

### Intermediate 2-I-29

### (3R)-1-{6-chloro-1-[3-(trifluoromethyl)phenyl]-1H-pyrazolo[3,4-d]pyrimidin-4-yl}-3-methylpyrrolidin-3-ol

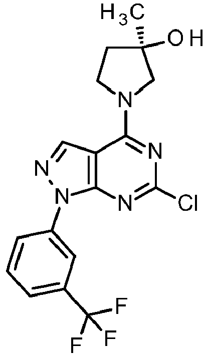

DIPEA (0.41 ml) was added to 4,6-dichloro-1-[3-(trifluoromethyl)phenyl]-1H-pyrazolo[3,4-d]pyrimidine (130 mg, 0.39 mmol) and (3R)-3-methylpyrrolidin-3-ol (79 mg, 0.78 mmol) in 2-methyltetrahydrofuran (2.5 ml) and the mixture was stirred at rt for 16 h. Standard workup and purification by HPLC afforded 135 mg of the title compound.
LC-MS (method a01): Rₜ = 1.34 min; MS (ESIpos): m/z = 398 [M+H]⁺

### Intermediate 2-I-30

### tert-butyl (1-{6-chloro-1-[3-(trifluoromethyl)phenyl]-1H-pyrazolo[3,4-d]pyrimidin-4-yl}-3,3-difluoropiperidin-4-yl)carbamate

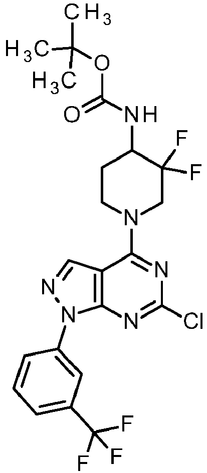

DIPEA (0.94 ml) was added to 4,6-dichloro-1-[3-(trifluoromethyl)phenyl]-1H-pyrazolo[3,4-d]-pyrimidine (300 mg, 0.90 mmol) and tert-butyl (3,3-difluoropiperidin-4-yl)carbamate (319 mg, 1.35 mmol) in 2-methyltetrahydrofuran (4.0 ml) and the mixture was stirred at rt for 16 h. Standard workup and purification by HPLC afforded 397 mg of the title compound.
LC-MS (method a01): Rₜ = 1.55 min; MS (ESIpos): m/z = 533 [M+H]⁺

### Intermediate 2-I-31

### 1-(1-{6-chloro-1-[3-(trifluoromethyl)phenyl]-1H-pyrazolo[3,4-d]pyrimidin-4-yl}azetidin-3-yl)pyrrolidin-2-one

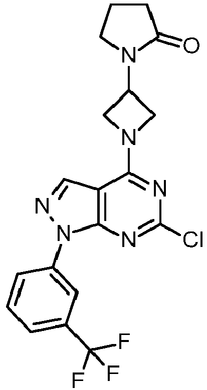

### Step a)

### benzyl 3-(4-chlorobutanamido)azetidine-1-carboxylate

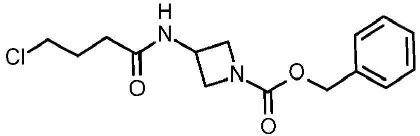

To a solution of benzyl 3-aminoazetidine-1-carboxylate (500 mg, 2.42 mmol) in DCM (10 ml) were added triethylamine (510 µl, 3.6 mmol) and 4-chlorobutanoyl chloride (410 mg, 2.91 mmol) at 0 °C . The reaction mixture was stirred at 25 °C for 2 h. The mixture was poured into water and extracted with ethyl acetate. The organic phase was washed with brine, dried over anhydrous sodium sulfate, filtered and concentrated to give benzyl 3-(4-chlorobutanamido)azetidine-1-carboxylate (500 mg, 66% yield) as a yellow solid.
¹H NMR (400 MHz, DMSO-*d₆*): δ [ppm] = 8.54 (d, *J* = 7.2 Hz, 1H), 7.40-7.29 (m, 5H), 5.04 (s, 2H), 4.50-4.40 (m, 1 H), 4.15 (s, 2H), 3.76 (s, 2H), 3.63 (t, *J* = 6.8 Hz, 2H), 2.24 (t, *J* = 7.2 Hz, 2H), 1.99-1.87 (m, 2H).

### Step b)

### benzyl 3-(2-oxopyrrolidin-1-yl)azetidine-1-carboxylate

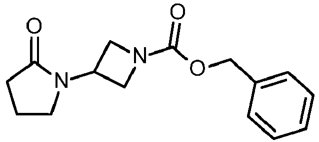

To a solution of benzyl 3-(4-chlorobutanamido)azetidine-1-carboxylate (400 mg, 1.29 mmol) in tetrahydrofuran (5.0 ml) was added sodium hydroxide (103 mg, 2.57 mmol) at rt. The reaction mixture was stirred at 25 °C for 12 h. The mixture was poured into water and extracted with ethyl acetate. The organic phase was washed with brine, dried over anhydrous sodium sulfate, filtered and concentrated to give benzyl 3-(2-oxopyrrolidin-1-yl)azetidine-1-carboxylate (300 mg, 85% yield) as a white solid.
¹H NMR (400 MHz, CDCl₃): δ [ppm] = 7.40-7.31 (m, 5H), 5.11 (s, 2H), 5.07-5.00 (m, 1H), 4.24 (t, *J* = 8.8 Hz, 2H), 4.07 (dd, *J* = 9.6, 5.6 Hz, 2H), 3.56 (t, *J* = 6.8 Hz, 2H), 2.42 (t, *J* = 8.0 Hz, 2H), 2.14-2.05 (m, 2H).

### Step c)

### 1-(azetidin-3-yl)pyrrolidin-2-one

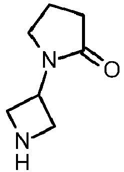

To a solution of benzyl 3-(2-oxopyrrolidin-1-yl)azetidine-1-carboxylate (370 mg, 1.35 mmol) in methanol (10 ml) was added palladium on carbon (200 mg, 10% purity) at rt. The reaction mixture was stirred at rt for 12 h under hydrogen atmosphere (15 psi). The reaction mixture was filtered and the filtrate was concentrated to give 1-(azetidin-3-yl)pyrrolidin-2-one (160 mg, 85% yield) as yellow oil.
¹H NMR (400 MHz, CDCl₃): δ [ppm] = 5.10-4.98 (m, 1H), 3.79-3.75 (m, 2H), 3.64-3.53 (m, 2H), 3.50-3.45 (m, 2H), 2.43-2.37 (m, 2H), 2.12-2.00 (m, 2H).

### Step d)

### 1-(1-{6-chloro-1-[3-(trifluoromethyl)phenyl]-1H-pyrazolo[3,4-d]pyrimidin-4-yl}azetidin-3-yl)pyrrolidin-2-one

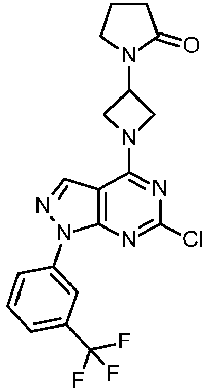

To a solution of 4,6-dichloro-1-[3-(trifluoromethyl)phenyl]-1H-pyrazolo[3,4-d]pyrimidine (380 mg, 1.14 mmol) in 2-methyltetrahydrofuran (5 ml) were added DIPEA (800 µl, 4.6 mmol) and 1-(azetidin-3-yl)pyrrolidin-2-one (160 mg, 1.14 mmol) at 0 °C. The mixture was stirred at rt for 12 h. The reaction mixture was poured into water and extracted with ethyl acetate. The organic layers were dried over anhydrous sodium sulfate and concentrated to give 1-(1-{6-chloro-1-[3-(trifluoromethyl)phenyl]-1H-pyrazolo[3,4-d]pyrimidin-4-yl}azetidin-3-yl)pyrrolidin-2-one (160 mg, 97% purity, 31% yield) as a white solid.
LC-MS (method C): Rₜ = 0.987 min; MS (ESIpos): m/z = 437.1 [M+H]⁺.
¹H NMR (400 MHz, CDCl₃): δ [ppm] = 8.5 (d, *J* = 8.0 Hz, 1H), 8.44 (s, 1H), 7.96 (s, 1H), 7.67-7.56 (m, 2H), 5.35-5.25 (m, 1H), 4.80-4.32 (m, 4H), 3.63 (t, *J* = 7.2 Hz, 2H), 2.48 (t, *J* = 8.0 Hz, 2H), 2.22-2.11 (m, 2H).

### Intermedidate 2-I-32

### 1-{6-chloro-1-[3-(trifluoromethyl)phenyl]-1H-pyrazolo[3,4-d]pyrimidin-4-yl}-4-(pyrrolidin-1-ylmethyl)piperidin-4-ol

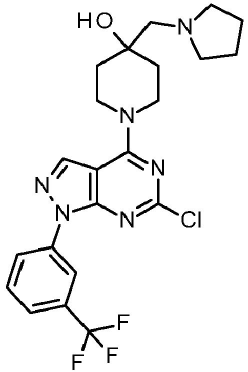

A solution of 4,6-dichloro-1-[3-(trifluoromethyl)phenyl]-1H-pyrazolo[3,4-d]pyrimidine (120 mg and 4-[(pyrrolidin-1-yl)methyl]piperidin-4-ol in 2-methyltetrahydrofuran was treated with DIPEA (0.25 ml) and the mixture was stirred at rt for 16 h. Standard workup and purification by HPLC (method HT_PREP_bas) afforded 90 mg of the title compound.
LC-MS (method HTpost_bas): Rₜ = 1.71 min; MS (ESIpos): m/z = 481 [M+H]⁺

### Intermediate 2-I-33

### 4-(1-{6-chloro-1-[3-(trifluoromethyl)phenyl]-1H-pyrazolo[3,4-d]pyrimidin-4-yl}azetidin-3-yl)piperazin-2-one

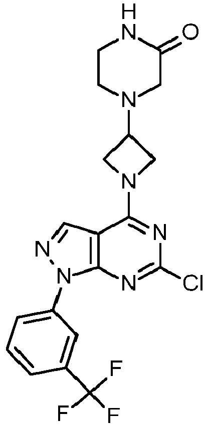

A solution of 4,6-dichloro-1-[3-(trifluoromethyl)phenyl]-1 H-pyrazolo[3,4-d]pyrimidine (100 mg) and 4-(azetidin-3-yl)piperazin-2-one dihydrochloride (75 mg) in 2-methyltetrahydrofuran was treated with DIPEA (0.21 ml) and the mixture was stirred at rt for 16 h. Water was added to the reaction mixture and the mixture was extracted with ethyl acetate three times and the organic layers were concentrated. The crude product was used without further purification.

### Intermediate 2-I-34

### (3R)-1-{6-chloro-1-[3-(trifluoromethyl)phenyl]-1H-pyrazolo[3,4-d]pyrimidin-4-yl}pyrrolidin-3-ol

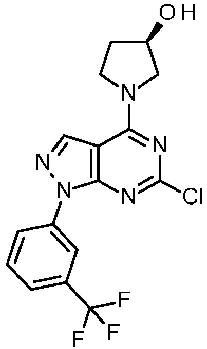

A solution of 4,6-dichloro-1-[3-(trifluoromethyl)phenyl]-1H-pyrazolo[3,4-d]pyrimidine (100 mg) and (3R)-pyrrolidin-3-ol-hydrogen chloride in 2-methyltetrahydrofuran (2.0 ml) was treated with DIPEA (0.21 ml) and the mixture was stirred at rt for 16h. Standard workup and purification by HPLC (method HT_PREP_bas) afforded 80 mg of the title compound.
LC-MS (method HTpost_bas): Rₜ = 1.40 min; MS (ESIpos): m/z = 384 [M+H]⁺

### Intermediate 2-I-35

### 6-chloro-4-[3-(3,3-difluoropyrrolidin-1-yl)azetidin-1-yl]-1-[3-(trifluoromethyl)phenyl]-1H-pyrazolo[3,4-d]pyrimidine

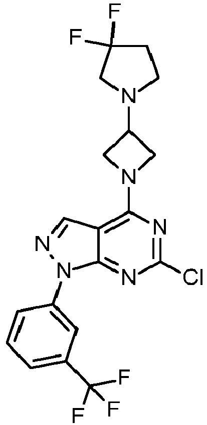

A solution of 4,6-dichloro-1-[3-(trifluoromethyl)phenyl]-1H-pyrazolo[3,4-d]pyrimidine (200 mg) and 1-(azetidin-3-yl)-3,3-difluoropyrrolidine dihydrochloride (155 mg) (CAS 1403766-97-1) in 2-methyltetrahydrofuran (4.0 ml) was treated with DIPEA (0.42 ml) and the mixture was stirred at RT for 16 h. Standard workup and purification by column chromatography (Biotage ULTRA 25g column, hexane / ethyl acetate) afforded 230 mg of the title compound.
LC-MS (method a01): Rₜ = 1.46 min; MS (ESIpos): m/z = 459 [M+H]⁺

### Intermediate 2-I-36

### 6-chloro-4-(3-fluoro[1,3'-biazetidin]-1'-yl)-1-[3-(trifluoromethyl)phenyl]-1H-pyrazolo[3,4-d]pyrimidine

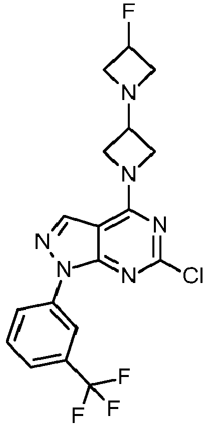

A solution of 4,6-dichloro-1-[3-(trifluoromethyl)phenyl]-1H-pyrazolo[3,4-d]pyrimidine (344 mg) and 3-fluoro-1,3'-biazetidine oxalic acid (250 mg) (CAS 1523571-87-0) in 2-methyltetrahydrofuran (6.9 ml) was treated with DIPEA (0.72 ml) and the mixture was stirred at RT for 72 h. Standard workup and purification by column chromatography (Biotage ULTRA 25 g column, hexane / ethyl acetate) afforded 160 mg of the title compound.
MS (ESIpos): m/z = 427 [M+H]⁺

### Intermediate 2-I-37

### 4-(1-{6-chloro-1-[3-(trifluoromethyl)phenyl]-1H-pyrazolo[3,4-d]pyrimidin-4-yl}azetidin-3-yl)morpholin-3-one

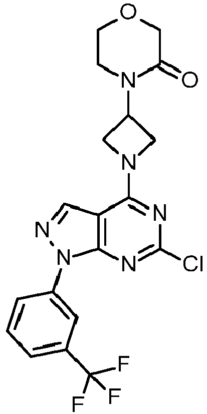

### Step a)

### benzyl 3-[(2-hydroxyethyl)amino]azetidine-1-carboxylate

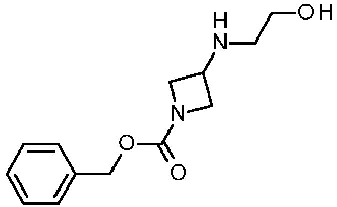

To a mixture of benzyl 3-oxoazetidine-1-carboxylate (10.0 g, 48.7 mmol) and 2-aminoethan-1-ol (4.46 g, 73.1 mmol) in methanol (100 ml) was added acetic acid (280 µl, 4.9 mmol). The mixture was stirred at 60 °C for 1 hour. Then sodium cyanoborohydride (6.12 g, 97.5 mmol) was added at rt. The mixture was stirred at rt for 12 h. The mixture was concentrated and purified by silica gel chromatography (1000 mesh, petroleum ether: ethyl acetate = 1: 1, then 0: 1, then ethyl acetate: methanol = 10: 1) to give benzyl 3-[(2-hydroxyethyl)amino]azetidine-1-carboxylate (4.30 g) as a yellow oil.
LC-MS (method **C**): Rₜ = 0.185 min; MS (ESIpos): m/z = 251.6

### Step b)

### benzyl 3-[(chloroacetyl)(2-hydroxyethyl)amino]azetidine-1-carboxylate

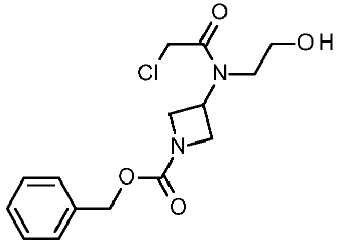

To a solution of benzyl 3-[(2-hydroxyethyl)amino]azetidine-1-carboxylate (4.30 g) in DCM (80 ml) were added DIPEA (3.8 ml, 22 mmol) and chloroacetyl chloride (1.22 g, 10.8 mmol) at 0 °C . The mixture was stirred at rt for 1 hour. The mixture was diluted with DCM and washed with saturated ammonium chloride solution and brine. The organic phase was dried over anhydrous sodium sulfate, filtered and concentrated to give a residue. The residue was purified by silica gel chromatography (1000 mesh, petroleum ether: ethyl acetate = 5: 1, then 1: 1) to give benzyl 3-[(chloroacetyl)(2-hydroxyethyl)amino]azetidine-1-carboxylate (2.40 g) as a yellow oil.
LC-MS (method **C**): Rₜ = 0.680 min; MS (ESIpos): m/z = 327.1

### Step c)

### benzyl 3-(3-oxomorpholin-4-yl)azetidine-1-carboxylate

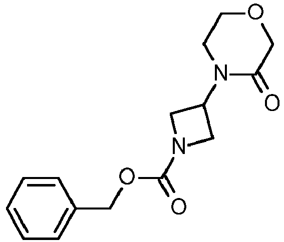

To a solution of benzyl 3-[(chloroacetyl)(2-hydroxyethyl)amino]azetidine-1-carboxylate (1.80 g) in THF (40 ml) was added sodium hydroxide (330 mg, 8.26 mmol) at 0 °C. After stirring at rt for 12 h, the mixture was diluted with ethyl acetate and washed with water. The organic phase was concentrated and purified by silica gel chromatography (1000 mesh, petroleum ether: ethyl acetate = 5: 1, then 1: 1) to give benzyl 3-(3-oxomorpholin-4-yl)azetidine-1-carboxylate (1.30 g) as a yellow oil.
¹H NMR (400 MHz, DMSO-*d₆*) δ [ppm] = 7.40-7.28 (m, 5H), 5.20-5.09 (m, 1H), 5.04 (s, 1H), 4.19-4.09 (m, 4H), 4.04 (s, 2H), 3.85 (t, *J* = 4.8 Hz, 2H), 3.49 (t, *J* = 5.2 Hz, 2H).

### Step d)

### 4-(azetidin-3-yl)morpholin-3-one

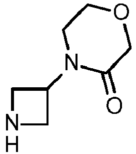

To a solution of benzyl 3-(3-oxomorpholin-4-yl)azetidine-1-carboxylate (700 mg, 2.41 mmol) in ethanol (15 ml) was added palladium on carbon (100 mg, 10% Pd). The mixture was stirred at rt under hydrogen atmosphere (15 psi) for 12 h. The mixture was filtered and the filtrate was concentrated to give 4-(azetidin-3-yl)morpholin-3-one (350 mg) as a yellow oil
¹H NMR (400 MHz, DMSO-*d₆*) δ [ppm] = 5.13-5.00 (m, 1H), 4.01 (s, 2H), 3.85 (t, *J* = 5.2 Hz, 2H), 3.64-3.55 (m, 2H), 3.51-3.43 (m, 4H).

### Step e)

To a solution of 4,6-dichloro-1-[3-(trifluoromethyl)phenyl]-1H-pyrazolo[3,4-d]pyrimidine (746 mg) in 2-methyltetrahydrofuran (20 ml) were added DIPEA (1.2 ml, 6.7 mmol) and 4-(azetidin-3-yl)morpholin-3-one (350 mg) at 0 °C. The mixture was stirred at rt for 12 h. The mixture was diluted with ethyl acetate and washed with brine. The organic phase was concentrated and purified by column chromatography (1000 mesh, petroleum ether: ethyl acetate = 2: 1, then 1: 1, then 1: 2) to give 4-(1-{6-chloro-1-[3-(trifluoromethyl)phenyl]-1 H-pyrazolo[3,4-d]pyrimidin-4-yl}azetidin-3-yl)morpholin-3-one (688 mg) as a yellow solid.
LC-MS (method **C**): Rₜ = 0.959 min; MS (ESIpos): m/z = 453.3
¹H NMR (400 MHz, DMSO-*d₆*) δ [ppm] = 8.48-8.42 (m, 2H), 8.39 (s, 1H), 7.83 (t, *J* = 8.4 Hz, 1H), 7.75 (d, *J* = 7.6 Hz, 1H), 5.50-5.36 (m, 1H), 4.80-4.66 (m, 2H), 4.55-4.44 (m, 2H), 4.11 (s, 2H), 3.95-3.84 (m, 2H), 3.61 (t, *J* = 4.8 Hz, 2H).

### Intermediate 2-I-38

### (3S)-1-{6-chloro-1-[3-(trifluoromethyl)phenyl]-1H-pyrazolo[3,4-d]pyrimidin-4-yl}-3-methylpyrrolidin-3-ol

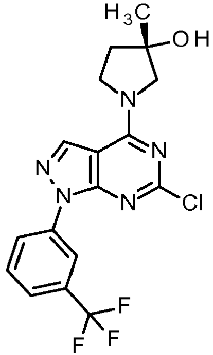

A solution of 4,6-dichloro-1-[3-(trifluoromethyl)phenyl]-1H-pyrazolo[3,4-d]pyrimidine (150 mg) and (3S)-3-methylpyrrolidin-3-ol (50 mg) in 2-methyltetrahydrofuran (3.0 ml) was treated with DIPEA (0.31 ml) and the mixture was stirred at RT for 16 h. Standard workup and purification by HPLC (method HT_PREP_bas) afforded 130 mg of the title compound.
LC-MS (method HTpost_bas): Rₜ = 1.44 min; MS (ESIpos): m/z = 398 [M+H]⁺

### Intermediate 2-II-1

### 2-(1-{6-chloro-1-[3-(difluoromethyl)phenyl]-1H-pyrazolo[3,4-d]pyrimidin-4-yl}piperidin-4-yl)propan-2-ol

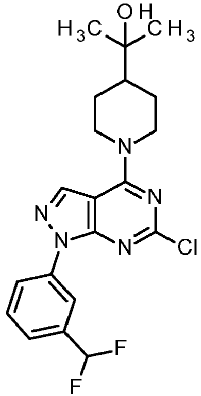

To solution of 4,6-dichloro-1-[3-(difluoromethyl)phenyl]-1H-pyrazolo[3,4-d]pyrimidine (1.00 g, 75% purity, 2.38 mmol) in 2-methyltetrahydrofuran (10 ml) were added DIPEA (1.2 ml, 7.1 mmol) and 2-(piperidin-4-yl)propan-2-ol (341 mg, 2.38 mmol) at 0°C. After stirring at rt for 12 h, the reaction mixture was filtered, and the filter cake was washed with 2-methyltetrahydrofuran. The filter cake was purified by silica gel chromatography (1000 mesh, petroleum ether: ethyl acetate = 10: 1, then 5: 1, 2: 1) to give 2-(1-{6-chloro-1-[3-(difluoromethyl)phenyl]-1H-pyrazolo[3,4-d]pyrimidin-4-yl}piperidin-4-yl)propan-2-ol (410 mg) as a yellow solid.
LC-MS (method **C**): Rₜ = 1.033 min; MS (ESIpos): m/z = 422.3 [M+H]⁺.
¹H NMR (400 MHz, DMSO-*d₆*): δ [ppm] = 0.86 (s, 1H), 8.31-8.26 (m, 2H), 7.73 (t, *J* = 8.0 Hz, 1H), 7.58 (d, *J* = 7.6 Hz 1H), 7.17 (t, *J* = 55.6 Hz, 1H), 5.05-4.40 (m, 2H), 4.20 (s, 1H), 3.29-2.77 (m, 2H), 1.91 (s, 2H), 1.64-1.58 (m, 1H), 1.42-1.22 (m, 2H), 1.06 (s, 6H).

### Intermediate 2-II-2

### 1-(1-{6-chloro-1-[3-(difluoromethyl)phenyl]-1H-pyrazolo[3,4-d]pyrimidin-4-yl}azetidin-3-yl)-2-methylpropan-2-ol

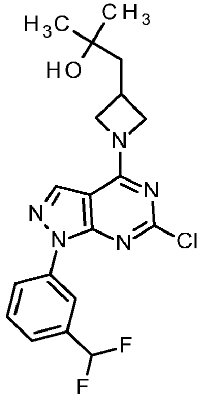

DIPEA (1.2 ml) was added to a solution of 4,6-dichloro-1-[3-(difluoromethyl)phenyl]-1H-pyrazolo[3,4-d]pyrimidine (750 mg, 2.38 mmol) and 1-(azetidin-3-yl)-2-methylpropan-2-ol (338 mg, 2.62 mmol) in 2-methyltetrahydrofuran (5.6 ml) and the mixture was stirred at rt for 16 h. Standard workup afforded the title compound as crude product which was used without further purification.

### Intermediate 2-II-3

### 6-chloro-1-[3-(difluoromethyl)phenyl]-4-[3-(morpholin-4-yl)azetidin-1-yl]-1H-pyrazolo[3,4-d]pyrimidine

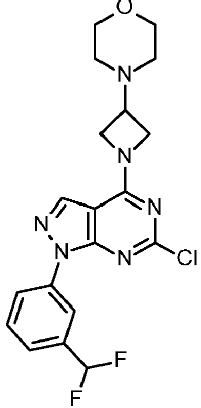

A solution of 4,6-dichloro-1-[3-(difluoromethyl)phenyl]-1H-pyrazolo[3,4-d]pyrimidine (1.00 g, 3.17 mmol) and 4-(azetidin-3-yl)morpholine dihydrochloride (751 mg, 3.49 mmol) in 2-methyltetrahydrofuran (15 ml) was treated with DIPEA (1.7 ml, 9.5 mmol) and the mixture was stirred at rt for 72 h. Standard workup and purification (Biotage ULTRA 50 g column, ethyl acetate / ethanol) afforded 860 mg of the title compound.

### Intermediate 2-II-4

### methyl (1-{6-chloro-1-[3-(difluoromethyl)phenyl]-1H-pyrazolo[3,4-d]pyrimidin-4-yl}azetidin-3-yl)acetate

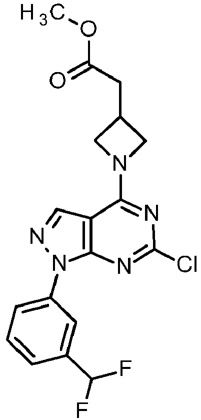

DIPEA (1.7 ml) was added to 4,6-dichloro-1-[3-(difluoromethyl)phenyl]-1H-pyrazolo[3,4-d]pyrimidine (1.00 g, 3.17 mmol) and methyl azetidin-3-ylacetate trifluoroacetate in 2-methyltetrahydrofuran (15 ml) and the mixture was stirred at rt for 68 h. Standard workup and purification by column chromatography (hexane / ethyl acetate) afforded 965 mg of the title compound.
LC-MS (method a01): Rₜ = 1.30 min; MS (ESIpos): m/z = 408 [M+H]⁺

### Intermediate 2-II-5

### 1-(1-{6-chloro-1-[3-(difluoromethyl)phenyl]-1H-pyrazolo[3,4-d]pyrimidin-4-yl}azetidin-3-yl)-4-methylpiperidin-4-ol

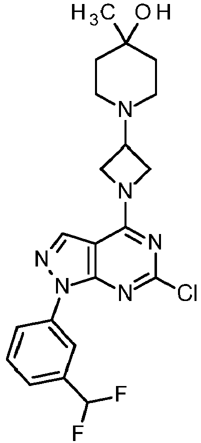

A solution of 4,6-dichloro-1-[3-(difluoromethyl)phenyl]-1 H-pyrazolo[3,4-d]pyrimidine (295 mg) and 1-(azetidin-3-yl)-4-methylpiperidin-4-ol dihydrochloride (250 mg) in 2-methyltetrahydrofuran (4.2 ml) was treated with DIPEA (0.49 ml) and the mixture was stirred at rt for 16 h. Standard workup and purification by column chromatography (Biotage ULTRA 10 g column, hexane / ethyl acetate / ethanol) afforded 310 mg of the title compound.

### Intermediate 2-II-6-1

### rel-(3R,4R)-1-{6-chloro-1-[3-(difluoromethyl)phenyl]-1H-pyrazolo[3,4-d]pyrimidin-4-yl}-4-fluoropyrrolidin-3-ol

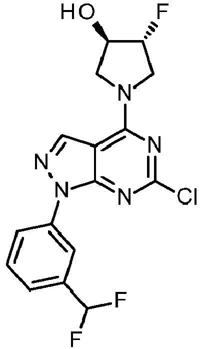

A solution of 4,6-dichloro-1-[3-(difluoromethyl)phenyl]-1 H-pyrazolo[3,4-d]pyrimidine (150 mg) and rel-(3R,4R)-4-fluoropyrrolidin-3-ol hydrochloride (74 mg) in 2-methyltetrahydrofuran (2 ml) was treated with DIPEA (0.25 ml) and the mixture was stirred at rt for 16 h. Standard workup and purification by HPLC afforded 160 mg of the title compound.

### Intermediate 2-II-6-2

### (3S,4S)-1-{6-chloro-1-[3-(difluoromethyl)phenyl]-1H-pyrazolo[3,4-d]pyrimidin-4-yl}-4-fluoropyrrolidin-3-ol

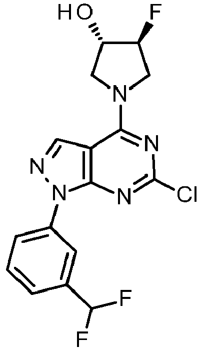

The synthesis was performed analogously to intermediate 2-II-6-1 using (3S,4S)-4-fluoropyrrolidin-3-ol hydrochloride.
LC-MS (method a01): Rₜ = 1.20 min; MS (ESIpos): m/z = 384 [M+H]⁺

### Intermediate 2-II-6-3

### (3R,4R)-1-{6-chloro-1-[3-(difluoromethyl)phenyl]-1H-pyrazolo[3,4-d]pyrimidin-4-yl}-4-fluoropyrrolidin-3-ol

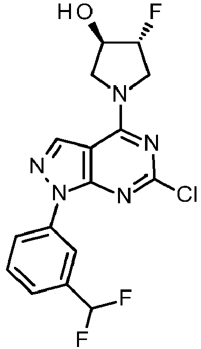

The synthesis was performed analogously to intermediate 2-II-6-1 using (3R,4R)-4-fluoropyrrolidin-3-ol hydrochloride (1:1)
LC-MS (method a01): Rₜ = 1.20 min; MS (ESIpos): m/z = 384 [M+H]⁺

### Intermediate 2-II-7

### rel-(3R,4S)-1-{6-chloro-1-[3-(difluoromethyl)phenyl]-1H-pyrazolo[3,4-d]pyrimidin-4-yl}-4-methylpyrrolidin-3-ol

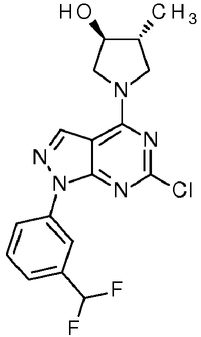

A solution of 4,6-dichloro-1-[3-(difluoromethyl)phenyl]-1H-pyrazolo[3,4-d]pyrimidine (150 mg) and rel-(3S,4R)-4-methylpyrrolidin-3-ol hydrochloride (72 mg) in 2-methyltetrahydrofuran (2 ml) was treated with DIPEA (0.25 ml) and the mixture was stirred at rt for 16 h. Standard workup and purification by HPLC afforded 140 mg of the title compound.

### Intermediate 2-II-8

### 1-{6-chloro-1-[3-(difluoromethyl)phenyl]-1H-pyrazolo[3,4-d]pyrimidin-4-yl}-3-(trifluoromethyl)pyrrolidin-3-ol

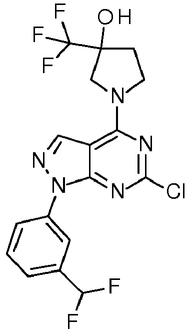

A solution of 4,6-dichloro-1-[3-(difluoromethyl)phenyl]-1 H-pyrazolo[3,4-d]pyrimidine (150 mg) and 3-(trifluoromethyl)pyrrolidin-3-ol hydrochloride (100 mg) in 2-methyltetrahydrofuran (2 ml) was treated with DIPEA (0.25 ml) and the mixture was stirred at rt for 16 h. Standard workup and purification by HPLC afforded 60 mg of the title compound.

### Intermediate 2-II-9

### tert-butyl [1-(1-{6-chloro-1-[3-(difluoromethyl)phenyl]-1H-pyrazolo[3,4-d]pyrimidin-4-yl}piperidin-4-yl)cyclopropyl]carbamate

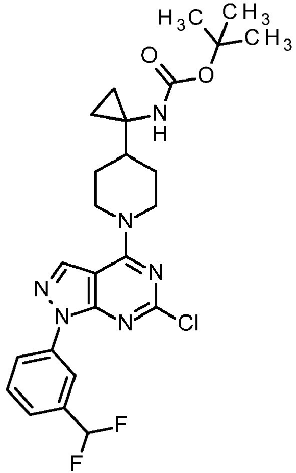

A solution of 4,6-dichloro-1-[3-(difluoromethyl)phenyl]-1 H-pyrazolo[3,4-d]pyrimidine (200 mg) and tert-butyl [1-(piperidin-4-yl)cyclopropyl]carbamate in 2-methyltetrahydrofuran (3.0 ml) was treated with DIPEA (0.55 ml) and the mixture was stirred at RT for 16 h. Standard workup and column chromatography (Biotage ULTRA 10 g column eluent: hexane / ethyl acetate) afforded 300 mg of the crude title compound.
LC-MS (OA01b02): Rₜ = 1.52 min; MS (ESIpos): m/z = 519 [M+H]⁺

### Intermediate 2-II-10

### 6-chloro-1-[3-(difluoromethyl)phenyl]-4-[3-methyl-3-(morpholin-4-yl)azetidin-1-yl]-1H-pyrazolo[3,4-d]pyrimidine

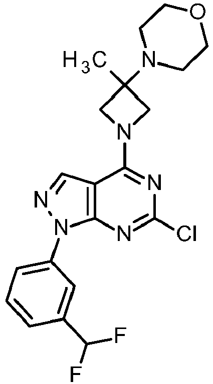

A solution of 4,6-dichloro-1-[3-(difluoromethyl)phenyl]-1H-pyrazolo[3,4-d]pyrimidine (200 mg) and 4-(3-methylazetidin-3-yl)morpholine hydrochloride (135 mg) in 2-methyltetrahydrofuran (3.0 ml) was treated with DIPEA (0.33 ml) and the mixture was stirred at rt for 16 h. Standard workup and purification by HPLC (method HT_PREP_acid) afforded 90 mg of the title compound.
LC-MS (method HTpost_acid): Rₜ = 1.04 min; MS (ESIpos): m/z = 435 [M+H]⁺

Additional intermediates of type 2-II were prepared in a similar fashion as described before using 4,6-dichloro-1-[3-(difluoromethyl)phenyl]-1H-pyrazolo[3,4-d]pyrimidine and the corresponding amine:

### Intermediate 2-II-11

### (3R)-1-{6-chloro-1-[3-(difluoromethyl)phenyl]-1H-pyrazolo[3,4-d]pyrimidin-4-yl}-3-methylpyrrolidin-3-ol

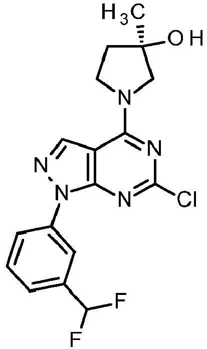

LC-MS (method a01): Rₜ = 1.21 min; MS (ESIpos): m/z = 380 [M+H]⁺

### Intermediate 2-II-12

### (3R)-1-{6-chloro-1-[3-(difluoromethyl)phenyl]-1H-pyrazolo[3,4-d]pyrimidin-4-yl}pyrrolidin-3-ol

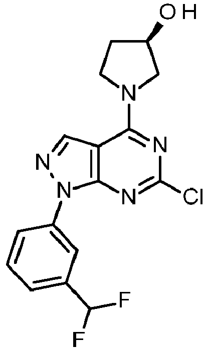

LC-MS (method a01): Rₜ = 1.14 min; MS (ESIpos): m/z = 366 [M+H]⁺

### Intermediate 2-II-13

### 4-(1-{6-chloro-1-[3-(difluoromethyl)phenyl]-1H-pyrazolo[3,4-d]pyrimidin-4-yl}azetidin-3-yl)piperazin-2-one

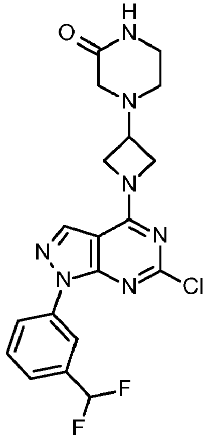

MS (ESIpos): m/z = 434 [M+H]⁺

### Intermediate 2-II-14

### methyl 2-(1-{6-chloro-1-[3-(difluoromethyl)phenyl]-1H-pyrazolo[3,4-d]pyrimidin-4-yl}azetidin-3-yl)-2-methylpropanoate

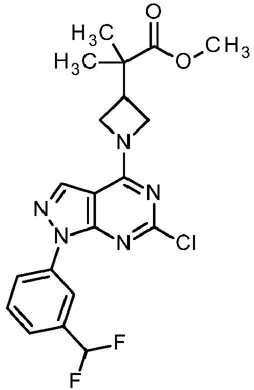

LC-MS (method HTpost_acid): Rₜ = 1.37 min; MS (ESIpos): m/z = 436 [M+H]⁺

### Intermediate 2-III-1

### 2-(1-{6-chloro-1-[3-(trifluoromethoxy)phenyl]-1H-pyrazolo[3,4-d]pyrimidin-4-yl}piperidin-4-yl)propan-2-ol

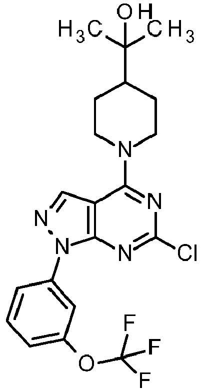

To solution of 4,6-dichloro-1-[3-(trifluoromethoxy)phenyl]-1H-pyrazolo[3,4-d]pyrimidine (680 mg, crude batch) in 2-methyltetrahydrofuran (10 ml) were added DIPEA (0.68 ml, 3.9 mmol) and 2-(piperidin-4-yl)propan-2-ol (187 mg, 1.31 mmol) at 0° C. After stirring at rt for 12 h, water was added to the reaction mixture. The mixture was extracted with ethyl acetate. The organic layers were dried over anhydrous sodium sulfate and concentrated to give a residue. The residue was purified by silica gel chromatography (1000 mesh, petroleum ether: ethyl acetate = 8: 1, then 5: 1, then 2: 1) to give 2-(1-{6-chloro-1-[3-(trifluoromethoxy)phenyl]-1H-pyrazolo[3,4-d]pyrimidin-4-yl}piperidin-4-yl)propan-2-ol (323 mg) as a yellow solid.
LC-MS (method **C**): Rₜ = 1.178 min; MS (ESIpos): m/z = 456.3
¹H NMR (400 MHz, CDCl₃) δ [ppm] = 8.25-8.20 (m, 1H), 8.15-8.07 (m, 2H), 7.54 (t, *J* = 8.4 Hz, 1H), 7.21-7.16 (m, 1H), 5.25-4.40 (m, 2H), 3.45-2.85 (m, 2H), 2.10-1.95 (m, 2H), 1.77-1.65 (m, 1H), 1.55-1.37 (m, 2H), 1.29-1.22 (m, 7H).

### Intermediate 2-IV-1

### 2-(1-{6-chloro-1-[3-(difluoromethoxy)phenyl]-1H-pyrazolo[3,4-d]pyrimidin-4-yl}piperidin-4-yl)propan-2-ol

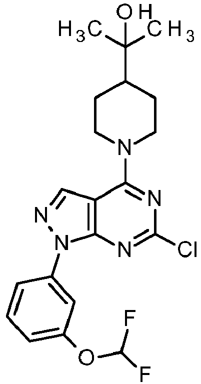

To solution of [4,6-dichloro-1-[3-(difluoromethoxy)phenyl]-1H-pyrazolo[3,4-d]pyrimidine (700 mg, 2.11 mmol) in 2-methyltetrahydrofuran (15 ml) were added DIPEA (1.1 ml, 6.3 mmol) and 2-(piperidin-4-yl)propan-2-ol (303 mg, 2.11 mmol) at 0° C. After stirring at rt for 12 h, the reaction mixture was filtered, and the filter cake was washed with 2-methyltetrahydrofuran. The filter cake was purified by silica gel chromatography (1000 mesh, petroleum ether: ethyl acetate = 10: 1, then 5: 1, 2: 1) to give 2-(1-{6-chloro-1-[3-(difluoromethoxy)phenyl]-1H-pyrazolo[3,4-d]pyrimidin-4-yl}piperidin-4-yl)propan-2-ol (320 mg) as a yellow solid.
LC-MS (method **C**): Rₜ = 1.045 min; MS (ESIpos): m/z = 438.3 [M+H]⁺.
¹H NMR (400 MHz, DMSO-*d₆*): δ [ppm] = 8.64 (s, 1H), 8.01 (dd, *J* = 8.0, 1.2 Hz, 1H), 7.91 (t, *J* = 2.0 Hz, 1H), 7.63 (t, *J* = 8.0 Hz, 1H), 7.34 (t, *J* = 73.6 Hz, 1H), 7.20 (dd, *J* = 8.0, 2.0 Hz, 1H), 5.05-4.37 (m, 2H), 4.22 (s, 1H), 3.31-2.97 (m, 2H), 1.91 (br.s, 2H), 1.64-1.58 (m, 1H), 1.43-1.16 (m, 2H), 1.06 (s, 6H).

### Intermediate 2-IV-2

### tert-butyl [1-(1-{6-chloro-1-[3-(difluoromethoxy)phenyl]-1H-pyrazolo[3,4-d]pyrimidin-4-yl}piperidin-4-yl)cyclopropyl]carbamate

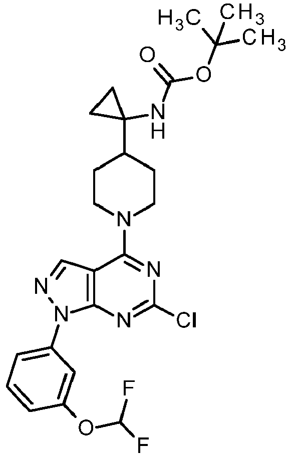

A solution of [4,6-dichloro-1-[3-(difluoromethoxy)phenyl]-1H-pyrazolo[3,4-d]pyrimidine (200 mg) and tert-butyl [1-(piperidin-4-yl)cyclopropyl]carbamate] (160 mg) in 2-methyltetrahydrofuran (3.0 ml) was treated with DIPEA (0.53 ml) and the mixture was stirred at rt for 16 h. Standard workup afforded a crude product which was treated with 2-methoxy-2-methylpropane. The resulting solid was filtered, washed with 2-methoxy-2-methylpropane and dried to afford 250 mg of the title compound.
LC-MS (method b02): Rₜ = 1.53 min; MS (ESIpos): m/z = 535 [M+H]⁺

### Intermediate 2-IV-3

### 1-(1-{6-chloro-1-[3-(difluoromethoxy)phenyl]-1H-pyrazolo[3,4-d]pyrimidin-4-yl}azetidin-3-yl)-2-methylpropan-2-ol

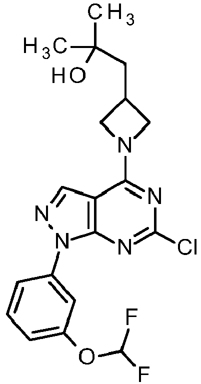

A solution of [4,6-dichloro-1-[3-(difluoromethoxy)phenyl]-1H-pyrazolo[3,4-d]pyrimidine (120 mg) and 1-(azetidin-3-yl)-2-methylpropan-2-ol (52 mg) in 2-methyltetrahydrofuran (2.0 ml) was treated with DIPEA (0.32 ml) and the mixture was stirred at rt for 16 h. Then 1-(azetidin-3-yl)-2-methylpropan-2-ol (20 mg) and NMP (1 ml) was added and the mixture was stirred at rt for 16 h. Standard workup and HPLC purification afforded 75 mg of the title compound.
LC-MS (method a01): Rₜ = 1.28 min; MS (ESIpos): m/z = 424 [M+H]⁺

### Intermediate 2-IV-4

### (3S,4S)-1-{6-chloro-1-[3-(difluoromethoxy)phenyl]-1H-pyrazolo[3,4-d]pyrimidin-4-yl}-4-fluoropyrrolidin-3-ol

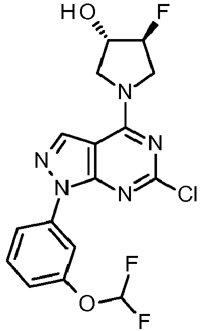

A solution of [4,6-dichloro-1-[3-(difluoromethoxy)phenyl]-1H-pyrazolo[3,4-d]pyrimidine (250 mg) and (3S,4S)-4-fluoropyrrolidin-3-ol hydrochloride (118 mg) in 2-methyltetrahydrofuran (3.5 ml) was treated with DIPEA (0.66 ml) and the mixture was stirred at rt for 16 h. Standard workup and HPLC purification (water + 0.1% formic acid / acetonitrile) afforded 245 mg of the title compound.
LC-MS (method a01): Rₜ = 1.21 min; MS (ESIpos): m/z = 400 [M+H]⁺

### Intermediate 2-V-1

### 1-{6-chloro-1-[3-(methanesulfonyl)phenyl]-1H-pyrazolo[3,4-d]pyrimidin-4-yl}-3-ethylazetidin-3-ol

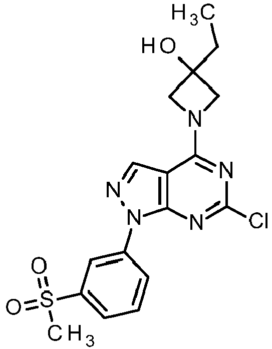

DIPEA (630 µl, 3.6 mmol) was added to 4,6-dichloro-1-[3-(methylsulfonyl)phenyl]-1H-pyrazolo[3,4-d]pyrimidine (250 mg, 728 µmol) and 3-ethylazetidin-3-ol hydrochloride (150 mg, 1.09 mmol) in 2-methyltetrahydrofuran and the mixture was stirred for 19.5 h at rt. Standard workup and purification by HPLC (acetonitrile / water buffered with ammonia) afforded the title compound (129 mg, 0.32 mmol).
LC-MS (method b02): Rₜ = 1.02 min; MS (ESIpos): m/z = 408 [M+H]⁺

### Intermediate 2-VI-1

### Ethyl 3-{6-chloro-4-[4-(2-hydroxypropan-2-yl)piperidin-1-yl]-1H-pyrazolo[3,4-d]-pyrimidin-1-yl}benzoate

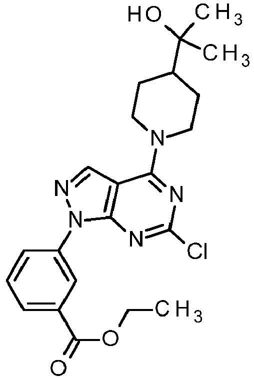

Reaction of ethyl 3-(4,6-dichloro-1H-pyrazolo[3,4-d]pyrimidin-1-yl)benzoate (500 mg, 1.48 mmol) and 2-(piperidin-4-yl)propan-2-ol (234 mg, 1.63 mmol) similar to the preparation of intermediate 2-II-9 afforded 490 mg of the title compound
LC-MS (method a01): Rₜ = 1.41 min; MS (ESIpos): m/z = 444 [M+H]⁺.

### Examples

### Example I-1-1

### (4S)-4-({4-[3-(morpholin-4-yl)azetidin-1-yl]-1-[3-(trifluoromethyl)phenyl]-1H-pyrazolo-[3,4-d]pyrimidin-6-yl}amino)pyrrolidin-2-one

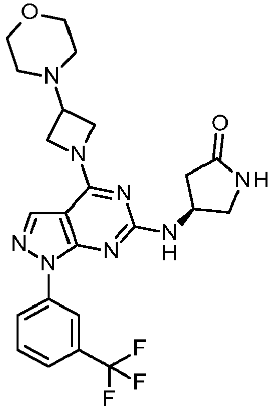

A solution of 6-chloro-4-[3-(morpholin-4-yl)azetidin-1-yl]-1-[3-(trifluoromethyl)phenyl]-1H-pyrazolo[3,4-d]pyrimidine (80.0 mg, 0.18 mmol, 1.0 eq.) and (4S)-4-aminopyrrolidin-2-one hydrochloride (1.5 eq., 37.3 mg, 0.27 mmol) in NMP (2.0 ml) was treated with DIPEA (0.16 ml) and the mixture was stirred at 110°C for 16 h. Then 1 eq. (4S)-4-aminopyrrolidin-2-one hydrochloride and 3 eq. DIPEA were added and the mixture was stirred at 110°C for 24 h. Standard workup and purification by HPLC (method HT_PREP_acid) afforded 34 mg of the title compound.
LC-MS (method HTpost_acid): Rₜ = 0.83 min; MS (ESIpos): m/z = 503 [M+H]⁺
¹H-NMR (400 MHz, DMSO-d6) δ [ppm]: 2.278 (0.87), 2.293 (0.94), 2.323 (2.11), 2.327 (2.34), 2.332 (2.13), 2.402 (6.55), 2.518 (5.36), 2.523 (3.53), 2.532 (1.36), 2.540 (1.55), 2.553 (1.47), 2.665 (0.91), 2.669 (1.28), 2.674 (0.94), 3.215 (1.19), 3.362 (3.70), 3.375 (2.26), 3.380 (2.15), 3.392 (0.98), 3.624 (11.45), 3.634 (7.13), 3.989 (0.77), 4.187 (0.91), 4.268 (1.04), 4.430 (0.91), 4.542 (1.26), 4.556 (1.23), 7.518 (0.83), 7.605 (2.62), 7.629 (6.55), 7.731 (1.28), 7.749 (1.94), 7.769 (0.98), 8.092 (16.00), 8.500 (0.74), 8.570 (0.40), 8.840 (0.83).

### Example I-1-2

### (4R)-4-({4-[3-(morpholin-4-yl)azetidin-1-yl]-1-[3-(trifluoromethyl)phenyl]-1H-pyrazolo-[3,4-d]pyrimidin-6-yl}amino)pyrrolidin-2-one

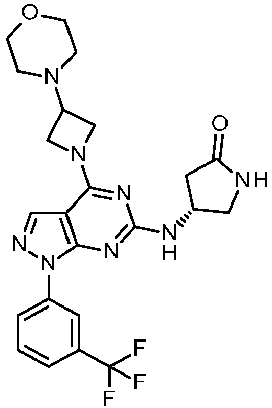

Reaction of 6-chloro-4-[3-(morpholin-4-yl)azetidin-1-yl]-1-[3-(trifluoromethyl)phenyl]-1H-pyrazolo[3,4-d]pyrimidine (80.0 mg, 0.18 mmol) and (4R)-4-aminopyrrolidin-2-one hydrochloride (37.3 mg, 0.27 mmol) using similar conditions to the preparation of example I-1-1 afforded 37 mg of the title compound.
LC-MS (method HTpost_acid): Rₜ = 0.83 min; MS (ESIpos): m/z = 503 [M+H]⁺
¹H-NMR (400 MHz, DMSO-d6) δ [ppm]: 2.277 (1.05), 2.292 (1.15), 2.322 (2.67), 2.326 (2.93), 2.331 (2.73), 2.402 (8.00), 2.518 (9.35), 2.522 (5.83), 2.531 (2.34), 2.539 (2.96), 2.553 (1.71), 2.664 (1.28), 2.669 (1.71), 2.673 (1.32), 3.202 (1.42), 3.374 (2.77), 3.391 (1.25), 3.623 (13.73), 3.983 (0.95), 4.186 (1.15), 4.264 (1.25), 4.426 (1.15), 4.542 (1.55), 4.557 (1.51), 7.520 (1.02), 7.605 (3.06), 7.628 (7.84), 7.731 (1.48), 7.750 (2.30), 7.769 (1.15), 8.092 (16.00), 8.454 (0.46), 8.500 (0.92), 8.839 (1.02).

### Example I-2-1

### (4S)-4-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one

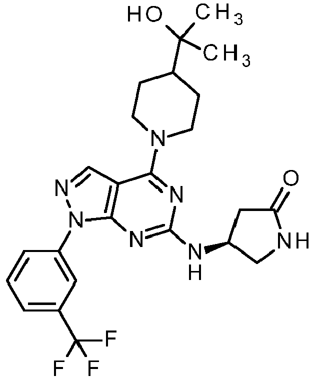

A solution of 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol) and (4S)-4-aminopyrrolidin-2-one hydrochloride (1.5 eq., 37.3 mg, 0.27 mmol) in NMP (2.0 ml) was treated with DIPEA (0.16 ml) and the mixture was stirred at 110°C for 16 h. Then 1 eq. (4S)-4-aminopyrrolidin-2-one hydrochloride and 3 eq. DIPEA were added and the mixture was stirred at 110°C for 24h.Standard workup and purification by HPLC (method HT_PREP_acid) afforded 54 mg of the title compound.
LC-MS (method HTpost_acid): Rₜ = 1.11 min; MS (ESIpos): m/z = 504 [M+H]⁺
¹H-NMR (400 MHz, DMSO-d6) δ [ppm]: 1.051 (16.00), 1.229 (0.53), 1.256 (0.55), 1.561 (0.45), 1.834 (0.75), 1.865 (0.69), 2.074 (1.60), 2.269 (0.59), 2.285 (0.62), 2.311 (0.84), 2.327 (1.24), 2.518 (2.14), 2.523 (1.53), 2.540 (0.44), 2.669 (0.42), 3.615 (0.56), 4.186 (2.46), 7.610 (0.75), 7.636 (1.47), 7.749 (0.57), 8.345 (4.20).

### Example I-2-2

### (4R)-4-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one

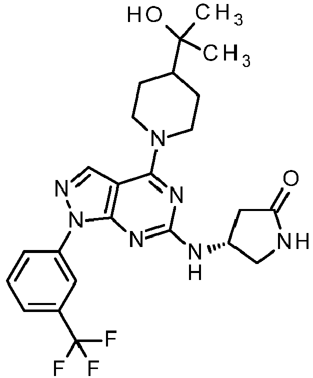

A mixture of 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol), (4R)-4-aminopyrrolidin-2-one hydrochloride (37.3 mg, 0.27 mmol) and DIPEA (0.16 ml) in 1-methyl-2-pyrrolidinone (2.0 ml, 20 mmol) was heated at 110°C in for 16 h. Then 1 eq. (4R)-4-aminopyrrolidin-2-one hydrochloride and 3 eq. DIPEA were added and the mixture was stirred at 110°C. Standard workup and purification by HPLC (method HT_PREP_acid) afforded 56 mg of the title compound.
LC-MS (method HTpost_acid): Rₜ = 1.11 min; MS (ESIpos): m/z = 504 [M+H]⁺
¹H-NMR (400 MHz, DMSO-d6) δ [ppm]: 1.050 (16.00), 1.229 (0.54), 1.254 (0.57), 1.560 (0.46), 1.834 (0.78), 1.863 (0.71), 2.074 (1.09), 2.270 (0.61), 2.285 (0.63), 2.311 (0.85), 2.326 (1.12), 2.518 (1.65), 2.522 (1.20), 2.539 (0.41), 3.615 (0.57), 4.185 (3.81), 7.608 (0.77), 7.629 (1.33), 7.637 (1.42), 7.747 (0.59), 8.343 (4.12).

### Example I-2-3

### 4-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one

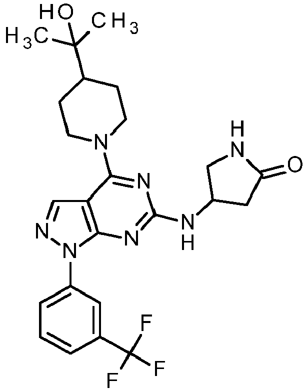

The title compound was prepared in a similar fashion to the preparation of example I-2-1 by the reaction of 2-(1-{6-chloro-1-[3-(trifluoromethyl)phenyl]-1H-pyrazolo[3,4-d]pyrimidin-4-yl}piperidin-4-yl)propan-2-ol (60.0 mg, 0.14 mmol) with 4-aminopyrrolidin-2-one hydrochloride (27.9 mg) at 80°C for 16 h and 100°C for 10 h. Puri fication by HPLC (method HT_PREP _acid) afforded 16 mg of the title compound.
LC-MS (method HTpost_acid): Rₜ = 1.15 min; MS (ESIpos): m/z = 504 [M+H]⁺
¹H-NMR (400 MHz, DMSO-d6) δ [ppm]: 1.050 (16.00), 1.229 (0.53), 1.255 (0.55), 1.561 (0.45), 1.834 (0.72), 1.863 (0.66), 2.269 (0.62), 2.284 (0.62), 2.311 (0.85), 2.326 (1.27), 2.518 (2.10), 2.522 (1.55), 2.539 (0.72), 2.668 (0.47), 3.615 (0.55), 4.188 (4.04), 7.610 (0.74), 7.636 (1.44), 7.748 (0.57), 8.345 (4.21).

### Example I-3-1

### (4S)-4-({4-(3-ethyl-3-hydroxyazetidin-1-yl)-1-[3-(trifluoromethyl)phenyl]-1H-pyrazolo[3,4-d]pyrimidin-6-yl}amino)pyrrolidin-2-one

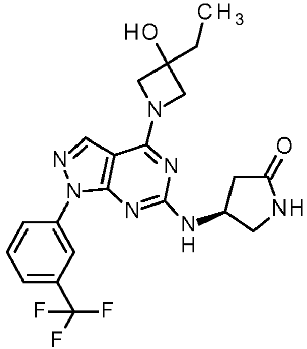

A solution of 1-{6-chloro-1-[3-(trifluoromethyl)phenyl]-1H-pyrazolo[3,4-d]pyrimidin-4-yl}-3-ethylazetidin-3-ol (50.0 mg, 0.13 mmol, 1.0 equivalent) and (4S)-4-aminopyrrolidin-2-one hydrochloride (25.8 mg, 0.19 mmol) in NMP (2.0 ml, 20 mmol) was treated with DIPEA (0.11 ml) and the mixture was stirred at 110°C for 16 h. Then 1 eq. (4S)-4-aminopyrrolidin-2-one hydrochloride (25.8 mg, 189 µmol) and 3 eq. DIPEA were added and the mixture was stirred at 110°C for 24 h. Standard workup and purification by HPLC (method HT_PREP_acid) afforded 32.0 mg of the title compound.
LC-MS (method HTpost_acid): Rₜ = 1.02 min; MS (ESIpos): m/z = 462 [M+H]⁺
¹H-NMR (400 MHz, DMSO-d6) δ [ppm]: 0.916 (6.46), 0.934 (15.30), 0.953 (6.96), 1.714 (1.80), 1.732 (5.13), 1.750 (4.91), 1.768 (1.53), 2.280 (1.15), 2.294 (1.18), 2.323 (2.65), 2.327 (2.65), 2.332 (2.33), 2.523 (5.11), 2.540 (2.88), 2.552 (1.58), 2.665 (1.10), 2.669 (1.45), 3.213 (1.80), 3.601 (1.58), 3.621 (2.23), 3.644 (1.43), 3.950 (1.48), 4.032 (1.35), 4.164 (1.53), 4.317 (1.53), 4.539 (1.70), 4.554 (1.65), 5.763 (1.35), 7.518 (1.05), 7.604 (2.95), 7.629 (7.36), 7.731 (1.43), 7.751 (2.25), 7.770 (1.15), 8.114 (16.00), 8.228 (1.60), 8.492 (0.95), 8.850 (1.08).

### Example I-3-2

### (4R)-4-({4-(3-ethyl-3-hydroxyazetidin-1-yl)-1-[3-(trifluoromethyl)phenyl]-1H-pyrazolo[3,4-d]pyrimidin-6-yl}amino)pyrrolidin-2-one

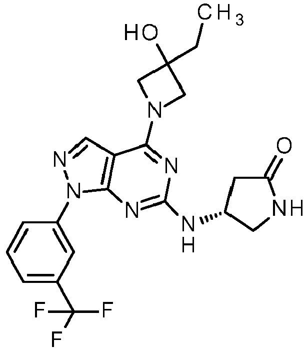

Reaction of 1-{6-chloro-1-[3-(trifluoromethyl)phenyl]-1H-pyrazolo[3,4-d]pyrimidin-4-yl}-3-ethylazetidin-3-ol (50.0 mg, 0.13 mmol) with (4R)-4-aminopyrrolidin-2-one hydrochloride (25.8 mg, 0.19 mmol) using conditions similar to the preparation of example I-3-1 afforded 35.0 mg of the title compound.
LC-MS (method HTpost_acid): Rₜ = 1.02 min; MS (ESIpos): m/z = 462 [M+H]⁺
¹H-NMR (400 MHz, DMSO-d6) δ [ppm]: 0.916 (6.11), 0.934 (14.91), 0.952 (6.68), 1.713 (1.60), 1.731 (4.79), 1.750 (4.58), 1.767 (1.42), 2.279 (1.04), 2.295 (1.07), 2.322 (2.14), 2.326 (2.00), 2.331 (1.77), 2.336 (1.77), 2.518 (5.14), 2.522 (3.24), 2.532 (1.62), 2.539 (5.13), 2.553 (1.30), 2.664 (0.72), 2.668 (0.97), 2.673 (0.73), 3.209 (1.40), 3.602 (1.40), 3.620 (1.99), 3.643 (1.27), 3.949 (1.34), 4.030 (1.25), 4.164 (1.37), 4.317 (1.40), 4.522 (0.90), 4.540 (1.54), 4.555 (1.52), 5.763 (1.75), 7.515 (0.97), 7.603 (2.74), 7.629 (6.13), 7.731 (1.34), 7.750 (2.09), 7.770 (1.05), 8.112 (16.00), 8.259 (0.63), 8.498 (0.87), 8.848 (0.99).

### Example I-4-1

### 2-(1-{6-[(1,1-dioxidotetrahydrothiophen-3-yl)amino]-1-[3-(trifluoromethyl)phenyl]-1H-pyrazolo[3,4-d]pyrimidin-4-yl}piperidin-4-yl)propan-2-ol

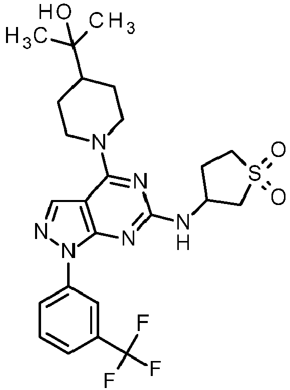

A solution of 2-(1-{6-chloro-1-[3-(trifluoromethyl)phenyl]-1H-pyrazolo[3,4-d]pyrimidin-4-yl}piperidin-4-yl)propan-2-ol (60.0 mg, 0.14 mmol) and tetrahydrothiophen-3-amine 1,1-dioxide (27.7 mg, 0.21 mmol) in NMP (1.0 ml, 10 mmol) was treated with DIPEA (0.12 ml) and the mixture was stirred at 100°C for 16 h. Standard workup and purification by HPLC (method HT_PREP_acid) afforded 10 mg of the title compound.
LC-MS (method HTpost_acid): Rₜ = 1.26 min; MS (ESIpos): m/z = 539 [M+H]⁺
¹H-NMR (400 MHz, DMSO-d6) δ [ppm]: 1.054 (16.00), 1.237 (0.56), 1.267 (0.57), 1.569 (0.50), 1.842 (0.79), 1.872 (0.72), 2.214 (0.43), 2.235 (0.47), 2.247 (0.57), 2.269 (0.57), 2.322 (0.41), 2.327 (0.56), 2.331 (0.41), 2.518 (2.44), 2.523 (1.54), 2.664 (0.43), 2.669 (0.57), 2.673 (0.41), 3.139 (0.47), 3.370 (0.52), 3.386 (0.56), 3.493 (0.66), 3.513 (0.72), 3.527 (0.61), 3.546 (0.57), 4.192 (4.39), 4.639 (0.41), 4.655 (0.41), 7.624 (0.75), 7.644 (1.02), 7.729 (0.65), 7.749 (1.02), 7.769 (0.47), 8.369 (4.68).

### Example I-4-2 and Example I-4-3

### (+)-2-(1-{6-[(1,1-dioxidotetrahydrothiophen-3-yl)amino]-1-[3-(trifluoromethyl)phenyl]-1H-pyrazolo[3,4-d]pyrimidin-4-yl}piperidin-4-yl)propan-2-ol (Example I-4-2)

### (-)-2-(1-{6-[(1,1-dioxidotetrahydrothiophen-3-yl)amino]-1-[3-(trifluoromethyl)phenyl]-1H-pyrazolo[3,4-d]pyrimidin-4-yl}piperidin-4-yl)propan-2-ol (Example I-4-3)

200 mg 2-(1-{6-[(1,1-dioxidotetrahydrothiophen-3-yl)amino]-1-[3-(trifluoromethyl)phenyl]-1H-pyrazolo[3,4-d]pyrimidin-4-yl}piperidin-4-yl)propan-2-ol (see example I-3-1, batch prepared by an independent experiment) were subjected to chiral HPLC.

### Methods:

Instrument: Agilent: 1260, Aurora SFC-module; column: Chiralpak IG 5µm 100x4.6mm;
Eluent A: CO2, Eluent B: methanol + 0.2 Vol-% Ammonia (32%); Isocratic: 40%B; flow 4.0 ml/min; temperature: 37.5°C;
Preparation: Instrument: Sepiatec: Prep SFC100; column: Chiralpak IG 5µm 250x20mm; eluent A CO2, eluent B: methanol + 0.2 Vol-% ammonia (32%); Isocratic: 40%B; flow 80.0 ml/min: 89 mg of enantiomer 1 (retention time 10.5 - 17.0 min, (+)-2-(1-{6-[(1,1-dioxidotetrahydrothiophen-3-yl)amino]-1-[3-(trifluoromethyl)phenyl]-1H-pyrazolo[3,4-d]-pyrimidin-4-yl}piperidin-4-yl)propan-2-ol (Example I-4-2)) and 102 mg of enantiomer 2 (retention time 19.5 - 30.5 min, (-)-2-(1-{6-[(1,1-dioxidotetrahydrothiophen-3-yl)amino]-1-[3-(trifluoromethyl)phenyl]-1H-pyrazolo[3,4-d]pyrimidin-4-yl}piperidin-4-yl)propan-2-ol (Example I-4-3)) were obtained after evaporation, addition of 2-methylpropan-2-ol and freeze drying. Optical rotation (wave length 589 nm, temperature 20.0°C, concentration 1.0000 g / 100 ml): enantiomer 1: 29.8° +/- 0.61°; enantiomer 2: -26.1° +/- 0.49°.

### Example I-5

### 2-(1-{6-[(1,1-dioxidotetrahydro-2H-thiopyran-4-yl)amino]-1-[3-(trifluoromethyl)phenyl]-1H-pyrazolo[3,4-d]pyrimidin-4-yl)piperidin-4-yl)propan-2-ol

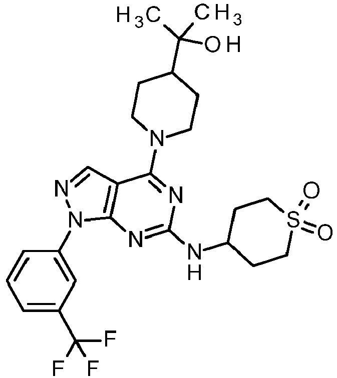

Reaction using 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol), tetrahydro-2H-thiopyran-4-amine 1,1-dioxide, DIPEA and NMP using conditions similar to the preparation of example I-3-1 afforded 41 mg of the title compound after purification by HPLC (method HT_PREP_acid).
LC-MS (method HTpost_acid): Rₜ = 1.22 min; MS (ESIpos): m/z = 553 [M+H]⁺
¹H-NMR (400 MHz, DMSO-d6) δ [ppm]: 1.054 (16.00), 1.223 (0.54), 1.232 (0.58), 1.254 (0.61), 1.262 (0.58), 1.558 (0.48), 1.838 (0.76), 1.867 (0.70), 2.076 (0.49), 2.098 (0.59), 2.229 (0.56), 2.518 (2.08), 2.522 (1.33), 2.539 (1.29), 3.198 (1.15), 4.187 (4.90), 7.618 (0.65), 7.637 (0.83), 7.765 (0.43), 8.336 (4.19).

### Example I-6

### 4-({4-[4-(2-hydroxypropan-2-yl)piperidin-1-yl]-1-[3-(trifluoromethyl)phenyl]-1H-pyrazolo-[3,4-d]pyrimidin-6-yl}amino)-5,5-dimethylpyrrolidin-2-one

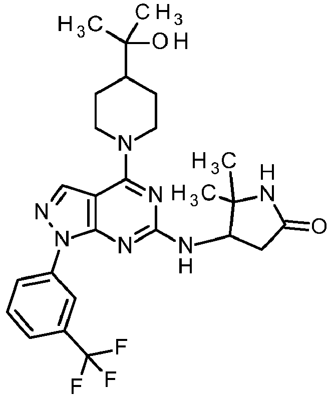

DIPEA (0.19 ml) was added to 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol) and 4-amino-5,5-dimethylpyrrolidin-2-one (46.6 mg, 0.36 mmol) in NMP and the mixture was stirred at 130°C for 72 h. Standard workup and purification by HPLC (method HT_PREP_bas) afforded 46.0 mg of the title compound.
LC-MS (method HTpost_bas): Rₜ = 1.21 min; MS (ESIpos): m/z = 532 [M+H]⁺
¹H-NMR (400 MHz, DMSO-d6) δ [ppm]: 1.052 (16.00), 1.076 (9.74), 1.231 (0.83), 1.261 (0.90), 1.287 (10.86), 1.525 (0.41), 1.555 (0.66), 1.834 (1.09), 1.865 (0.98), 2.447 (2.85), 2.470 (3.12), 2.518 (1.13), 2.523 (0.82), 2.539 (0.58), 3.005 (0.48), 4.188 (6.56), 4.576 (0.45), 4.598 (0.55), 7.213 (0.54), 7.235 (0.44), 7.605 (1.24), 7.625 (1.68), 7.735 (2.36), 7.763 (0.48), 8.324 (6.84), 8.510 (0.46), 8.532 (0.47), 8.770 (0.69).

### Example I-7-1

### (5R)-5-({4-[4-(2-hydroxypropan-2-yl)piperidin-1-yl]-1-[3-(trifluoromethyl)phenyl]-1H-pyrazolo[3,4-d]pyrimidin-6-yl}amino)piperidin-2-one

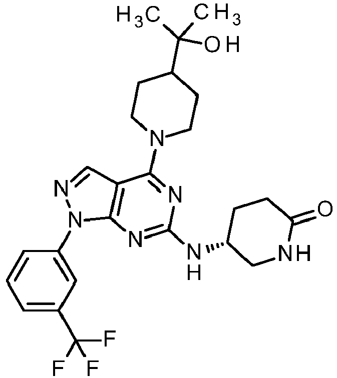

Reaction of 2-(1-{6-chloro-1-[3-(trifluoromethyl)phenyl]-1H-pyrazolo[3,4-d]pyrimidin-4-yl}-piperidin-4-yl)propan-2-ol (80.0 mg, 0.18 mmol), (5R)-5-aminopiperidin-2-one hydrochloride (54.8 mg, 0.36 mmol), DIPEA (0.19 ml) and NMP (2.0 ml) at 110°C for 72 h and standard workup afforded 15.0 mg of the title compound after purification by HPLC (acetonitrile / water + 0.1% formic acid).
MS (ESIpos): m/z = 518 [M+H]⁺
¹H-NMR (400 MHz, DMSO-d6) δ [ppm]: 1.054 (16.00), 1.107 (3.01), 1.227 (0.72), 1.254 (0.58), 1.558 (0.44), 1.837 (0.73), 1.868 (0.65), 2.071 (0.63), 2.082 (0.54), 2.098 (0.55), 2.111 (1.24), 2.133 (0.53), 2.160 (0.50), 2.518 (1.42), 2.523 (0.98), 2.877 (0.48), 4.187 (3.55), 7.057 (0.41), 7.606 (0.70), 7.626 (0.91), 7.717 (0.58), 7.752 (0.50), 8.328 (4.25).

### Example I-7-2

### (5S)-5-({4-[4-(2-hydroxypropan-2-yl)piperidin-1-yl]-1-[3-(trifluoromethyl)phenyl]-1H-pyrazolo[3,4-d]pyrimidin-6-yl}amino)piperidin-2-one

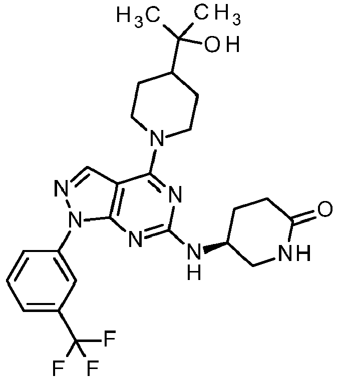

Reaction using 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol), (5S)-5-aminopiperidin-2-one hydrochloride (54.8 mg, 0.36 mmol), DIPEA (0.19 ml) and NMP (2.0 ml) at 110°C for 72 h and standard workup afforded 3.7 mg of the title compound after HPLC (column XBrigde C18 5 µ 50 x 50mm; acetonitrile / water + 0.1% formic acid).
MS (ESIpos): m/z = 518 [M+H]⁺
¹H-NMR (400 MHz, DMSO-d6) δ [ppm]: 1.054 (16.00), 1.227 (0.73), 1.254 (0.58), 1.558 (0.45), 1.837 (0.76), 1.867 (0.67), 2.071 (0.63), 2.082 (0.55), 2.098 (0.55), 2.114 (1.25), 2.133 (0.53), 2.160 (0.50), 2.518 (1.33), 2.523 (0.92), 4.187 (2.52), 7.057 (0.42), 7.606 (0.70), 7.625 (0.94), 7.717 (0.58), 7.752 (0.51), 8.328 (4.16).

### Example I-8

### 4-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylpyrrolidin-2-one

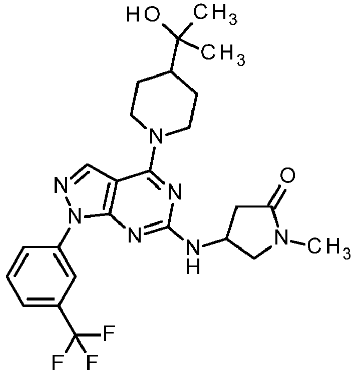

Reaction using 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol), 4-amino-1-methylpyrrolidin-2-one hydrochloride (54.8 mg, 0.36 mmol), DIPEA (0.19 ml) and NMP (2.0 ml) at 120°C for 16 h and standard workup afforded 35.0 mg of the title compound after purification by HPLC (method HT_PREP_acid).
LC-MS (method HTpost_acid): Rₜ = 1.18 min; MS (ESIpos): m/z = 518 [M+H]⁺
¹H-NMR (400 MHz, DMSO-d6) δ [ppm]: 1.051 (16.00), 1.229 (0.52), 1.255 (0.56), 1.559 (0.48), 1.834 (0.78), 1.864 (0.71), 2.414 (0.42), 2.427 (0.40), 2.518 (1.19), 2.522 (0.72), 2.609 (0.55), 2.630 (0.61), 2.651 (0.44), 2.664 (0.40), 2.669 (0.57), 2.673 (0.60), 2.729 (10.28), 3.719 (0.52), 4.189 (2.79), 7.613 (0.71), 7.632 (0.92), 7.751 (0.55), 8.347 (4.55).

### Example I-9

### (3R)-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylpyrrolidin-2-one

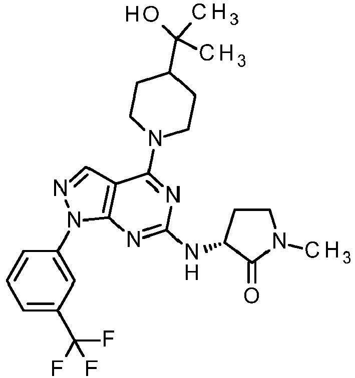

Reaction using 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol), (3R)-3-amino-1-methylpyrrolidin-2-one (41.5 mg, 0.36 mmol), DIPEA (0.19 ml) and NMP (2.0 ml) at 120°C for 16 h and standard workup afforded 38.0 mg of the title compound after purification by HPLC (Method HT_PREP _acid).
LC-MS (method HTpost_acid): Rₜ = 1.19 min; MS (ESIpos): m/z = 518 [M+H]⁺
¹H-NMR (400 MHz, DMSO-d6) δ [ppm]: 1.051 (16.00), 1.219 (0.54), 1.250 (0.60), 1.559 (0.48), 1.831 (0.74), 1.862 (0.67), 2.322 (0.48), 2.327 (0.61), 2.331 (0.49), 2.518 (2.05), 2.523 (1.29), 2.539 (0.46), 2.669 (0.50), 2.767 (9.14), 4.190 (1.12), 7.603 (0.75), 7.623 (0.99), 7.747 (0.61), 8.339 (2.96), 8.666 (1.20).

### Example I-10

### 2-(1-{6-[(1,1-dioxidotetrahydro-2H-thiopyran-3-yl)amino]-1-[3-(trifluoromethyl)phenyl]-1H-pyrazolo[3,4-d]pyrimidin-4-yl)piperidin-4-yl)propan-2-ol

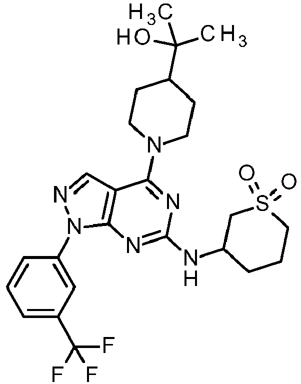

Reaction using 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol), tetrahydro-2H-thiopyran-3-amine 1,1-dioxide hydrochloride (67.5 mg, 0.36 mmol), DIPEA (0.19 ml) and NMP (2.0 ml) at 120°C for 16 h and standard workup afforded 35.0 mg of the title compound after purification by HPLC (method HT_PREP_acid).
LC-MS (method HTpost_acid): Rₜ = 1.24 min; MS (ESIpos): m/z = 553 [M+H]⁺
¹H-NMR (400 MHz, DMSO-d6) δ [ppm]: 1.052 (16.00), 1.231 (0.49), 1.261 (0.51), 1.564 (0.56), 1.835 (1.00), 1.864 (0.92), 2.067 (0.66), 2.331 (0.46), 2.518 (2.73), 2.522 (1.66), 2.673 (0.46), 3.087 (1.07), 3.102 (1.00), 3.427 (0.54), 3.458 (0.46), 4.190 (4.01), 7.614 (0.72), 7.633 (1.05), 7.697 (0.51), 7.716 (0.79), 8.354 (3.40), 8.627 (0.46).

### Example I-11-1

### (3S)-3-({4-[4-(2-hydroxypropan-2-yl)piperidin-1-yl]-1-[3-(trifluoromethyl)phenyl]-1H-pyrazolo[3,4-d]pyrimidin-6-yl}amino)piperidin-2-one

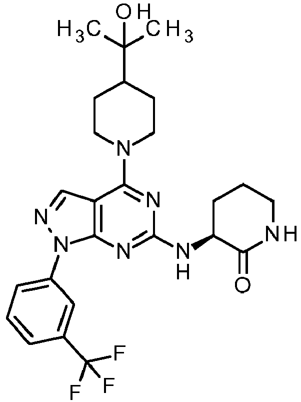

Reaction using 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol), (3S)-3-aminopiperidin-2-one hydrochloride (54.8 mg, 0.36 mmol), DIPEA (0.19 ml) and NMP (2.0 ml) at 120°C for 16 h and standard workup afforded 56.0 mg of the title compound after purification by HPLC (method HT_PREP _acid).
LC-MS (method HTpost_acid): Rₜ = 1.23 min; MS (ESIpos): m/z = 518 [M+H]⁺
¹H-NMR (400 MHz, DMSO-d6) δ [ppm]: 1.052 (16.00), 1.224 (0.56), 1.232 (0.59), 1.254 (0.63), 1.263 (0.61), 1.562 (0.52), 1.838 (1.23), 1.863 (1.10), 2.518 (2.25), 2.522 (1.35), 3.186 (1.02), 4.184 (3.16), 7.612 (0.72), 7.631 (0.95), 7.731 (0.51), 7.752 (0.82), 8.335 (3.60).

### Example I-11-2

### (3R)-3-({4-[4-(2-hydroxypropan-2-yl)piperidin-1-yl]-1-[3-(trifluoromethyl)phenyl]-1H-pyrazolo[3,4-d]pyrimidin-6-yl}amino)piperidin-2-one

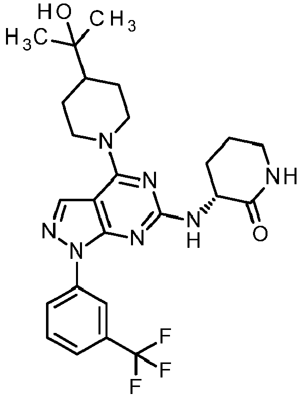

Reaction using 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol), (3R)-3-aminopiperidin-2-one (41.5 mg, 0.36 mmol), DIPEA (0.19 ml) and NMP (2.0 ml) at 120°C for 16 h and standard workup afforded 56.1 mg of the title compound after purification by HPLC (method HT_PREP_acid). LC-MS (method HTpost_acid): Rₜ = 1.23 min; MS (ESIpos): m/z = 518 [M+H]⁺
¹H-NMR (400 MHz, DMSO-d6) δ [ppm]: 1.052 (16.00), 1.224 (0.56), 1.232 (0.59), 1.255 (0.64), 1.263 (0.60), 1.561 (0.52), 1.838 (1.21), 1.862 (1.10), 2.518 (1.87), 2.523 (1.21), 3.181 (1.01), 3.186 (1.01), 4.184 (2.83), 7.612 (0.72), 7.631 (0.94), 7.731 (0.51), 7.752 (0.82), 8.335 (3.67).

### Example I-12

### (3S)-3-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one

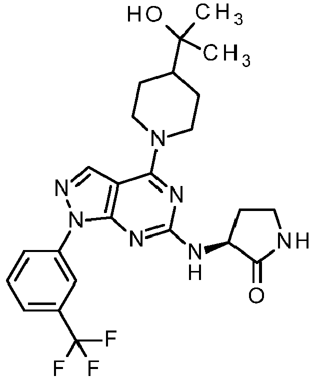

DIPEA (0.19 ml) was added to 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol), (3S)-3-aminopyrrolidin-2-one (36.4 mg, 0.36 mmol) in NMP (2.0 ml) and the mixture was stirred at 120°C for 16 h. Standard workup and purification by HPLC (method HT_PREP_acid) afforded 32.0 mg of the title compound.
LC-MS (method HTpost_acid): Rₜ = 1.13 min; MS (ESIpos): m/z = 504 [M+H]⁺
¹H-NMR (400 MHz, DMSO-d6) δ [ppm]: 1.051 (16.00), 1.222 (0.57), 1.230 (0.60), 1.253 (0.65), 1.262 (0.62), 1.562 (0.53), 1.836 (0.82), 1.867 (0.74), 2.326 (0.47), 2.518 (1.41), 2.522 (0.87), 2.539 (0.84), 3.209 (0.54), 3.231 (0.92), 3.249 (0.82), 4.189 (2.72), 4.405 (0.56), 4.431 (0.54), 7.608 (0.70), 7.627 (0.90), 7.758 (0.52), 8.339 (3.70), 8.590 (0.42), 8.607 (0.42), 8.673 (1.65) .

### Example I-13-1

### 2-[1-(6-{[(3S)-1-methylpyrrolidin-3-yl]amino}-1-[3-(trifluoromethyl)phenyl]-1H-pyrazolo[3,4-d]pyrimidin-4-yl)piperidin-4-yl]propan-2-ol

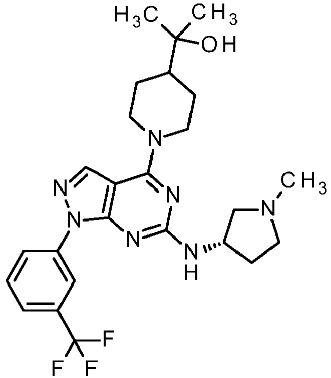

Reaction using 2-(1-{6-chloro-1-[3-(trifluoromethyl)phenyl]-1H-pyrazolo[3,4-d]pyrimidin-4-yl}piperidin-4-yl)propan-2-ol (90.0 mg), (3S)-1-methylpyrrolidin-3-amine dihydrochloride (70.8 mg), DIPEA (0.21 ml) and NMP (2.0 ml) at 120°C for 16 h and standard workup afforded 10.0 mg of the title compound after purification by HPLC (method HT_PREP_bas).
LC-MS (method HTpost_bas): Rₜ = 1.38 min; MS (ESIpos): m/z = 504 [M+H]⁺
¹H-NMR (400 MHz, DMSO-d6) δ [ppm]: 1.050 (16.00), 1.224 (0.53), 1.249 (0.56), 1.552 (0.46), 1.828 (0.77), 1.858 (0.71), 2.235 (11.01), 2.398 (0.47), 2.413 (0.48), 2.518 (2.29), 2.522 (1.51), 2.551 (0.40), 2.768 (0.61), 2.786 (0.76), 2.791 (0.69), 2.809 (0.55), 4.182 (3.16), 7.080 (0.44), 7.597 (0.71), 7.616 (0.93), 7.743 (0.59), 8.315 (4.04).

### Example I-13-2

### 2-[1-(6-{[(3R)-1-methylpyrrolidin-3-yl]amino}-1-[3-(trifluoromethyl)phenyl]-1H-pyrazolo[3,4-d]pyrimidin-4-yl)piperidin-4-yl]propan-2-ol

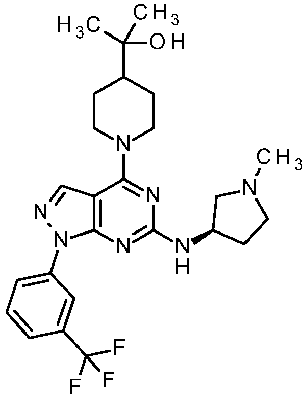

Reaction of 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol) and (3R)-1-methylpyrrolidin-3-amine (36.4 mg, 0.36 mmol) and DIPEA (0.19 ml) in 1-methyl-2-pyrrolidinone (2.0 ml) at 130°C for 16 h and standard workup afforded 23 mg of the title compound after purification by HPLC (method HT_PREP_bas).
LC-MS (method HTpost_bas): Rₜ = 1.39 min; MS (ESIpos): m/z = 504 [M+H]⁺
¹H-NMR (400 MHz, DMSO-d6) δ [ppm]: 1.050 (16.00), 1.225 (0.52), 1.247 (0.53), 1.552 (0.43), 1.829 (0.73), 1.860 (0.67), 2.084 (1.02), 2.235 (10.89), 2.300 (0.74), 2.518 (2.68), 2.523 (1.80), 2.769 (0.57), 2.786 (0.69), 2.791 (0.63), 2.809 (0.51), 4.181 (4.59), 7.597 (0.66), 7.617 (0.84), 7.722 (0.40), 7.742 (0.61), 8.316 (3.93), 8.332 (0.51), 8.421 (0.45).

### Example I-14

### 2-[1-(6-{[1-(methylsulfonyl)azetidin-3-yl]amino}-1-[3-(trifluoromethyl)phenyl]-1H-pyrazolo[3,4-d]pyrimidin-4-yl)piperidin-4-yl]propan-2-ol

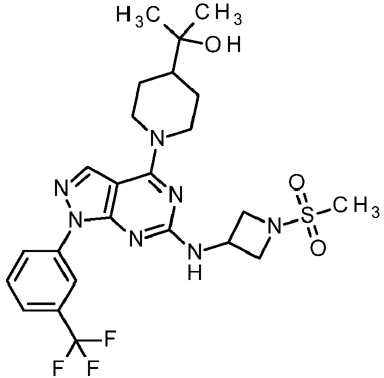

Reaction using 2-(1-{6-chloro-1-[3-(trifluoromethyl)phenyl]-1H-pyrazolo[3,4-d]pyrimidin-4-yl}piperidin-4-yl)propan-2-ol (90.0 mg), 1-(methanesulfonyl)azetidin-3-amine (61.5 mg), DIPEA (0.21 ml) and NMP (2.0 ml) at 120°C for 16 h and standard workup afforded 53.0 mg of the title compound after purification by HPLC (method HT_PREP_acid).
LC-MS (method HTpost_acid): Rₜ = 1.24 min; MS (ESIpos): m/z = 554 [M+H]⁺
¹H-NMR (400 MHz, DMSO-d6) δ [ppm]: 1.054 (16.00), 1.234 (0.61), 1.256 (0.66), 1.264 (0.63), 1.567 (0.53), 1.845 (0.79), 1.875 (0.72), 2.074 (7.87), 2.518 (1.47), 2.522 (0.91), 3.032 (14.37), 3.886 (0.76), 3.904 (1.28), 3.922 (0.91), 4.098 (1.15), 4.117 (2.12), 4.137 (1.04), 4.191 (3.98), 4.628 (0.49), 4.644 (0.48), 7.629 (0.89), 7.648 (1.20), 7.774 (0.48), 8.365 (4.20).

### Example I-15

### (3R)-3-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one

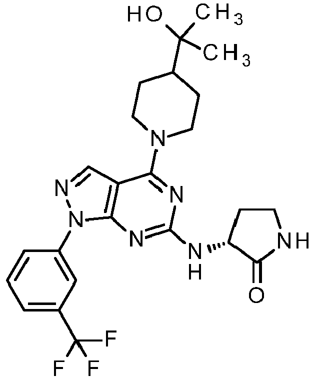

Reaction using 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol), (3R)-3-aminopyrrolidin-2-one (36.4 mg, 0.36 mmol), DIPEA (0.19 ml) and NMP (2.0 ml) at 120°C for 16 h and standard workup afforded 16.0 mg of the title compound after purification by HPLC (method HT_PREP_acid). LC-MS (method HTpost_acid): Rₜ = 1.13 min; MS (ESIpos): m/z = 504 [M+H]⁺
¹H-NMR (400 MHz, DMSO-d6) δ [ppm]: 1.051 (16.00), 1.222 (0.57), 1.231 (0.60), 1.253 (0.65), 1.562 (0.53), 1.836 (0.82), 1.865 (0.75), 2.327 (0.46), 2.518 (1.43), 2.523 (0.93), 2.539 (1.31), 3.209 (0.53), 3.232 (0.92), 3.249 (0.81), 4.190 (3.05), 4.405 (0.55), 4.431 (0.53), 7.607 (0.70), 7.627 (0.89), 7.757 (0.51), 8.339 (3.78), 8.591 (0.42), 8.608 (0.43), 8.672 (1.61).

### Example I-16-1

### 2-(1-{6-[(3S)-tetrahydrofuran-3-ylamino]-1-[3-(trifluoromethyl)phenyl]-1H-pyrazolo[3,4-d]pyrimidin-4-yl}piperidin-4-yl)propan-2-ol

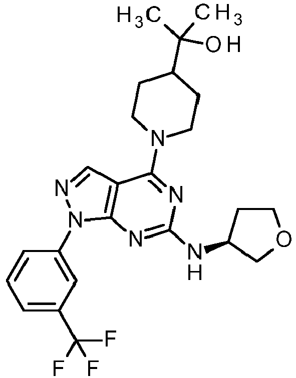

A solution of 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol) and (3S)-tetrahydrofuran-3-amine 4-methylbenzenesulfonate (70.7 mg, 0.27 mmol) in NMP (2.0 ml) was treated with DIPEA (0.19 ml) and the mixture was stirred at 120°C for 16 h.Standard workup and purification by HPLC (method HT_PREP_acid) afforded 49.0 mg of the title compound.
LC-MS (method HTpost_acid): Rₜ = 1.32 min; MS (ESIpos): m/z = 491 [M+H]⁺
¹H-NMR (400 MHz, DMSO-d6) δ [ppm]: 1.051 (16.00), 1.229 (0.54), 1.253 (0.57), 1.558 (0.46), 1.832 (0.76), 1.863 (0.69), 1.961 (0.44), 2.142 (0.42), 2.161 (0.47), 2.518 (2.25), 2.522 (1.41), 2.539 (0.73), 3.692 (0.44), 3.706 (0.48), 3.712 (0.94), 3.727 (0.93), 3.732 (0.65), 3.747 (0.56), 3.808 (0.52), 3.826 (1.09), 3.846 (0.86), 3.910 (0.91), 3.925 (1.12), 3.932 (0.98), 3.947 (0.86), 4.182 (4.55), 7.222 (0.43), 7.604 (0.73), 7.623 (0.97), 7.726 (0.43), 7.746 (0.67), 8.334 (4.02).

### Example I-16-2

### 2-(1-{6-[(3R)-tetrahydrofuran-3-ylamino]-1-[3-(trifluoromethyl)phenyl]-1H-pyrazolo[3,4-d]pyrimidin-4-yl}piperidin-4-yl)propan-2-ol

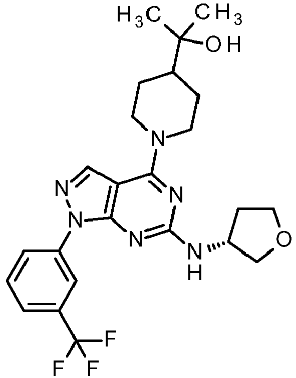

Synthesis as described for the previous example using 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol) and (3R)-tetrahydrofuran-3-amine 4-methylbenzenesulfonate (70.7 mg, 0.27 mmol) afforded 10.0 mg of the title compound.
LC-MS (method HTpost_acid): Rₜ = 1.32 min; MS (ESIpos): m/z = 491 [M+H]⁺
¹H-NMR (400 MHz, DMSO-d6) δ [ppm]: 1.051 (16.00), 1.230 (0.53), 1.252 (0.56), 1.558 (0.46), 1.833 (0.76), 1.862 (0.70), 1.961 (0.43), 2.142 (0.42), 2.161 (0.46), 2.518 (2.37), 2.522 (1.42), 2.539 (0.98), 3.692 (0.44), 3.706 (0.48), 3.712 (0.94), 3.726 (0.94), 3.732 (0.66), 3.747 (0.56), 3.808 (0.52), 3.826 (1.09), 3.846 (0.86), 3.910 (0.91), 3.925 (1.13), 3.932 (0.98), 3.947 (0.86), 4.182 (4.44), 7.222 (0.42), 7.604 (0.72), 7.623 (0.97), 7.726 (0.42), 7.746 (0.67), 8.334 (4.18).

### Example I-17

### (4S)-4-({4-[4-(2-hydroxypropan-2-yl)piperidin-1-yl]-1-[3-(trifluoromethyl)phenyl]-1H-pyrazolo[3,4-d]pyrimidin-6-yl}amino)-3,3-dimethylpyrrolidin-2-one

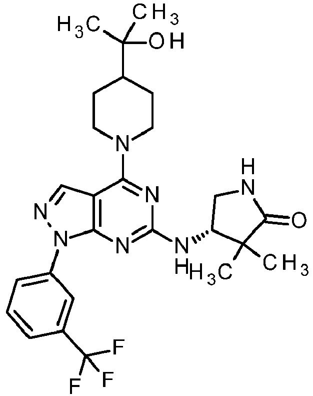

A solution of 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol) and (4S)-4-amino-3,3-dimethylpyrrolidin-2-one (46.6 mg, 0.36 mmol) in NMP (2.0 ml) was treated with DIPEA (0.19 ml) and the mixture was stirred at 130°C for 72 h. Standard workup and purification by HPLC (method HT_PREP _bas) afforded 64.0 mg of the title compound.
LC-MS (method HTpost_bas): Rₜ = 1.22 min; MS (ESIpos): m/z = 532 [M+H]⁺
¹H-NMR (400 MHz, DMSO-d6) δ [ppm]: 0.931 (9.83), 1.053 (16.00), 1.104 (12.76), 1.231 (0.77), 1.242 (0.72), 1.262 (0.87), 1.528 (0.42), 1.558 (0.67), 1.836 (1.02), 1.867 (0.92), 2.518 (2.45), 2.523 (1.70), 2.540 (1.80), 3.007 (0.47), 3.127 (0.42), 3.149 (0.77), 3.171 (0.50), 3.392 (0.75), 3.413 (1.32), 3.435 (0.60), 4.190 (5.17), 4.640 (0.52), 7.131 (0.47), 7.610 (2.85), 7.624 (1.72), 7.723 (0.72), 7.743 (1.17), 7.763 (0.55), 8.332 (6.02), 8.800 (0.52).

### Example I-18

### 3-ethyl-1-(6-{[(3S)-1-methylpyrrolidin-3-yl]amino}-1-[3-(trifluoromethyl)phenyl]-1H-pyrazolo[3,4-d]pyrimidin-4-yl)azetidin-3-ol

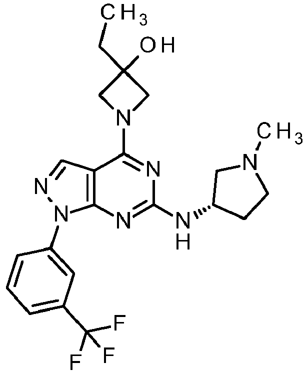

DIPEA (0.21 ml) was added to 1-{6-chloro-1-[3-(trifluoromethyl)phenyl]-1H-pyrazolo[3,4-d]pyrimidin-4-yl}-3-ethylazetidin-3-ol (80.0 mg, 0.20 mmol) and (3S)-1-methylpyrrolidin-3-amine dihydrochloride (69.6 mg, 0.40 mmol) in NMP (2.2 ml) and the mixture was stirred at 130°C for 16 h. Standard workup and purification by HPLC (method HT_PREP_bas) afforded 17.0 mg of the title compound.
¹H-NMR (400 MHz, DMSO-d6) δ [ppm]: 0.914 (2.52), 0.932 (6.18), 0.950 (2.80), 1.709 (0.66), 1.727 (1.94), 1.745 (2.04), 1.764 (0.99), 2.149 (0.40), 2.166 (0.51), 2.185 (0.49), 2.200 (0.40), 2.229 (16.00), 2.407 (0.94), 2.424 (1.08), 2.518 (4.34), 2.522 (2.91), 2.548 (0.46), 2.794 (0.69), 2.812 (0.94), 2.834 (0.64), 3.939 (0.51), 4.018 (0.51), 4.150 (0.55), 4.306 (0.88), 5.751 (2.93), 7.226 (0.51), 7.592 (1.08), 7.612 (1.40), 7.723 (0.59), 7.744 (0.92), 7.763 (0.47), 8.085 (7.61), 8.475 (0.44), 8.959 (0.64).

### Example I-19

### 1-{6-[(1,1-dioxidotetrahydro-2H-thiopyran-4-yl)amino]-1-[3-(trifluoromethyl)phenyl]-1H-pyrazolo[3,4-d]pyrimidin-4-yl}-3-ethlazetidin-3-ol

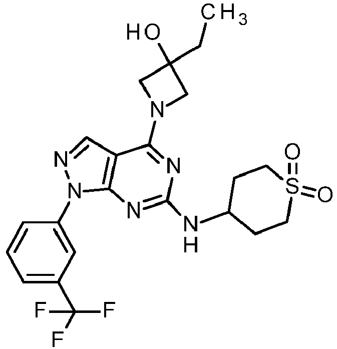

Reaction using 1-{6-chloro-1-[3-(trifluoromethyl)phenyl]-1H-pyrazolo[3,4-d]pyrimidin-4-yl}-3-ethylazetidin-3-ol (50.0 mg, 126 µmol), tetrahydro-2H-thiopyran-4-amine 1,1-dioxide, DIPEA and NMP using conditions similar to the preparation of example I-3-1 afforded 29.0 mg of the title compound after purification by HPLC (method HT_PREP_acid).
LC-MS (method HTpost_acid): Rₜ = 1.13 min; MS (ESIpos): m/z = 511 [M+H]⁺
¹H-NMR (400 MHz, DMSO-d6) δ [ppm]: 0.918 (4.60), 0.937 (11.17), 0.947 (2.37), 0.954 (5.29), 1.715 (1.24), 1.733 (3.68), 1.752 (3.54), 1.769 (1.17), 2.074 (1.49), 2.094 (1.81), 2.109 (1.38), 2.221 (1.84), 2.518 (6.55), 2.522 (4.07), 2.539 (16.00), 2.692 (1.12), 3.194 (4.69), 3.282 (0.67), 3.959 (1.04), 4.040 (1.15), 4.141 (1.40), 4.312 (1.12), 5.757 (3.73), 7.365 (0.62), 7.614 (1.86), 7.633 (2.35), 7.749 (0.88), 7.769 (1.38), 7.790 (0.83), 8.106 (13.17), 8.419 (1.04), 8.432 (0.94), 8.573 (0.96), 8.683 (0.41), 8.757 (0.69).

### Example I-20-1

### (4R)-4-({4-[(3S,4S)-3,4-dihydroxypyrrolidin-1-yl]-1-[3-(trifluoromethyl)phenyl]-1H-pyrazolo[3,4-d]pyrimidin-6-yl}amino)pyrrolidin-2-one

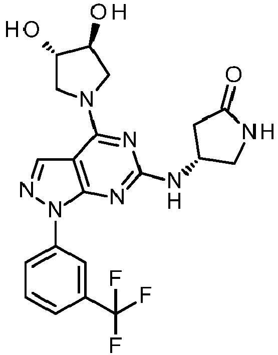

Reaction using (3S,4S)-1-{6-chloro-1-[3-(trifluoromethyl)phenyl]-1H-pyrazolo[3,4-d]pyrimidin-4-yl}pyrrolidine-3,4-diol (60.0 mg, 150 µmol), (4R)-4-aminopyrrolidin-2-one hydrochloride, DIPEA and NMP using conditions similar to the preparation of example I-3-1 afforded 34.0 mg of the title compound after purification by HPLC (method HT_PREP_acid).
LC-MS (method HTpost_acid): Rₜ = 0.85 min; MS (ESIpos): m/z = 464 [M+H]⁺
¹H-NMR (400 MHz, DMSO-d6) δ [ppm]: 2.297 (2.29), 2.313 (2.40), 2.322 (1.32), 2.326 (1.73), 2.331 (1.51), 2.338 (3.45), 2.354 (3.32), 2.518 (8.72), 2.522 (6.26), 2.539 (3.64), 2.562 (1.89), 2.664 (1.16), 2.668 (1.54), 2.673 (1.16), 3.229 (1.43), 3.601 (3.24), 3.616 (2.59), 3.630 (4.48), 3.640 (3.10), 3.659 (2.78), 3.679 (3.70), 3.954 (1.78), 3.963 (2.19), 3.981 (1.81), 3.990 (1.73), 4.035 (3.35), 4.164 (3.13), 4.568 (1.24), 5.239 (2.59), 5.345 (2.70), 7.369 (1.13), 7.603 (2.62), 7.622 (3.64), 7.640 (6.15), 7.730 (1.21), 7.749 (1.86), 8.243 (16.00), 8.520 (0.62), 8.620 (0.46), 8.887 (0.59).

### Example I-20-2

### (4S)-4-({4-[(3S,4S)-3,4-dihydroxypyrrolidin-1-yl]-1-[3-(trifluoromethyl)phenyl]-1H-pyrazolo[3,4-d]pyrimidin-6-yl}amino)pyrrolidin-2-one

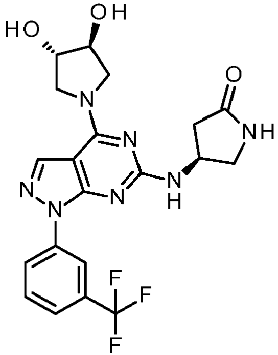

Reaction using (3S,4S)-1-{6-chloro-1-[3-(trifluoromethyl)phenyl]-1H-pyrazolo[3,4-d]pyrimidin-4-yl}pyrrolidine-3,4-diol (65.0 mg, 0.16 mmol), (4S)-4-aminopyrrolidin-2-one hydrochloride, DIPEA and NMP using conditions similar to the preparation of example I-3-1 afforded 18.0 mg of the title compound after purification by HPLC (method HT_PREP_acid).
LC-MS (method HTpost_acid): Rₜ = 0.85 min; MS (ESIpos): m/z = 464 [M+H]⁺
¹H-NMR (400 MHz, DMSO-d6) δ [ppm]: 2.296 (1.21), 2.313 (1.33), 2.323 (1.40), 2.327 (1.85), 2.332 (1.78), 2.337 (2.07), 2.354 (1.78), 2.518 (6.42), 2.523 (4.99), 2.527 (4.14), 2.540 (1.50), 2.548 (2.23), 2.569 (1.88), 2.665 (1.05), 2.669 (1.36), 2.673 (1.00), 3.202 (1.59), 3.215 (1.88), 3.226 (2.00), 3.240 (1.93), 3.605 (3.57), 3.611 (3.19), 3.632 (6.61), 3.655 (3.02), 3.687 (2.97), 3.951 (1.93), 3.961 (2.38), 3.978 (1.95), 3.988 (1.83), 4.036 (3.49), 4.163 (3.16), 4.568 (1.33), 5.235 (2.07), 5.346 (2.16), 7.368 (1.14), 7.603 (2.73), 7.623 (4.18), 7.634 (6.32), 7.729 (1.28), 7.748 (1.93), 8.243 (16.00), 8.530 (0.64), 8.622 (0.50), 8.885 (0.67).

### Example I-21

### (4S)-4-({4-[(3R)-3-(morpholin-4-yl)pyrrolidin-1-yl]-1-[3-(trifluoromethyl)phenyl]-1H-pyrazolo[3,4-d]pyrimidin-6-yl}amino)pyrrolidin-2-one

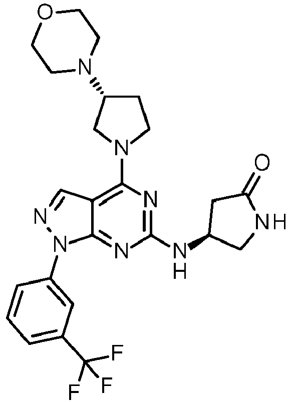

A solution of 6-chloro-4-[(3R)-3-(morpholin-4-yl)pyrrolidin-1-yl]-1-[3-(trifluoromethyl)phenyl]-1H-pyrazolo[3,4-d]pyrimidine (120 mg) and (4S)-4-aminopyrrolidin-2-one hydrochloride (72.4 mg) in NMP (2.0 ml) was treated with DIPEA (0.28 ml) and the mixture was stirred at 130°C for 16 h. Standard workup and purification by HPLC (method HT_PREP_bas) afforded 31 mg of the title compound.
LC-MS (method HTpost_bas): Rₜ = 1.06 min; MS (ESIpos): m/z = 517 [M+H]⁺
¹H-NMR (400 MHz, DMSO-d6) δ [ppm]: 0.931 (0.89), 0.947 (0.85), 1.228 (0.61), 1.751 (0.73), 1.775 (0.97), 1.801 (0.77), 1.922 (0.77), 1.952 (1.06), 1.974 (0.85), 2.162 (1.10), 2.290 (3.33), 2.305 (3.29), 2.332 (5.32), 2.347 (3.86), 2.413 (1.58), 2.522 (13.52), 2.539 (10.23), 2.556 (4.30), 2.665 (1.75), 2.669 (2.27), 2.673 (1.66), 2.692 (0.45), 2.884 (1.14), 3.010 (1.10), 3.205 (2.31), 3.216 (2.36), 3.498 (1.83), 3.521 (2.11), 3.544 (1.26), 3.609 (13.85), 3.619 (16.00), 3.740 (1.22), 3.758 (1.26), 3.904 (1.58), 3.924 (1.87), 3.967 (1.99), 3.985 (2.68), 4.010 (1.87), 4.562 (1.83), 7.362 (1.79), 7.601 (4.35), 7.622 (7.80), 7.725 (1.95), 7.745 (2.92), 8.188 (4.59), 8.309 (5.04), 8.496 (1.10), 8.611 (0.77), 8.874 (0.97).

### Example I-22

### N-[(3R)-1,1-dioxidotetrahydrothiophen-3-yl]-4-[3-(morpholin-4-yl)azetidin-1-yl]-1-[3-(trifluoromethyl)phenyl]-1H-pyrazolo[3,4-d]pyrimidin-6-amine

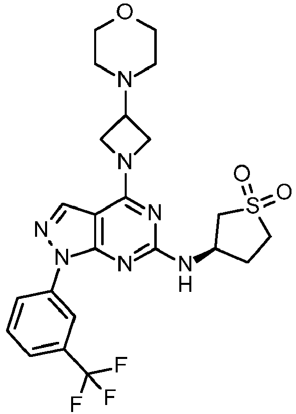

Reaction using 6-chloro-4-[3-(morpholin-4-yl)azetidin-1-yl]-1-[3-(trifluoromethyl)phenyl]-1H-pyrazolo[3,4-d]pyrimidine (100 mg, 0.23 mmol), (3R)tetrahydrothiophen-3-amine 1,1-dioxide hydrochloride (78.2 mg, 0.46 mmol), DIPEA (0.24 ml) and NMP (2.0 ml) at 130°C for 72 h and standard workup afforded 40.0 mg of the title compound after purification by HPLC (acetonitrile / water + 0.1% formic acid).
¹H-NMR (400 MHz, DMSO-d6) δ [ppm]: 2.188 (0.66), 2.208 (1.77), 2.230 (2.08), 2.242 (2.50), 2.263 (2.36), 2.283 (0.97), 2.326 (2.26), 2.668 (2.12), 2.728 (0.42), 2.888 (0.42), 3.103 (2.19), 3.124 (3.16), 3.138 (3.19), 3.157 (3.02), 3.178 (1.42), 3.373 (5.41), 3.386 (4.82), 3.403 (2.64), 3.419 (1.32), 3.495 (1.80), 3.514 (2.05), 3.528 (1.84), 3.547 (1.67), 3.626 (14.02), 4.000 (1.25), 4.203 (1.53), 4.286 (1.70), 4.441 (1.60), 4.617 (1.39), 4.634 (2.46), 4.651 (2.36), 4.668 (1.28), 7.589 (1.01), 7.620 (3.61), 7.638 (4.55), 7.728 (2.57), 7.749 (4.16), 7.769 (2.01), 8.115 (16.00), 8.529 (1.21), 8.713 (0.97).

### Example I-23

### N-(1,1-dioxidotetrahydro-2H-thiopyran-4-yl)-4-[3-(morpholin-4-yl)azetidin-1-yl]-1-[3-(trifluoromethyl)phenyl]-1H-pyrazolo[3,4-d]pyrimidin-6-amine

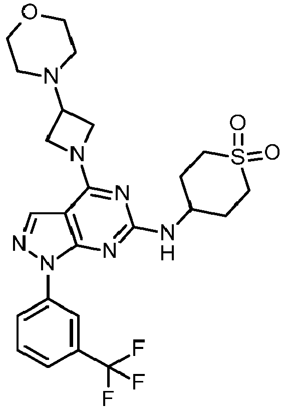

DIPEA (0.19 ml) was added to 6-chloro-4-[3-(morpholin-4-yl)azetidin-1-yl]-1-[3-(trifluoromethyl)phenyl]-1H-pyrazolo[3,4-d]pyrimidine (1 equivalent, 80.0 mg, 0.18 mmol) and tetrahydro-2H-thiopyran-4-amine 1,1-dioxide (54.4 mg) in NMP (2.0 ml). The mixture was stirred at 100°C for 16 h, then again tetrahydro-2H-thiopyran-4-amine 1,1-dioxide (1 equivalent) and DIPEA (3 equivalents) were added and stirring was continued for 72 h at 130°C. Standard workup and purification by HPLC (method HT_PREP_acid) afforded 28.0 mg of the title compound.
LC-MS (method HTpost_acid): Rₜ = 0.92 min; MS (ESIpos): m/z = 552 [M+H]⁺
¹H-NMR (400 MHz, DMSO-d6) δ [ppm]: 2.073 (1.97), 2.094 (2.36), 2.112 (1.85), 2.222 (2.56), 2.404 (7.35), 2.518 (7.67), 2.523 (5.19), 2.540 (1.93), 3.193 (7.12), 3.366 (3.89), 3.378 (2.36), 3.626 (10.46), 3.996 (0.94), 4.113 (1.26), 4.195 (1.14), 4.273 (1.18), 4.425 (1.02), 7.367 (0.79), 7.616 (2.48), 7.635 (3.14), 7.750 (1.22), 7.770 (1.85), 8.087 (16.00), 8.421 (0.39), 8.573 (1.18), 8.745 (0.86).

### Example I-24

### (4S)-4-({4-[4-(2-hydroxy-2-methylpropyl)piperidin-1-yl]-1-[3-(trifluoromethyl)phenyl]-1H-pyrazolo[3,4-d]pyrimidin-6-yl}amino)pyrrolidin-2-one

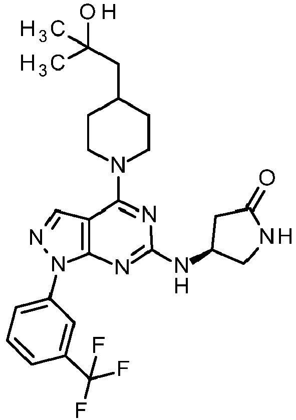

DIPEA (0.23 ml) was added to 1-(1-{6-chloro-1-[3-(trifluoromethyl)phenyl]-1H-pyrazolo[3,4-d]pyrimidin-4-yl}piperidin-4-yl)-2-methylpropan-2-ol (100 mg, 0.22 mmol) and (4S)-4-aminopyrrolidin-2-one hydrochloride (60.2 mg, 0.44 mmol) in NMP (1.5 ml) and the mixture was stirred for 16 h at 130°C. Standard workup and purification by HPLC (method HT_PREP _acid) afforded 63 mg of the title compound.
LC-MS (method a01): Rₜ = 1.21 min; MS (ESIpos): m/z = 518 [M+H]⁺
¹H-NMR (400 MHz, DMSO-d6) δ [ppm]: 1.129 (16.00), 1.158 (0.47), 1.188 (0.46), 1.317 (1.62), 1.330 (1.61), 1.887 (0.57), 1.921 (0.48), 2.074 (0.79), 2.265 (0.47), 2.280 (0.48), 2.307 (0.65), 2.322 (0.96), 2.326 (0.57), 2.518 (1.90), 2.523 (1.24), 2.669 (0.44), 3.319 (0.60), 4.132 (3.78), 7.608 (0.58), 7.631 (1.20), 7.747 (0.44), 8.332 (3.24).

### Example I-25-1

### 2-methyl-1-[1-(6-{[(3R)-1-methylpyrrolidin-3-yl]amino}-1-[3-(trifluoromethyl)phenyl]-1H-pyrazolo[3,4-d]pyrimidin-4-yl)piperidin-4-yl]propan-2-ol

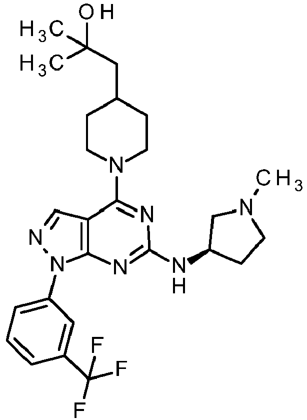

Reaction of 1-(1-{6-chloro-1-[3-(trifluoromethyl)phenyl]-1H-pyrazolo[3,4-d]pyrimidin-4-yl}piperidin-4-yl)-2-methylpropan-2-ol (100 mg, 0.22 mmol), (3R)-1-methylpyrrolidin-3-amine (44.1 mg, 0.44 mmol) and DIPEA (0.23 ml) in NMP (1.5 ml) at 130°C for 16 h and standard workup afforded 28.3 mg of the title compound after purification by HPLC (method HT_PREP _bas) .
LC-MS (method HTpost_bas): Rₜ = 1.48 min; MS (ESIneg): m/z = 516 [M-H]⁻
¹H-NMR (400 MHz, DMSO-d6) δ [ppm]: 1.128 (16.00), 1.187 (0.47), 1.316 (1.74), 1.330 (1.76), 1.882 (0.58), 1.915 (0.52), 2.234 (8.19), 2.299 (0.58), 2.518 (1.94), 2.523 (1.30), 2.782 (0.46), 4.129 (3.56), 7.596 (0.52), 7.615 (0.68), 7.741 (0.51), 8.301 (3.04), 8.318 (0.49).

### Example I-25-2

### 2-methyl-1-[1-(6-{[(3S)-1-methylpyrrolidin-3-yl]amino}-1-[3-(trifluoromethyl)phenyl]-1H-pyrazolo[3,4-d]pyrimidin-4-yl)piperidin-4-yl]propan-2-ol

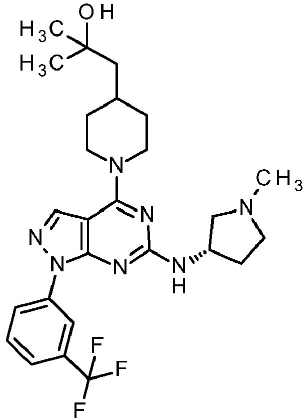

Reaction of 1-(1-{6-chloro-1-[3-(trifluoromethyl)phenyl]-1H-pyrazolo[3,4-d]pyrimidin-4-yl}piperidin-4-yl)-2-methylpropan-2-ol (100 mg, 0.22 mmol), (3S)-1-methylpyrrolidin-3-amine dihydrochloride (76.3 mg, 0.44 mmol) and DIPEA (0.23 ml) in NMP (1.5 ml) at 130°C for 16 h and standard workup afforded 28.2 mg of the title compound after purification by HPLC (method HT_PREP_bas).
LC-MS (method HTpost_bas): Rₜ = 1.48 min; MS (ESIneg): m/z = 516 [M-H]⁻
¹H-NMR (400 MHz, DMSO-d6) δ [ppm]: 1.128 (16.00), 1.182 (0.52), 1.316 (1.74), 1.329 (1.79), 1.880 (0.62), 1.914 (0.55), 2.233 (8.76), 2.518 (2.03), 2.522 (1.37), 2.782 (0.52), 4.129 (2.31), 7.595 (0.57), 7.615 (0.72), 7.741 (0.47), 8.301 (3.23).

### Example I-26-1

### 4-[3-(1,1-dioxidothiomorpholin-4-yl)azetidin-1-yl]-N-[(3R)-tetrahydrofuran-3-yl]-1-[3-(trifluoromethyl)phenyl]-1H-pyrazolo[3,4-d]pyrimidin-6-amine

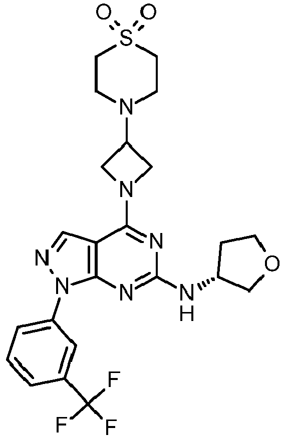

DIPEA (0.15 ml) was added to a solution of 6-chloro-4-[3-(1,1-dioxidothiomorpholin-4-yl)azetidin-1-yl]-1-[3-(trifluoromethyl)phenyl]-1H-pyrazolo[3,4-d]pyrimidine (70.0 mg, 0.14 mmol) and (3R)-tetrahydrofuran-3-amine 4-methylbenzenesulfonate (74.6 mg, 0.29 mmol) in NMP (2.0 ml) and the mixture was stirred at 130°C for 16 h. Standard workup and purification by HPLC (method HT_PREP_acid) afforded 32.0 mg of the title compound.
LC-MS (method HTpost_acid): Rₜ = 1.22 min; MS (ESIpos): m/z = 538 [M+H]⁺
¹H-NMR (400 MHz, DMSO-d6) δ [ppm]: 1.953 (1.06), 1.966 (1.18), 2.112 (0.70), 2.131 (1.86), 2.149 (1.98), 2.162 (1.55), 2.168 (1.04), 2.181 (1.40), 2.200 (0.53), 2.336 (0.46), 2.518 (5.12), 2.522 (3.26), 2.539 (0.44), 2.885 (7.15), 3.131 (0.87), 3.169 (8.12), 3.628 (1.43), 3.645 (2.25), 3.658 (2.95), 3.670 (1.84), 3.685 (2.25), 3.699 (1.96), 3.705 (3.63), 3.720 (3.65), 3.725 (2.56), 3.739 (2.20), 3.796 (1.74), 3.814 (3.72), 3.834 (2.97), 3.852 (1.21), 3.919 (2.30), 3.934 (2.97), 3.941 (2.73), 3.957 (2.61), 4.255 (1.35), 4.393 (1.45), 4.459 (1.06), 7.399 (0.97), 7.601 (2.66), 7.621 (3.46), 7.727 (1.50), 7.747 (2.37), 7.766 (1.16), 7.927 (0.41), 8.078 (16.00), 8.478 (0.80), 8.907 (0.89).

### Example I-26-2

### 4-[3-(1,1-dioxidothiomorpholin-4-yl)azetidin-1-yl]-N-[(3S)-tetrahydrofuran-3-yl]-1-[3-(trifluoromethyl)phenyl]-1H-pyrazolo[3,4-d]pyrimidin-6-amine

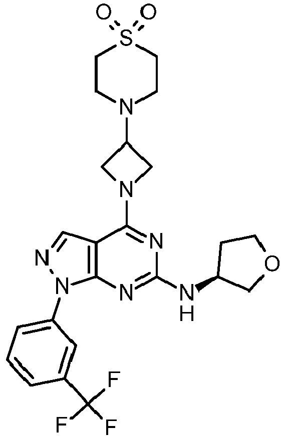

Reaction of 6-chloro-4-[3-(1,1-dioxidothiomorpholin-4-yl)azetidin-1-yl]-1-[3-(trifluoromethyl)-phenyl]-1H-pyrazolo[3,4-d]pyrimidine (70.0 mg, 0.14 mmol) and (3S)-tetrahydrofuran-3-amine 4-methylbenzenesulfonate (74.6 mg, 0.29 mmol) as described for the previous example and purification by HPLC (method HT_PREP_acid) afforded 32 mg of the title compound.
LC-MS (method HTpost_acid): Rₜ = 1.22 min; MS (ESIpos): m/z = 538 [M+H]⁺
¹H-NMR (400 MHz, DMSO-d6) δ [ppm]: 1.953 (1.07), 1.967 (1.21), 2.112 (0.76), 2.132 (1.88), 2.149 (1.99), 2.162 (1.54), 2.168 (1.07), 2.181 (1.40), 2.200 (0.53), 2.518 (6.40), 2.522 (3.99), 2.539 (0.79), 2.884 (7.27), 3.131 (1.15), 3.169 (8.28), 3.628 (1.52), 3.646 (2.33), 3.658 (2.98), 3.670 (1.91), 3.685 (2.25), 3.699 (2.02), 3.705 (3.65), 3.720 (3.71), 3.725 (2.58), 3.739 (2.19), 3.796 (1.71), 3.814 (3.71), 3.834 (3.00), 3.852 (1.21), 3.918 (2.30), 3.934 (3.00), 3.941 (2.75), 3.957 (2.64), 4.252 (1.40), 4.393 (1.49), 4.459 (1.04), 7.400 (0.98), 7.602 (2.64), 7.621 (3.51), 7.728 (1.52), 7.747 (2.41), 7.766 (1.15), 8.071 (1.18), 8.078 (16.00), 8.474 (0.81), 8.906 (0.93).

### Example I-27

### (4S)-4-({4-[4-(morpholin-4-yl)piperidin-1-yl]-1-[3-(trifluoromethyl)phenyl]-1H-pyrazolo[3,4-d]pyrimidin-6-yl}amino)pyrrolidin-2-one

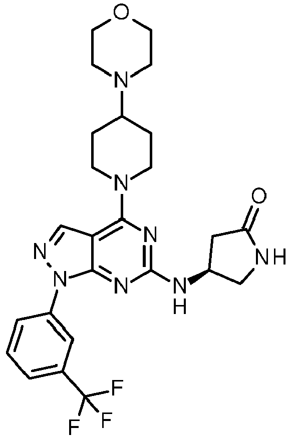

A solution of 6-chloro-4-[4-(morpholin-4-yl)piperidin-1-yl]-1-[3-(trifluoromethyl)phenyl]-1H-pyrazolo[3,4-d]pyrimidine (80.0 mg, 0.17 mmmol) and (4S)-4-aminopyrrolidin-2-one hydrochloride (46.8 mg, 0.34 mmol) in NMP was treated with DIPEA (0.18 ml) and the mixture was stirred at 130°C for 72 h. Standard workup and purification by HPLC (method HT_PREP_bas) afforded 50.0 mg of the title compound.
LC-MS (method HTpost_bas): Rₜ = 1.10 min; MS (ESIpos): m/z = 531 [M+H]⁺
¹H-NMR (400 MHz, DMSO-d6) δ [ppm]: 1.405 (1.73), 1.431 (1.83), 1.898 (2.65), 1.926 (2.31), 2.073 (0.72), 2.083 (2.02), 2.266 (2.12), 2.281 (2.27), 2.307 (2.99), 2.323 (3.71), 2.332 (0.96), 2.464 (6.84), 2.476 (9.93), 2.518 (6.36), 2.522 (5.35), 2.539 (10.99), 2.563 (1.06), 2.664 (0.77), 2.669 (1.06), 2.673 (0.77), 3.147 (1.40), 3.206 (1.40), 3.292 (0.43), 3.551 (7.61), 3.563 (10.12), 3.573 (7.52), 3.595 (1.40), 3.614 (1.78), 3.637 (1.16), 4.544 (1.16), 4.629 (1.16), 7.396 (1.25), 7.610 (2.60), 7.632 (4.48), 7.639 (4.29), 7.728 (1.25), 7.748 (1.93), 7.767 (0.96), 8.342 (16.00), 8.506 (0.67), 8.848 (0.67).

### Example I-28

### (4S)-4-({4-(morpholin-4-yl)-1-[3-(trifluoromethyl)phenyl]-1H-pyrazolo[3,4-d]pyrimidin-6-yl}amino)pyrrolidin-2-one

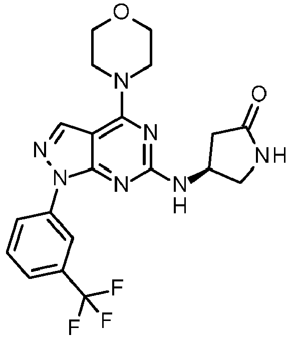

A solution of 6-chloro-4-(morpholin-4-yl)-1-[3-(trifluoromethyl)phenyl]-1H-pyrazolo[3,4-d]-pyrimidine (80.0 mg, 0.21 mmol) and (4S)-4-aminopyrrolidin-2-one hydrochloride (56.9 mg, 0.42 mol) in NMP (2.0 ml) was treated with DIPEA (0.22 ml) and the mixture was stirred at 130°C for 72 h. Standard workup and purification by HPLC (method HT_PREP_acid) afforded 73.0 mg of the title compound.
LC-MS (method HTpost_acid): Rₜ = 1.06 min; MS (ESIpos): m/z = 448 [M+H]⁺
¹H-NMR (400 MHz, DMSO-d6) δ [ppm]: 2.074 (5.91), 2.083 (1.02), 2.260 (1.35), 2.274 (1.46), 2.302 (1.85), 2.317 (2.10), 2.327 (0.76), 2.332 (0.50), 2.518 (2.91), 2.523 (2.27), 2.539 (0.86), 2.550 (0.84), 2.569 (0.65), 2.664 (0.41), 2.669 (0.59), 2.673 (0.43), 3.212 (0.95), 3.599 (0.99), 3.618 (1.46), 3.640 (0.90), 3.737 (8.81), 3.748 (7.05), 3.850 (7.50), 3.862 (8.93), 3.873 (5.25), 4.546 (0.79), 7.452 (1.20), 7.615 (2.48), 7.635 (3.99), 7.646 (3.27), 7.730 (1.13), 7.750 (1.74), 7.768 (0.88), 8.352 (16.00), 8.499 (0.67), 8.841 (0.63).

### Example I-29

### (1R,3S)-3-({4-[4-(2-hydroxypropan-2-yl)piperidin-1-yl]-1-[3-(trifluoromethyl)phenyl]-1H-pyrazolo[3,4-d]pyrimidin-6-yl}amino)cyclopentanol

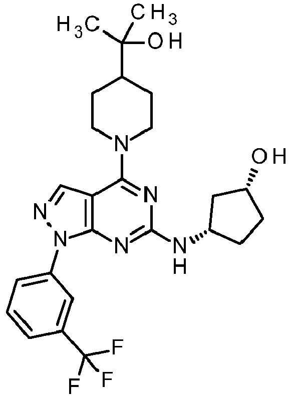

DIPEA (0.19 ml) was added to 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol) and (1R,3S)-3-aminocyclopentanol hydrochloride (50.1 mg, 0.36 mmol) in NMP (2.0 ml) and the mixture was stirred at 130°C for 72 h. Standard workup and purification by HPLC (method HT_PREP _bas) afforded 66.0 mg of the title compound.
¹H-NMR (400 MHz, DMSO-d6) δ [ppm]: 1.049 (16.00), 1.222 (0.50), 1.246 (0.55), 1.505 (0.49), 1.520 (0.64), 1.536 (0.43), 1.550 (0.46), 1.596 (0.45), 1.606 (0.42), 1.694 (0.43), 1.712 (0.74), 1.720 (0.70), 1.826 (0.74), 1.855 (0.67), 2.518 (1.23), 2.523 (0.78), 2.540 (0.55), 3.325 (0.99), 4.120 (0.56), 4.133 (0.57), 4.181 (5.13), 4.636 (1.99), 4.646 (1.93), 7.589 (0.77), 7.609 (0.99), 7.715 (0.49), 7.735 (0.81), 8.306 (4.36).

### Example I-30

### (4S)-4-({4-[3-(4-hydroxy-4-methylpiperidin-1-yl)azetidin-1-yl]-1-[3-(trifluoromethyl)phenyl]-1H-pyrazolo[3,4-d]pyrimidin-6-yl)amino)pyrrolidin-2-one

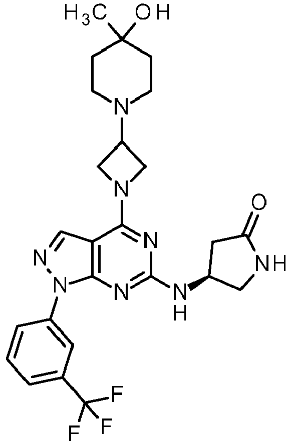

DIPEA (0.18 ml) was added to 1-(1-{6-chloro-1-[3-(trifluoromethyl)phenyl]-1H-pyrazolo[3,4-d]pyrimidin-4-yl}azetidin-3-yl)-4-methylpiperidin-4-ol (80.0 mg, 0.17 mmol) and (4S)-4-aminopyrrolidin-2-one hydrochloride (46.8 mg, 0.34 mmol) in NMP (2.0 ml) and the mixture was stirred at 130°C for 72h. Standard workup and purification by HPLC afforded 37.6 mg of the title compound.
LC-MS (method HTpost_acid): Rₜ = 0.74 min; MS (ESIpos): m/z = 531 [M+H]⁺
¹H-NMR (400 MHz, DMSO-d6) δ [ppm]: 1.112 (16.00), 1.446 (0.46), 1.469 (1.88), 1.493 (4.70), 1.526 (0.57), 2.279 (1.37), 2.296 (1.79), 2.318 (2.11), 2.323 (2.47), 2.327 (2.64), 2.331 (2.21), 2.429 (1.31), 2.518 (5.14), 2.523 (3.67), 2.551 (0.78), 2.660 (0.46), 2.665 (0.95), 2.669 (1.29), 2.673 (0.93), 2.678 (0.42), 3.217 (1.29), 3.599 (0.95), 3.619 (1.24), 3.641 (0.84), 3.932 (0.59), 4.186 (1.35), 4.428 (0.72), 4.522 (0.53), 4.540 (0.84), 4.554 (0.82), 7.510 (0.53), 7.603 (1.56), 7.626 (4.15), 7.728 (0.74), 7.748 (1.12), 7.768 (0.57), 8.098 (8.69), 8.172 (5.23), 8.500 (0.46), 8.841 (0.51).

### Example I-31

### (3R,4R)-1-(6-{[(3S)-1-methylpyrrolidin-3-yl]amino}-1-[3-(trifluoromethyl)phenyl]-1H-pyrazolo[3,4-d]pyrimidin-4-yl)pyrrolidine-3,4-diol

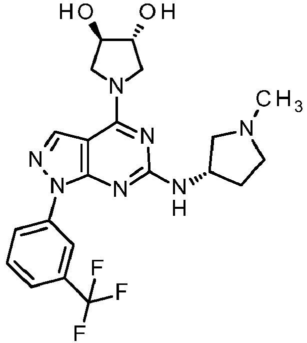

DIPEA (0.23 ml) was added to (3R,4R)-1-{6-chloro-1-[3-(trifluoromethyl)phenyl]-1H-pyrazolo[3,4-d]pyrimidin-4-yl}pyrrolidine-3,4-diol (90.5 mg) und (3S)-1-methylpyrrolidin-3-amine dihydrochloride (76.8 mg, 0.44 mmol) in NMP (1.5 ml) and the mixture was stirred for 19 h at 130°C. The mixture was filtered and diluted with 750 µl DMSO. Purification by HPLC (method HT_PREP_bas) afforded 17.4 mg of the title compound.
LC-MS (method HTpost_bas): Rₜ = 1.10 min; MS (ESIpos): m/z = 464 [M+H]⁺
¹H-NMR (400 MHz, DMSO-d6) δ [ppm]: 0.923 (1.22), 0.937 (10.03), 0.941 (3.58), 0.954 (10.03), 0.958 (1.87), 1.774 (0.52), 1.789 (0.55), 2.180 (0.49), 2.202 (0.51), 2.216 (0.42), 2.242 (16.00), 2.419 (1.16), 2.437 (1.20), 2.455 (0.74), 2.518 (4.05), 2.523 (2.73), 2.536 (0.90), 2.551 (0.69), 2.799 (1.04), 2.817 (1.25), 2.822 (1.14), 2.840 (0.95), 2.970 (0.48), 3.005 (0.68), 3.592 (1.21), 3.620 (1.42), 3.679 (1.34), 3.944 (0.69), 3.954 (0.88), 3.971 (0.72), 3.981 (0.69), 4.030 (1.17), 4.160 (1.14), 5.222 (0.87), 5.331 (0.94), 7.057 (0.65), 7.591 (0.95), 7.610 (1.24), 7.724 (0.51), 7.743 (0.77), 7.762 (0.43), 8.215 (7.02), 8.392 (0.42).

### Example I-32

### (4S)-4-({4-(3-hydroxyazetidin-1-yl)-1-[3-(trifluoromethyl)phenyl]-1H-pyrazolo[3,4-d]pyrimidin-6-yl}amino)pyrrolidin-2-one

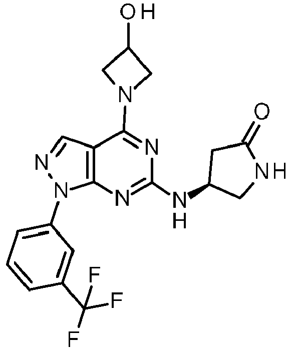

DIPEA (0.20 ml) was added to 1-{6-chloro-1-[3-(trifluoromethyl)phenyl]-1H-pyrazolo[3,4-d]pyrimidin-4-yl}azetidin-3-ol (70.0 mg, 0.19 mmol) and (4S)-4-aminopyrrolidin-2-one hydrochloride (51.7 mg, 0.38 mmol) in NMP (1.5 ml) and the mixture was stirred 19 h at 130°C. Water and ethyl acetate were added and the precipitated solid was washed with water and 2-methoxy-2-methylpropane and dried in vacuum affording 42.6 mg of the title compound.
¹H-NMR (400 MHz, DMSO-d6) δ [ppm]: 1.153 (0.63), 1.171 (1.21), 1.189 (0.57), 1.986 (2.34), 2.277 (0.95), 2.292 (1.00), 2.318 (1.72), 2.322 (1.74), 2.326 (1.82), 2.331 (1.95), 2.518 (4.63), 2.522 (3.15), 2.530 (1.32), 2.551 (1.06), 2.664 (0.83), 2.668 (1.15), 2.673 (0.82), 3.205 (0.96), 3.599 (1.09), 3.620 (1.56), 3.642 (1.00), 3.907 (0.50), 4.016 (0.70), 4.034 (0.70), 4.118 (0.52), 4.377 (0.50), 4.500 (0.41), 4.516 (0.91), 4.535 (1.46), 4.550 (1.46), 4.566 (1.00), 4.583 (0.63), 4.660 (1.09), 4.670 (1.69), 4.681 (1.28), 4.686 (1.43), 4.697 (0.80), 5.890 (5.77), 5.906 (5.69), 7.534 (0.76), 7.603 (2.28), 7.627 (5.60), 7.731 (1.09), 7.750 (1.72), 7.769 (0.85), 8.102 (16.00), 8.498 (0.70), 8.845 (0.78).

### Example I-33

### N-[(3S)-1,1-dioxidotetrahydrothiophen-3-yl]-4-[4-(morpholin-4-yl)piperidin-1-yl]-1-[3-(trifluoromethyl)phenyl]-1H-pyrazolo[3,4-d]pyrimidin-6-amine

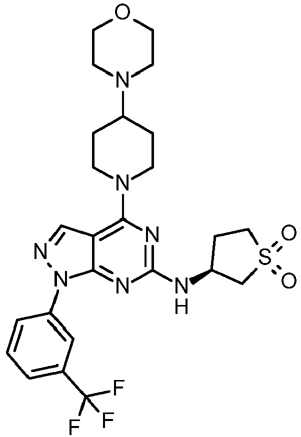

DIPEA (0.22 ml) was added to 6-chloro-4-[4-(morpholin-4-yl)piperidin-1-yl]-1-[3-(trifluoromethyl)phenyl]-1H-pyrazolo[3,4-d]pyrimidine (100 mg, 0.21 mmol) and (3S)tetrahydrothiophen-3-amine 1,1-dioxide hydrochloride (73.5 mg, 0.43 mmol) in NMP (1.5 ml) and the mixture was stirred for 42 h at 130°C. Standard workup and purification by HPLC (method HT_PREP_bas) afforded 23.4 mg of the title compound.
LC-MS (method HTpost_bas): Rₜ = 1.20 min; MS (ESIpos): m/z = 566 [M+H]⁺
¹H-NMR (400 MHz, DMSO-d6) δ [ppm]: 1.416 (1.73), 1.444 (1.83), 1.907 (2.74), 1.935 (2.40), 2.192 (0.58), 2.212 (1.43), 2.225 (0.88), 2.233 (1.61), 2.246 (1.95), 2.254 (0.85), 2.266 (1.95), 2.287 (0.85), 2.337 (0.64), 2.470 (8.21), 2.518 (8.27), 2.523 (6.17), 2.533 (2.31), 2.540 (15.03), 2.560 (0.91), 2.678 (0.61), 3.143 (2.74), 3.352 (1.31), 3.371 (1.52), 3.386 (1.70), 3.400 (1.28), 3.419 (0.70), 3.494 (2.37), 3.513 (2.62), 3.528 (2.37), 3.555 (7.97), 3.566 (10.49), 3.577 (7.67), 4.639 (2.01), 7.448 (1.06), 7.627 (2.49), 7.646 (3.44), 7.731 (2.16), 7.751 (3.44), 7.771 (1.61), 8.369 (16.00), 8.529 (0.76), 8.701 (0.52).

### Example I-34

### N-[(3S)-1,1-dioxidotetrahydrothiophen-3-yl]-4-[3-(morpholin-4-yl)azetidin-1-yl]-1-[3-(trifluoromethyl)phenyl]-1H-pyrazolo[3,4-d]pyrimidin-6-amine

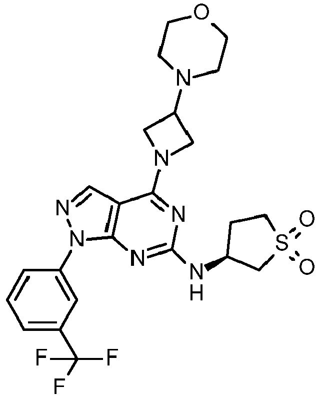

DIPEA (0,24 ml) was added to 6-chloro-4-[3-(morpholin-4-yl)azetidin-1-yl]-1-[3-(trifluoromethyl)phenyl]-1H-pyrazolo[3,4-d]pyrimidine (100 mg, 0.23 mmol) and (3S)tetra-hydrothiophen-3-amine 1,1-dioxide hydrochloride (78.2 mg, 0.46 mmol) in NMP (1.5 ml) and the mixture was stirred for 42 h at 130°C. Standard workup and purification by HPLC (method HT_PREP_acid) afforded 23.4 mg of the title compound.
LC-MS (method HTpost_acid): Rₜ = 0.94 min; MS (ESIpos): m/z = 538 [M+H]⁺
¹H-NMR (400 MHz, DMSO-d6) δ [ppm]: 2.189 (0.50), 2.209 (1.30), 2.222 (0.78), 2.230 (1.43), 2.242 (1.72), 2.250 (0.76), 2.263 (1.76), 2.284 (0.78), 2.407 (5.80), 2.449 (0.80), 2.465 (1.24), 2.518 (4.56), 2.523 (3.13), 2.540 (1.56), 3.104 (1.32), 3.124 (1.91), 3.138 (1.91), 3.157 (1.89), 3.178 (0.87), 3.355 (2.15), 3.361 (1.72), 3.373 (3.24), 3.387 (2.76), 3.403 (1.50), 3.420 (0.72), 3.496 (1.09), 3.515 (1.22), 3.529 (1.09), 3.548 (1.02), 3.616 (5.73), 3.627 (8.57), 3.636 (5.67), 4.007 (0.70), 4.200 (0.85), 4.287 (0.98), 4.438 (0.91), 4.617 (0.91), 4.634 (1.56), 4.651 (1.50), 4.668 (0.80), 7.586 (0.54), 7.619 (2.34), 7.639 (2.97), 7.728 (1.78), 7.749 (2.86), 7.768 (1.33), 8.113 (16.00), 8.529 (0.67), 8.711 (0.56).

### Example I-35

### (4S)-4-[(4-{3-[(methylsulfonyl)methyl]azetidin-1-yl}-1-[3-(trifluoromethyl)phenyl]-1H-pyrazolo[3,4-d]pyrimidin-6-yl)amino]pyrrolidin-2-one

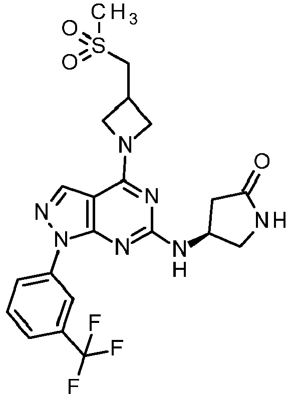

DIPEA (0.17 ml) was added to 6-chloro-4-{3-[(methanesulfonyl)methyl]azetidin-1-yl}-1-[3-(trifluoromethyl)phenyl]-1H-pyrazolo[3,4-d]pyrimidine (75.0 mg) and (4S)-4-aminopyrrolidin-2-one hydrochloride (45.0 mg) in NMP (1.5 ml) and the mixture was stirred at 130°C for 17 h. Standard workup and purification by HPLC (method HT_PREP_acid) afforded 49.9 mg of the title compound.
LC-MS (method HTpost_acid): Rₜ = 1.01 min; MS (ESIpos): m/z = 510 [M+H]⁺
¹H-NMR (400 MHz, DMSO-d6) δ [ppm]: 2.274 (0.46), 2.289 (0.50), 2.317 (0.79), 2.322 (0.84), 2.326 (1.02), 2.331 (1.01), 2.518 (2.43), 2.522 (1.54), 2.536 (0.63), 2.539 (0.66), 2.556 (0.45), 2.668 (0.51), 3.016 (16.00), 3.208 (0.53), 3.365 (0.75), 3.384 (0.57), 3.602 (0.71), 3.625 (2.99), 3.643 (2.38), 4.053 (0.44), 4.255 (0.50), 4.323 (0.40), 4.540 (0.72), 4.555 (0.72), 4.624 (0.44), 7.545 (0.45), 7.606 (1.29), 7.627 (2.04), 7.636 (2.73), 7.731 (0.60), 7.751 (0.93), 7.771 (0.46), 8.075 (10.83), 8.500 (0.40), 8.843 (0.47).

### Example I-36

### N-[(3S)-1-methylpyrrolidin-3-yl]-4-{3-[(methylsulfonyl)methyl]azetidin-1-yl}-1-[3-(trifluoromethyl)phenyl]-1H-pyrazolo[3,4-d]pyrimidin-6-amine

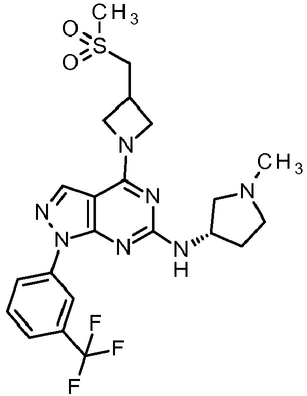

DIPEA (0.17 ml) was added to 6-chloro-4-{3-[(methanesulfonyl)methyl]azetidin-1-yl}-1-[3-(trifluoromethyl)phenyl]-1H-pyrazolo[3,4-d]pyrimidine (75 mg) and (3S)-1-methylpyrrolidin-3-amine dihydrochloride (57 mg) in NMP (1.5 ml) and the mixture was stirred at 130°C for 17 h. Standard workup and purification by HPLC (method HT_PREP_bas) afforded 12 mg of the title compound.
LC-MS (method HTpost_bas): Rₜ = 1.23 min; MS (ESIneg): m/z = 508 [M-H]⁻
¹H-NMR (400 MHz, DMSO-d6) δ [ppm]: 1.780 (0.40), 2.166 (0.44), 2.230 (16.00), 2.423 (0.92), 2.518 (4.67), 2.522 (3.05), 2.529 (1.21), 2.791 (0.55), 2.810 (0.77), 2.831 (0.48), 2.839 (0.44), 3.013 (11.95), 3.371 (0.60), 3.621 (1.76), 3.638 (1.57), 4.318 (0.68), 7.258 (0.47), 7.596 (0.98), 7.615 (1.28), 7.724 (0.53), 7.745 (0.81), 7.765 (0.40), 8.049 (7.99), 8.458 (0.40), 8.957 (0.61).

### Example I-37

### rel-(3R,4R)-4-({4-[3-(morpholin-4-yl)azetidin-1-yl]-1-[3-(trifluoromethyl)phenyl]-1H-pyrazolo[3,4-d]pyrimidin-6-yl}amino)tetrahydrothiophene-3-ol 1,1-dioxide

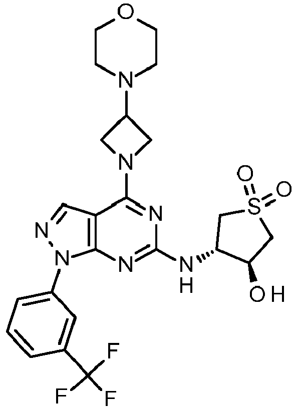

DIPEA (0.19 ml) was added to 6-chloro-4-[3-(morpholin-4-yl)azetidin-1-yl]-1-[3-(trifluoromethyl)phenyl]-1H-pyrazolo[3,4-d]pyrimidine (80.0 mg, 0.18 mmol) and rel-(3R,4R)-4-aminotetrahydrothiophene-3-ol 1,1-dioxide (55.1 mg) in NMP (1.5 ml) and the mixture was stirred at 130°C for 19.5 h. Standard workup and purification by HPLC (method HT_PREP _acid) afforded 29.6 mg of the title compound.
LC-MS (method a01): Rₜ = 0.93 min; MS (ESIpos): m/z = 554 [M+H]⁺
¹H-NMR (400 MHz, DMSO-d6) δ [ppm]: 2.407 (7.12), 2.518 (5.25), 2.523 (3.66), 2.539 (6.03), 2.692 (0.48), 3.364 (3.22), 3.377 (3.17), 3.389 (2.08), 3.406 (1.09), 3.446 (2.30), 3.464 (2.42), 3.477 (1.72), 3.495 (1.52), 3.616 (7.02), 3.627 (10.55), 3.636 (6.97), 4.016 (0.90), 4.205 (1.04), 4.295 (1.26), 4.444 (1.21), 4.670 (2.30), 5.855 (2.37), 7.068 (0.77), 7.621 (2.40), 7.640 (3.24), 7.726 (2.47), 7.746 (3.99), 7.766 (1.84), 8.123 (16.00), 8.510 (0.73), 8.708 (0.99).

### Example I-38

### (4S)-4-({4-[3-(dimethylamino)-3-methylazetidin-1-yl]-1-[3-(trifluoromethyl)phenyl]-1H-pyrazolo[3,4-d]pyrimidin-6-yl}amino)pyrrolidin-2-one

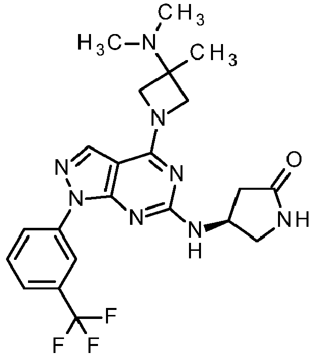

A solution of 1-{6-chloro-1-[3-(trifluoromethyl)phenyl]-1H-pyrazolo[3,4-d]pyrimidin-4-yl}-N,N,3-trimethylazetidin-3-amine (80.0 mg) and (4S)-4-aminopyrrolidin-2-one hydrochloride (53.2 mg) in NMP (1.5 ml) was treated with DIPEA (0.20 ml) and the mixture was stirred at 130°C for 16h. Standard workup and purification by HPLC (method HT_PREP_bas) afforded 55.7 mg of the title compound.
LC-MS (method HTpost_bas): Rₜ = 1.12 min; MS (ESIpos): m/z = 475 [M+H]⁺
¹H-NMR (400 MHz, DMSO-d6) δ [ppm]: 1.294 (7.32), 2.146 (16.00), 2.322 (0.58), 2.327 (0.63), 2.332 (0.55), 2.336 (0.44), 2.518 (1.61), 2.523 (1.12), 2.669 (0.40), 3.620 (0.49), 7.604 (0.68), 7.628 (1.69), 7.750 (0.51), 8.098 (4.67).

### Example I-39

### 4-[3-(dimethylamino)-3-methylazetidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine

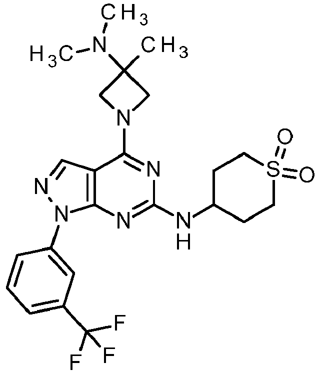

A solution of 1-{6-chloro-1-[3-(trifluoromethyl)phenyl]-1H-pyrazolo[3,4-d]pyrimidin-4-yl}-N,N,3-trimethylazetidin-3-amine (80.0 mg) and tetrahydro-2H-thiopyran-4-amine 1,1-dioxide (58.1 mg) in NMP (1.5 ml) was treated with DIPEA (0.20 ml) and the mixture was stirred at 130°C for 16h. Standard workup and purification by HPLC (method HT_PREP_bas) afforded 67.2 mg of the title compound.
LC-MS (method HTpost_bas): Rₜ = 1.22 min; MS (ESIpos): m/z = 524 [M+H]⁺
¹H-NMR (400 MHz, DMSO-d6) δ [ppm]: 1.295 (7.38), 2.075 (0.49), 2.096 (0.60), 2.115 (0.49), 2.148 (16.00), 2.165 (0.89), 2.223 (0.61), 2.518 (1.01), 2.523 (0.72), 2.539 (0.45), 3.195 (1.68), 7.614 (0.62), 7.633 (0.81), 7.768 (0.47), 8.091 (4.95).

### Example I-40

### 2-[1-(6-{[(3S,5S)-5-methylpyrrolidin-3-yl]amino}-1-[3-(trifluoromethyl)phenyl]-1H-pyrazolo[3,4-d]pyrimidin-4-yl)piperidin-4-yl]propan-2-ol

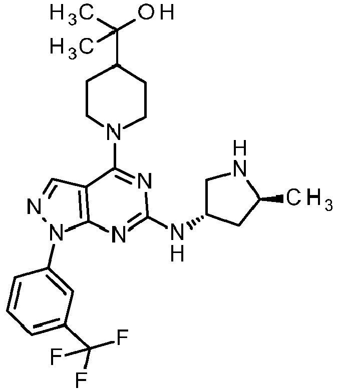

### Step a)

### tert-butyl (2S,4S)-4-({4-[4-(2-hydroxypropan-2-yl)piperidin-1-yl]-1-[3-(trifluoromethyl)phenyl]-1H-pyrazolo[3,4-d]pyrimidin-6-yl}amino)-2-methylpyrrolidine-1-carboxylate

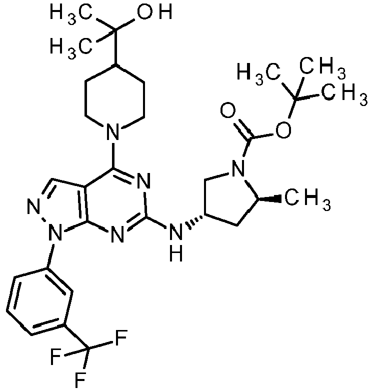

DIPEA (0.19 ml) was added to 2-(1-{6-chloro-1-[3-(trifluoromethyl)phenyl]-1H-pyrazolo[3,4-d]pyrimidin-4-yl}piperidin-4-yl)propan-2-ol (80.5 mg, 0.18 mmol) and tert-butyl (2S,4S)-4-amino-2-methylpyrrolidine-1-carboxylate (55.0 mg) in NMP (1.5 ml) and the mixture was stirred for 67.5 h at rt, 23 h at 100°C and 23 h at 130°C. Standard workup and purification by HPLC (acetonitrile / water + 0.1% formic acid) afforded tert-butyl (2S,4S)-4-({4-[4-(2-hydroxypropan-2-yl)piperidin-1-yl]-1-[3-(trifluoromethyl)phenyl]-1H-pyrazolo[3,4-d]pyrimidin-6-yl}amino)-2-methylpyrrolidine-1-carboxylate (76 mg).
LC-MS (method a01): Rₜ = 1.61 min; MS (ESIpos): m/z = 604 [M+H]⁺

### Step b)

tert-butyl (2S,4S)-4-({4-[4-(2-hydroxypropan-2-yl)piperidin-1-yl]-1-[3-(trifluoromethyl)phenyl]-1H-pyrazolo[3,4-d]pyrimidin-6-yl}amino)-2-methylpyrrolidine-1-carboxylate (76.0 mg, 0.13 mmol) in dichlormethane (2 ml) was treated with trifluoroacetic acid (29 µl) and the mixture was stirred at rt for 68 h and 4.5 h at 40°C. Water was added and the mixture was extracted three times with ethyl acetate. The organic layers were concentrated and the residue was purified by HPLC (method HT_PREP_bas) to afford 29.1 mg 2-[1-(6-{[(3S,5S)-5-methylpyrrolidin-3-yl]amino}-1-[3-(trifluoromethyl)phenyl]-1H-pyrazolo[3,4-d]pyrimidin-4-yl)piperidin-4-yl]propan-2-ol.
LC-MS (method HTpost_bas): Rₜ = 1.36 min; MS (ESIpos): m/z = 504 [M+H]⁺
¹H-NMR (400 MHz, DMSO-d6) δ [ppm]: 1.051 (16.00), 1.071 (4.61), 1.180 (0.43), 1.196 (0.51), 1.223 (0.52), 1.248 (0.51), 1.551 (0.59), 1.828 (0.72), 1.858 (0.92), 2.327 (0.49), 2.518 (1.97), 2.523 (1.31), 2.665 (0.47), 2.669 (0.63), 2.674 (0.51), 3.193 (0.47), 3.212 (0.47), 3.234 (0.76), 3.251 (0.82), 3.262 (0.79), 3.279 (0.82), 4.183 (1.55), 7.598 (0.61), 7.617 (0.83), 7.738 (0.62), 8.313 (3.24), 8.510 (0.43).

### Example I-41-1

### 3-methyl-1-(6-{[(3R)-1-(methylsulfonyl)pyrrolidin-3-yl]amino}-1-[3-(trifluoromethyl)-phenyl]-1H-pyrazolo[3,4-d]pyrimidin-4-yl)azetidin-3-ol

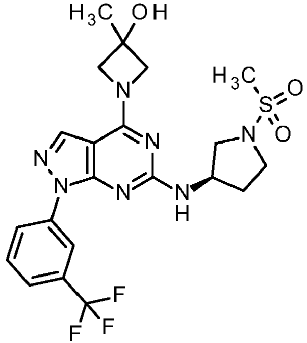

DIPEA (0.22 ml) was added to 1-{6-chloro-1-[3-(trifluoromethyl)phenyl]-1H-pyrazolo[3,4-d]pyrimidin-4-yl}-3-methylazetidin-3-ol (80.0 mg, 0.21 mmol) and (3R)-1-(methylsulfonyl)-pyrrolidin-3-amine hydrochloride (83.7 mg, 0.42 mmol) in NMP (1.5 ml) and the mixture was stirred 6 h at 130°C, 66 h at rt and 20.5 h at 130°C. Standard workup and purification by HPLC (method HT_PREP_acid) afforded 75.6 mg of the title compound.
LC-MS (method HTpost_acid): Rₜ = 1.11 min; MS (ESIpos): m/z = 512 [M+H]⁺
¹H-NMR (400 MHz, DMSO-d6) δ [ppm]: 1.479 (9.28), 2.003 (0.55), 2.019 (0.63), 2.035 (0.49), 2.074 (1.38), 2.197 (0.48), 2.214 (0.59), 2.229 (0.52), 2.518 (1.81), 2.522 (1.20), 2.888 (16.00), 2.935 (0.46), 3.274 (1.04), 3.289 (1.35), 3.294 (1.60), 3.298 (1.75), 3.308 (2.07), 3.314 (2.86), 3.319 (3.62), 3.334 (12.54), 3.428 (0.72), 3.446 (1.16), 3.452 (0.69), 3.464 (0.73), 3.470 (0.89), 3.488 (0.47), 3.550 (0.60), 3.566 (0.72), 3.575 (0.64), 3.592 (0.54), 4.030 (0.68), 4.230 (0.48), 4.307 (0.47), 4.400 (0.51), 4.414 (0.90), 4.428 (0.89), 4.443 (0.48), 5.846 (0.76), 7.610 (1.07), 7.629 (1.41), 7.726 (0.81), 7.747 (1.34), 7.766 (0.65), 8.103 (9.56), 8.160 (1.13).

### Example I-41-2

### 3-methyl-1-(6-{[(3S)-1-(methylsulfonyl)pyrrolidin-3-yl]amino}-1-[3-(trifluoromethyl)-phenyl]-1H-pyrazolo[3,4-d]pyrimidin-4-yl)azetidin-3-ol

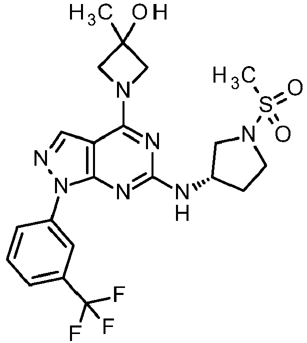

DIPEA (0.22 ml) was added to 1-{6-chloro-1-[3-(trifluoromethyl)phenyl]-1H-pyrazolo[3,4-d]pyrimidin-4-yl}-3-methylazetidin-3-ol (80.0 mg, 0.21 mmol) and (3S)-1-(methylsulfonyl)-pyrrolidin-3-amine hydrochloride (83.7 mg, 0.42 mmol) in NMP (1.5 ml) and the mixture was stirred 6 h at 130°C, 66 h at rt and 20.5 h at 130°C. Standard workup and purification by HPLC (method HT_PREP_acid) afforded 78.0 mg of the title compound.
LC-MS (method HTpost_acid): Rₜ = 1.10 min; MS (ESIpos): m/z = 512 [M+H]⁺
¹H-NMR (400 MHz, DMSO-d6) δ [ppm]: 1.479 (9.53), 2.004 (0.58), 2.019 (0.67), 2.036 (0.52), 2.074 (0.99), 2.197 (0.50), 2.214 (0.62), 2.230 (0.55), 2.246 (0.41), 2.518 (1.72), 2.522 (1.13), 2.539 (0.66), 2.889 (16.00), 2.936 (0.74), 3.274 (0.99), 3.290 (1.25), 3.294 (1.48), 3.299 (1.58), 3.309 (1.67), 3.314 (2.16), 3.319 (2.42), 3.333 (3.45), 3.378 (0.42), 3.428 (0.69), 3.447 (1.16), 3.453 (0.69), 3.464 (0.74), 3.471 (0.91), 3.488 (0.47), 3.551 (0.62), 3.566 (0.75), 3.576 (0.67), 3.592 (0.56), 4.032 (0.73), 4.230 (0.51), 4.307 (0.49), 4.400 (0.53), 4.414 (0.94), 4.428 (0.93), 4.443 (0.51), 5.758 (0.80), 5.845 (0.49), 7.608 (1.10), 7.628 (1.47), 7.725 (0.84), 7.746 (1.40), 7.766 (0.69), 8.102 (8.45), 8.146 (2.11).

### Example I-42

### 4-[3-(dimethylamino)azetidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine

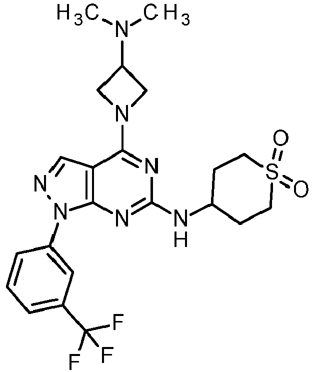

DIPEA (0.21 ml) was added to 1-{6-chloro-1-[3-(trifluoromethyl)phenyl]-1H-pyrazolo[3,4-d]pyrimidin-4-yl}-N,N-dimethylazetidin-3-amine (80.0 mg, 0.20 mol) and tetrahydro-2H-thiopyran-4-amine 1,1-dioxide hydrochloride (74.9 mg, 0.40 mmol) in NMP (1.5 ml) and the mixture was stirred for 17 h at 130°C. Standard workup and purification by HPLC (method HT_PREP_bas) afforded 19.1 mg of the title compound.
LC-MS (method HTpost_bas): Rₜ = 1.17 min; MS (ESIpos): m/z = 510 [M+H]⁺
¹H-NMR (400 MHz, DMSO-d6) δ [ppm]: 2.075 (0.48), 2.095 (0.61), 2.112 (0.48), 2.160 (16.00), 2.178 (0.88), 2.221 (0.64), 2.518 (2.48), 2.523 (1.70), 3.193 (1.67), 3.281 (0.41), 3.287 (0.44), 3.299 (0.75), 3.316 (0.63), 4.201 (0.41), 7.615 (0.63), 7.635 (0.81), 7.769 (0.47), 8.102 (4.73).

### Example I-43

### N-(3-methyl-1,1-dioxidotetrahydrothiophen-3-yl)-4-[3-(morpholin-4-yl)azetidin-1-yl]-1-[3-(trifluoromethyl)phenyl]-1H-pyrazolo[3,4-d]pyrimidin-6-amine

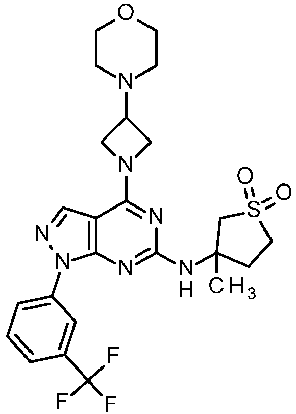

DIPEA (0.11 ml) was added to 6-chloro-4-[3-(morpholin-4-yl)azetidin-1-yl]-1-[3-(trifluoromethyl)phenyl]-1H-pyrazolo[3,4-d]pyrimidine (45.0 mg, 0.10 mmol, 1.0 eq.) and 3-methyltetrahydrothiophen-3-amine 1,1-dioxide hydrochloride (38.1 mg) in NMP (1.5 ml) and the mixture was stirred at 130°C for 26 h and at rt for 66 h. Then the mixture was heated for 1 h at 160°C in a microwave reactor. Potassium carbon ate (3 eq.) was added and the mixture was stirred for 50 h at 130°C. Standard workup and purification by HPLC (method HT_PREP _bas) afforded 2.5 mg of the title compound.
LC-MS (method b02): Rₜ = 1.23 min; MS (ESIpos): m/z = 552 [M+H]⁺
¹H-NMR (400 MHz, CHLOROFORM-d) δ [ppm]: 1.572 (4.84), 1.774 (16.00), 2.159 (0.46), 2.224 (0.61), 2.244 (0.89), 2.258 (0.75), 2.266 (0.74), 2.281 (0.97), 2.300 (0.72), 2.491 (4.36), 2.998 (0.61), 3.012 (0.74), 3.031 (0.58), 3.038 (0.55), 3.046 (0.45), 3.145 (2.72), 3.180 (2.87), 3.201 (0.66), 3.214 (0.68), 3.219 (0.75), 3.233 (1.35), 3.247 (0.94), 3.252 (0.95), 3.266 (0.72), 3.399 (0.71), 3.410 (0.46), 3.422 (1.80), 3.440 (2.10), 3.453 (1.69), 3.474 (0.57), 3.773 (4.35), 3.784 (6.56), 3.795 (4.47), 4.137 (1.38), 4.171 (1.34), 4.285 (0.83), 4.451 (0.46), 5.161 (3.38), 7.006 (0.86), 7.516 (1.50), 7.528 (1.38), 7.535 (2.23), 7.605 (1.50), 7.625 (2.38), 7.645 (1.06), 7.813 (13.16), 8.357 (1.40), 8.377 (1.32), 8.605 (2.00).

### Example I-44

### (4S)-4-({4-[3-(dimethylamino)azetidin-1-yl]-1-[3-(trifluoromethyl)phenyl]-1H-pyrazolo-[3,4-d]pyrimidin-6-yl}amino)pyrrolidin-2-one

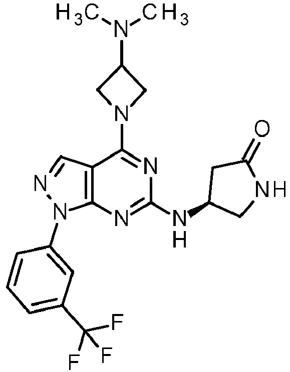

DIPEA (0.21 ml) was added to 1-{6-chloro-1-[3-(trifluoromethyl)phenyl]-1H-pyrazolo[3,4-d]-pyrimidin-4-yl}-N,N-dimethylazetidin-3-amine (80.0 mg, 0.20 mmol) and (4S)-4-aminopyrrolidin-2-one hydrochloride (55.1 mg, 0.40 mmol) in NMP (1.5 ml) and the mixture was stirred for 17 h at 130°C. Standard workup and purification by HPLC (method HT_PREP _bas) afforded 40.1 mg of the title compound.
LC-MS (method HTpost_bas): Rₜ = 1.06 min; MS (ESIpos): m/z = 461 [M+H]⁺
¹H-NMR (400 MHz, DMSO-d6) δ [ppm]: 2.157 (16.00), 2.322 (0.79), 2.326 (0.85), 2.331 (0.71), 2.518 (2.30), 2.522 (1.59), 2.530 (0.45), 2.664 (0.44), 2.668 (0.60), 2.673 (0.42), 3.283 (0.42), 3.296 (0.72), 3.314 (0.58), 3.619 (0.53), 7.605 (0.71), 7.628 (1.72), 7.750 (0.51), 8.108 (4.24).

### Example I-45

### 4-[3-(2-aminopropan-2-yl)azetidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine

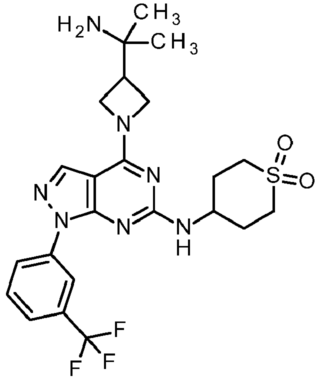

### Step a)

### tert-butyl [2-(1-{6-[(1,1-dioxidotetrahydro-2H-thiopyran-4-yl)amino]-1-[3-(trifluoromethyl)phenyl]-1H-pyrazolo[3,4-d]pyrimidin-4-yl}azetidin-3-yl)propan-2-yl]carbamate

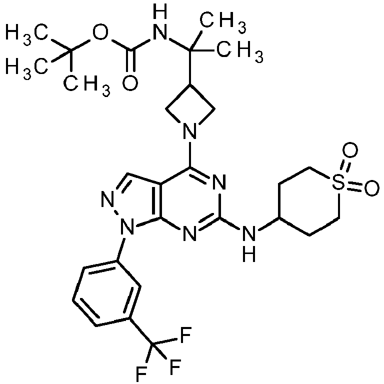

A solution of tert-butyl [2-(1-{6-chloro-1-[3-(trifluoromethyl)phenyl]-1H-pyrazolo[3,4-d]pyrimidin-4-yl}azetidin-3-yl)propan-2-yl]carbamate (200 mg, 0.39 mmol) and tetrahydro-2H-thiopyran-4-amine 1,1-dioxide (117 mg, 0.78 mmol) in NMP (4.3 ml) was stirred at 130°C for 40 h. Standard workup afforded tert-butyl [2-(1-{6-[(1,1-dioxidotetrahydro-2H-thiopyran-4-yl)amino]-1-[3-(trifluoromethyl)phenyl]-1H-pyrazolo[3,4-d]pyrimidin-4-yl}azetidin-3-yl)propan-2-yl]carbamate as a crude product which was used in the next step without further purification.

### Step b)

A solution of tert-butyl [2-(1-{6-[(1,1-dioxidotetrahydro-2H-thiopyran-4-yl)amino]-1-[3-(trifluoromethyl)phenyl]-1H-pyrazolo[3,4-d]pyrimidin-4-yl}azetidin-3-yl)propan-2-yl]carbamate (245 mg, 393 µmol) in dichlormethane (3.7 ml) was treated with trifluoroacetic acid (1.9 ml) and the mixture was stirred at rt for 16 h. The mixture was concentrated and the residue was purified by HPLC (method HT_PREP_bas) to afford 76 mg 4-[3-(2-aminopropan-2-yl)azetidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine
LC-MS (method HTpost_bas): Rₜ = 1.15 min; MS (ESIpos): m/z = 524 [M+H]⁺
¹H-NMR (400 MHz, DMSO-d6) δ [ppm]: 1.007 (16.00), 1.552 (1.38), 2.076 (1.42), 2.096 (1.66), 2.114 (1.35), 2.221 (1.83), 2.518 (4.74), 2.522 (3.01), 2.539 (0.46), 2.736 (0.98), 2.753 (1.46), 2.772 (1.03), 3.193 (5.17), 3.378 (0.41), 4.029 (1.90), 4.287 (2.07), 7.292 (0.59), 7.609 (1.83), 7.629 (2.36), 7.745 (0.89), 7.766 (1.40), 7.785 (0.74), 8.071 (12.75), 8.529 (0.55), 8.576 (0.76), 8.751 (0.59).

### Example I-46

### 4-[4-(2-aminopropan-2-yl)piperidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine

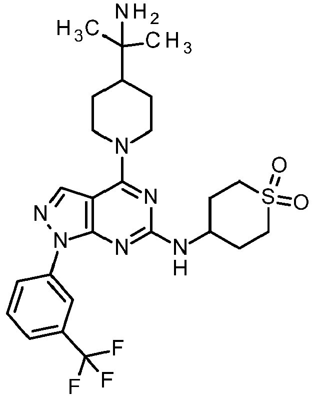

A solution of 4,6-dichloro-1-[3-(trifluoromethyl)phenyl]-1H-pyrazolo[3,4-d]pyrimidine (120 mg, 0.36 mmol) and 2-(piperidin-4-yl)propan-2-amine dihydrochloride (85.3 mg, 0.40 mmol) in NMP (2.0 ml) was treated with DIPEA (0.38 ml) and the mixture was stirred at RT for 16h. tetrahydro-2H-thiopyran-4-amine 1,1-dioxide (108 mg, 721 µmol) was added, the mixture was stirred at 130°C for 16h. Water was added to the reaction mixture and the mixture was extracted with ethyl acetate three times, the organic layers were filtered using a water-repellent filter and concentrated. Purification by HPLC (method HT_PREP_bas) afforded 56.0 mg of the title compound.
LC-MS (method HTpost_bas): Rₜ = 1.25 min; MS (ESIpos): m/z = 552 [M+H]⁺
¹H-NMR (400 MHz, DMSO-d6) δ [ppm]: 0.972 (16.00), 1.224 (0.67), 1.247 (0.72), 1.255 (0.68), 1.414 (0.62), 1.444 (0.63), 1.841 (0.88), 1.871 (0.80), 2.075 (0.56), 2.097 (0.67), 2.226 (0.65), 2.518 (2.17), 2.523 (1.39), 2.539 (0.59), 2.673 (0.43), 3.007 (0.41), 3.199 (1.37), 7.618 (0.74), 7.637 (0.93), 7.766 (0.49), 8.333 (4.47).

### Example I-47

### (4S)-4-({4-[4-(2-aminopropan-2-yl)piperidin-1-yl]-1-[3-(trifluoromethyl)phenyl]-1H-pyrazolo[3,4-d]pyrimidin-6-yl}amino)pyrrolidin-2-one

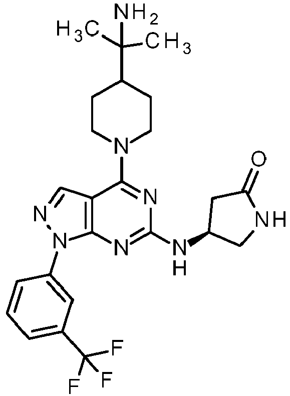

Reaction of 4,6-dichloro-1-[3-(trifluoromethyl)phenyl]-1H-pyrazolo[3,4-d]pyrimidine (120 mg, 360 nmol), 2-(piperidin-4-yl)propan-2-amine dihydrochloride (85.3 mg, 0.40 mmol), (4S)-4-aminopyrrolidin-2-one hydrochloride (98.4 mg, 0.72 mmol) and DIPEA (0.38 ml) in NMP (2.0 ml) using conditions similar to the preparation of the example before afforded 70.0 mg of the title compound.
LC-MS (method HTpost_bas): Rₜ = 1.15 min; MS (ESIpos): m/z = 503 [M+H]⁺
¹H-NMR (400 MHz, DMSO-d6) δ [ppm]: 0.968 (16.00), 1.224 (0.61), 1.247 (0.65), 1.417 (0.63), 1.447 (0.62), 1.838 (0.84), 1.869 (0.78), 2.269 (0.67), 2.284 (0.69), 2.311 (0.94), 2.326 (1.47), 2.518 (2.93), 2.522 (1.99), 2.539 (1.12), 2.664 (0.43), 2.669 (0.58), 2.673 (0.42), 3.223 (0.42), 3.596 (0.44), 3.615 (0.62), 7.371 (0.41), 7.610 (0.85), 7.636 (1.58), 7.729 (0.41), 7.749 (0.64), 8.342 (4.44).

### Example I-48

### (4S)-4-({4-[4-(1-aminocyclopropyl)piperidin-1-yl]-1-[3-(trifluoromethyl)phenyl]-1H-pyrazolo[3,4-d]pyrimidin-6-yl}amino)pyrrolidin-2-one

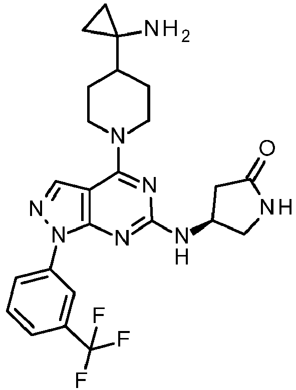

### Step a)

### tert-butyl {1-[1-(6-{[(3S)-5-oxopyrrolidin-3-yl]amino}-1-[3-(trifluoromethyl)phenyl]-1H-pyrazolo[3,4-d]pyrimidin-4-yl)piperidin-4-yl]cyclopropyl}carbamate

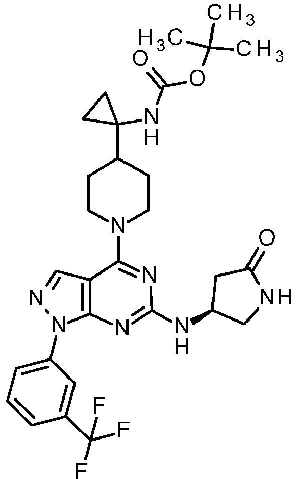

DIPEA (0.19 ml) was added to tert-butyl [1-(1-{6-chloro-1-[3-(trifluoromethyl)phenyl]-1H-pyrazolo[3,4-d]pyrimidin-4-yl}piperidin-4-yl)cyclopropyl]carbamate (100 mg, 0.19 mmol) and (4S)-4-aminopyrrolidin-2-one hydrochloride (38.2 mg, 0.28 mmol) in NMP (1.5 ml) and the mixture was stirred for 18 h at 130°C. Standard workup and purification by HPLC (acetonitrile / water + 0.1% formic acid) afforded 70.0 mg tert-butyl {1-[1-(6-{[(3S)-5-oxopyrrolidin-3-yl]amino}-1-[3-(trifluoromethyl)phenyl]-1H-pyrazolo[3,4-d]pyrimidin-4-yl)piperidin-4-yl]cyclopropyl}carbamate.
LC-MS (method a01): Rₜ = 1.36 min; MS (ESIpos): m/z = 601 [M+H]⁺

### Step b)

TFA (500 µl, 6.5 mmol) was added to tert-butyl {1-[1-(6-{[(3S)-5-oxopyrrolidin-3-yl]amino}-1-[3-(trifluoromethyl)phenyl]-1H-pyrazolo[3,4-d]pyrimidin-4-yl)piperidin-4-yl]cyclopropyl}carbamate (70.0 mg) in DCM and the mixture was stirred at rt for 17 h and concentrated. Purification by HPLC (method HT_PREP_bas) afforded 43.7 mg (4S)-4-({4-[4-(1-aminocyclopropyl)piperidin-1-yl]-1-[3-(trifluoromethyl)phenyl]-1H-pyrazolo[3,4-d]pyrimidin-6-yl}amino)pyrrolidin-2-one.
LC-MS (method HTpost_bas): Rₜ = 1.14 min; MS (ESIpos): m/z = 501 [M+H]⁺
¹H-NMR (400 MHz, DMSO-d6) δ [ppm]: 0.322 (3.94), 0.339 (13.68), 0.381 (14.28), 0.398 (3.66), 1.069 (2.42), 1.483 (4.01), 1.508 (4.24), 1.539 (2.29), 1.609 (6.60), 1.672 (5.73), 1.701 (4.26), 2.273 (3.64), 2.288 (3.89), 2.314 (5.48), 2.330 (6.58), 2.564 (4.21), 2.670 (1.60), 2.999 (2.32), 3.230 (3.19), 3.598 (2.57), 3.618 (3.56), 3.641 (2.39), 4.547 (2.27), 4.755 (1.69), 7.383 (2.24), 7.611 (4.76), 7.640 (8.47), 7.730 (2.42), 7.751 (3.59), 8.350 (16.00), 8.500 (1.42), 8.855 (1.32).

### Example I-49

### (4S)-4-({4-[3-(2-hydroxy-2-methylpropyl)azetidin-1-yl]-1-[3-(trifluoromethyl)phenyl]-1H-pyrazolo[3,4-d]pyrimidin-6-yl}amino)pyrrolidin-2-one

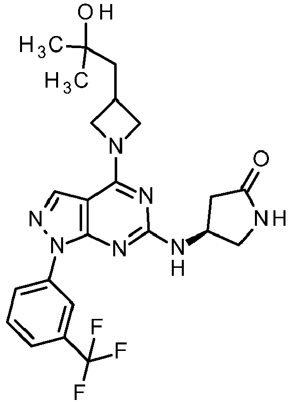

### Step a)

### methyl [1-(6-{[(3S)-5-oxopyrrolidin-3-yl]amino}-1-[3-(trifluoromethyl)phenyl]-1H-pyrazolo[3,4-d]pyrimidin-4-yl)azetidin-3-yl]acetate

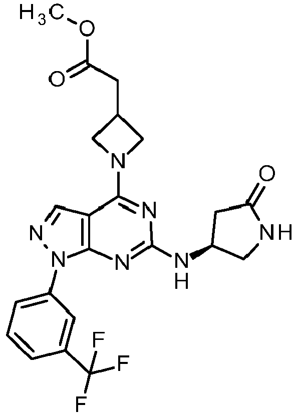

A mixture of methyl (1-{6-chloro-1-[3-(trifluoromethyl)phenyl]-1H-pyrazolo[3,4-d]pyrimidin-4-yl}azetidin-3-yl)acetate (429 mg, 1.01 mmol) and (4S)-4-aminopyrrolidin-2-one hydrochloride (275 mg, 2.02 mmol, CAS 672883-63-5) in NMP (3.0 ml) was treated with DIPEA (1.1 ml) and the mixture was stirred at 130°C for 17 h. Water was added and the mixture was extracted with ethyl acetate three times. The combined organic layers were washed with brine and filtered through a water-repellent filter and evaporated. The residue was treated with DMSO and water, the resulting solid was filtered, washed with water and 2-methoxy-2-methylpropane and dried in vacuum affording 341 mg methyl [1-(6-{[(3S)-5-oxopyrrolidin-3-yl]amino}-1-[3-(trifluoromethyl)phenyl]-1H-pyrazolo[3,4-d]pyrimidin-4-yl)azetidin-3-yl]acetate.
LC-MS (method a01): Rₜ = 1.12 min; MS (ESIpos): m/z = 490 [M+H]⁺

### Step b)

A solution of methyl [1-(6-{[(3S)-5-oxopyrrolidin-3-yl]amino}-1-[3-(trifluoromethyl)phenyl]-1H-pyrazolo[3,4-d]pyrimidin-4-yl)azetidin-3-yl]acetate (130 mg, 0.24 mmol) in 2-methyltetrahydrofuran (3.0 ml) was cooled with an ice bath. Then methylmagnesium bromide solution (3.4M in 2-methyltetrahydrofuran, 700 µl, 2.4 mmol) was added dropwise. The mixture was stirred for 30 min with cooling by the ice bath and then at rt for 19 h. Sat. aq. ammonium chloride solution was added, the mixture was extracted with ethyl acetate three times and the combined organic layers were concentrated. Purification by HPLC afforded 30.4 mg (4S)-4-({4-[3-(2-hydroxy-2-methylpropyl)azetidin-1-yl]-1-[3-(trifluoromethyl)phenyl]-1H-pyrazolo[3,4-d]pyrimidin-6-yl}amino)pyrrolidin-2-one.
LC-MS (method HTpost_acid): Rₜ = 1.03 min; MS (ESIpos): m/z = 490 [M+H]⁺
¹H-NMR (400 MHz, DMSO-d6) δ [ppm]: 1.102 (16.00), 1.762 (2.01), 1.779 (2.05), 2.116 (0.79), 2.318 (0.67), 2.322 (0.88), 2.327 (1.16), 2.331 (1.00), 2.522 (2.58), 2.548 (0.46), 2.664 (0.54), 2.669 (0.74), 2.673 (0.55), 3.045 (0.51), 3.063 (0.41), 3.202 (0.42), 3.596 (0.43), 3.615 (0.64), 4.259 (4.07), 4.535 (0.84), 4.549 (0.85), 7.601 (0.92), 7.623 (2.48), 7.726 (0.46), 7.747 (0.73), 8.050 (5.39).

### Example I-50

### [1-(6-{[(3S)-5-oxopyrrolidin-3-yl]amino}-1-[3-(trifluoromethyl)phenyl]-1H-pyrazolo[3,4-d]pyrimidin-4-yl)azetidin-3-yl]acetic acid

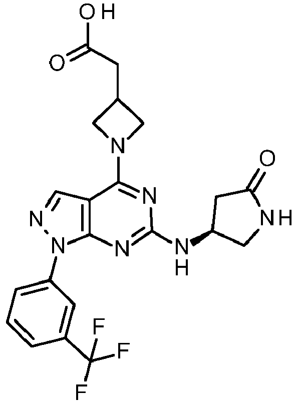

Aq. sodium hydroxide solution (1.4 ml, 2.0 M) was added to methyl [1-(6-{[(3S)-5-oxopyrrolidin-3-yl]amino}-1-[3-(trifluoromethyl)phenyl]-1H-pyrazolo[3,4-d]pyrimidin-4-yl)azetidin-3-yl]acetate (210 mg) in 2-methyltetrahydrofuran (2.0 ml) and the mixture was stirred 2.5 h at rt. The mixture was acidified by aqueous citric acid solution to pH 4 and the resulting solid was filtered, washed with water and three times with 2-methoxy-2-methylpropane and dried affording 179 mg of the title compound (crude product). 40 mg of the resulting batch was purified by HPLC (method HT_PREP_acid) affording 27 mg of the title compound.
LC-MS (method a01): Rₜ = 0.97 min; MS (ESIpos): m/z = 476 [M+H]⁺
¹H-NMR (400 MHz, DMSO-d6) δ [ppm]: 2.275 (0.79), 2.291 (0.84), 2.318 (1.44), 2.322 (1.47), 2.326 (1.71), 2.331 (1.82), 2.518 (4.52), 2.522 (3.10), 2.539 (10.69), 2.549 (0.89), 2.664 (0.85), 2.669 (1.15), 2.673 (0.99), 2.691 (4.74), 2.711 (5.09), 3.062 (0.55), 3.077 (0.99), 3.097 (1.32), 3.116 (1.04), 3.131 (0.69), 3.210 (1.44), 3.597 (1.14), 3.616 (1.56), 3.639 (1.00), 3.849 (0.77), 4.090 (0.84), 4.259 (0.70), 4.519 (1.31), 4.535 (1.99), 4.551 (1.76), 7.488 (0.69), 7.599 (2.04), 7.626 (4.20), 7.726 (0.97), 7.745 (1.54), 7.764 (0.79), 8.063 (16.00), 8.500 (0.62), 8.844 (0.69).

### Example I-51

### (4S)-4-[(4-{3-[2-(morpholin-4-yl)-2-oxoethyl]azetidin-1-yl}-1-[3-(trifluoromethyl)phenyl]-1H-pyrazolo[3,4-d]pyrimidin-6-yl)amino]pyrrolidin-2-one

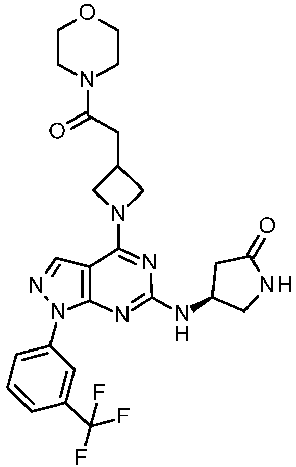

DIPEA (0.11 ml) was added to [1-(6-{[(3S)-5-oxopyrrolidin-3-yl]amino}-1-[3-(trifluoromethyl)phenyl]-1H-pyrazolo[3,4-d]pyrimidin-4-yl)azetidin-3-yl]acetic acid (70.0 mg, crude product), morpholine (17 µl, 0.19 mmol) and HATU (CAS 148893-10-1, 73.9 mg, 0.19 mmol) in NMP (1.0 ml) and the mixture was stirred 19 h at rt. The reaction mixture was filtered and the filtrate was diluted with DMSO and purified by HPLC (method HT_PREP _bas) affording 42 mg of the title compound
LC-MS (method HTpost_bas): Rₜ = 1.03 min; MS (ESIpos): m/z = 545 [M+H]⁺
¹H-NMR (400 MHz, DMSO-d6) δ [ppm]: 0.936 (2.64), 0.953 (2.52), 2.276 (0.88), 2.292 (0.97), 2.318 (1.78), 2.322 (2.01), 2.327 (2.24), 2.331 (2.29), 2.518 (8.09), 2.522 (5.27), 2.539 (2.06), 2.548 (1.09), 2.664 (1.11), 2.669 (1.48), 2.673 (1.13), 2.692 (0.60), 2.814 (3.65), 2.834 (4.00), 3.089 (0.97), 3.106 (1.25), 3.124 (0.97), 3.202 (1.09), 3.428 (5.43), 3.438 (9.41), 3.450 (8.00), 3.533 (3.93), 3.545 (4.76), 3.557 (3.40), 3.566 (4.32), 3.578 (4.79), 3.589 (3.21), 3.617 (1.64), 3.638 (0.97), 3.821 (0.99), 4.039 (1.09), 4.268 (0.95), 4.534 (2.29), 4.550 (2.47), 4.567 (1.43), 7.483 (0.79), 7.600 (2.29), 7.627 (4.88), 7.726 (1.09), 7.746 (1.66), 7.764 (0.83), 8.062 (16.00), 8.493 (0.69), 8.847 (0.76).

### Example I-52

### (4S)-4-[(4-{3-[2-(6-oxa-3-azabicyclo[3.1.1]heptan-3-yl)-2-oxoethyl]azetidin-1-yl}-1-[3-(trifluoromethyl)phenyl]-1H-pyrazolo[3,4-d]pyrimidin-6-yl)amino]pyrrolidin-2-one

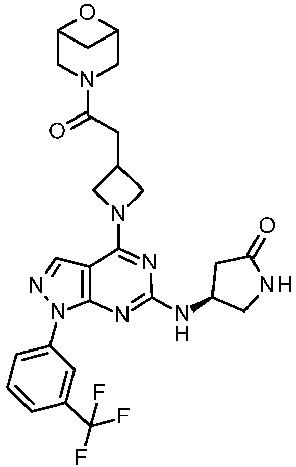

DIPEA (0.11 ml) was added to [1-(6-{[(3S)-5-oxopyrrolidin-3-yl]amino}-1-[3-(trifluoromethyl)-phenyl]-1H-pyrazolo[3,4-d]pyrimidin-4-yl)azetidin-3-yl]acetic acid (70.0 mg, batch contained impurities), 6-oxa-3-azabicyclo[3.1.1]heptane 4-methylbenzenesulfonate (52.7 mg, 0.19 mmol)] and HATU (CAS 148893-10-1 , 73.9 mg, 0.19 mmol) in NMP (1.0 ml) and the mixture was stirred at rt for 16 h. The mixture was filtered and DMSO (1 ml) was added. Purification by HPLC (method HT_PREP_bas) afforded 46 mg of the title compound.
LC-MS (method HTpost_bas): Rₜ = 1.00 min; MS (ESIpos): m/z = 557 [M+H]⁺
¹H-NMR (400 MHz, DMSO-d6) δ [ppm]: 0.936 (1.54), 0.953 (1.50), 1.746 (3.79), 1.768 (3.82), 2.279 (0.99), 2.294 (1.02), 2.322 (2.87), 2.326 (2.90), 2.332 (2.46), 2.336 (2.12), 2.518 (8.60), 2.523 (5.66), 2.539 (2.80), 2.551 (0.99), 2.660 (0.68), 2.664 (1.50), 2.669 (2.12), 2.673 (1.54), 2.678 (0.68), 2.692 (0.61), 2.831 (1.23), 2.848 (1.19), 2.886 (1.23), 3.038 (0.89), 3.055 (2.08), 3.076 (2.05), 3.093 (0.92), 3.142 (1.09), 3.162 (1.54), 3.181 (1.60), 3.197 (1.54), 3.405 (2.15), 3.438 (3.00), 3.608 (4.54), 3.641 (4.09), 3.694 (1.98), 3.734 (2.18), 3.764 (0.96), 3.859 (1.13), 4.085 (1.19), 4.297 (1.06), 4.521 (1.06), 4.539 (1.94), 4.554 (2.39), 4.573 (2.35), 4.589 (6.41), 4.605 (5.77), 7.494 (0.78), 7.601 (2.56), 7.628 (5.70), 7.728 (1.19), 7.747 (1.88), 7.767 (0.92), 8.073 (16.00), 8.501 (0.75), 8.851 (0.82).

### Example I-53

### N-cyclopropyl-4-[3-(1,1-dioxidothiomorpholin-4-yl)azetidin-1-yl]-1-[3-(trifluoromethyl)-phenyl]-1H-pyrazolo[3,4-d]pyrimidin-6-amine

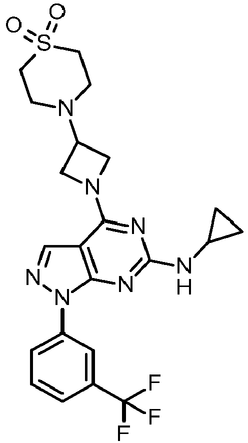

A solution of 6-chloro-4-[3-(1,1-dioxidothiomorpholin-4-yl)azetidin-1-yl]-1-[3-(trifluoromethyl)-phenyl]-1H-pyrazolo[3,4-d]pyrimidine (70.0 mg, 0.14 mmol) and cyclopropanamine (50 µl, 720 µmol) in NMP (1.6 ml) was treated with DIPEA (0.10 ml) and the mixture was stirred in a microwave at 130°C for 5 h. Standard workup and purification by HPLC (method HT_PREP _bas) afforded 50.0 mg of the title compound.
LC-MS (method HTpost_bas): Rₜ = 1.26 min; MS (ESIpos): m/z = 508 [M+H]⁺
¹H-NMR (400 MHz, DMSO-d6) δ [ppm]: 0.495 (1.39), 0.507 (4.59), 0.516 (5.23), 0.522 (5.40), 0.532 (1.81), 0.681 (3.69), 0.694 (3.84), 0.972 (0.42), 2.331 (1.05), 2.518 (7.57), 2.522 (4.67), 2.539 (2.86), 2.673 (1.22), 2.722 (1.15), 2.881 (6.89), 3.166 (7.67), 3.621 (0.44), 3.639 (1.27), 3.651 (2.00), 3.664 (1.34), 3.681 (0.56), 3.962 (0.76), 4.256 (1.10), 4.453 (0.85), 7.391 (0.98), 7.589 (2.61), 7.608 (3.42), 7.717 (2.08), 7.737 (3.47), 7.757 (1.69), 8.077 (16.00), 8.536 (1.47), 8.554 (1.42), 9.133 (0.51).

### Example I-54

### N-(3,3-difluorocyclobutyl)-4-[3-(dimethylamino)azetidin-1-yl]-1-[3-(trifluoromethyl)-phenyl]-1H-pyrazolo[3,4-d]pyrimidin-6-amine

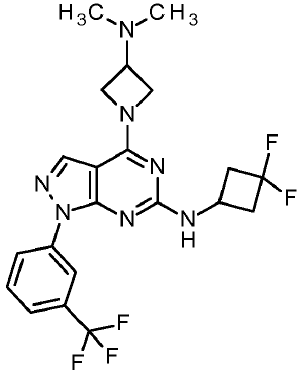

DIPEA (0.21 ml) was added to 1-{6-chloro-1-[3-(trifluoromethyl)phenyl]-1H-pyrazolo[3,4-d]-pyrimidin-4-yl}-N,N-dimethylazetidin-3-amine (80.0 mg, 0.20 mmol) and 3,3-difluorocyclo-butanamine hydrochloride (57.9 mg, 0.40 mmol) in NMP (1.5 ml) and the mixture was stirred at 130°C for 24 h. DMSO was added and the mixture was purified by HPLC (acetonitrile/ water + ammonia) affording 21.7 mg of the title compound.
¹H-NMR (400 MHz, DMSO-d6) δ [ppm]: 2.159 (16.00), 2.327 (0.44), 2.523 (1.49), 2.540 (0.86), 2.646 (0.42), 2.665 (0.81), 2.669 (0.84), 2.673 (0.74), 2.691 (0.53), 2.709 (0.42), 2.938 (0.59), 2.958 (0.60), 2.972 (0.56), 3.285 (0.47), 3.297 (0.77), 3.315 (0.74), 4.193 (0.76), 5.759 (1.31), 7.609 (0.84), 7.629 (1.09), 7.732 (0.65), 7.752 (0.92), 7.773 (0.44), 8.114 (3.95).

### Example I-55

### N-[cis-3-(methylsulfonyl)cyclobutyl]-4-[3-(morpholin-4-yl)azetidin-1-yl]-1-[3-(trifluoromethyl)phenyl]-1H-pyrazolo[3,4-d]pyrimidin-6-amine

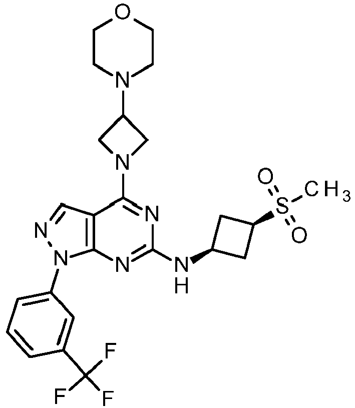

DIPEA (0.19 ml) was added to 6-chloro-4-[3-(morpholin-4-yl)azetidin-1-yl]-1-[3-(trifluoromethyl)phenyl]-1H-pyrazolo[3,4-d]pyrimidine (80.0 mg, 0.18 mmol) and cis-3-(methylsulfonyl)cyclobutanamine hydrochloride (67.7 mg, 365 µmol, CAS 1408074-56-5) in NMP (1.5 ml) and the mixture was stirred for 19.5 h at 130°C. Standard workup and purification by HPLC (method HT_PREP_acid) afforded 27.9 mg of the title compound.
LC-MS (method a01): Rₜ = 0.99 min; MS (ESIpos): m/z = 552 [M+H]⁺
¹H-NMR (400 MHz, DMSO-d6) δ [ppm]: 2.292 (0.49), 2.315 (1.19), 2.322 (1.39), 2.327 (1.02), 2.332 (0.83), 2.338 (1.16), 2.345 (1.36), 2.368 (0.83), 2.404 (2.30), 2.518 (2.11), 2.523 (1.50), 2.540 (2.21), 2.610 (0.73), 2.665 (0.54), 2.669 (0.67), 2.673 (0.48), 2.889 (16.00), 3.362 (0.83), 3.374 (0.52), 3.379 (0.51), 3.615 (2.29), 3.625 (3.44), 3.635 (2.28), 3.714 (0.57), 3.734 (0.87), 3.754 (0.52), 4.279 (0.55), 7.610 (0.83), 7.629 (1.09), 7.732 (0.43), 7.752 (0.61), 8.084 (5.78).

### Example I-56

### N-[trans-3-(methylsulfonyl)cyclobutyl]-4-[3-(morpholin-4-yl)azetidin-1-yl]-1-[3-(trifluoromethyl)phenyl]-1H-pyrazolo[3,4-d]pyrimidin-6-amine

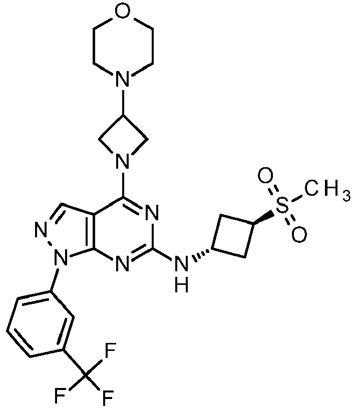

DIPEA (0.19 ml) was added to 6-chloro-4-[3-(morpholin-4-yl)azetidin-1-yl]-1-[3-(trifluoromethyl)phenyl]-1H-pyrazolo[3,4-d]pyrimidine (80.0 mg, 0.18 mmol) and trans-3-(methylsulfonyl)cyclobutanamine hydrochloride (67.7 mg, 365 µmol, CAS 1408075-97-7) in NMP (1.5 ml) and the mixture was stirred for 19.5 h at 130°C Standard workup and purification by HPLC afforded 26.1 mg of the title compound.
LC-MS (method a01): Rₜ = 0.97 min; MS (ESIpos): m/z = 552 [M+H]⁺
¹H-NMR (400 MHz, DMSO-d6) δ [ppm]: 2.327 (0.41), 2.408 (1.43), 2.518 (1.70), 2.523 (1.22), 2.539 (16.00), 2.669 (0.51), 2.673 (0.44), 2.692 (1.02), 2.710 (0.49), 2.926 (5.02), 3.367 (0.54), 3.615 (1.42), 3.626 (2.17), 3.636 (1.43), 7.607 (0.68), 7.626 (0.96), 7.709 (0.64), 7.729 (1.00), 7.749 (0.46), 8.085 (3.82).

### Example I-57

### 2-(1-{6-[(trans-3-amino-3-methylcyclobutyl)amino]-1-[3-(trifluoromethyl)phenyl]-1H-pyrazolo[3,4-d]pyrimidin-4-yl}piperidin-4-yl)propan-2-ol

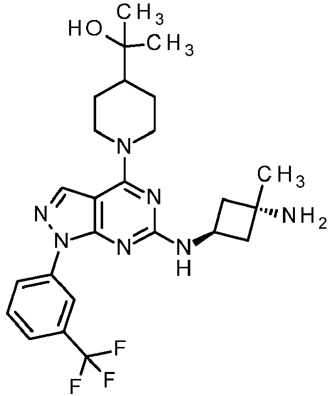

DIPEA (0.19 ml) was added to 2-(1-{6-chloro-1-[3-(trifluoromethyl)phenyl]-1H-pyrazolo[3,4-d]pyrimidin-4-yl}piperidin-4-yl)propan-2-ol (80.0 mg, 0.18 mmol) and tert-butyl (trans-3-amino-1-methylcyclobutyl)carbamate (72.9 mg, 0.36 mmol) in NMP (1.7 ml) and the mixture was stirred at 130°C for 16 h. After standard workup an d evaporation of solvents the residue was dissolved in DCM (1.8 ml) and trifluoroacetic acid (0.91 ml) was added and the mixture was stirred at rt for 16 h. Evaporation of solvents and purification by HPLC (method HT_PREP _bas) afforded 23.0 mg of the title compound.
LC-MS (method HTpost_bas): Rₜ = 1.28 min; MS (ESIpos): m/z = 504 [M+H]⁺
¹H-NMR (400 MHz, DMSO-d6) δ [ppm]: 1.051 (16.00), 1.218 (10.78), 1.247 (0.57), 1.553 (0.42), 1.826 (1.07), 1.857 (0.83), 1.923 (0.53), 1.947 (0.77), 1.970 (0.61), 2.198 (0.57), 2.518 (1.80), 2.523 (1.24), 2.540 (1.19), 4.181 (4.38), 7.597 (0.70), 7.616 (0.93), 7.714 (0.48), 7.734 (0.78), 8.303 (4.40).

### Example I-58

### cis-1-methyl-N³-{4-(morpholin-4-yl)-1-[3-(trifluoromethyl)phenyl]-1H-pyrazolo[3,4-d]pyrimidin-6-yl}cyclobutane-1,3-diamine

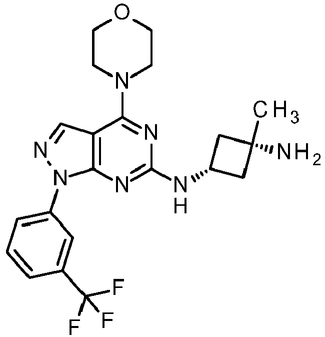

DIPEA (0.22 ml) was added to 6-chloro-4-(morpholin-4-yl)-1-[3-(trifluoromethyl)phenyl]-1H-pyrazolo[3,4-d]pyrimidine (80.0 mg, 0.21 mmol) and tert-butyl (cis-3-amino-1-methylcyclobutyl)carbamate (83.5 mg, 0.42 mmol) in NMP (2.0 ml) and the mixture was stirred at 130°C for 16 h. After standard workup and evaporation of solvents, DCM (2.0 ml) and trifluoroacetic acid (1.0 ml) were added and the mixture was stirred at rt for 16 h. Evaporation of solvents and purification by HPLC (method HT_PREP_bas) afforded 76.7 mg of the title compound.
LC-MS (method HTpost_bas): Rₜ = 1.24 min; MS (ESIpos): m/z = 448 [M+H]⁺
¹H-NMR (400 MHz, DMSO-d6) δ [ppm]: 1.205 (15.10), 1.824 (3.03), 1.845 (4.42), 1.851 (4.41), 1.873 (2.68), 2.074 (1.19), 2.323 (2.34), 2.327 (2.78), 2.331 (2.51), 2.336 (2.22), 2.347 (2.39), 2.518 (3.73), 2.523 (2.56), 2.540 (0.75), 2.665 (0.75), 2.669 (1.00), 2.673 (0.71), 3.728 (8.54), 3.739 (7.24), 3.832 (7.44), 3.844 (9.03), 3.996 (0.69), 4.015 (1.27), 4.034 (1.25), 4.053 (0.71), 7.204 (1.31), 7.604 (2.61), 7.624 (3.51), 7.716 (2.14), 7.736 (3.44), 7.756 (1.58), 8.318 (16.00), 8.356 (1.08), 8.373 (1.05), 8.538 (0.53), 9.173 (1.73).

### Example I-59

### 4-[3-methyl-3-(morpholin-4-yl)azetidin-1-yl]-N-[trans-3-(methylsulfonyl)cyclobutyl]-1-[3-(trifluoromethyl)phenyl]-1H-pyrazolo[3,4-d]pyrimidin-6-amine

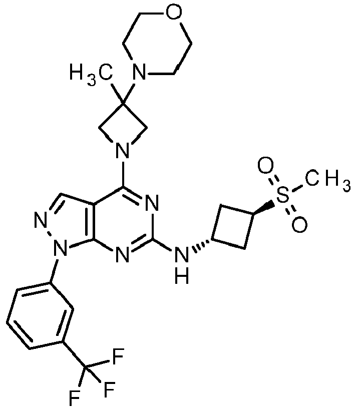

DIPEA (0.18 ml) was added to 6-chloro-4-[3-methyl-3-(morpholin-4-yl)azetidin-1-yl]-1-[3-(trifluoromethyl)phenyl]-1H-pyrazolo[3,4-d]pyrimidine (80.0 mg, 0.18 m mol) and trans-3-(methylsulfonyl)cyclobutanamine hydrochloride in NMP (1.5 ml) and the mixture was stirred 19 h at 120°C. Standard workup and purification by HPLC (method HT_PREP_acid) afforded 64.0 mg of the title compound.
LC-MS (method HTpost_acid): Rₜ = 0.92 min; MS (ESIpos): m/z = 566 [M+H]⁺
¹H-NMR (400 MHz, DMSO-d6) δ [ppm]: 1.332 (16.00), 2.322 (0.82), 2.326 (1.12), 2.332 (0.81), 2.455 (6.34), 2.464 (5.60), 2.518 (3.99), 2.522 (2.59), 2.539 (1.00), 2.664 (1.02), 2.668 (1.37), 2.673 (1.19), 2.678 (0.93), 2.715 (1.23), 2.927 (13.79), 3.616 (5.60), 3.808 (0.74), 3.844 (0.70), 3.855 (0.91), 3.867 (1.07), 3.879 (0.82), 3.891 (0.56), 3.957 (0.89), 3.977 (0.75), 4.050 (0.84), 4.069 (0.95), 4.233 (0.95), 4.253 (0.77), 4.491 (0.63), 7.607 (2.03), 7.627 (2.59), 7.710 (1.82), 7.730 (2.85), 7.749 (1.33), 8.073 (12.17), 8.526 (0.54), 8.749 (0.60).

### Example I-60

### N-(1,1-dioxidotetrahydro-2H-thiopyran-4-yl)-4-[3-(morpholin-4-ylmethyl)azetidin-1-yl]-1-[3-(trifluoromethyl)phenyl]-1H-pyrazolo[3,4-d]pyrimidin-6-amine

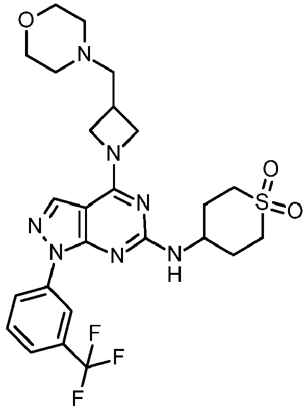

DIPEA (0.17 ml) was added to 6-chloro-4-{3-[(morpholin-4-yl)methyl]azetidin-1-yl}-1-[3-(trifluoromethyl)phenyl]-1H-pyrazolo[3,4-d]pyrimidine and tetrahydro-2H-thiopyran-4-amine 1,1-dioxide in NMP (2.0 ml) and the mixture was stirred for 42 h at 120°C. Standard workup and purification by HPLC (method HT_PREP_bas) afforded 49.0 mg of the title compound.
LC-MS (method HTpost_bas): Rₜ = 1.17 min; MS (ESIpos): m/z = 566 [M+H]⁺
¹H-NMR (400 MHz, DMSO-d6) δ [ppm]: 2.074 (1.72), 2.091 (1.99), 2.109 (1.53), 2.218 (2.07), 2.322 (1.04), 2.326 (1.39), 2.332 (1.04), 2.336 (0.66), 2.390 (6.04), 2.518 (3.46), 2.522 (2.48), 2.539 (2.52), 2.621 (1.88), 2.660 (0.93), 2.664 (1.32), 2.668 (1.57), 2.673 (1.14), 3.037 (1.01), 3.056 (1.28), 3.074 (0.97), 3.193 (5.32), 3.566 (7.41), 3.578 (11.90), 3.589 (7.31), 3.805 (0.91), 4.057 (1.18), 4.229 (0.91), 4.490 (0.99), 7.329 (0.66), 7.610 (2.19), 7.629 (2.77), 7.744 (1.03), 7.765 (1.57), 7.785 (0.85), 8.088 (16.00), 8.406 (0.62), 8.416 (0.50), 8.578 (0.97), 8.746 (0.72).

### Example I-61

### 4-methyl-1-[1-(6-{[trans-3-(methylsulfonyl)cyclobutyl]amino}-1-[3-(trifluoromethyl)-phenyl]-1H-pyrazolo[3,4-d]pyrimidin-4-yl)azetidin-3-yl]piperidin-4-ol

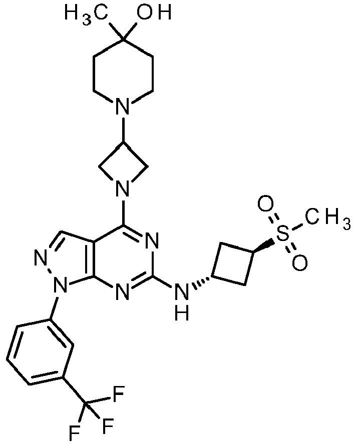

A solution of 1-(1-{6-chloro-1-[3-(trifluoromethyl)phenyl]-1H-pyrazolo[3,4-d]pyrimidin-4-yl}azetidin-3-yl)-4-methylpiperidin-4-ol (80.0 mg, 0.17 mol) and trans-3-(methylsulfonyl)-cyclobutanamine hydrochloride (41.4 mg, 0.22 mmol) in NMP (1.6 ml) was treated with DIPEA (0.18 ml) and the mixture was stirred at 130°C for 16 h. Standard workup and purification by HPLC (method HT_PREP_bas) afforded 67.0 mg of the title compound.
LC-MS (method HTpost_bas): Rₜ = 1.08 min; MS (ESIpos): m/z = 580 [M+H]⁺
¹H-NMR (400 MHz, DMSO-d6) δ [ppm]: 1.113 (16.00), 1.448 (0.48), 1.470 (1.94), 1.495 (5.02), 1.527 (0.63), 2.304 (1.47), 2.323 (1.59), 2.327 (1.73), 2.332 (1.41), 2.518 (3.89), 2.523 (2.60), 2.665 (0.87), 2.669 (1.17), 2.674 (1.11), 2.679 (0.99), 2.713 (1.38), 2.926 (11.54), 3.844 (0.60), 3.855 (0.90), 3.867 (1.11), 3.879 (0.93), 3.891 (0.78), 3.902 (0.66), 4.167 (5.32), 4.423 (0.78), 4.471 (0.75), 7.603 (2.09), 7.623 (2.63), 7.706 (1.73), 7.727 (2.72), 7.746 (1.26), 8.089 (8.46), 8.523 (0.57), 8.750 (0.63).

### Example I-62

### 4-[4-(1-aminocyclopropyl)piperidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine

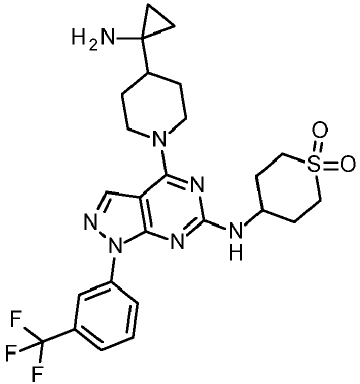

### Step a)

### tert-butyl [1-(1-{6-[(1,1-dioxidotetrahydro-2H-thiopyran-4-yl)amino]-1-[3-(trifluoromethyl)phenyl]-1H-pyrazolo[3,4-d]pyrimidin-4-yl}piperidin-4-yl)cyclopropyl]carbamate

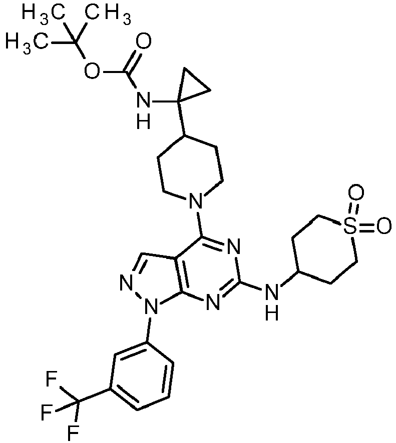

A solution of tert-butyl [1-(1-{6-chloro-1-[3-(trifluoromethyl)phenyl]-1H-pyrazolo[3,4-d]pyrimidin-4-yl}piperidin-4-yl)cyclopropyl]carbamate (100 mg, 0.19 mmol) and tetrahydro-2H-thiopyran-4-amine 1,1-dioxide (55.6 mg, 0.37 mmol) in NMP (2.0 ml) was treated with DIPEA (0.19 ml) and the mixture was stirred at 130°C for 16 h. Standard workup afforded a crude product, which was used without further purification.

### Step b)

A solution of tert-butyl [1-(1-{6-[(1,1-dioxidotetrahydro-2H-thiopyran-4-yl)amino]-1-[3-(trifluoromethyl)phenyl]-1H-pyrazolo[3,4-d]pyrimidin-4-yl}piperidin-4-yl)cyclopropyl]carbamate (120 mg) in dichlormethane (2.0 ml) was treated with TFA (1.0 ml) and the mixture was stirred at rt for 72 h and concentrated. The crude product was purified by HPLC (method HT_PREP _bas) affording 55.0 mg 4-[4-(1-aminocyclopropyl)piperidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine.
LC-MS (method HTpost_bas): Rₜ = 1.23 min; MS (ESIpos): m/z = 550 [M+H]⁺
¹H-NMR (400 MHz, DMSO-d6) δ [ppm]: 0.325 (2.46), 0.342 (9.44), 0.349 (6.96), 0.378 (6.78), 0.384 (9.96), 0.402 (2.36), 1.038 (0.75), 1.068 (1.57), 1.097 (0.94), 1.443 (0.80), 1.453 (0.94), 1.475 (2.31), 1.484 (2.41), 1.506 (2.56), 1.516 (2.43), 1.538 (1.17), 1.548 (1.02), 1.642 (2.31), 1.673 (3.98), 1.703 (2.71), 2.078 (2.01), 2.100 (2.39), 2.114 (1.79), 2.136 (0.99), 2.231 (2.34), 2.318 (0.57), 2.322 (1.14), 2.326 (1.49), 2.331 (1.09), 2.336 (0.52), 2.518 (5.66), 2.522 (3.65), 2.539 (0.89), 2.659 (0.50), 2.664 (1.07), 2.669 (1.44), 2.673 (1.07), 2.678 (0.47), 2.994 (1.24), 3.140 (0.80), 3.202 (4.77), 4.104 (0.99), 4.766 (0.77), 7.221 (0.92), 7.620 (2.51), 7.639 (3.18), 7.749 (1.14), 7.768 (1.69), 8.342 (16.00), 8.573 (0.99), 8.756 (0.65).

### Example I-63

### trans-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-(trifluoromethyl)cyclobutanol

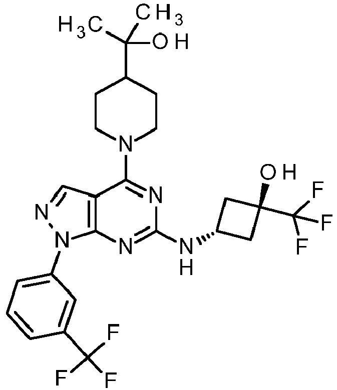

A solution of 2-(1-{6-chloro-1-[3-(trifluoromethyl)phenyl]-1H-pyrazolo[3,4-d]pyrimidin-4-yl}-piperidin-4-yl)propan-2-ol (80.0 mg, 0.18 mmol) and trans-3-amino-1-(trifluoromethyl)cyclobutanol hydrochloride (CAS 1408075-16-0) in NMP (2.0 ml) was treated with DIPEA (0.19 ml) and the mixture was stirred at 130°C for 16h. Standard workup and purification by HPLC afforded 68 mg of the title compound.
¹H-NMR (400 MHz, DMSO-d6) δ [ppm]: 1.052 (16.00), 1.229 (0.54), 1.256 (0.57), 1.563 (0.45), 1.836 (0.79), 1.865 (0.72), 2.323 (0.76), 2.327 (0.86), 2.332 (0.72), 2.349 (1.15), 2.370 (1.30), 2.518 (2.71), 2.523 (1.82), 2.665 (0.42), 2.669 (0.61), 2.673 (0.44), 4.187 (4.56), 6.412 (0.81), 7.432 (0.40), 7.615 (0.71), 7.634 (0.89), 7.725 (0.45), 7.744 (0.71), 8.332 (4.68).

### Example I-64-1

### 2-(1-{6-[(3R)-1-azabicyclo[2.2.2]octan-3-ylamino]-1-[3-(trifluoromethyl)phenyl]-1H-pyrazolo[3,4-d]pyrimidin-4-yl}piperidin-4-yl)propan-2-ol

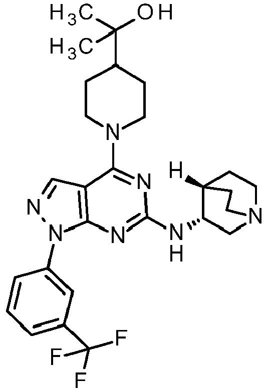

DIPEA (0.19 ml) was added to 2-(1-{6-chloro-1-[3-(trifluoromethyl)phenyl]-1H-pyrazolo[3,4-d]pyrimidin-4-yl}piperidin-4-yl)propan-2-ol (80 mg) and (3R)-quinuclidin-3-amine dihydrochloride (54.3 mg, CAS 123536-14-1) in NMP (1.5 ml) and the mixture was stirred at rt for 20 h. Standard workup and purification by HPLC (Instrument: Waters Autopurificationsystem; Column: XBrigde C18 5µ, 100x30mm; eluent A: water + 0.2 vol % aq. ammonia (32%); eluent B: acetonitril; gradient: 0.0-0.5 min 50% B (35-70 ml/min), 0.5-5.5 min 50-85% B; flow: 70 ml/min; temperature: 25°C; DAD scan: 210-400 nm) afforded a crude product which was then purified again by HPLC (Instrument: Sepiatec: Prep SFC100; Column: Chiralpak IC 5µ 250x30mm; eluent A: CO2; eluent B: methanol + 0.2 vol % aq. ammonia (32%); isocratic: 25%B; flow: 100 ml/min; temperature: 40°C; BPR: 150 bar; UV: 254) to afford 15.2 mg of the title compound.
LC-MS (OA01b02): Rt = 1.52 min; MS (ESIpos): m/z = 530 [M+H]⁺
1H-NMR (500 MHz, DMSO-d6) δ [ppm]: 0.000 (2.04), 0.994 (0.47), 1.079 (16.00), 1.245 (0.83), 1.280 (0.52), 1.295 (0.57), 1.305 (0.99), 1.314 (0.84), 1.319 (0.79), 1.330 (1.05), 1.339 (0.97), 1.363 (0.71), 1.585 (0.55), 1.878 (0.96), 1.904 (0.92), 1.936 (0.65), 1.956 (0.47), 2.348 (0.41), 2.504 (0.86), 2.508 (0.71), 2.699 (0.60), 2.814 (0.47), 2.819 (0.49), 2.987 (0.69), 3.007 (0.64), 3.054 (0.96), 3.080 (1.26), 3.104 (0.74), 3.186 (6.27), 3.896 (0.72), 4.652 (0.64), 4.677 (0.60), 7.571 (0.65), 7.587 (0.76), 7.721 (0.54), 7.737 (0.91), 7.753 (0.45), 8.248 (2.84), 8.477 (0.60), 8.493 (0.58), 8.771 (1.08).

### Example I-64-2

### 2-[1-(6-{[(3S)-1-azabicyclo[2.2.2]octan-3-yl]amino}-1-[3-(trif)uoromethy))pheny)]-1H-pyrazolo[3,4-d]pyrimidin-4-yl)piperidin-4-yl]propan-2-ol

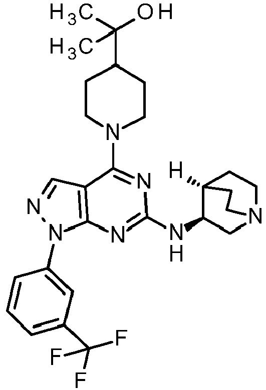

Synthesis as described for the previous example but using (3S)-quinuclidin-3-amine dihydrochloride (54.3 mg, CAS 119904-90-4) afforded 14 mg of the title compound.
LC-MS (OA01b02): Rₜ = 1.52 min; MS (ESIpos): m/z = 530 [M+H]⁺
¹H-NMR (400 MHz, DMSO-d6) δ [ppm]: 0.762 (0.44), 0.786 (0.62), 0.804 (0.75), 0.825 (0.58), 0.840 (0.66), 0.847 (0.62), 0.854 (0.62), 0.874 (0.53), 0.892 (0.80), 0.909 (0.75), 0.958 (0.49), 0.974 (0.66), 0.977 (0.75), 0.996 (0.62), 1.052 (16.00), 1.106 (0.40), 1.115 (0.49), 1.121 (0.71), 1.130 (0.44), 1.175 (0.80), 1.234 (1.33), 1.244 (1.37), 1.267 (1.42), 1.276 (1.42), 1.308 (0.98), 1.337 (0.58), 1.349 (0.49), 1.362 (0.49), 1.413 (0.53), 1.432 (0.49), 1.532 (0.53), 1.562 (0.75), 1.815 (0.44), 1.850 (1.42), 1.884 (1.82), 1.935 (0.62), 2.095 (0.53), 2.233 (0.53), 2.322 (0.75), 2.326 (1.02), 2.331 (0.75), 2.522 (3.59), 2.601 (0.53), 2.631 (0.80), 2.659 (0.75), 2.664 (0.93), 2.669 (1.11), 2.673 (0.80), 2.796 (0.62), 2.824 (0.44), 2.874 (0.49), 2.896 (0.93), 2.922 (0.71), 3.037 (0.49), 4.200 (2.44), 4.848 (0.49), 4.878 (0.49), 4.980 (0.44), 5.004 (0.40), 7.610 (0.89), 7.629 (1.15), 7.743 (0.71), 7.763 (1.15), 7.783 (0.62), 8.339 (3.28), 8.478 (0.80), 8.498 (0.75), 8.797 (1.02).

### Example I-65

### 2-[1-(6-{[rel-(3R,4R)-4-fluoropyrrolidin-3-yl]amino}-1-[3-(trifluoromethyl)phenyl]-1H-pyrazolo[3,4-d]pyrimidin-4-yl)piperidin-4-yl]propan-2-ol

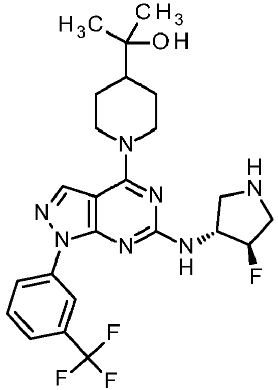

### Step a)

### tert-butyl rel-(3R,4R)-3-fluoro-4-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidine-1-carboxylate

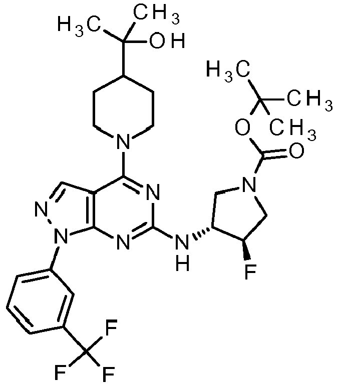

A solution of 2-(1-{6-chloro-1-[3-(trifluoromethyl)phenyl]-1H-pyrazolo[3,4-d]pyrimidin-4-yl}-piperidin-4-yl)propan-2-ol (100 mg, 0.23 mmol) and tert-butyl rel-(3R,4R)-3-amino-4-fluoro-pyrrolidine-1-carboxylate (92.9 mg, 0.46 mmol, CAS 1363382-79-9) in NMP (2.5 ml) was treated with DIPEA (0.24 ml) and the mixture was stirred at 130°C for 16 h. Standard workup afforded tert-butyl rel-(3R,4R)-3-fluoro-4-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidine-1-carboxylate (90 mg) which was directly used in the next step.
LC-MS (method HTpost_bas): Rₜ = 1.52 min; MS (ESIpos): m/z = 608 [M+H]⁺

### Step b)

A solution of tert-butyl rel-(3R,4R)-3-fluoro-4-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidine-1-carboxylate (90 mg, 0.15 mmol) in dichlormethane (1.7 ml) was treated with TFA (0.34 ml) and the mixture was stirred at rt for 16 h. The mixture was concentrated and purified by HPLC (method HT_PREP_bas) to afford 41 mg 2-[1-(6-{[rel-(3R,4R)-4-fluoropyrrolidin-3-yl]amino}-1-[3-(trifluoromethyl)phenyl]-1H-pyrazolo[3,4-d]pyrimidin-4-yl)piperidin-4-yl]propan-2-ol.
LC-MS (method HTpost_bas): Rₜ = 1.22 min; MS (ESIpos): m/z = 508 [M+H]⁺
¹H-NMR (400 MHz, DMSO-d6) δ [ppm]: 1.051 (16.00), 1.232 (0.46), 1.258 (0.50), 1.832 (0.73), 1.862 (0.66), 2.326 (0.43), 2.518 (1.42), 2.522 (1.01), 2.668 (0.44), 2.883 (0.56), 2.915 (0.44), 2.948 (0.60), 2.994 (0.52), 3.017 (0.47), 3.171 (0.55), 3.188 (0.62), 3.201 (0.55), 3.217 (0.49), 4.185 (3.39), 4.968 (0.41), 5.110 (0.40), 7.602 (0.74), 7.621 (1.04), 7.699 (0.74), 7.719 (1.16), 7.739 (0.54), 8.353 (3.84).

### Example I-66

### cis-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylcyclobutanecarboxylic acid

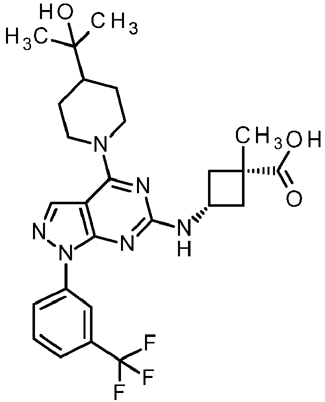

### Step a)

### ethyl cis-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylcyclobutanecarboxylate

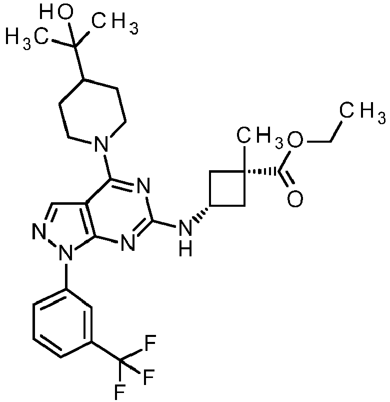

A solution of 2-(1-{6-chloro-1-[3-(trifluoromethyl)phenyl]-1H-pyrazolo[3,4-d]pyrimidin-4-yl}-piperidin-4-yl)propan-2-ol (80.0 mg, 182 µmol) and ethyl cis-3-amino-1-methylcyclobutanecarboxylate (57.2 mg, 364 µmol, CAS 1408075-83-1) in NMP (2.0 ml) was treated with DIPEA (0.19 ml) and the mixture was stirred at 130°C for 16 h. Standard workup and purification by HPLC (method HT_PREP _bas) afforded 60 mg ethyl cis-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylcyclobutanecarboxylate
LC-MS (method HTpost_bas): Rₜ = 1.55 min; MS (ESIpos): m/z = 561 [M+H]⁺

### Step b)

A solution of ethyl cis-3-({4-[4-(2-hydroxypropan-2-yl)piperidin-1-yl]-1-[3-(trifluoromethyl)-phenyl]-1H-pyrazolo[3,4-d]pyrimidin-6-yl}amino)-1-methylcyclobutanecarboxylate (60 mg, 0.11 mmol)in 2-methyltetrahydrofuran (2.0 ml) was treated with 2M aq. sodium hydroxide solution (1.0 ml) and the mixture was stirred at RT for 16 h. 10% aq. citric acid solution and ethyl acetate were added and stirring was continued for 5 min. The organic layer was separated and the mixture was extracted with ethyl acetate, the combined organic layers were concentrated and the residue was purified by HPLC (method HT_PREP_acid) to afford 20 mg cis-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylcyclobutanecarboxylic acid.
LC-MS (method HTpost_acid): Rₜ = 1.28 min; MS (ESIpos): m/z = 533 [M+H]⁺
¹H-NMR (400 MHz, DMSO-d6) δ [ppm]: 0.000 (3.85), 1.053 (16.00), 1.224 (0.71), 1.250 (0.76), 1.392 (7.53), 1.555 (0.59), 1.831 (0.91), 1.861 (0.85), 2.079 (2.15), 2.216 (0.97), 2.353 (0.75), 2.376 (1.14), 2.401 (0.62), 2.994 (0.47), 4.184 (2.37), 4.399 (0.66), 4.418 (0.65), 7.356 (0.46), 7.609 (0.82), 7.628 (1.05), 7.726 (0.55), 7.745 (0.87), 7.765 (0.42), 8.320 (4.07), 9.160 (0.54).

### Example I-67

### 2-methyl-1-[1-(6-{[trans-3-(methylsulfonyl)cyclobutyl]amino}-1-[3-(trifluoromethyl)-phenyl]-1H-pyrazolo[3,4-d]pyrimidin-4-yl)azetidin-3-yl]propan-2-ol

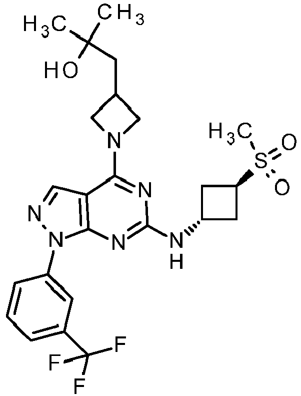

DIPEA (0.23 ml) was added to 1-(1-{6-chloro-1-[3-(trifluoromethyl)phenyl]-1H-pyrazolo[3,4-d]pyrimidin-4-yl}azetidin-3-yl)-2-methylpropan-2-ol (98.0 mg) and trans-3-(methylsulfonyl)-cyclobutanamine hydrochloride (82.9 mg, 446 µmol) in NMP (1.5 ml) and the mixture was stirred 16.5 h at 120°C. Standard workup and purification by HPLC (method HT_PREP_acid) afforded two batches oft he product (23.4 mg and 28.4 mg).
LC-MS (method HTpost_acid): Rₜ = 1.10 min; MS (ESIpos): m/z = 539 [M+H]⁺
¹H-NMR (400 MHz, DMSO-d6) δ [ppm]: 1.104 (16.00), 1.764 (1.79), 1.782 (1.81), 2.327 (0.48), 2.518 (2.07), 2.522 (1.29), 2.665 (0.50), 2.669 (0.69), 2.673 (0.64), 2.678 (0.54), 2.711 (0.64), 2.924 (8.20), 3.046 (0.48), 3.853 (0.63), 3.863 (0.64), 4.261 (3.25), 7.602 (0.97), 7.621 (1.14), 7.705 (0.85), 7.725 (1.34), 7.745 (0.63), 8.040 (5.20).

### Example I-68

### 2-methyl-1-[1-(6-{[1-(methylsulfonyl)azetidin-3-yl]amino}-1-[3-(trifluoromethyl)phenyl]-1H-pyrazolo[3,4-d]pyrimidin-4-yl)azetidin-3-yl]propan-2-ol

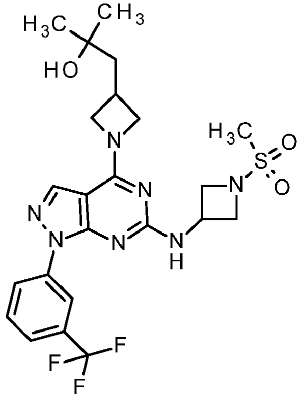

DIPEA (0.23 ml) was added to 1-(1-{6-chloro-1-[3-(trifluoromethyl)phenyl]-1H-pyrazolo[3,4-d]pyrimidin-4-yl}azetidin-3-yl)-2-methylpropan-2-ol (98.0 mg) and 1-(methylsulfonyl)azetidin-3-amine (67.1 mg, 0.45 mmol) in NMP (1.5 ml) and the mixture was stirred for 16.5 h at 120°C Standard workup and purification by HPLC (method HT_PREP_acid) afforded 55 mg of the title compound.
LC-MS (method HTpost_acid): Rₜ = 1.17 min; MS (ESIpos): m/z = 540 [M+H]⁺
¹H-NMR (400 MHz, DMSO-d6) δ [ppm]: 1.104 (16.00), 1.765 (2.16), 1.783 (2.21), 2.074 (1.22), 2.327 (0.40), 2.518 (1.90), 2.522 (1.17), 2.669 (0.42), 3.025 (15.31), 3.051 (0.57), 3.069 (0.41), 3.879 (0.83), 3.897 (1.19), 3.914 (0.81), 4.092 (0.89), 4.110 (1.10), 4.264 (3.86), 4.551 (0.42), 4.619 (0.54), 4.635 (0.52), 7.619 (0.84), 7.639 (1.09), 7.755 (0.44), 7.774 (0.68), 8.067 (5.51).

### Example I-69

### (3R,4S)-4-({4-[3-(morpholin-4-yl)azetidin-1-yl]-1-[3-(trifluoromethyl)phenyl]-1H-pyrazolo[3,4-d]pyrimidin-6-yl}amino)tetrahydrofuran-3-ol

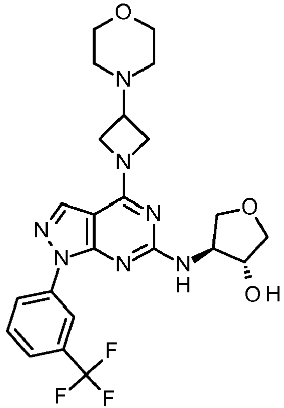

DIPEA (0.19 ml) was added to 6-chloro-4-[3-(morpholin-4-yl)azetidin-1-yl]-1-[3-(trifluoromethyl)phenyl]-1H-pyrazolo[3,4-d]pyrimidine (80.0 mg) and (3R,4S)-4-aminotetra-hydrofuran-3-ol (37.6 mg) in NMP (1.5 ml) and the mixture was stirred for 20 h at 130°C. Addition of water led to the precipitation of a solid, which was filtered, washed with water and 2-methoxy-2-methylpropane. After drying, 60 mg of the title compound was obtained.
LC-MS (OA01b02): Rₜ = 1.10 min; MS (ESIpos): m/z = 506 [M+H]⁺
¹H-NMR (400 MHz, DMSO-d6) δ [ppm]: -0.008 (1.92), 0.008 (2.04), 1.157 (0.42), 1.175 (0.77), 1.193 (0.38), 1.991 (1.50), 2.335 (1.23), 2.339 (0.65), 2.407 (6.19), 2.521 (5.92), 2.526 (3.85), 2.677 (1.15), 2.681 (0.50), 3.368 (2.19), 3.379 (1.38), 3.385 (1.27), 3.397 (0.58), 3.515 (3.04), 3.520 (3.04), 3.538 (3.27), 3.544 (3.38), 3.618 (6.35), 3.628 (9.58), 3.639 (6.62), 3.893 (3.00), 3.905 (3.46), 3.916 (3.00), 3.928 (2.92), 4.002 (0.77), 4.020 (0.85), 4.038 (0.73), 4.054 (1.92), 4.068 (2.92), 4.075 (1.96), 4.089 (2.46), 4.144 (1.46), 4.289 (2.73), 4.433 (0.85), 5.151 (6.31), 5.161 (6.27), 7.328 (0.81), 7.605 (2.35), 7.624 (3.12), 7.723 (1.50), 7.743 (2.42), 7.762 (1.15), 8.091 (16.00), 8.578 (0.92), 8.788 (0.77).

### Example I-70

### rel-(3R,4R)-4-({4-[3-(morpholin-4-yl)azetidin-1-yl]-1-[3-(trifluoromethyl)phenyl]-1H-pyrazolo[3,4-d]pyrimidin-6-yl}amino)tetrahydrofuran-3-ol

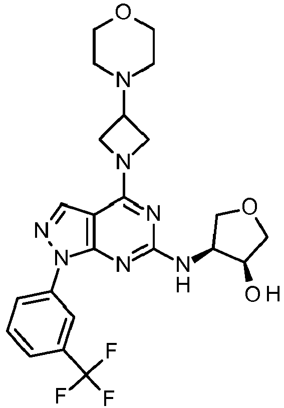

6-chloro-4-[3-(morpholin-4-yl)azetidin-1-yl]-1-[3-(trifluoromethyl)phenyl]-1H-pyrazolo[3,4-d]pyrimidine (80.0 mg) and rel-(3R,4R)-4-aminotetrahydrofuran-3-ol (37.6 mg, 365 µmol, CAS 535936-61-9) were transformed as described in the previous example to 57 mg of the title compound
LC-MS (method b02): Rₜ = 1.13 min; MS (ESIpos): m/z = 506 [M+H]⁺
¹H-NMR (400 MHz, DMSO-d6) δ [ppm]: -0.008 (2.11), 0.008 (2.29), 0.934 (1.84), 0.951 (1.87), 1.106 (0.63), 2.178 (0.48), 2.407 (6.01), 2.521 (6.58), 2.525 (4.44), 2.695 (2.51), 3.285 (0.69), 3.303 (0.94), 3.349 (1.57), 3.366 (2.14), 3.378 (1.33), 3.618 (8.66), 3.624 (11.38), 3.636 (6.58), 3.641 (6.10), 3.647 (3.98), 3.921 (1.81), 3.933 (2.20), 3.945 (1.78), 3.956 (1.84), 4.014 (2.90), 4.033 (4.56), 4.052 (2.54), 4.192 (0.94), 4.272 (1.96), 4.353 (1.24), 4.434 (0.91), 5.320 (3.20), 5.332 (3.35), 6.474 (0.85), 7.608 (2.42), 7.627 (3.08), 7.734 (1.84), 7.754 (2.99), 7.775 (1.39), 8.103 (16.00), 8.406 (0.45), 8.424 (0.72), 8.450 (0.69), 8.911 (0.78).

### Example I-71

### trans-1-methyl-N³-{4-[3-(methylsulfonyl)azetidin-1-yl]-1-[3-(trifluoromethyl)phenyl]-1H-pyrazolo[3,4-d]pyrimidin-6-yl}cyclobutane-1,3-diamine

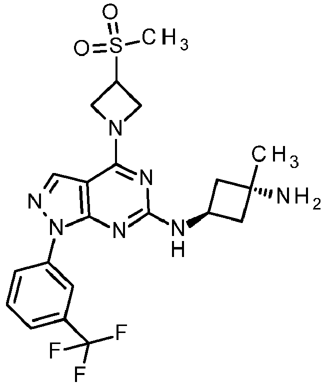

The synthesis was performed using 6-chloro-4-[3-(methylsulfonyl)azetidin-1-yl]-1-[3-(trifluoromethyl)phenyl]-1H-pyrazolo[3,4-d]pyrimidine (86 mg) and tert-butyl (trans-3-amino-1-methylcyclobutyl)carbamate (76 mg) analogously the preparation of example I-57 to afford 59 mg of the title compound.
LC-MS (method HTpost_bas): Rₜ = 1.14 min; MS (ESIpos): m/z = 496 [M+H]⁺
¹H-NMR (400 MHz, DMSO-d6) δ [ppm]: -0.008 (0.49), 0.008 (0.49), 1.219 (16.00), 1.944 (0.89), 1.966 (1.13), 2.210 (0.84), 2.521 (2.22), 2.526 (1.43), 2.543 (0.53), 3.120 (15.62), 4.362 (0.46), 4.526 (0.87), 7.492 (0.43), 7.610 (0.96), 7.629 (1.27), 7.728 (0.62), 7.748 (1.00), 7.768 (0.47), 8.130 (7.92), 8.530 (0.41), 8.547 (0.49), 8.914 (0.59).

### Example I-72

### cis-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol

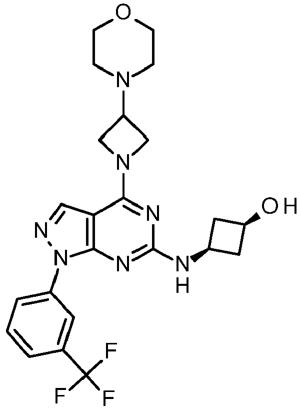

DIPEA (0.19 ml) was added to 6-chloro-4-[3-(morpholin-4-yl)azetidin-1-yl]-1-[3-(trifluoromethyl)phenyl]-1H-pyrazolo[3,4-d]pyrimidine (80.0 mg, 182 µmol) and cis-3-amino-cyclobutanol hydrochloride (45.1 mg, 365 µmol) in NMP (1.5 ml) and the mixture was stirred for 19 h at 130°C. Standard workup and purification by HPLC (method HT_PREP _bas) afforded 67 mg of the title compound.
LC-MS (method HTpost_bas): Rₜ = 1.17 min; MS (ESIpos): m/z = 490 [M+H]⁺
¹H-NMR (400 MHz, DMSO-d6) δ [ppm]: 1.795 (1.51), 1.802 (1.21), 1.817 (3.44), 1.823 (3.33), 1.838 (3.35), 1.845 (3.64), 1.860 (1.27), 1.867 (1.64), 2.078 (2.18), 2.403 (7.43), 2.522 (4.99), 2.526 (3.29), 2.543 (0.65), 2.610 (2.51), 3.356 (2.69), 3.368 (1.67), 3.386 (0.74), 3.616 (7.44), 3.627 (11.44), 3.637 (7.55), 3.796 (1.27), 3.814 (1.75), 3.832 (2.28), 3.848 (2.10), 3.866 (1.17), 3.976 (0.86), 4.176 (1.03), 4.248 (1.10), 4.411 (0.94), 5.056 (1.96), 7.434 (1.16), 7.595 (2.96), 7.614 (3.86), 7.724 (1.67), 7.744 (2.68), 7.764 (1.30), 8.061 (16.00), 8.078 (0.41), 8.460 (1.04), 9.042 (1.50).

### Example I-73

### rel-(1R,2S)-2-({4-[3-(morpholin-4-yl)azetidin-1-yl]-1-[3-(trifluoromethyl)phenyl]-1H-pyrazolo[3,4-d]pyrimidin-6-yl}amino)cyclobutanol

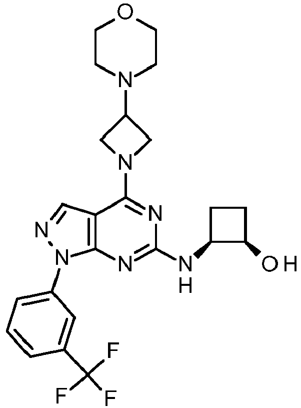

The synthesis was performed analogously to example I-72 using 45 mg rel-(1R,2S)-2-aminocyclobutanol hydrochloride affording 15.5 mg of the title compound (HPLC method: method HT_PREP_bas).
LC-MS (method HTpost_bas): Rₜ = 1.25 min; MS (ESIpos): m/z = 490 [M+H]⁺
¹H-NMR (400 MHz, DMSO-d6) δ [ppm]: 1.810 (0.88), 1.978 (0.55), 1.990 (0.81), 2.003 (1.37), 2.037 (0.90), 2.061 (1.60), 2.078 (2.51), 2.088 (1.97), 2.405 (5.37), 2.522 (2.81), 2.526 (1.83), 2.543 (0.49), 3.144 (1.05), 3.360 (1.92), 3.372 (1.23), 3.389 (0.52), 3.616 (5.39), 3.627 (8.35), 3.637 (5.55), 3.999 (0.67), 4.182 (0.89), 4.260 (0.88), 4.318 (0.92), 4.389 (1.44), 5.144 (1.55), 5.156 (1.39), 6.595 (2.73), 6.610 (2.77), 7.596 (2.31), 7.616 (3.02), 7.724 (2.10), 7.744 (3.46), 7.764 (1.71), 8.078 (16.00), 8.465 (0.62), 8.990 (0.76).

### Example I-74

### trans-3-({4-[3-(morpholin-4-yl)azetidin-1-yl]-1-[3-(trifluoromethyl)phenyl]-1H-pyrazolo-[3,4-d]pyrimidin-6-yl}amino)cyclobutanol

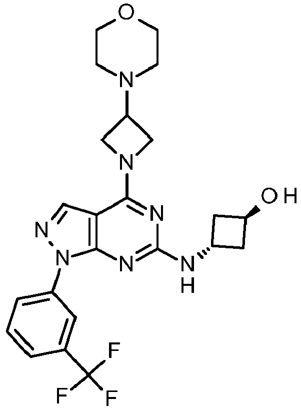

The synthesis was performed analogously to example I-72 using 45 mg trans-3-aminocyclo-butanol hydrochloride affording 68 mg of the title compound (HPLC method: method HT_PREP _bas).
LC-MS (method HTpost_bas): Rₜ = 1.15 min; MS (ESIpos): m/z = 490 [M+H]⁺
¹H-NMR (400 MHz, DMSO-d6) δ [ppm]: 2.077 (0.78), 2.165 (2.52), 2.180 (2.49), 2.282 (2.24), 2.320 (1.22), 2.325 (1.70), 2.330 (2.04), 2.335 (1.50), 2.339 (0.81), 2.405 (6.80), 2.521 (6.78), 2.526 (4.33), 2.663 (0.55), 2.667 (1.25), 2.672 (1.68), 2.677 (1.24), 2.681 (0.55), 3.310 (0.43), 3.360 (2.42), 3.373 (1.48), 3.377 (1.49), 3.390 (0.67), 3.617 (6.70), 3.628 (10.26), 3.638 (6.79), 3.980 (0.75), 4.241 (1.72), 4.284 (1.76), 4.300 (2.33), 4.315 (2.09), 4.330 (1.21), 4.415 (0.87), 4.968 (2.66), 4.982 (2.37), 7.442 (1.12), 7.594 (2.83), 7.614 (3.73), 7.717 (2.10), 7.738 (3.51), 7.757 (1.66), 8.066 (16.00), 8.479 (0.94), 8.959 (1.25).

### Example I-75

### rel-(1R,2R)-2-({4-[3-(morpholin-4-yl)azetidin-1-yl]-1-[3-(trifluoromethyl)phenyl]-1H-pyrazolo[3,4-d]pyrimidin-6-yl}amino)cyclobutanol

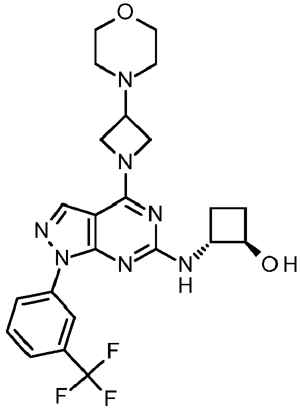

The synthesis was performed analogously to example I-72 using 45 mg rel-(1R,2R)-2-amino-cyclobutanol hydrochloride affording 54 mg of the title compound (method: method HT_PREP _bas) .
LC-MS (method HTpost_bas): Rₜ = 1.18 min; MS (ESIpos): m/z = 490 [M+H]⁺
¹H-NMR (400 MHz, DMSO-d6) δ [ppm]: 1.239 (0.80), 1.262 (0.70), 1.385 (0.79), 1.408 (1.02), 1.432 (0.69), 1.953 (1.27), 1.974 (1.35), 1.997 (1.05), 2.339 (0.48), 2.406 (5.54), 2.521 (4.36), 2.526 (2.82), 2.543 (1.50), 3.350 (1.48), 3.362 (1.98), 3.374 (1.26), 3.379 (1.22), 3.391 (0.54), 3.617 (5.59), 3.628 (8.63), 3.638 (5.73), 4.026 (1.12), 4.044 (1.27), 4.160 (1.09), 4.250 (0.93), 4.417 (0.71), 5.179 (2.01), 5.195 (1.80), 7.436 (0.82), 7.600 (1.98), 7.619 (2.58), 7.728 (1.07), 7.748 (1.55), 7.767 (0.76), 8.065 (16.00), 8.521 (0.80), 8.536 (0.96), 8.954 (1.21).

### Example I-76

### cis-3-({4-[3-methyl-3-(morpholin-4-yl)azetidin-1-yl]-1-[3-(trifluoromethyl)phenyl]-1H-pyrazolo[3,4-d]pyrimidin-6-yl}amino)cyclobutanol

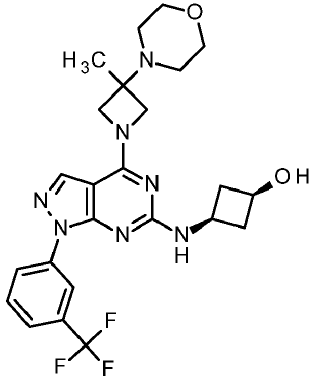

A solution of 6-chloro-4-[3-methyl-3-(morpholin-4-yl)azetidin-1-yl]-1-[3-(trifluoromethyl)-phenyl]-1H-pyrazolo[3,4-d]pyrimidine (70 mg) and cis-3-aminocyclobutanol hydrochloride (38 mg) in NMP (1.6 ml) was treated with DIPEA (0.16 ml) and the mixture was stirred at 130°C for 16 h. Standard workup and purification by HPLC (method HT_PREP _acid) afforded 56 mg of the title compound.
LC-MS (method HTpost_acid): Rₜ = 1.00 min; MS (ESIpos): m/z = 504 [M+H]⁺
¹H-NMR (400 MHz, DMSO-d6) δ [ppm]: 1.329 (15.35), 1.355 (0.42), 1.798 (0.79), 1.804 (0.59), 1.819 (1.77), 1.825 (1.70), 1.841 (1.70), 1.847 (1.86), 1.862 (0.61), 1.869 (0.83), 2.332 (0.47), 2.450 (5.09), 2.523 (1.46), 2.528 (1.01), 2.544 (0.61), 2.615 (1.32), 2.669 (0.48), 2.674 (0.54), 3.337 (16.00), 3.797 (1.31), 3.832 (1.16), 3.849 (0.99), 3.949 (0.70), 4.040 (0.80), 4.061 (0.88), 4.217 (0.83), 4.236 (0.69), 5.060 (0.88), 7.421 (0.61), 7.596 (1.57), 7.615 (2.04), 7.725 (0.89), 7.745 (1.39), 7.764 (0.68), 8.049 (8.54), 8.463 (0.54), 9.044 (0.77).

### Example I-77

### trans-1-methyl-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol

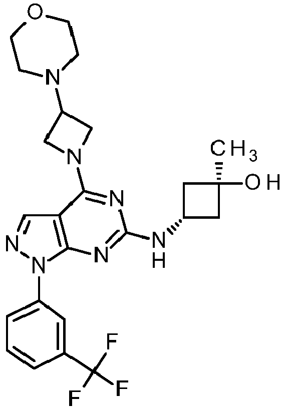

A solution of 6-chloro-4-[3-(morpholin-4-yl)azetidin-1-yl]-1-[3-(trifluoromethyl)phenyl]-1H-pyrazolo[3,4-d]pyrimidine (80.0 mg) and trans-3-amino-1-methylcyclobutanol hydrochloride (CAS number 1523571-03-0) (50 mg) in NMP (2.0 ml) was treated with DIPEA (0.19 ml) and the mixture was stirred at 130°C for 16 h. Standard workup and purification by HPLC (method HT_PREP_bas) afforded 64 mg of the title compound.
LC-MS (method HTpost_bas): Rₜ = 1.23 min; MS (ESIpos): m/z = 504 [M+H]⁺
¹H-NMR (500 MHz, DMSO-d6) δ [ppm]: -0.004 (8.08), 1.269 (16.00), 2.011 (1.15), 2.358 (1.35), 2.361 (1.43), 2.398 (3.02), 2.514 (2.06), 2.518 (1.60), 2.522 (1.21), 2.635 (0.47), 3.353 (1.53), 3.362 (0.83), 3.367 (0.84), 3.377 (0.40), 3.614 (3.24), 3.623 (5.11), 3.631 (3.41), 4.177 (0.42), 4.245 (0.49), 4.409 (0.43), 4.746 (3.20), 7.383 (0.51), 7.593 (1.27), 7.608 (1.58), 7.714 (0.84), 7.730 (1.40), 7.746 (0.68), 8.055 (6.48), 8.529 (0.47), 8.870 (0.61).

### Example I-78

### cis-1-methyl-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol

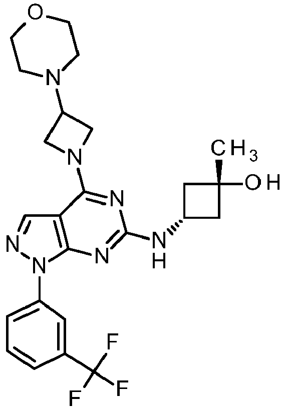

A solution of 6-chloro-4-[3-(morpholin-4-yl)azetidin-1-yl]-1-[3-(trifluoromethyl)phenyl]-1H-pyrazolo[3,4-d]pyrimidine (80.0 mg) and cis-3-amino-1-methylcyclobutanol hydrochloride (CAS number 1523606-23-6) (50.2 mg) in NMP (2.0 ml) was treated with DIPEA (0.19 ml) and the mixture was stirred at 130°C for 16 h. Standard workup and purification by HPLC (method HT_PREP_bas) afforded 66 mg of the title compound.
LC-MS (method HTpost_bas): Rₜ = 1.25 min; MS (ESIpos): m/z = 504 [M+H]⁺
¹H-NMR (500 MHz, DMSO-d6) δ [ppm]: 1.275 (16.00), 1.997 (2.12), 2.019 (3.98), 2.037 (2.49), 2.361 (4.01), 2.365 (3.72), 2.369 (3.01), 2.403 (6.28), 2.518 (3.07), 2.522 (2.92), 2.526 (2.32), 2.544 (0.85), 2.635 (0.74), 2.639 (1.02), 2.642 (0.76), 3.356 (3.27), 3.366 (1.92), 3.370 (1.80), 3.380 (0.85), 3.618 (6.85), 3.627 (10.77), 3.635 (7.11), 3.863 (1.20), 3.876 (1.22), 3.969 (0.77), 4.172 (0.86), 4.252 (0.96), 4.411 (0.87), 4.923 (4.96), 7.423 (1.64), 7.435 (1.58), 7.596 (2.78), 7.612 (3.44), 7.717 (2.10), 7.733 (3.58), 7.748 (1.74), 8.061 (13.46), 8.349 (1.44), 8.365 (1.40), 9.164 (2.43).

### Example I-79

### 1-(1-{6-[(trans-3-hydroxycyclobutyl)amino]-1-[3-(trifluoromethyl)phenyl]-1H-pyrazolo-[3,4-d]pyrimidin-4-yl}azetidin-3-yl)-4-methylpiperidin-4-ol

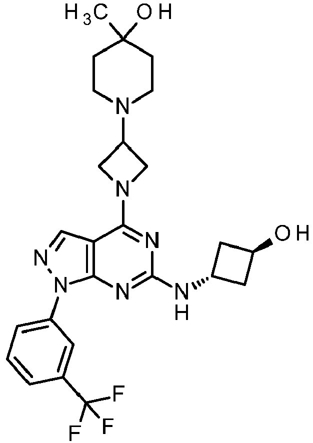

A solution of 1-(1-{6-chloro-1-[3-(trifluoromethyl)phenyl]-1H-pyrazolo[3,4-d]pyrimidin-4-yl}-azetidin-3-yl)-4-methylpiperidin-4-ol (80 mg) and trans-3-aminocyclobutanol hydrochloride (42 mg) in NMP (2.0 ml) was treated with DIPEA (0.18 ml) and the mixture was stirred at 130°C for 24 h. Standard workup and purification by HPLC (method HT_PREP_bas) afforded 62 mg of the title compound.
LC-MS (method HTpost_bas): Rₜ = 1.07 min; MS (ESIpos): m/z = 518 [M+H]⁺
¹H-NMR (400 MHz, DMSO-d6) δ [ppm]: 1.115 (16.00), 1.449 (0.49), 1.472 (2.01), 1.496 (5.11), 1.529 (0.65), 2.166 (1.67), 2.179 (1.64), 2.297 (2.53), 2.326 (1.78), 2.331 (1.80), 2.426 (1.48), 2.526 (2.94), 2.543 (0.61), 2.668 (0.59), 2.673 (0.78), 2.677 (0.57), 3.922 (0.60), 4.166 (5.59), 4.269 (0.52), 4.285 (0.85), 4.300 (1.31), 4.315 (1.22), 4.330 (0.71), 4.415 (0.70), 4.969 (1.66), 4.982 (1.47), 7.434 (0.73), 7.592 (1.73), 7.611 (2.27), 7.715 (1.27), 7.736 (2.08), 7.755 (1.00), 8.070 (8.42), 8.478 (0.65), 8.959 (0.83).

### Example I-80

### 1-(1-{6-[(1,1-dioxidotetrahydro-2H-thiopyran-4-yl)amino]-1-[3-(trifluoromethyl)phenyl]-1H-pyrazolo[3,4-d]pyrimidin-4-yl}azetidin-3-yl)-4-methylpiperidin-4-ol

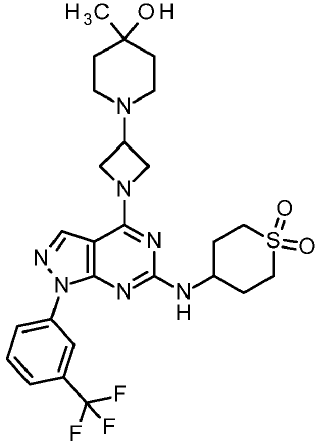

A solution of 1-(1-{6-chloro-1-[3-(trifluoromethyl)phenyl]-1H-pyrazolo[3,4-d]pyrimidin-4-yl}-azetidin-3-yl)-4-methylpiperidin-4-ol (65 mg) and tetrahydro-2H-thiopyran-4-amine 1,1-dioxide (41.5 mg) in NMP (2.0 ml) was treated with DIPEA (0.15 ml) and the mixture was stirred at 130°C for 40 h. Standard workup and purification by HPLC (method HT_PREP_bas) afforded 48 mg of the title compound.
LC-MS (method HTpost_bas): Rₜ = 1.11 min; MS (ESIpos): m/z = 580 [M+H]⁺
¹H-NMR (400 MHz, DMSO-d6) δ [ppm]: -0.004 (8.52), 1.113 (16.00), 1.446 (0.50), 1.469 (2.00), 1.494 (4.98), 1.527 (0.61), 2.074 (1.71), 2.093 (1.44), 2.110 (1.13), 2.222 (1.60), 2.301 (1.56), 2.322 (1.61), 2.326 (1.70), 2.331 (1.36), 2.427 (1.49), 2.518 (2.87), 2.522 (1.80), 2.664 (0.56), 2.669 (0.75), 2.673 (0.55), 3.191 (4.04), 3.937 (0.62), 4.104 (0.74), 4.167 (4.94), 4.191 (1.39), 4.425 (0.76), 7.360 (0.48), 7.612 (1.54), 7.631 (1.91), 7.747 (0.73), 7.766 (1.10), 8.090 (8.97), 8.564 (0.68), 8.746 (0.53).

### Example I-81

### N-[1-(ethylsulfonyl)azetidin-3-yl]-4-[3-(morpholin-4-yl)azetidin-1-yl]-1-[3-(trifluoromethyl)phenyl]-1H-pyrazolo[3,4-d]pyrimidin-6-amine

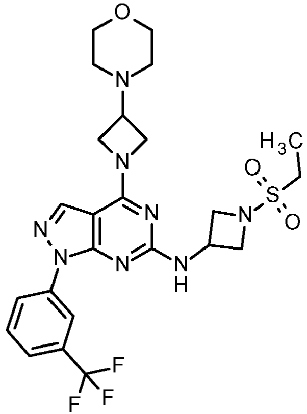

A mixture of 6-chloro-4-[3-(morpholin-4-yl)azetidin-1-yl]-1-[3-(trifluoromethyl)phenyl]-1H-pyrazolo[3,4-d]pyrimidine (84 mg) und 1-(ethylsulfonyl)azetidin-3-amine trifluoroacetate (80 mg) in NMP (2.0 ml) was treated with DIPEA (0.27 ml) and the mixture was stirred at 130°C for 43 h. Standard workup and purification by HPLC (method HT_PREP _acid) afforded 14 mg of the title compound.
LC-MS (HTpost1_acid): Rₜ = 1.00 min; MS (ESIpos): m/z = 567 [M+H]⁺
¹H-NMR (400 MHz, DMSO-d6) δ [ppm]: 1.194 (0.41), 1.223 (7.10), 1.242 (16.00), 1.260 (7.39), 2.410 (4.06), 2.521 (3.77), 2.526 (2.40), 2.543 (0.54), 3.120 (2.36), 3.138 (7.62), 3.156 (7.40), 3.175 (2.18), 3.372 (2.75), 3.620 (4.13), 3.630 (6.24), 3.640 (4.20), 3.905 (2.11), 3.924 (3.51), 3.942 (2.73), 4.004 (0.62), 4.097 (1.86), 4.199 (0.72), 4.283 (0.70), 4.439 (0.67), 4.598 (0.73), 4.611 (0.73), 7.624 (1.49), 7.644 (1.98), 7.755 (0.76), 7.774 (1.12), 7.791 (0.66), 7.876 (0.41), 8.111 (9.22), 8.267 (0.62), 8.510 (0.48), 8.531 (0.45), 8.752 (0.45).

### Example I-82

### N-[trans-3-(ethylsulfonyl)cyclobutyl]-4-[3-(morpholin-4-yl)azetidin-1-yl]-1-[3-(trifluoromethyl)phenyl]-1H-pyrazolo[3,4-d]pyrimidin-6-amine

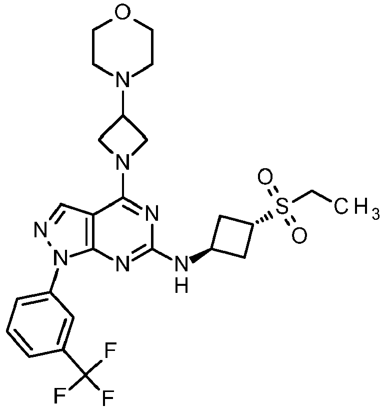

A solution of 6-chloro-4-[3-(morpholin-4-yl)azetidin-1-yl]-1-[3-(trifluoromethyl)phenyl]-1H-pyrazolo[3,4-d]pyrimidine (80.0 mg) and trans-3-(ethylsulfonyl)cyclobutanamine hydrochloride (73 mg) in NMP (2.0 ml) was treated with DIPEA (0.19 ml) and the mixture was stirred at 130°C for 16 h. Standard workup and purification by HPLC (method HT_PREP _acid) afforded 24 mg of the title compound.
LC-MS (method HTpost_acid): Rₜ = 0.93 min; MS (ESIpos): m/z = 566 [M+H]⁺
¹H-NMR (400 MHz, DMSO-d6) δ [ppm]: 1.190 (7.52), 1.208 (16.00), 1.227 (7.80), 2.331 (0.92), 2.410 (6.80), 2.673 (1.14), 2.724 (2.16), 3.018 (1.53), 3.035 (4.12), 3.054 (4.09), 3.072 (1.51), 3.369 (2.85), 3.629 (9.28), 3.915 (1.53), 3.927 (1.27), 3.985 (0.91), 4.194 (0.98), 4.272 (1.09), 4.450 (1.40), 7.610 (2.64), 7.629 (3.93), 7.711 (2.25), 7.731 (3.55), 7.752 (1.63), 8.087 (9.39), 8.517 (0.90), 8.766 (1.04).

### Example I-83

### 2-[1-(6-{[trans-3-(methylsulfonyl)cyclobutyl]amino}-1-[3-(trifluoromethyl)phenyl]-1H-pyrazolo[3,4-d]pyrimidin-4-yl)azetidin-3-yl]propan-2-ol

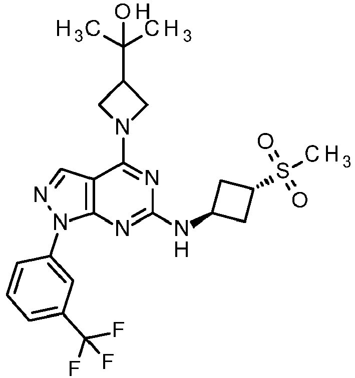

A solution of 2-(1-{6-chloro-1-[3-(trifluoromethyl)phenyl]-1H-pyrazolo[3,4-d]pyrimidin-4-yl}-azetidin-3-yl)propan-2-ol (75.0 mg) and trans-3-(methylsulfonyl)cyclobutanamine hydrochloride in NMP (2.0 ml) was treated with DIPEA (0.19 ml) and the mixture was stirred at 130°C for 40 h. Standard workup and purification by HPLC (method HT_PREP _acid) afforded 22 mg of the title compound.
LC-MS (method HTpost_acid): Rₜ = 1.07 min; MS (ESIpos): m/z = 525 [M+H]⁺
¹H-NMR (400 MHz, DMSO-d6) δ [ppm]: 1.107 (15.69), 2.321 (0.43), 2.326 (0.93), 2.330 (1.26), 2.335 (0.95), 2.340 (0.44), 2.521 (5.88), 2.526 (3.61), 2.543 (0.95), 2.668 (1.23), 2.673 (1.72), 2.677 (1.62), 2.682 (1.43), 2.716 (2.00), 2.774 (0.50), 2.793 (1.21), 2.811 (1.74), 2.830 (1.24), 2.847 (0.48), 2.930 (16.00), 3.858 (1.20), 3.869 (1.34), 3.881 (1.04), 4.066 (2.36), 4.263 (0.79), 4.283 (1.74), 4.299 (1.57), 4.313 (1.35), 4.333 (1.60), 4.355 (0.62), 4.491 (0.73), 4.627 (7.33), 7.579 (0.73), 7.605 (2.54), 7.624 (3.23), 7.709 (2.50), 7.729 (3.94), 7.749 (1.83), 8.099 (15.51), 8.530 (0.77), 8.761 (0.86).

### Example I-84

### (3R,4R)-1-(6-{[1-(ethylsulfonyl)azetidin-3-yl]amino}-1-[3-(trifluoromethyl)phenyl]-1H-pyrazolo[3,4-d]pyrimidin-4-yl)pyrrolidine-3,4-diol

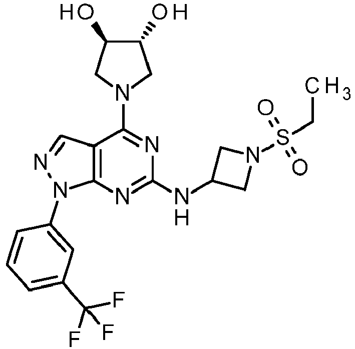

A solution of (3R,4R)-1-{6-chloro-1-[3-(trifluoromethyl)phenyl]-1H-pyrazolo[3,4-d]pyrimidin-4-yl}pyrrolidine-3,4-diol (80.0 mg) and 1-(ethylsulfonyl)azetidin-3-amine trifluoroacetate (111 mg) in NMP (2.0 ml) was treated with DIPEA (0.21 ml) and the mixture was stirred at 130°C for 40 h. Standard workup and purification by HPLC (method HT_PREP _acid) afforded 35 mg of the title compound.
LC-MS (method HTpost_acid): Rₜ = 1.03 min; MS (ESIpos): m/z = 528 [M+H]⁺
¹H-NMR (400 MHz, DMSO-d6) δ [ppm]: 1.229 (6.87), 1.248 (16.00), 1.266 (7.32), 2.522 (2.69), 2.526 (1.76), 2.543 (1.97), 3.130 (2.23), 3.149 (7.43), 3.167 (7.28), 3.185 (2.06), 3.610 (1.58), 3.638 (1.80), 3.693 (3.08), 3.923 (1.79), 3.942 (3.03), 3.959 (3.03), 3.969 (1.29), 3.988 (0.94), 3.997 (0.89), 4.049 (1.87), 4.089 (1.76), 4.109 (3.31), 4.128 (1.56), 4.172 (1.64), 4.629 (0.67), 4.643 (0.65), 5.250 (1.45), 5.351 (1.43), 7.623 (1.44), 7.642 (1.89), 7.752 (0.75), 7.772 (0.96), 8.265 (8.90).

### Example I-85

### (3R,4R)-1-(6-{[(1s,3S)-3-hydroxycyclobutyl]amino}-1-[3-(trifluoromethyl)phenyl]-1H-pyrazolo[3,4-d]pyrimidin-4-yl)pyrrolidine-3,4-diol

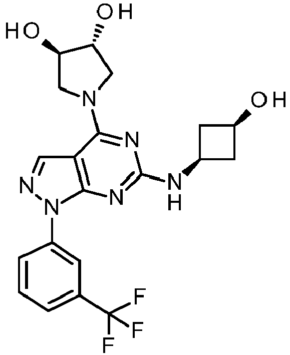

A solution of (3R,4R)-1-{6-chloro-1-[3-(trifluoromethyl)phenyl]-1H-pyrazolo[3,4-d]pyrimidin-4-yl}pyrrolidine-3,4-diol (80 mg) and cis-3-aminocyclobutanol hydrochloride (49.5 mg) in NMP (2.0 ml) was treated with DIPEA (0.21 ml) and the mixture was stirred at 130°C for 40 h. Standard workup and purification by HPLC (method HT_PREP _acid) afforded 30 mg of the title compound.
LC-MS (method HTpost_acid): Rₜ = 0.88 min; MS (ESIpos): m/z = 451 [M+H]⁺
¹H-NMR (400 MHz, DMSO-d6) δ [ppm]: 1.810 (1.28), 1.833 (2.89), 1.838 (2.64), 1.859 (2.99), 1.881 (1.46), 2.321 (0.53), 2.325 (1.22), 2.330 (1.71), 2.335 (1.21), 2.339 (0.55), 2.521 (7.14), 2.526 (4.45), 2.543 (6.57), 2.631 (2.22), 2.663 (1.22), 2.667 (1.66), 2.672 (1.97), 2.677 (1.39), 2.681 (0.67), 3.590 (2.84), 3.617 (3.32), 3.678 (2.98), 3.834 (1.82), 3.852 (2.12), 3.871 (1.43), 3.941 (1.64), 3.951 (2.03), 3.969 (1.66), 3.978 (1.58), 4.032 (3.07), 4.156 (2.75), 5.060 (0.90), 5.220 (1.09), 5.328 (1.14), 7.265 (0.84), 7.593 (2.49), 7.612 (3.23), 7.724 (1.28), 7.744 (2.06), 7.764 (1.02), 8.207 (16.00), 8.479 (0.68), 9.095 (0.82).

### Example I-86

### (3R,4R)-4-fluoro-1-(6-{[(1r,3R)-3-(methylsulfonyl)cyclobutyl]amino}-1-[3-(trifluoromethyl)phenyl]-1H-pyrazolo[3,4-d]pyrimidin-4-yl)pyrrolidin-3-ol

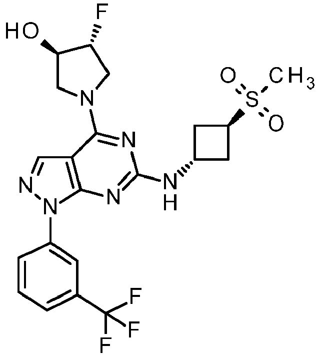

A solution of (3R,4R)-1-{6-chloro-1-[3-(trifluoromethyl)phenyl]-1H-pyrazolo[3,4-d]pyrimidin-4-yl}-4-fluoropyrrolidin-3-ol (80 mg) and trans-3-(methylsulfonyl)cyclobutanamine hydrochloride (73.9 mg) in NMP (2.0 ml) was treated with DIPEA (0.21 ml) and the mixture was stirred at 130°C for 16 h. Standard workup and purification by HPLC (method HT_PREP _acid) afforded 68 mg of the title compound.
LC-MS (method HTpost_acid): Rₜ = 1.08 min; MS (ESIpos): m/z = 515 [M+H]⁺
¹H-NMR (400 MHz, DMSO-d6) δ [ppm]: 2.078 (1.50), 2.522 (6.09), 2.526 (4.50), 2.543 (2.28), 2.733 (2.89), 2.942 (15.69), 3.701 (0.64), 3.766 (1.40), 3.795 (1.77), 3.849 (1.81), 3.862 (1.66), 3.883 (2.68), 3.895 (1.87), 3.968 (0.88), 4.002 (1.49), 4.037 (1.40), 4.069 (1.31), 4.170 (0.53), 4.367 (0.83), 4.490 (1.38), 5.054 (0.75), 5.137 (0.87), 5.183 (0.76), 5.262 (0.86), 5.669 (0.67), 5.776 (0.76), 7.550 (0.86), 7.618 (3.13), 7.638 (4.26), 7.721 (3.50), 7.741 (5.58), 7.761 (2.52), 8.265 (16.00), 8.293 (0.77), 8.557 (0.82), 8.621 (0.77), 8.774 (0.82).

### Example I-87

### (3R,4R)-1-(6-{[(1r,3R)-3-(ethylsulfonyl)cyclobutyl]amino}-1-[3-(trifluoromethyl)phenyl]-1H-pyrazolo[3,4-d]pyrimidin-4-yl)-4-fluoropyrrolidin-3-ol

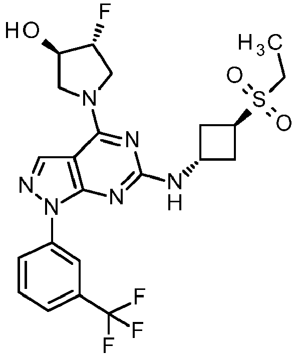

A solution of (3R,4R)-1-{6-chloro-1-[3-(trifluoromethyl)phenyl]-1H-pyrazolo[3,4-d]pyrimidin-4-yl}-4-fluoropyrrolidin-3-ol (80 mg) and trans-3-(ethylsulfonyl)cyclobutanamine hydrochloride (79.5 mg) in NMP (2.0 ml) was treated with DIPEA (0.21 ml) and the mixture was stirred at 130°C for 16 h. Standard workup and purification by HPLC (method HT_PREP _acid) afforded 75 mg of the title compound.
LC-MS (method HTpost_acid): Rₜ = 1.12 min; MS (ESIpos): m/z = 529 [M+H]⁺
¹H-NMR (400 MHz, DMSO-d6) δ [ppm]: 1.197 (6.88), 1.216 (16.00), 1.234 (7.34), 2.523 (2.76), 2.527 (1.99), 2.544 (0.70), 2.678 (0.41), 2.739 (1.26), 3.049 (1.64), 3.067 (1.65), 3.767 (0.63), 3.795 (0.80), 3.848 (0.61), 3.881 (0.55), 3.929 (1.08), 3.964 (0.54), 4.002 (0.66), 4.036 (0.62), 4.068 (0.59), 4.477 (0.75), 5.666 (0.51), 5.776 (0.61), 7.556 (0.40), 7.618 (1.40), 7.638 (1.93), 7.720 (1.52), 7.741 (2.43), 7.761 (1.12), 8.265 (6.74).

### Example I-88

### (3R,4R)-4-fluoro-1-(6-{[(1r,3R)-3-hydroxycyclobutyl]amino}-1-[3-(trifluoromethyl)-phenyl]-1H-pyrazolo[3,4-d]pyrimidin-4-yl)pyrrolidin-3-ol

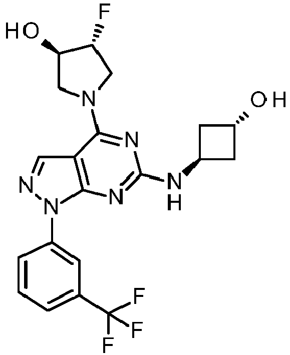

A solution of (3R,4R)-1-{6-chloro-1-[3-(trifluoromethyl)phenyl]-1H-pyrazolo[3,4-d]pyrimidin-4-yl}-4-fluoropyrrolidin-3-ol (80 mg) and trans-3-aminocyclobutanol hydrochloride (49 mg) in NMP (2.0 ml) was treated with DIPEA (0.21 ml) and the mixture was stirred at 130°C for 16 h. Standard workup and purification by HPLC (method HT_PREP_acid) afforded 66 mg of the title compound.
LC-MS (method HTpost_acid): Rₜ = 1.05 min; MS (ESIpos): m/z = 453 [M+H]⁺
¹H-NMR (500 MHz, DMSO-d6) δ [ppm]: 2.161 (2.08), 2.187 (4.78), 2.196 (4.56), 2.298 (3.70), 2.518 (7.76), 2.522 (7.51), 2.525 (6.17), 2.543 (2.65), 3.679 (0.96), 3.758 (2.03), 3.780 (2.41), 3.853 (2.01), 3.877 (1.72), 3.941 (0.83), 3.966 (1.39), 3.992 (2.09), 4.015 (2.09), 4.070 (0.84), 4.151 (0.79), 4.175 (0.57), 4.261 (1.23), 4.324 (2.78), 4.467 (1.70), 4.989 (2.13), 5.053 (1.35), 5.148 (1.62), 5.243 (1.19), 5.644 (0.79), 5.763 (0.85), 7.363 (1.83), 7.604 (5.22), 7.620 (6.51), 7.729 (3.01), 7.745 (5.01), 7.761 (2.50), 8.229 (3.83), 8.239 (16.00), 8.496 (1.36), 8.988 (1.69).

### Example I-89

### (3R,4R)-4-fluoro-1-(6-{[(1s,3S)-3-hydroxycyclobutyl]amino}-1-[3-(trifluoromethyl)-phenyl]-1H-pyrazolo[3,4-d]pyrimidin-4-yl)pyrrolidin-3-ol

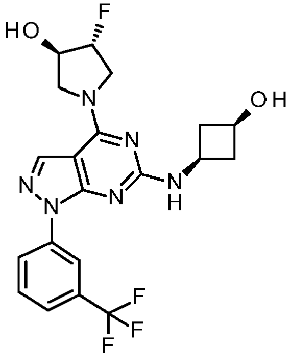

A solution of (3R,4R)-1-{6-chloro-1-[3-(trifluoromethyl)phenyl]-1H-pyrazolo[3,4-d]pyrimidin-4-yl}-4-fluoropyrrolidin-3-ol (80 mg) and cis-3-aminocyclobutanol hydrochloride (49 mg) in NMP (2.0 ml) was treated with DIPEA (0.21 ml) and the mixture was stirred at 130°C for 16 h. Standard workup and purification by HPLC (method HT_PREP_acid) afforded 57 mg of the title compound.
LC-MS (method HTpost_acid): Rₜ = 1.06 min; MS (ESIpos): m/z = 453 [M+H]⁺
¹H-NMR (500 MHz, DMSO-d6) δ [ppm]: 1.822 (2.25), 1.841 (5.52), 1.862 (5.65), 1.879 (2.48), 2.358 (1.07), 2.361 (2.24), 2.365 (3.26), 2.368 (2.33), 2.372 (1.02), 2.518 (8.67), 2.522 (8.49), 2.525 (6.81), 2.543 (2.31), 2.631 (5.52), 2.635 (6.88), 2.639 (7.68), 2.642 (6.39), 3.664 (0.85), 3.755 (2.29), 3.778 (3.04), 3.850 (4.84), 3.860 (4.68), 3.964 (1.40), 3.990 (2.10), 4.007 (2.20), 4.069 (0.92), 4.150 (0.82), 4.169 (0.59), 4.352 (1.39), 4.464 (1.53), 5.050 (2.55), 5.068 (2.25), 5.145 (2.08), 5.240 (1.26), 5.762 (1.63), 7.355 (1.74), 7.605 (5.12), 7.620 (6.26), 7.736 (2.22), 7.751 (3.27), 8.235 (16.00), 8.316 (0.88), 8.478 (1.47), 8.618 (0.57), 9.077 (1.95).

### Example I-90

### cis-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylcyclobutanol

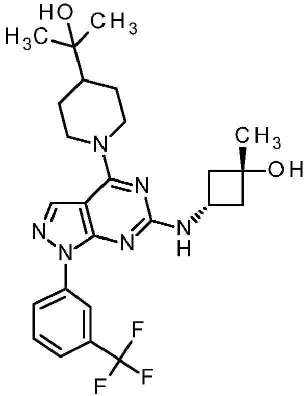

A solution of 2-(1-{6-chloro-1-[3-(trifluoromethyl)phenyl]-1H-pyrazolo[3,4-d]pyrimidin-4-yl}piperidin-4-yl)propan-2-ol (80 mg) and cis-3-amino-1-methylcyclobutanol hydrochloride (CAS number 1523606-23-6) (50 mg) in NMP (2.0 ml) was treated with DIPEA (0.19 ml) and the mixture was stirred at 130°C for 16 h. Standard workup and purification by HPLC (method HT_PREP_acid) afforded 72 mg of the title compound.
LC-MS (method HTpost_acid): Rₜ = 1.25 min; MS (ESIpos): m/z = 505 [M+H]⁺
¹H-NMR (500 MHz, DMSO-d6) δ [ppm]: 1.053 (16.00), 1.225 (0.48), 1.243 (0.52), 1.279 (5.04), 1.554 (0.42), 1.833 (0.65), 1.855 (0.60), 2.003 (0.48), 2.023 (0.88), 2.040 (0.53), 2.366 (0.92), 2.369 (0.88), 2.518 (0.85), 2.522 (0.80), 2.526 (0.65), 3.871 (0.45), 3.885 (0.44), 4.183 (4.00), 4.931 (1.07), 7.603 (0.68), 7.618 (0.85), 7.719 (0.49), 7.736 (0.84), 8.312 (4.31), 9.181 (0.44).

### Example I-91

### trans-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylcyclobutanol

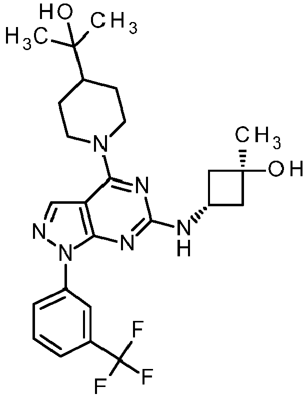

A solution of 2-(1-{6-chloro-1-[3-(trifluoromethyl)phenyl]-1H-pyrazolo[3,4-d]pyrimidin-4-yl}piperidin-4-yl)propan-2-ol (80 mg) and trans-3-amino-1-methylcyclobutanol hydrochloride (50 mg) in NMP (2.0 ml) was treated with DIPEA (0.19 ml) and the mixture was stirred at 130°C for 16 h. Standard workup and purification by HPLC (method HT_PREP _acid) afforded 40 mg of the title compound.
LC-MS (method HTpost_acid): Rₜ = 1.22 min; MS (ESIpos): m/z = 505 [M+H]⁺
¹H-NMR (500 MHz, DMSO-d6) δ [ppm]: -0.004 (7.64), 1.051 (16.00), 1.225 (0.47), 1.251 (0.54), 1.275 (4.49), 1.831 (0.63), 1.855 (0.60), 2.005 (0.62), 2.027 (0.55), 2.358 (0.72), 2.361 (0.87), 2.365 (0.71), 2.514 (1.36), 2.518 (1.28), 2.522 (1.04), 2.539 (0.56), 2.635 (0.50), 4.181 (4.70), 4.759 (1.07), 7.598 (0.70), 7.614 (0.87), 7.715 (0.46), 7.731 (0.76), 8.305 (4.39).

### Example I-92

### 3-ethyl-1-(6-{[trans-3-(methylsulfonyl)cyclobutyl]amino}-1-[3-(trifluoromethyl)phenyl]-1H-pyrazolo[3,4-d]pyrimidin-4-yl)azetidin-3-ol

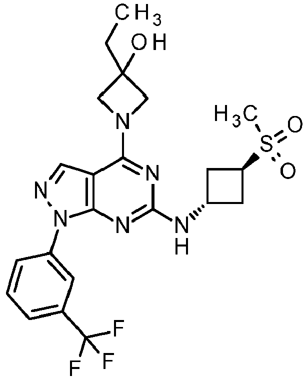

DIPEA (0.10 ml) was added to 1-{6-chloro-1-[3-(trifluoromethyl)phenyl]-1H-pyrazolo[3,4-d]-pyrimidin-4-yl}-3-ethylazetidin-3-ol (39 mg) and trans-3-(methylsulfonyl)cyclobutanamine hydrochloride (36 mg) in NMP (1.0 ml) and the mixture was stirred for 16 h at 130°C. Standard workup and purification by HPLC (method HT_PREP _acid) afforded 36 mg of the title compound.
LC-MS (method HTpost_acid): Rₜ = 1.09 min; MS (ESIpos): m/z = 511 [M+H]⁺
¹H-NMR (400 MHz, DMSO-d6) δ [ppm]: 0.923 (5.24), 0.942 (12.81), 0.959 (5.89), 1.721 (1.30), 1.739 (3.96), 1.757 (3.76), 1.775 (1.16), 2.325 (0.69), 2.331 (1.00), 2.335 (0.71), 2.521 (4.31), 2.526 (3.03), 2.543 (2.14), 2.668 (1.00), 2.672 (1.43), 2.677 (1.32), 2.718 (1.78), 2.930 (14.27), 3.859 (1.05), 3.871 (1.25), 3.882 (1.01), 3.954 (1.12), 4.033 (1.21), 4.163 (1.10), 4.318 (1.19), 4.493 (0.79), 5.762 (5.69), 7.609 (2.73), 7.628 (3.82), 7.712 (2.47), 7.732 (3.93), 7.752 (1.81), 8.109 (16.00), 8.524 (0.80), 8.760 (0.91).

### Example I-93

### 1-(6-{[trans-3-(methylsulfonyl)cyclobutyl]amino}-1-[3-(trifluoromethyl)phenyl]-1H-pyrazolo[3,4-d]pyrimidin-4-yl)piperidin-4-ol

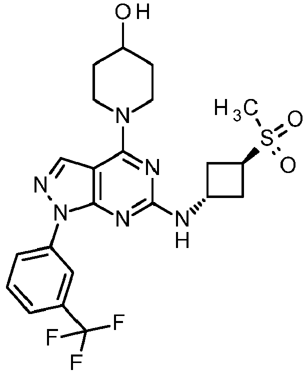

DIPEA (0.21 ml) was added to 1-{6-chloro-1-[3-(trifluoromethyl)phenyl]-1H-pyrazolo[3,4-d]-pyrimidin-4-yl}piperidin-4-ol (80 mg) and trans-3-(methylsulfonyl)cyclobutanamine hydrochloride (56 mg) in NMP (1.5 ml) and the mixture was stirred for 17 h at 130°C. Standard workup and purification by HPLC (method HT_PREP _acid) afforded 70 mg of the title compound.
LC-MS (method HTpost_acid): Rₜ = 1.07 min; MS (ESIpos): m/z = 511 [M+H]⁺
¹H-NMR (400 MHz, DMSO-d6) δ [ppm]: 1.399 (0.71), 1.421 (1.72), 1.432 (1.37), 1.444 (1.80), 1.465 (0.80), 1.853 (1.64), 1.876 (1.43), 2.332 (0.56), 2.438 (1.14), 2.455 (1.32), 2.462 (1.98), 2.468 (1.61), 2.473 (2.15), 2.479 (1.93), 2.523 (2.22), 2.528 (1.41), 2.669 (0.63), 2.674 (0.88), 2.678 (0.83), 2.720 (1.49), 2.937 (16.00), 3.484 (1.41), 3.509 (2.33), 3.534 (1.45), 3.800 (0.58), 3.811 (0.95), 3.820 (1.30), 3.830 (1.31), 3.840 (1.13), 3.851 (0.92), 3.869 (0.91), 3.881 (1.05), 3.892 (0.83), 4.238 (1.36), 4.495 (0.61), 4.819 (4.93), 4.829 (4.76), 7.458 (0.76), 7.618 (1.93), 7.637 (2.72), 7.716 (2.06), 7.736 (3.27), 7.756 (1.48), 8.344 (11.15), 8.545 (0.58), 8.747 (0.52).

### Example I-94

### (3S,4S)-4-fluoro-1-(6-{[(1r,3S)-3-(methylsulfonyl)cyclobutyl]amino}-1-[3-(trifluoromethyl)phenyl]-1H-pyrazolo[3,4-d]pyrimidin-4-yl)pyrrolidin-3-ol

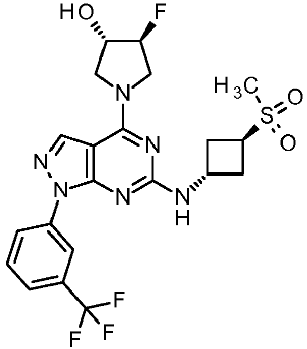

DIPEA (0.16 ml) was added to (3S,4S)-1-{6-chloro-1-[3-(trifluoromethyl)phenyl]-1H-pyrazolo-[3,4-d]pyrimidin-4-yl}-4-fluoropyrrolidin-3-ol (76 mg, crude batch) and trans-3-(methylsulfonyl)cyclobutanamine hydrochloride (42 mg) in NMP (1.5 ml) and the mixture was stirred 15.5 h at 130°C. Standard workup and purification by HPLC (method HT_PREP _acid) afforded 56 mg of the title compound.
LC-MS (method HTpost_acid): Rₜ = 1.08 min; MS (ESIpos): m/z = 515 [M+H]⁺
¹H-NMR (400 MHz, DMSO-d6) δ [ppm]: 2.543 (2.72), 2.733 (3.16), 2.942 (16.00), 3.705 (0.67), 3.766 (1.47), 3.794 (1.84), 3.849 (1.90), 3.860 (1.83), 3.883 (2.90), 3.895 (2.07), 3.969 (0.91), 4.002 (1.59), 4.035 (1.53), 4.069 (1.40), 4.172 (0.55), 4.370 (0.90), 4.488 (1.51), 5.056 (0.81), 5.137 (0.92), 5.183 (0.81), 5.264 (0.91), 5.667 (0.95), 5.773 (1.16), 7.553 (0.93), 7.618 (3.14), 7.638 (4.39), 7.721 (3.37), 7.741 (5.42), 7.762 (2.50), 8.265 (14.86), 8.557 (0.91), 8.620 (0.85), 8.775 (0.91).

### Example I-95

### (3S,4S)-1-(6-{[(1r,3S)-3-(ethylsulfonyl)cyclobutyl]amino}-1-[3-(trifluoromethyl)phenyl]-1H-pyrazolo[3,4-d]pyrimidin-4-yl)-4-fluoropyrrolidin-3-ol

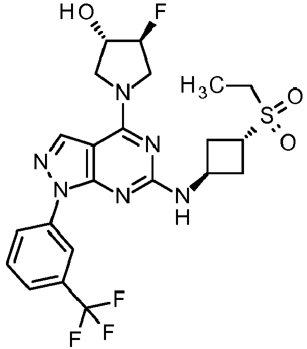

DIPEA (0.16 ml) was added to (3S,4S)-1-{6-chloro-1-[3-(trifluoromethyl)phenyl]-1H-pyrazolo-[3,4-d]pyrimidin-4-yl}-4-fluoropyrrolidin-3-ol (76 mg, crude batch) and trans-3-(ethylsulfonyl)-cyclobutanamine hydrochloride (44.8 mg) in NMP (1.5 ml) and the mixture was stirred 15.5 h at 130°C. Standard workup and purification by HPLC (method HT_PREP _acid) afforded 62 mg of the title compound.
LC-MS (method HTpost_acid): Rₜ = 1.12 min; MS (ESIpos): m/z = 529 [M+H]⁺
¹H-NMR (400 MHz, DMSO-d6) δ [ppm]: 1.197 (7.27), 1.215 (16.00), 1.234 (7.52), 2.522 (3.18), 2.526 (2.16), 2.543 (1.14), 2.739 (1.47), 3.049 (1.92), 3.066 (1.89), 3.767 (0.74), 3.794 (0.92), 3.847 (0.71), 3.880 (0.64), 3.928 (1.26), 3.965 (0.63), 4.004 (0.77), 4.035 (0.72), 4.068 (0.68), 4.377 (0.45), 4.482 (0.87), 5.136 (0.46), 5.263 (0.44), 5.665 (0.55), 5.773 (0.67), 7.555 (0.49), 7.618 (1.62), 7.638 (2.20), 7.720 (1.73), 7.741 (2.71), 7.760 (1.24), 8.265 (7.38), 8.543 (0.45), 8.784 (0.45).

### Example I-96

### (3S,4S)-4-fluoro-1-(6-{[(1r,3S)-3-hydroxycyclobutyl]amino}-1-[3-(trifluoromethyl)-phenyl]-1H-pyrazolo[3,4-d]pyrimidin-4-yl)pyrrolidin-3-ol

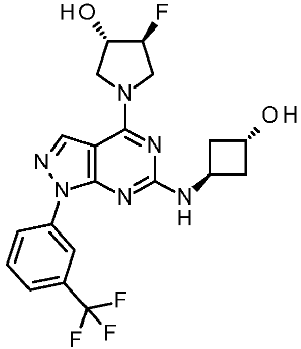

DIPEA (0.16 ml) was added to (3S,4S)-1-{6-chloro-1-[3-(trifluoromethyl)phenyl]-1H-pyrazolo[3,4-d]pyrimidin-4-yl}-4-fluoropyrrolidin-3-ol (76 mg, crude batch) and trans-3-amino-cyclobutanol hydrochloride (28 mg) in NMP (1.5 ml) and the mixture was stirred 15.5 h at 130°C. Standard workup and purification by HPLC (method HT_PREP _acid) afforded 61 mg of the title compound.
LC-MS (method HTpost_acid): Rₜ = 1.05 min; MS (ESIpos): m/z = 453 [M+H]⁺
¹H-NMR (400 MHz, DMSO-d6) δ [ppm]: 2.154 (1.84), 2.185 (4.62), 2.199 (4.45), 2.296 (3.71), 2.322 (2.77), 2.327 (3.06), 2.331 (3.18), 2.336 (2.41), 2.522 (6.92), 2.527 (4.34), 2.543 (2.64), 2.669 (1.22), 2.673 (1.64), 2.678 (1.19), 3.687 (0.91), 3.756 (1.85), 3.783 (2.31), 3.852 (1.96), 3.883 (1.66), 3.930 (0.90), 3.994 (2.07), 4.026 (1.93), 4.054 (1.79), 4.091 (0.48), 4.160 (0.72), 4.188 (0.52), 4.261 (1.19), 4.333 (2.89), 4.469 (1.56), 4.983 (2.91), 5.042 (1.29), 5.129 (1.12), 5.168 (1.21), 5.257 (1.07), 5.644 (1.19), 5.757 (1.32), 7.361 (1.87), 7.602 (4.60), 7.622 (6.01), 7.725 (3.11), 7.745 (5.12), 7.765 (2.43), 8.205 (1.18), 8.240 (16.00), 8.499 (1.32), 8.989 (1.57).

### Example I-97

### (3S,4S)-4-fluoro-1-(6-{[(1s,3R)-3-hydroxycyclobutyl]amino}-1-[3-(trifluoromethyl)-phenyl]-1H-pyrazolo[3,4-d]pyrimidin-4-yl)pyrrolidin-3-ol

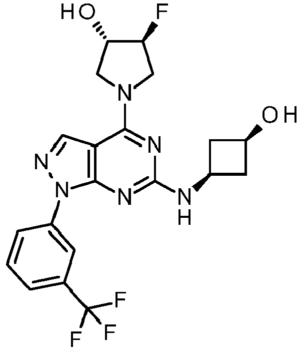

DIPEA (0.16 ml) was added to (3S,4S)-1-{6-chloro-1-[3-(trifluoromethyl)phenyl]-1H-pyrazolo-[3,4-d]pyrimidin-4-yl}-4-fluoropyrrolidin-3-ol (76 mg, crude batch) and cis-3-aminocyclobutanol hydrochloride (28 mg) in NMP (1.5 ml) and the mixture was stirred 15.5 h at 130°C. Standard workup and purification by HPLC (method HT_PREP _acid) afforded 60 mg of the title compound.
LC-MS (method HTpost_acid): Rₜ = 1.06 min; MS (ESIpos): m/z = 453 [M+H]⁺
¹H-NMR (400 MHz, DMSO-d6) δ [ppm]: 1.816 (2.06), 1.838 (5.06), 1.864 (5.29), 1.887 (2.26), 2.327 (1.42), 2.331 (1.92), 2.336 (1.41), 2.457 (1.01), 2.526 (6.18), 2.543 (2.36), 2.626 (4.23), 2.664 (2.52), 2.668 (2.87), 2.673 (2.85), 2.678 (1.97), 3.680 (0.86), 3.753 (2.06), 3.782 (2.99), 3.847 (4.97), 3.863 (4.38), 3.954 (1.21), 3.988 (2.14), 4.022 (2.10), 4.052 (1.97), 4.084 (0.51), 4.156 (0.71), 4.185 (0.47), 4.355 (1.28), 4.466 (1.39), 5.039 (2.21), 5.066 (2.42), 5.126 (1.29), 5.163 (1.38), 5.252 (1.15), 5.639 (1.44), 5.759 (1.56), 7.354 (1.73), 7.603 (4.57), 7.622 (5.88), 7.731 (2.33), 7.751 (3.63), 7.771 (1.79), 8.218 (1.38), 8.235 (16.00), 8.475 (1.44), 8.618 (0.59), 9.077 (1.79).

### Example I-98

### (3S,4S)-1-{6-[(4S,6s)-1-azaspiro[3.3]heptan-6-ylamino]-1-[3-(trifluoromethyl)phenyl]-1H-pyrazolo[3,4-d]pyrimidin-4-yl}-4-fluoropyrrolidin-3-ol

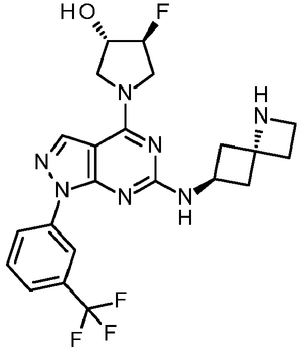

DIPEA (0.16 ml) was added to (3S,4S)-1-{6-chloro-1-[3-(trifluoromethyl)phenyl]-1H-pyrazolo-[3,4-d]pyrimidin-4-yl}-4-fluoropyrrolidin-3-ol (76 mg) and tert-butyl (4r,6s)-6-amino-1-azaspiro[3.3]heptane-1-carboxylate (48 mg) in NMP (1.5 ml) and the mixture was stirred for 15 h at 130°C. Standard workup and evaporation affo rded afforded a residue (86 mg) which was dissolved in DCM (1.5 ml). TFA (0.12 ml) was added and the mixture was stirred at RT for 16.5h. Again, TFA (0.5 ml) was added and stirring was continued for 22 h at 40°C. Purification by HPLC (method HT_PREP _bas) afforded 49 mg of the title compound.
LC-MS (method HTpost_bas): Rₜ = 1.46 min; MS (ESIpos): m/z = 478 [M+H]⁺
¹H-NMR (400 MHz, DMSO-d6) δ [ppm]: 2.230 (8.80), 2.247 (7.65), 2.473 (4.94), 2.522 (12.84), 2.526 (8.74), 2.543 (6.61), 2.990 (0.52), 3.265 (9.01), 3.283 (15.94), 3.301 (11.99), 3.666 (1.93), 3.753 (2.16), 3.781 (2.64), 3.849 (1.75), 3.878 (1.59), 3.953 (1.30), 3.986 (2.28), 4.021 (2.24), 4.051 (2.12), 4.089 (0.69), 4.157 (1.90), 4.347 (1.46), 4.466 (1.50), 5.037 (1.27), 5.127 (1.21), 5.163 (1.32), 5.254 (1.19), 5.648 (0.82), 5.767 (0.80), 7.315 (1.56), 7.603 (4.14), 7.622 (5.29), 7.731 (2.29), 7.750 (3.40), 7.768 (1.73), 8.237 (16.00), 8.477 (0.81), 8.531 (1.92), 8.599 (1.08), 8.952 (1.35).

### Example I-99

### 4-(3-fluoroazetidin-1-yl)-N-[trans-3-(methylsulfonyl)cyclobutyl]-1-[3-(trifluoromethyl)-phenyl]-1H-pyrazolo[3,4-d]pyrimidin-6-amine

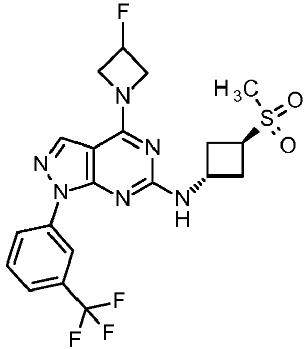

DIPEA (0.21 ml) was added to 6-chloro-4-(3-fluoroazetidin-1-yl)-1-[3-(trifluoromethyl)phenyl]-1H-pyrazolo[3,4-d]pyrimidine (80 mg) and trans-3-(methylsulfonyl)cyclobutanamine hydrochloride (56 mg) and the mixture was stirred for 20 h at 130°C. Standard workup and purification by HPLC (method HT_PREP _acid) afforded 67 mg of the title compound.
LC-MS (method HTpost_acid): Rₜ = 1.17 min; MS (ESIpos): m/z = 485 [M+H]⁺
¹H-NMR (400 MHz, DMSO-d6) δ [ppm]: 2.079 (0.51), 2.332 (0.46), 2.545 (3.40), 2.674 (0.74), 2.679 (0.71), 2.727 (2.05), 2.932 (16.00), 3.855 (0.83), 3.867 (1.20), 3.878 (1.44), 3.890 (1.13), 3.902 (0.77), 4.489 (1.28), 4.620 (0.83), 5.521 (0.71), 5.528 (0.98), 5.535 (1.16), 5.542 (0.93), 5.671 (0.96), 5.678 (1.15), 5.685 (0.96), 5.693 (0.67), 7.617 (2.40), 7.637 (3.34), 7.719 (3.22), 7.738 (4.48), 7.758 (1.92), 8.116 (13.77), 8.519 (0.92), 8.754 (1.12).

### Example I-100

### 1-{6-[(trans-3-hydroxycyclobutyl)amino]-1-[3-(trif)uoromethyl)phenyl]-1H-pyrazolo[3,4-d]pyrimidin-4-yl}piperidin-4-ol

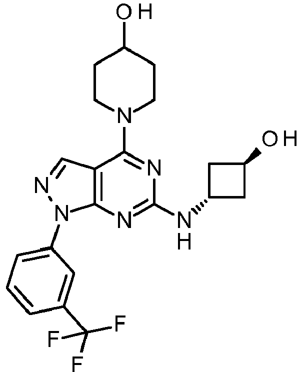

DIPEA (0.32 ml) was added to 1-{6-chloro-1-[3-(trifluoromethyl)phenyl]-1H-pyrazolo[3,4-d]-pyrimidin-4-yl}piperidin-4-ol (123 mg) and trans-3-aminocyclobutanol hydrochloride (57 mg) in NMP (1.5 ml) and the mixture was stirred for 20 h at 130°C. Standard workup and purification by HPLC (method HT_PREP _acid) afforded 80 mg of the title compound.
LC-MS (method HTpost_acid): Rₜ = 1.06 min; MS (ESIpos): m/z = 449 [M+H]⁺
¹H-NMR (400 MHz, DMSO-d6) δ [ppm]: 1.413 (3.92), 1.425 (3.27), 1.436 (4.11), 1.458 (1.89), 1.844 (4.00), 1.868 (3.51), 2.079 (3.23), 2.176 (4.04), 2.278 (3.96), 2.331 (1.72), 2.544 (2.20), 2.673 (1.32), 3.464 (3.06), 3.490 (5.01), 3.515 (3.15), 3.801 (2.13), 3.811 (2.79), 3.821 (2.76), 3.831 (2.08), 4.242 (4.52), 4.311 (2.86), 4.810 (8.73), 4.821 (8.60), 4.982 (5.30), 4.995 (4.76), 7.278 (2.36), 7.601 (4.20), 7.620 (5.57), 7.719 (3.04), 7.739 (4.96), 7.758 (2.35), 8.319 (16.00), 8.482 (1.36), 8.968 (1.51).

### Example I-101

### (3R,4R)-1-(6-{[(1r,3R)-3-hydroxycyclobutyl]amino}-1-[3-(trifluoromethyl)phenyl]-1H-pyrazolo[3,4-d]pyrimidin-4-yl)pyrrolidine-3,4-diol

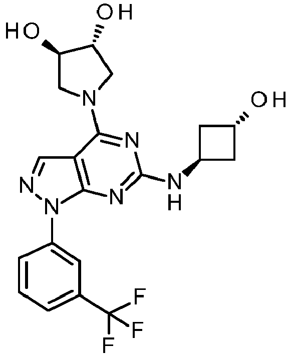

A solution of (3R,4R)-1-{6-chloro-1-[3-(trifluoromethyl)phenyl]-1H-pyrazolo[3,4-d]pyrimidin-4-yl}pyrrolidine-3,4-diol (80 mg) and trans-3-aminocyclobutanol hydrochloride (49.5 mg) in NMP (2.0 ml) was treated with DIPEA (0.21 ml) and the mixture was stirred at 130°C for 40 h. Standard workup and purification by HPLC (method HT_PREP _acid) afforded 34 mg of the title compound.
LC-MS (method HTpost_acid): Rₜ = 0.86 min; MS (ESIpos): m/z = 451 [M+H]⁺
¹H-NMR (400 MHz, DMSO-d6) δ [ppm]: 2.149 (0.68), 2.180 (1.72), 2.193 (1.60), 2.294 (1.34), 2.322 (0.91), 2.326 (1.07), 2.331 (1.12), 2.336 (0.78), 2.522 (2.42), 2.526 (1.56), 2.668 (0.50), 2.673 (0.69), 2.678 (0.52), 3.593 (1.96), 3.621 (2.17), 3.688 (2.54), 3.719 (0.63), 3.945 (1.06), 3.954 (1.29), 3.971 (1.07), 3.982 (1.01), 4.034 (1.98), 4.159 (1.89), 4.308 (0.94), 4.323 (1.12), 7.274 (0.61), 7.592 (1.71), 7.612 (2.26), 7.718 (1.18), 7.738 (1.98), 7.758 (0.94), 8.147 (16.00), 8.212 (10.71), 8.505 (0.44), 9.001 (0.48).

### Example I-102

### (3R)-1-(6-{[(1r,3R)-3-hydroxycyclobutyl]amino}-1-[3-(trifluoromethyl)phenyl]-1H-pyrazolo[3,4-d]pyrimidin-4-yl)-3-methylpyrrolidin-3-ol

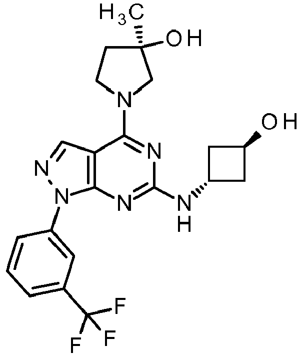

DIPEA (0.18 ml) was added to (3R)-1-{6-chloro-1-[3-(trifluoromethyl)phenyl]-1H-pyrazolo[3,4-d]pyrimidin-4-yl}-3-methylpyrrolidin-3-ol (67.5 mg) and trans-3-aminocyclobutanol hydrochloride (31.5 mg) in NMP (1.5 ml) and the mixture was stirred for 16 h at 130°C Standard workup and purification by HPLC (method HT_PREP _acid) afforded 45 mg of the title compound.
LC-MS (method HTpost_acid): Rₜ = 1.07 min; MS (ESIpos): m/z = 449 [M+H]⁺
¹H-NMR (400 MHz, DMSO-d6) δ [ppm]: 1.371 (14.18), 1.418 (16.00), 1.856 (0.99), 1.881 (1.67), 1.902 (1.94), 1.997 (1.42), 2.019 (3.80), 2.037 (2.80), 2.143 (1.94), 2.176 (4.85), 2.188 (4.61), 2.284 (3.92), 2.321 (2.21), 2.326 (2.65), 2.330 (2.89), 2.335 (2.03), 2.521 (6.68), 2.526 (4.45), 2.543 (2.52), 2.663 (0.69), 2.668 (1.59), 2.673 (2.09), 2.677 (1.53), 2.681 (0.69), 3.369 (2.01), 3.399 (1.92), 3.658 (8.92), 3.712 (2.31), 3.742 (1.85), 3.793 (1.12), 3.849 (2.93), 3.868 (3.49), 3.888 (1.59), 4.309 (2.91), 4.323 (2.68), 4.897 (6.78), 4.964 (7.81), 4.981 (4.33), 7.224 (1.64), 7.589 (5.09), 7.608 (6.73), 7.714 (3.63), 7.734 (6.04), 7.754 (2.89), 8.148 (7.17), 8.161 (1.49), 8.189 (10.03), 8.501 (1.22), 8.998 (1.33).

### Example I-103

### (3R)-3-methyl-1-(6-{[(1r,3R)-3-(methylsulfonyl)cyclobutyl]amino}-1-[3-(trifluoromethyl)-phenyl]-1H-pyrazolo[3,4-d]pyrimidin-4-yl)pyrrolidin-3-ol

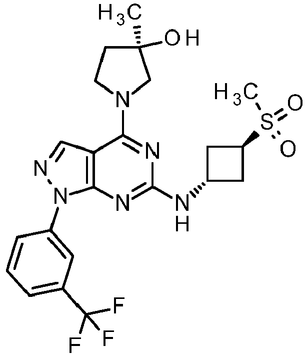

DIPEA (0.18 ml) was added to (3R)-1-{6-chloro-1-[3-(trifluoromethyl)phenyl]-1H-pyrazolo[3,4-d]pyrimidin-4-yl}-3-methylpyrrolidin-3-ol (67.5 mg) and trans-3-(methylsulfonyl)cyclobutan-amine hydrochloride (47.3 mg) in NMP (1.5 ml) and the mixture was stirred for 16 h at 130°C Standard workup and purification by HPLC (method HT_PREP _acid) afforded 43 mg of the title compound.
LC-MS (method HTpost_acid): Rₜ = 1.13 min; MS (ESIpos): m/z = 511 [M+H]⁺
¹H-NMR (400 MHz, DMSO-d6) δ [ppm]: 1.377 (6.54), 1.422 (8.66), 1.868 (0.45), 1.894 (0.77), 1.910 (0.96), 2.003 (0.71), 2.025 (1.86), 2.044 (1.39), 2.325 (0.79), 2.330 (1.06), 2.335 (0.76), 2.521 (5.22), 2.526 (3.53), 2.543 (0.94), 2.667 (0.92), 2.672 (1.27), 2.677 (1.11), 2.681 (0.94), 2.721 (1.87), 2.937 (16.00), 3.376 (0.86), 3.406 (0.92), 3.667 (4.43), 3.708 (1.19), 3.738 (0.82), 3.795 (0.56), 3.860 (2.12), 3.875 (2.73), 3.897 (1.38), 4.518 (0.64), 4.915 (2.72), 4.971 (2.86), 7.416 (0.53), 7.605 (2.23), 7.624 (3.08), 7.711 (2.42), 7.731 (3.85), 7.751 (1.77), 8.175 (4.30), 8.213 (5.23), 8.563 (0.57), 8.775 (0.50).

### Example I-104

### trans-3-({4-[(4RS)-4-amino-3,3-difluoropiperidin-1-yl]-1-[3-(trifluoromethyl)phenyl]-1H-pyrazolo[3,4-d]pyrimidin-6-yl}amino)cyclobutanol

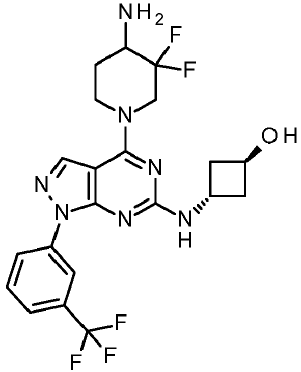

DIPEA (0.15 ml) was added to tert-butyl (1-{6-chloro-1-[3-(trifluoromethyl)phenyl]-1H-pyrazolo[3,4-d]pyrimidin-4-yl}-3,3-difluoropiperidin-4-yl)carbamate (79 mg) and trans-3-aminocyclobutanol hydrochloride (26 mg) in NMP (1.5 m) and the mixture was stirred for 19.5 at 130°C. Standard workup afforded a residue, which was dissolved in DCM (2.0 ml). TFA (0.5 ml) was added and the mixture was stirred at RT for 16.5 h. Evaporation and purification by HPLC (method HT_PREP _bas) afforded 39 mg of the title compound..
LC-MS (method HTpost_bas): Rₜ = 1.20 min; MS (ESIpos): m/z = 484 [M+H]⁺
¹H-NMR (400 MHz, DMSO-d6) δ [ppm]: 1.579 (1.22), 1.607 (1.28), 1.882 (2.41), 1.923 (1.94), 1.947 (1.45), 2.087 (0.41), 2.185 (2.85), 2.199 (2.83), 2.285 (2.63), 2.321 (1.57), 2.325 (1.98), 2.331 (2.16), 2.335 (1.51), 2.339 (0.70), 2.521 (5.61), 2.526 (3.83), 2.543 (2.07), 2.663 (0.54), 2.668 (1.19), 2.672 (1.70), 2.677 (1.21), 2.681 (0.52), 3.143 (0.42), 3.173 (0.53), 3.186 (0.91), 3.198 (0.98), 3.210 (0.98), 3.230 (1.04), 3.244 (0.99), 3.255 (0.89), 3.572 (1.02), 3.598 (1.70), 3.622 (1.08), 3.796 (0.91), 3.825 (1.01), 3.850 (1.00), 3.885 (1.02), 4.256 (1.72), 4.303 (1.86), 4.317 (1.85), 4.655 (1.00), 4.673 (0.96), 4.999 (5.95), 5.012 (5.29), 7.443 (1.43), 7.616 (3.40), 7.635 (4.60), 7.728 (2.35), 7.748 (3.88), 7.768 (1.84), 8.399 (16.00), 8.480 (1.08), 8.946 (1.49).

### Example I-105

### 4-[(4RS)-4-amino-3,3-difluoropiperidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine

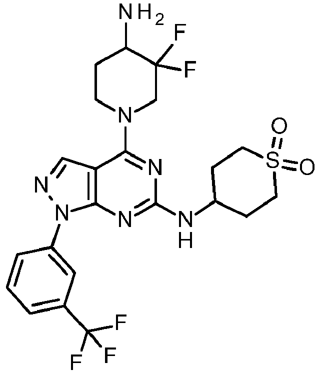

DIPEA (0.15 ml) was added to tert-butyl (1-{6-chloro-1-[3-(trifluoromethyl)phenyl]-1H-pyrazolo[3,4-d]pyrimidin-4-yl}-3,3-difluoropiperidin-4-yl)carbamate (79 mg) and tetrahydro-2H-thiopyran-4-amine 1,1-dioxide (31 mg) in NMP (1.5 m) and the mixture was stirred for 19.5 at 130°C. Standard workup afforded a residue, which was dissolved in DCM (2.0 ml). TFA (0.5 ml) was added and the mixture was stirred at RT for 16.5 h. Evaporation and purification by HPLC (method HT_PREP _bas) afforded 47 mg of the title compound.
LC-MS (method HTpost_bas): Rt = 1.23 min; MS (ESIpos): m/z = 546 [M+H]⁺
¹H-NMR (400 MHz, DMSO-d6) δ [ppm]: 1.580 (1.25), 1.608 (1.38), 1.834 (3.97), 1.928 (1.57), 1.951 (1.34), 1.962 (1.36), 2.087 (2.43), 2.098 (2.40), 2.242 (2.21), 2.334 (1.41), 2.339 (0.68), 2.521 (6.51), 2.526 (4.14), 2.542 (1.46), 3.203 (5.44), 3.587 (1.05), 3.614 (1.76), 3.639 (1.14), 3.812 (1.03), 3.841 (1.13), 3.868 (1.10), 3.902 (1.12), 4.107 (0.92), 4.271 (1.06), 4.672 (0.96), 7.376 (1.05), 7.638 (2.71), 7.658 (3.45), 7.782 (1.65), 8.426 (16.00), 8.563 (1.32), 8.737 (1.06).

### Example I-106

### N-(1,1-dioxidotetrahydro-2H-thiopyran-4-yl)-4-[3-methyl-3-(morpholin-4-yl)azetidin-1-yl]-1-[3-(trifluoromethyl)phenyl]-1H-pyrazolo[3,4-d]pyrimidin-6-amine

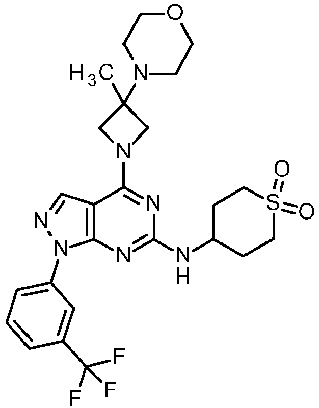

A mixture of 6-chloro-4-[3-methyl-3-(morpholin-4-yl)azetidin-1-yl]-1-[3-(trifluoromethyl)phenyl]-1 H-pyrazolo[3,4-d]pyrimidine (80 mg) and tetrahydro-2H-thiopyran-4-amine 1,1-dioxide (39.5 mg) in NMP (1.5 ml) was treated with DIPEA (0.18 ml) and the mixture was stirred for 42 h at 120°C. Standard workup and purification by HPLC (method HT_PREP _bas) afforded 67 mg of the title compound.
LC-MS (method HTpost_bas): Rₜ = 1.20 min; MS (ESIpos): m/z = 566 [M+H]⁺
¹H-NMR (400 MHz, DMSO-d6) δ [ppm]: 1.331 (16.00), 1.352 (0.66), 2.074 (1.34), 2.094 (1.34), 2.113 (1.07), 2.131 (0.53), 2.223 (1.40), 2.451 (5.57), 2.518 (4.04), 2.522 (2.79), 2.539 (1.63), 3.194 (4.23), 3.617 (5.60), 3.794 (0.73), 3.815 (0.85), 3.965 (0.83), 3.985 (0.72), 4.052 (0.99), 4.072 (1.30), 4.233 (1.01), 4.254 (0.85), 7.354 (0.46), 7.616 (1.45), 7.636 (1.86), 7.750 (0.68), 7.770 (1.07), 7.789 (0.57), 8.075 (11.76), 8.578 (0.64), 8.745 (0.50).

### Example 1-107

### (3R,4R)-1-(6-1[(1s,3S)-3-hydroxy-3-methylcyclobutyl]amino}-1-[3-(trifluoromethyl)-phenyl]-1H-pyrazolo[3,4-d]pyrimidin-4-yl)pyrrolidine-3,4-diol

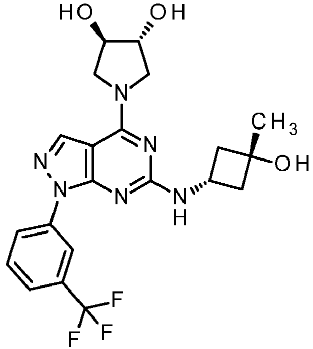

A solution of (3R,4R)-1-{6-chloro-1-[3-(trifluoromethyl)phenyl]-1H-pyrazolo[3,4-d]pyrimidin-4-yl}pyrrolidine-3,4-diol (55 mg) and cis-3-amino-1-methylcyclobutanol hydrochloride (CAS number 1523606-23-6) (38 mg) in NMP (2.0 ml) was treated with DIPEA (0.14 ml) and the mixture was stirred at 130°C for 16 h. Standard wor kup and purification by HPLC (method HT_PREP _acid) afforded 29 mg of the title compound.
LC-MS (method HTpost_acid): Rₜ = 0.97 min; MS (ESIpos): m/z = 465 [M+H]⁺
¹H-NMR (400 MHz, DMSO-d6) δ [ppm]: 1.283 (16.00), 2.008 (1.67), 2.035 (2.88), 2.058 (1.96), 2.322 (0.44), 2.326 (0.94), 2.331 (1.36), 2.335 (1.16), 2.340 (0.95), 2.351 (1.56), 2.369 (2.28), 2.395 (1.27), 2.522 (4.12), 2.526 (2.87), 2.668 (0.86), 2.672 (1.22), 2.677 (0.84), 3.592 (2.35), 3.619 (2.82), 3.675 (2.40), 3.887 (0.82), 3.906 (0.87), 3.943 (1.52), 3.952 (1.74), 3.969 (1.37), 3.980 (1.26), 4.032 (2.22), 4.156 (2.28), 4.922 (0.62), 5.224 (0.45), 5.329 (0.48), 7.253 (0.96), 7.594 (1.90), 7.614 (2.52), 7.716 (1.49), 7.736 (2.45), 7.756 (1.16), 8.172 (3.35), 8.210 (12.02), 8.372 (0.79), 8.390 (0.78), 9.212 (1.24).

### Example 1-108

### (3R,4R)-1-(6-{[(1r,3R)-3-hydroxy-3-methylcyclobutyl]amino}-1-[3-(trifluoromethyl)-phenyl]-1H-pyrazolo[3,4-d]pyrimidin-4-yl)pyrrolidine-3,4-diol

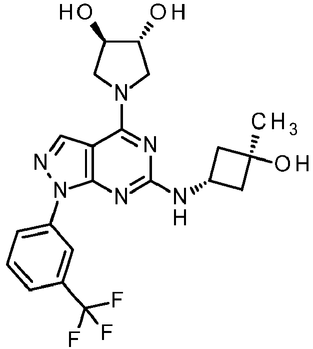

A solution of (3R,4R)-1-{6-chloro-1-[3-(trifluoromethyl)phenyl]-1H-pyrazolo[3,4-d]pyrimidin-4-yl}pyrrolidine-3,4-diol (55 mg) and trans-3-amino-1-methylcyclobutanol hydrochloride (CAS number 1523571-03-0) (38 mg), in NMP (2.0 ml) was treated with DIPEA (0.14 ml) and the mixture was stirred at 130°C for 16 h. Standard wor kup and purification by HPLC (method HT_PREP _acid) afforded 31 mg of the title compound.
LC-MS (method HTpost_acid): Rₜ = 0.93 min; MS (ESIpos): m/z = 465 [M+H]⁺
¹H-NMR (400 MHz, DMSO-d6) δ [ppm]: 1.281 (16.00), 2.007 (1.04), 2.022 (1.40), 2.326 (1.21), 2.331 (1.59), 2.335 (1.46), 2.340 (1.19), 2.358 (1.09), 2.521 (3.09), 2.526 (2.13), 2.543 (0.48), 2.668 (0.61), 2.672 (0.85), 2.677 (0.61), 3.591 (1.69), 3.618 (1.96), 3.688 (1.40), 3.942 (0.97), 3.952 (1.21), 3.969 (0.99), 3.980 (0.93), 4.034 (1.64), 4.161 (1.61), 4.753 (1.40), 5.223 (0.77), 5.331 (0.84), 7.218 (0.49), 7.593 (1.46), 7.613 (1.96), 7.715 (1.05), 7.735 (1.75), 7.755 (0.85), 8.207 (9.50), 8.271 (0.78), 8.569 (0.44), 8.913 (0.43).

### Example 1-109

### 1-(1-{6-[(cis-3-hydroxycyclobutyl)amino]-1-[3-(trifluoromethyl)phenyl]-1H-pyrazolo[3,4-d]pyrimidin-4-yl}azetidin-3-yl)-4-methylpiperidin-4-ol

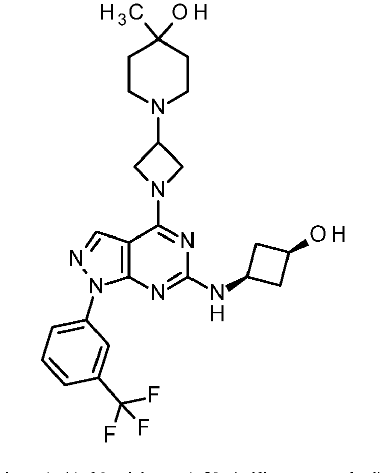

DIPEA (0.22 ml) was added to 1-(1-{6-chloro-1-[3-(trifluoromethyl)phenyl]-1H-pyrazolo[3,4-d]-pyrimidin-4-yl}azetidin-3-yl)-4-methylpiperidin-4-ol (100 mg) and cis-3-aminocyclobutanol hydrochloride (53 mg) in NMP (2.0 ml) and the mixture was stirred at 130°C for 16 h. Standard workup and purification by HPLC (method HT_PREP _bas) afforded 70 mg oft he title compound.
LC-MS (method HTpost_bas): Rₜ = 1.10 min; MS (ESIpos): m/z = 518 [M+H]⁺
¹H-NMR (400 MHz, DMSO-d6) δ [ppm]: 1.115 (16.00), 1.449 (0.45), 1.471 (1.85), 1.495 (4.66), 1.528 (0.54), 1.794 (0.82), 1.801 (0.63), 1.816 (1.83), 1.822 (1.77), 1.837 (1.77), 1.843 (1.91), 1.858 (0.66), 1.865 (0.87), 2.301 (1.31), 2.322 (1.21), 2.326 (1.49), 2.331 (1.62), 2.336 (1.15), 2.424 (1.28), 2.522 (2.98), 2.527 (2.09), 2.544 (0.72), 2.611 (1.35), 2.664 (0.45), 2.668 (0.79), 2.673 (1.05), 2.678 (0.75), 3.794 (0.67), 3.811 (0.93), 3.830 (1.27), 3.847 (1.19), 3.864 (0.72), 3.883 (0.43), 3.914 (0.54), 4.170 (5.58), 4.415 (0.59), 5.058 (1.04), 7.420 (0.62), 7.592 (1.56), 7.612 (1.99), 7.722 (0.86), 7.743 (1.40), 7.763 (0.66), 8.066 (7.42), 8.458 (0.55), 9.041 (0.79).

### Example 1-110

### 1-(1-{6-[(trans-3-hydroxy-3-methylcyclobutyl)amino]-1-[3-(trifluoromethyl)phenyl]-1H-pyrazolo[3,4-d]pyrimidin-4-yl}azetidin-3-yl)-4-methylpiperidin-4-ol

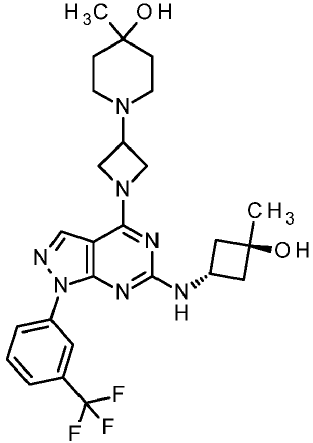

DIPEA (0.17 ml) was added to 1-(1-{6-chloro-1-[3-(trifluoromethyl)phenyl]-1H-pyrazolo[3,4-d]-pyrimidin-4-yl}azetidin-3-yl)-4-methylpiperidin-4-ol (80 mg) and trans-3-amino-1-methylcyclobutanol hydrochloride (CAS number 1523571-03-0) (34 mg) in NMP (1.5 ml) and the mixture was stirred at 130°C for 15 h. Standard workup and purification by HPLC (method HT_PREP _acid) afforded 53 mg of the title compound.
LC-MS (method HTpost_acid): Rₜ = 0.81 min; MS (ESIpos): m/z = 532 [M+H]⁺
¹H-NMR (400 MHz, DMSO-d6) δ [ppm]: 1.115 (12.94), 1.272 (16.00), 1.472 (1.50), 1.496 (3.91), 1.528 (0.47), 2.013 (1.16), 2.029 (1.15), 2.042 (1.04), 2.307 (1.42), 2.327 (1.72), 2.331 (1.87), 2.336 (1.64), 2.426 (1.09), 2.522 (2.30), 2.527 (1.35), 2.673 (0.47), 3.345 (4.10), 3.922 (0.43), 4.170 (1.40), 4.308 (0.43), 4.413 (0.52), 4.747 (0.71), 7.373 (0.50), 7.593 (1.24), 7.612 (1.63), 7.712 (0.94), 7.732 (1.53), 7.752 (0.72), 8.065 (6.46), 8.159 (1.98), 8.538 (0.49), 8.871 (0.59).

### Example 1-111

### 1-(1-{6-[(cis-3-hydroxy-3-methylcyclobutyl)amino]-1-[3-(trifluoromethyl)phenyl]-1H-pyrazolo[3,4-d]pyrimidin-4-yl}azetidin-3-yl)-4-methylpiperidin-4-ol

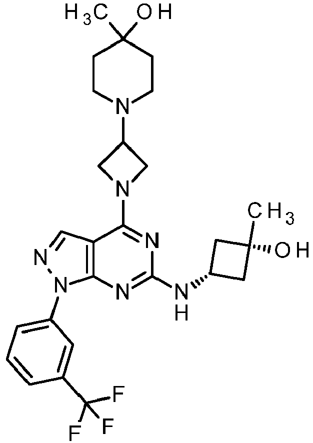

DIPEA (0.17 ml) was added to 1-(1-{6-chloro-1-[3-(trifluoromethyl)phenyl]-1H-pyrazolo[3,4-d]-pyrimidin-4-yl}azetidin-3-yl)-4-methylpiperidin-4-ol (80 mg) and cis-3-amino-1-methylcyclo-butanol (34 mg) in NMP (1.5 ml) and the mixture was stirred at 130°C for 15 h. Standard workup and purification by HPLC (method HT_PREP _acid) afforded 62 mg of the title compound.
LC-MS (method HTpost_acid): Rₜ = 0.84 min; MS (ESIpos): m/z = 532 [M+H]⁺
¹H-NMR (400 MHz, DMSO-d6) δ [ppm]: 1.115 (16.00), 1.274 (10.31), 1.450 (0.45), 1.471 (1.84), 1.496 (4.76), 1.529 (0.57), 1.992 (1.21), 2.014 (2.18), 2.019 (2.27), 2.041 (1.41), 2.303 (1.42), 2.327 (1.88), 2.331 (2.31), 2.336 (2.19), 2.355 (1.96), 2.374 (1.17), 2.425 (1.30), 2.522 (2.21), 2.527 (1.52), 2.669 (0.42), 2.673 (0.58), 2.678 (0.42), 3.332 (5.12), 3.861 (0.75), 3.878 (0.89), 3.895 (0.84), 4.169 (1.65), 4.413 (0.58), 4.918 (0.73), 7.411 (0.92), 7.424 (0.89), 7.591 (1.54), 7.610 (2.05), 7.710 (1.31), 7.730 (2.15), 7.750 (1.01), 8.065 (8.06), 8.154 (3.30), 8.346 (0.82), 8.367 (0.80), 9.164 (1.37).

### Example 1-112

### 1-(1-{6-[(cis-3-hydroxycyclobutyl)amino]-1-[3-(trifluoromethyl)phenyl]-1H-pyrazolo[3,4-d]pyrimidin-4-yl}azetidin-3-yl)pyrrolidin-2-one

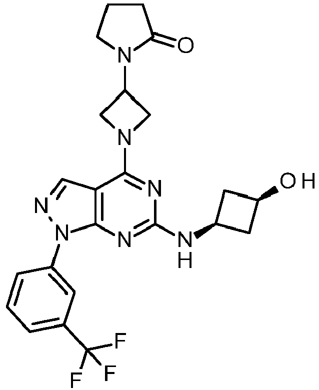

DIPEA (0.19 ml) was added to 1-(1-{6-chloro-1-[3-(trifluoromethyl)phenyl]-1H-pyrazolo[3,4-d]-pyrimidin-4-yl}azetidin-3-yl)pyrrolidin-2-one (80 mg) und cis-3-aminocyclobutanol hydrochloride (45 mg) in NMP (1.5 ml) and the mixture was stirred at 130°C for 16 h. Standard workup and purification by HPLC (method HT_PREP _acid) afforded 35 mg of the title compound.
LC-MS (method HTpost_acid): Rₜ = 1.05 min; MS (ESIpos): m/z = 488 [M+H]⁺
¹H-NMR (400 MHz, DMSO-d6) δ [ppm]: 1.797 (1.12), 1.803 (1.02), 1.818 (2.81), 1.824 (2.73), 1.840 (2.73), 1.845 (2.94), 1.860 (1.10), 1.867 (1.23), 1.946 (0.86), 1.966 (2.90), 1.984 (4.62), 2.004 (3.33), 2.021 (1.20), 2.078 (2.13), 2.269 (5.12), 2.289 (8.04), 2.309 (3.87), 2.321 (0.57), 2.326 (0.94), 2.331 (1.27), 2.335 (0.92), 2.521 (4.92), 2.526 (3.13), 2.615 (2.26), 2.663 (0.69), 2.668 (1.09), 2.672 (1.33), 2.677 (0.93), 3.575 (3.57), 3.592 (6.16), 3.609 (3.52), 3.799 (1.12), 3.816 (1.49), 3.834 (1.92), 3.850 (1.76), 3.867 (0.97), 4.363 (0.79), 4.519 (0.85), 5.059 (2.28), 5.073 (3.05), 5.088 (2.24), 5.103 (1.20), 5.122 (0.45), 7.498 (1.16), 7.600 (2.66), 7.619 (3.43), 7.729 (1.57), 7.749 (2.49), 7.769 (1.19), 8.054 (16.00), 8.443 (1.05), 8.461 (1.05), 9.041 (1.56).

### Example 1-113

### 1-(1-{6-[(trans-3-hydroxycyclobutyl)amino]-1-[3-(trifluoromethyl)phenyl]-1H-pyrazolo-[3,4-d]pyrimidin-4-yl}azetidin-3-yl)pyrrolidin-2-one

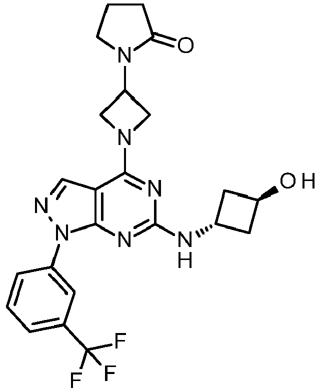

DIPEA (0.19 ml) was added to 1-(1-{6-chloro-1-[3-(trifluoromethyl)phenyl]-1H-pyrazolo[3,4-d]-pyrimidin-4-yl}azetidin-3-yl)pyrrolidin-2-one (80 mg) und trans-3-aminocyclobutanol hydrochloride (45 mg) in NMP (1.5 ml) and the mixture was stirred for 16.5 h at 130°C. Standard workup and purification by HPLC (method HT_PREP _acid) afforded 36 mg of the title compound.
LC-MS (method HTpost_acid): Rₜ = 1.04 min; MS (ESIpos): m/z = 488 [M+H]⁺
¹H-NMR (400 MHz, DMSO-d6) δ [ppm]: 1.948 (0.84), 1.967 (2.83), 1.986 (4.48), 2.005 (3.25), 2.022 (1.18), 2.170 (2.47), 2.184 (2.46), 2.270 (6.94), 2.291 (9.94), 2.311 (5.02), 2.326 (1.16), 2.331 (1.11), 2.336 (0.77), 2.522 (3.45), 2.527 (2.15), 2.669 (0.51), 2.673 (0.72), 2.678 (0.52), 3.578 (3.49), 3.596 (6.02), 3.613 (3.43), 4.241 (1.16), 4.274 (1.10), 4.289 (1.72), 4.304 (2.45), 4.319 (2.34), 4.334 (1.59), 4.517 (0.87), 4.977 (2.35), 4.989 (2.10), 5.058 (0.58), 5.077 (1.32), 5.092 (2.01), 5.107 (1.14), 5.112 (1.14), 5.126 (0.45), 7.509 (1.22), 7.598 (2.72), 7.618 (3.59), 7.722 (2.06), 7.742 (3.40), 7.762 (1.62), 8.057 (16.00), 8.481 (1.04), 8.963 (1.49).

### Example I-114

### 1-{6-[(cis-3-hydroxycyclobutyl)amino]-1-[3-(trifluoromethyl)phenyl]-1H-pyrazolo[3,4-d]pyrimidin-4-yl}-4-(pyrrolidin-1-ylmethyl)piperidin-4-ol

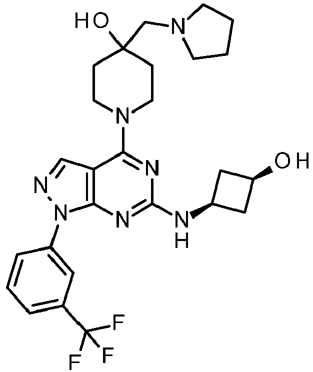

A solution of 1-{6-chloro-1-[3-(trifluoromethyl)phenyl]-1H-pyrazolo[3,4-d]pyrimidin-4-yl}-4-(pyrrolidin-1-ylmethyl)piperidin-4-ol (90 mg) and cis-3-aminocyclobutanol hydrochloride (46 mg) in NMP (2.0 ml) was treated with DIPEA (0.20 ml) and the mixture was stirred at 130°C for 16 h. Standard workup and purification by HPLC (method HT_PREP _bas) afforded 68 mg of the title compound
LC-MS (method HTpost_bas): Rₜ = 1.36 min; MS (ESIpos): m/z = 532 [M+H]⁺
¹H-NMR (400 MHz, DMSO-d6) δ [ppm]: 1.591 (7.57), 1.640 (14.61), 1.795 (1.58), 1.802 (1.40), 1.817 (3.64), 1.824 (3.51), 1.838 (3.55), 1.845 (3.78), 1.860 (1.43), 1.866 (1.64), 2.326 (1.47), 2.330 (2.02), 2.335 (1.45), 2.444 (15.49), 2.521 (7.28), 2.526 (4.93), 2.561 (13.19), 2.614 (2.71), 2.668 (1.87), 2.672 (2.24), 2.677 (1.64), 3.130 (0.60), 3.495 (1.49), 3.789 (1.32), 3.805 (1.69), 3.823 (2.07), 3.841 (2.67), 3.858 (2.49), 3.875 (1.30), 3.894 (0.42), 4.330 (4.15), 5.063 (1.69), 7.250 (1.25), 7.598 (3.23), 7.617 (4.26), 7.723 (1.74), 7.743 (2.85), 7.762 (1.43), 8.308 (0.53), 8.323 (16.00), 8.457 (0.93), 9.062 (1.13).

### Example I-115

### 4-(1-{6-[(cis-3-hydroxycyclobutyl)amino]-1-[3-(trifluoromethyl)phenyl]-1H-pyrazolo[3,4-d]pyrimidin-4-yl}azetidin-3-yl)piperazin-2-one

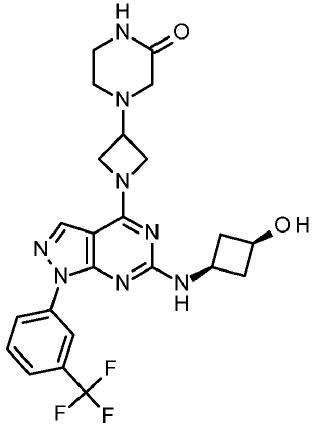

A solution of 4-(1-{6-chloro-1-[3-(trifluoromethyl)phenyl]-1H-pyrazolo[3,4-d]pyrimidin-4-yl}-azetidin-3-yl)piperazin-2-one (80 mg) and cis-3-aminocyclobutanol hydrochloride (44 mg) in NMP (2.0 ml) was treated with DIPEA (0.19 ml) and the mixture was stirred at 130°C for 16 h. Standard workup and purification by HPLC (method HT_PREP _acid) afforded 26 mg of the title compound.
LC-MS (method HTpost_acid): Rₜ = 0.96 min; MS (ESIpos): m/z = 503 [M+H]⁺
¹H-NMR (400 MHz, DMSO-d6) δ [ppm]: 1.797 (1.65), 1.804 (1.35), 1.819 (3.87), 1.825 (3.73), 1.840 (3.73), 1.847 (4.05), 1.861 (1.47), 1.868 (1.81), 2.326 (1.04), 2.331 (1.46), 2.336 (1.06), 2.522 (5.15), 2.526 (3.22), 2.543 (0.86), 2.611 (5.97), 2.664 (1.07), 2.668 (1.45), 2.672 (1.75), 2.678 (1.27), 3.010 (9.18), 3.205 (5.60), 3.451 (0.68), 3.469 (1.69), 3.481 (2.68), 3.493 (1.72), 3.511 (0.74), 3.813 (1.86), 3.832 (2.36), 3.847 (2.10), 3.864 (1.25), 3.988 (1.02), 4.203 (1.25), 4.303 (1.39), 4.435 (1.28), 5.057 (2.04), 7.448 (1.34), 7.595 (3.40), 7.614 (4.27), 7.724 (1.92), 7.745 (3.01), 7.764 (1.41), 7.829 (5.56), 8.056 (16.00), 8.209 (0.42), 8.462 (1.22), 9.041 (1.71).

### Example I-116

### cis-3-({4-[3-(2-hydroxypropan-2-yl)azetidin-1-yl]-1-[3-(trifluoromethyl)phenyl]-1H-pyrazolo[3,4-d]pyrimidin-6-yl}amino)cyclobutanol

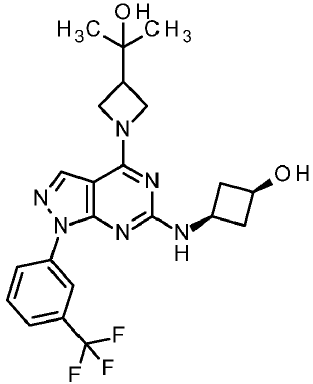

A solution of 2-(1-{6-chloro-1-[3-(trifluoromethyl)phenyl]-1H-pyrazolo[3,4-d]pyrimidin-4-yl}-azetidin-3-yl)propan-2-ol (80 mg) and cis-3-aminocyclobutanol hydrochloride (48 mg) in NMP (1.5 ml) was treated with DIPEA (0.20 ml) and the mixture was stirred at 130°C for 16 h. Standard workup and purification by HPLC (method HT_PREP _acid) afforded 17 mg of the title compound.
¹H-NMR (400 MHz, DMSO-d6) δ [ppm]: -0.008 (1.56), 0.008 (1.56), 1.100 (16.00), 1.795 (0.95), 1.817 (2.49), 1.843 (2.67), 1.865 (1.07), 2.326 (0.83), 2.331 (1.15), 2.335 (0.83), 2.521 (3.93), 2.526 (2.59), 2.543 (3.00), 2.608 (2.15), 2.663 (0.57), 2.668 (0.97), 2.673 (1.26), 2.677 (0.91), 2.681 (0.43), 2.763 (0.51), 2.782 (1.32), 2.799 (2.01), 2.818 (1.38), 2.836 (0.53), 3.804 (1.13), 3.821 (1.80), 3.840 (2.19), 3.858 (1.46), 3.876 (0.55), 4.051 (2.35), 4.268 (1.76), 4.284 (1.58), 4.298 (1.44), 4.319 (1.70), 4.339 (0.65), 4.613 (1.05), 5.057 (0.61), 7.377 (0.97), 7.589 (2.53), 7.608 (3.26), 7.721 (1.40), 7.741 (2.27), 7.761 (1.09), 8.070 (14.18), 8.152 (11.69), 8.461 (0.85), 9.050 (1.17).

### Example I-117

### (3R)-1-(6-{[(1s,3S)-3-hydroxycyclobutyl]amino}-1-[3-(trifluoromethyl)phenyl]-1H-pyrazolo[3,4-d]pyrimidin-4-yl)pyrrolidin-3-ol

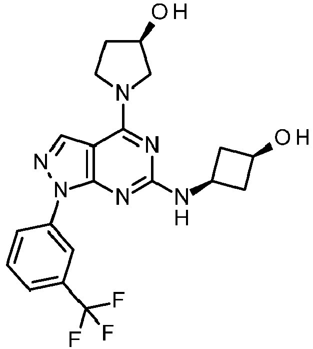

A solution of (3R)-1-{6-chloro-1-[3-(trifluoromethyl)phenyl]-1H-pyrazolo[3,4-d]pyrimidin-4-yl}-pyrrolidin-3-ol (80.0 mg) and cis-3-aminocyclobutanol hydrochloride (51.5 mg) in NMP (2.0 ml) was treated with DIPEA (0.22 ml) and the mixture was stirred at 130°C for 16 h. Standard workup and purification by HPLC (method HT_PREP _acid) afforded 59 mg of the title compound.
LC-MS (method HTpost_acid): Rₜ = 0.98 min; MS (ESIpos): m/z = 435 [M+H]⁺
¹H-NMR (400 MHz, DMSO-d6) δ [ppm]: -0.149 (0.43), 1.809 (5.01), 1.832 (12.35), 1.857 (13.27), 1.880 (6.71), 1.974 (2.77), 1.999 (3.81), 2.030 (3.76), 2.078 (9.48), 2.119 (2.90), 2.141 (2.43), 2.331 (3.11), 2.544 (3.69), 2.620 (10.08), 2.668 (4.30), 2.673 (4.23), 3.585 (5.91), 3.610 (7.61), 3.654 (5.62), 3.825 (15.14), 3.842 (16.00), 3.859 (12.82), 3.875 (8.77), 4.375 (5.11), 4.501 (4.04), 5.032 (9.21), 5.053 (7.03), 5.139 (5.42), 7.250 (3.43), 7.591 (10.37), 7.610 (13.55), 7.722 (5.66), 7.742 (9.04), 7.761 (4.62), 8.154 (10.55), 8.196 (13.56), 8.478 (3.05), 8.633 (1.36), 9.095 (3.57).

### Example I-118

### cis-3-({4-[3-(3,3-difluoropyrrolidin-1-yl)azetidin-1-yl]-1-[3-(trifluoromethyl)phenyl]-1H-pyrazolo[3,4-d]pyrimidin-6-yl}amino)cyclobutanol

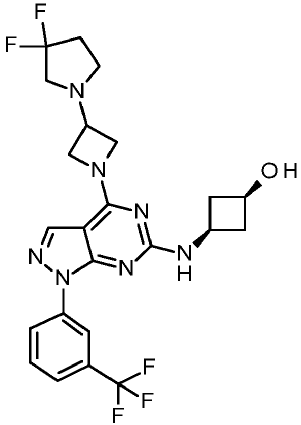

A solution of 6-chloro-4-[3-(3,3-difluoropyrrolidin-1-yl)azetidin-1-yl]-1-[3-(trifluoromethyl)-phenyl]-1H-pyrazolo[3,4-d]pyrimidine (80 mg) and cis-3-aminocyclobutanol hydrochloride (43 mg) in NMP (3.0 ml) was treated with DIPEA (0.18 ml) and the mixture was stirred at 130°C for 16 h. Standard workup and purification by HPLC (method HT_PREP _acid) afforded 11 mg of the title compound.
¹H-NMR (400 MHz, DMSO-d6) δ [ppm]: 1.792 (1.61), 1.799 (1.35), 1.814 (3.87), 1.820 (3.80), 1.836 (3.80), 1.842 (4.13), 1.857 (1.46), 1.864 (1.75), 2.240 (1.06), 2.257 (2.19), 2.277 (2.70), 2.295 (4.31), 2.312 (2.89), 2.322 (2.52), 2.327 (3.07), 2.331 (3.32), 2.350 (1.06), 2.518 (8.07), 2.522 (5.19), 2.539 (0.80), 2.608 (3.07), 2.659 (1.10), 2.664 (1.79), 2.669 (2.26), 2.673 (1.68), 2.776 (4.68), 2.793 (8.66), 2.811 (4.02), 2.981 (1.72), 3.012 (3.11), 3.041 (1.72), 3.572 (0.62), 3.633 (2.52), 3.792 (1.42), 3.809 (2.01), 3.827 (2.70), 3.844 (2.48), 3.861 (1.39), 4.006 (0.95), 4.207 (1.17), 4.311 (1.28), 4.417 (1.13), 5.053 (2.41), 5.066 (2.16), 7.432 (1.42), 7.592 (3.47), 7.612 (4.46), 7.722 (1.94), 7.742 (3.03), 7.761 (1.46), 8.040 (16.00), 8.455 (1.28), 9.036 (1.79).

### Example I-119

### trans-3-({4-(3-fluoro[1,3'-biazetidin]-1'-yl)-1-[3-(trifluoromethyl)phenyl]-1H-pyrazolo[3,4-d]pyrimidin-6-yl}amino)cyclobutanol

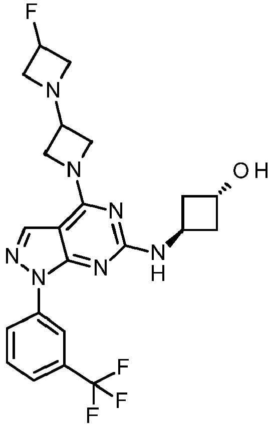

A solution of 6-chloro-4-(3-fluoro[1,3'-biazetidin]-1'-yl)-1-[3-(trifluoromethyl)phenyl]-1H-pyrazolo[3,4-d]pyrimidine (50 mg) and trans-3-aminocyclobutanol hydrochloride (29 mg) in NMP (2.0 ml) was treated with DIPEA (0.12 ml) and the mixture was stirred at 130°C for 16 h. Standard workup and purification by HPLC (water + 0.1% formic acid / acetonitrile) afforded 11 mg of the title compound.
¹H-NMR (400 MHz, DMSO-d6) δ [ppm]: 0.000 (2.93), 1.232 (1.34), 1.713 (0.48), 2.164 (3.51), 2.180 (3.25), 2.283 (3.03), 2.326 (1.98), 2.331 (2.36), 2.335 (1.69), 2.521 (8.13), 2.526 (5.23), 2.668 (1.43), 2.672 (1.94), 2.677 (1.37), 2.716 (1.02), 3.250 (2.77), 3.590 (2.10), 3.604 (2.84), 3.629 (3.03), 3.646 (2.80), 3.669 (2.01), 3.696 (0.83), 3.707 (1.66), 3.724 (3.03), 3.735 (2.14), 3.752 (1.05), 3.869 (1.02), 4.188 (1.98), 4.307 (2.58), 4.389 (1.12), 4.978 (1.53), 5.141 (1.24), 5.152 (1.88), 5.164 (1.18), 5.285 (1.18), 5.297 (1.78), 5.311 (1.18), 7.429 (1.43), 7.593 (3.44), 7.613 (4.69), 7.717 (2.55), 7.736 (4.24), 7.757 (2.04), 7.894 (0.41), 8.061 (16.00), 8.407 (0.67), 8.422 (0.80), 8.479 (1.27), 8.956 (1.66).

### Example I-120

### trans-3-({4-(3-fluoro[1,3'-biazetidin]-1'-yl)-1-[3-(trifluoromethyl)phenyl]-1H-pyrazolo[3,4-d]pyrimidin-6-yl}amino)-1-methylcyclobutanol

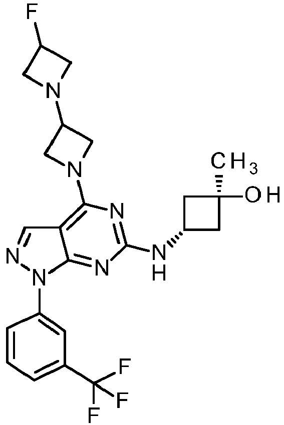

A solution of 6-chloro-4-(3-fluoro[1,3'-biazetidin]-1'-yl)-1-[3-(trifluoromethyl)phenyl]-1H-pyrazolo[3,4-d]pyrimidine (50 mg) and trans-3-amino-1-methylcyclobutanol hydrochloride (CAS number 1523571-03-0) (32 mg) in NMP (2.0 ml) was treated with DIPEA (0.12 ml) and the mixture was stirred at 130°C for 16 h. Standard workup and purification by HPLC (water + 0.1% formic acid / acetonitrile) afforded 14 mg of the title compound.
1H-NMR (400 MHz, DMSO-d6) δ [ppm]: 1.229 (0.52), 1.237 (0.40), 1.268 (16.00), 2.007 (1.23), 2.022 (1.23), 2.037 (1.08), 2.323 (1.32), 2.327 (1.69), 2.332 (1.45), 2.337 (1.08), 2.350 (0.94), 2.518 (3.59), 2.523 (2.39), 2.665 (0.69), 2.669 (0.93), 2.674 (0.65), 2.713 (0.46), 3.243 (1.04), 3.586 (0.76), 3.601 (1.07), 3.626 (1.14), 3.642 (1.05), 3.665 (0.72), 3.703 (0.65), 3.709 (0.75), 3.720 (1.20), 3.730 (0.82), 3.737 (0.75), 4.175 (0.69), 4.307 (0.53), 4.378 (0.43), 4.747 (1.45), 5.136 (0.47), 5.149 (0.73), 5.160 (0.44), 5.281 (0.47), 5.293 (0.72), 5.307 (0.44), 7.371 (0.52), 7.591 (1.31), 7.611 (1.71), 7.710 (0.99), 7.730 (1.57), 7.750 (0.72), 8.053 (6.52), 8.405 (0.43), 8.420 (0.47), 8.531 (0.52), 8.865 (0.61).

### Example I-121

### 4-(1-{6-[(cis-3-hydroxycyclobutyl)amino]-1-[3-(trifluoromethyl)phenyl]-1H-pyrazolo[3,4-d]pyrimidin-4-yl}azetidin-3-yl)morpholin-3-one

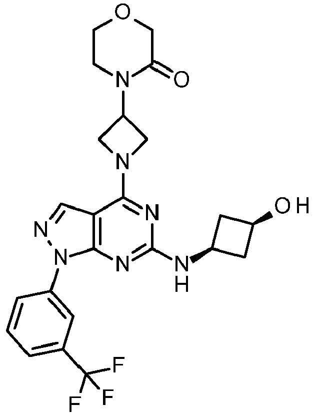

A solution of 4-(1-{6-chloro-1-[3-(trifluoromethyl)phenyl]-1H-pyrazolo[3,4-d]pyrimidin-4-yl}-azetidin-3-yl)morpholin-3-one (80 mg) and cis-3-aminocyclobutanol hydrochloride (44 mg) in NMP (3.0 ml) was treated with DIPEA (0.18 ml) and the mixture was stirred at 130°C for 16 h. Standard workup and purification by HPLC (method HT_PREP _acid) afforded 38 mg of the title compound.
1H-NMR (400 MHz, DMSO-d6) δ [ppm]: 1.798 (0.87), 1.804 (0.79), 1.819 (2.13), 1.825 (2.07), 1.841 (2.09), 1.846 (2.23), 1.862 (0.85), 1.868 (0.91), 2.326 (0.63), 2.331 (0.89), 2.336 (0.65), 2.522 (3.69), 2.526 (2.33), 2.544 (0.41), 2.616 (1.76), 2.664 (0.59), 2.668 (0.83), 2.673 (1.01), 2.678 (0.73), 3.580 (2.94), 3.593 (5.19), 3.605 (3.45), 3.800 (0.87), 3.817 (1.16), 3.835 (1.54), 3.852 (1.48), 3.870 (1.07), 3.885 (2.98), 3.897 (4.04), 3.909 (2.51), 4.097 (16.00), 4.350 (0.73), 4.569 (0.77), 5.064 (1.52), 5.076 (1.36), 5.417 (1.01), 7.503 (0.93), 7.600 (2.05), 7.619 (2.62), 7.729 (1.16), 7.750 (1.89), 7.769 (0.89), 8.053 (11.27), 8.445 (0.83), 8.461 (0.81), 9.041 (1.24).

### Example I-122

### 4-(1-{6-[(trans-3-hydroxy-3-methylcyclobutyl)amino]-1-[3-(trifluoromethyl)phenyl]-1H-pyrazolo[3,4-d]pyrimidin-4-yl}azetidin-3-yl)morpholin-3-one

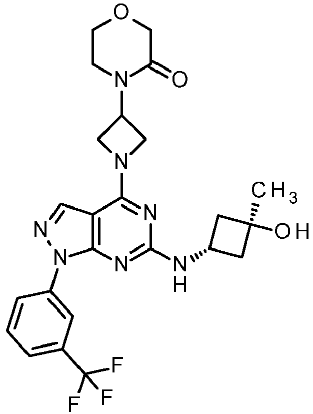

A solution of 4-(1-{6-chloro-1-[3-(trifluoromethyl)phenyl]-1H-pyrazolo[3,4-d]pyrimidin-4-yl}-azetidin-3-yl)morpholin-3-one (80 mg) and trans-3-amino-1-methylcyclobutanol hydrochloride (CAS number 1523571-03-0) (36.5 mg) in NMP (3.0 ml) was treated with DIPEA (0.19 ml) and the mixture was stirred at 130°C for 16 h. Stan dard workup and purification by HPLC (method HT_PREP _acid) afforded 31 mg of the title compound.
1H-NMR (400 MHz, DMSO-d6) δ [ppm]: 1.271 (16.00), 1.304 (0.48), 2.000 (1.04), 2.014 (1.28), 2.030 (1.28), 2.045 (1.20), 2.323 (0.93), 2.327 (1.24), 2.331 (1.19), 2.336 (1.01), 2.359 (1.01), 2.518 (2.28), 2.523 (1.49), 2.664 (0.47), 2.669 (0.64), 2.673 (0.47), 3.580 (1.93), 3.592 (3.37), 3.605 (2.25), 3.881 (1.79), 3.894 (2.56), 3.906 (1.59), 4.093 (10.69), 4.304 (0.71), 4.561 (0.51), 4.754 (4.05), 5.418 (0.59), 7.460 (0.57), 7.597 (1.33), 7.616 (1.70), 7.716 (0.99), 7.736 (1.60), 7.756 (0.77), 8.049 (8.48), 8.512 (0.53), 8.529 (0.56), 8.874 (0.76).

### Example I-123

### trans-1-methyl-3-({4-[3-methyl-3-(morpholin-4-yl)azetidin-1-yl]-1-[3-(trifluoromethyl)-phenyl]-1H-pyrazolo[3,4-d]pyrimidin-6-yl}amino)cyclobutanol

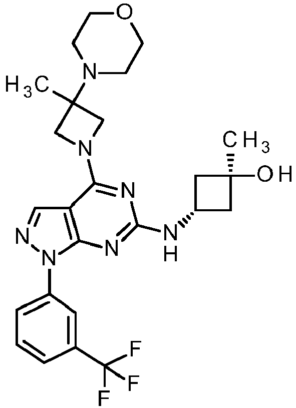

A solution of 6-chloro-4-[3-methyl-3-(morpholin-4-yl)azetidin-1-yl]-1-[3-(trifluoromethyl)-phenyl]-1H-pyrazolo[3,4-d]pyrimidine (75 mg) and trans-3-amino-1-methylcyclobutanol hydrochloride (CAS number 1523571-03-0) (CAS number 1523571-03-0) (34 mg) in NMP (2.8 ml) was treated with DIPEA (0.17 ml) and the mixture was stirred at 130°C for 16 h. Standard workup and purification by HPLC (method HT_PREP _acid) afforded 18 mg of the title compound.
1H-NMR (400 MHz, DMSO-d6) δ [ppm]: 1.269 (16.00), 1.327 (12.84), 2.011 (1.24), 2.037 (1.09), 2.322 (1.17), 2.327 (1.48), 2.331 (1.36), 2.354 (0.96), 2.447 (4.52), 2.518 (2.97), 2.522 (1.94), 2.539 (1.68), 2.664 (0.57), 2.669 (0.77), 2.673 (0.57), 3.613 (4.77), 3.796 (0.60), 3.948 (0.61), 4.039 (0.69), 4.057 (0.75), 4.213 (0.73), 4.232 (0.62), 4.745 (2.46), 7.376 (0.54), 7.593 (1.32), 7.612 (1.74), 7.711 (0.99), 7.731 (1.62), 7.752 (0.75), 8.045 (7.43), 8.534 (0.53), 8.871 (0.65).

### Example I-124

### (3S)-1-(6-{[(1s,3R)-3-hydroxycyclobutyl]amino}-1-[3-(trifluoromethyl)phenyl]-1H-pyrazolo[3,4-d]pyrimidin-4-yl)-3-methylpyrrolidin-3-ol

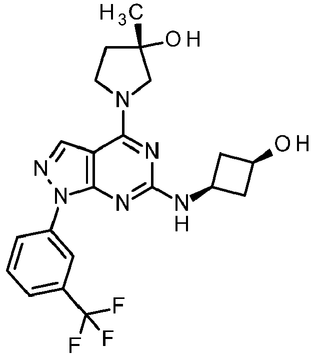

A solution of (3S)-1-{6-chloro-1-[3-(trifluoromethyl)phenyl]-1H-pyrazolo[3,4-d]pyrimidin-4-yl}-3-methylpyrrolidin-3-ol (65 mg) and cis-3-aminocyclobutanol hydrochloride (40 mg) in NMP (2.0 ml) was treated with DIPEA (0.17 ml) and the mixture was stirred at 130°C for 16 h. Standard workup and purification by HPLC (method HT_PREP _acid) afforded 51 mg of the title compound.
LC-MS (method HTpost_acid): Rₜ = 1.02 min; MS (ESIpos): m/z = 449 [M+H]⁺
¹H-NMR (400 MHz, DMSO-d6) δ [ppm]: 1.370 (15.28), 1.417 (16.00), 1.809 (1.94), 1.830 (5.26), 1.852 (6.21), 1.876 (3.58), 1.895 (2.06), 1.995 (1.52), 2.017 (3.95), 2.035 (2.89), 2.326 (1.21), 2.331 (1.60), 2.336 (1.18), 2.522 (5.99), 2.526 (3.82), 2.544 (1.93), 2.616 (4.27), 2.664 (1.52), 2.668 (1.86), 2.673 (2.02), 2.678 (1.42), 3.657 (9.08), 3.701 (1.92), 3.733 (1.48), 3.844 (6.65), 3.864 (6.12), 4.895 (4.30), 4.963 (3.66), 5.058 (2.52), 7.208 (1.32), 7.589 (4.86), 7.609 (6.30), 7.720 (2.56), 7.739 (4.13), 7.759 (2.02), 8.144 (6.65), 8.184 (9.14), 8.223 (0.44), 8.473 (1.29), 8.633 (0.57), 9.089 (1.52).

### Example II-1

### (4S)-4-({1-[3-(difluoromethyl)phenyl]-4-[3-(morpholin-4-yl)azetidin-1-yl]-1H-pyrazolo[3,4-d]pyrimidin-6-yl}amino)pyrrolidin-2-one

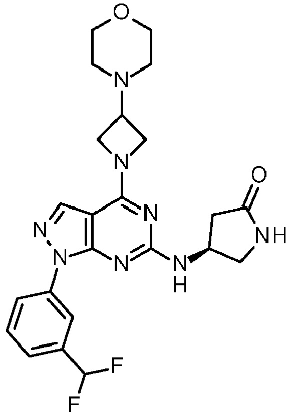

DIPEA (0.20 ml) was added to 6-chloro-1-[3-(difluoromethyl)phenyl]-4-[3-(morpholin-4-yl)-azetidin-1-yl]-1H-pyrazolo[3,4-d]pyrimidine (80 mg, 0.19 mmol) and (4S)-4-aminopyrrolidin-2-one hydrochloride (51.9 mg, 0.38 mmol) in NMP (2.1 ml) and the mixture was stirred at 130°C for 20 h. The mixture was treated with water and ethyl acetate, the precipitated solid was separated by filtration, washed with water and 2-methoxy-2-methylpropane and dried to afford 28.0 mg of the title compound.
¹H-NMR (400 MHz, DMSO-d6) δ [ppm]: 2.288 (1.33), 2.327 (3.69), 2.553 (2.16), 2.669 (2.10), 3.199 (1.63), 3.361 (4.32), 3.625 (15.73), 3.990 (1.16), 4.186 (1.43), 4.263 (1.60), 4.429 (1.40), 4.522 (0.50), 4.536 (1.36), 4.554 (2.30), 4.571 (2.23), 4.587 (1.26), 6.991 (2.23), 7.131 (4.56), 7.270 (2.10), 7.438 (4.59), 7.457 (5.79), 7.616 (7.92), 7.648 (3.36), 7.667 (1.73), 8.069 (16.00), 8.415 (1.33), 8.538 (0.77), 8.605 (1.13).

### Example II-2

### 1-[3-(difluoromethyl)phenyl]-N-(1,1-dioxidotetrahydro-2H-thiopyran-4-yl)-4-[3-(morpholin-4-yl)azetidin-1-yl]-1H-pyrazolo[3,4-d]pyrimidin-6-amine

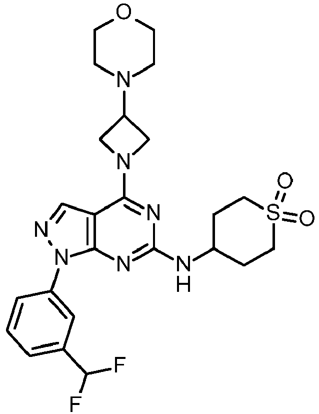

DIPEA (0.20 ml) was added to 6-chloro-1-[3-(difluoromethyl)phenyl]-4-[3-(morpholin-4-yl)-azetidin-1-yl]-1H-pyrazolo[3,4-d]pyrimidine (80 mg, 0.19 mmol) and tetrahydro-2H-thiopyran-4-amine 1,1-dioxide (56.7 mg, 0.38 mmol) in NMP (2.1 ml) and the mixture was stirred at 130°C for 20 h. Standard workup and purification by HPLC (method HT_PREP _acid) afforded 38 mg of the title compound.
LC-MS (method HTpost_acid): Rₜ = 0.81 min; MS (ESIpos): m/z = 534 [M+H]⁺
¹H-NMR (400 MHz, DMSO-d6) δ [ppm]: 0.934 (0.56), 0.951 (0.56), 2.024 (0.61), 2.050 (1.89), 2.073 (3.00), 2.100 (1.00), 2.232 (1.89), 2.318 (0.78), 2.322 (1.50), 2.326 (2.00), 2.331 (1.44), 2.336 (0.78), 2.402 (5.94), 2.518 (7.22), 2.522 (4.72), 2.664 (1.50), 2.668 (1.83), 2.673 (1.33), 2.678 (0.67), 2.838 (0.44), 2.849 (0.44), 3.150 (1.11), 3.191 (4.67), 3.226 (2.56), 3.254 (0.94), 3.262 (0.89), 3.376 (2.22), 3.625 (8.50), 3.992 (0.72), 4.124 (1.06), 4.191 (0.89), 4.265 (0.94), 4.422 (0.78), 7.020 (0.89), 7.160 (1.72), 7.299 (1.39), 7.445 (2.56), 7.465 (2.94), 7.645 (1.17), 7.665 (1.89), 7.684 (0.94), 8.039 (0.61), 8.063 (16.00), 8.422 (0.83), 8.620 (0.67).

### Example II-3

### 1-[3-(difluoromethyl)phenyl]-N-[(3S)-1,1-dioxidotetrahydrothiophen-3-yl]-4-[3-(morpholin-4-yl)azetidin-1-yl]-1H-pyrazolo[3,4-d]pyrimidin-6-amine

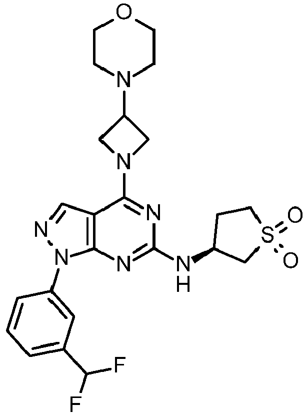

DIPEA (0.20 ml) was added to 6-chloro-1-[3-(difluoromethyl)phenyl]-4-[3-(morpholin-4-yl)-azetidin-1-yl]-1H-pyrazolo[3,4-d]pyrimidine (80 mg, 0.18 mmol, 1 eq.) and (3S)tetrahydrothiophen-3-amine 1,1-dioxide hydrochloride (65.3 mg, 0.38 mmol) in NMP (2.1 ml) and the mixture was stirred at 130°C for 20 h. Then 1 eq. of (3S)tetrahydrothiophen-3-amine 1,1-dioxide hydrochloride and 3 eq. DIPEA were added and the mixture was stirred at 130°C for 20 h. Standard workup and purification by HPLC (method HT_PREP _acid) afforded 18 mg of the title compound.
LC-MS (method HTpost_acid): Rₜ = 1.12 min; MS (ESIneg): m/z = 518 [M-H]⁻
¹H-NMR (400 MHz, DMSO-d6) δ [ppm]: 2.185 (0.40), 2.206 (1.13), 2.219 (0.71), 2.227 (1.30), 2.239 (1.55), 2.260 (1.53), 2.281 (0.63), 2.336 (0.55), 2.406 (5.80), 2.459 (1.15), 2.518 (5.23), 2.522 (3.51), 2.539 (0.67), 2.837 (0.44), 2.849 (0.42), 3.033 (1.66), 3.106 (0.97), 3.127 (1.66), 3.148 (1.68), 3.160 (1.53), 3.168 (1.20), 3.181 (2.20), 3.202 (1.36), 3.380 (5.54), 3.414 (1.53), 3.523 (1.76), 3.542 (1.93), 3.556 (1.68), 3.575 (1.66), 3.625 (8.48), 4.002 (0.71), 4.198 (0.86), 4.283 (0.92), 4.438 (0.86), 4.618 (0.97), 4.634 (1.66), 4.653 (1.60), 4.669 (0.88), 6.990 (2.39), 7.129 (5.06), 7.268 (2.14), 7.455 (2.56), 7.474 (3.04), 7.545 (0.52), 7.629 (1.85), 7.648 (3.04), 7.668 (1.51), 8.038 (0.52), 8.091 (16.00), 8.278 (0.82), 8.399 (0.67), 8.549 (0.50).

### Example II-4

### 2-[1-(1-[3-(difluoromethyl)phenyl]-6-{[(3S)-1-methylpyrrolidin-3-yl]amino}-1H-pyrazolo-[3,4-d]pyrimidin-4-yl)piperidin-4-yl]propan-2-ol

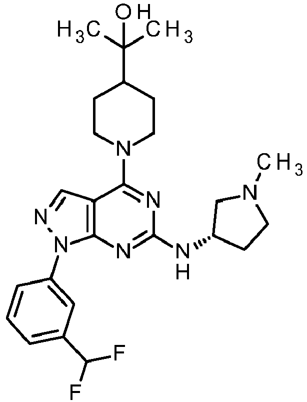

DIPEA (0.16 ml) was added to 2-(1-{6-chloro-1-[3-(difluoromethyl)phenyl]-1H-pyrazolo[3,4-d]-pyrimidin-4-yl}piperidin-4-yl)propan-2-ol (65.0 mg, 0.15 mmol) and (3S)-1-methylpyrrolidin-3-amine dihydrochloride (53.3 mg, 0.31 mmol) in NMP (1.9 ml) and the mixture was stirred at 130°C for 16 h. Standard workup and purification by HPLC (method HT_PREP_bas) afforded 17.0 mg of the title compound.
LC-MS (method HTpost_bas): Rₜ = 1.28 min; MS (ESIneg): m/z = 484 [M-H]⁻
¹H-NMR (400 MHz, DMSO-d6) δ [ppm]: 1.051 (16.00), 1.226 (0.50), 1.257 (0.51), 1.551 (0.42), 1.829 (0.72), 1.859 (0.65), 2.235 (11.59), 2.302 (0.55), 2.422 (0.55), 2.518 (1.80), 2.523 (1.44), 2.798 (0.52), 4.181 (3.46), 6.982 (0.81), 7.121 (1.34), 7.261 (0.57), 7.432 (0.79), 7.451 (0.93), 7.640 (0.68), 8.286 (3.87).

### Example II-5

### 2-(1-{1-[3-(difluoromethyl)phenyl]-6-[(1,1-dioxidotetrahydro-2H-thiopyran-4-yl)amino]-1H-pyrazolo[3,4-d]pyrimidin-4-yl}piperidin-4-yl)propan-2-ol

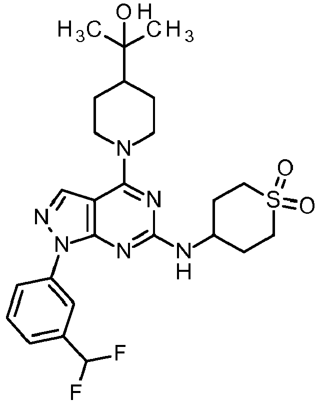

A solution of 2-(1-{6-chloro-1-[3-(difluoromethyl)phenyl]-1H-pyrazolo[3,4-d]pyrimidin-4-yl}-piperidin-4-yl)propan-2-ol (65.0 mg, 0.15 mol) and tetrahydro-2H-thiopyran-4-amine 1,1-dioxide (46.0 mg, 0.31 mmol) in NMP (2.0 ml) was treated with DIPEA (0.16 ml) and the mixture was stirred at 130°C for 16 h. 1 eq. tetrah ydro-2H-thiopyran-4-amine 1,1-dioxide and 3 eq. DIPEA were added and the mixture was stirred at 130°C for 20 h. Standard workup and purification by HPLC (method HT_PREP_bas) afforded 43.0 mg of the title compound.
LC-MS (method HTpost_bas): Rₜ = 1.15 min; MS (ESIpos): m/z = 535 [M+H]⁺
¹H-NMR (400 MHz, DMSO-d6) δ [ppm]: 1.054 (16.00), 1.231 (0.65), 1.254 (0.63), 1.262 (0.60), 1.555 (0.47), 1.836 (0.77), 1.866 (0.70), 2.054 (0.59), 2.074 (0.95), 2.078 (0.70), 2.243 (0.51), 2.518 (1.68), 2.523 (1.06), 3.190 (1.00), 4.188 (5.26), 7.158 (0.73), 7.451 (0.82), 7.470 (0.99), 7.662 (0.55), 8.308 (4.45).

### Example II-6

### 2-[1-(1-[3-(difluoromethyl)phenyl]-6-{[(3S)-1,1-dioxidotetrahydrothiophen-3-yl]amino}-1H-pyrazolo[3,4-d]pyrimidin-4-yl)piperidin-4-yl]propan-2-ol

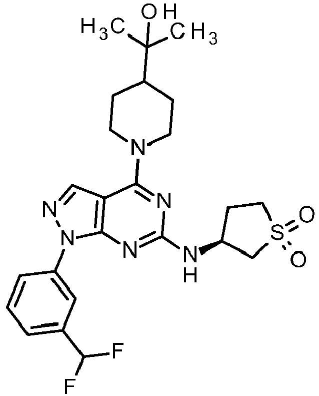

A solution of 2-(1-{6-chloro-1-[3-(difluoromethyl)phenyl]-1H-pyrazolo[3,4-d]pyrimidin-4-yl}-piperidin-4-yl)propan-2-ol (65.0 mg, 0.15 mmol, 1.0 eq.) and (3S)tetrahydrothiophen-3-amine 1,1-dioxide hydrochloride (52.9 mg, 308 µmol) in NMP (2.0 ml) was treated with DIPEA (0.16 ml) and the mixture was stirred at 130°C for 16 h. Then 1 eq. (3S)tetrahydrothiophen-3-amine 1,1-dioxide hydrochloride and 3 eq. DIPEA were added and the mixture was stirred at 130°C for 20 h. Standard workup and purification by HPLC (method HT_PREP_bas) afforded 40.0 mg of the title compound.
LC-MS (method HTpost_bas): Rₜ = 1.14 min; MS (ESIpos): m/z = 521 [M+H]⁺
¹H-NMR (400 MHz, DMSO-d6) δ [ppm]: 1.054 (16.00), 1.238 (0.56), 1.265 (0.58), 1.567 (0.48), 1.841 (0.83), 1.870 (0.73), 2.232 (0.45), 2.245 (0.53), 2.266 (0.53), 2.518 (1.90), 2.523 (1.28), 3.134 (0.72), 3.198 (0.50), 3.362 (0.72), 3.366 (0.67), 3.381 (0.61), 3.519 (0.47), 3.537 (0.53), 3.552 (0.45), 3.571 (0.42), 4.192 (4.58), 4.639 (0.44), 4.657 (0.45), 6.992 (0.83), 7.132 (1.75), 7.271 (0.73), 7.462 (0.84), 7.481 (1.03), 7.630 (0.64), 7.650 (1.11), 7.669 (0.55), 8.342 (4.54).

### Example II-7

### 1-(1-{1-[3-(difluoromethyl)phenyl]-6-[(1,1-dioxidotetrahydro-2H-thiopyran-4-yl)amino]-1H-pyrazolo[3,4-d]pyrimidin-4-yl}azetidin-3-yl)-2-methylpropan-2-ol

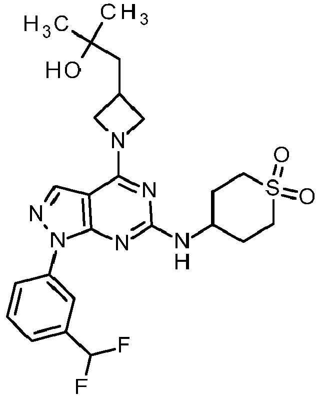

DIPEA (0.22 ml) was added to 1-(1-{6-chloro-1-[3-(difluoromethyl)phenyl]-1H-pyrazolo[3,4-d]-pyrimidin-4-yl}azetidin-3-yl)-2-methylpropan-2-ol (85.0 mg, 0.21 mmol) and tetrahydro-2H-thiopyran-4-amine 1,1-dioxide (62.2 mg, 0.42 mmol) in NMP (1.5 ml) and the mixture was stirred 17 h at 120°C. Standard workup and purification by HPLC (method HT_PREP_acid) afforded 39.7 mg of the title compound.
LC-MS (method HTpost_acid): Rₜ = 1.00 min; MS (ESIpos): m/z = 521 [M+H]⁺
¹H-NMR (400 MHz, DMSO-d6) δ [ppm]: 1.102 (16.00), 1.760 (2.18), 1.778 (2.21), 2.047 (0.74), 2.073 (0.87), 2.230 (0.75), 2.518 (1.65), 2.522 (1.10), 2.539 (0.45), 3.041 (0.53), 3.058 (0.41), 3.150 (0.43), 3.191 (1.91), 3.224 (1.02), 4.102 (0.43), 4.258 (1.28), 7.160 (0.75), 7.298 (0.45), 7.441 (0.97), 7.460 (1.14), 7.641 (0.47), 7.661 (0.80), 7.681 (0.40), 8.016 (5.53), 8.163 (0.65).

### Example II-8

### 1-[3-(difluoromethyl)phenyl]-N-[trans-3-(methylsulfonyl)cyclobutyl]-4-[3-(morpholin-4-yl)azetidin-1-yl]-1H-pyrazolo[3,4-d]pyrimidin-6-amine

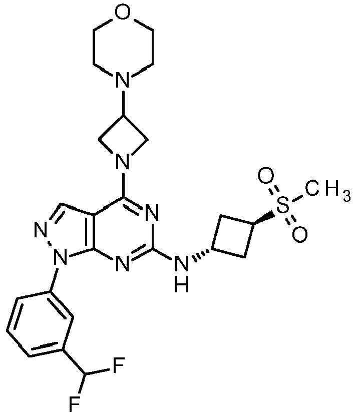

DIPEA (0.20 ml) was added to 6-chloro-1-[3-(difluoromethyl)phenyl]-4-[3-(morpholin-4-yl)-azetidin-1-yl]-1H-pyrazolo[3,4-d]pyrimidine (80 mg, 190 µmol) and trans-3-(methylsulfonyl)cyclobutanamine hydrochloride (52.9 mg, 285 µmol) in NMP (1.5 ml) and the mixture was stirred 16 h at 130°C. After removal of solids by filtration, DMSO (1 ml) was added and the solution was purified by HPLC (method HT_PREP_bas) to afford 62.1 mg of the title compound.
LC-MS (method HTpost_bas): Rₜ = 1.01 min; MS (ESIpos): m/z = 534 [M+H]⁺
¹H-NMR (400 MHz, DMSO-d6) δ [ppm]: 0.931 (2.86), 0.948 (2.84), 0.952 (0.54), 2.075 (0.67), 2.327 (0.53), 2.332 (0.40), 2.405 (2.95), 2.518 (2.37), 2.523 (1.54), 2.539 (0.49), 2.665 (0.50), 2.669 (0.68), 2.673 (0.62), 2.716 (0.89), 2.947 (16.00), 3.346 (0.72), 3.364 (0.98), 3.376 (0.62), 3.625 (4.12), 3.829 (0.48), 3.841 (0.67), 3.853 (0.86), 3.864 (0.65), 3.875 (0.45), 4.265 (0.42), 4.541 (0.48), 4.558 (0.46), 6.968 (0.81), 7.107 (1.65), 7.247 (0.74), 7.450 (1.16), 7.469 (1.39), 7.615 (1.27), 7.634 (1.92), 7.655 (0.98), 8.061 (6.90).

### Example II-9

### (1-{1-[3-(difluoromethyl)phenyl]-6-[(1,1-dioxidotetrahydro-2H-thiopyran-4-yl)amino]-1H-pyrazolo[3,4-d]pyrimidin-4-yl}azetidin-3-yl)acetic acid

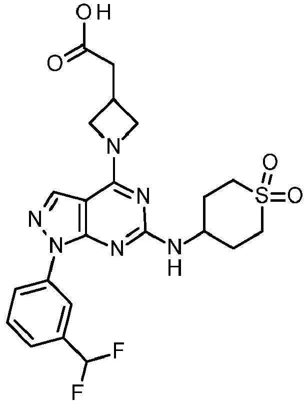

### Step a)

### methyl (1-{1-[3-(difluoromethyl)phenyl]-6-[(1,1-dioxidotetrahydro-2H-thiopyran-4-yl)-amino]-1H-pyrazolo[3,4-d]pyrimidin-4-yl}azetidin-3-yl)acetate

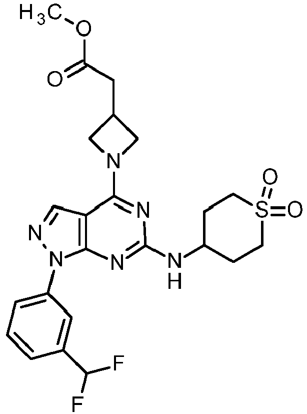

DIPEA (0.48 ml) was added to methyl (1-{6-chloro-1-[3-(difluoromethyl)phenyl]-1H-pyrazolo-[3,4-d]pyrimidin-4-yl}azetidin-3-yl)acetate (200 mg) and tetrahydro-2H-thiopyran-4-amine 1,1-dioxide (103 mg, 0.69 mmol) in NMP (2.0 ml) and the mixture was stirred 21 h at 130°C. A spatula of tetrahydro-2H-thiopyran-4-amine 1,1-dioxide was added and the mixture was stirred 22 h at 130°C. Standard workup afforded a c rude product, which was used in step b).

### Step b)

Aq. sodium hydroxide solution (1.7 ml, 2.0 M) was added to methyl (1-{1-[3-(difluoromethyl)-phenyl]-6-[(1,1-dioxidotetrahydro-2H-thiopyran-4-yl)amino]-1H-pyrazolo[3,4-d]pyrimidin-4-yl}-azetidin-3-yl)acetate (240 mg, batch contained residual NMP) in 2-methyltetrahydrofuran (1.5 ml) and the mixture was stirred at rt for 19 h. The mixture was acidified to pH 4 using aq. citric acid (10% solution), then the mixture was diluted with water and a small amount of ethyl acetate and the resulting solid was filtered, washed with water and 2-methoxy-2-methylpropane twice. The solid was then treated with DMSO, the solid was filtered, stirred in 2-methoxy-2-methylpropane, filtered, washed with 2-methoxy-2-methylpropane and dried to afford 81.7mg (1-{1-[3-(difluoromethyl)phenyl]-6-[(1,1-dioxidotetrahydro-2H-thiopyran-4-yl)-amino]-1H-pyrazolo[3,4-d]pyrimidin-4-yl}azetidin-3-yl)acetic acid.
LC-MS (method a01): Rₜ = 0.97 min; MS (ESIpos): m/z = 507 [M+H]⁺
¹H-NMR (400 MHz, DMSO-d6) δ [ppm]: 1.104 (1.17), 2.023 (0.42), 2.049 (1.41), 2.074 (1.65), 2.100 (0.72), 2.231 (1.30), 2.337 (0.50), 2.518 (5.73), 2.523 (4.01), 2.540 (16.00), 2.674 (1.33), 2.679 (0.93), 2.689 (4.01), 2.708 (4.30), 3.074 (1.09), 3.093 (0.98), 3.112 (0.77), 3.147 (0.93), 3.181 (2.49), 3.193 (2.81), 3.228 (1.96), 3.256 (0.90), 3.859 (0.58), 4.105 (1.09), 4.270 (0.53), 4.535 (0.58), 7.019 (0.58), 7.159 (1.22), 7.298 (0.90), 7.441 (1.86), 7.461 (2.23), 7.641 (0.80), 7.661 (1.35), 7.682 (0.72), 8.032 (13.74), 8.418 (0.56), 8.627 (0.48).

### Example II-10

### 2-(1-{1-[3-(difluoromethyl)phenyl]-6-[(1,1-dioxidotetrahydro-2H-thiopyran-4-yl)amino]-1H-pyrazolo[3,4-d]pyrimidin-4-yl}azetidin-3-yl)-1-(morpholin-4-yl)ethanone

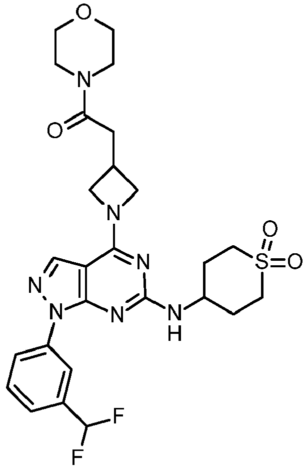

DIPEA (120 µl, 680 µmol) was added to (1-{1-[3-(difluoromethyl)phenyl]-6-[(1,1-dioxidotetrahydro-2H-thiopyran-4-yl)amino]-1H-pyrazolo[3,4-d]pyrimidin-4-yl}azetidin-3-yl)acetic acid (75.4 mg), morpholine (24 µl, 270 µmol) and HATU (77.3 mg, 203 µmol) in NMP (1.5 ml) and the mixture was stirred at rt for 19 h. Standard workup and purification by HPLC (method HT_PREP_bas) afforded 59.1 mg of the title compound.
¹H-NMR (400 MHz, DMSO-d6) δ [ppm]: 1.229 (0.53), 2.048 (1.63), 2.074 (4.43), 2.099 (0.85), 2.230 (1.54), 2.331 (0.75), 2.518 (3.68), 2.522 (2.52), 2.673 (0.79), 2.812 (3.42), 2.831 (3.79), 3.083 (0.85), 3.102 (1.13), 3.121 (0.90), 3.146 (1.09), 3.181 (2.89), 3.194 (3.12), 3.230 (2.16), 3.257 (0.77), 3.428 (4.73), 3.438 (8.11), 3.450 (6.89), 3.532 (3.44), 3.545 (4.13), 3.556 (2.85), 3.566 (3.64), 3.579 (3.91), 3.589 (2.29), 3.828 (0.85), 4.031 (0.92), 4.108 (0.85), 4.271 (0.77), 4.543 (0.94), 7.019 (0.66), 7.158 (1.43), 7.296 (0.94), 7.440 (2.22), 7.459 (2.59), 7.641 (0.96), 7.660 (1.58), 7.679 (0.81), 8.028 (16.00), 8.413 (0.66), 8.635 (0.58).

### Example II-11

### 2-[1-(1-[3-(difluoromethyl)phenyl]-6-{[trans-3-(methylsulfonyl)cyclobutyl]amino}-1H-pyrazolo[3,4-d]pyrimidin-4-yl)piperidin-4-yl]propan-2-ol

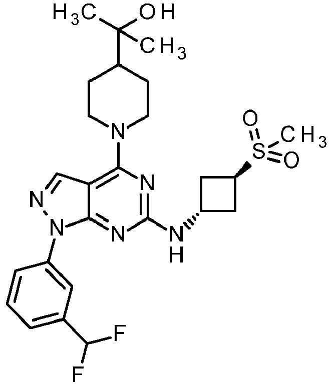

A solution of 2-(1-{6-chloro-1-[3-(difluoromethyl)phenyl]-1H-pyrazolo[3,4-d]pyrimidin-4-yl}piperidin-4-yl)propan-2-ol (100 mg, 0.24 mmol) and trans-3-(methylsulfonyl)cyclobutanamine hydrochloride (0.47 mmol) in NMP (2.6 ml) was treated with DIPEA (250 µl, 1.4 mmol) and the mixture was stirred at 130°C for 16 h. Standard workup and purification by HPLC (method method HT_PREP_bas; the resulting product was then subjected to additional purification by using method method HT_PREP_acid) afforded 70.0 mg of the title compound.
LC-MS (method HTpost_acid): Rₜ = 1.08 min; MS (ESIpos): m/z = 535 [M+H]⁺
¹H-NMR (400 MHz, DMSO-d6) δ [ppm]: 1.052 (16.00), 1.230 (0.61), 1.253 (0.65), 1.559 (0.51), 1.834 (0.82), 1.866 (0.74), 2.073 (0.56), 2.435 (0.48), 2.459 (0.98), 2.470 (1.19), 2.522 (0.81), 2.669 (0.41), 2.715 (0.67), 2.950 (11.98), 3.007 (0.41), 3.844 (0.46), 3.856 (0.60), 3.868 (0.44), 4.187 (0.93), 4.544 (0.54), 4.562 (0.53), 6.972 (0.59), 7.112 (1.21), 7.251 (0.55), 7.456 (0.88), 7.475 (1.04), 7.616 (0.84), 7.636 (1.38), 7.656 (0.69), 8.308 (3.95).

### Example II-12

### 2-[1-(1-[3-(difluoromethyl)phenyl]-6-{[1-(methylsulfonyl)azetidin-3-yl]amino}-1H-pyrazolo[3,4-d]pyrimidin-4-yl)piperidin-4-yl]propan-2-ol

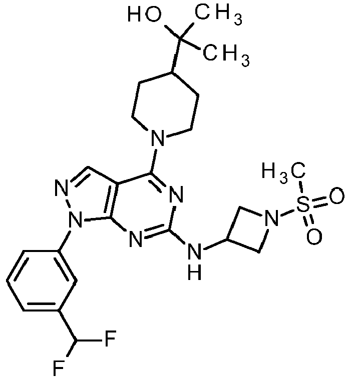

DIPEA (250 µl, 1.4 mmol) was added to (2-(1-{6-chloro-1-[3-(difluoromethyl)phenyl]-1H-pyrazolo[3,4-d]pyrimidin-4-yl}piperidin-4-yl)propan-2-ol (100 mg, 0.24 mmol) and 1-(methylsulfonyl)azetidin-3-amine (71.2 mg, 0.47 mmol) in NMP (1.5 ml) and the mixture was stirred 15.5 h at 120°C. Standard workup and purification by HPLC (method HT_PREP_acid) afforded 55.3 mg of the title compound.
¹H-NMR (400 MHz, DMSO-d6) δ [ppm]: 1.054 (16.00), 1.234 (0.76), 1.256 (0.70), 1.266 (0.65), 1.565 (0.51), 1.844 (0.76), 1.873 (0.69), 2.518 (1.29), 2.522 (0.88), 2.539 (0.67), 3.033 (15.64), 3.877 (0.74), 3.896 (1.21), 3.914 (0.85), 4.108 (0.69), 4.127 (1.21), 4.146 (0.61), 4.189 (4.73), 4.638 (0.61), 4.654 (0.59), 7.158 (0.53), 7.461 (0.86), 7.481 (1.02), 7.672 (0.57), 8.337 (4.46).

### Example II-13

### 1-[3-(difluoromethyl)phenyl]-N-[1-(methylsulfonyl)azetidin-3-yl]-4-[3-(morpholin-4-yl)azetidin-1-yl]-1H-pyrazolo[3,4-d]pyrimidin-6-amine

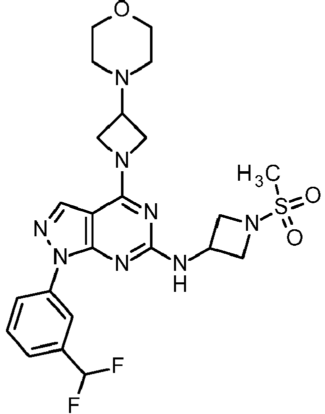

DIPEA (250 µl, 1.4 mmol) was added to 6-chloro-1-[3-(difluoromethyl)phenyl]-4-[3-(morpholin-4-yl)azetidin-1-yl]-1H-pyrazolo[3,4-d]pyrimidine (100 mg, 0.24 mmol) and 1-(methylsulfonyl)-azetidin-3-amine (71.4 mg) in NMP (1.5 ml) and the mixture was stirred for 15.5 h at 120°C. Standard workup and purification by HPLC (method HT_PREP_acid) afforded two batches (19.3 mg and 32.1 mg) of the title compound.
¹H-NMR (400 MHz, DMSO-d6) δ [ppm]: 2.407 (2.22), 2.518 (1.55), 2.522 (1.08), 2.539 (0.78), 3.027 (16.00), 3.351 (0.62), 3.368 (0.79), 3.381 (0.50), 3.616 (2.26), 3.627 (3.33), 3.877 (0.71), 3.894 (1.16), 3.911 (0.79), 4.129 (0.94), 4.633 (0.72), 4.649 (0.68), 7.160 (0.58), 7.455 (1.01), 7.474 (1.18), 7.655 (0.42), 7.672 (0.63), 8.086 (6.09).

### Example II-14

### 1-[1-(1-[3-(difluoromethyl)phenyl]-6-{[1-(methylsulfonyl)azetidin-3-yl]amino}-1H-pyrazolo[3,4-d]pyrimidin-4-yl)azetidin-3-yl]-2-methylpropan-2-ol

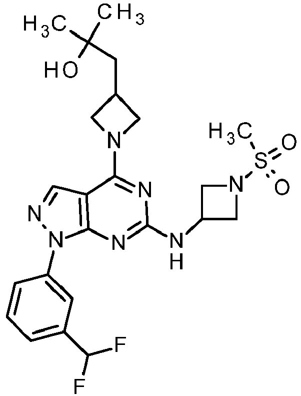

DIPEA (0.26 ml) was added to 1-(1-{6-chloro-1-[3-(difluoromethyl)phenyl]-1H-pyrazolo[3,4-d]-pyrimidin-4-yl}azetidin-3-yl)-2-methylpropan-2-ol (100 mg) and 1-(methylsulfonyl)azetidin-3-amine (73.7 mg, 0.49 mmol) in NMP (1.5 ml) and the mixture was stirred for 15.5 h at 120°C. Standard workup and purification by HPLC (Method HT_PREP_acid) afforded two fractions (21.7 mg and 21.3 mg) of the title compound.
¹H-NMR (400 MHz, DMSO-d6) δ [ppm]: 1.105 (16.00), 1.765 (2.12), 1.784 (2.15), 2.326 (0.55), 2.518 (2.53), 2.522 (1.62), 2.539 (0.45), 2.664 (0.40), 2.668 (0.54), 2.673 (0.41), 3.026 (14.79), 3.051 (0.56), 3.871 (0.87), 3.888 (1.18), 3.906 (0.79), 4.071 (0.49), 4.126 (0.78), 4.263 (1.94), 4.632 (0.64), 4.648 (0.61), 7.158 (0.61), 7.450 (0.93), 7.469 (1.11), 7.652 (0.41), 7.671 (0.65), 8.043 (6.23).

### Example II-15

### 1-{1-[3-(difluoromethyl)phenyl]-6-[(1,1-dioxidotetrahydro-2H-thiopyran-4-yl)amino]-1H-pyrazolo[3,4-d]pyrimidin-4-yl}-3-(trifluoromethyl)pyrrolidin-3-ol

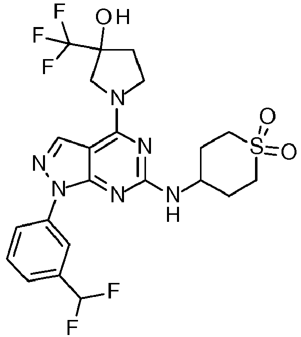

A solution of 1-{6-chloro-1-[3-(difluoromethyl)phenyl]-1H-pyrazolo[3,4-d]pyrimidin-4-yl}-3-(trifluoromethyl)pyrrolidin-3-ol (60.0 mg, 0.14 mmol) and tetrahydro-2H-thiopyran-4-amine 1,1-dioxide (41.3 mg, 0.28 mmol) in NMP (1.5 ml) was treated with DIPEA (0.14 ml) and the mixture was stirred at 130°C for 16 h. Standard workup and purification by HPLC (method HT_PREP_bas) afforded 45.0 mg of the title compound.
LC-MS (method HTpost_bas): Rₜ = 1.13 min; MS (ESIpos): m/z = 547 [M+H]⁺
¹H-NMR (400 MHz, DMSO-d6) δ [ppm]: 2.060 (8.96), 2.073 (9.49), 2.086 (10.88), 2.243 (13.33), 2.326 (4.80), 2.399 (4.27), 2.430 (4.59), 2.668 (3.95), 3.191 (15.57), 3.245 (9.81), 3.761 (3.84), 3.792 (4.05), 3.929 (8.96), 3.958 (8.64), 4.071 (7.47), 4.094 (4.91), 4.143 (4.59), 6.633 (13.23), 6.707 (0.75), 7.025 (2.88), 7.166 (6.29), 7.269 (4.05), 7.300 (3.95), 7.455 (11.52), 7.474 (13.55), 7.566 (1.07), 7.670 (7.36), 8.234 (16.00), 8.436 (4.80), 8.451 (4.48), 8.501 (2.45), 8.574 (3.41), 8.647 (3.09).

### Example II-16-1

### rel-(3R,4R)-1-(1-[3-(difluoromethyl)phenyl]-6-{[trans-3-(methylsulfonyl)cyclobutyl]-amino)-1H-pyrazolo[3,4-d]pyrimidin-4-yl)-4-fluoropyrrolidin-3-ol

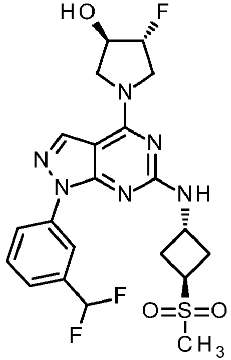

A solution of rel-(3R,4R)-1-{6-chloro-1-[3-(difluoromethyl)phenyl]-1H-pyrazolo[3,4-d]pyrimidin-4-yl}-4-fluoropyrrolidin-3-ol (160 mg, 417 µmol) and trans-3-(methylsulfonyl)cyclobutanamine hydrochloride (155 mg, 834 µmol) in NMP (3.0 ml) was treated with DIPEA (0.44 ml) and the mixture was stirred at 130°C for 16 h. Standard workup and purification by HPLC (method HT_PREP_bas) afforded 103 mg of the title compound.
LC-MS (method HTpost_bas): Rₜ = 1.00 min; MS (ESIpos): m/z = 497 [M+H]⁺
¹H-NMR (400 MHz, DMSO-d6) δ [ppm]: 2.073 (0.67), 2.539 (0.74), 2.728 (1.05), 2.955 (16.00), 3.762 (0.52), 3.791 (0.65), 3.839 (0.85), 3.852 (0.85), 3.864 (1.08), 3.874 (0.88), 3.887 (0.58), 3.998 (0.51), 4.014 (0.50), 4.031 (0.50), 4.063 (0.47), 4.570 (0.59), 4.587 (0.56), 5.660 (0.50), 5.762 (0.64), 5.770 (0.61), 6.977 (0.77), 7.116 (1.59), 7.255 (0.72), 7.459 (1.29), 7.478 (1.56), 7.622 (1.11), 7.642 (1.83), 7.662 (0.91), 8.236 (3.96), 8.451 (0.47).

### Example II-16-2

### (3S,4S)-1-(1-[3-(difluoromethyl)phenyl]-6-{[trans-3-(methanesulfonyl)cyclobutyl]amino}-1H-pyrazolo[3,4-d]pyrimidin-4-yl)-4-fluoropyrrolidin-3-ol

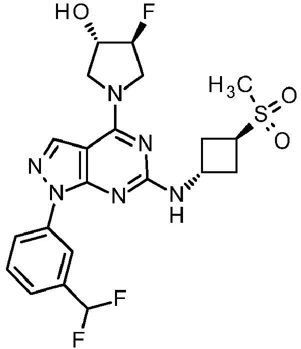

A solution of (3S,4S)-1-{6-chloro-1-[3-(difluoromethyl)phenyl]-1H-pyrazolo[3,4-d]pyrimidin-4-yl}-4-fluoropyrrolidin-3-ol (71 mg) and trans-3-(methylsulfonyl)cyclobutanamine hydrochloride (48 mg) in NMP (1.5 ml) was treated with DIPEA (0.18 ml) and the mixture was stirred at 120°C for 16.5 h and at 130°C for 23 h. Standard wo rkup and purification by HPLC (method HT_PREP_acid) afforded 64 mg of the title compound.
LC-MS (method HTpost_acid): Rₜ = 0.96 min; MS (ESIpos): m/z = 497 [M+H]⁺
¹H-NMR (400 MHz, DMSO-d6) δ [ppm]: -0.008 (0.49), 0.008 (0.46), 2.466 (0.41), 2.521 (2.22), 2.526 (1.53), 2.543 (0.97), 2.731 (0.73), 2.958 (16.00), 3.795 (0.47), 3.843 (0.61), 3.856 (0.60), 3.867 (0.79), 3.878 (0.64), 3.891 (0.41), 4.572 (0.43), 4.591 (0.41), 6.981 (0.66), 7.120 (1.35), 7.260 (0.60), 7.462 (0.96), 7.481 (1.18), 7.626 (0.93), 7.647 (1.53), 7.666 (0.78), 8.183 (0.71), 8.240 (3.59).

### Example II-16-3

### (3R,4R)-1-(1-[3-(difluoromethyl)phenyl]-6-{[trans-3-(methanesulfonyl)cyclobutyl]-amino)-1H-pyrazolo[3,4-d]pyrimidin-4-yl)-4-fluoropyrrolidin-3-ol

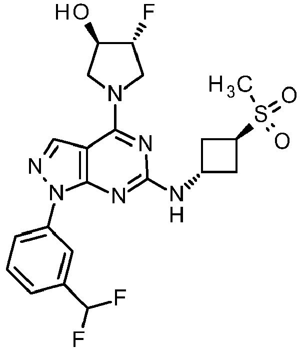

A solution of (3R,4R)-1-{6-chloro-1-[3-(difluoromethyl)phenyl]-1H-pyrazolo[3,4-d]pyrimidin-4-yl}-4-fluoropyrrolidin-3-ol (70 mg) and trans-3-(methylsulfonyl)cyclobutanamine hydrochloride (45 mg) in NMP (1.5 ml) was treated with DIPEA (0.17 ml) and the mixture was stirred at 120°C for 23 h Standard workup and purification by HPLC (method HT_PREP_acid) afforded 55.5 mg of the title compound.
LC-MS (method HTpost_acid): Rₜ = 0.96 min; MS (ESIpos): m/z = 497 [M+H]⁺
¹H-NMR (400 MHz, DMSO-d6) δ [ppm]: 2.522 (1.75), 2.526 (1.28), 2.543 (1.25), 2.732 (0.75), 2.960 (16.00), 3.795 (0.48), 3.844 (0.62), 3.856 (0.62), 3.868 (0.79), 3.878 (0.65), 3.892 (0.42), 4.574 (0.43), 4.592 (0.41), 6.981 (0.67), 7.121 (1.38), 7.260 (0.61), 7.462 (0.98), 7.482 (1.21), 7.627 (0.94), 7.647 (1.55), 7.667 (0.78), 8.166 (1.00), 8.240 (3.64).

### Example II-17-1

### rel-(3R,4S)-1-(1-[3-(difluoromethyl)phenyl]-6-{[trans-3-(methylsulfonyl)cyclobutyl]-amino}-1H-pyrazolo[3,4-d]pyrimidin-4-yl)-4-methylpyrrolidin-3-ol

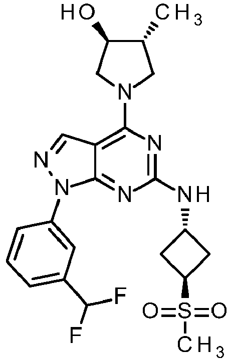

A solution of rel-(3R,4S)-1-{6-chloro-1-[3-(difluoromethyl)phenyl]-1H-pyrazolo[3,4-d]pyrimidin-4-yl}-4-methylpyrrolidin-3-ol (140 mg) and trans-3-(methylsulfonyl)cyclobutanamine hydrochloride (137 mg) in NMP (2.0 ml) was treated with DIPEA (0.39 ml) and the mixture was stirred at 130°C for 16 h. Standard workup and purification by HPLC (method HT_PREP _acid) afforded 122 mg of the title compound (pure diastereomer).
LC-MS (method HTpost_acid): Rₜ = 0.94 min; MS (ESIpos): m/z = 493 [M+H]⁺
¹H-NMR (400 MHz, DMSO-d6) δ [ppm]: 0.995 (1.62), 1.013 (1.77), 1.035 (2.70), 1.053 (2.57), 2.280 (0.44), 2.326 (0.56), 2.539 (0.56), 2.669 (0.69), 2.718 (1.16), 2.952 (16.00), 3.409 (0.48), 3.420 (0.51), 3.433 (0.57), 3.446 (0.64), 3.526 (0.48), 3.544 (0.47), 3.842 (1.10), 3.853 (1.11), 3.917 (0.58), 3.977 (0.46), 4.011 (0.88), 4.028 (1.28), 4.552 (0.42), 4.570 (0.75), 4.589 (0.70), 5.188 (0.57), 5.283 (0.69), 6.973 (0.89), 7.113 (1.84), 7.252 (0.85), 7.448 (1.40), 7.467 (1.60), 7.614 (1.22), 7.634 (2.06), 7.654 (1.02), 8.158 (1.84), 8.182 (2.19), 8.434 (0.41).

### Example II-17-2

### (3R*,4S*)-1-(1-[3-(difluoromethyl)phenyl]-6-{[trans-3-(methylsulfonyl)cyclobutyl]amino}-1H-pyrazolo[3,4-d]pyrimidin-4-yl)-4-methylpyrrolidin-3-ol (single enantiomer 1)

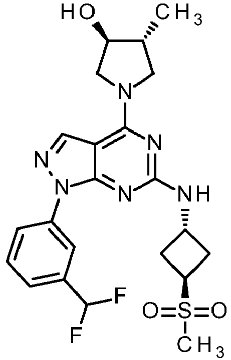

A sample of example II-17-1 was purified by chiral HPLC:
Preparative method: Instrument: PrepCon Labomatic HPLC; Column: Chiralpak IE 5µ, 250x30; eluent A: hexane + 0.1 vol % diethylamine; eluent B: ethanol + 0.1 vol % diethylamine; isocratic: 60%A+40%B; flow: 40 ml/min; temperature: 25°C; UV: 254 nm Analytical method: Instrument: Waters Alliance 2695; Column: Chiralpak IE 3µ, 100x4.6; eluent A: hexane + 0.1 vol % diethylamine; eluent B: ethanol; isocratic: 60%A+40%B; flow: 1.4 ml/min; temperature: 25°C; UV: 254 nm.
51 mg of isomer 1 with Rt = 6.14 min have been obtained after chiral HPLC.
LC-MS (method a01): Rₜ = 0.99 min; MS (ESIpos): m/z = 493 [M+H]⁺
¹H-NMR (400 MHz, DMSO-d6) δ [ppm]: 0.700 (0.68), 0.747 (0.60), 0.824 (0.55), 0.999 (2.03), 1.015 (2.20), 1.039 (3.19), 1.056 (3.10), 1.154 (1.72), 1.234 (1.96), 1.262 (0.73), 1.295 (0.51), 2.280 (0.46), 2.330 (0.88), 2.672 (1.08), 2.719 (1.40), 2.954 (16.00), 3.412 (0.72), 3.426 (0.73), 3.437 (0.77), 3.448 (0.82), 3.529 (0.59), 3.548 (0.59), 3.854 (1.31), 3.921 (0.68), 3.981 (0.50), 4.032 (1.46), 4.572 (0.84), 4.591 (0.84), 5.190 (0.72), 5.294 (0.84), 6.976 (0.96), 7.115 (1.92), 7.255 (0.89), 7.404 (0.44), 7.451 (1.54), 7.470 (1.80), 7.617 (1.27), 7.637 (2.16), 7.657 (1.10), 8.160 (1.87), 8.185 (2.22), 8.441 (0.50).

### Example II-17-3

### (3R*,4S*)-1-(1-[3-(difluoromethyl)phenyl]-6-{[trans-3-(methylsulfonyl)cyclobutyl]amino}-1H-pyrazolo[3,4-d]pyrimidin-4-yl)-4-methylpyrrolidin-3-ol (single enantiomer 2)

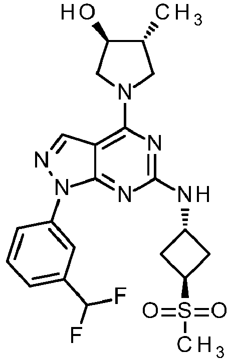

56 mg of isomer 2 with Rt = 4.93 min have been obtained after chrial HPLC (see example before).
LC-MS (method a01): Rₜ = 1.00 min; MS (ESIpos): m/z = 493 [M+H]⁺
¹H-NMR (400 MHz, DMSO-d6) δ [ppm]: 0.749 (0.49), 0.768 (0.50), 0.999 (1.59), 1.016 (1.73), 1.039 (2.69), 1.056 (2.60), 1.141 (1.32), 1.158 (1.50), 1.232 (0.94), 1.262 (0.41), 2.331 (0.50), 2.526 (2.07), 2.668 (0.48), 2.672 (0.66), 2.678 (0.57), 2.719 (1.05), 2.954 (16.00), 3.412 (0.45), 3.424 (0.47), 3.437 (0.52), 3.449 (0.59), 3.530 (0.43), 3.547 (0.45), 3.846 (1.00), 3.857 (1.01), 3.921 (0.52), 3.982 (0.41), 4.014 (0.78), 4.032 (1.17), 4.574 (0.67), 4.592 (0.65), 5.189 (0.56), 5.286 (0.68), 5.296 (0.66), 6.976 (0.86), 7.116 (1.77), 7.255 (0.80), 7.451 (1.26), 7.470 (1.47), 7.617 (1.16), 7.638 (1.95), 7.657 (0.98), 8.161 (1.75), 8.185 (2.08).

### Example II-18

### 2-(1-{6-[(4r,6s)-1-azaspiro[3.3]heptan-6-ylamino]-1-[3-(difluoromethyl)phenyl]-1H-pyrazolo[3,4-d]pyrimidin-4-yl}piperidin-4-yl)propan-2-ol

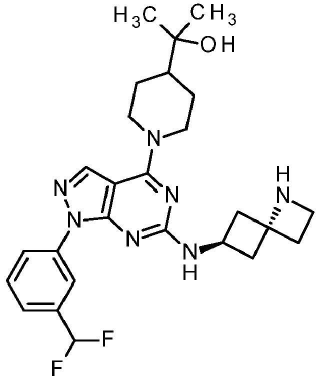

A solution of 2-(1-{6-chloro-1-[3-(difluoromethyl)phenyl]-1H-pyrazolo[3,4-d]pyrimidin-4-yl}-piperidin-4-yl)propan-2-ol (120 mg, 284 µmol) and tert-butyl (trans-4,6)-6-amino-1-azaspiro-[3.3]heptane-1-carboxylate (CAS 1638769-04-6) in NMP (3.1 ml) was treated with DIPEA (0.30 ml) and the mixture was stirred at 130°C for 16h. Standard workup led to a residue, which was dissolved in DCM (3.2 ml). TFA (0.66 ml) was added and the mixture was stirred for 16 h at rt and concentrated. Purification by HPLC (method HT_PREP_bas) afforded 53 mg of the title compound.
LC-MS (method HTpost_bas): Rₜ = 1.20 min; MS (ESIpos): m/z = 498 [M+H]⁺
¹H-NMR (400 MHz, DMSO-d6) δ [ppm]: 1.035 (0.70), 1.052 (12.38), 1.222 (0.48), 1.246 (0.49), 1.827 (0.67), 1.858 (0.61), 2.236 (0.73), 2.253 (0.88), 2.260 (1.30), 2.269 (1.07), 2.278 (1.94), 2.297 (0.89), 2.518 (1.74), 2.523 (1.21), 2.539 (16.00), 2.577 (0.56), 2.596 (0.67), 2.609 (0.57), 2.629 (0.48), 2.692 (0.43), 4.186 (1.46), 7.131 (0.63), 7.441 (0.63), 7.460 (0.72), 7.642 (0.53), 8.286 (2.95).

### Example II-19

### 1-[1-(1-[3-(difluoromethyl)phenyl]-6-{[trans-3-(methylsulfonyl)cyclobutyl]amino}-1H-pyrazolo[3,4-d]pyrimidin-4-yl)azetidin-3-yl]-2-methylpropan-2-ol

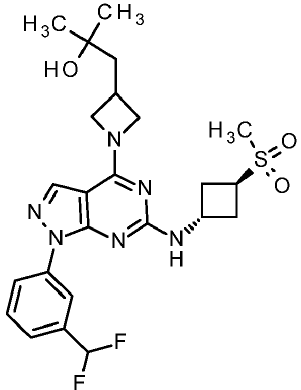

DIPEA (0.31 ml) was added to 1-(1-{6-chloro-1-[3-(difluoromethyl)phenyl]-1H-pyrazolo[3,4-d]-pyrimidin-4-yl}azetidin-3-yl)-2-methylpropan-2-ol (120 mg) and trans-3-(methylsulfonyl)cyclobutanamine hydrochloride (109 mg) in NMP (2.0 ml) and the mixture was stirred at 120°C for 17 h. Standard workup and purification by HPLC (method HT_PREP_acid) afforded 90 mg of the title compound.
LC-MS (method HTpost_acid): Rₜ = 0.98 min; MS (ESIpos): m/z = 521 [M+H]⁺
¹H-NMR (400 MHz, DMSO-d6) δ [ppm]: 1.103 (16.00), 1.762 (1.76), 1.781 (1.79), 2.451 (0.73), 2.463 (0.78), 2.518 (1.04), 2.522 (0.70), 2.539 (1.10), 2.669 (0.49), 2.673 (0.48), 2.678 (0.43), 2.711 (0.60), 2.944 (11.40), 3.043 (0.48), 3.823 (0.54), 3.835 (0.71), 3.846 (0.80), 3.858 (0.61), 4.262 (0.94), 4.523 (0.53), 4.541 (0.80), 4.560 (0.69), 6.965 (0.60), 7.104 (1.27), 7.243 (0.56), 7.445 (0.84), 7.464 (1.02), 7.610 (0.86), 7.630 (1.41), 7.650 (0.71), 8.014 (5.70), 8.178 (0.53).

### Example II-20

### 4-[4-(1-aminocyclopropyl)piperidin-1-yl]-1-[3-(difluoromethyl)phenyl]-N-[1-(methylsulfonyl)azetidin-3-yl]-1H-pyrazolo[3,4-d]pyrimidin-6-amine

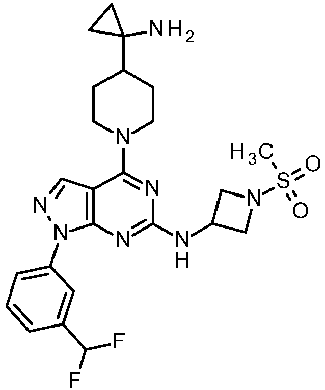

### Step a)

### tert-butyl {1-[1-(1-[3-(difluoromethyl)phenyl]-6-{[1-(methylsulfonyl)azetidin-3-yl]amino}-1H-pyrazolo[3,4-d]pyrimidin-4-yl)piperidin-4-yl]cyclopropyl}carbamate

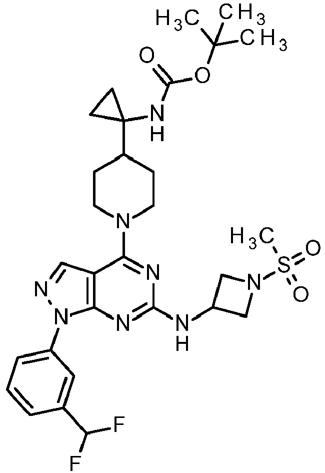

A solution of tert-butyl [1-(1-{6-chloro-1-[3-(difluoromethyl)phenyl]-1H-pyrazolo[3,4-d]-pyrimidin-4-yl}piperidin-4-yl)cyclopropyl]carbamate (80 mg) and 1-(methylsulfonyl)azetidin-3-amine (46 mg) in NMP (2.0 ml) was treated with DIPEA (0.16 ml) and the mixture was stirred at 130°C for 72 h. Standard workup and purification by HPLC afforded 70 mg tert-butyl {1-[1-(1-[3-(difluoromethyl)phenyl]-6-{[1-(methylsulfonyl)azetidin-3-yl]amino}-1H-pyrazolo[3,4-d]pyrimidin-4-yl)piperidin-4-yl]cyclopropyl}carbamate
LC-MS (method HTpost_bas): Rₜ = 1.33 min; MS (ESIpos): m/z = 633 [M+H]⁺

### Step b)

A solution of tert-butyl {1-[1-(1-[3-(difluoromethyl)phenyl]-6-{[1-(methylsulfonyl)azetidin-3-yl]amino}-1H-pyrazolo[3,4-d]pyrimidin-4-yl)piperidin-4-yl]cyclopropyl}carbamate (70 mg) in dichlormethane (1,3 ml) was treated with TFA (0.26 ml) and the mixture was stirred at RT for 16 h. The mixture was concentrated and the residue was purified by HPLC (method HT_PREP_bas) affording 16 mg 4-[4-(1-aminocyclopropyl)piperidin-1-yl]-1-[3-(difluoromethyl)phenyl]-N-[1-(methylsulfonyl)azetidin-3-yl]-1H-pyrazolo[3,4-d]pyrimidin-6-amine.
LC-MS (method HTpost_bas): Rₜ = 1.13 min; MS (ESIpos): m/z = 533 [M+H]⁺
¹H-NMR (400 MHz, DMSO-d6) δ [ppm]: 0.326 (0.69), 0.343 (2.81), 0.350 (2.06), 0.379 (2.00), 0.386 (2.95), 0.403 (0.70), 1.074 (0.49), 1.478 (0.69), 1.488 (0.72), 1.510 (0.76), 1.519 (0.72), 1.680 (1.15), 1.710 (0.80), 2.518 (1.86), 2.523 (1.25), 2.540 (2.49), 2.953 (0.68), 3.034 (16.00), 3.880 (0.88), 3.899 (1.41), 3.917 (1.07), 4.111 (0.72), 4.130 (1.30), 4.149 (0.66), 4.641 (0.59), 4.657 (0.59), 7.159 (0.57), 7.463 (0.93), 7.483 (1.09), 7.655 (0.51), 7.674 (0.66), 8.343 (4.56).

### Example II-21

### 1-[3-(difluoromethyl)phenyl]-4-[3-methyl-3-(morpholin-4-yl)azetidin-1-yl]-N-[trans-3-(methylsulfonyl)cyclobutyl]-1H-pyrazolo[3,4-d]pyrimidin-6-amine

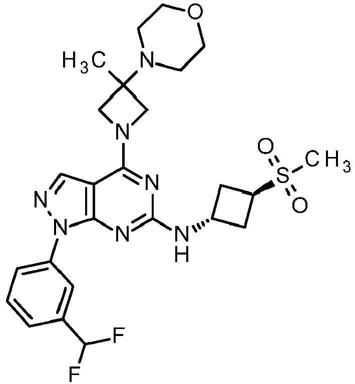

A solution of 6-chloro-1-[3-(difluoromethyl)phenyl]-4-[3-methyl-3-(morpholin-4-yl)azetidin-1-yl]-1H-pyrazolo[3,4-d]pyrimidine (90 mg) and trans-3-(methylsulfonyl)cyclobutanamine hydrochloride (77 mg) in NMP (1.9 ml) was treated with DIPEA (0.22 ml) and the mixture was stirred at 130°C for 16 h. Standard workup and purification by HPLC (method HT_PREP _bas) afforded 86 mg of the title compound.
LC-MS (method HTpost_bas): Rₜ = 1.06 min; MS (ESIpos): m/z = 548 [M+H]⁺
¹H-NMR (400 MHz, DMSO-d6) δ [ppm]: 0.000 (9.32), 1.334 (9.71), 2.077 (0.66), 2.331 (0.44), 2.458 (3.55), 2.521 (2.10), 2.526 (1.30), 2.667 (0.47), 2.672 (0.65), 2.677 (0.58), 2.682 (0.48), 2.718 (0.76), 2.951 (16.00), 3.619 (3.44), 3.818 (0.52), 3.831 (0.59), 3.842 (0.70), 3.854 (0.84), 3.866 (0.60), 3.878 (0.43), 3.957 (0.51), 3.978 (0.45), 4.051 (0.51), 4.071 (0.56), 4.234 (0.55), 4.253 (0.46), 4.547 (0.42), 4.566 (0.40), 6.972 (0.77), 7.111 (1.59), 7.251 (0.70), 7.454 (1.07), 7.473 (1.29), 7.618 (1.14), 7.638 (1.82), 7.658 (0.90), 8.053 (8.04).

### Example II-22

### (3S,4S)-1-(1-[3-(difluoromethyl)phenyl]-6-{[1-(methylsulfonyl)azetidin-3-yl]amino}-1H-pyrazolo[3,4-d]pyrimidin-4-yl)-4-fluoropyrrolidin-3-ol

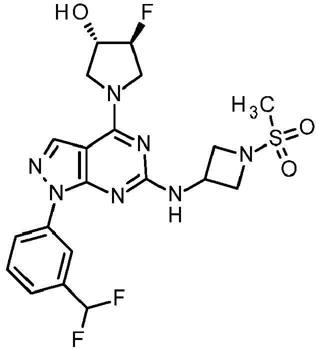

DIPEA (0.18 ml) was added to (3S,4S)-1-{6-chloro-1-[3-(difluoromethyl)phenyl]-1H-pyrazolo-[3,4-d]pyrimidin-4-yl}-4-fluoropyrrolidin-3-ol (71 mg) and 1-(methylsulfonyl)azetidin-3-amine (52 mg) in NMP (1.5 ml) and the mixture was stirred at 120°C for 16.5 h and 23h at 130°C for 23 h. Standard workup and purification by HPLC (method HT_PREP_acid) afforded 43 mg of the title compound.
LC-MS (method HTpost_acid): Rₜ = 1.00 min; MS (ESIpos): m/z = 498 [M+H]⁺
¹H-NMR (400 MHz, DMSO-d6) δ [ppm]: 2.521 (1.36), 2.526 (0.88), 2.543 (0.44), 3.039 (16.00), 3.802 (0.48), 3.846 (0.41), 3.898 (0.72), 3.916 (1.19), 3.934 (0.84), 4.013 (0.46), 4.127 (0.81), 4.146 (1.47), 4.167 (0.78), 4.668 (0.45), 4.683 (0.44), 7.164 (0.47), 7.467 (0.92), 7.486 (1.11), 7.681 (0.65), 8.266 (3.20).

### Example II-23

### (3R,4R)-1-(1-[3-(difluoromethyl)phenyl]-6-{[1-(methylsulfonyl)azetidin-3-yl]amino}-1H-pyrazolo[3,4-d]pyrimidin-4-yl)-4-fluoropyrrolidin-3-ol

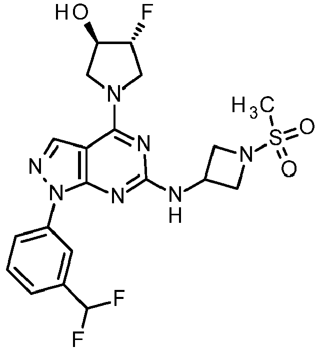

DIPEA (0.17 ml) was added to (3R,4R)-1-{6-chloro-1-[3-(difluoromethyl)phenyl]-1H-pyrazolo-[3,4-d]pyrimidin-4-yl}-4-fluoropyrrolidin-3-ol (70 mg) and 1-(methylsulfonyl)azetidin-3-amine (49 mg) in NMP (1.5 ml) and the mixture was stirred at 120°C for 16 h and 23 h at 130°C for 23 h. Standard workup and purification by HPLC (method HT_PREP_acid) afforded 42 mg of the title compound.
LC-MS (method HTpost_acid): Rₜ = 1.00 min; MS (ESIpos): m/z = 498 [M+H]+
¹H-NMR (400 MHz, DMSO-d6) δ [ppm]: -0.008 (0.48), 0.008 (0.49), 2.521 (1.61), 2.525 (1.05), 2.542 (0.51), 3.038 (16.00), 3.775 (0.41), 3.803 (0.50), 3.846 (0.42), 3.878 (0.41), 3.899 (0.73), 3.916 (1.22), 3.933 (0.86), 4.011 (0.47), 4.126 (0.83), 4.146 (1.50), 4.166 (0.79), 4.666 (0.46), 4.682 (0.46), 5.657 (0.44), 5.665 (0.45), 5.766 (0.52), 5.776 (0.50), 7.163 (0.48), 7.467 (0.94), 7.486 (1.13), 7.681 (0.67), 8.266 (3.22).

### Example II-24

### cis-3-({1-[3-(difluoromethyl)phenyl]-4-[3-(morpholin-4-yl)azetidin-1-yl]-1H-pyrazolo[3,4-d]pyrimidin-6-yl}amino)cyclobutanol

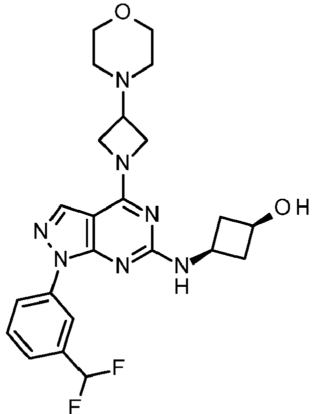

A solution of 6-chloro-1-[3-(difluoromethyl)phenyl]-4-[3-(morpholin-4-yl)azetidin-1-yl]-1H-pyrazolo[3,4-d]pyrimidine (100 mg) and cis-3-aminocyclobutanol hydrochloride (59 mg) in NMP (2.0 ml) was treated with DIPEA (0.25 ml) and the mixture was stirred at 130°C for 40 h. Standard workup and purification by HPLC (method HT_PREP_bas) afforded 80 mg of the title compound.
LC-MS (method HTpost_bas): Rₜ = 1.00 min; MS (ESIpos): m/z = 472 [M+H]⁺
¹H-NMR (400 MHz, DMSO-d6) δ [ppm]: 1.791 (1.40), 1.798 (1.05), 1.813 (3.01), 1.819 (2.95), 1.834 (2.94), 1.840 (3.17), 1.855 (1.10), 1.862 (1.48), 2.078 (1.29), 2.326 (0.60), 2.331 (0.81), 2.336 (0.66), 2.402 (6.28), 2.522 (2.89), 2.526 (1.70), 2.619 (2.01), 2.668 (0.84), 2.673 (0.92), 2.678 (0.69), 3.326 (1.80), 3.363 (1.51), 3.381 (0.70), 3.616 (6.32), 3.626 (9.75), 3.636 (6.69), 3.842 (1.70), 3.973 (0.68), 4.178 (0.87), 4.241 (0.93), 4.405 (0.80), 5.027 (2.37), 5.041 (2.25), 6.996 (1.25), 7.136 (2.56), 7.179 (0.47), 7.276 (1.24), 7.354 (0.86), 7.427 (2.73), 7.446 (3.24), 7.622 (1.54), 7.643 (2.62), 7.662 (1.30), 8.034 (16.00), 8.381 (1.94), 8.403 (2.08), 8.753 (1.02).

### Example II-25

### trans-3-({1-[3-(difluoromethyl)phenyl]-4-[3-(morpholin-4-yl)azetidin-1-yl]-1H-pyrazolo-[3,4-d]pyrimidin-6-yl}amino)cyclobutanol

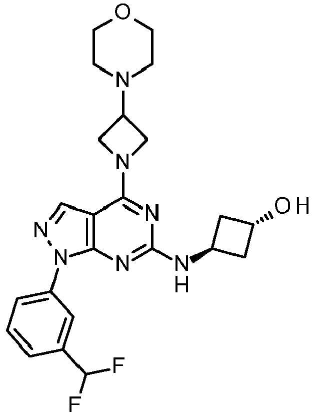

DIPEA (0.25 ml) was added to 6-chloro-1-[3-(difluoromethyl)phenyl]-4-[3-(morpholin-4-yl)-azetidin-1-yl]-1H-pyrazolo[3,4-d]pyrimidine (100 mg) and trans-3-aminocyclobutanol hydrochloride (59 mg) in NMP (2.0 ml) and the mixture was stirred at 130°C for 40 h. Water and ethyl acetate was added to the reaction mixture. The precipitated solid was collected by filtration and washed with water and 2-methoxy-2-methylpropane. The solid was dried in a vacuum oven (15 mbar, 30°C) to afford 75 mg of the title compound.
LC-MS (method b02): Rₜ = 1.03 min; MS (ESIpos): m/z = 472 [M+H]⁺
¹H-NMR (400 MHz, DMSO-d6) δ [ppm]: 1.106 (0.60), 1.157 (0.82), 1.175 (1.57), 1.193 (0.77), 1.991 (3.18), 2.176 (2.08), 2.268 (1.98), 2.325 (0.98), 2.330 (1.23), 2.335 (0.98), 2.405 (6.30), 2.521 (3.76), 2.526 (2.44), 2.667 (0.74), 2.672 (1.02), 2.677 (0.74), 3.132 (0.48), 3.357 (2.49), 3.369 (1.44), 3.374 (1.41), 3.386 (0.56), 3.616 (6.25), 3.627 (9.51), 3.637 (6.36), 3.980 (0.67), 4.002 (0.71), 4.020 (1.02), 4.038 (0.90), 4.267 (1.58), 4.284 (1.90), 4.298 (2.27), 4.313 (1.96), 4.411 (0.81), 4.962 (2.37), 4.976 (2.03), 6.982 (2.82), 7.122 (5.99), 7.262 (2.64), 7.382 (0.85), 7.429 (2.88), 7.448 (3.34), 7.617 (1.98), 7.638 (3.43), 7.657 (1.76), 8.040 (16.00), 8.052 (0.45), 8.442 (0.90), 8.663 (0.88).

### Example II-26

### 2-(1-{1-[3-(difluoromethyl)phenyl]-6-[(1,1-dioxidotetrahydro-2H-thiopyran-4-yl)amino]-1H-pyrazolo[3,4-d]pyrimidin-4-yl}azetidin-3-yl)-2-methylpropanoic acid

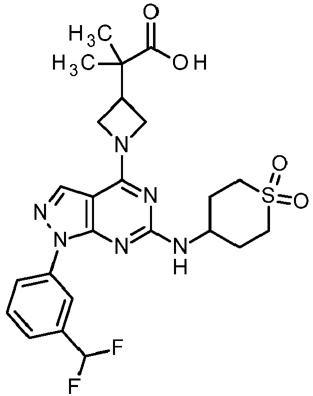

DIPEA (0.29 ml) was added to methyl 2-(1-{6-chloro-1-[3-(difluoromethyl)phenyl]-1H-pyrazolo[3,4-d]pyrimidin-4-yl}azetidin-3-yl)-2-methylpropanoate (120 mg) and tetrahydro-2H-thiopyran-4-amine 1,1-dioxide hydrochloride (102 mg) in NMP (2.0 ml) and the mixture was stirred for 120 h at 130°C. Aqueous sodium hydroxide solution (2M, 1 ml) was added and the mixture was stirred at 80°C for 16 h. The mixture was treated with aqueous citric acid solution (10%) and ethyl acetate and stirred for 5 min. The mixture was extracted with ethyl acetate three times, the organic layers were concentrated. Purification by HPLC (method HT_PREP _acid) afforded 5 mg of the title compound.
LC-MS (method HTpost_acid): Rₜ = 1.03 min; MS (ESIpos): m/z = 535 [M+H]⁺
¹H-NMR (400 MHz, DMSO-d6) δ [ppm]: 1.160 (16.00), 1.211 (0.41), 2.053 (0.74), 2.078 (0.86), 2.239 (0.75), 2.324 (0.70), 2.329 (0.98), 2.334 (0.69), 2.520 (2.84), 2.525 (2.12), 2.542 (0.59), 2.667 (0.71), 2.671 (0.98), 2.676 (0.69), 3.060 (0.42), 3.074 (0.60), 3.090 (0.43), 3.150 (0.58), 3.184 (1.46), 3.198 (1.42), 3.233 (1.10), 3.271 (0.60), 3.988 (0.43), 4.132 (0.65), 4.256 (0.50), 4.412 (0.52), 7.161 (0.71), 7.445 (1.09), 7.464 (1.24), 7.644 (0.48), 7.663 (0.75), 8.075 (7.03).

### Example II-27

### 4-(1-{1-[3-(difluoromethyl)phenyl]-6-[(trans-3-hydroxycyclobutyl)amino]-1H-pyrazolo[3,4-d]pyrimidin-4-yl}azetidin-3-yl)piperazin-2-one

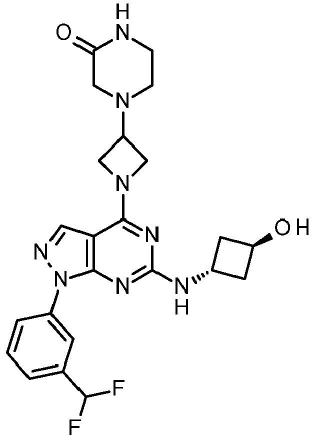

A solution of 4-(1-{6-chloro-1-[3-(difluoromethyl)phenyl]-1H-pyrazolo[3,4-d]pyrimidin-4-yl}-azetidin-3-yl)piperazin-2-one (80 mg) and trans-3-aminocyclobutanol hydrochloride (46 mg in NMP (2.0 ml) was treated with DIPEA (0.19 ml) and the mixture was stirred at 130°C for 72 h. Standard workup and purification by HPLC (method HT_PREP_bas) afforded 43 mg of the title compound.)
LC-MS (method HTpost_bas): Rₜ = 0.86 min; MS (ESIpos): m/z = 485 [M+H]⁺
¹H-NMR (400 MHz, DMSO-d6) δ [ppm]: 2.176 (2.08), 2.269 (1.98), 2.334 (1.07), 2.339 (0.53), 2.521 (4.32), 2.526 (2.83), 2.542 (1.54), 2.606 (2.24), 2.676 (1.09), 2.681 (0.53), 3.011 (5.33), 3.205 (3.95), 3.452 (0.48), 3.470 (1.20), 3.482 (1.91), 3.494 (1.23), 3.499 (1.20), 3.511 (0.53), 3.994 (0.74), 4.284 (2.22), 4.299 (2.83), 4.313 (2.48), 4.434 (0.95), 4.963 (2.29), 4.976 (2.01), 6.983 (2.79), 7.122 (5.96), 7.262 (2.56), 7.398 (0.83), 7.430 (2.88), 7.449 (3.23), 7.618 (1.92), 7.637 (3.26), 7.658 (1.61), 7.829 (4.05), 8.036 (16.00), 8.376 (0.53), 8.407 (0.82), 8.430 (0.88), 8.663 (0.91).

### Example II-28

### 1-[1-(1-[3-(difluoromethyl)phenyl]-6-{[1-(ethylsulfonyl)azetidin-3-yl]amino}-1H-pyrazolo[3,4-d]pyrimidin-4-yl)azetidin-3-yl]-2-methylpropan-2-ol

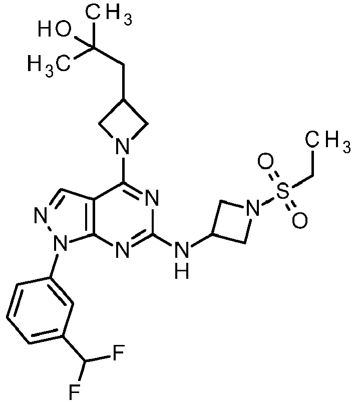

A solution of 1-(1-{6-chloro-1-[3-(difluoromethyl)phenyl]-1H-pyrazolo[3,4-d]pyrimidin-4-yl}-azetidin-3-yl)-2-methylpropan-2-ol (80 mg) and 1-(ethylsulfonyl)azetidin-3-amine trifluoroacetate in NMP (2.2 ml) was treated with DIPEA (0.20 ml) and the mixture was stirred at 130°C for 16 h. Standard workup and purification by HPLC (method HT_PREP_acid) afforded 27 mg of the title compound.
LC-MS (method HTpost_acid): Rₜ = 1.10 min; MS (ESIpos): m/z = 536 [M+H]⁺
¹H-NMR (400 MHz, DMSO-d6) δ [ppm]: 1.108 (16.00), 1.224 (3.51), 1.243 (7.96), 1.261 (3.68), 1.769 (2.06), 1.786 (2.10), 2.521 (1.32), 2.526 (0.88), 2.543 (0.84), 3.053 (0.50), 3.123 (1.16), 3.141 (3.46), 3.160 (3.40), 3.178 (0.98), 3.893 (0.93), 3.912 (1.53), 3.930 (1.09), 4.090 (0.74), 4.263 (3.41), 4.604 (0.47), 4.621 (0.44), 7.154 (0.70), 7.450 (0.89), 7.469 (1.07), 7.648 (0.42), 7.667 (0.68), 8.042 (6.22).

### Example II-29

### (3R)-1-(1-[3-(difluoromethyl)phenyl]-6-{[1-(methylsulfonyl)azetidin-3-yl]amino}-1H-pyrazolo[3,4-d]pyrimidin-4-yl)-3-methylpyrrolidin-3-ol

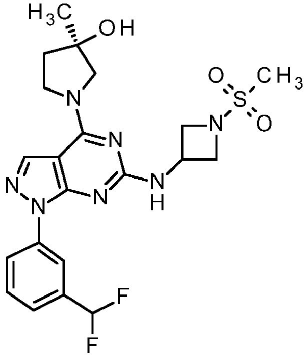

A solution of (3R)-1-{6-chloro-1-[3-(difluoromethyl)phenyl]-1H-pyrazolo[3,4-d]pyrimidin-4-yl}-3-methylpyrrolidin-3-ol (100 mg) and 1-(methanesulfonyl)azetidin-3-amine (79 mg) in NMP (2.0 ml) was treated with DIPEA (0.28 ml) and the mixture was stirred at 130°C for 16 h. Standard workup and purification by HPLC (method HT_PREP_bas) afforded 44 mg of the title compound.
LC-MS (method HTpost_bas): Rₜ = 1.05 min; MS (ESIpos): m/z = 494 [M+H]⁺
¹H-NMR (400 MHz, DMSO-d6) δ [ppm]: 1.379 (2.68), 1.424 (3.42), 2.027 (0.72), 2.046 (0.54), 2.521 (1.29), 2.526 (0.89), 3.036 (16.00), 3.415 (0.48), 3.677 (1.68), 3.713 (0.49), 3.888 (1.13), 3.905 (1.14), 4.115 (0.67), 4.134 (1.15), 4.153 (0.60), 4.655 (0.47), 4.671 (0.46), 4.907 (1.55), 4.971 (1.59), 7.160 (0.56), 7.455 (0.97), 7.474 (1.14), 7.652 (0.40), 7.671 (0.58), 8.177 (1.72), 8.215 (2.21).

### Example II-30

### 2-[1-(6-{[1-(cyclopropylsulfonyl)azetidin-3-yl]amino}-1-[3-(difluoromethyl)phenyl]-1H-pyrazolo[3,4-d]pyrimidin-4-yl)piperidin-4-yl]propan-2-ol

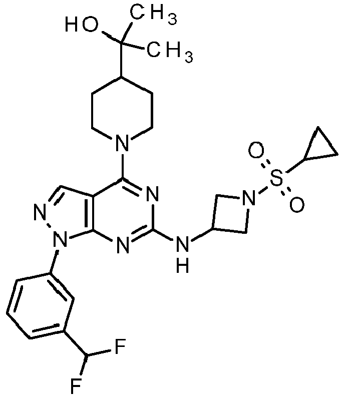

DIPEA (0.24 ml) was added to 2-(1-{6-chloro-1-[3-(difluoromethyl)phenyl]-1H-pyrazolo[3,4-d]-pyrimidin-4-yl}piperidin-4-yl)propan-2-ol (80 mg) and 1-(cyclopropylsulfonyl)azetidin-3-amine trifluoroacetate in NMP (1.5 ml) and the mixture was stirred for 42 h at 130°C. Standard workup and purification by HPLC (method HT_PREP_acid) afforded 25 mg of the title compound.
LC-MS (method HTpost_acid): Rₜ = 1.19 min; MS (ESIpos): m/z = 562 [M+H]⁺
¹H-NMR (400 MHz, DMSO-d6) δ [ppm]: 0.930 (1.33), 0.935 (1.34), 0.942 (1.26), 0.947 (1.56), 0.957 (0.54), 1.027 (0.56), 1.038 (1.51), 1.047 (1.88), 1.057 (16.00), 1.073 (1.05), 1.238 (0.57), 1.261 (0.60), 1.269 (0.58), 1.569 (0.47), 1.845 (0.72), 1.876 (0.66), 2.521 (2.01), 2.526 (1.24), 2.745 (0.48), 2.752 (0.53), 2.764 (0.96), 2.776 (0.49), 2.784 (0.46), 3.949 (0.96), 3.968 (1.58), 3.985 (1.17), 4.142 (0.80), 4.193 (3.02), 7.154 (0.59), 7.463 (0.81), 7.482 (0.95), 7.652 (0.66), 7.670 (0.82), 8.339 (3.93).

### Example II-31

### 2-[1-(1-[3-(difluoromethyl)phenyl]-6-{[1-(ethylsulfonyl)azetidin-3-yl]amino}-1H-pyrazolo[3,4-d]pyrimidin-4-yl)piperidin-4-yl]propan-2-ol

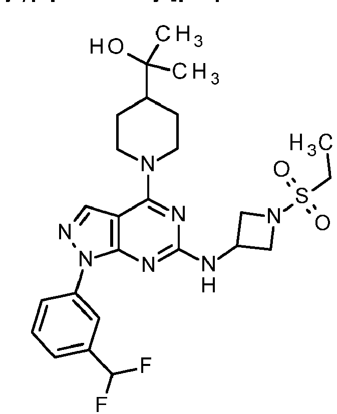

Reaction of 2-(1-{6-chloro-1-[3-(difluoromethyl)phenyl]-1H-pyrazolo[3,4-d]pyrimidin-4-yl}-piperidin-4-yl)propan-2-ol (80 mg) and 1-(ethylsulfonyl)azetidin-3-amine trifluoroacetate (70 mg) using conditions as described for the example II-30 afforded 25 mg of the title compound. LC-MS (method HTpost_acid): Rₜ = 1.18 min; MS (ESIpos): m/z = 550 [M+H]⁺
¹H-NMR (400 MHz, DMSO-d6) δ [ppm]: 1.057 (16.00), 1.227 (3.90), 1.245 (8.03), 1.264 (4.07), 1.567 (0.51), 1.846 (0.79), 1.876 (0.71), 2.521 (1.27), 2.526 (0.75), 3.129 (1.15), 3.147 (3.62), 3.166 (3.55), 3.184 (1.04), 3.901 (1.09), 3.920 (1.70), 3.937 (1.33), 4.091 (0.62), 4.108 (1.08), 4.127 (0.56), 4.192 (4.47), 4.618 (0.45), 4.633 (0.44), 7.154 (0.66), 7.462 (0.84), 7.481 (1.02), 7.650 (0.70), 7.671 (0.77), 8.337 (4.16).

### Example II-32

### 1-[3-(difluoromethyl)phenyl]-N-[1-(ethylsulfonyl)azetidin-3-yl]-4-[3-(morpholin-4-yl)azetidin-1-yl]-1H-pyrazolo[3,4-d]pyrimidin-6-amine

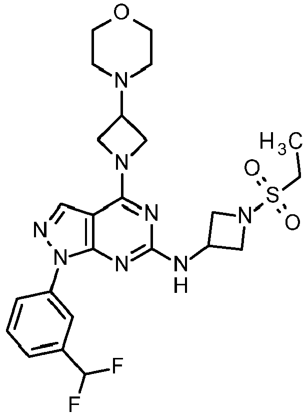

Reaction of 6-chloro-1-[3-(difluoromethyl)phenyl]-4-[3-(morpholin-4-yl)azetidin-1-yl]-1H-pyrazolo[3,4-d]pyrimidine (80 mg) and 1-(ethylsulfonyl)azetidin-3-amine trifluoroacetate (79 mg) using conditions as described for example II-30 afforded 11 mg of the title compound. LC-MS (method HTpost_acid): Rₜ = 0.87 min; MS (ESIpos): m/z = 549 [M+H]⁺
¹H-NMR (400 MHz, DMSO-d6) δ [ppm]: 1.224 (7.16), 1.242 (16.00), 1.260 (7.48), 1.306 (0.75), 2.410 (4.59), 2.521 (5.95), 2.525 (3.68), 3.122 (2.23), 3.140 (7.05), 3.159 (6.85), 3.177 (2.09), 3.370 (2.65), 3.586 (0.51), 3.629 (7.07), 3.898 (2.15), 3.917 (3.67), 3.934 (2.73), 4.007 (0.71), 4.108 (1.70), 4.197 (0.84), 4.284 (0.79), 4.439 (0.73), 4.589 (0.64), 4.605 (1.15), 4.621 (1.09), 4.637 (0.59), 7.014 (0.72), 7.154 (1.46), 7.294 (0.69), 7.455 (1.96), 7.474 (2.41), 7.651 (0.92), 7.671 (1.48), 7.690 (0.85), 7.815 (0.47), 8.087 (10.74), 8.097 (0.52), 8.374 (0.76), 8.514 (0.80), 8.562 (0.51).

### Example II-33

### (3R)-1-(1-[3-(difluoromethyl)phenyl]-6-{[(1r,3R)-3-hydroxycyclobutyl]amino}-1H-pyrazolo[3,4-d]pyrimidin-4-yl)pyrrolidin-3-ol

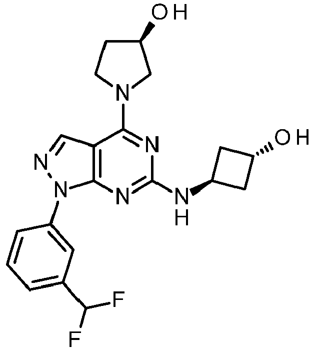

Reaction of (3R)-1-{6-chloro-1-[3-(difluoromethyl)phenyl]-1H-pyrazolo[3,4-d]pyrimidin-4-yl}pyrrolidin-3-ol (80 mg) and trans-3-aminocyclobutanol hydrochloride (54 mg) using conditions as described for example II-30 afforded 56 mg of the title compound.
LC-MS (method HTpost_bas): Rₜ = 0.96 min; MS (ESIpos): m/z = 417 [M+H]⁺
¹H-NMR (400 MHz, DMSO-d6) δ [ppm]: 1.906 (1.05), 2.000 (1.48), 2.034 (1.50), 2.078 (16.00), 2.120 (1.25), 2.183 (3.93), 2.270 (3.64), 2.321 (1.25), 2.326 (1.60), 2.331 (1.87), 2.335 (1.29), 2.522 (5.43), 2.526 (3.57), 2.668 (1.04), 2.672 (1.48), 2.678 (1.02), 3.588 (2.08), 3.612 (3.14), 3.661 (2.62), 3.692 (1.18), 3.785 (1.20), 3.815 (2.85), 3.828 (2.97), 3.837 (2.92), 3.849 (2.20), 3.865 (1.36), 3.877 (1.37), 3.893 (1.07), 4.309 (3.33), 4.323 (3.03), 4.377 (2.33), 4.502 (1.68), 4.961 (5.42), 4.974 (4.90), 5.025 (2.97), 5.032 (2.96), 5.134 (2.39), 5.142 (2.22), 6.983 (5.56), 7.123 (12.05), 7.181 (1.58), 7.263 (4.99), 7.428 (5.19), 7.448 (6.16), 7.617 (3.68), 7.636 (6.32), 7.656 (3.14), 8.130 (4.53), 8.173 (6.03), 8.463 (1.23), 8.542 (0.92), 8.701 (0.94).

### Example II-34

### (3R)-1-(1-[3-(difluoromethyl)phenyl]-6-{[(1r,3R)-3-(methylsulfonyl)cyclobutyl]amino}-1H-pyrazolo[3,4-d]pyrimidin-4-yl)pyrrolidin-3-ol

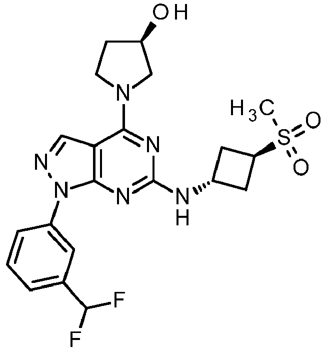

Reaction of (3R)-1-{6-chloro-1-[3-(difluoromethyl)phenyl]-1H-pyrazolo[3,4-d]pyrimidin-4-yl}pyrrolidin-3-ol (80 mg) and trans-3-(methylsulfonyl)cyclobutanamine (65 mg) using conditions as described for example II-30 afforded 40 mg of the title compound after HPLC (method HT_PREP_bas).
LC-MS (method HTpost_bas): Rₜ = 0.98 min; MS (ESIpos): m/z = 479 [M+H]⁺
¹H-NMR (400 MHz, DMSO-d6) δ [ppm]: 2.521 (1.56), 2.526 (1.12), 2.535 (0.60), 2.672 (0.40), 2.722 (0.78), 2.955 (16.00), 3.597 (0.49), 3.622 (0.71), 3.659 (0.54), 3.825 (0.75), 3.835 (0.93), 3.846 (1.14), 3.858 (1.15), 3.868 (0.73), 3.881 (0.58), 4.389 (0.41), 4.574 (0.61), 4.593 (0.61), 5.046 (0.53), 5.143 (0.63), 5.151 (0.61), 6.977 (0.80), 7.116 (1.64), 7.256 (0.74), 7.451 (1.05), 7.470 (1.23), 7.618 (1.05), 7.638 (1.75), 7.658 (0.87), 8.158 (1.25), 8.199 (1.66).

### Example II-35

### (3R)-1-(1-[3-(difluoromethyl)phenyl]-6-{[(1r,3R)-3-(methylsulfonyl)cyclobutyl]amino}-1H-pyrazolo[3,4-d]pyrimidin-4-yl)-3-methylpyrrolidin-3-ol

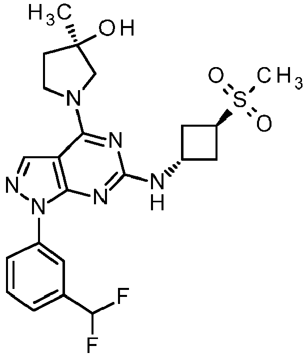

Reaction of (3R)-1-{6-chloro-1-[3-(difluoromethyl)phenyl]-1H-pyrazolo[3,4-d]pyrimidin-4-yl}-3-methylpyrrolidin-3-ol (100 mg) and trans-3-(methylsulfonyl)cyclobutanamine hydrochloride (98 mg) using conditions as described for example II-30 afforded 73 mg of the title compound after HPLC (method HT_PREP_bas).
LC-MS (method HTpost_bas): Rₜ = 1.01 min; MS (ESIpos): m/z = 493 [M+H]⁺
¹H-NMR (400 MHz, DMSO-d6) δ [ppm]: 1.376 (2.61), 1.421 (3.42), 2.022 (0.74), 2.041 (0.54), 2.521 (1.88), 2.526 (1.30), 2.542 (0.40), 2.672 (0.55), 2.676 (0.49), 2.681 (0.42), 2.720 (0.82), 2.955 (16.00), 2.987 (0.53), 3.669 (1.83), 3.702 (0.46), 3.834 (0.54), 3.846 (0.88), 3.857 (1.23), 3.869 (0.96), 3.878 (0.87), 4.570 (0.58), 4.589 (0.55), 4.912 (1.25), 4.967 (1.39), 6.976 (0.71), 7.116 (1.48), 7.255 (0.67), 7.450 (1.02), 7.469 (1.19), 7.616 (1.00), 7.636 (1.66), 7.656 (0.84), 8.149 (1.75), 8.187 (2.17).

### Example II-36

### 1-(1-{6-[(4r,6s)-1-azaspiro[3.3]heptan-6-ylamino]-1-[3-(difluoromethyl)phenyl]-1H-pyrazolo[3,4-d]pyrimidin-4-yl}azetidin-3-yl)-2-methylpropan-2-ol

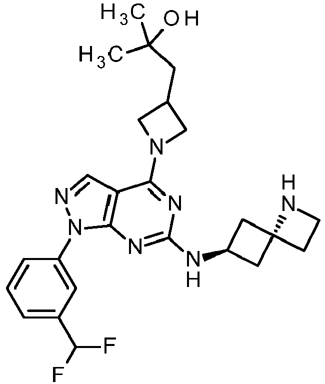

### Step a)

### tert-butyl (4r,6s)-6-({1-[3-(difluoromethyl)phenyl]-4-[3-(2-hydroxy-2-methylpropyl)-azetidin-1-yl]-1H-pyrazolo[3,4-d]pyrimidin-6-yl}amino)-1-azaspiro[3.3]heptane-1-carboxylate

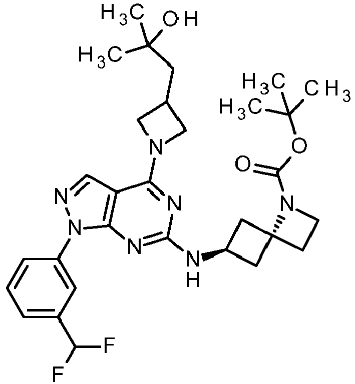

A solution of 1-(1-{6-chloro-1-[3-(difluoromethyl)phenyl]-1H-pyrazolo[3,4-d]pyrimidin-4-yl}-azetidin-3-yl)-2-methylpropan-2-ol (80 mg) and tert-butyl (4r,6s)-6-amino-1-azaspiro[3.3]heptane-1-carboxylate (71 mg) (CAS number 1638769-04-6) in NMP (2.2 ml) was treated with DIPEA (0.20 ml) and the mixture was stirred at 130°C for 16 h.Standard workup and purification by HPLC (method HT_PREP_bas) afforded 50 mg of tert-butyl (4r,6s)-6-({1-[3-(difluoromethyl)phenyl]-4-[3-(2-hydroxy-2-methylpropyl)azetidin-1-yl]-1H-pyrazolo[3,4-d]pyrimidin-6-yl}amino)-1-azaspiro[3.3]heptane-1-carboxylate.
LC-MS (method HTpost_bas): Rₜ = 1.38 min; MS (ESIpos): m/z = 584 [M+H]⁺

### Step b)

A solution of tert-butyl (4r,6s)-6-({1-[3-(difluoromethyl)phenyl]-4-[3-(2-hydroxy-2-methylpropyl)azetidin-1-yl]-1H-pyrazolo[3,4-d]pyrimidin-6-yl}amino)-1-azaspiro[3.3]heptane-1-carboxylate in dichlormethane (0.24 ml) was treated with TFA (0.12 ml) and the mixture was stirred at rt for 78 h. The mixture was concentrated and purified by HPLC (method HT_PREP _bas) to afford 20 mg 1-[1-(6-{[(4r,6s)-1-azaspiro[3.3]heptan-6-yl]amino}-1-[3-(difluoromethyl)phenyl]-1H-pyrazolo[3,4-d]pyrimidin-4-yl)azetidin-3-yl]-2-methylpropan-2-ol
LC-MS (method HTpost_bas): Rₜ = 1.14 min; MS (ESIpos): m/z = 484 [M+H]⁺
¹H-NMR (400 MHz, DMSO-d6) δ [ppm]: 1.103 (16.00), 1.759 (1.94), 1.777 (1.97), 2.194 (1.22), 2.211 (2.24), 2.229 (1.63), 2.453 (0.64), 2.521 (1.09), 2.526 (0.72), 2.543 (1.07), 3.035 (0.47), 3.254 (1.17), 3.272 (2.07), 3.290 (1.30), 4.258 (1.11), 6.990 (0.42), 7.130 (0.90), 7.269 (0.49), 7.424 (0.84), 7.443 (0.99), 7.619 (0.44), 7.639 (0.76), 7.990 (5.48).

### Example III-1

### (4S)-4-({4-[4-(2-hydroxypropan-2-yl)piperidin-1-yl]-1-[3-(trifluoromethoxy)phenyl]-1H-pyrazolo[3,4-d]pyrimidin-6-yl}amino)pyrrolidin-2-one

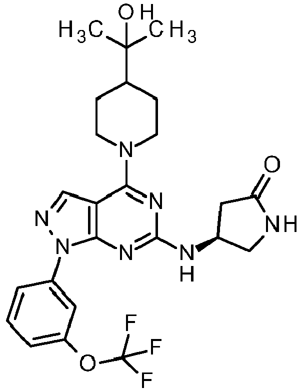

DIPEA (0.23 ml) was added to 2-(1-{6-chloro-1-[3-(trifluoromethoxy)phenyl]-1H-pyrazolo[3,4-d]pyrimidin-4-yl}piperidin-4-yl)propan-2-ol (100 mg, 0.22 mmol) and (4S)-4-aminopyrrolidin-2-one hydrochloride (59.9 mg, 0.44 mmol) in NMP (1.5 ml) and the mixture was stirred for 20 h at 130°C. Standard workup and purification by HPLC (method HT_PREP_acid) afforded 75.3 mg of the title compound.
LC-MS (method HTpost_acid): Rₜ = 1.14 min; MS (ESIpos): m/z = 520 [M+H]⁺
¹H-NMR (400 MHz, DMSO-d6) δ [ppm]: 1.050 (16.00), 1.227 (0.50), 1.251 (0.54), 1.560 (0.43), 1.832 (0.72), 1.862 (0.65), 2.258 (0.74), 2.274 (0.74), 2.300 (1.01), 2.316 (1.04), 2.323 (0.44), 2.327 (0.55), 2.518 (2.17), 2.523 (1.52), 2.527 (0.83), 2.549 (0.54), 2.669 (0.52), 3.192 (0.41), 3.204 (0.43), 3.582 (0.61), 3.600 (0.76), 3.605 (0.68), 3.624 (0.56), 4.183 (5.43), 4.556 (0.48), 4.572 (0.48), 7.247 (0.63), 7.268 (0.70), 7.353 (0.54), 7.368 (0.51), 7.614 (0.40), 7.634 (1.87), 8.328 (4.44).

### Example IV-1

### (4S)-4-({1-[3-(difluoromethoxy)phenyl]-4-[4-(2-hydroxypropan-2-yl)piperidin-1-yl]-1H-pyrazolo[3,4-d]pyrimidin-6-yl}amino)pyrrolidin-2-one

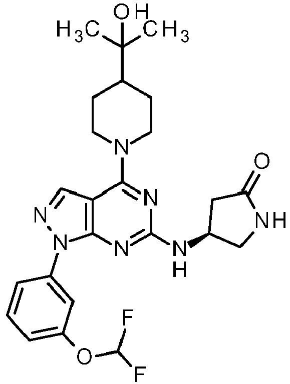

DIPEA (0.24 ml) was added to 2-(1-{6-chloro-1-[3-(difluoromethoxy)phenyl]-1H-pyrazolo[3,4-d]pyrimidin-4-yl}piperidin-4-yl)propan-2-ol (100 mg, 0.23 mmol) and (4S)-4-aminopyrrolidin-2-one hydrochloride (62.4 mg, 0.46 mmol) in NMP (1.5 ml) and the mixture was stirred for 20 h at 130°C. Standard workup and purification by HPLC (method HT_PREP_acid) afforded 68.9 mg of the title compound.
LC-MS (method HTpost_acid): Rₜ = 1.02 min; MS (ESIpos): m/z = 502 [M+H]⁺
¹H-NMR (400 MHz, DMSO-d6) δ [ppm]: 1.049 (16.00), 1.227 (0.49), 1.251 (0.53), 1.558 (0.42), 1.830 (0.71), 1.861 (0.66), 2.074 (2.02), 2.253 (0.69), 2.269 (0.71), 2.295 (0.96), 2.311 (0.99), 2.518 (1.22), 2.522 (0.85), 2.529 (0.47), 2.539 (2.58), 3.186 (0.41), 3.198 (0.43), 3.608 (0.54), 4.181 (1.28), 4.568 (0.48), 4.584 (0.47), 7.054 (0.68), 7.060 (0.69), 7.074 (0.75), 7.080 (0.76), 7.131 (0.57), 7.316 (1.85), 7.333 (0.80), 7.501 (0.56), 7.542 (0.63), 7.621 (1.61), 8.165 (0.41), 8.305 (4.09).

### Example IV-2

### 2-[1-(1-[3-(difluoromethoxy)phenyl]-6-{[trans-3-(methylsulfonyl)cyclobutyl]amino}-1H-pyrazolo[3,4-d]pyrimidin-4-yl)piperidin-4-yl]propan-2-ol

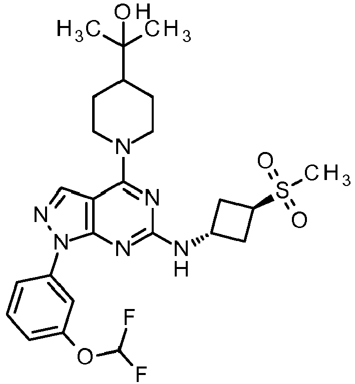

DIPEA (0.14 ml) was added to 2-[1-(1-[3-(difluoromethoxy)phenyl]-6-{[trans-3-(methylsulfonyl)cyclobutyl]amino}-1H-pyrazolo[3,4-d]pyrimidin-4-yl)piperidin-4-yl]propan-2-ol (57.0 mg) and trans-3-(methylsulfonyl)cyclobutanamine hydrochloride (31 mg) in NMP (1.5 ml) and the mixture was stirred at 130°C for 19 h. Standard workup and purification by HPLC (method HT_PREP_acid) afforded 42 mg of the title compound.
LC-MS (method HTpost_acid): Rₜ = 1.12 min; MS (ESIpos): m/z = 551 [M+H]⁺
¹H-NMR (400 MHz, DMSO-d6) δ [ppm]: 1.051 (16.00), 1.228 (0.53), 1.251 (0.57), 1.559 (0.45), 1.834 (0.70), 1.865 (0.64), 2.326 (0.43), 2.518 (1.90), 2.522 (1.24), 2.664 (0.47), 2.668 (0.68), 2.673 (0.66), 2.678 (0.53), 2.703 (0.62), 2.942 (12.54), 3.862 (0.46), 4.181 (5.04), 4.518 (0.47), 4.537 (0.45), 7.061 (0.67), 7.066 (0.68), 7.081 (0.74), 7.086 (0.76), 7.394 (0.43), 7.508 (1.35), 7.528 (2.40), 7.549 (1.22), 8.121 (0.67), 8.163 (0.89), 8.165 (0.99), 8.168 (0.80), 8.186 (0.82), 8.189 (0.80), 8.297 (4.29).

### Example IV-3

### 4-[4-(1-aminocyclopropyl)piperidin-1-yl]-1-[3-(difluoromethoxy)phenyl]-N-[1-(methylsulfonyl)azetidin-3-yl]-1H-pyrazolo[3,4-d]pyrimidin-6-amine

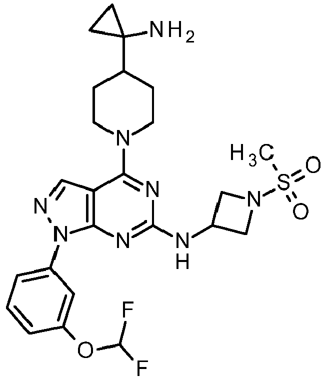

### Step a)

### tert-butyl {1-[1-(1-[3-(difluoromethoxy)phenyl]-6-{[1-(methylsulfonyl)azetidin-3-yl]amino}-1H-pyrazolo[3,4-d]pyrimidin-4-yl)piperidin-4-yl]cyclopropyl}carbamate

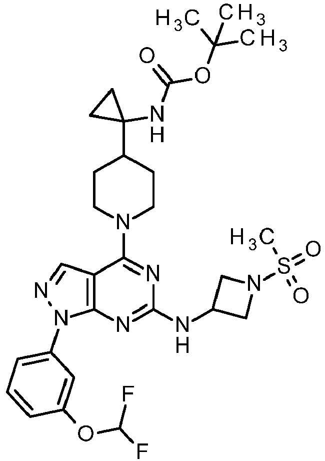

A solution of tert-butyl [1-(1-{6-chloro-1-[3-(difluoromethoxy)phenyl]-1H-pyrazolo[3,4-d]pyrimidin-4-yl}piperidin-4-yl)cyclopropyl]carbamate (80 mg) and 1-(methylsulfonyl)azetidin-3-amine (45 mg) in NMP (1.9 ml) was treated with DIPEA (0.16 ml) and the mixture was stirred at 130°C for 72 h. Standard workup and purification by HPLC afforded 70 mg tert-butyl {1-[1-(1-[3-(difluoromethoxy)phenyl]-6-{[1-(methylsulfonyl)azetidin-3-yl]amino}-1H-pyrazolo[3,4-d]pyrimidin-4-yl)piperidin-4-yl]cyclopropyl}carbamate
LC-MS (method HTpost_bas): Rₜ = 1.34 min; MS (ESIpos): m/z = 649 [M+H]⁺

### Step b)

A solution of tert-butyl {1-[1-(1-[3-(difluoromethoxy)phenyl]-6-{[1-(methylsulfonyl)azetidin-3-yl]amino}-1H-pyrazolo[3,4-d]pyrimidin-4-yl)piperidin-4-yl]cyclopropyl}carbamate (70 mg) in dichlormethane (1.2 ml) was treated with TFA (0.25 ml) and the mixture was stirred at RT for 16 h. The mixture was concentrated and the residue was purified by HPLC to afford 18 mg 4-[4-(1-aminocyclopropyl)piperidin-1-yl]-1-[3-(difluoromethoxy)phenyl]-N-[1-(methylsulfonyl)azetidin-3-yl]-1H-pyrazolo[3,4-d]pyrimidin-6-amine.
LC-MS (method HTpost_bas): Rₜ = 1.15 min; MS (ESIpos): m/z = 549 [M+H]⁺
¹H-NMR (400 MHz, DMSO-d6) δ [ppm]: 0.325 (0.80), 0.342 (3.16), 0.348 (2.29), 0.378 (2.26), 0.384 (3.32), 0.401 (0.80), 1.073 (0.56), 1.473 (0.80), 1.482 (0.82), 1.504 (0.89), 1.515 (0.83), 1.536 (0.41), 1.630 (0.71), 1.677 (1.27), 1.708 (0.91), 2.518 (2.46), 2.523 (1.61), 2.540 (1.00), 2.950 (0.76), 3.034 (16.00), 3.876 (1.09), 3.895 (1.74), 3.913 (1.30), 4.095 (1.35), 4.114 (2.50), 4.135 (1.22), 4.648 (0.48), 4.665 (0.83), 4.682 (0.84), 4.700 (0.54), 7.076 (0.87), 7.081 (0.88), 7.102 (0.91), 7.157 (1.24), 7.341 (2.51), 7.526 (1.26), 7.549 (0.46), 7.569 (0.64), 7.622 (0.52), 8.142 (0.92), 8.162 (0.93), 8.332 (4.77).

### Example IV-4

### 1-[1-(1-[3-(difluoromethoxy)phenyl]-6-{[trans-3-(methylsulfonyl)cyclobutyl]amino}-1H-pyrazolo[3,4-d]pyrimidin-4-yl)azetidin-3-yl]-2-methylpropan-2-ol

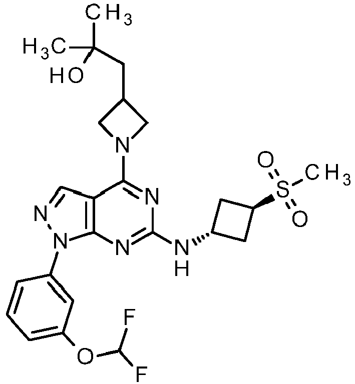

A solution of 1-(1-{6-chloro-1-[3-(difluoromethoxy)phenyl]-1H-pyrazolo[3,4-d]pyrimidin-4-yl}azetidin-3-yl)-2-methylpropan-2-ol (75.0 mg, 0.18 mmol) and trans-3-(methylsulfonyl)-cyclobutanamine hydrochloride 65.7 mg, 0.35 mmol) in NMP (2.0 ml) was treated with DIPEA (0.18 ml) and the mixture was stirred at 130°C for 16 h. Standard workup and HPLC purifaction (method HT_PREP_bas) afforded 47.0 mg of the title compound.
LC-MS (method HTpost_acid): Rₜ = 1.00 min; MS (ESIpos): m/z = 537 [M+H]⁺
¹H-NMR (400 MHz, DMSO-d6) δ [ppm]: 0.000 (8.79), 1.106 (16.00), 1.765 (1.68), 1.783 (1.72), 2.463 (0.71), 2.474 (0.85), 2.521 (1.29), 2.526 (0.79), 2.667 (0.54), 2.672 (0.71), 2.677 (0.67), 2.681 (0.59), 2.702 (0.64), 2.937 (14.49), 3.045 (0.47), 3.844 (0.64), 3.855 (0.68), 4.261 (3.12), 4.498 (0.40), 4.516 (0.75), 4.535 (0.79), 4.553 (0.44), 7.053 (0.72), 7.058 (0.73), 7.073 (0.79), 7.079 (0.81), 7.505 (1.42), 7.526 (2.58), 7.546 (1.38), 8.006 (5.40), 8.134 (0.83), 8.165 (0.83), 8.187 (0.73).

### Example IV-5

### (3S,4S)-1-(1-[3-(difluoromethoxy)phenyl]-6-{[trans-3-(methylsulfonyl)cyclobutyl]amino}-1H-pyrazolo[3,4-d]pyrimidin-4-yl)-4-fluoropyrrolidin-3-ol

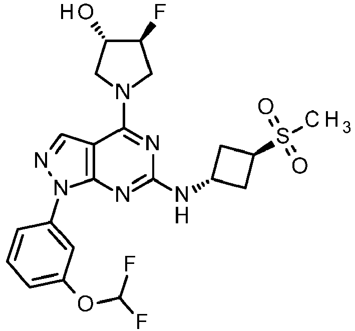

A solution of (3S,4S)-1-{6-chloro-1-[3-(difluoromethoxy)phenyl]-1H-pyrazolo[3,4-d]pyrimidin-4-yl}-4-fluoropyrrolidin-3-ol (80 mg, 0.20 mmol) and trans-3-(methylsulfonyl)cyclobutanamine hydrochloride (74.3 mg, 400 µmol) in NMP (2.1 ml) was treated with DIPEA (0.21 ml) and the mixture was stirred at 130°C for 16 h. Standard workup and purification by HPLC (method HT_PREP_acid) afforded 63 mg of the title compound.
LC-MS (method HTpost_acid): Rₜ = 0.98 min; MS (ESIpos): m/z = 513 [M+H]⁺
¹H-NMR (400 MHz, DMSO-d6) δ [ppm]: -0.009 (0.54), 0.007 (0.54), 2.472 (0.74), 2.521 (3.07), 2.525 (2.69), 2.542 (2.18), 2.549 (0.86), 2.709 (0.95), 2.722 (1.18), 2.949 (16.00), 3.756 (0.53), 3.783 (0.63), 3.849 (0.69), 3.861 (0.83), 3.873 (1.07), 3.884 (0.74), 3.896 (0.53), 3.990 (0.59), 4.025 (0.56), 4.057 (0.49), 4.541 (0.60), 4.561 (0.61), 7.065 (1.05), 7.070 (1.08), 7.085 (1.15), 7.090 (1.19), 7.488 (0.57), 7.517 (2.41), 7.538 (3.94), 7.558 (2.02), 8.146 (1.27), 8.176 (1.88), 8.195 (1.31), 8.227 (5.92).

### Example IV-6

### (3S,4S)-1-(1-[3-(difluoromethoxy)phenyl]-6-{[1-(methylsulfonyl)azetidin-3-yl]amino}-1H-pyrazolo[3,4-d]pyrimidin-4-yl)-4-fluoropyrrolidin-3-ol

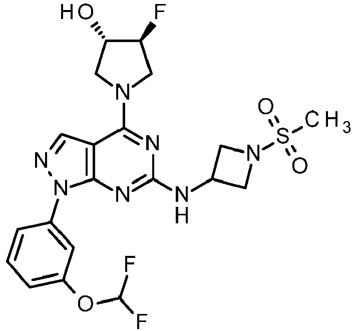

A solution of (3S,4S)-1-{6-chloro-1-[3-(difluoromethoxy)phenyl]-1H-pyrazolo[3,4-d]pyrimidin-4-yl}-4-fluoropyrrolidin-3-ol (80 mg, 0.20 mmol) and 1-(methylsulfonyl)azetidin-3-amine (74.3 mg, 0.40 mmol) in NMP (2.1 ml) was treated with DIPEA (0.21 ml) and the mixture was stirred at 130°C for 16 h. Standard workup and purification by HPLC (method HT_PREP _acid) afforded 51 mg of the title compound.
LC-MS (method HTpost_acid): Rₜ = 1.03 min; MS (ESIpos): m/z = 514 [M+H]⁺
¹H-NMR (400 MHz, DMSO-d6) δ [ppm]: 0.000 (7.51), 2.521 (1.05), 2.526 (0.67), 3.039 (16.00), 3.793 (0.44), 3.893 (0.70), 3.911 (1.16), 3.928 (0.81), 4.002 (0.44), 4.113 (1.03), 4.133 (1.90), 4.153 (0.93), 4.692 (0.55), 4.709 (0.52), 7.079 (0.69), 7.084 (0.70), 7.099 (0.73), 7.104 (0.75), 7.165 (0.90), 7.349 (1.86), 7.534 (0.95), 8.173 (0.68), 8.192 (0.64), 8.254 (3.39).

### Example IV-7

### (3S,4S)-1-{1-[3-(difluoromethoxy)phenyl]-6-[(1,1-dioxidotetrahydro-2H-thiopyran-4-yl)amino]-1H-pyrazolo[3,4-d]pyrimidin-4-yl}-4-fluoropyrrolidin-3-ol

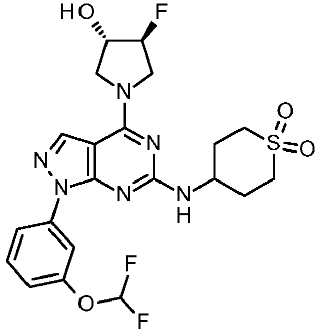

A solution of (3S,4S)-1-{6-chloro-1-[3-(difluoromethoxy)phenyl]-1H-pyrazolo[3,4-d]pyrimidin-4-yl}-4-fluoropyrrolidin-3-ol (80 mg, 0.20 mmol) and tetrahydro-2H-thiopyran-4-amine 1,1-dioxide hydrochloride (74 mg, 0.40 mmol) in NMP (2.1 ml) was treated with DIPEA (0.21 ml) and the mixture was stirred at 130°C for 40 h. Standard workup and purification by HPLC (method HT_PREP_acid) afforded 54 mg of the title compound.
LC-MS (method HTpost_acid): Rₜ = 1.02 min; MS (ESIpos): m/z = 513 [M+H]⁺
¹H-NMR (400 MHz, DMSO-d6) δ [ppm]: 2.077 (2.62), 2.102 (3.05), 2.237 (3.29), 2.521 (6.32), 2.526 (4.12), 2.543 (1.78), 3.203 (5.27), 3.756 (1.75), 3.784 (2.10), 3.870 (1.42), 3.900 (1.18), 3.959 (1.00), 3.992 (1.79), 4.026 (1.91), 4.057 (1.92), 4.162 (1.82), 4.186 (1.68), 4.356 (1.04), 4.472 (1.12), 5.039 (1.16), 5.132 (0.98), 5.165 (1.19), 5.257 (0.97), 5.653 (1.30), 5.762 (1.67), 7.071 (3.27), 7.090 (3.53), 7.154 (7.52), 7.255 (1.23), 7.339 (15.90), 7.524 (7.55), 7.567 (1.96), 8.211 (4.34), 8.226 (16.00).

### Example V-1

### 3-ethyl-1-(6-{[(3S)-1-methylpyrrolidin-3-yl]amino}-1-[3-(methylsulfonyl)phenyl]-1H-pyrazolo[3,4-d]pyrimidin-4-yl)azetidin-3-ol

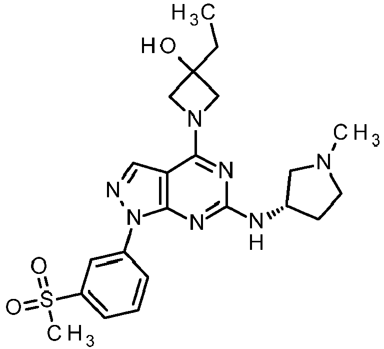

DIPEA (0.17 ml) was added to (1-{6-chloro-1-[3-(methanesulfonyl)phenyl]-1H-pyrazolo[3,4-d]pyrimidin-4-yl}-3-ethylazetidin-3-ol (65.0 mg, 0.16 mmol) and (3S)-1-methylpyrrolidin-3-amine dihydrochloride (55.2 mg, 0.32 mmol) in NMP (1.0 ml) and the mixture was stirred for 17 h at 130°C. The mixture was filtered, DMSO (1 ml) was added and the solution was purified by HPLC (column XBrigde C18 5µ 50 x 50 mm; water + 0.2 Vol% aq. ammonia (32%), acetonitrile) affording 3.5 mg of the title compound.
MS (ESIpos): m/z = 472 [M+H]⁺

### Example V-2

### 3-ethyl-1-{1-[3-(methylsulfonyl)phenyl]-6-[(3R)-tetrahydrofuran-3-ylamino]-1H-pyrazolo[3,4-d]pyrimidin-4-yl}azetidin-3-ol

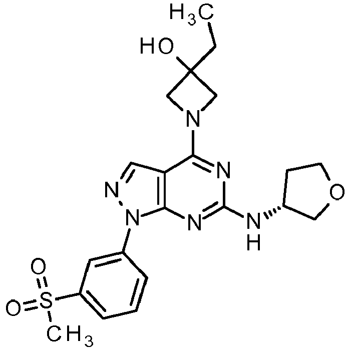

DIPEA (0.17 ml) was added to 1-{6-chloro-1-[3-(methanesulfonyl)phenyl]-1H-pyrazolo[3,4-d]pyrimidin-4-yl}-3-ethylazetidin-3-ol (65.0 mg, 0.16 mmol) and (3R)-tetrahydrofuran-3-amine 4-methylbenzenesulfonate (82.7 mg, 0.32 mmol) in NMP (1.0 ml) and the mixture was stirred for 17 h at 130°C. The mixture was filtered, DMSO (1 ml) was added and the solution was purified by HPLC (method HT_PREP_acid) affording the title compound (34.8 mg).
LC-MS (method HTpost_acid): Rₜ = 0.87 min; MS (ESIpos): m/z = 459 [M+H]⁺
¹H-NMR (400 MHz, DMSO-d6) δ [ppm]: 0.917 (5.72), 0.936 (13.47), 0.954 (6.28), 1.715 (1.59), 1.732 (4.58), 1.751 (4.37), 1.769 (1.44), 1.924 (1.38), 1.939 (1.47), 1.952 (1.09), 2.186 (1.14), 2.327 (0.87), 2.522 (3.04), 2.668 (0.88), 3.267 (15.52), 3.619 (1.17), 3.688 (1.33), 3.703 (1.66), 3.708 (3.10), 3.723 (3.13), 3.728 (2.25), 3.743 (1.77), 3.796 (1.60), 3.815 (3.55), 3.834 (2.89), 3.853 (1.11), 3.965 (1.89), 3.984 (2.01), 4.030 (1.36), 4.164 (1.36), 4.316 (1.35), 4.449 (1.57), 5.755 (4.87), 7.373 (1.11), 7.763 (0.57), 7.786 (5.11), 7.791 (5.29), 7.797 (7.25), 8.113 (16.00), 8.528 (0.97), 9.100 (1.14).

### Example VI-1

### 2-[1-(1-[3-(2-hydroxypropan-2-yl)phenyl]-6-{[(3S)-1-methylpyrrolidin-3-yl]amino}-1H-pyrazolo[3,4-d]pyrimidin-4-yl)piperidin-4-yl]propan-2-ol

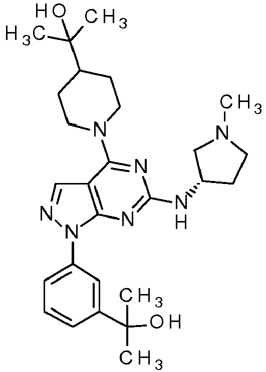

### Step a)

### ethyl 3-(4-[4-(2-hydroxypropan-2-yl)piperidin-1-yl]-6-{[(3S)-1-methylpyrrolidin-3-yl]-amino}-1H-pyrazolo[3,4-d]pyrimidin-1-yl)benzoate

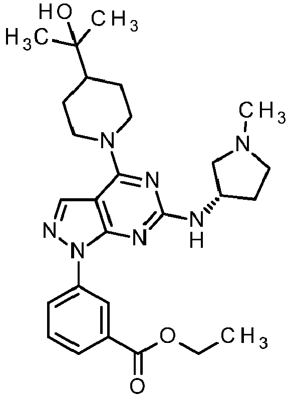

A solution of ethyl 3-{6-chloro-4-[4-(2-hydroxypropan-2-yl)piperidin-1-yl]-1H-pyrazolo[3,4-d]pyrimidin-1-yl}benzoate (150 mg, 0.34 mmol) and (3S)-1-methylpyrrolidin-3-amine dihydrochloride (117 mg, 0.68 mmol) in NMP (3.0 ml) was treated with DIPEA (0.35 ml) and the mixture was stirred at 130°C for 16 h. Standard workup and purification by HPLC (method HT_PREP_bas) afforded 32 mg ethyl 3-(4-[4-(2-hydroxypropan-2-yl)piperidin-1-yl]-6-{[(3S)-1-methylpyrrolidin-3-yl]amino}-1H-pyrazolo[3,4-d]pyrimidin-1-yl)benzoate.
LC-MS (HTpost1_bas): Rₜ = 1.30 min; MS (ESIpos): m/z = 508 [M+H]⁺
¹H-NMR (400 MHz, DMSO-d6) δ [ppm]: 1.051 (16.00), 1.226 (0.72), 1.248 (0.73), 1.333 (3.26), 1.351 (6.80), 1.368 (3.26), 1.552 (0.54), 1.744 (0.42), 1.758 (0.45), 1.828 (0.97), 1.859 (0.89), 2.238 (9.14), 2.308 (0.51), 2.322 (0.51), 2.326 (0.65), 2.404 (0.70), 2.425 (0.89), 2.442 (0.95), 2.668 (0.63), 2.843 (0.45), 2.991 (0.51), 4.181 (3.66), 4.339 (1.01), 4.357 (2.92), 4.375 (3.03), 4.392 (1.27), 4.731 (0.42), 7.625 (0.45), 7.645 (0.87), 7.664 (0.52), 7.825 (1.24), 7.844 (1.00), 8.291 (3.24).

### Step b)

A solution of ethyl 3-(4-[4-(2-hydroxypropan-2-yl)piperidin-1-yl]-6-{[(3S)-1-methylpyrrolidin-3-yl]amino}-1H-pyrazolo[3,4-d]pyrimidin-1-yl)benzoate (25.0 mg, 49.2 µmol) in 2-methyltetrahydrofuran (1.2 ml) under nitrogen was cooled with an ice-cold water bath. Then methylmagnesium bromide (160 µl, 3.0 M) was carefully added. The mixture was stirred for 30 min with cooling, then the mixture stirred at rt for 72 h. Saturated aq. ammonium chloride solution and ethyl acetate was added and the mixture was stirred for 30 min. The mixture was extracted with ethyl acetate three times, the organic layers were filtered using a water-repellent filter and concentrated. Purification by HPLC (method HT_PREP_bas) afforded 5 mg of the title compound.
¹H-NMR (400 MHz, DMSO-d6) δ [ppm]: 1.051 (15.77), 1.222 (0.55), 1.245 (0.58), 1.474 (16.00), 1.548 (0.50), 1.722 (0.45), 1.736 (0.47), 1.824 (0.79), 1.854 (0.72), 2.230 (10.71), 2.297 (0.73), 2.331 (0.42), 2.376 (0.42), 2.390 (0.53), 2.411 (0.50), 2.428 (0.55), 2.518 (2.12), 2.522 (1.61), 2.539 (1.27), 2.809 (0.48), 2.982 (0.42), 4.179 (3.40), 4.356 (0.41), 5.100 (2.21), 7.348 (0.42), 7.367 (0.94), 7.384 (0.62), 7.403 (0.70), 8.217 (3.70).

### BIOLOGICAL ASSAYS

Examples were tested in selected biological assays one or more times. When tested more than once, data are reported as either average values or as median values, wherein
- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values or median values calculated utilizing data sets obtained from testing of one or more synthetic batch.
The in vitro activity of the compounds of the present invention can be demonstrated in the following assays:

### Example Bio-1

### Human IRAK4 kinase activity inhibition assay

The IRAK4-inhibitory activity of the inventive substances was measured in the IRAK4 TR-FRET assay (TR-FRET = Time Resolved Fluorescence Resonance Energy Transfer) described hereinafter.

Recombinant fusion protein from N-terminal GST (glutathione S-transferase) and human IRAK4, expressed in baculovirus-infected insect cells (Hi5, BTI-TN-5B1-4, cell line purchased from Invitrogen, catalogue No. B855-02) and purified via affinity chromatography, was used as enzyme. The substrate used for the kinase reaction was the biotinylated peptide biotin-Ahx-KKARFSRFAGSSPSQASFAEPG (C-terminus in amide form) which can be purchased, for example, from Biosyntan GmbH (Berlin-Buch).
For the assay, 11 different concentrations in the range from 20 µM to 0.073 nM were prepared from a 2 mM DMSO solution of the test substance. 50 nl of the respective 100fold concentrated test compound solutions in DMSO were pipetted into a black low-volume 384-well microtitre plate or a black 1536-well microtiter plate (both Greiner Bio-One, Frickenhausen, Germany), 2 µl of a solution of IRAK4 in assay buffer [50 mM HEPES pH 7.5, 5mM MgCl2, 1.0 mM dithiothreitol, 30 µM activated sodium orthovanadate, 0.1% (w/v) of bovine gamma-globulin (BGG) 0.04% (v/v) nonidet-P40 (Sigma)] were added and the mixture was incubated for 15 min to allow prebinding of the substances to the enzyme prior to the kinase reaction. The kinase reaction was then started by addition of 3 µl of a solution of adenosine triphosphate (ATP, 1.67 mM = final concentration in 5 µl of assay volume: 1 mM) and peptide substrate (0.83 µM = final concentration in 5 µl assay volume: 0.5 µM) in assay buffer, and the resulting mixture was incubated at 22°C for the reaction time of 45 min. The concentration of the IRAK4 was adjusted to the respective activity of the enzyme and set such that the assay was carried out in the linear range. Typical concentrations were in the order of about 0.2 nM. The reaction was stopped by addition of 5 µl of a solution of TR-FRET detection reagents [0.1 µM streptavidin-XL665 (Cisbio Bioassays; France, catalogue No. 610SAXLG)] and 1.5 nM anti-phosphoserine antibody [Merck Millipore, "STK Antibody", catalogue No. 35-002] and 0.6 nM LANCE EU-W1024-labelled anti-mouse-IgG antibody (Perkin-Elmer, product No. AD0077; alternatively, it is possible to use a terbium cryptate-labelled anti-mouse-IgG antibody from Cisbio Bioassays) in aqueous EDTA solution (100 mM EDTA, 0.4 % [w/v] bovine serum albumin [BSA] in 25 mM HEPES pH 7.5).
The resulting mixture was incubated at 22°C for 1 h to allow formation of a complex of the biotinylated phosphorylated substrate and the detection reagents. The amount of the phosphorylated substrate was then evaluated by measuring the resonance energy transfer from europium chelate-labelled anti-mouse-IgG antibody to streptavidin-XL665. To this end, the fluorescence emissions at 620 nm and 665 nm were measured after excitation at 350 nm in a TR-FRET measuring instrument, for example a Rubystar (BMG Labtechnologies, Offenburg, Germany) or a Viewlux (Perkin-Elmer). The ratio of the emissions at 665 nm and 622 nm was taken as a measure of the amount of phosphorylated substrate. The data were normalized (enzyme reaction without test substance = 0% inhibition; all other assay components but no enzyme = 100% inhibition). Typically, the test substances were tested on the same microtitre plates at 11 different concentrations in the range from 20 µM to 0.073 nM (20 µM, 5.7 µM, 1.6 µM, 0.47 µM, 0.13 µM, 38 nM, 11 nM, 3.1 nM, 0.89 nM, 0.25 nM and 0.073 nM, the dilution series prepared separately before the assay on the level of the 100fold concentrated solutions in DMSO by serial dilutions, exact concentrations may vary depending pipettors used) in duplicate values for each concentration and IC50-values were calculated using Genedata Screener™ software.

The data in table 2 demonstrate that the compounds of the general formula (I) display strong inhibition of human IRAK4 (IC₅₀ < 500 nm). Most of the compounds show even double or single digit nanomolar potency values (IC₅₀ < 100 nM) demonstrating that the compounds are useful for the treatment of diseases which are related to IRAK4.

### Production of recombinant canine IRAK4 protein (Full length, wild-type)

### Expression construct and cloning of expression vector:

The applied DNA expression constructs which encode the following protein sequence were optimized for expression in insect cells and synthesized by the GeneArt Technology at Life Technologies:
- IRAK4 dog_1: amino acid 2-459 (UNIPROT: E2RDL0) with a TEV (Tobacco Etch Virus) protease cleavage site at the N-terminus.

This expression construct additionally encoded att-site sequences at the 5' and 3' end for subcloning into destination vectors using the Gateway Technology. The applied Destination vector was pD-INS6, a Baculovirus transfer vector (based on vector pVL1393, Pharmingen). Destination vector pD-INX1 provides an N-terminal fusion of a GST-tag to the integrated gene construct and pD-INS6 a N-terminal fusion of a His6-tag, respectively. IRAK4 dog_1 was cloned into pD-INS6, the resulting vector was termed pD-Ins6_IRAK4 dog_1.

### Baculovirus generation and protein expression for IRAK4 dog 1:

A recombinant baculovirus stock for expression of pD-INS6_IRAK4 canine_1 was generated by co transfection of Sf9 insect cells with the separate transfer vector and commercial Baculovirus DNA (FlashBac Gold, Oxford Expression Technologies) according the instruction manual of the manufacturer. After 5 days the supernatant of the transfected cells containing the recombinant Baculovirus encoding the IRAK4 canine_1 protein was used for further infection of Sf9 cells for virus amplification whereby the virus titer was monitored using qPCR. Hi5 cells were grown (Insect-xpress medium, Lonza, 27 °C, 100 rpm) in a shaked 24-well plate with 2 mL culture volume and infected at a cell density of 106 cells/mL with recombinant baculovirus stocks at a multiplicity of infection of 3 and incubated for 48 h. Subsequently the cells were harvested by centrifugation (800 xg) and cell pellet frozen at -80 °C.

### Protein purification

Purification of the His-IRAK4 canine_1 fusion protein was achieved by affinity chromatography using Ni Sepharose High Performance matrix (GE Healthcare Life Sciences) at 4 °C.

The pelleted cells (from 0.8 mL culture) were resuspended in Lysis-Buffer (A: 50 mM Tris pH 8,0, 150 mM NaCl, 10 % Glycerol, 2 mM DTT, Complete Inhibitor without EDTA (Roche Diagnostics), 0.1 % NP40, 40 mM Imidazole) in a 24 well plate and incubated on ice for for 1 h. The lysate was then transferred to a 96-microtiter plate and centrifuge for 30 min (3200 xg). The supernatant was further transferred to a TurboFilter (Qiagen) 96 -plates to remove DNA and insoluble material by centrifugation (500 xg). The filtrate was incubated with the Ni Sepharose (50 µl bed volume, equilibrated with wash buffer 50 mM Tris pH 8,0, 150 mM NaCl, 10 % Glycerol, 2 mM DTT, 40 mM Imidazole) in 96-formate at 150 min., centrifuged and washed twice with wash buffer (each 500 µl, 500 xg). Washed beads were incubated with 0.1 mL elution buffer (50 mM Tris pH 8,0, 150 mM NaCl, 10 % Glycerol, 2 mM DTT, 500 mM Imidazole) for 90 min. and subsequently eluted by centrifugation, followed by a second incubation and elution with 0.1 mL elution buffer. The buffer of the eluate was adjusted to storage buffer 50 mM Tris pH 8,0, 150 mM NaCl, 10 % Glycerol, 2 mM DTT using Zeba Spin Desalting Plate (Pierce) and the sample stored after freezing in liquid nitrogen at -80 °C.

### Sequence; His-tag-IRAK4 dog 1

MTSHHHHHHSSMGSRTSLYKKAGFDYDIPTTENLYFQGNKPITSSTYVRCLNLGLIRKLSDFI
DPQEGWKKLAVAIKKPSGDDRYNQFHIRRFEALLQTGKSPTCELLFDWGTTNCTVGDLVDLL
IQNEFFAPASLLLPDAIPKTPNTVPSEEAVTVQQQQMPLNGKDRTFVLPVQNFEQNYMPPDS
SSPENTSLEGSDTRFHSFSFYELKNVTNNFDERPISLGGNKMGEGGFGVVYKGYVNNKTVA
VKKLAAMVDISTEELKQQFDQEIKVMAKCQHENLVELLGFSSDGDDLCLVYVYMPNGSLLDR
LSCLDGTPPLSWHMRCKIAQGAANGISFLHENHHIHRDIKSANILLDEDFTAKVSDFGLARAS
EKFAQTVMTSRIVGTTAYMAPEALRGEITPKSDIYSFGVVLLEIITGLPAVDEHREPQLLLDIKE
EIEDEEKTIEDYIDPKMNDTDPTSIETMYSVASQCLHEKKNKRPDIEKVQQLLQDLTAS

### Canine Irak4 kinase assay

Inhibitory activity of compounds of the present invention against canine Irak4 was quantified employing the TR-FRET based Irak4 kinase activity assay as described in the following paragraphs (TR-FRET = Time Resolved Fluorescence Resonance Energy Transfer).
N-terminally His₆-tagged recombinant canine Irak4, expressed in baculovirus-infected insect cells and purified via affinity chromatography using Ni-Sepharose as described above was used as kinase. As substrate for the kinase reaction the biotinylated peptide biotin-Ahx-KKARFSRFAGSSPSQASFAEPG (C-terminus in amide form) was used which can be purchased e.g. from the company Biosynthan GmbH (Berlin-Buch, Germany).
For the assay 50 nl of a 100fold concentrated solution of the test compound in DMSO was pipetted into either a black low volume 384well microtiter plate or a black 1536well microtiter plate (both Greiner Bio-One, Frickenhausen, Germany), 2 µl of a solution of Irak4 in aqueous assay buffer [50 mM Hepes pH 7.5, 5 mM MgCl₂, 1mM dithiothreitol, 0.5 mM EGTA, 30 µM activated sodium ortho-vanadate, 0.1 % (w/v) bovine gamma globulin, 0.04% (v/v) Nonidet-P40 (Sigma)] were added and the mixture was incubated for 15 min at 22°C to allow pre-binding of the test compounds to the enzyme before the start of the kinase reaction. Then the kinase reaction was started by the addition of 3 µL of a solution of adenosine-tri-phosphate (ATP, 1.67 mM= final concentration in the 5 µL assay volume is 1 mM) and substrate (0.83 µM = final conccentration in the 5 µL assay volume is 0.5 µM) in assay buffer and the resulting mixture was incubated for a reaction time of 45 min at 22°C. The concentration of canine Irak4 was adjusted depending on the activity of the enzyme lot and was chosen appropriate to have the assay in the linear range, a typical concentration was 0.1 nM. The reaction was stopped by the addition of 3 µl of a solution of TR-FRET detection reagents (100 nM streptavidine-XL665 [Cisbio Bioassays, Codolet, France] and 1.5 nM anti-phosho-Serine antibody [Merck Millipore, cat. # 35-002] and 0.6 nM LANCE EU-W1024 labeled anti-mouse IgG antibody [Perkin-Elmer, product no. AD0077, as an alternative a Terbium-cryptate-labeled anti-mouse IgG antibody from Cisbio Bioassays can be used]) in an aqueous EDTA-solution (100 mM EDTA, 0.4 % (w/v) bovine serum albumin in 25 mM HEPES pH 7.5).
The resulting mixture was incubated 1 h at 22°C to allow the binding of the biotinylated phosphorylated peptide to the streptavidine-XL665 and the PT66-Tb-Cryptate. Subsequently the amount of phosphorylated substrate was evaluated by measurement of the resonance energy transfer from the Eu-chelate to the streptavidine-XL665. Therefore, the fluorescence emissions at 622 nm and 665 nm after excitation at 337 nm were measured in a TR-FRET reader, e.g. a Pherastar or Pherastar FS (both from BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and IC₅₀-values were calculated using Genedata Screener™ software.

The data in table 2 demonstrate that the compounds of the general formula (I) display surprisingly strong inhibition of canine IRAK4 (IC₅₀ < 50 nm) indicating that the compounds are useful for the treatment of diseases in animals which are associated with IRAK4.

**Table 2: IC₅₀-values of example compounds in the IRAK4 human kinase assay and IC₅₀-values from canine kinase assay**

| **Example Compound** | **Human IRAK4 IC₅₀ [mol/l]** | **Canine IRAK4 IC₅₀ [mol/l]** |
|---|---|---|
| I-1-1 | 3.04 E-9 | 3.19 E-9 |
| I-1-2 | 8.45 E-8 | |
| I-2-1 | 7.19 E-9 | 8.70 E-9 |
| I-2-2 | 3.14 E-8 | |
| I-2-3 | 9.69 E-9 | |
| I-3-1 | 9.76 E-9 | |
| I-3-2 | 1.67 E-7 | |
| I-4-1 | 1.22 E-8 | |
| I-4-2 | 8.27 E-9 | 8.54 E-9 |
| I-4-3 | 6.85 E-8 | |
| I-5 | 1.05 E-8 | 9.27 E-9 |
| I-6 | 7.68 E-8 | |
| I-7-1 | 1.14 E-8 | |
| I-7-2 | 3.38 E-8 | |
| I-8 | 4.14 E-8 | |
| I-9 | 5.95 E-8 | |
| I-10 | 1.31 E-7 | |
| I-11-1 | 1.39 E-7 | |
| I-11-2 | 1.51 E-7 | |
| I-12 | 5.12 E-8 | |
| I-13-1 | 1.31 E-8 | 2.64 E-8 |
| I-13-2 | 3.27 E-8 | |
| I-14 | 1.20 E-8 | 1.27 E-8 |
| I-15 | 6.20 E-8 | |
| I-16-1 | 1.94 E-8 | |
| I-16-2 | 1.61 E-8 | |
| I-17 | 4.42 E-7 | |
| I-18 | 1.84 E-8 | |
| I-19 | 2.70 E-8 | |
| I-20-1 | 2.78 E-7 | |
| I-20-2 | 1.49 E-8 | |
| I-21 | 7.80 E-8 | |
| I-22 | 9.93 E-8 | |
| I-23 | 8.82 E-9 | 5.41 E-9 |
| I-24 | 7.08 E-9 | |
| I-25-1 | 4.03 E-8 | |
| I-25-2 | 1.20 E-8 | |
| I-26-1 | 3.73 E-8 | |
| I-26-2 | 5.78 E-8 | |
| I-27 | 7.01 E-9 | |
| I-28 | 2.39 E-8 | |
| I-29 | 1.08 E-8 | |
| I-30 | 6.15 E-9 | |
| I-31 | 1.26 E-8 | |
| I-32 | 2.87 E-8 | |
| I-33 | 8.42 E-8 | |
| I-34 | 8.51 E-8 | |
| I-35 | 1.65 E-8 | |
| I-36 | 4.50 E-8 | |
| I-37 | 1.15 E-8 | |
| I-38 | 1.62 E-8 | |
| I-39 | 2.45 E-8 | |
| I-40 | 3.68 E-9 | |
| I-41-1 | 2.50 E-7 | |
| I-41-2 | 4.00 E-7 | |
| I-42 | 3.20 E-8 | |
| I-43 | 2.80 E-7 | |
| I-44 | 1.57 E-8 | |
| I-45 | 5.10 E-9 | |
| I-46 | 1.40 E-9 | |
| I-47 | 8.61 E-10 | |
| I-48 | 1.12 E-9 | |
| I-49 | 3.85 E-9 | |
| I-50 | 4.84 E-8 | |
| I-51 | 2.50 E-9 | |
| I-52 | 7.99 E-9 | |
| I-53 | 9.98 E-8 | |
| I-54 | 2.97 E-7 | |
| I-55 | 1.56 E-8 | |
| I-56 | 3.46 E-9 | |
| I-57 | 1.82 E-9 | 1.98 E-9 |
| I-58 | 2.30 E-8 | |
| I-59 | 3.63 E-9 | |
| I-60 | 3.42 E-8 | |
| I-61 | 3.28 E-9 | |
| I-62 | 2.57 E-9 | |
| I-63 | 3.06 E-8 | |
| I-64-1 | 8.58 E-9 | |
| I-64-2 | 5.89 E-8 | |
| I-65 | 1.56 E-8 | |
| I-66 | 1.38 E-7 | |
| I-67 | 5.33 E-9 | |
| I-68 | 2.30 E-8 | |
| I-69 | 2.62 E-8 | |
| I-70 | 2.50 E-8 | |
| I-71 | 1.53 E-8 | |
| I-72 | 7.31 E-9 | |
| I-73 | 2.80 E-8 | |
| I-74 | 4.83 E-9 | |
| I-75 | 1.40 E-8 | |
| I-76 | 9.61 E-9 | |
| I-77 | 4.80 E-9 | |
| I-78 | 5.62 E-9 | |
| I-79 | 7.75 E-9 | |
| I-80 | 1.14 E-8 | |
| I-81 | 8.32 E-8 | |
| I-82 | 1.15 E-8 | |
| I-83 | 5.53 E-9 | |
| I-84 | 4.83 E-8 | |
| I-85 | 2.41 E-9 | |
| I-86 | 3.66 E-9 | |
| I-87 | 7.87 E-9 | |
| I-88 | 3.94 E-9 | |
| I-89 | 4.72 E-9 | |
| I-90 | 2.47 E-8 | |
| I-91 | 6.53 E-9 | |
| I-92 | 5.61 E-9 | |
| I-93 | 1.12 E-8 | |
| I-94 | 4.28 E-9 | |
| I-95 | 2.50 E-8 | |
| I-96 | 8.10 E-9 | |
| I-97 | 8.16 E-9 | |
| I-98 | 9.99 E-9 | |
| I-99 | 1.62 E-8 | |
| I-100 | 4.99 E-8 | |
| I-101 | 2.81 E-9 | |
| I-102 | 4.99 E-8 | |
| I-103 | 1.14 E-8 | |
| I-104 | 1.78 E-7 | |
| I-105 | 2.80 E-7 | |
| I-106 | 9.09 E-9 | |
| I-107 | 8.11 E-9 | |
| I-108 | 3.72 E-9 | |
| I-109 | 1.06 E-8 | |
| I-110 | 1.18 E-8 | |
| I-111 | 1.11 E-8 | |
| I-112 | 3.24 E-9 | |
| I-113 | 3.60 E-9 | |
| I-114 | 8.49 E-9 | |
| I-115 | 7.51 E-9 | |
| I-116 | 1.68 E-8 | |
| I-117 | 2.34 E-8 | |
| I-118 | 5.13 E-9 | |
| I-119 | 1.65 E-8 | |
| I-120 | 1.66 E-8 | |
| I-121 | 3.65 E-9 | |
| I-122 | 2.75 E-9 | |
| I-123 | 7.98 E-9 | |
| I-124 | 2.25 E-8 | |
| II-1 | 3.46 E-9 | |
| II-2 | 6.68 E-9 | |
| II-3 | 2.48 E-8 | |
| II-4 | 3.33 E-9 | |
| II-5 | 3.45 E-9 | |
| II-6 | 5.49 E-9 | |
| II-7 | 5.37 E-9 | |
| II-8 | 2.01 E-9 | |
| II-9 | 8.49 E-8 | |
| II-10 | 4.31 E-9 | |
| II-11 | 2.67 E-9 | |
| II-12 | 2.98 E-9 | |
| II-13 | 1.33 E-8 | |
| II-14 | 7.01 E-9 | |
| II-15 | 4.52 E-8 | |
| II-16-1 | 1.36 E-9 | |
| II-16-2 | 2.82 E-9 | |
| II-16-3 | 1.62 E-9 | |
| II-17-1 | 2.64 E-9 | |
| II-17-2 | 2.10 E-9 | |
| II-17-3 | 4.17 E-9 | |
| II-18 | 1.40 E-9 | |
| II-19 | 1.63 E-9 | |
| II-20 | 1.39 E-9 | |
| II-21 | 1.80 E-9 | |
| II-22 | 4.53 E-8 | |
| II-23 | 1.62 E-8 | |
| II-24 | 5.65 E-9 | |
| II-25 | 6.15 E-9 | |
| II-26 | 7.36 E-8 | |
| II-27 | 4.75 E-9 | |
| II-28 | 4.00 E-8 | |
| II-29 | 6.71 E-8 | |
| II-30 | 1.62 E-8 | |
| II-31 | 9.22 E-9 | |
| II-32 | 5.67 E-8 | |
| II-33 | 1.65 E-8 | |
| II-34 | 3.49 E-9 | |
| II-35 | 6.80 E-9 | |
| II-36 | 2.76 E-9 | |
| III-1 | 1.91 E-8 | |
| IV-1 | 8.06 E-9 | 6.52 E-9 |
| IV-2 | 3.42 E-9 | |
| IV-3 | 6.61 E-9 | |
| IV-4 | 4.78 E-9 | |
| IV-5 | 1.12 E-8 | |
| IV-6 | 1.93 E-7 | |
| IV-7 | 4.64 E-8 | |
| V-1 | 1.70 E-7 | |
| V-2 | 1.79 E-7 | |
| VI-1 | 7.73 E-8 | |

### Example Bio 2

### Determination of metabolic stability in dog and human liver microsomes

The *in vitro* metabolic stability of test compounds was determined by incubating them at 1 µM in a suspension liver microsomes in 100 mM phosphate buffer, pH7.4 (NaH₂PO₄ x H₂O + Na₂HPO₄ x 2H₂O) and at a protein concentration of 0.5 mg/mL at 37° C. The microsomes were activated by adding a co-factor mix containing 8 mM Glukose-6-Phosphat, 4 mM MgCl₂, 0.5 mM NADP and 1 IU/mL G-6-P-Dehydrogenase in phosphate buffer, pH 7.4. The metabolic assay was started shortly afterwards by adding the test compound to the incubation at a final volume of 1 mL. Organic solvent in the incubations was limited to ≤0.01 % dimethylsulfoxide (DMSO) and ≤1% acetonitril. During incubation, the microsomal suspensions were continuously shaken at 580 rpm, and aliquots were taken at 2, 8, 16, 30, 45 and 60 min, to which equal volumes of cold methanol were immediately added. Samples were frozen at -20° C over night, subsequently centrifuged for 15 min at 3000 rpm and the supernatant was analyzed with an Agilent 1200 HPLC-system with LCMS/MS detection.

The half-life of a test compound was determined from the concentration-time plot. From the half-life the intrinsic clearances were calculated. Together with the additional parameters liver blood flow, specific liver weight and microsomal protein content the hepatic *in vivo* blood clearance (CL) and the maximal oral bioavailability (Fₘₐₓ) were calculated using the "well stirred" liver model. The following parameter values were used: Liver blood flow - 1.32 L/h/kg (human), 2.1 L/h/kg (dog); specific liver weight - 21 g/kg (human), 39 g/kg (dog); microsomal protein content - 40 mg/g.

For classification of the results the following criteria were used:
- Fₘₐₓ > 70 % (= CL < 0.4 L/h/kg human; < 0.6 Uh/kg dog) was classified as high metabolic stability (high),
- Fₘₐₓ between 30 and 70 % (CL between 0.4 and 0.9 L/h/kg human; between 0.6 and 1.5 L/h/kg dog) as moderate metabolic stability (moderate) and
- Fₘₐₓ < 30 % (= CL > 0.9 L/h/kg human; > 1.5 Uh/kg dog) as low metabolic stability (low).

The data shown in table 3 demonstrate that the compounds of the general formula (I) show medium to high metabolic stability. The majority of compounds show high metabolic stability (Fₘₐₓ > 70%). The compounds tested in dog liver microsomes also show a high stability.

**Table 3: Results from metabolic stability assay in dog and human liver microsomes**

| Example Compound | Fmax, human microsomes [%] | CLblood, human microsomes [L/h/kg] | Fmax, dog microsomes [%] | CLblood, dog microsomes [L/h/kg] |
|---|---|---|---|---|
| I-2-1 | 76 | 0.32 | | |
| I-3-1 | 65 | 0.46 | | |
| I-4-2 | 89 | 0.14 | | |
| I-5 | 93 | 0.09 | | |
| I-13-1 | 62 | 0.50 | | |
| I-14 | 95 | 0.07 | | |
| I-16-2 | 30 | 0.92 | | |
| I-22 | 74 | 0.34 | | |
| I-23 | 87 | 0.17 | | |
| I-27 | 87 | 0.17 | | |
| I-29 | 68 | 0.43 | | |
| I-30 | 100 (stable) | < 0.01 (stable) | | |
| I-31 | 100 (stable) | < 0.01 (stable) | | |
| I-37 | 97 | 0.05 | | |
| I-40 | 95 | 0.07 | | |
| I-45 | 96 | 0.05 | | |
| I-46 | 81 | 0.25 | | |
| I-47 | 87 | 0.17 | | |
| I-48 | 100 (stable) | < 0.01 (stable) | | |
| I-49 | 90 | 0.13 | | |
| I-51 | 81 | 0.25 | | |
| I-52 | 100 (stable) | < 0.01 (stable) | | |
| I-56 | 95 | 0.06 | 97 | 0.06 |
| I-57 | 97 | 0.04 | | |
| I-58 | 100 (stable) | < 0.01 (stable) | | |
| I-59 | 74 | 0.34 | | |
| I-60 | 72 | 0.37 | | |
| I-61 | 77 | 0.31 | | |
| I-62 | 100 (stable) | < 0.01 (stable) | | |
| I-64-1 | 76 | 0.32 | | |
| I-67 | 82 | 0.24 | | |
| I-68 | 99 | 0.02 | | |
| I-72 | 79 | 0.28 | 95 | 0.10 |
| I-74 | 91 | 0.11 | | |
| I-76 | 77 | 0.30 | | |
| I-77 | 72 | 0.36 | | |
| I-78 | 76 | 0.32 | | |
| I-79 | 69 | 0.41 | | |
| I-83 | 83 | 0.23 | | |
| I-85 | 100 (stable) | < 0.01 (stable) | | |
| I-86 | 81 | 0.25 | | |
| I-88 | 57 | 0.57 | | |
| I-89 | 72 | 0.37 | | |
| I-91 | 97 | 0.04 | | |
| I-92 | 73 | 0.36 | | |
| I-94 | 88 | 0.16 | | |
| I-98 | 100 (stable) | < 0.01 (stable) | | |
| I-101 | 73 | 0.35 | | |
| I-106 | 48 | 0.68 | | |
| I-107 | 96 | 0.05 | | |
| I-108 | 88 | 0.16 | | |
| I-109 | 75 | 0.33 | | |
| I-111 | 90 | 0.13 | | |
| I-112 | 71 | 0.39 | | |
| I-113 | 98 | 0.03 | | |
| I-114 | 84 | 0.21 | | |
| I-115 | 100 (stable) | < 0.01 (stable) | | |
| II-1 | 100 (stable) | < 0.01 (stable) | | |
| II-2 | 88 | 0.15 | 97 | 0.07 |
| II-4 | 100 (stable) | < 0.01 (stable) | 100 (stable) | < 0.01 (stable) |
| II-5 | 93 | 0.10 | 89 | 0.24 |
| II-6 | 86 | 0.19 | 78 | 0.46 |
| II-7 | 100 (stable) | < 0.01 (stable) | | |
| II-8 | 100 (stable) | < 0.01 (stable) | | |
| II-10 | 68 | 0.42 | | |
| II-11 | 81 | 0.25 | | |
| II-12 | 81 | 0.25 | 80 | 0.42 |
| II-14 | 100 (stable) | < 0.01 (stable) | | |
| II-16-2 | 100 (stable) | < 0.01 (stable) | | |
| II-16-3 | 94 | 0.08 | | |
| II-17-1 | 93 | 0.10 | | |
| II-17-2 | 79 | 0.28 | | |
| II-18 | 93 | 0.10 | | |
| II-19 | 82 | 0.23 | | |
| II-20 | 97 | 0.04 | | |
| II-21 | 95 | 0.07 | | |
| II-24 | 100 (stable) | < 0.01 (stable) | | |
| II-27 | 100 (stable) | < 0.01 (stable) | | |
| II-31 | 78 | 0.29 | | |
| II-34 | 95 | 0.07 | | |
| II-35 | 93 | 0.10 | | |
| IV-1 | 86 | 0.18 | | |
| IV-2 | 76 | 0.32 | 100 (stable) | < 0.01 (stable) |
| IV-3 | 89 | 0.14 | | |
| IV-4 | 58 | 0.55 | | |

### Example Bio 3

### Cellular Potency as determined by in vitro LPS (lipopolysaccharide)-induced cytokine production in human PBMCs (peripheral blood mononuclear cells)

The effect of the compounds of the general formula (I) on induced cytokine production in human PBMCs was examined.

Cytokine production was induced here by LPS, a TLR4 ligand, which leads to activation of the IRAK4-mediated signal path.

The human PBMCs were obtained from anti-coagulated human whole blood. To this end, 15 mL of Ficoll-Paque (Biochrom, Cat. No. L6115) were initially charged in Leucosep tubes and 20 mL of human blood were added. After centrifugation of the blood at 800 g for 15 min at room temperature, the plasma including the platelets was removed and discarded. The PBMCs were transferred into centrifugation tubes and made up with PBS (phosphate-buffered saline) (Gibco, Cat. No. 14190). The cell suspension was centrifuged at room temperature at 250 g for 10 min and the supernatant was discarded. The PBMCs were resuspended in complete medium (RPMI 1640, without L-glutamine (PAA, Cat. No. E15-039), 10% FCS; 50 U/mL penicillin, 50 µg/mL streptomycin (PAA, Cat. No. P11-010) and 1% L-glutamine (Sigma, Cat. No. G7513)).
The assay was also carried out in complete medium. The PBMCs were sown in 96-well plates at a cell density of 2.5x10⁵ cells/well. The compounds according to the invention were subjected to serial dilution in a constant volume of 100% DMSO and used in the assay at 8 different concentrations in the range from 10 µM to 3 nM such that the final DMSO concentration was 0.4% DMSO. Prior to the actual stimulation, the cells were then preincubated therewith for 30 min. To induce cytokine secretion, the cells were stimulated with 0.1 µg/mL LPS (Sigma, *Escherichia coli* 0128:B12, Cat. No. L2887) for 24 hours. Cell viability was determined using the CellTiter-Glo luminescent assay (Promega, Cat. No. G7571 (G755/G756A)) in accordance with the manufacturer's instructions. The amount of secreted TNF-α in the cell culture supernatant was determined using the Human ProInflammatory 9-Plex Tissue Culture Kit (MSD, Cat. No. K15007B) in accordance with the instructions of the manufacturer.
By way of example, Example Compounds II-2, I-40, I-56, I-61 have activity ≤ 500 nM.

### Example Bio 4

### In Vivo model of TLR-mediated inflammation

The compounds of the general formula (I) are tested in an in vivo TLR-mediated inflammation model for their in vivo efficacy.

This mechanistic model shows potential efficacy of the compounds in TLR4-mediated diseases, as a LPS-mediated inflammation model is applied. LPS is administered i.p. to female Balb/c mice (about 8 weeks old, Charles River Laboratories, Germany) and the effect of the compounds on LPS mediated cytokine secretion is examined. There are 10 animals in each group. The control group is treated with the vehicles used for dissolving the substance and the LPS.
0,2 mg LPS/kg body weight (Sigma, Kat-Nr. L4391 Lipopolysaccharides from E. coli 0111:B4) dissolved in PBS are administered i.p. to each of the groups treated with substance and the positive control group to induce inflammation. The substance (via oral dosing) or its vehicle in the positive control group is administered before the administration of LPS. 1.5 hours after administration of the LPS, TNF-α is determined in the plasma after the final removal of blood using the Mouse ProInflammatory 7-Plex Tissue Culture Kit (MSD, Cat. No. K15012B) in accordance with the manufacturer's instructions. Aim of the experiment is to demonstrate efficacy of the compounds on the inhibition of LPS induced TNF-α release in the plasma.

### Example Bio 5

### In vivo Imiquimod-induced Psoriasis model in mice

The compounds of the general formula (I) are tested for their effects in an Imiquimod (IMQ)-induced psoriasis model in mice.

Topical application of IMQ, a TLR7/8 ligand and potent immune activator, induces and exacerbates psoriasis in mice. For this purpose, female Balb/c mice (approx. 9 weeks of age; Charles River, Germany; n= 10 mice/group) receive daily a topical application for 6 days of 62.5mg of commercially available IMQ cream (5% Aldara©) on the beforehand (day -1) shaved (∼2cm x 3cm), depilated (Pilka© cream) back skin, and in addition 7mg on the outer right ear skin for 6 consecutive days, translating in a daily dose of approximately 3.5mg IMQ, which was published by van der Fits, Mourits, Voerman, Kant, Boon, Laman, Cornelissen, Mus, Florencia, Prens, Lubberts, J Immunol, 2009; 182: 5836-5845 to induce most optimal and reproducible a psoriasis-like phenotype in mice. Healthy control mice are included in the study and receive a vehicle cream (Vaseline) instead of IMQ.

Before starting treatment on the day of disease induction, animals are randomized based on body weight to the respective treatment group. The oral treatment with different dosages of the test substance is starting with the disease induction on day 0. Next to healthy controls, a disease control group is included. Both control groups are treated p.o. with vehicle of the test substance only.

On a daily base the psoriasis severity on the back skin of each mouse is evaluated by using a modified scoring system developed by *van der Fits et al.* (van der Fits, Mourits, Voerman, Kant, Boon, Laman, Cornelissen, Mus, Florencia, Prens, Lubberts, J Immunol, 2009; 182: 5836-5845), which is based on the clinical PASI (psoriasis area and severity index) and takes erythema, scaling and skin thickening into account. Each parameter is thereby scored independently on a scale from 0 to 3 (0 = none; 1 = slight; 2 = moderate; 3 = marked). The cumulative score (erythema plus scaling plus skin thickening), named PASI score (scale 0 to 9), serves then as a marker for the severity of back skin inflammation. In addition, ear thickness of the right ear is determined daily by the use of an automatized caliper (Bayer AG, Germany). At day 6, animals are sacrificed, and the right ear of each mouse is collected for *ex vivo* analysis of inflammation markers (e.g. cytokines, elastase activity). Each ear is frozen in liquid nitrogen and stored at -80°C until processing for inflammation marker analysis.

Skin samples from the back are collected in addition for histopathology evaluation (after hematoxylin and eosin (HE) staining).

The aim of the experiment is to demonstrate efficacy of the compounds by inhibition of inflammatory skin diseases, such as psoriasis.

### Example Bio 6

### In vivo - Letrozole-induced Polycystic Ovary Syndrome in rats

The compounds of the general formula (I) are tested for their effects in a Polycystic Ovary Syndrome (PCOS) model in rats.

Han Wistar rats are randomly divided into 3 groups with n=8 animals per group. Rats in the control group receive vehicle (for example a solutol, ethanol, or water-based vehicle) only once daily per os (po), whereas rats of the other two groups are administered letrozole at a concentration of 1 mg/kg body weight dissolved in 0.5 % carboxymethylcellulose (CMC) once daily for consecutive 28 days. Animals of the third group receiving letrozole additionally receive the test compound once daily using for example a solutol, ethanol, water-based vehicle orally dispensed for consecutive 28 days. Vaginal smears are performed, and the rat weights are recorded daily. At day 27 animals are fasted. An oral glucose tolerance test is performed, and weight of the animals is determined. Rats are euthanized, ovaries are removed and weighed. One ovary of each rat is fixed in formaldehyde for histological examination, whereas the other ovary is stored at -80°C for mRNA and protein analyses. Fat tissue and liver are also removed and from each tissue one aliquot is fixed in formaldehyde and the other aliquot is shock frozen for further mRNA analyses.

Vaginal Smears: the stage of the estrous cycle is determined by microscopic analysis of the predominant cell type in the daily vaginal smears.

Ovarian morphology: sections from the ovarian tissue are taken from the part of the ovary with largest diameter, stained with hematoxylin and eosin. Aim of the experiment is demonstrate efficacy of the test compound on the inhibition of letrozol induced PCOS phenotype compared to the control group

### Example Bio 7

### In vivo adjuvant-induced arthritis (AIA) model in the rat. Said model is predictive for AOSD (adult morbus still's disease) as well, see Akt Rheumatol 2017; 42(01): 37-45.

To determine the anti-inflammatory activity of the compounds of the general formula (I), they are examined for their in vivo efficacy in a rat arthritis model. The rat AIA model is characterized by a rapid onset and progression to severe polyarticular inflammation leading to permanent joint malformation.

For this purpose, adjuvant-induced arthritis is initiated on day 0 in male Lewis rats (approximately 100-125g, Charles River Laboratories, Germany) by a subcutaneous injection of 100 µl complete Freund's adjuvant (CFA) (containing 100mg *M. tuberculosis* H37Ra [Difo Lab, Cat. No. -231141] dissolved in 6ml incomplete Freund's adjuvant [Difco Lab, Cat. No. - 263910]) at the base of the tail. Beforehand, animals are randomized based on their body weight with n = 8 rats per respective treatment or control group. Both a healthy control and a disease control group are included in the study. Each control group is given p.o. treatment only with the vehicle of the test substance. The oral treatment with different dosages of the test substance starts on day 0. Assessment of the arthritis severity is performed once pre-treatment (starting condition of the animals on day 0), and from day 8 onwards on a daily base until study end (day 20) by macroscopic observation of the disease activity score (rating of the severity of arthritis based on a points system). In this scoring system, according to the extent of joint inflammation, points were awarded from 0 to 4 for the presence of an erythema including joint swelling (0 = none; 1 = slight; 2 = moderate; 3 = distinct; 4 = severe) for both hind paws and added up. Next to this, the extent of joint swelling is detected by the hind paw volume (via the use of a plethysmometer [IITC Life Science Inc, USA]) and by an automatized caliper measuring the ankle sagittal x transversal (once pre-treatment and from day 8 onwards 3 times weekly). Statistical analysis is effected using single-factor variance analysis (ANOVA) and comparison with the control group by means of multiple comparative analysis (Dunnett's test).

Using the compounds of the general formula (I), the inhibition of disease severity in rat adjuvant-induced arthritis can be observed.

### Example Bio 8

### In vivo collagen antibody-induced arthritis (CAIA) model in the mouse

The anti-inflammatory effect of the inventive compounds of the general formula (I) was examined in a further murine arthritis model.

In mice, a primary i.v. injection of a collagen antibody-cocktail followed by a boost immunization with LPS induces an erosive polyarthritis (Holmdahl et al., APMIS 1989 97(7):575-84; McCann et al., Arthritis Res Ther, 2010 12(3): R107), and represents thereby an animal model for indications like rheumatoid arthritis, psoriatric arthritis, reactive arthritis and ankylosing spondylitis.

In this murine arthritis model, the anti-inflammatory activity of the compounds of the general formula (I) are evaluated. At day 0, female Balb/c mice (approximately 7 weeks old, Charles River Laboratories, Germany; n= 8 mice/group) are administered i.v. with 400µl/mouse of a collagen-antibody cocktail (10mg/ml dissolved in PBS; ArthritoMab, MD Bioproducts, USA). Healthy controls, which are included in the study, receive PBS only. Before starting treatment on the day of disease induction, animals are randomized based on body weight to the respective treatment group. The treatment with different dosages of the test substance is conducted in a preventative manner, i.e. starting from day 0, by oral administration. Next to healthy controls, a disease control group is included. Both control groups are treated p.o. with vehicle of the test substance only. The boost immunization with endotoxin is performed on day 3 by an intraperitoneal injection of 100µl LPS (0.5mg/ml; ArthritoMab Kit, MD Bioproducts, USA) per animal.

The assessment of the arthritis severity is visually evaluated once before onset of disease (day 0) and in a daily fashion after disease development (> day 8) by the disease activity score based on redness and swelling of arthritic joints using the scoring system described by *Nandakumar & Holmendahl* (Nandakumar KS, Holmdahl R. Collagen antibody induced arthritis. Methods Mol Med. 2007; 136:215-23). In this scoring system each inflamed toe or knuckle gives one point, whereas an inflamed wrist or ankle gives five points, resulting in a score of between 0 and 15 (5 toes + 5 knuckles + 5 wrist/ankle) for each paw and between 0 and max. 60 points for each mouse. Next to this, pain-related functional disability is determined by the grip strength of each mouse via a grip strength meter (IITC Life Science Inc., USA). Furthermore, joint swelling either is determined by measuring of joint thickness (sagittal x transversal in mm) with an automatized caliper (Bayer AG, Germany) or by analysis of hind paw volume via a plethysmomether (IITC Life Science Inc., USA). At day 14 the experiment is terminated and biopsies of joints from hind paws are taken for histopathological analysis and for ex vivo analysis of pro-inflammatory markers in the arthritic joint tissue. The histopathological evaluation is performed in a blinded fashion using a scoring system which rates the pathological joint inflammation and damage by points (0 = none; 1 = slight; 2 = moderate; 3 = severe), analyzing parameters like inflammation/immune cell infiltration, synovial hyperplasia, pannus formation, cartilage degradation, bone damage, periosteal changes and vasculitis.

Statistical analysis is affected using single-factor variance analysis (ANOVA) and comparison with the control group by means of multiple comparative analysis (Dunnett's test).

Using the compounds of the general formula (I), the inhibition of disease severity in mouse collagen antibody-induced arthritis can be observed.

### Example Bio 9

### In vivo MOG₃₃₋₃₅-induced chronic EAE (Experimental Autoimmune Encephalomyelitis) model in mice

To determine the anti-inflammatory activity of the compounds of the general formula (I), these are examined in an experimental animal model of multiple sclerosis (MS) for their in vivo efficacy. The chronic MOG33-35 (myelin oligodendrocyte glycoprotein)-induced EAE (experimental autoimmune encephalomyelitis) model is the standard animal model for testing pharmacological substances for potential use in MS patients.

The compounds of the general formula (I) are evaluated regarding their effects in multiple sclerosis (MS) using an experimental autoimmune encephalomyelitis (EAE) model in mice.

Fur this purpose, female C57BL/6 mice (19-20g; Jackson Laboratories, Bar Harbour, USA; n= 10-15 mice/group) are randomized based on the body weight. EAE is induced on day 0 by a single subcutaneous injection of 0.1ml on the back of each mouse containing 200µg of MOG 33-35 peptide emulsified in 100µl CFA (complete Freund'sch adjuvant), 2mg/ml supplemented with *Mycobacterium tuberculosis* 8mg/ml; strain H37RA). In addition, each mouse receives a total of two i.p. injections of 200ng of pertussis toxin (PTx) dissolved in 0.1ml PBS (2µg/ml], with one injection on day 0 and another on day 2. A naïve, healthy control group as well as an EAE disease control is included in the study, and both are treated with vehicle of test substance only. Daily treatment with different oral doses of test substance starts with the disease induction on day 0. All mice are weighed daily. Symptoms of EAE are assessed also on a daily base, starting on day 4 and continuing until end of the study (day 30), according to the following scoring scale:

| **Score** | **Description** |
|---|---|
| 0 | Normal |
| 1 | Limp tail |
| 2 | Limp tail and weakness of hind legs |
| 3 | Limp tail and complete paralysis of hind legs (most common) |
| | -OR- Limp tail with paralysis of one front and one hind leg |
| | -OR- ALL of: Severe head tilting |
| 4 | Limp tail, complete hind leg and partial front leg paralysis |
| 5 | Complete hind and complete front leg paralysis, no movement around the |
| | cage -OR- Mouse is spontaneously rolling in the cage -OR- Mouse is |
| | found dead due to paralysis. |

Mice are euthanized when they have reached an EAE score of 5 (tetraplegia), or have reached a score of 4 on two consecutive days, or have lost 30% of their initial body weight. All surving mice are sacrificed at the completion of the study (day 30). Blood is collected for evaluation of inflammatory markers (e.g. cytokines). Animals are then perfused with formalin and the spinal cord is collected and stored in 10% formalin for subsequent histopathological evaluation analyzing for instance inflammation and demyelination.

Statistical analysis is affected using single-factor variance analysis (ANOVA) and comparison with the control group by means of multiple comparative analysis (Dunnett's test).

The aim of the experiment is to demonstrate inhibitory effects of the compounds of the general formula (I) in vivo in inflammatory CNS indications, like for instance MS.

### Example Bio 10

### In vivo adoptive transfer EAE model in mice

The compounds of the general formula (I) are evaluated regarding their therapeutic effects in MS using an adoptive transfer EAE model in mice.

In the adoptive transfer EAE model, donor mice are immunized to generate encephalitogenic T cells, which are then activated in cell culture and transferred into recipient mice to induce EAE. Because fully encephalitogenic T cells are transferred into the recipient mice, this is considered a model of therapeutic treatment.

Female donor SJL mice (Jackson Laboratories, Bar Harbour, USA) are immunized with PLP₁₃₉₋₁₅₁/CFA (Hooke Laboratories, USA) on day -13. Ten days later (day -3) spleen and lymphnodes (LNs) are harvested from all euthanized donor mice. The spleens and LNs are pooled and a cell suspension is prepared, which is cultured with 3.5 million cells/ml in T150 flasks in the presence of PLP₁₃₉₋₁₅₁ peptide (20µg/ml) for 3 consecutive days to generate encephalitogenic T cells. On day 0, EAE is induced in recipient mice. Therefore, the *in vitro* cultured cells are collected, spun down and resuspended in RPMI1640 media (without FCS) before they are injected (6 million cells/mouse) intraperitoneally into the randomized (based on body weight), recipient SJL mice (6 weeks of age). A healthy, naïve control group is included in the study, which is left uninjected. Next to the healthy control, an EAE disease control group is included in the study, and both are treated with vehicle of test substance only. Daily treatment of recipient mice with different oral doses of test substance starts with the adoptive EAE transfer of encephalitogenic cells on day 0. All mice are weighed 3 times per week starting day -1. Symptoms of EAE are assessed also on a daily base, starting on day 5 and continuing until termination of the study (day 20), according to the following scoring scale:

| **Score** | **Clinical observations** |
|---|---|
| 0 | Normal |
| 1 | Limp tail |
| 2 | Limp tail and weakness of hind legs |
| 3 | Limp tail and complete paralysis of hind legs (most common) |
| | -OR- Limp tail with paralysis of one front and one hind leg |
| | -OR- ALL of: Severe head tilting, walking only along the edges of the cage, pushing against the cage wall, spinning when pickedup by the tail |
| 4 | Limp tail, complete hind leg and partial front leg paralysis |
| 5 | Complete hind and complete front leg paralysis, no movement around the cage |
| | -OR- Mouse is spontaneously rolling in the cage |
| | -OR- Mouse is found dead due to paralysis. |

Mice are euthanized when they have reached an EAE score of 5 (tetraplegia) or have reached a score of 4 on two consecutive days, or have lost 30% of their initial body weight. All surving mice are sacrificed at the completion of the study (day 20). Blood is collected for evaluation of inflammatory markers (e.g. cytokines). Animals are then perfused with formalin and the spinal cord is collected and stored in 10% formalin for subsequent histopathological evaluation analyzing for instance inflammation and demyelination.

Statistical analysis is affected using single-factor variance analysis (ANOVA) and comparison with the control group by means of multiple comparative analysis (Dunnett's test).

The aim of the experiment is to demonstrate inhibitory effects of the compounds in vivo in inflammatory CNS indications, like for instance MS.

### Example Bio 11

### In vivo SLE model in MRL/MpJ-Fas^{lpr}/J mice

The compounds of the general formula (I) are evaluated regarding their therapeutic effects on systemic lupus erythematosus (SLE) in MRL/MpJ-Fas^{lpr}/J mice, which is one of the most commonly used spontaneous models of SLE.
MRL/MpJ-Fas^{lpr}/J mice are homozygous for spontaneous mutation in the Fas gene. As a result of the mutation, aberrant T cells (CD4⁻CD8⁻, TCR^{-low}) in these mice proliferate much more than in normal mice, leading to development of enlarged lymph nodes and spleen. Autoimmune responses occur spontaneously in these mice, resulting in arthritis and glomerulonephritis, with production of anti-nuclear antibodies detectable as early as 8 weeks of age. Extensive kidney pathology is found at 4 to 7 months of age. The pathology resembles SLE in people.
The study starts when female MRL/MpJ-Fas^{lpr}/J mice (Jackson Laboratories, Bar Harbour, USA; n= 15 mice/group) are 11 weeks old, all having detectable concentrations of protein in urine. At 12 weeks of age, the mice are assigned to the treatment groups in a balanced manner, to achieve similar average body weight and similar proteinuria across the groups. A SLE disease control group, treated with vehicle of test substance only, was included in the study. Daily treatment of different oral doses of test substance started with the group assignment and continued until the end of the study (20 weeks of age). During the duration of the study mice are weighed weekly starting at week 11. Next to this, once weekly the protein concentration in the collected 24h urine is dertermined by urine dip sticks (Roche Diagnostics Chemstrip 2GP, Germany). Proteinuria is expressed as a score from 0 to 4:
**0** - no protein
**1** - traces of protein (< 30 mg/dL)
**2** - 30-100 mg/dL
**3** - 100-500 mg/dL
**4** - > 500 mg/dL
At 20 weeks of age, study is terminated, and serum is collected to measure anti-dsDNA antibodies from each individual mouse using ELISA. Furthermore, Kidney is collected and analyzed regarding glomerular, tubular and interstitial changes by histopathology. Statistical analysis is affected using single-factor variance analysis (ANOVA) and comparison with the control group by means of multiple comparative analysis (Dunnett's test).
The aim of the experiment is to demonstrate inhibitory effects of the compounds in a murine SLE model.

The compounds of general formula (I) have surprisingly been found to effectively inhibit IRAK4 for which data are given in biological experimental section, see example Bio 1 as shown above.
In addition, the compounds of formula (I) show high stability in the presence of human liver microsomes indicating a favorable pharmacokinetic profile (see example Bio 2). Selected compounds show also high potency in a cellular assay based on PBMCs (see example Bio 3). Overall, the data of the present invention indicate that compounds of formula (I) can be used for a variety of indications such as for treatment or prophylaxis of proliferative disorders, of autoimmune disorders, of metabolic and inflammatory disorders, for example rheumatoid arthritis, spondyloarthritis (especially psoriatic spondyloarthritis and Bechterev's disease), chronic obstructive pulmonary disease (abbreviation: COPD), multiple sclerosis, systemic lupus erythematosus, psoriasis, gout, metabolic syndrome, fatty liver hepatitis, insulin resistance, polycystic ovary syndrome (PCOS), endometriosis and inflammation-induced or chronic pain, and of lymphoma in human as well as of allergic and/or inflammatory diseases in animals, especially of atopic dermatitis and/or Flea Allergy Dermatitis, and especially in domestic animals, particularly in dogs..
The compounds of formula (I) also surprisingly inhibit the IRAK4 enzyme in a biochemical assay based on canine enzyme. The compounds show high stability in the presence of dog liver microsomes indicating a favorable pharmacokinetic profile.

## Claims

1. Compounds of general formula (I): 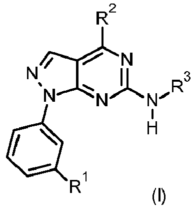 wherein
R¹ represents C₁-C₄-alkyl, C₁-C₄-alkoxy, C₁-C₆-alkylsulfonyl, or cyano,
• wherein said C₁-C₄-alkyl is optionally substituted with one or more substituents which are the same or different and independently selected from the group consisting of fluorine atom and hydroxy, and
• wherein said C₁-C₄-alkoxy is substituted with 1 to 6 fluorine atoms;
R² represents 4- to 7-membered monocyclic saturated heterocycloalkyl containing one ring nitrogen atom and optionally one additional heteroatom or heteroatom-containing group independently selected from the group consisting of N, O, S, S(=O), S(=O)₂ and S(=O)(=NH),
• wherein said 4- to 7-membered monocyclic saturated heterocycloalkyl is bound to the rest of the molecule via any nitrogen atom, and
• wherein said 4- to 7-membered monocyclic saturated heterocycloalkyl is optionally substituted with one or more substituents which are the same or different and independently selected from the group consisting of an oxo substituent, halogen, hydroxy, cyano, C₁-C₆-alkoxy, CO₂H, NH₂, NR^{a}R^{b}, -S(=O)₂R^{b}, -S(=O)₂R⁴ -(C=O)R^{b}, -(C=O)NH₂, -(C=O)NR^{a}R^{b}, -(C=O)R⁴, R⁵, R⁶, R⁷, R⁸, and R⁹;
R³ represents 4- to 7-membered monocyclic saturated heterocycloalkyl containing one to two heteroatom or heteroatom-containing groups selected from N, O, S, S(=O), S(=O)₂ and S(=O)(=NH), C₃-C₆-cycloalkyl, 6- to 11-membered heterospirocycloalkyl containing one heteroatom or heteroatom-containing group independently selected from N, O and S(=O)₂, or 6- to 12-membered bridged heterocycloalkyl group containing one heteroatom or heteroatom-containing group independently selected from N, O, and S(=O)₂,
• wherein said 4- to 7-membered monocyclic saturated heterocycloalkyl, said 6- to 11-membered heterospirocycloalkyl and said 6- to 12-membered bridged heterocycloalkyl group are bound to the rest of the molecule via any of the carbon atoms, and
• wherein said 4- to 7-membered monocyclic saturated heterocycloalkyl is optionally substituted with one or more substituents which are the same or different and independently selected from the group consisting of oxo substituent, halogen, hydroxy, cyano, CO₂H, C₁-C₆-alkoxy, NH₂, NR^{a}R^{b}, -S(=O)₂R^{b}, -S(=O)₂R^{b}, -S(=O)₂NR^{a}R^{b}, cyclopropyl, cyclobutyl, -CH₂-CH₂-forming a cyclopropyl group together with any carbon atom of the 4- to 7-membered saturated heterocycloalkyl, and C₁-C₆-alkyl optionally substituted with one to five halogens atoms or with one hydroxy,
• wherein said C₃-C₆-cycloalkyl is optionally substituted with one or more substituents which are the same or different and independently selected from the group consisting of oxo substituent, halogen, hydroxy, cyano, CO₂H, C₁-C₆-alkoxy, NH₂, NR^{a}R^{b}, -S(=O)₂R^{b}, -S(=O)₂NH₂, -S(=O)₂NR^{a}R^{b}, C(=O)R^{b}, -C(=O)NH₂, -C(=O)NR^{a}R^{b}, and C₁-C₆-alkyl, wherein said C₁-C₆-alkyl is optionally substituted with one to five halogen atoms or with one hydroxy,
• wherein said 6- to 11-membered heterospirocycloalkyl is optionally substituted with one or more substituents which are the same or different and independently selected from the group consisting of C₁-C₄-alkyl, fluorine atom, hydroxy, cyano, CO₂H, NH₂, and -S(=O)₂R^{b}, and
• wherein said 6- to 12-membered bridged heterocycloalkyl group is optionally substituted with one or more substituent which are the same or different and independently selected from the group consisting of C₁-C₆-alkyl, fluorine atom, and hydroxy;
R⁴ represents 4- to 7-membered monocyclic saturated heterocycloalkyl group containing one nitrogen atom and optionally one additional heteroatom or heteroatom-containing group independently selected from N, O, and S(=O)₂, or 6- to 12-membered bridged heterocycloalkyl containing one nitrogen atom and optionally one additional heteroatom or heteroatom-containing group independently selected from the group consisting of N, O, and S(=O)₂,
• wherein said 4- to 7-membered monocyclic saturated heterocycloalkyl and said 6- to 12-membered bridged heterocycloalkyl are bound to the rest of the molecule via any nitrogen atom,
• wherein said 4- to 7-membered monocyclic saturated heterocycloalkyl and said 6- to 12-membered bridged heterocycloalkyl are optionally substituted with one or more substituents which are the same or different and independently selected from the group consisting of C₁-C₆-alkyl, fluorine atom, and hydroxy;
R⁵ represents C₁-C₆-alkyl group optionally substituted with one or more substituents which are the same or different and independently selected from the group consisting of halogen, hydroxy, CO₂H, cyclopropyl, cyano, C₁-C₆-alkoxy, NH₂, NR^{a}R^{b}, -S(=O)₂R^{b}, -S(=O)₂R⁴ -(C=O)R^{b}, -(C=O)NH₂,-(C=O)R^{a}R^{b}, -(C=O)R⁴, 4- to 7-membered monocyclic saturated heterocycloalkyl containing one or two identical or different heteroatoms or heteroatom-containing groups independently selected from the group consisting of N, O, S, S(=O), and S(=O)₂, 6- to 12-membered bridged heterocycloalkyl group containing one nitrogen atom and optionally one additional heteroatom or heteroatom-containing group independently selected from the group consisting of N, O, and S(=O)₂, and 6- to 11-membered heterospirocycloalkyl containing one nitrogen atom and optionally one additional heteroatom or heteroatom-containing group independently selected from the group consisting of N, O, and S(=O)₂,
• wherein said 6- to 12-membered bridged heterocycloalkyl group and said 6- to 11-membered heterospirocycloalkyl are connected to the rest of the molecule via any nitrogen atom, and
• wherein said 4- to 7-membered monocyclic saturated heterocycloalkyl and said 6- to 12-membered bridged heterocycloalkyl group are optionally substituted with one or more substituents which are the same or different and independently selected from the group consisting of C₁-C₆-alkyl, fluorine atom, oxo substituent, and hydroxy, and
• wherein said 6- to 11-membered heterospirocycloalkyl is optionally substituted with one or more substituents which are the same or different and independently selected from the group consisting of C₁-C₄-alkyl, fluorine atom, hydroxy, cyano, CO₂H, NH₂, and -S(=O)₂R^{b};
R⁶ represents -C₃-C₆-cycloalkyl optionally substituted with one or more substituents which are the same or different and independently selected from the group consisting of halogen, C₁-C₆-alkyl, hydroxy, NH₂, and NR^{a}R^{b};
R⁷ represents 4- to 7-membered monocyclic saturated heterocycloalkyl containing one or two identical or different heteroatoms or heteroatom-containing groups independently selected from the group consisting of N, O, S, S(=O), S(=O)₂ and S(=O)(=NH),
• wherein said 4- to 7-membered monocyclic saturated heterocycloalkyl is optionally substituted with one or more substituents which are the same or different and independently selected from the group consisting of an oxo substituent, halogen, hydroxy, NH₂, NR^{a}R^{b}, cyano, C₁-C₆-alkoxy, CO₂H, -S(=O)₂R^{b}, and C₁-C₆-alkyl,
- wherein said C₁-C₆-alkyl is optionally substituted with one or more substituents which are the same or different and independently selected from the group consisting of fluorine atom, hydroxy, NH₂, and NR^{a}R^{b};
R⁸ represents 6- to 12-membered bridged heterocycloalkyl containing one nitrogen atom and optionally one additional heteroatom or heteroatom-containing group independently selected from the group consisting of N, O, and S(=O)₂,
• wherein said 6- to 12-membered bridged heterocycloalkyl is connected to the rest of the molecule via any nitrogen atom, and
• wherein said 6- to 12-membered bridged heterocycloalkyl is optionally substituted with one or more substituents which are the same or different and independently selected from the group consisting of C₁-C₆-alkyl, fluorine atom, and hydroxy;
R⁹ represents 6- to 11-membered heterospirocycloalkyl containing one nitrogen atom and optionally one additional heteroatom or heteroatom-containing group independently selected from the group consisting of N, O and S(=O)₂,
• wherein said 6- to 11-membered heterospirocycloalkyl is connected to the rest of the molecule via any nitrogen atom, and
• wherein said 6- to 11-membered heterospirocycloalkyl is optionally substituted with one or more substituents which are the same or different and independently selected from the group consisting of C₁-C₄-alkyl, fluorine atom, hydroxy, cyano, CO₂H, NH₂, and -S(=O)₂C₁-C₆-alkyl;
R^{a} represents hydrogen or C₁-C₆-alkyl,
R^{b} represents C₁-C₆-alkyl, and
R^{c} represents C₃-C₆-cycloalkyl;
and stereoisomers, tautomers, N-oxides, hydrates, solvates, and salts thereof, and mixtures of same.

2. A compound according to claim 1, namely
| Example | Name |
|---|---|
| I-1-1 | (4S)-4-({4-[3-(morpholin-4-yl)azetidin-1-yl]-1-[3-(trifluoromethyl)phenyl]-1H-pyrazolo[3,4-d]pyrimidin-6-yl}amino)pyrrolidin-2-one |
| I-1-2 | (4R)-4-({4-[3-(morpholin-4-yl)azetidin-1-yl]-1-[3-(trifluoromethyl)phenyl]-1H-pyrazolo[3,4-d]pyrimidin-6-yl}amino)pyrrolidin-2-one |
| I-2-1 | (4S)-4-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one |
| I-2-2 | ((4R)-4-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one |
| I-2-3 | 4-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one |
| I-3-1 | (4S)-4-({4-(3-ethyl-3-hydroxyazetidin-1-yl)-1-[3-(trifluoromethyl)phenyl]-1H-pyrazolo[3,4-d]pyrimidin-6-yl}amino)pyrrolidin-2-one |
| I-3-2 | (4R)-4-({4-(3-ethyl-3-hydroxyazetidin-1-yl)-1-[3-(trifluoromethyl)phenyl]-1H-pyrazolo[3,4-d]pyrimidin-6-yl}amino)pyrrolidin-2-one |
| I-4-1 | 2-(1-{6-[(1,1-dioxidotetrahydrothiophen-3-yl)amino]-1-[3-(trifluoromethyl)phenyl]-1H-pyrazolo[3,4-d]pyrimidin-4-yl}piperidin-4-yl)propan-2-ol |
| I-4-2 | (+)-2-(1-{6-[(1,1-dioxidotetrahydrothiophen-3-yl)amino]-1-[3-(trifluoromethyl)phenyl]-1H-pyrazolo[3,4-d]pyrimidin-4-yl}piperidin-4-yl)propan-2-ol |
| I-4-3 | (-)-2-(1-{6-[(1,1-dioxidotetrahydrothiophen-3-yl)amino]-1-[3-(trifluoromethyl)phenyl]-1H-pyrazolo[3,4-d]pyrimidin-4-yl}piperidin-4-yl)propan-2-ol |
| I-5 | 2-(1-{6-[(1,1-dioxidotetrahydro-2H-thiopyran-4-yl)amino]-1-[3-(trifluoromethyl)phenyl]-1H-pyrazolo[3,4-d]pyrimidin-4-yl}piperidin-4-yl)propan-2-ol |
| I-6 | 4-({4-[4-(2-hydroxypropan-2-yl)piperidin-1-yl]-1-[3-(trifluoromethyl)phenyl]-1H-pyrazolo[3,4-d]pyrimidin-6-yl}amino)-5,5-dimethylpyrrolidin-2-one |
| I-7-1 | (5R)-5-({4-[4-(2-hydroxypropan-2-yl)piperidin-1-yl]-1-[3-(trifluoromethyl)phenyl]-1H-pyrazolo[3,4-d]pyrimidin-6-yl}amino)piperidin-2-one |
| I-7-2 | (5S)-5-({4-[4-(2-hydroxypropan-2-yl)piperidin-1-yl]-1-[3-(trifluoromethyl)phenyl]-1H-pyrazolo[3,4-d]pyrimidin-6-yl}amino)piperidin-2-one |
| I-8 | 4-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylpyrrolidin-2-one |
| I-9 | (3R)-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylpyrrolidin-2-one |
| I-10 | 2-(1-{6-[(1,1-dioxidotetrahydro-2H-thiopyran-3-yl)amino]-1-[3-(trifluoromethyl)phenyl]-1H-pyrazolo[3,4-d]pyrimidin-4-yl}piperidin-4-yl)propan-2-ol |
| I-11-1 | (3S)-3-({4-[4-(2-hydroxypropan-2-yl)piperidin-1-yl]-1-[3-(trifluoromethyl)phenyl]-1H-pyrazolo[3,4-d]pyrimidin-6-yl}amino)piperidin-2-one |
| I-11-2 | (3R)-3-({4-[4-(2-hydroxypropan-2-yl)piperidin-1-yl]-1-[3-(trifluoromethyl)phenyl]-1H-pyrazolo[3,4-d]pyrimidin-6-yl}amino)piperidin-2-one |
| I-12 | (3S)-3-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one |
| I-13-1 | 2-[1-(6-{[(3S)-1-methylpyrrolidin-3-yl]amino}-1-[3-(trifluoromethyl)phenyl]-1H-pyrazolo[3,4-d]pyrimidin-4-yl)piperidin-4-yl]propan-2-ol |
| I-13-2 | 2-[1-(6-{[(3R)-1-methylpyrrolidin-3-yl]amino}-1-[3-(trifluoromethyl)phenyl]-1H-pyrazolo[3,4-d]pyrimidin-4-yl)piperidin-4-yl]propan-2-ol |
| I-14 | 2-[1-(6-{[1-(methylsulfonyl)azetidin-3-yl]amino}-1-[3-(trifluoromethyl)phenyl]-1H-pyrazolo[3,4-d]pyrimidin-4-yl)piperidin-4-yl]propan-2-ol |
| I-15 | (3R)-3-({4-[4-(2-hydroxypropan-2-yl)piperidin-1-yl]-1-[3-(trifluoromethyl)phenyl]-1H-pyrazolo[3,4-d]pyrimidin-6-yl}amino)pyrrolidin-2-one |
| I-16-1 | 2-(1-{6-[(3S)-tetrahydrofuran-3-ylamino]-1-[3-(trifluoromethyl)phenyl]-1H-pyrazolo[3,4-d]pyrimidin-4-yl}piperidin-4-yl)propan-2-ol |
| I-16-2 | 2-(1-{6-[(3R)-tetrahydrofuran-3-ylamino]-1-[3-(trifluoromethyl)phenyl]-1H-pyrazolo[3,4-d]pyrimidin-4-yl}piperidin-4-yl)propan-2-ol |
| I-17 | (4S)-4-({4-[4-(2-hydroxypropan-2-yl)piperidin-1-yl]-1-[3-(trifluoromethyl)phenyl]-1H-pyrazolo[3,4-d]pyrimidin-6-yl}amino)-3,3-dimethylpyrrolidin-2-one |
| I-18 | 3-ethyl-1-(6-{[(3S)-1-methylpyrrolidin-3-yl]amino}-1-[3-(trifluoromethyl)phenyl]-1H-pyrazolo[3,4-d]pyrimidin-4-yl)azetidin-3-ol |
| I-19 | 1-{6-[(1,1-dioxidotetrahydro-2H-thiopyran-4-yl)amino]-1-[3-(trifluoromethyl)phenyl]-1H-pyrazolo[3,4-d]pyrimidin-4-yl}-3-ethylazetidin-3-ol |
| I-20-1 | (4R)-4-({4-[(3S,4S)-3,4-dihydroxypyrrolidin-1-yl]-1-[3-(trifluoromethyl)phenyl]-1H-pyrazolo[3,4-d]pyrimidin-6-yl}amino)pyrrolidin-2-one |
| I-20-2 | (4S)-4-({4-[(3S,4S)-3,4-dihydroxypyrrolidin-1-yl]-1-[3-(trifluoromethyl)phenyl]-1H-pyrazolo[3,4-d]pyrimidin-6-yl}amino)pyrrolidin-2-one |
| I-21 | (4S)-4-({4-[(3R)-3-(morpholin-4-yl)pyrrolidin-1-yl]-1-[3-(trifluoromethyl)phenyl]-1H-pyrazolo[3,4-d]pyrimidin-6-yl}amino)pyrrolidin-2-one |
| I-22 | N-[(3R)-1,1-dioxidotetrahydrothiophen-3-yl]-4-[3-(morpholin-4-yl)azetidin-1-yl]-1-[3-(trifluoromethyl)phenyl]-1H-pyrazolo[3,4-d]pyrimidin-6-amine |
| I-23 | N-(1,1-dioxidotetrahydro-2H-thiopyran-4-yl)-4-[3-(morpholin-4-yl)azetidin-1-yl]-1-[3-(trifluoromethyl)phenyl]-1H-pyrazolo[3,4-d]pyrimidin-6-amine |
| I-24 | (4S)-4-({4-[4-(2-hydroxy-2-methylpropyl)piperidin-1-yl]-1-[3-(trifluoromethyl)phenyl]-1H-pyrazolo[3,4-d]pyrimidin-6-yl}amino)pyrrolidin-2-one |
| I-25-1 | 2-methyl-1-[1-(6-{[(3R)-1-methylpyrrolidin-3-yl]amino}-1-[3-(trifluoromethyl)phenyl]-1H-pyrazolo[3,4-d]pyrimidin-4-yl)piperidin-4-yl]propan-2-ol |
| I-25-2 | 2-methyl-1-[1-(6-{[(3S)-1-methylpyrrolidin-3-yl]amino}-1-[3-(trifluoromethyl)phenyl]-1H-pyrazolo[3,4-d]pyrimidin-4-yl)piperidin-4-yl]propan-2-ol |
| I-26-1 | 4-[3-(1,1-dioxidothiomorpholin-4-yl)azetidin-1-yl]-N-[(3R)-tetrahydrofuran-3-yl]-1-[3-(trifluoromethyl)phenyl]-1H-pyrazolo[3,4-d]pyrimidin-6-amine |
| I-26-2 | 4-[3-(1,1-dioxidothiomorpholin-4-yl)azetidin-1-yl]-N-[(3S)-tetrahydrofuran-3-yl]-1-[3-(trifluoromethyl)phenyl]-1H-pyrazolo[3,4-d]pyrimidin-6-amine |
| I-27 | (4S)-4-({4-[4-(morpholin-4-yl)piperidin-1-yl]-1-[3-(trifluoromethyl)phenyl]-1H-pyrazolo[3,4-d]pyrimidin-6-yl}amino)pyrrolidin-2-one |
| I-28 | (4S)-4-({4-(morpholin-4-yl)-1-[3-(trifluoromethyl)phenyl]-1H-pyrazolo[3,4-d]pyrimidin-6-yl}amino)pyrrolidin-2-one |
| I-29 | (1 R,3S)-3-({4-[4-(2-hydroxypropan-2-yl)piperidin-1-yl]-1-[3-(trifluoromethyl)phenyl]-1H-pyrazolo[3,4-d]pyrimidin-6-yl}amino)cyclopentanol |
| I-30 | (4S)-4-({4-[3-(4-hydroxy-4-methylpiperidin-1-yl)azetidin-1-yl]-1-[3-(trifluoromethyl)phenyl]-1H-pyrazolo[3,4-d]pyrimidin-6-yl}amino)pyrrolidin-2-one |
| I-31 | (3R,4R)-1-(6-{[(3S)-1-methylpyrrolidin-3-yl]amino}-1-[3-(trifluoromethyl)phenyl]-1H-pyrazolo[3,4-d]pyrimidin-4-yl)pyrrolidine-3,4-diol |
| I-32 | (4S)-4-({4-(3-hydroxyazetidin-1-yl)-1-[3-(trifluoromethyl)phenyl]-1 H-pyrazolo[3,4-d]pyrimidin-6-yl}amino)pyrrolidin-2-one |
| I-33 | N-[(3S)-1,1-dioxidotetrahydrothiophen-3-yl]-4-[4-(morpholin-4-yl)piperidin-1-yl]-1-[3-(trifluoromethyl)phenyl]-1H-pyrazolo[3,4-d]pyrimidin-6-amine |
| I-34 | N-[(3S)-1,1-dioxidotetrahydrothiophen-3-yl]-4-[3-(morpholin-4-yl)azetidin-1-yl]-1-[3-(trifluoromethyl)phenyl]-1H-pyrazolo[3,4-d]pyrimidin-6-amine |
| I-35 | (4S)-4-[(4-{3-[(methylsulfonyl)methyl]azetidin-1-yl}-1-[3-(trifluoromethyl)phenyl]-1H-pyrazolo[3,4-d]pyrimidin-6-yl)amino]pyrrolidin-2-one |
| I-36 | N-[(3S)-1-methylpyrrolidin-3-yl]-4-{3-[(methylsulfonyl)methyl]azetidin-1-yl}-1-[3-(trifluoromethyl)phenyl]-1 H-pyrazolo[3,4-d]pyrimidin-6-amine |
| I-37 | rel-(3R,4R)-4-({4-[3-(morpholin-4-yl)azetidin-1-yl]-1-[3-(trifluoromethyl)phenyl]-1H-pyrazolo[3,4-d]pyrimidin-6-yl}amino)tetrahydrothiophene-3-ol 1,1-dioxide |
| I-38 | (4S)-4-({4-[3-(dimethylamino)-3-methylazetidin-1-yl]-1-[3-(trifluoromethyl)phenyl]-1H-pyrazolo[3,4-d]pyrimidin-6-yl}amino)pyrrolidin-2-one |
| I-39 | 4-[3-(dimethylamino)-3-methylazetidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine |
| I-40 | 2-[1-(6-{[(3S,5S)-5-methylpyrrolidin-3-yl]amino}-1-[3-(trifluoromethyl)phenyl]-1H-pyrazolo[3,4-d]pyrimidin-4-yl)piperidin-4-yl]propan-2-ol |
| I-41-1 | 3-methyl-1-(6-{[(3R)-1-(methylsulfonyl)pyrrolidin-3-yl]amino}-1-[3-(trifluoromethyl)phenyl]-1H-pyrazolo[3,4-d]pyrimidin-4-yl)azetidin-3-ol |
| I-41-2 | 3-methyl-1-(6-{[(3S)-1-(methylsulfonyl)pyrrolidin-3-yl]amino}-1-[3-(trifluoromethyl)phenyl]-1H-pyrazolo[3,4-d]pyrimidin-4-yl)azetidin-3-ol |
| I-42 | 4-[3-(dimethylamino)azetidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine |
| I-43 | N-(3-methyl-1,1-dioxidotetrahydrothiophen-3-yl)-4-[3-(morpholin-4-yl)azetidin-1-yl]-1-[3-(trifluoromethyl)phenyl]-1H-pyrazolo[3,4-d]pyrimidin-6-amine |
| I-44 | (4S)-4-({4-[3-(dimethylamino)azetidin-1-yl]-1-[3-(trifluoromethyl)phenyl]-1H-pyrazolo[3,4-d]pyrimidin-6-yl}amino)pyrrolidin-2-one |
| I-45 | 4-[3-(2-aminopropan-2-yl)azetidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine |
| I-46 | 4-[4-(2-aminopropan-2-yl)piperidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine |
| I-47 | (4S)-4-({4-[4-(2-aminopropan-2-yl)piperidin-1-yl]-1-[3-(trifluoromethyl)phenyl]-1H-pyrazolo[3,4-d]pyrimidin-6-yl}amino)pyrrolidin-2-one |
| I-48 | (4S)-4-({4-[4-(1-aminocyclopropyl)piperidin-1-yl]-1-[3-(trifluoromethyl)phenyl]-1H-pyrazolo[3,4-d]pyrimidin-6-yl}amino)pyrrolidin-2-one |
| I-49 | (4S)-4-({4-[3-(2-hydroxy-2-methylpropyl)azetidin-1-yl]-1-[3-(trifluoromethyl)phenyl]-1H-pyrazolo[3,4-d]pyrimidin-6-yl}amino)pyrrolidin-2-one |
| I-50 | [1-(6-{[(3S)-5-oxopyrrolidin-3-yl]amino}-1-[3-(trifluoromethyl)phenyl]-1 H-pyrazolo[3,4-d]pyrimidin-4-yl)azetidin-3-yl]acetic acid |
| I-51 | (4S)-4-[(4-{3-[2-(morpholin-4-yl)-2-oxoethyl]azetidin-1-yl}-1-[3-(trifluoromethyl)phenyl]-1H-pyrazolo[3,4-d]pyrimidin-6-yl)amino]pyrrolidin-2-one |
| I-52 | (4S)-4-[(4-{3-[2-(6-oxa-3-azabicyclo[3.1.1]heptan-3-yl)-2-oxoethyl]azetidin-1-yl}-1-[3-(trifluoromethyl)phenyl]-1H-pyrazolo[3,4-d]pyrimidin-6-yl)amino]pyrrolidin-2-one |
| I-53 | N-cyclopropyl-4-[3-(1,1-dioxidothiomorpholin-4-yl)azetidin-1-yl]-1-[3-(trifluoromethyl)phenyl]-1H-pyrazolo[3,4-d]pyrimidin-6-amine |
| I-54 | N-(3,3-difluorocyclobutyl)-4-[3-(dimethylamino)azetidin-1-yl]-1-[3-(trifluoromethyl)phenyl]-1H-pyrazolo[3,4-d]pyrimidin-6-amine |
| I-55 | N-[cis-3-(methylsulfonyl)cyclobutyl]-4-[3-(morpholin-4-yl)azetidin-1-yl]-1-[3-(trifluoromethyl)phenyl]-1H-pyrazolo[3,4-d]pyrimidin-6-amine |
| I-56 | N-[trans-3-(methylsulfonyl)cyclobutyl]-4-[3-(morpholin-4-yl)azetidin-1-yl]-1-[3-(trifluoromethyl)phenyl]-1H-pyrazolo[3,4-d]pyrimidin-6-amine |
| I-57 | 2-(1-{6-[(trans-3-amino-3-methylcyclobutyl)amino]-1-[3-(trifluoromethyl)phenyl]-1H-pyrazolo[3,4-d]pyrimidin-4-yl}piperidin-4-yl)propan-2-ol |
| I-58 | cis-1-methyl-N³-{4-(morpholin-4-yl)-1-[3-(trifluoromethyl)phenyl]-1H-pyrazolo[3,4-d]pyrimidin-6-yl}cyclobutane-1,3-diamine |
| I-59 | 4-[3-methyl-3-(morpholin-4-yl)azetidin-1-yl]-N-[trans-3-(methylsulfonyl)cyclobutyl]-1-[3-(trifluoromethyl)phenyl]-1H-pyrazolo[3,4-d]pyrimidin-6-amine |
| I-60 | N-(1,1-dioxidotetrahydro-2H-thiopyran-4-yl)-4-[3-(morpholin-4-ylmethyl)azetidin-1-yl]-1-[3-(trifluoromethyl)phenyl]-1H-pyrazolo[3,4-d]pyrimidin-6-amine |
| I-61 | 4-methyl-1-[1-(6-{[trans-3-(methylsulfonyl)cyclobutyl]amino}-1-[3-(trifluoromethyl)phenyl]-1H-pyrazolo[3,4-d]pyrimidin-4-yl)azetidin-3-yl]piperidin-4-ol |
| I-62 | 4-[4-(1-aminocyclopropyl)piperidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine |
| I-63 | trans-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-(trifluoromethyl)cyclobutanol |
| I-64-1 | 2-(1-{6-[(3R)-1-azabicyclo[2.2.2]octan-3-ylamino]-1-[3-(trifluoromethyl)phenyl]-1H-pyrazolo[3,4-d]pyrimidin-4-yl}piperidin-4-yl)propan-2-ol |
| I-64-2 | 2-(1-{6-[(3S)-1-azabicyclo[2.2.2]octan-3-ylamino]-1-[3-(trifluoromethyl)phenyl]-1H-pyrazolo[3,4-d]pyrimidin-4-yl}piperidin-4-yl)propan-2-ol |
| I-65 | 2-[1-(6-{[rel-(3R,4R)-4-fluoropyrrolidin-3-yl]amino}-1-[3-(trifluoromethyl)phenyl]-1H-pyrazolo[3,4-d]pyrimidin-4-yl)piperidin-4-yl]propan-2-ol |
| I-66 | cis-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylcyclobutanecarboxylic acid |
| I-67 | 2-methyl-1-[1-(6-{[trans-3-(methylsulfonyl)cyclobutyl]amino}-1-[3-(trifluoromethyl)phenyl]-1H-pyrazolo[3,4-d]pyrimidin-4-yl)azetidin-3-yl]propan-2-ol |
| I-68 | 2-methyl-1-[1-(6-{[1-(methylsulfonyl)azetidin-3-yl]amino}-1-[3-(trifluoromethyl)phenyl]-1H-pyrazolo[3,4-d]pyrimidin-4-yl)azetidin-3-yl]propan-2-ol |
| I-69 | (3R,4S)-4-({4-[3-(morpholin-4-yl)azetidin-1-yl]-1-[3-(trifluoromethyl)phenyl]-1H-pyrazolo[3,4-d]pyrimidin-6-yl}amino)tetrahydrofuran-3-ol |
| I-70 | rel-(3R,4R)-4-({4-[3-(morpholin-4-yl)azetidin-1-yl]-1-[3-(trifluoromethyl)phenyl]-1H-pyrazolo[3,4-d]pyrimidin-6-yl}amino)tetrahydrofuran-3-ol |
| I-71 | trans-1-methyl-N³-{4-[3-(methylsulfonyl)azetidin-1-yl]-1-[3-(trifluoromethyl)phenyl]-1H-pyrazolo[3,4-d]pyrimidin-6-yl}cyclobutane-1,3-diamine |
| I-72 | cis-3-({4-[3-(morpholin-4-yl)azetidin-1-yl]-1-[3-(trifluoromethyl)phenyl]-1 H-pyrazolo[3,4-d]pyrimidin-6-yl}amino)cyclobutanol |
| I-73 | rel-(1 R,2S)-2-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-74 | trans-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-75 | rel-(1 R,2R)-2-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-76 | cis-3-({4-[3-methyl-3-(morpholin-4-yl)azetidin-1-yl]-1-[3-(trifluoromethyl)phenyl]-1H-pyrazolo[3,4-d]pyrimidin-6-yl}amino)cyclobutanol |
| I-77 | trans-1-methyl-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-78 | cis-1-methyl-3-({4-[3-(morpholin-4-yl)azetidin-1-yl]-1-[3-(trifluoromethyl)phenyl]-1H-pyrazolo[3,4-d]pyrimidin-6-yl}amino)cyclobutanol |
| I-79 | 1-(1-{6-[(trans-3-hydroxycyclobutyl)amino]-1-[3-(trifluoromethyl)phenyl]-1H-pyrazolo[3,4-d]pyrimidin-4-yl}azetidin-3-yl)-4-methylpiperidin-4-ol |
| I-80 | 1-(1-{6-[(1,1-dioxidotetrahydro-2H-thiopyran-4-yl)amino]-1-[3-(trifluoromethyl)phenyl]-1H-pyrazolo[3,4-d]pyrimidin-4-yl}azetidin-3-yl)-4-methylpiperidin-4-ol |
| I-81 | N-[1-(ethylsulfonyl)azetidin-3-yl]-4-[3-(morpholin-4-yl)azetidin-1-yl]-1-[3-(trifluoromethyl)phenyl]-1H-pyrazolo[3,4-d]pyrimidin-6-amine |
| I-82 | N-[trans-3-(ethylsulfonyl)cyclobutyl]-4-[3-(morpholin-4-yl)azetidin-1-yl]-1-[3-(trifluoromethyl)phenyl]-1H-pyrazolo[3,4-d]pyrimidin-6-amine |
| I-83 | 2-[1-(6-{[trans-3-(methylsulfonyl)cyclobutyl]amino}-1-[3-(trifluoromethyl)phenyl]-1H-pyrazolo[3,4-d]pyrimidin-4-yl)azetidin-3-yl]propan-2-ol |
| I-84 | (3R,4R)-1-(6-{[1-(ethylsulfonyl)azetidin-3-yl]amino}-1-[3-(trifluoromethyl)phenyl]-1H-pyrazolo[3,4-d]pyrimidin-4-yl)pyrrolidine-3,4-diol |
| I-85 | (3R,4R)-1-(6-{[(1s,3S)-3-hydroxycyclobutyl]amino}-1-[3-(trifluoromethyl)phenyl]-1H-pyrazolo[3,4-d]pyrimidin-4-yl)pyrrolidine-3,4-diol |
| I-86 | (3R,4R)-4-fluoro-1-(6-{[(1r,3R)-3-(methylsulfonyl)cyclobutyl]amino}-1-[3-(trifluoromethyl)phenyl]-1H-pyrazolo[3,4-d]pyrimidin-4-yl)pyrrolidin-3-ol |
| I-87 | (3R,4R)-1-(6-{[(1r,3R)-3-(ethylsulfonyl)cyclobutyl]amino}-1-[3-(trifluoromethyl)phenyl]-1H-pyrazolo[3,4-d]pyrimidin-4-yl)-4-fluoropyrrolidin-3-ol |
| I-88 | (3R,4R)-4-fluoro-1-(6-{[(1r,3R)-3-hydroxycyclobutyl]amino}-1-[3-(trifluoromethyl)phenyl]-1H-pyrazolo[3,4-d]pyrimidin-4-yl)pyrrolidin-3-ol |
| I-89 | (3R,4R)-4-fluoro-1-(6-{[(1s,3S)-3-hydroxycyclobutyl]amino}-1-[3-(trifluoromethyl)phenyl]-1H-pyrazolo[3,4-d]pyrimidin-4-yl)pyrrolidin-3-ol |
| I-90 | cis-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylcyclobutanol |
| I-91 | trans-3-({4-[4-(2-hydroxypropan-2-yl)piperidin-1-yl]-1-[3-(trifluoromethyl)phenyl]-1H-pyrazolo[3,4-d]pyrimidin-6-yl}amino)-1-methylcyclobutanol |
| I-92 | 3-ethyl-1-(6-{[trans-3-(methylsulfonyl)cyclobutyl]amino}-1-[3-(trifluoromethyl)phenyl]-1H-pyrazolo[3,4-d]pyrimidin-4-yl)azetidin-3-ol |
| I-93 | 1-(6-{[trans-3-(methylsulfonyl)cyclobutyl]amino}-1-[3-(trifluoromethyl)phenyl]-1H-pyrazolo[3,4-d]pyrimidin-4-yl)piperidin-4-ol |
| I-94 | (3S,4S)-4-fluoro-1-(6-{[(1r,3S)-3-(methylsulfonyl)cyclobutyl]amino}-1-[3-(trifluoromethyl)phenyl]-1H-pyrazolo[3,4-d]pyrimidin-4-yl)pyrrolidin-3-ol |
| I-95 | (3S,4S)-1-(6-{[(1r,3S)-3-(ethylsulfonyl)cyclobutyl]amino}-1-[3-(trifluoromethyl)phenyl]-1H-pyrazolo[3,4-d]pyrimidin-4-yl)-4-fluoropyrrolidin-3-ol |
| I-96 | (3S,4S)-4-fluoro-1-(6-{[(1r,3S)-3-hydroxycyclobutyl]amino}-1-[3-(trifluoromethyl)phenyl]-1H-pyrazolo[3,4-d]pyrimidin-4-yl)pyrrolidin-3-ol |
| I-97 | (3S,4S)-4-fluoro-1-(6-{[(1s,3R)-3-hydroxycyclobutyl]amino}-1-[3-(trifluoromethyl)phenyl]-1H-pyrazolo[3,4-d]pyrimidin-4-yl)pyrrolidin-3-ol |
| I-98 | (3S,4S)-1-{6-[(4S,6s)-1-azaspiro[3.3]heptan-6-ylamino]-1-[3-(trifluoromethyl)phenyl]-1H-pyrazolo[3,4-d]pyrimidin-4-yl}-4-fluoropyrrolidin-3-ol |
| I-99 | 4-(3-fluoroazetidin-1-yl)-N-[trans-3-(methylsulfonyl)cyclobutyl]-1-[3-(trifluoromethyl)phenyl]-1H-pyrazolo[3,4-d]pyrimidin-6-amine |
| I-100 | 1-{6-[(trans-3-hydroxycyclobutyl)amino]-1-[3-(trifluoromethyl)phenyl]-1H-pyrazolo[3,4-d]pyrimidin-4-yl}piperidin-4-ol |
| I-101 | (3R,4R)-1-(6-{[(1r,3R)-3-hydroxycyclobutyl]amino}-1-[3-(trifluoromethyl)phenyl]-1H-pyrazolo[3,4-d]pyrimidin-4-yl)pyrrolidine-3,4-diol |
| I-102 | (3R)-1-(6-{[(1r,3R)-3-hydroxycyclobutyl]amino}-1-[3-(trifluoromethyl)phenyl]-1H-pyrazolo[3,4-d]pyrimidin-4-yl)-3-methylpyrrolidin-3-ol |
| I-103 | (3R)-3-methyl-1-(6-{[(1r,3R)-3-(methylsulfonyl)cyclobutyl]amino}-1-[3-(trifluoromethyl)phenyl]-1H-pyrazolo[3,4-d]pyrimidin-4-yl)pyrrolidin-3-ol |
| I-104 | trans-3-({4-[(4RS)-4-amino-3,3-difluoropiperidin-1-yl]-1-[3-(trifluoromethyl)phenyl]-1H-pyrazolo[3,4-d]pyrimidin-6-yl}amino)cyclobutanol |
| I-105 | 4-[(4RS)-4-amino-3,3-difluoropiperidin-1-yl]-N-(1,1-dioxidotetrahydro-2H-thiopyran-4-yl)-1-[3-(trifluoromethyl)phenyl]-1H-pyrazolo[3,4-d]pyrimidin-6-amine |
| I-106 | N-(1,1-dioxidotetrahydro-2H-thiopyran-4-yl)-4-[3-methyl-3-(morpholin-4-yl)azetidin-1-yl]-1-[3-(trifluoromethyl)phenyl]-1H-pyrazolo[3,4-d]pyrimidin-6-amine |
| I-107 | (3R,4R)-1-(6-{[(1s,3S)-3-hydroxy-3-methylcyclobutyl]amino}-1-[3-(trifluoromethyl)phenyl]-1H-pyrazolo[3,4-d]pyrimidin-4-yl)pyrrolidine-3,4-diol |
| I-108 | (3R,4R)-1-(6-{[(1r,3R)-3-hydroxy-3-methylcyclobutyl]amino}-1-[3-(trifluoromethyl)phenyl]-1H-pyrazolo[3,4-d]pyrimidin-4-yl)pyrrolidine-3,4-diol |
| I-109 | 1-(1-{6-[(cis-3-hydroxycyclobutyl)amino]-1-[3-(trifluoromethyl)phenyl]-1H-pyrazolo[3,4-d]pyrimidin-4-yl}azetidin-3-yl)-4-methylpiperidin-4-ol |
| I-110 | 1-(1-{6-[(trans-3-hydroxy-3-methylcyclobutyl)amino]-1-[3-(trifluoromethyl)phenyl]-1H-pyrazolo[3,4-d]pyrimidin-4-yl}azetidin-3-yl)-4-methylpiperidin-4-ol |
| I-111 | 1-(1-{6-[(cis-3-hydroxy-3-methylcyclobutyl)amino]-1-[3-(trifluoromethyl)phenyl]-1H-pyrazolo[3,4-d]pyrimidin-4-yl}azetidin-3-yl)-4-methylpiperidin-4-ol |
| I-112 | 1-(1-{6-[(cis-3-hydroxycyclobutyl)amino]-1-[3-(trifluoromethyl)phenyl]-1H-pyrazolo[3,4-d]pyrimidin-4-yl}azetidin-3-yl)pyrrolidin-2-one |
| I-113 | 1-(1-{6-[(trans-3-hydroxycyclobutyl)amino]-1-[3-(trifluoromethyl)phenyl]-1H-pyrazolo[3,4-d]pyrimidin-4-yl}azetidin-3-yl)pyrrolidin-2-one |
| I-114 | 1-{6-[(cis-3-hydroxycyclobutyl)amino]-1-[3-(trifluoromethyl)phenyl]-1H-pyrazolo[3,4-d]pyrimidin-4-yl}-4-(pyrrolidin-1-ylmethyl)piperidin-4-ol |
| I-115 | 4-(1-{6-[(cis-3-hydroxycyclobutyl)amino]-1-[3-(trifluoromethyl)phenyl]-1H-pyrazolo[3,4-d]pyrimidin-4-yl}azetidin-3-yl)piperazin-2-one |
| I-116 | cis-3-({4-[3-(2-hydroxypropan-2-yl)azetidin-1-yl]-1-[3-(trifluoromethyl)phenyl]-1H-pyrazolo[3,4-d]pyrimidin-6-yl}amino)cyclobutanol |
| I-117 | (3R)-1-(6-{[(1s,3S)-3-hydroxycyclobutyl]amino}-1-[3-(trifluoromethyl)phenyl]-1H-pyrazolo[3,4-d]pyrimidin-4-yl)pyrrolidin-3-ol |
| I-118 | cis-3-({4-[3-(3,3-difluoropyrrolidin-1-yl)azetidin-1-yl]-1-[3-(trifluoromethyl)phenyl]-1H-pyrazolo[3,4-d]pyrimidin-6-yl}amino)cyclobutanol |
| I-119 | trans-3-({4-(3-fluoro[1,3'-biazetidin]-1'-yl)-1-[3-(trifluoromethyl)phenyl]-1H-pyrazolo[3,4-d]pyrimidin-6-yl}amino)cyclobutanol |
| I-120 | trans-3-({4-(3-fluoro[1,3'-biazetidin]-1'-yl)-1-[3-(trifluoromethyl)phenyl]-1H-pyrazolo[3,4-d]pyrimidin-6-yl}amino)-1-methylcyclobutanol |
| I-121 | 4-(1-{6-[(cis-3-hydroxycyclobutyl)amino]-1-[3-(trifluoromethyl)phenyl]-1H-pyrazolo[3,4-d]pyrimidin-4-yl}azetidin-3-yl)morpholin-3-one |
| I-122 | 4-(1-{6-[(trans-3-hydroxy-3-methylcyclobutyl)amino]-1-[3-(trifluoromethyl)phenyl]-1H-pyrazolo[3,4-d]pyrimidin-4-yl}azetidin-3-yl)morpholin-3-one |
| I-123 | trans-1-methyl-3-({4-[3-methyl-3-(morpholin-4-yl)azetidin-1-yl]-1-[3-(trifluoromethyl)phenyl]-1H-pyrazolo[3,4-d]pyrimidin-6-yl}amino)cyclobutanol |
| I-124 | (3S)-1-(6-{[(1s,3R)-3-hydroxycyclobutyl]amino}-1-[3-(trifluoromethyl)phenyl]-1H-pyrazolo[3,4-d]pyrimidin-4-yl)-3-methylpyrrolidin-3-ol |
| II-1 | (4S)-4-({1-[3-(difluoromethyl)phenyl]-4-[3-(morpholin-4-yl)azetidin-1-yl]-1H-pyrazolo[3,4-d]pyrimidin-6-yl}amino)pyrrolidin-2-one |
| II-2 | 1-[3-(difluoromethyl)phenyl]-N-(1,1-dioxidotetrahydro-2H-thiopyran-4-yl)-4-[3-(morpholin-4-yl)azetidin-1-yl]-1H-pyrazolo[3,4-d]pyrimidin-6-amine |
| II-3 | 1-[3-(difluoromethyl)phenyl]-N-[(3S)-1,1-dioxidotetrahydrothiophen-3-yl]-4-[3-(morpholin-4-yl)azetidin-1-yl]-1H-pyrazolo[3,4-d]pyrimidin-6-amine |
| II-4 | 2-[1-(1-[3-(difluoromethyl)phenyl]-6-{[(3S)-1-methylpyrrolidin-3-yl]amino}-1H-pyrazolo[3,4-d]pyrimidin-4-yl)piperidin-4-yl]propan-2-ol |
| II-5 | 2-(1-{1-[3-(difluoromethyl)phenyl]-6-[(1,1-dioxidotetrahydro-2H-thiopyran-4-yl)amino]-1H-pyrazolo[3,4-d]pyrimidin-4-yl}piperidin-4-yl)propan-2-ol |
| II-6 | 2-[1-(1-[3-(difluoromethyl)phenyl]-6-{[(3S)-1,1-dioxidotetrahydrothiophen-3-yl]amino}-1H-pyrazolo[3,4-d]pyrimidin-4-yl)piperidin-4-yl]propan-2-ol |
| II-7 | 1-(1-{1-[3-(difluoromethyl)phenyl]-6-[(1,1-dioxidotetrahydro-2H-thiopyran-4-yl)amino]-1H-pyrazolo[3,4-d]pyrimidin-4-yl}azetidin-3-yl)-2-methylpropan-2-ol |
| II-8 | 1-[3-(difluoromethyl)phenyl]-N-[trans-3-(methylsulfonyl)cyclobutyl]-4-[3-(morpholin-4-yl)azetidin-1-yl]-1H-pyrazolo[3,4-d]pyrimidin-6-amine |
| II-9 | (1-{1-[3-(difluoromethyl)phenyl]-6-[(1,1-dioxidotetrahydro-2H-thiopyran-4-yl)amino]-1 H-pyrazolo[3,4-d]pyrimidin-4-yl}azetidin-3-yl)acetic acid |
| II-10 | 2-(1-{1-[3-(difluoromethyl)phenyl]-6-[(1,1-dioxidotetrahydro-2H-thiopyran-4-yl)amino]-1H-pyrazolo[3,4-d]pyrimidin-4-yl}azetidin-3-yl)-1-(morpholin-4-yl)ethanone |
| II-11 | 2-[1-(1-[3-(difluoromethyl)phenyl]-6-{[trans-3-(methylsulfonyl)cyclobutyl]amino}-1H-pyrazolo[3,4-d]pyrimidin-4-yl)piperidin-4-yl]propan-2-ol |
| II-12 | 2-[1-(1-[3-(difluoromethyl)phenyl]-6-{[1-(methylsulfonyl)azetidin-3-yl]amino}-1H-pyrazolo[3,4-d]pyrimidin-4-yl)piperidin-4-yl]propan-2-ol |
| II-13 | 1-[3-(difluoromethyl)phenyl]-N-[1-(methylsulfonyl)azetidin-3-yl]-4-[3-(morpholin-4-yl)azetidin-1-yl]-1H-pyrazolo[3,4-d]pyrimidin-6-amine |
| II-14 | 1-[1-(1-[3-(difluoromethyl)phenyl]-6-{[1-(methylsulfonyl)azetidin-3-yl]amino}-1H-pyrazolo[3,4-d]pyrimidin-4-yl)azetidin-3-yl]-2-methylpropan-2-ol |
| II-15 | 1-{1-[3-(difluoromethyl)phenyl]-6-[(1,1-dioxidotetrahydro-2H-thiopyran-4-yl)amino]-1H-pyrazolo[3,4-d]pyrimidin-4-yl}-3-(trifluoromethyl)pyrrolidin-3-ol |
| II-16-1 | rel-(3R,4R)-1-(1-[3-(difluoromethyl)phenyl]-6-{[trans-3-(methylsulfonyl)cyclobutyl]amino}-1H-pyrazolo[3,4-d]pyrimidin-4-yl)-4-fluoropyrrolidin-3-ol |
| II-16-2 | (3S,4S)-1-(1-[3-(difluoromethyl)phenyl]-6-{[trans-3-(methanesulfonyl)cyclobutyl]amino}-1H-pyrazolo[3,4-d]pyrimidin-4-yl)-4-fluoropyrrolidin-3-ol |
| II-16-3 | (3R,4R)-1-(1-[3-(difluoromethyl)phenyl]-6-{[trans-3-(methanesulfonyl)cyclobutyl]amino}-1H-pyrazolo[3,4-d]pyrimidin-4-yl)-4-fluoropyrrolidin-3-ol |
| II-17-1 | rel-(3R,4S)-1-(1-[3-(difluoromethyl)phenyl]-6-{[trans-3-(methylsulfonyl)cyclobutyl]amino}-1H-pyrazolo[3,4-d]pyrimidin-4-yl)-4-methylpyrrolidin-3-ol |
| II-17-2 | (3R*,4S*)-1-(1-[3-(difluoromethyl)phenyl]-6-{[trans-3-(methylsulfonyl)cyclobutyl]amino}-1H-pyrazolo[3,4-d]pyrimidin-4-yl)-4-methylpyrrolidin-3-ol (single enantiomer 1) |
| II-17-3 | (3R*,4S*)-1-(1-[3-(difluoromethyl)phenyl]-6-{[trans-3-(methylsulfonyl)cyclobutyl]amino}-1H-pyrazolo[3,4-d]pyrimidin-4-yl)-4-methylpyrrolidin-3-ol (single enantiomer 2) |
| II-18 | 2-(1-{6-(*trans*-1-azaspiro[3.3]heptan-6-ylamino)-1-[3-(difluoromethyl)phenyl]-1H-pyrazolo[3,4-d]pyrimidin-4-yl}piperidin-4-yl)propan-2-ol |
| II-19 | 1-[1-(1-[3-(difluoromethyl)phenyl]-6-{[trans-3-(methylsulfonyl)cyclobutyl]amino}-1H-pyrazolo[3,4-d]pyrimidin-4-yl)azetidin-3-yl]-2-methylpropan-2-ol |
| II-20 | 4-[4-(1-aminocyclopropyl)piperidin-1-yl]-1-[3-(difluoromethyl)phenyl]-N-[1-(methylsulfonyl)azetidin-3-yl]-1H-pyrazolo[3,4-d]pyrimidin-6-amine |
| II-21 | 1-[3-(difluoromethyl)phenyl]-4-[3-methyl-3-(morpholin-4-yl)azetidin-1-yl]-N-[trans-3-(methylsulfonyl)cyclobutyl]-1H-pyrazolo[3,4-d]pyrimidin-6-amine |
| II-22 | (3S,4S)-1-(1-[3-(difluoromethyl)phenyl]-6-{[1-(methylsulfonyl)azetidin-3-yl]amino}-1H-pyrazolo[3,4-d]pyrimidin-4-yl)-4-fluoropyrrolidin-3-ol |
| II-23 | (3R,4R)-1-(1-[3-(difluoromethyl)phenyl]-6-{[1-(methylsulfonyl)azetidin-3-yl]amino}-1H-pyrazolo[3,4-d]pyrimidin-4-yl)-4-fluoropyrrolidin-3-ol |
| II-24 | cis-3-({1-[3-(difluoromethyl)phenyl]-4-[3-(morpholin-4-yl)azetidin-1-yl]-1H-pyrazolo[3,4-d]pyrimidin-6-yl}amino)cyclobutanol |
| II-25 | trans-3-({1-[3-(difluoromethyl)phenyl]-4-[3-(morpholin-4-yl)azetidin-1-yl]-1H-pyrazolo[3,4-d]pyrimidin-6-yl}amino)cyclobutanol |
| II-26 | 2-(1-{1-[3-(difluoromethyl)phenyl]-6-[(1,1-dioxidotetrahydro-2H-thiopyran-4-yl)amino]-1H-pyrazolo[3,4-d]pyrimidin-4-yl}azetidin-3-yl)-2-methylpropanoic acid |
| II-27 | 4-(1-{1-[3-(difluoromethyl)phenyl]-6-[(trans-3-hydroxycyclobutyl)amino]-1H-pyrazolo[3,4-d]pyrimidin-4-yl}azetidin-3-yl)piperazin-2-one |
| II-28 | 1-[1-(1-[3-(difluoromethyl)phenyl]-6-{[1-(ethylsulfonyl)azetidin-3-yl]amino}-1H-pyrazolo[3,4-d]pyrimidin-4-yl)azetidin-3-yl]-2-methylpropan-2-ol |
| II-29 | (3R)-1-(1-[3-(difluoromethyl)phenyl]-6-{[1-(methylsulfonyl)azetidin-3-yl]amino}-1H-pyrazolo[3,4-d]pyrimidin-4-yl)-3-methylpyrrolidin-3-ol |
| II-30 | 2-[1-(6-{[1-(cyclopropylsulfonyl)azetidin-3-yl]amino}-1-[3-(difluoromethyl)phenyl]-1H-pyrazolo[3,4-d]pyrimidin-4-yl)piperidin-4-yl]propan-2-ol |
| II-31 | 2-[1-(1-[3-(difluoromethyl)phenyl]-6-{[1-(ethylsulfonyl)azetidin-3-yl]amino}-1H-pyrazolo[3,4-d]pyrimidin-4-yl)piperidin-4-yl]propan-2-ol |
| II-32 | 1-[3-(difluoromethyl)phenyl]-N-[1-(ethylsulfonyl)azetidin-3-yl]-4-[3-(morpholin-4-yl)azetidin-1-yl]-1H-pyrazolo[3,4-d]pyrimidin-6-amine |
| II-33 | (3R)-1-(1-[3-(difluoromethyl)phenyl]-6-{[(1r,3R)-3-hydroxycyclobutyl]amino}-1H-pyrazolo[3,4-d]pyrimidin-4-yl)pyrrolidin-3-ol |
| II-34 | (3R)-1-(1-[3-(difluoromethyl)phenyl]-6-{[(1r,3R)-3-(methylsulfonyl)cyclobutyl]amino}-1H-pyrazolo[3,4-d]pyrimidin-4-yl)pyrrolidin-3-ol |
| II-35 | (3R)-1-(1-[3-(difluoromethyl)phenyl]-6-{[(1r,3R)-3-(methylsulfonyl)cyclobutyl]amino}-1H-pyrazolo[3,4-d]pyrimidin-4-yl)-3-methylpyrrolidin-3-ol |
| II-36 | 1-(1-{6-[(4r,6s)-1-azaspiro[3.3]heptan-6-ylamino]-1-[3-(difluoromethyl)phenyl]-1H-pyrazolo[3,4-d]pyrimidin-4-yl}azetidin-3-yl)-2-methylpropan-2-ol |
| III-1 | (4S)-4-({4-[4-(2-hydroxypropan-2-yl)piperidin-1-yl]-1-[3-(trifluoromethoxy)phenyl]-1H-pyrazolo[3,4-d]pyrimidin-6-yl}amino)pyrrolidin-2-one |
| IV-1 | (4S)-4-({1-[3-(difluoromethoxy)phenyl]-4-[4-(2-hydroxypropan-2-yl)piperidin-1-yl]-1H-pyrazolo[3,4-d]pyrimidin-6-yl}amino)pyrrolidin-2-one |
| IV-2 | 2-[1-(1-[3-(difluoromethoxy)phenyl]-6-{[trans-3-(methylsulfonyl)cyclobutyl]amino}-1H-pyrazolo[3,4-d]pyrimidin-4-yl)piperidin-4-yl]propan-2-ol |
| IV-3 | 4-[4-(1-aminocyclopropyl)piperidin-1-yl]-1-[3-(difluoromethoxy)phenyl]-N-[1-(methylsulfonyl)azetidin-3-yl]-1H-pyrazolo[3,4-d]pyrimidin-6-amine |
| IV-4 | 1-[1-(1-[3-(difluoromethoxy)phenyl]-6-{[trans-3-(methylsulfonyl)cyclobutyl]amino}-1H-pyrazolo[3,4-d]pyrimidin-4-yl)azetidin-3-yl]-2-methylpropan-2-ol |
| IV-5 | (3S,4S)-1-(1-[3-(difluoromethoxy)phenyl]-6-{[trans-3-(methylsulfonyl)cyclobutyl]amino}-1H-pyrazolo[3,4-d]pyrimidin-4-yl)-4-fluoropyrrolidin-3-ol |
| IV-6 | (3S,4S)-1-(1-[3-(difluoromethoxy)phenyl]-6-{[1-(methylsulfonyl)azetidin-3-yl]amino}-1H-pyrazolo[3,4-d]pyrimidin-4-yl)-4-fluoropyrrolidin-3-ol |
| IV-7 | (3S,4S)-1-{1-[3-(difluoromethoxy)phenyl]-6-[(1,1-dioxidotetrahydro-2H-thiopyran-4-yl)amino]-1H-pyrazolo[3,4-d]pyrimidin-4-yl}-4-fluoropyrrolidin-3-ol |
| V-1 | 3-ethyl-1-(6-{[(3S)-1-methylpyrrolidin-3-yl]amino}-1-[3-(methylsulfonyl)phenyl]-1H-pyrazolo[3,4-d]pyrimidin-4-yl)azetidin-3-ol |
| V-2 | 3-ethyl-1-{1-[3-(methylsulfonyl)phenyl]-6-[(3R)-tetrahydrofuran-3-ylamino]-1H-pyrazolo[3,4-d]pyrimidin-4-yl}azetidin-3-ol |
| VI-1 | 2-[1-(1-[3-(2-hydroxypropan-2-yl)phenyl]-6-{[(3S)-1-methylpyrrolidin-3-yl]amino}-1H-pyrazolo[3,4-d]pyrimidin-4-yl)piperidin-4-yl]propan-2-ol |

3. A compound of general formula (I) according to any one of claims 1 to 2 for use in the treatment and/ or prophylaxis of a disease in humans and animals.

4. A pharmaceutical composition comprising a compound of general formula (I) according to any one of claims 1 to 2 and one or more pharmaceutically acceptable excipients.

5. Use of a compound of general formula (I) according to any one of claims 1 to 2 for the treatment or prophylaxis of a disease in humans and animals.

6. Use of a compound of general formula (I) according to any one of claims 1 to 2 for the preparation of a medicament for the treatment or prophylaxis of a disease in humans and animals.

7. Use according to claim 5 or 6, wherein the disease is a human disease.

8. Use according to claim 7, wherein the disease is a proliferative disorder, autoimmune disorder, metabolic or inflammatory disorder **characterized by** an overreacting immune system, in particular rheumatological disorder, inflammatory skin disorder, cardiovascular disorder, lung disorder, eye disorder, neurological disorder, pain disorder and cancer.

9. Use according to claim 8, wherein the disease is rheumatoid arthritis, spondyloarthritis (especially psoriatic spondyloarthritis and Bechterev's disease), chronic obstructive pulmonary disease (COPD), multiple sclerosis, systemic lupus erythematosus, psoriasis, gout, metabolic syndrome, insulin resistance, polycystic ovary syndrome (PCOS), endometriosis or inflammation-induced or chronic pain, or lymphoma.

10. Use according to claim 5 or 6, wherein the disease is an allergic and/or inflammatory disease in domestic animals, in particular canine atopic dermatitis or flea allergy dermatitis in dogs or cats.


SEQUENCE LISTING

<110> Bayer AG
      Bayer Pharma Aktiengesellschaft

<120> Substituted pyrazolopyrimidines as IRAK4 inhibitors

<130> BHC193003 EP

<160> 1

<170> BiSSAP 1.3.6

<210> 1
<211> 497
<212> PRT
<213> Artificial Sequence

<220>
<223> His-tagged IRAK4 of dog

<400> 1
Met Thr Ser His His His His His His Ser Ser Met Gly Ser Arg Thr
1               5                   10                  15
Ser Leu Tyr Lys Lys Ala Gly Phe Asp Tyr Asp Ile Pro Thr Thr Glu
            20                  25                  30
Asn Leu Tyr Phe Gln Gly Asn Lys Pro Ile Thr Ser Ser Thr Tyr Val
        35                  40                  45
Arg Cys Leu Asn Leu Gly Leu Ile Arg Lys Leu Ser Asp Phe Ile Asp
    50                  55                  60
Pro Gln Glu Gly Trp Lys Lys Leu Ala Val Ala Ile Lys Lys Pro Ser
65                  70                  75                  80
Gly Asp Asp Arg Tyr Asn Gln Phe His Ile Arg Arg Phe Glu Ala Leu
                85                  90                  95
Leu Gln Thr Gly Lys Ser Pro Thr Cys Glu Leu Leu Phe Asp Trp Gly
            100                 105                 110
Thr Thr Asn Cys Thr Val Gly Asp Leu Val Asp Leu Leu Ile Gln Asn
        115                 120                 125
Glu Phe Phe Ala Pro Ala Ser Leu Leu Leu Pro Asp Ala Ile Pro Lys
    130                 135                 140
Thr Pro Asn Thr Val Pro Ser Glu Glu Ala Val Thr Val Gln Gln Gln
145                 150                 155                 160
Gln Met Pro Leu Asn Gly Lys Asp Arg Thr Phe Val Leu Pro Val Gln
                165                 170                 175
Asn Phe Glu Gln Asn Tyr Met Pro Pro Asp Ser Ser Ser Pro Glu Asn
            180                 185                 190
Thr Ser Leu Glu Gly Ser Asp Thr Arg Phe His Ser Phe Ser Phe Tyr
        195                 200                 205
Glu Leu Lys Asn Val Thr Asn Asn Phe Asp Glu Arg Pro Ile Ser Leu
    210                 215                 220
Gly Gly Asn Lys Met Gly Glu Gly Gly Phe Gly Val Val Tyr Lys Gly
225                 230                 235                 240
Tyr Val Asn Asn Lys Thr Val Ala Val Lys Lys Leu Ala Ala Met Val
                245                 250                 255
Asp Ile Ser Thr Glu Glu Leu Lys Gln Gln Phe Asp Gln Glu Ile Lys
            260                 265                 270
Val Met Ala Lys Cys Gln His Glu Asn Leu Val Glu Leu Leu Gly Phe
        275                 280                 285
Ser Ser Asp Gly Asp Asp Leu Cys Leu Val Tyr Val Tyr Met Pro Asn
    290                 295                 300
Gly Ser Leu Leu Asp Arg Leu Ser Cys Leu Asp Gly Thr Pro Pro Leu
305                 310                 315                 320

Ser Trp His Met Arg Cys Lys Ile Ala Gln Gly Ala Ala Asn Gly Ile
                325                 330                 335
Ser Phe Leu His Glu Asn His His Ile His Arg Asp Ile Lys Ser Ala
            340                 345                 350
Asn Ile Leu Leu Asp Glu Asp Phe Thr Ala Lys Val Ser Asp Phe Gly
        355                 360                 365
Leu Ala Arg Ala Ser Glu Lys Phe Ala Gln Thr Val Met Thr Ser Arg
    370                 375                 380
Ile Val Gly Thr Thr Ala Tyr Met Ala Pro Glu Ala Leu Arg Gly Glu
385                 390                 395                 400
Ile Thr Pro Lys Ser Asp Ile Tyr Ser Phe Gly Val Val Leu Leu Glu
                405                 410                 415
Ile Ile Thr Gly Leu Pro Ala Val Asp Glu His Arg Glu Pro Gln Leu
            420                 425                 430
Leu Leu Asp Ile Lys Glu Glu Ile Glu Asp Glu Glu Lys Thr Ile Glu
        435                 440                 445
Asp Tyr Ile Asp Pro Lys Met Asn Asp Thr Asp Pro Thr Ser Ile Glu
    450                 455                 460
Thr Met Tyr Ser Val Ala Ser Gln Cys Leu His Glu Lys Lys Asn Lys
465                 470                 475                 480
Arg Pro Asp Ile Glu Lys Val Gln Gln Leu Leu Gln Asp Leu Thr Ala
                485                 490                 495
Ser